# EUROPEAN PATENT APPLICATION

(11) **EP 4 814 000 A1**
(43) Date of publication of application: **30.09.2026**
(21) Application number: 24884931.7
(22) Date of filing: 31.10.2024
(51) Int. Cl.: C07K 14/135, C12N 15/45, C07K 16/10, A61K 39/155, A61P 31/14

(54) **RSV ANTIGEN, NUCLEIC ACID, RECOMBINANT EXPRESSION VECTOR, PHARMACEUTICAL COMPOSITION AND USE THEREOF**

(30) Priority: 31.10.2023 CN 202311439797; 12.01.2024 CN 202410052814; 06.02.2024 CN 202410173687; 23.09.2024 CN 202411331507
(71) Applicant: Shanghai Rnacure Biopharma Co., Ltd., Shanghai 201906 (CN); Walvax Biotechnology Co., Ltd., Kunming, Yunnan 650101 (CN)
(72) Inventor: LIN, Jinzhong, Shanghai 201906 (CN); LU, Jing, Shanghai 201906 (CN); LI, Sheng, Shanghai 201906 (CN); GUO, Yu, Shanghai 201906 (CN); GU, Hao, Shanghai 201906 (CN); HUANG, Wei, Shanghai 201906 (CN); TAN, Shudan, Shanghai 201906 (CN)
(74) Representative: Ipsilon Belgium
(86) International application number: PCT/CN2024/129091
(87) International publication number: WO 2025/092933

(57) **Abstract**

The present invention relates to a respiratory syncytial virus (RSV) antigen, a nucleic acid encoding the same, a recombinant expression vector, and a pharmaceutical composition comprising the antigen or nucleic acid, as well as uses thereof.

The RSV antigen disclosed herein exhibits enhanced immunogenicity compared to RSV antigens known in the prior art, is capable of inducing higher neutralizing antibody titers, and provides improved protective efficacy. The RSV antigen of the present invention maintains a greater proportion of the prefusion conformation, thereby preferentially inducing neutralizing antibodies targeting the prefusion conformation while reducing the induction of antibodies directed against the postfusion conformation, resulting in an improved safety profile.

The RSV antigen, nucleic acid, recombinant expression vector, and pharmaceutical composition provided herein have broad application prospects in the manufacture of medicaments for alleviating, preventing, and/or treating diseases caused by RSV.

## Description

ST.26 SEQUENCE LISING - HUMAN READABLE FORMAT

Invention Title | 一种RSV抗原、核酸、重组表达载体、药物组合物及其应用 (zh)
Applicant File Reference | P24407313C
Applicant Name | 上海蓝鹊生物医药有限公司
Applicant Name Latin | Shanghai RNACure Biopharma Co., Ltd.
Inventor Name | -
Inventor Name Latin | -
File name | [P24407313C]SEQ.xml
DTD version | V1_3
Software name | WIPO Sequence
Software version | 2.3.0
Production date | 2024-10-31
Original free text language Code | -
Non English free text language Code | -
Application number | -
Filing date | -
Earliest priority number | 2023114397970
Earliest priority date | 2023-10-31
Sequence Total Quantity | 202

SEQ ID NO: 1
length 574
molecule type AA
division PAT

Key Location/Qualifiers
source 1..574
/mol_type=protein
/organism=synthetic construct

Sequence
MELPILKTNA ITTILAAVTL CFASSQNITE EFYQSTCSAV SKGYLSALRT GWYTSVITIE 60
LSNIKENKCN GTDAKVKLIK QELDKYKNAV TELQLLMQST PAANSRARRE LPRFMNYTLN 120
NTKNTNVTLS KKRKRRFLGF LLGVGSAIAS GIAVSKVLHL EGEVNKIKSA LLSTNKAVVS 180
LSNGVSVLTS KVLDLKNYID KQLLPIVNKQ SCSISNIETV IEFQQKNNRL LEITREFSVN 240
AGVTTPVSTY MLTNSELLSL INDMPITNDQ KKLMSSNVQI VRQQSYSIMS IIKEEVLAYV 300
VQLPLYGVID TPCWKLHTSP LCTTNTKEGS NICLTRTDRG WYCDNAGSVS FFPQAETCKV 360
QSNRVFCDTM NSLTLPSEVN LCNIDIFNPK YDCKIMTSKT DVSSSVITSL GAIVSCYGKT 420
KCTASNKNRG IIKTFSNGCD YVSNKGVDTV SVGNTLYYVN KQEGKSLYVK GEPIINFYDP 480
LVFPSDEFDA SISQVNEKIN QSLAFIRKSD ELLHNVNAGK STTNIMITTI IIVIIVILLA 540
LIAVGLLLYC KARSTPVTLS KDQLSGINNI AFSN 574

SEQ ID NO: 2
length 7
molecule type AA
division PAT

Key Location/Qualifiers
source 1..7
/mol_type=protein

/organism=synthetic construct

Sequence
GGSGGGS | 7

SEQ ID NO: | 3
length | 6
molecule type | AA
division | PAT

Key | Location/Qualifiers
source | 1..6
/mol_type=protein
/organism=synthetic construct

Sequence
GGSGGS | 6

SEQ ID NO: | 4
length | 5
molecule type | AA
division | PAT

Key | Location/Qualifiers
source | 1..5
/mol_type=protein
/organism=synthetic construct

Sequence
GGSGS | 5

SEQ ID NO: | 5
length | 4
molecule type | AA
division | PAT

Key | Location/Qualifiers
source | 1..4
/mol_type=protein
/organism=synthetic construct

Sequence
GSGS | 4

SEQ ID NO: | 6
length | 28
molecule type | AA
division | PAT

Key | Location/Qualifiers
source | 1..28
/mol_type=protein
/organism=synthetic construct

Sequence
EDKIEEILSK IYHIENEIAR IKKLIGEA | 28

SEQ ID NO: | 7
length | 44
molecule type | AA
division | PAT

Key                 Location/Qualifiers
source              1..44
                    /mol_type=protein
                    /organism=synthetic construct

Sequence
WPWYIWLGFI AGLIAIVMVT IMLCCMTSCC SCLKGCCSCG SCCK                    44

SEQ ID NO:          8
length              21
molecule type       AA
division            PAT

Key                 Location/Qualifiers
source              1..21
                    /mol_type=protein
                    /organism=synthetic construct

Sequence
NEKINQISAS IRKIDESISQ I                                             21

SEQ ID NO:          9
length              39
molecule type       AA
division            PAT

Key                 Location/Qualifiers
source              1..39
                    /mol_type=protein
                    /organism=synthetic construct

Sequence
NEKINQISAS IRKIDESINE KINQISASIR KIDESISQI                          39

SEQ ID NO:          10
length              574
molecule type       AA
division            PAT

Key                 Location/Qualifiers
source              1..574
                    /mol_type=protein
                    /organism=synthetic construct

Sequence
MELLILKANA ITTILTAVTF CFASGQNITE EFYQSTCSAV SKGYLSALRT GWYTSVITIE  60
LSNIKENKCN GTDAKVKLIK QELDKYKNAV TELQLLMQST PATNNRARRE LPRFMNYTLN  120
NAKKTNVTLS KKRKRRFLGF LLGVGSAIAS GVAVCKVLHL EGEVNKIKSA LLSTNKAVVS  180
LSNGVSVLTF KVLDLKNYID KQLLPILNKQ SCSISNIETV IEFQQKNNRL LEITREFSVN  240
AGVTTPVSTY MLTNSELLSL INDMPITNDQ KKLMSNNVQI VRQQSYSIMC IIKEEVLAYV  300
VQLPLYGVID TPCWKLHTSP LCTTNTKEGS NICLTRTDRG WYCDNAGSVS FFPQAETCKV  360
QSNRVFCDTM NSLTLPSEVN LCNVDIFNPK YDCKIMTSKT DVSSSVITSL GAIVSCYGKT  420
KCTASNKNRG IIKTFSNGCD YVSNKGVDTV SVGNTLYYVN KQEGKSLYVK GEPIINFYDP  480
LVFPSDEFDA SISQVNEKIN QSLAFIRKSD ELLHNVNAGK STTNIMITTI IIVIIVILLS  540
LIAVGLLLYC KARSTPVTLS KDQLSGINNI AFSN                               574

SEQ ID NO:          11
length              539
molecule type       AA
division            PAT

Key                 Location/Qualifiers
source              1..539
                    /mol_type=protein
                    /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIP  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360
MTSKTDVSSS VITSLGAIVS CYGKTKCTAS NKNRGIIKTF SNGCDYVSNK GVDTVSVGNT  420
LYYVNKQEGK SLYVKGEPII NFYDPLVFPS CEFCASISQV NEKINQSLAF IRKSDELLHN  480
VNAGKSTTNI MITTIIIVII VILLALIAVG LLLYCKARST PVTLSKDQLS GINNIAFSN   539

SEQ ID NO:          12
length              538
molecule type       AA
division            PAT

Key                 Location/Qualifiers
source              1..538
                    /mol_type=protein
                    /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGSGS AIASGIAVCK  120
VLHLEGEVNK IKSALLSTNK AVVSLSNGVS VLTFKVLDLK NYIDKQLLPI VNKQSCSIPN  180
IETVIEFQQK NNRLLEITRE FSVNAGVTTP VSTYMLTNSE LLSLINDMPI TNDQKKLMSS  240
NVQIVRQQSY SIMCIIKEEV LAYVVQLPLY GVIDTPCWKL HTSPLCTTNT KEGSNICLTR  300
TDRGWYCDNA GSVSFFPQAE TCKVQCNRVF CDTMNSLTLP SEVNLCNIDI FNPKYDCKIM  360
TSKTDVSSSV ITSLGAIVSC YGKTKCTASN KNRGIIKTFS NGCDYVSNKG VDTVSVGNTL  420
YYVNKQEGKS LYVKGEPIIN FYDPLVFPSC EFCASISQVN EKINQSLAFI RKSDELLHNV  480
NAGKSTTNIM ITTIIIVIIV ILLALIAVGL LLYCKARSTP VTLSKDQLSG INNIAFSN    538

SEQ ID NO:          13
length              532
molecule type       AA
division            PAT

Key                 Location/Qualifiers
source              1..532
                    /mol_type=protein
                    /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGSGS AIASGIAVCK  120
VLHLEGEVNK IKSALLSTNK AVVSLSNGVS VLTFKVLDLK NYIDKQLLPI VNKQSCSIPN  180
IETVIEFQQK NNRLLEITRE FSVNAGVTTP VSTYMLTNSE LLSLINDMPI TNDQKKLMSS  240
NVQIVRQQSY SIMCIIKEEV LAYVVQLPLY GVIDTPCWKL HTSPLCTTNT KEGSNICLTR  300
TDRGWYCDNA GSVSFFPQAE TCKVQCNRVF CDTMNSLTLP SEVNLCNIDI FNPKYDCKIM  360
TSKTDVSSSV ITSLGAIVSC YGKTKCTASN KNRGIIKTFS NGCDYVSNKG VDTVSVGNTL  420
YYVNKQEGKS LYVKGEPIIN FYDPLVFPSC EFCASISQVN EKINQSLAFI RKSDELLHNV  480
NAGKSTTNWP WYIWLGFIAG LIAIVMVTIM LCCMTSCCSC LKGCCSCGSC CK          532

SEQ ID NO:          14
length              566

molecule type        AA
division             PAT

Key                  Location/Qualifiers
source               1..566
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGSGS AIASGIAVCK  120
VLHLEGEVNK IKSALLSTNK AVVSLSNGVS VLTFKVLDLK NYIDKQLLPI VNKQSCSIPN  180
IETVIEFQQK NNRLLEITRE FSVNAGVTTP VSTYMLTNSE LLSLINDMPI TNDQKKLMSS  240
NVQIVRQQSY SIMCIIKEEV LAYVVQLPLY GVIDTPCWKL HTSPLCTTNT KEGSNICLTR  300
TDRGWYCDNA GSVSFFPQAE TCKVQCNRVF CDTMNSLTLP SEVNLCNIDI FNPKYDCKIM  360
TSKTDVSSSV ITSLGAIVSC YGKTKCTASN KNRGIIKTFS NGCDYVSNKG VDTVSVGNTL  420
YYVNKQEGKS LYVKGEPIIN FYDPLVFPSC EFCASISQVN EKINQSLAFI RKSDELLHNV  480
EDKIEEILSK IYHIENEIAR IKKLIGEANA GKSTTNIMIT TIIIVIIVIL LALIAVGLLL  540
YCKARSTPVT LSKDQLSGIN NIAFSN                                      566

SEQ ID NO:           15
length               560
molecule type        AA
division             PAT

Key                  Location/Qualifiers
source               1..560
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGSGS AIASGIAVCK  120
VLHLEGEVNK IKSALLSTNK AVVSLSNGVS VLTFKVLDLK NYIDKQLLPI VNKQSCSIPN  180
IETVIEFQQK NNRLLEITRE FSVNAGVTTP VSTYMLTNSE LLSLINDMPI TNDQKKLMSS  240
NVQIVRQQSY SIMCIIKEEV LAYVVQLPLY GVIDTPCWKL HTSPLCTTNT KEGSNICLTR  300
TDRGWYCDNA GSVSFFPQAE TCKVQCNRVF CDTMNSLTLP SEVNLCNIDI FNPKYDCKIM  360
TSKTDVSSSV ITSLGAIVSC YGKTKCTASN KNRGIIKTFS NGCDYVSNKG VDTVSVGNTL  420
YYVNKQEGKS LYVKGEPIIN FYDPLVFPSC EFCASISQVN EKINQSLAFI RKSDELLHNV  480
EDKIEEILSK IYHIENEIAR IKKLIGEANA GKSTTNWPWY IWLGFIAGLI AIVMVTIMLC  540
CMTSCCSCLK GCCSCGSCCK                                             560

SEQ ID NO:           16
length               537
molecule type        AA
division             PAT

Key                  Location/Qualifiers
source               1..537
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGSGSA IASGIAVCKV  120
LHLEGEVNKI KSALLSTNKA VVSLSNGVSV LTFKVLDLKN YIDKQLLPIV NKQSCSIPNI  180
ETVIEFQQKN NRLLEITREF SVNAGVTTPV STYMLTNSEL LSLINDMPIT NDQKKLMSSN  240
VQIVRQQSYS IMCIIKEEVL AYVVQLPLYG VIDTPCWKLH TSPLCTTNTK EGSNICLTRT  300
DRGWYCDNAG SVSFFPQAET CKVQCNRVFC DTMNSLTLPS EVNLCNIDIF NPKYDCKIMT  360
SKTDVSSSVI TSLGAIVSCY GKTKCTASNK NRGIIKTFSN GCDYVSNKGV DTVSVGNTLY  420
YVNKQEGKSL YVKGEPIINF YDPLVFPSCE FCASISQVNE KINQSLAFIR KSDELLHNVN  480

AGKSTTNIMI TTIIIVIIVI LLALIAVGLL LYCKARSTPV TLSKDQLSGI NNIAFSN 537

SEQ ID NO: 17
length 531
molecule type AA
division PAT

Key Location/Qualifiers
source 1..531
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSGSA IASGIAVCKV 120
LHLEGEVNKI KSALLSTNKA VVSLSNGVSV LTFKVLDLKN YIDKQLLPIV NKQSCSIPNI 180
ETVIEFQQKN NRLLEITREF SVNAGVTTPV STYMLTNSEL LSLINDMPIT NDQKKLMSSN 240
VQIVRQQSYS IMCIIKEEVL AYVVQLPLYG VIDTPCWKLH TSPLCTTNTK EGSNICLTRT 300
DRGWYCDNAG SVSFFPQAET CKVQCNRVFC DTMNSLTLPS EVNLCNIDIF NPKYDCKIMT 360
SKTDVSSSVI TSLGAIVSCY GKTKCTASNK NRGIIKTFSN GCDYVSNKGV DTVSVGNTLY 420
YVNKQEGKSL YVKGEPIINF YDPLVFPSCE FCASISQVNE KINQSLAFIR KSDELLHNVN 480
AGKSTTNWPW YIWLGFIAGL IAIVMVTIML CCMTSCCSCL KGCCSCGSCC K 531

SEQ ID NO: 18
length 565
molecule type AA
division PAT

Key Location/Qualifiers
source 1..565
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSGSA IASGIAVCKV 120
LHLEGEVNKI KSALLSTNKA VVSLSNGVSV LTFKVLDLKN YIDKQLLPIV NKQSCSIPNI 180
ETVIEFQQKN NRLLEITREF SVNAGVTTPV STYMLTNSEL LSLINDMPIT NDQKKLMSSN 240
VQIVRQQSYS IMCIIKEEVL AYVVQLPLYG VIDTPCWKLH TSPLCTTNTK EGSNICLTRT 300
DRGWYCDNAG SVSFFPQAET CKVQCNRVFC DTMNSLTLPS EVNLCNIDIF NPKYDCKIMT 360
SKTDVSSSVI TSLGAIVSCY GKTKCTASNK NRGIIKTFSN GCDYVSNKGV DTVSVGNTLY 420
YVNKQEGKSL YVKGEPIINF YDPLVFPSCE FCASISQVNE KINQSLAFIR KSDELLHNVE 480
DKIEEILSKI YHIENEIARI KKLIGEANAG KSTTNIMITT IIIVIIVILL ALIAVGLLLY 540
CKARSTPVTL SKDQLSGINN IAFSN 565

SEQ ID NO: 19
length 559
molecule type AA
division PAT

Key Location/Qualifiers
source 1..559
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSGSA IASGIAVCKV 120
LHLEGEVNKI KSALLSTNKA VVSLSNGVSV LTFKVLDLKN YIDKQLLPIV NKQSCSIPNI 180
ETVIEFQQKN NRLLEITREF SVNAGVTTPV STYMLTNSEL LSLINDMPIT NDQKKLMSSN 240
VQIVRQQSYS IMCIIKEEVL AYVVQLPLYG VIDTPCWKLH TSPLCTTNTK EGSNICLTRT 300

DRGWYCDNAG SVSFFPQAET CKVQCNRVFC DTMNSLTLPS EVNLCNIDIF NPKYDCKIMT 360
SKTDVSSSVI TSLGAIVSCY GKTKCTASNK NRGIIKTFSN GCDYVSNKGV DTVSVGNTLY 420
YVNKQEGKSL YVKGEPIINF YDPLVFPSCE FCASISQVNE KINQSLAFIR KSDELLHNVE 480
DKIEEILSKI YHIENEIARI KKLIGEANAG KSTTNWPWYI WLGFIAGLIA IVMVTIMLCC 540
MTSCCSCLKG CCSCGSCCK 559

SEQ ID NO: 20
length 536
molecule type AA
division PAT

Key Location/Qualifiers
source 1..536
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGSGSGSAI ASGIAVCKVL 120
HLEGEVNKIK SALLSTNKAV VSLSNGVSVL TFKVLDLKNY IDKQLLPIVN KQSCSIPNIE 180
TVIEFQQKNN RLLEITREFS VNAGVTTPVS TYMLTNSELL SLINDMPITN DQKKLMSSNV 240
QIVRQQSYSI MCIIKEEVLA YVVQLPLYGV IDTPCWKLHT SPLCTTNTKE GSNICLTRTD 300
RGWYCDNAGS VSFFPQAETC KVQCNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSCEF CASISQVNEK INQSLAFIRK SDELLHNVNA 480
GKSTTNIMIT TIIIVIIVIL LALIAVGLLL YCKARSTPVT LSKDQLSGIN NIAFSN 536

SEQ ID NO: 21
length 530
molecule type AA
division PAT

Key Location/Qualifiers
source 1..530
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGSGSGSAI ASGIAVCKVL 120
HLEGEVNKIK SALLSTNKAV VSLSNGVSVL TFKVLDLKNY IDKQLLPIVN KQSCSIPNIE 180
TVIEFQQKNN RLLEITREFS VNAGVTTPVS TYMLTNSELL SLINDMPITN DQKKLMSSNV 240
QIVRQQSYSI MCIIKEEVLA YVVQLPLYGV IDTPCWKLHT SPLCTTNTKE GSNICLTRTD 300
RGWYCDNAGS VSFFPQAETC KVQCNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSCEF CASISQVNEK INQSLAFIRK SDELLHNVNA 480
GKSTTNWPWY IWLGFIAGLI AIVMVTIMLC CMTSCCSCLK GCCSCGSCCK 530

SEQ ID NO: 22
length 564
molecule type AA
division PAT

Key Location/Qualifiers
source 1..564
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGSGSGSAI ASGIAVCKVL 120

HLEGEVNKIK SALLSTNKAV VSLSNGVSVL TFKVLDLKNY IDKQLLPIVN KQSCSIPNIE 180
TVIEFQQKNN RLLEITREFS VNAGVTTPVS TYMLTNSELL SLINDMPITN DQKKLMSSNV 240
QIVRQQSYSI MCIIKEEVLA YVVQLPLYGV IDTPCWKLHT SPLCTTNTKE GSNICLTRTD 300
RGWYCDNAGS VSFFPQAETC KVQCNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSCEF CASISQVNEK INQSLAFIRK SDELLHNVED 480
KIEEILSKIY HIENEIARIK KLIGEANAGK STTNIMITTI IIVIIVILLA LIAVGLLLYC 540
KARSTPVTLS KDQLSGINNI AFSN 564

SEQ ID NO: 23
length 558
molecule type AA
division PAT

Key Location/Qualifiers
source 1..558
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGSGSGSAI ASGIAVCKVL 120
HLEGEVNKIK SALLSTNKAV VSLSNGVSVL TFKVLDLKNY IDKQLLPIVN KQSCSIPNIE 180
TVIEFQQKNN RLLEITREFS VNAGVTTPVS TYMLTNSELL SLINDMPITN DQKKLMSSNV 240
QIVRQQSYSI MCIIKEEVLA YVVQLPLYGV IDTPCWKLHT SPLCTTNTKE GSNICLTRTD 300
RGWYCDNAGS VSFFPQAETC KVQCNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSCEF CASISQVNEK INQSLAFIRK SDELLHNVED 480
KIEEILSKIY HIENEIARIK KLIGEANAGK STTNWPWYIW LGFIAGLIAI VMVTIMLCCM 540
TSCCSCLKGC CSCGSCCK 558

SEQ ID NO: 24
length 535
molecule type AA
division PAT

Key Location/Qualifiers
source 1..535
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSAIA SGIAVCKVLH 120
LEGEVNKIKS ALLSTNKAVV SLSNGVSVLT FKVLDLKNYI DKQLLPIVNK QSCSIPNIET 180
VIEFQQKNNR LLEITREFSV NAGVTTPVST YMLTNSELLS LINDMPITND QKKLMSSNVQ 240
IVRQQSYSIM CIIKEEVLAY VVQLPLYGVI DTPCWKLHTS PLCTTNTKEG SNICLTRTDR 300
GWYCDNAGSV SFFPQAETCK VQCNRVFCDT MNSLTLPSEV NLCNIDIFNP KYDCKIMTSK 360
TDVSSSVITS LGAIVSCYGK TKCTASNKNR GIIKTFSNGC DYVSNKGVDT VSVGNTLYYV 420
NKQEGKSLYV KGEPIINFYD PLVFPSCEFC ASISQVNEKI NQSLAFIRKS DELLHNVNAG 480
KSTTNIMITT IIIVIIVILL ALIAVGLLLY CKARSTPVTL SKDQLSGINN IAFSN 535

SEQ ID NO: 25
length 529
molecule type AA
division PAT

Key Location/Qualifiers
source 1..529
/mol_type=protein

/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSAIA SGIAVCKVLH 120
LEGEVNKIKS ALLSTNKAVV SLSNGVSVLT FKVLDLKNYI DKQLLPIVNK QSCSIPNIET 180
VIEFQQKNNR LLEITREFSV NAGVTTPVST YMLTNSELLS LINDMPITND QKKLMSSNVQ 240
IVRQQSYSIM CIIKEEVLAY VVQLPLYGVI DTPCWKLHTS PLCTTNTKEG SNICLTRTDR 300
GWYCDNAGSV SFFPQAETCK VQCNRVFCDT MNSLTLPSEV NLCNIDIFNP KYDCKIMTSK 360
TDVSSSVITS LGAIVSCYGK TKCTASNKNR GIIKTFSNGC DYVSNKGVDT VSVGNTLYYV 420
NKQEGKSLYV KGEPIINFYD PLVFPSCEFC ASISQVNEKI NQSLAFIRKS DELLHNVNAG 480
KSTTNWPWYI WLGFIAGLIA IVMVTIMLCC MTSCCSCLKG CCSCGSCCK 529

SEQ ID NO: 26
length 563
molecule type AA
division PAT

Key Location/Qualifiers
source 1..563
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSAIA SGIAVCKVLH 120
LEGEVNKIKS ALLSTNKAVV SLSNGVSVLT FKVLDLKNYI DKQLLPIVNK QSCSIPNIET 180
VIEFQQKNNR LLEITREFSV NAGVTTPVST YMLTNSELLS LINDMPITND QKKLMSSNVQ 240
IVRQQSYSIM CIIKEEVLAY VVQLPLYGVI DTPCWKLHTS PLCTTNTKEG SNICLTRTDR 300
GWYCDNAGSV SFFPQAETCK VQCNRVFCDT MNSLTLPSEV NLCNIDIFNP KYDCKIMTSK 360
TDVSSSVITS LGAIVSCYGK TKCTASNKNR GIIKTFSNGC DYVSNKGVDT VSVGNTLYYV 420
NKQEGKSLYV KGEPIINFYD PLVFPSCEFC ASISQVNEKI NQSLAFIRKS DELLHNVEDK 480
IEEILSKIYH IENEIARIKK LIGEANAGKS TTNIMITTII IVIIVILLAL IAVGLLLYCK 540
ARSTPVTLSK DQLSGINNIA FSN 563

SEQ ID NO: 27
length 557
molecule type AA
division PAT

Key Location/Qualifiers
source 1..557
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSAIA SGIAVCKVLH 120
LEGEVNKIKS ALLSTNKAVV SLSNGVSVLT FKVLDLKNYI DKQLLPIVNK QSCSIPNIET 180
VIEFQQKNNR LLEITREFSV NAGVTTPVST YMLTNSELLS LINDMPITND QKKLMSSNVQ 240
IVRQQSYSIM CIIKEEVLAY VVQLPLYGVI DTPCWKLHTS PLCTTNTKEG SNICLTRTDR 300
GWYCDNAGSV SFFPQAETCK VQCNRVFCDT MNSLTLPSEV NLCNIDIFNP KYDCKIMTSK 360
TDVSSSVITS LGAIVSCYGK TKCTASNKNR GIIKTFSNGC DYVSNKGVDT VSVGNTLYYV 420
NKQEGKSLYV KGEPIINFYD PLVFPSCEFC ASISQVNEKI NQSLAFIRKS DELLHNVEDK 480
IEEILSKIYH IENEIARIKK LIGEANAGKS TTNWPWYIWL GFIAGLIAIV MVTIMLCCMT 540
SCCSCLKGCC SCGSCCK 557

SEQ ID NO: 28
length 567
molecule type AA

division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIEEILS KIYHIENEIA RIKKLIGEAN AGKSTTNIMI TTIIIVIIVI LLALIAVGLL 540
LYCKARSTPV TLSKDQLSGI NNIAFSN 567

SEQ ID NO: 29
length 585
molecule type AA
division PAT

Key Location/Qualifiers
source 1..585
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIEEILS KIYHIENEIA RIKKLIGEAI YHIENEIARI KKLIGEANAG KSTTNIMITT 540
IIIVIIVILL ALIAVGLLLY CKARSTPVTL SKDQLSGINN IAFSN 585

SEQ ID NO: 30
length 578
molecule type AA
division PAT

Key Location/Qualifiers
source 1..578
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELIHN 480
VNGSINQINA SIRKINESIN GSINQINASI RKINESISQI NAGKSTTNIM ITTIIIVIIV 540

ILLALIAVGL LLYCKARSTP VTLSKDQLSG INNIAFSN 578

SEQ ID NO: 31
length 560
molecule type AA
division PAT

Key Location/Qualifiers
source 1..560
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELIHN 480
VNEKINQISA SIRKIDESIS QINAGKSTTN IMITTIIIVI IVILLALIAV GLLLYCKARS 540
TPVTLSKDQL SGINNIAFSN 560

SEQ ID NO: 32
length 578
molecule type AA
division PAT

Key Location/Qualifiers
source 1..578
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELIHN 480
VNEKINQISA SIRKIDESIN EKINQISASI RKIDESISQI NAGKSTTNIM ITTIIIVIIV 540
ILLALIAVGL LLYCKARSTP VTLSKDQLSG INNIAFSN 578

SEQ ID NO: 33
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240

SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS DEFDASISQV NEKINQSLAF IRKSDELLHN 480
VEDKIEEILS KIYHIENEIA RIKKLIGEAN AGKSTTNIMI TTIIIVIIVI LLALIAVGLL 540
LYCKARSTPV TLSKDQLSGI NNIAFSN 567

SEQ ID NO: 34
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIP 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIEEILS KIYHIENEIA RIKKLIGEAN AGKSTTNIMI TTIIIVIIVI LLALIAVGLL 540
LYCKARSTPV TLSKDQLSGI NNIAFSN 567

SEQ ID NO: 35
length 533
molecule type AA
division PAT

Key Location/Qualifiers
source 1..533
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIP 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VNAGKSTTNW PWYIWLGFIA GLIAIVMVTI MLCCMTSCCS CLKGCCSCGS CCK 533

SEQ ID NO: 36
length 561
molecule type AA
division PAT

Key Location/Qualifiers
source 1..561
/mol_type=protein
/organism=synthetic construct

Sequence

MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIP 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIEEILS KIYHIENEIA RIKKLIGEAN AGKSTTNWPW YIWLGFIAGL IAIVMVTIML 540
CCMTSCCSCL KGCCSCGSCC K 561

SEQ ID NO: 37
length 539
molecule type AA
division PAT

Key Location/Qualifiers
source 1..539
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VNAGKSTTNI MITTIIIVII VILLALIAVG LLLYCKARST PVTLSKDQLS GINNIAFSN 539

SEQ ID NO: 38
length 539
molecule type AA
division PAT

Key Location/Qualifiers
source 1..539
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS DEFDASISQV NEKINQSLAF IRKSDELLHN 480
VNAGKSTTNI MITTIIIVII VILLALIAVG LLLYCKARST PVTLSKDQLS GINNIAFSN 539

SEQ ID NO: 39
length 560
molecule type AA
division PAT

Key Location/Qualifiers
source 1..560

/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELIHN 480
VNGSINQINA SIRKINESIS QINAGKSTTN IMITTIIIVI IVILLALIAV GLLLYCKARS 540
TPVTLSKDQL SGINNIAFSN 560

SEQ ID NO: 40
length 560
molecule type AA
division PAT

Key Location/Qualifiers
source 1..560
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS DEFDASISQV NEKINQSLAF IRKSDELIHN 480
VNGSINQINA SIRKINESIS QINAGKSTTN IMITTIIIVI IVILLALIAV GLLLYCKARS 540
TPVTLSKDQL SGINNIAFSN 560

SEQ ID NO: 41
length 1722
molecule type RNA
division PAT

Key Location/Qualifiers
source 1..1722
/mol_type=other RNA
/organism=synthetic construct

Sequence
atggagctgc tgatcctgaa ggccaacgcc atcaccacaa tcctgaccgc cgtgacattt 60
tgcttcgcta gcggccagaa catcacagag gaattttacc agagcacctg cagcgctgtg 120
agcaagggct acctgtctgc tctgcggacc ggctggtaca ccagcgtgat cacaatcgag 180
ctgagcaaca tcaaggagaa caagtgcaac ggcaccgacg ccaaggtgaa gctgatcaag 240
caggagctgg acaagtacaa gaacgccgtc acagagcttc agctcctgat gcagagcacc 300
cctgccacca ataaccgggc ccggagagag ctgcccaggt tcatgaatta caccctgaac 360
aacgccaaga agaccaacgt gaccctgtct aagaagagaa agcggagatt cctgggcttc 420
ctgctgggag tgggatctgc tattgcttct ggcgtggctg tgtgcaaagt gctgcacctg 480
gagggagagg tgaacaagat caagagcgcc ctcctgagca ccaacaaagc cgtggtgagc 540
ctgagcaacg gagtgagcgt gctgaccttc aaggtgctgg acctgaagaa ttacatcgac 600
aagcagctgc tgcctatcct gaacaagcag agctgcagca tcagcaacat cgagacggtg 660
atcgagtttc agcagaagaa caaccggctg ctggagatca cccgggagtt tagcgtgaac 720
gccggcgtga ccacacccgt gagcacctac atgctgacca acagcgagct gctgtctctg 780

atcaacgaca tgcccatcac caacgatcag aagaagctga tgagcaacaa cgtgcagatc 840
gtgcggcagc agagctacag catcatgtgc atcatcaagg aagaggtgct ggcctacgtg 900
gtgcagctgc ccctgtacgg cgtgatcgat accccatgct ggaagctgca caccagccca 960
ctgtgtacca ccaacaccaa agagggaagc aacatttgtc tgacacggac agataggggc 1020
tggtactgtg ataatgccgg cagcgtctcc ttctttcccc aggccgagac atgcaaggtg 1080
cagagcaacc gggtgttctg cgacaccatg aacagcctga cactgcccag cgaggtgaat 1140
ctgtgcaacg tggacatctt caaccccaag tacgactgca agatcatgac cagcaagaca 1200
gacgtgtcta gcagcgtcat taccagcctg ggcgctatcg tgagctgcta cggcaagacc 1260
aagtgcacag ccagcaacaa gaaccggggg atcatcaaga ccttctccaa cggctgcgac 1320
tacgtgagca ataagggcgt ggacaccgtg agcgtgggca ataccctgta ctacgtgaac 1380
aaacaggagg gcaagagcct gtacgtgaag ggcgagccca tcatcaactt ctacgatcct 1440
ctggtgttcc ccagcgacga gtttgacgcc agcatctccc aggtgaacga gaagatcaac 1500
cagagcctgg ccttcatcag gaagagcgac gagctcctgc acaacgtcaa cgccggcaaa 1560
agcaccacaa acatcatgat caccaccatc atcatcgtga ttatcgtgat cctgctgagc 1620
ctgatcgccg tgggcctgct gctgtattgc aaggccagga gcacccccgt gacactgagc 1680
aaggaccagc tgtctggcat caacaacatc gccttcagca ac 1722

SEQ ID NO: 42
length 1617
molecule type RNA
division PAT

Key Location/Qualifiers
source 1..1617
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcccc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtgaacgccg gcaagagcac caccaacatc atgatcacca ccatcatcat cgtgatcatt 1500
gtgatcctgc tggccctgat cgccgtgggc ctgctgctgt actgtaaggc caggtctacc 1560
cctgtgaccc tgagcaagga tcagctgagc ggcatcaata atatcgcctt ctccaac 1617

SEQ ID NO: 43
length 1614
molecule type RNA
division PAT

Key                 Location/Qualifiers
source              1..1614
                    /mol_type=other RNA
                    /organism=synthetic construct

Sequence
atggagctgc ccatcctgaa gaccaatgcc attaccacaa tcctggccgc cgtgacactg   60
tgtttcgcta gcagccagaa catcaccgag gagttttatc agagcacctg ctctgccgtg   120
tctaagggct acctgggcgc cctgaggacc ggctggtaca ccagcgtgat caccatcgag   180
ctgagcaata tcaaggagaa caagtgcaac ggcacagatg ccaaggtgaa gctgatcaag   240
caggagctgg acaagtacaa gaacgccgtg actgatctgc agctgctgat gcagagcaca   300
ccttgtggag gatctggagg ttctggcagc gctattgcca gcggcatcgc tgtgtgcaag   360
gtgctgcacc tggaaggcga agtgaacaag atcaagagtg ctctgctgag caccaacaag   420
gctgtggtga gcctgagcaa cggcgtgagc gtgctgacct tcaaggtcct ggacctgaag   480
aattacatcg acaaacagct gctccccatc gtgaacaagc agagctgcag catccccaac   540
atcgagacag tgatcgagtt tcagcagaag aacaaccggc tgctggagat caccagggag   600
tttagcgtga acgccggcgt gaccaccccc gtgagcacct acatgctgac caacagcgag   660
ctgctcagcc tgatcaacga catgcctatc accaacgatc agaagaagct gatgagcagc   720
aacgtgcaga tcgtgaggca gcagagctac agcatcatgt gtatcatcaa ggaggaggtg   780
ctggcctacg tggtgcagct gcctctgtac ggcgtgatcg acaccccatg ttggaagctg   840
cacacaagcc ccctgtgtac caccaacacc aaagagggct ctaacatctg tctgaccagg   900
acagataggg gatggtactg cgacaatgcc ggaagcgtga gctttttccc tcaggccgag   960
acatgcaagg tccagtgcaa cagggtgttc tgcgacacca tgaacagcct gaccctgcca   1020
agcgaggtga acctgtgcaa catcgacatc ttcaacccca agtacgactg caagatcatg   1080
accagcaaga ccgacgtgag ctctagcgtc atcaccagcc tgggcgctat cgtgtcttgc   1140
tacggcaaga ccaaatgcac cgccagcaac aagaaccggg gcatcatcaa gaccttcagc   1200
aacggctgcg actacgtgag caacaagggc gtggacaccg ttagcgtggg caatacactg   1260
tactacgtga ataagcagga aggcaagagc ctgtacgtga agggcgagcc catcatcaac   1320
ttctacgacc ccctggtgtt ccccagctgc gagttttgcg ccagcatcag ccaggtgaac   1380
gagaagatca atcagagcct ggccttcatc cggaagagcg acgagctgct gcacaacgtc   1440
aacgccggca agagcacaac caacatcatg atcaccacca ttatcatcgt catcatcgtg   1500
atcctgctgg ccctgatcgc cgtgggcctg ctgctgtact gcaaagccag aagcacccct   1560
gtgactctga gcaaggacca gctgagcggc atcaataaca tcgccttctc caac         1614

SEQ ID NO:          44
length              1596
molecule type       RNA
division            PAT

Key                 Location/Qualifiers
source              1..1596
                    /mol_type=other RNA
                    /organism=synthetic construct

Sequence
atggagctgc ccatcctgaa gaccaatgcc attaccacaa tcctggccgc cgtgacactg   60
tgtttcgcta gcagccagaa catcaccgag gagttttatc agagcacctg ctctgccgtg   120
tctaagggct acctgggcgc cctgaggacc ggctggtaca ccagcgtgat caccatcgag   180
ctgagcaata tcaaggagaa caagtgcaac ggcacagatg ccaaggtgaa gctgatcaag   240
caggagctgg acaagtacaa gaacgccgtg actgatctgc agctgctgat gcagagcaca   300
ccttgtggag gatctggagg ttctggcagc gctattgcca gcggcatcgc tgtgtgcaag   360
gtgctgcacc tggaaggcga agtgaacaag atcaagagtg ctctgctgag caccaacaag   420
gctgtggtga gcctgagcaa cggcgtgagc gtgctgacct tcaaggtcct ggacctgaag   480
aattacatcg acaaacagct gctccccatc gtgaacaagc agagctgcag catccccaac   540
atcgagacag tgatcgagtt tcagcagaag aacaaccggc tgctggagat caccagggag   600
tttagcgtga acgccggcgt gaccaccccc gtgagcacct acatgctgac caacagcgag   660
ctgctcagcc tgatcaacga catgcctatc accaacgatc agaagaagct gatgagcagc   720
aacgtgcaga tcgtgaggca gcagagctac agcatcatgt gtatcatcaa ggaggaggtg   780
ctggcctacg tggtgcagct gcctctgtac ggcgtgatcg acaccccatg ttggaagctg   840
cacacaagcc ccctgtgtac caccaacacc aaagagggct ctaacatctg tctgaccagg   900

acagataggg gatggtactg cgacaatgcc ggaagcgtga gctttttccc tcaggccgag 960
acatgcaagg tccagtgcaa cagggtgttc tgcgacacca tgaacagcct gaccctgcca 1020
agcgaggtga acctgtgcaa catcgacatc ttcaacccca agtacgactg caagatcatg 1080
accagcaaga ccgacgtgag ctctagcgtc atcaccagcc tgggcgctat cgtgtcttgc 1140
tacggcaaga ccaaatgcac cgccagcaac aagaaccggg gcatcatcaa gaccttcagc 1200
aacggctgcg actacgtgag caacaagggc gtggacaccg ttagcgtggg caatacactg 1260
tactacgtga ataagcagga aggcaagagc ctgtacgtga agggcgagcc catcatcaac 1320
ttctacgacc ccctggtgtt ccccagctgc gagttttgcg ccagcatcag ccaggtgaac 1380
gagaagatca atcagagcct ggccttcatc cggaagagcg acgagctgct gcacaacgtc 1440
aacgccggca agagcacaac caactggcct tggtacatct ggctgggctt catcgccggc 1500
ctgatcgcca tcgtgatggt gacaatcatg ctgtgctgta tgaccagctg ctgcagctgt 1560
ctgaagggct gctgctcctg cggatcttgt tgcaag 1596

SEQ ID NO: 45
length 1698
molecule type RNA
division PAT

Key Location/Qualifiers
source 1..1698
/mol_type=other RNA
/organism=synthetic construct

Sequence
atggagctgc ccatcctgaa gaccaatgcc attaccacaa tcctggccgc cgtgacactg 60
tgtttcgcta gcagccagaa catcaccgag gagttttatc agagcacctg ctctgccgtg 120
tctaagggct acctgggcgc cctgaggacc ggctggtaca ccagcgtgat caccatcgag 180
ctgagcaata tcaaggagaa caagtgcaac ggcacagatg ccaaggtgaa gctgatcaag 240
caggagctgg acaagtacaa gaacgccgtg actgatctgc agctgctgat gcagagcaca 300
ccttgtggag gatctggagg ttctggcagc gctattgcca gcggcatcgc tgtgtgcaag 360
gtgctgcacc tggaaggcga agtgaacaag atcaagagtg ctctgctgag caccaacaag 420
gctgtggtga gcctgagcaa cggcgtgagc gtgctgacct tcaaggtcct ggacctgaag 480
aattacatcg acaaacagct gctccccatc gtgaacaagc agagctgcag catccccaac 540
atcgagacag tgatcgagtt tcagcagaag aacaaccggc tgctggagat caccagggag 600
tttagcgtga acgccggcgt gaccaccccc gtgagcacct acatgctgac caacagcgag 660
ctgctcagcc tgatcaacga catgcctatc accaacgatc agaagaagct gatgagcagc 720
aacgtgcaga tcgtgaggca gcagagctac agcatcatgt gtatcatcaa ggaggaggtg 780
ctggcctacg tggtgcagct gcctctgtac ggcgtgatcg acaccccatg ttggaagctg 840
cacacaagcc ccctgtgtac caccaacacc aaagagggct ctaacatctg tctgaccagg 900
acagataggg gatggtactg cgacaatgcc ggaagcgtga gctttttccc tcaggccgag 960
acatgcaagg tccagtgcaa cagggtgttc tgcgacacca tgaacagcct gaccctgcca 1020
agcgaggtga acctgtgcaa catcgacatc ttcaacccca agtacgactg caagatcatg 1080
accagcaaga ccgacgtgag ctctagcgtc atcaccagcc tgggcgctat cgtgtcttgc 1140
tacggcaaga ccaaatgcac cgccagcaac aagaaccggg gcatcatcaa gaccttcagc 1200
aacggctgcg actacgtgag caacaagggc gtggacaccg ttagcgtggg caatacactg 1260
tactacgtga ataagcagga aggcaagagc ctgtacgtga agggcgagcc catcatcaac 1320
ttctacgacc ccctggtgtt ccccagctgc gagttttgcg ccagcatcag ccaggtgaac 1380
gagaagatca atcagagcct ggccttcatc cggaagagcg acgagctgct gcacaacgtc 1440
gaggacaaga tcgaggagat cctgagcaag atctaccaca tcgagaacga gatcgccagg 1500
atcaagaagc tgatcggcga ggccaacgcc ggcaagagca caaccaacat catgatcacc 1560
accattatca tcgtcatcat cgtgatcctg ctggccctga tcgccgtggg cctgctgctg 1620
tactgcaaag ccagaagcac ccctgtgact ctgagcaagg accagctgag cggcatcaat 1680
aacatcgcct tctccaac 1698

SEQ ID NO: 46
length 1680
molecule type RNA
division PAT

Key Location/Qualifiers

source                1..1680
                      /mol_type=other RNA
                      /organism=synthetic construct

Sequence
atggagctgc ccatcctgaa gaccaatgcc attaccacaa tcctggccgc cgtgacactg   60
tgtttcgcta gcagccagaa catcaccgag gagttttatc agagcacctg ctctgccgtg   120
tctaagggct acctgggcgc cctgaggacc ggctggtaca ccagcgtgat caccatcgag   180
ctgagcaata tcaaggagaa caagtgcaac ggcacagatg ccaaggtgaa gctgatcaag   240
caggagctgg acaagtacaa gaacgccgtg actgatctgc agctgctgat gcagagcaca   300
ccttgtggag gatctggagg ttctggcagc gctattgcca gcggcatcgc tgtgtgcaag   360
gtgctgcacc tggaaggcga agtgaacaag atcaagagtg ctctgctgag caccaacaag   420
gctgtggtga gcctgagcaa cggcgtgagc gtgctgacct tcaaggtcct ggacctgaag   480
aattacatcg acaaacagct gctccccatc gtgaacaagc agagctgcag catccccaac   540
atcgagacag tgatcgagtt tcagcagaag aacaaccggc tgctggagat caccagggag   600
tttagcgtga acgccggcgt gaccaccccc gtgagcacct acatgctgac caacagcgag   660
ctgctcagcc tgatcaacga catgcctatc accaacgatc agaagaagct gatgagcagc   720
aacgtgcaga tcgtgaggca gcagagctac agcatcatgt gtatcatcaa ggaggaggtg   780
ctggcctacg tggtgcagct gcctctgtac ggcgtgatcg acaccccatg ttggaagctg   840
cacacaagcc ccctgtgtac caccaacacc aaagagggct ctaacatctg tctgaccagg   900
acagataggg gatggtactg cgacaatgcc ggaagcgtga gctttttccc tcaggccgag   960
acatgcaagg tccagtgcaa cagggtgttc tgcgacacca tgaacagcct gaccctgcca   1020
agcgaggtga acctgtgcaa catcgacatc ttcaacccca agtacgactg caagatcatg   1080
accagcaaga ccgacgtgag ctctagcgtc atcaccagcc tgggcgctat cgtgtcttgc   1140
tacggcaaga ccaaatgcac cgccagcaac aagaaccggg gcatcatcaa gaccttcagc   1200
aacggctgcg actacgtgag caacaagggc gtggacaccg ttagcgtggg caatacactg   1260
tactacgtga ataagcagga aggcaagagc ctgtacgtga agggcgagcc catcatcaac   1320
ttctacgacc ccctggtgtt ccccagctgc gagttttgcg ccagcatcag ccaggtgaac   1380
gagaagatca atcagagcct ggccttcatc cggaagagcg acgagctgct gcacaacgtc   1440
gaggacaaga tcgaggagat cctgagcaag atctaccaca tcgagaacga gatcgccagg   1500
atcaagaagc tgatcggcga ggccaacgcc ggcaagagca caaccaactg gccttggtac   1560
atctggctgg gcttcatcgc cggcctgatc gccatcgtga tggtgacaat catgctgtgc   1620
tgcatgacca gctgttgtag ctgcctgaag ggctgctgct cctgtggatc ttgctgcaag   1680

SEQ ID NO:            47
length                1614
molecule type         RNA
division              PAT

Key                   Location/Qualifiers
source                1..1614
                      /mol_type=other RNA
                      /organism=synthetic construct

Sequence
atggagctgc ccatcctgaa gaccaatgcc attaccacca ttctggccgc cgtgaccctg   60
tgttttgcct ctagccagaa catcaccgag gaattttacc agagcacctg cagcgccgtg   120
agcaagggct acctgggcgc cctgagaacc ggctggtata cctccgtgat caccatcgag   180
ctgagcaata tcaaggagaa caagtgcaac ggcaccgatg ccaaggtgaa gctgattaag   240
caggagctgg acaagtacaa gaacgctgtg accgatctgc agctgctgat gcagagcaca   300
ccttgcggag gaagcggatc tggatctgcc attgccagcg gcatcgccgt gtgtaaggtg   360
ctgcacctgg aaggcgaggt gaacaagatt aagtcagccc tgctgtctac caacaaggcc   420
gtggtgtctc tgagcaacgg cgtgagcgtc ctgaccttca aggtgctgga cctgaagaat   480
tacatcgaca agcagctgct ccccatcgtc aacaagcaga gctgcagcat ccccaacatc   540
gagacagtga tcgagtttca gcagaagaac aaccggctgc tggagatcac cagggagttt   600
agcgtgaacg ccggcgtgac cacccccgtg agcacctaca tgctgaccaa cagcgagctg   660
ctcagcctga tcaacgacat gcctatcacc aacgaccaga agaagctgat gagctctaac   720
gtgcagatcg tgaggcagca gagctacagc atcatgtgta tcatcaagga ggaggtgctg   780
gcctacgtgg tgcagctgcc tctgtacggc gtgattgaca ccccctgctg gaagctgcat   840
accagcccac tgtgcaccac caataccaaa gagggctcta atatctgcct gacaaggacc   900

gacagaggct ggtactgtga taacgccggc agcgtgagct tctttcccca ggccgaaacc 960
tgtaaggtcc agtgcaacag ggtgttctgc gacaccatga acagcctgac actgccaagc 1020
gaggtcaacc tgtgcaacat cgacatcttc aaccccaagt acgactgcaa gatcatgacc 1080
agcaagaccg acgtgagcag cagcgtgatc acatctctgg gcgccatcgt gagctgctac 1140
ggcaagacca agtgcaccgc cagcaacaag aacaggggca tcatcaagac cttcagcaac 1200
ggctgcgact acgtgagcaa caagggcgtg gataccgtga gcgtgggaaa taccctgtat 1260
tacgtgaaca agcaggaggg caagagcctg tacgtgaagg gagagcccat catcaacttc 1320
tacgaccccc tggtgttccc cagctgcgag ttttgcgcca gcatcagcca ggtgaacgag 1380
aagatcaacc agagcctggc tttcatccgg aagagcgacg agctgctgca caacgtcaac 1440
gccggaaaga gcacaaccaa catcatgatc accaccatca tcatcgtgat cattgtgatc 1500
ctgctggccc tgatcgccgt gggcctgctg ctgtactgta aggccaggag cacccctgtg 1560
accctgagca aggatcagct gagcggcatt aacaatatcg ccttctccaa ctga 1614

SEQ ID NO: 48
length 1596
molecule type RNA
division PAT

Key Location/Qualifiers
source 1..1596
/mol_type=other RNA
/organism=synthetic construct

Sequence
atggagctgc ccatcctgaa gaccaatgcc attaccacca ttctggccgc cgtgaccctg 60
tgttttgcct ctagccagaa catcaccgag gaattttacc agagcacctg cagcgccgtg 120
agcaagggct acctgggcgc cctgagaacc ggctggtata cctccgtgat caccatcgag 180
ctgagcaata tcaaggagaa caagtgcaac ggcaccgatg ccaaggtgaa gctgattaag 240
caggagctgg acaagtacaa gaacgctgtg accgatctgc agctgctgat gcagagcaca 300
ccttgcggag gaagcggatc tggatctgcc attgccagcg gcatcgccgt gtgtaaggtg 360
ctgcacctgg aaggcgaggt gaacaagatt aagtcagccc tgctgtctac caacaaggcc 420
gtggtgtctc tgagcaacgg cgtgagcgtc ctgaccttca aggtgctgga cctgaagaat 480
tacatcgaca agcagctgct ccccatcgtc aacaagcaga gctgcagcat ccccaacatc 540
gagacagtga tcgagtttca gcagaagaac aaccggctgc tggagatcac cagggagttt 600
agcgtgaacg ccggcgtgac cacccccgtg agcacctaca tgctgaccaa cagcgagctg 660
ctcagcctga tcaacgacat gcctatcacc aacgaccaga agaagctgat gagctctaac 720
gtgcagatcg tgaggcagca gagctacagc atcatgtgta tcatcaagga ggaggtgctg 780
gcctacgtgg tgcagctgcc tctgtacggc gtgattgaca ccccctgctg gaagctgcat 840
accagcccac tgtgcaccac caataccaaa gagggctcta atatctgcct gacaaggacc 900
gacagaggct ggtactgtga taacgccggc agcgtgagct tctttcccca ggccgaaacc 960
tgtaaggtcc agtgcaacag ggtgttctgc gacaccatga acagcctgac actgccaagc 1020
gaggtcaacc tgtgcaacat cgacatcttc aaccccaagt acgactgcaa gatcatgacc 1080
agcaagaccg acgtgagcag cagcgtgatc acatctctgg gcgccatcgt gagctgctac 1140
ggcaagacca agtgcaccgc cagcaacaag aacaggggca tcatcaagac cttcagcaac 1200
ggctgcgact acgtgagcaa caagggcgtg gataccgtga gcgtgggaaa taccctgtat 1260
tacgtgaaca agcaggaggg caagagcctg tacgtgaagg gagagcccat catcaacttc 1320
tacgaccccc tggtgttccc cagctgcgag ttttgcgcca gcatcagcca ggtgaacgag 1380
aagatcaacc agagcctggc tttcatccgg aagagcgacg agctgctgca caacgtcaac 1440
gccggaaaga gcacaaccaa ctggccttgg tacatctggc tgggcttcat cgccggcctg 1500
atcgccatcg tgatggtgac aatcatgctg tgctgtatga ccagctgctg cagctgtctg 1560
aagggctgct gctcctgcgg atcttgttgc aagtga 1596

SEQ ID NO: 49
length 1698
molecule type RNA
division PAT

Key Location/Qualifiers
source 1..1698
/mol_type=other RNA

/organism=synthetic construct

Sequence
atggagctgc ccatcctgaa gaccaatgcc attaccacca ttctggccgc cgtgaccctg 60
tgttttgcct ctagccagaa catcaccgag gaattttacc agagcacctg cagcgccgtg 120
agcaagggct acctgggcgc cctgagaacc ggctggtata cctccgtgat caccatcgag 180
ctgagcaata tcaaggagaa caagtgcaac ggcaccgatg ccaaggtgaa gctgattaag 240
caggagctgg acaagtacaa gaacgctgtg accgatctgc agctgctgat gcagagcaca 300
ccttgcggag gaagcggatc tggatctgcc attgccagcg gcatcgccgt gtgtaaggtg 360
ctgcacctgg aaggcgaggt gaacaagatt aagtcagccc tgctgtctac caacaaggcc 420
gtggtgtctc tgagcaacgg cgtgagcgtc ctgaccttca aggtgctgga cctgaagaat 480
tacatcgaca agcagctgct ccccatcgtc aacaagcaga gctgcagcat ccccaacatc 540
gagacagtga tcgagtttca gcagaagaac aaccggctgc tggagatcac cagggagttt 600
agcgtgaacg ccggcgtgac cacccccgtg agcacctaca tgctgaccaa cagcgagctg 660
ctcagcctga tcaacgacat gcctatcacc aacgaccaga agaagctgat gagctctaac 720
gtgcagatcg tgaggcagca gagctacagc atcatgtgta tcatcaagga ggaggtgctg 780
gcctacgtgg tgcagctgcc tctgtacggc gtgattgaca ccccctgctg gaagctgcat 840
accagcccac tgtgcaccac caataccaaa gagggctcta atatctgcct gacaaggacc 900
gacagaggct ggtactgtga taacgccggc agcgtgagct tctttcccca ggccgaaacc 960
tgtaaggtcc agtgcaacag ggtgttctgc gacaccatga acagcctgac actgccaagc 1020
gaggtcaacc tgtgcaacat cgacatcttc aaccccaagt acgactgcaa gatcatgacc 1080
agcaagaccg acgtgagcag cagcgtgatc acatctctgg gcgccatcgt gagctgctac 1140
ggcaagacca agtgcaccgc cagcaacaag aacaggggca tcatcaagac cttcagcaac 1200
ggctgcgact acgtgagcaa caagggcgtg gataccgtga gcgtgggaaa taccctgtat 1260
tacgtgaaca agcaggaggg caagagcctg tacgtgaagg gagagcccat catcaacttc 1320
tacgaccccc tggtgttccc cagctgcgag ttttgcgcca gcatcagcca ggtgaacgag 1380
aagatcaacc agagcctggc tttcatccgg aagagcgacg agctgctgca caacgtcgag 1440
gacaagatcg aggagatcct gagcaagatc taccacatcg agaacgagat cgccaggatc 1500
aagaagctga tcggcgaggc caacgccgga aagagcacaa ccaacatcat gatcaccacc 1560
atcatcatcg tgatcattgt gatcctgctg gccctgatcg ccgtgggcct gctgctgtac 1620
tgtaaggcca ggagcacccc tgtgaccctg agcaaggatc agctgagcgg cattaacaat 1680
atcgccttct ccaactga 1698

SEQ ID NO: 50
length 1680
molecule type RNA
division PAT

Key Location/Qualifiers
source 1..1680
/mol_type=other RNA
/organism=synthetic construct

Sequence
atggagctgc ccatcctgaa gaccaatgcc attaccacca ttctggccgc cgtgaccctg 60
tgttttgcct ctagccagaa catcaccgag gaattttacc agagcacctg cagcgccgtg 120
agcaagggct acctgggcgc cctgagaacc ggctggtata cctccgtgat caccatcgag 180
ctgagcaata tcaaggagaa caagtgcaac ggcaccgatg ccaaggtgaa gctgattaag 240
caggagctgg acaagtacaa gaacgctgtg accgatctgc agctgctgat gcagagcaca 300
ccttgcggag gaagcggatc tggatctgcc attgccagcg gcatcgccgt gtgtaaggtg 360
ctgcacctgg aaggcgaggt gaacaagatt aagtcagccc tgctgtctac caacaaggcc 420
gtggtgtctc tgagcaacgg cgtgagcgtc ctgaccttca aggtgctgga cctgaagaat 480
tacatcgaca agcagctgct ccccatcgtc aacaagcaga gctgcagcat ccccaacatc 540
gagacagtga tcgagtttca gcagaagaac aaccggctgc tggagatcac cagggagttt 600
agcgtgaacg ccggcgtgac cacccccgtg agcacctaca tgctgaccaa cagcgagctg 660
ctcagcctga tcaacgacat gcctatcacc aacgaccaga agaagctgat gagctctaac 720
gtgcagatcg tgaggcagca gagctacagc atcatgtgta tcatcaagga ggaggtgctg 780
gcctacgtgg tgcagctgcc tctgtacggc gtgattgaca ccccctgctg gaagctgcat 840
accagcccac tgtgcaccac caataccaaa gagggctcta atatctgcct gacaaggacc 900
gacagaggct ggtactgtga taacgccggc agcgtgagct tctttcccca ggccgaaacc 960

tgtaaggtcc agtgcaacag ggtgttctgc gacaccatga acagcctgac actgccaagc 1020
gaggtcaacc tgtgcaacat cgacatcttc aaccccaagt acgactgcaa gatcatgacc 1080
agcaagaccg acgtgagcag cagcgtgatc acatctctgg gcgccatcgt gagctgctac 1140
ggcaagacca agtgcaccgc cagcaacaag aacaggggca tcatcaagac cttcagcaac 1200
ggctgcgact acgtgagcaa caagggcgtg gataccgtga gcgtgggaaa taccctgtat 1260
tacgtgaaca agcaggaggg caagagcctg tacgtgaagg gagagcccat catcaacttc 1320
tacgaccccc tggtgttccc cagctgcgag ttttgcgcca gcatcagcca ggtgaacgag 1380
aagatcaacc agagcctggc tttcatccgg aagagcgacg agctgctgca caacgtcgag 1440
gacaagatcg aggagatcct gagcaagatc taccacatcg agaacgagat cgccaggatc 1500
aagaagctga tcggcgaggc caacgccgga aagagcacaa ccaactggcc ttggtacatc 1560
tggctgggct tcatcgccgg cctgatcgcc atcgtgatgg tgacaatcat gctgtgctgc 1620
atgaccagct gttgtagctg cctgaagggc tgctgctcct gtggatcttg ctgcaagtga 1680

SEQ ID NO: 51
length 1611
molecule type RNA
division PAT

Key Location/Qualifiers
source 1..1611
/mol_type=other RNA
/organism=synthetic construct

Sequence
atggagctgc ctatcctgaa gaccaacgcc attaccacca ttctggccgc tgtgacactg 60
tgctttgcct ctagccagaa catcacagag gagttttatc agagcacctg cagcgccgtg 120
agcaagggct acctgggcgc cctgagaacc ggctggtaca ccagcgtgat caccatcgag 180
ctgtccaaca tcaaggaaaa caagtgcaac ggcaccgacg ccaaggtgaa gctgatcaag 240
caggagctgg ataaatataa gaacgccgtg accgatctgc agctgctgat gcagagtaca 300
ccttgtggct ctggcagtgg cagcgccatc gcctctggca tcgctgtgtg taaggtgctc 360
cacctggagg gcgaagtgaa taagatcaaa agcgccctgc tgtctaccaa caaggccgtg 420
gtgagcctgt ctaacggcgt gagcgtgctg accttcaagg tgctggacct gaaaaactac 480
atcgacaaac agctgctgcc catcgtgaac aagcagagct gcagcatccc caacatcgag 540
acagtgatcg agtttcagca gaagaacaac cggctgctgg agatcacccg ggagtttagc 600
gtgaacgccg gcgtgaccac ccctgtgagc acctacatgc tgaccaacag cgagctgctg 660
agcctgatca acgacatgcc tatcaccaac gatcagaaga agctgatgag cagcaacgtg 720
cagatcgtgc ggcagcagag ctacagcatt atgtgcatca ttaaggagga ggtgctggcc 780
tacgtggtgc agctgcccct gtacggcgtg atcgacacac cttgctggaa gctgcacaca 840
agcccactgt gcaccacaaa cacaaaagag ggcagcaaca tctgtctgac acggacagac 900
cggggctggt attgtgataa tgccggcagc gtgagcttct ttccccaggc tgaaacctgc 960
aaggtgcagt gcaatcgggt gttctgcgac accatgaata gcctgaccct gcccagcgag 1020
gtgaacctgt gcaacatcga catcttcaac cccaagtacg actgcaagat catgaccagc 1080
aagaccgacg tgagcagctc tgtgattacc agcctgggcg cgatcgtgtc ttgctacggc 1140
aagaccaagt gcaccgccag caacaagaac aggggcatca tcaagacatt cagcaacggc 1200
tgcgactacg tgagcaataa gggcgtggac accgtgagcg tcggcaacac actgtactac 1260
gtgaacaaac aggagggcaa aagcctgtac gtgaagggcg agcccatcat caacttctac 1320
gaccccctgg tgttccccag ctgcgagttt tgcgccagca tcagccaggt gaacgagaag 1380
atcaaccaga gcctggcctt tatcaggaag agcgacgagc tcctgcacaa cgtcaacgcc 1440
ggcaagagca ccaccaacat catgatcacc accatcatca ttgtgatcat cgtgatcctg 1500
ctggccctga ttgccgtggg cctgctgctg tactgcaagg ccagaagcac acccgtgaca 1560
ctgagcaagg atcagctgag cggcatcaac aacatcgcct tcagcaactg a 1611

SEQ ID NO: 52
length 1593
molecule type RNA
division PAT

Key Location/Qualifiers
source 1..1593
/mol_type=other RNA

/organism=synthetic construct

Sequence
atggagctgc ctatcctgaa gaccaacgcc attaccacca ttctggccgc tgtgacactg 60
tgctttgcct ctagccagaa catcacagag gagttttatc agagcacctg cagcgccgtg 120
agcaagggct acctgggcgc cctgagaacc ggctggtaca ccagcgtgat caccatcgag 180
ctgtccaaca tcaaggaaaa caagtgcaac ggcaccgacg ccaaggtgaa gctgatcaag 240
caggagctgg ataaatataa gaacgccgtg accgatctgc agctgctgat gcagagtaca 300
ccttgtggct ctggcagtgg cagcgccatc gcctctggca tcgctgtgtg taaggtgctc 360
cacctggagg gcgaagtgaa taagatcaaa agcgccctgc tgtctaccaa caaggccgtg 420
gtgagcctgt ctaacggcgt gagcgtgctg accttcaagg tgctggacct gaaaaactac 480
atcgacaaac agctgctgcc catcgtgaac aagcagagct gcagcatccc caacatcgag 540
acagtgatcg agtttcagca gaagaacaac cggctgctgg agatcacccg ggagtttagc 600
gtgaacgccg gcgtgaccac ccctgtgagc acctacatgc tgaccaacag cgagctgctg 660
agcctgatca acgacatgcc tatcaccaac gatcagaaga agctgatgag cagcaacgtg 720
cagatcgtgc ggcagcagag ctacagcatt atgtgcatca ttaaggagga ggtgctggcc 780
tacgtggtgc agctgcccct gtacggcgtg atcgacacac cttgctggaa gctgcacaca 840
agcccactgt gcaccacaaa cacaaaagag ggcagcaaca tctgtctgac acggacagac 900
cggggctggt attgtgataa tgccggcagc gtgagcttct ttccccaggc tgaaacctgc 960
aaggtgcagt gcaatcgggt gttctgcgac accatgaata gcctgaccct gcccagcgag 1020
gtgaacctgt gcaacatcga catcttcaac cccaagtacg actgcaagat catgaccagc 1080
aagaccgacg tgagcagctc tgtgattacc agcctgggcg cgatcgtgtc ttgctacggc 1140
aagaccaagt gcaccgccag caacaagaac aggggcatca tcaagacatt cagcaacggc 1200
tgcgactacg tgagcaataa gggcgtggac accgtgagcg tcggcaacac actgtactac 1260
gtgaacaaac aggagggcaa aagcctgtac gtgaagggcg agcccatcat caacttctac 1320
gaccccctgg tgttccccag ctgcgagttt tgcgccagca tcagccaggt gaacgagaag 1380
atcaaccaga gcctggcctt tatcaggaag agcgacgagc tcctgcacaa cgtcaacgcc 1440
ggcaagagca ccaccaactg gccttggtac atctggctgg gcttcatcgc cggcctgatc 1500
gccatcgtga tggtgacaat catgctgtgc tgtatgacca gctgctgcag ctgtctgaag 1560
ggctgctgct cctgcggatc ttgttgcaag tga 1593

SEQ ID NO: 53
length 1695
molecule type RNA
division PAT

Key Location/Qualifiers
source 1..1695
/mol_type=other RNA
/organism=synthetic construct

Sequence
atggagctgc ctatcctgaa gaccaacgcc attaccacca ttctggccgc tgtgacactg 60
tgctttgcct ctagccagaa catcacagag gagttttatc agagcacctg cagcgccgtg 120
agcaagggct acctgggcgc cctgagaacc ggctggtaca ccagcgtgat caccatcgag 180
ctgtccaaca tcaaggaaaa caagtgcaac ggcaccgacg ccaaggtgaa gctgatcaag 240
caggagctgg ataaatataa gaacgccgtg accgatctgc agctgctgat gcagagtaca 300
ccttgtggct ctggcagtgg cagcgccatc gcctctggca tcgctgtgtg taaggtgctc 360
cacctggagg gcgaagtgaa taagatcaaa agcgccctgc tgtctaccaa caaggccgtg 420
gtgagcctgt ctaacggcgt gagcgtgctg accttcaagg tgctggacct gaaaaactac 480
atcgacaaac agctgctgcc catcgtgaac aagcagagct gcagcatccc caacatcgag 540
acagtgatcg agtttcagca gaagaacaac cggctgctgg agatcacccg ggagtttagc 600
gtgaacgccg gcgtgaccac ccctgtgagc acctacatgc tgaccaacag cgagctgctg 660
agcctgatca acgacatgcc tatcaccaac gatcagaaga agctgatgag cagcaacgtg 720
cagatcgtgc ggcagcagag ctacagcatt atgtgcatca ttaaggagga ggtgctggcc 780
tacgtggtgc agctgcccct gtacggcgtg atcgacacac cttgctggaa gctgcacaca 840
agcccactgt gcaccacaaa cacaaaagag ggcagcaaca tctgtctgac acggacagac 900
cggggctggt attgtgataa tgccggcagc gtgagcttct ttccccaggc tgaaacctgc 960
aaggtgcagt gcaatcgggt gttctgcgac accatgaata gcctgaccct gcccagcgag 1020
gtgaacctgt gcaacatcga catcttcaac cccaagtacg actgcaagat catgaccagc 1080

aagaccgacg tgagcagctc tgtgattacc agcctgggcg cgatcgtgtc ttgctacggc 1140
aagaccaagt gcaccgccag caacaagaac aggggcatca tcaagacatt cagcaacggc 1200
tgcgactacg tgagcaataa gggcgtggac accgtgagcg tcggcaacac actgtactac 1260
gtgaacaaac aggagggcaa aagcctgtac gtgaagggcg agcccatcat caacttctac 1320
gaccccctgg tgttccccag ctgcgagttt tgcgccagca tcagccaggt gaacgagaag 1380
atcaaccaga gcctggcctt tatcaggaag agcgacgagc tcctgcacaa cgtcgaggac 1440
aagatcgagg agatcctgag caagatctac cacatcgaga acgagatcgc caggatcaag 1500
aagctgatcg gcgaggccaa cgccggcaag agcaccacca acatcatgat caccaccatc 1560
atcattgtga tcatcgtgat cctgctggcc ctgattgccg tgggcctgct gctgtactgc 1620
aaggccagaa gcacacccgt gacactgagc aaggatcagc tgagcggcat caacaacatc 1680
gccttcagca actga 1695

SEQ ID NO: 54
length 1677
molecule type RNA
division PAT

Key Location/Qualifiers
source 1..1677
/mol_type=other RNA
/organism=synthetic construct

Sequence
atggagctgc ctatcctgaa gaccaacgcc attaccacca ttctggccgc tgtgacactg 60
tgctttgcct ctagccagaa catcacagag gagttttatc agagcacctg cagcgccgtg 120
agcaagggct acctgggcgc cctgagaacc ggctggtaca ccagcgtgat caccatcgag 180
ctgtccaaca tcaaggaaaa caagtgcaac ggcaccgacg ccaaggtgaa gctgatcaag 240
caggagctgg ataaatataa gaacgccgtg accgatctgc agctgctgat gcagagtaca 300
ccttgtggct ctggcagtgg cagcgccatc gcctctggca tcgctgtgtg taaggtgctc 360
cacctggagg gcgaagtgaa taagatcaaa agcgccctgc tgtctaccaa caaggccgtg 420
gtgagcctgt ctaacggcgt gagcgtgctg accttcaagg tgctggacct gaaaaactac 480
atcgacaaac agctgctgcc catcgtgaac aagcagagct gcagcatccc caacatcgag 540
acagtgatcg agtttcagca gaagaacaac cggctgctgg agatcacccg ggagtttagc 600
gtgaacgccg gcgtgaccac ccctgtgagc acctacatgc tgaccaacag cgagctgctg 660
agcctgatca acgacatgcc tatcaccaac gatcagaaga agctgatgag cagcaacgtg 720
cagatcgtgc ggcagcagag ctacagcatt atgtgcatca ttaaggagga ggtgctggcc 780
tacgtggtgc agctgcccct gtacggcgtg atcgacacac cttgctggaa gctgcacaca 840
agcccactgt gcaccacaaa cacaaaagag ggcagcaaca tctgtctgac acggacagac 900
cggggctggt attgtgataa tgccggcagc gtgagcttct ttccccaggc tgaaacctgc 960
aaggtgcagt gcaatcgggt gttctgcgac accatgaata gcctgaccct gcccagcgag 1020
gtgaacctgt gcaacatcga catcttcaac cccaagtacg actgcaagat catgaccagc 1080
aagaccgacg tgagcagctc tgtgattacc agcctgggcg cgatcgtgtc ttgctacggc 1140
aagaccaagt gcaccgccag caacaagaac aggggcatca tcaagacatt cagcaacggc 1200
tgcgactacg tgagcaataa gggcgtggac accgtgagcg tcggcaacac actgtactac 1260
gtgaacaaac aggagggcaa aagcctgtac gtgaagggcg agcccatcat caacttctac 1320
gaccccctgg tgttccccag ctgcgagttt tgcgccagca tcagccaggt gaacgagaag 1380
atcaaccaga gcctggcctt tatcaggaag agcgacgagc tcctgcacaa cgtcgaggac 1440
aagatcgagg agatcctgag caagatctac cacatcgaga acgagatcgc caggatcaag 1500
aagctgatcg gcgaggccaa cgccggcaag agcaccacca actggccttg gtacatctgg 1560
ctgggcttca tcgccggcct gatcgccatc gtgatggtga caatcatgct gtgctgcatg 1620
accagctgtt gtagctgcct gaagggctgc tgctcctgtg gatcttgctg caagtga 1677

SEQ ID NO: 55
length 1608
molecule type RNA
division PAT

Key Location/Qualifiers
source 1..1608
/mol_type=other RNA

/organism=synthetic construct

Sequence
atggagctgc ccatcctgaa gaccaacgcc atcacaacaa tcctggccgc cgtgaccctg 60
tgcttcgcct ctagccagaa cattaccgag gagttttatc agagcacatg cagcgccgtg 120
agcaagggct acctgggcgc cctgaggacc ggctggtaca caagcgtgat tacaatcgag 180
ctgagcaaca ttaaggagaa caagtgcaac ggcaccgacg ccaaggtgaa gctgatcaag 240
caggagctgg acaagtacaa gaatgccgtg accgatctgc agctgctgat gcagagcaca 300
ccttgtggag gatctggcag cgccatcgct tctggcatcg ctgtgtgtaa ggtgctgcac 360
ctggagggag aggtgaataa gatcaagagc gccctgctga gcacaaacaa agccgtggtg 420
agcctgagca acggagtgag cgtgctgacc ttcaaggtgc tggacctgaa aaactacatc 480
gacaagcagc tgctccccat cgtgaacaag cagagctgca gcatccccaa catcgaaacc 540
gtgatcgagt ttcagcagaa gaacaacagg ctgctggaga tcacccggga gtttagcgtc 600
aacgccggcg tgaccacccc tgtgtccacc tacatgctga ccaacagcga gctgctgagc 660
ctgatcaacg acatgcccat caccaacgat cagaagaagc tgatgtctag caacgtgcag 720
atcgtgcggc agcagagcta cagcatcatg tgcatcatca aggaggaggt gctggcctat 780
gtggtgcagc tgcctctgta cggcgtgatc gacaccccct gctggaagtt gcatacaagc 840
cccctgtgca ccaccaatac caaggaaggc agcaatatct gcctgacaag aaccgataga 900
ggctggtatt gcgacaatgc cggcagcgtg agcttcttcc ctcaggctga aacatgcaag 960
gtgcagtgca accgggtgtt ctgcgacacc atgaacagcc tgaccctgcc cagcgaggtg 1020
aacctgtgca acatcgacat cttcaatccc aagtacgact gcaagatcat gaccagcaag 1080
accgacgtga gcagctctgt gatcaccagc ctgggagcca tcgtgagctg ctacggcaag 1140
accaagtgca ccgccagcaa taagaaccgg gggatcatta agaccttcag caacggctgc 1200
gactatgtga gcaacaaggg cgtggacacc gtgagcgtgg gcaacacact gtactacgtg 1260
aacaaacagg agggaaagag cctgtacgtg aagggcgagc ccatcatcaa cttctacgac 1320
cccctggtgt tccccagctg cgagttttgc gccagcatca gccaggtgaa cgagaagatc 1380
aatcagagcc tggccttcat caggaagagc gatgagctgc tgcacaacgt gaacgccggc 1440
aagagcacga ccaacatcat gatcaccacc atcattatcg tgatcatcgt gatcctgctg 1500
gccctgattg ccgtgggcct gctgctgtac tgcaaggcca ggtctacccc tgtgaccctg 1560
agcaaggacc agctgtctgg catcaacaac atcgccttct ctaattga 1608

SEQ ID NO: 56
length 1590
molecule type RNA
division PAT

Key Location/Qualifiers
source 1..1590
/mol_type=other RNA
/organism=synthetic construct

Sequence
atggagctgc ccatcctgaa gaccaacgcc atcacaacaa tcctggccgc cgtgaccctg 60
tgcttcgcct ctagccagaa cattaccgag gagttttatc agagcacatg cagcgccgtg 120
agcaagggct acctgggcgc cctgaggacc ggctggtaca caagcgtgat tacaatcgag 180
ctgagcaaca ttaaggagaa caagtgcaac ggcaccgacg ccaaggtgaa gctgatcaag 240
caggagctgg acaagtacaa gaatgccgtg accgatctgc agctgctgat gcagagcaca 300
ccttgtggag gatctggcag cgccatcgct tctggcatcg ctgtgtgtaa ggtgctgcac 360
ctggagggag aggtgaataa gatcaagagc gccctgctga gcacaaacaa agccgtggtg 420
agcctgagca acggagtgag cgtgctgacc ttcaaggtgc tggacctgaa aaactacatc 480
gacaagcagc tgctccccat cgtgaacaag cagagctgca gcatccccaa catcgaaacc 540
gtgatcgagt ttcagcagaa gaacaacagg ctgctggaga tcacccggga gtttagcgtc 600
aacgccggcg tgaccacccc tgtgtccacc tacatgctga ccaacagcga gctgctgagc 660
ctgatcaacg acatgcccat caccaacgat cagaagaagc tgatgtctag caacgtgcag 720
atcgtgcggc agcagagcta cagcatcatg tgcatcatca aggaggaggt gctggcctat 780
gtggtgcagc tgcctctgta cggcgtgatc gacaccccct gctggaagtt gcatacaagc 840
cccctgtgca ccaccaatac caaggaaggc agcaatatct gcctgacaag aaccgataga 900
ggctggtatt gcgacaatgc cggcagcgtg agcttcttcc ctcaggctga aacatgcaag 960
gtgcagtgca accgggtgtt ctgcgacacc atgaacagcc tgaccctgcc cagcgaggtg 1020
aacctgtgca acatcgacat cttcaatccc aagtacgact gcaagatcat gaccagcaag 1080

accgacgtga gcagctctgt gatcaccagc ctgggagcca tcgtgagctg ctacggcaag 1140
accaagtgca ccgccagcaa taagaaccgg gggatcatta agaccttcag caacggctgc 1200
gactatgtga gcaacaaggg cgtggacacc gtgagcgtgg gcaacacact gtactacgtg 1260
aacaaacagg agggaaagag cctgtacgtg aagggcgagc ccatcatcaa cttctacgac 1320
cccctggtgt tccccagctg cgagttttgc gccagcatca gccaggtgaa cgagaagatc 1380
aatcagagcc tggccttcat caggaagagc gatgagctgc tgcacaacgt gaacgccggc 1440
aagagcacga ccaactggcc ttggtacatc tggctgggct tcatcgccgg cctgatcgcc 1500
atcgtgatgg tgacaatcat gctgtgctgt atgaccagct gctgcagctg tctgaagggc 1560
tgctgctcct gcggatcttg ttgcaagtga 1590

SEQ ID NO: 57
length 1692
molecule type RNA
division PAT

Key Location/Qualifiers
source 1..1692
/mol_type=other RNA
/organism=synthetic construct

Sequence
atggagctgc ccatcctgaa gaccaacgcc atcacaacaa tcctggccgc cgtgaccctg 60
tgcttcgcct ctagccagaa cattaccgag gagttttatc agagcacatg cagcgccgtg 120
agcaagggct acctgggcgc cctgaggacc ggctggtaca caagcgtgat tacaatcgag 180
ctgagcaaca ttaaggagaa caagtgcaac ggcaccgacg ccaaggtgaa gctgatcaag 240
caggagctgg acaagtacaa gaatgccgtg accgatctgc agctgctgat gcagagcaca 300
ccttgtggag gatctggcag cgccatcgct tctggcatcg ctgtgtgtaa ggtgctgcac 360
ctggagggag aggtgaataa gatcaagagc gccctgctga gcacaaacaa agccgtggtg 420
agcctgagca acggagtgag cgtgctgacc ttcaaggtgc tggacctgaa aaactacatc 480
gacaagcagc tgctccccat cgtgaacaag cagagctgca gcatccccaa catcgaaacc 540
gtgatcgagt ttcagcagaa gaacaacagg ctgctggaga tcacccggga gtttagcgtc 600
aacgccggcg tgaccacccc tgtgtccacc tacatgctga ccaacagcga gctgctgagc 660
ctgatcaacg acatgcccat caccaacgat cagaagaagc tgatgtctag caacgtgcag 720
atcgtgcggc agcagagcta cagcatcatg tgcatcatca aggaggaggt gctggcctat 780
gtggtgcagc tgcctctgta cggcgtgatc gacaccccct gctggaagtt gcatacaagc 840
cccctgtgca ccaccaatac caaggaaggc agcaatatct gcctgacaag aaccgataga 900
ggctggtatt gcgacaatgc cggcagcgtg agcttcttcc ctcaggctga aacatgcaag 960
gtgcagtgca accgggtgtt ctgcgacacc atgaacagcc tgaccctgcc cagcgaggtg 1020
aacctgtgca acatcgacat cttcaatccc aagtacgact gcaagatcat gaccagcaag 1080
accgacgtga gcagctctgt gatcaccagc ctgggagcca tcgtgagctg ctacggcaag 1140
accaagtgca ccgccagcaa taagaaccgg gggatcatta agaccttcag caacggctgc 1200
gactatgtga gcaacaaggg cgtggacacc gtgagcgtgg gcaacacact gtactacgtg 1260
aacaaacagg agggaaagag cctgtacgtg aagggcgagc ccatcatcaa cttctacgac 1320
cccctggtgt tccccagctg cgagttttgc gccagcatca gccaggtgaa cgagaagatc 1380
aatcagagcc tggccttcat caggaagagc gatgagctgc tgcacaacgt ggaggacaag 1440
atcgaggaga tcctgagcaa gatctaccac atcgagaacg agatcgccag gatcaagaag 1500
ctgatcggcg aggccaacgc cggcaagagc acgaccaaca tcatgatcac caccatcatt 1560
atcgtgatca tcgtgatcct gctggccctg attgccgtgg gcctgctgct gtactgcaag 1620
gccaggtcta cccctgtgac cctgagcaag gaccagctgt ctggcatcaa caacatcgcc 1680
ttctctaatt ga 1692

SEQ ID NO: 58
length 1674
molecule type RNA
division PAT

Key Location/Qualifiers
source 1..1674
/mol_type=other RNA
/organism=synthetic construct

Sequence
atggagctgc ccatcctgaa gaccaacgcc atcacaacaa tcctggccgc cgtgaccctg 60
tgcttcgcct ctagccagaa cattaccgag gagttttatc agagcacatg cagcgccgtg 120
agcaagggct acctgggcgc cctgaggacc ggctggtaca caagcgtgat tacaatcgag 180
ctgagcaaca ttaaggagaa caagtgcaac ggcaccgacg ccaaggtgaa gctgatcaag 240
caggagctgg acaagtacaa gaatgccgtg accgatctgc agctgctgat gcagagcaca 300
ccttgtggag gatctggcag cgccatcgct tctggcatcg ctgtgtgtaa ggtgctgcac 360
ctggagggag aggtgaataa gatcaagagc gccctgctga gcacaaacaa agccgtggtg 420
agcctgagca acggagtgag cgtgctgacc ttcaaggtgc tggacctgaa aaactacatc 480
gacaagcagc tgctccccat cgtgaacaag cagagctgca gcatccccaa catcgaaacc 540
gtgatcgagt ttcagcagaa gaacaacagg ctgctggaga tcacccggga gtttagcgtc 600
aacgccggcg tgaccacccc tgtgtccacc tacatgctga ccaacagcga gctgctgagc 660
ctgatcaacg acatgcccat caccaacgat cagaagaagc tgatgtctag caacgtgcag 720
atcgtgcggc agcagagcta cagcatcatg tgcatcatca aggaggaggt gctggcctat 780
gtggtgcagc tgcctctgta cggcgtgatc gacaccccct gctggaagtt gcatacaagc 840
cccctgtgca ccaccaatac caaggaaggc agcaatatct gcctgacaag aaccgataga 900
ggctggtatt gcgacaatgc cggcagcgtg agcttcttcc ctcaggctga aacatgcaag 960
gtgcagtgca accgggtgtt ctgcgacacc atgaacagcc tgaccctgcc cagcgaggtg 1020
aacctgtgca acatcgacat cttcaatccc aagtacgact gcaagatcat gaccagcaag 1080
accgacgtga gcagctctgt gatcaccagc ctgggagcca tcgtgagctg ctacggcaag 1140
accaagtgca ccgccagcaa taagaaccgg gggatcatta agaccttcag caacggctgc 1200
gactatgtga gcaacaaggg cgtggacacc gtgagcgtgg gcaacacact gtactacgtg 1260
aacaaacagg agggaaagag cctgtacgtg aagggcgagc ccatcatcaa cttctacgac 1320
cccctggtgt tccccagctg cgagttttgc gccagcatca gccaggtgaa cgagaagatc 1380
aatcagagcc tggccttcat caggaagagc gatgagctgc tgcacaacgt ggaggacaag 1440
atcgaggaga tcctgagcaa gatctaccac atcgagaacg agatcgccag gatcaagaag 1500
ctgatcggcg aggccaacgc cggcaagagc acgaccaact ggccttggta catctggctg 1560
ggcttcatcg ccggcctgat cgccatcgtg atggtgacaa tcatgctgtg ctgcatgacc 1620
agctgttgta gctgcctgaa gggctgctgc tcctgtggat cttgctgcaa gtga 1674

SEQ ID NO: 59
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140

tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaggaca agatcgagga gatcctgagc aagatctacc acatcgagaa cgagatcgcc 1500
aggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacccctgtg accctgagca aggatcagct gagcggcatc 1680
aataatatcg ccttctccaa c 1701

SEQ ID NO: 60
length 1755
molecule type RNA
division PAT

Key Location/Qualifiers
source 1..1755
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaggaca agatcgagga gatcctgagc aagatctacc acatcgagaa cgagatcgcc 1500
aggatcaaga agctgatcgg cgaggccatc taccacatcg agaacgagat cgccaggatc 1560
aagaagctga tcggcgaggc caacgccggc aagagcacca ccaacatcat gatcaccacc 1620
atcatcatcg tgatcattgt gatcctgctg gccctgatcg ccgtgggcct gctgctgtac 1680
tgtaaggcca ggtctacccc tgtgaccctg agcaaggatc agctgagcgg catcaataat 1740
atcgccttct ccaac 1755

SEQ ID NO: 61
length 1734
molecule type RNA
division PAT

Key Location/Qualifiers
source 1..1734

/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct catccacaac 1440
gtgaatggca gcatcaatca gatcaatgcc agcatcagga agatcaatga gagcatcaat 1500
ggcagcatca atcagatcaa tgccagcatc aggaagatca atgagagcat ctcccagatc 1560
aacgccggca agagcaccac caacatcatg atcaccacca tcatcatcgt gatcattgtg 1620
atcctgctgg ccctgatcgc cgtgggcctg ctgctgtact gtaaggccag gtctacccct 1680
gtgaccctga gcaaggatca gctgagcggc atcaataata tcgccttctc caac 1734

SEQ ID NO: 62
length 1680
molecule type RNA
division PAT

Key Location/Qualifiers
source 1..1680
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900

agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct catccacaac 1440
gtgaatgaga agatcaatca gatcagcgcc agcatcagga agatcgatga gagcatctcc 1500
cagatcaacg ccggcaagag caccaccaac atcatgatca ccaccatcat catcgtgatc 1560
attgtgatcc tgctggccct gatcgccgtg ggcctgctgc tgtactgtaa ggccaggtct 1620
acccctgtga ccctgagcaa ggatcagctg agcggcatca ataatatcgc cttctccaac 1680

SEQ ID NO: 63
length 1734
molecule type RNA
division PAT

Key Location/Qualifiers
source 1..1734
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct catccacaac 1440
gtgaatgaga agatcaatca gatcagcgcc agcatcagga agatcgatga gagcatcaat 1500
gagaagatca atcagatcag cgccagcatc aggaagatcg atgagagcat ctcccagatc 1560
aacgccggca agagcaccac caacatcatg atcaccacca tcatcatcgt gatcattgtg 1620
atcctgctgg ccctgatcgc cgtgggcctg ctgctgtact gtaaggccag gtctacccct 1680
gtgaccctga gcaaggatca gctgagcggc atcaataata tcgccttctc caac 1734

SEQ ID NO: 64
length 1701
molecule type RNA
division PAT

Key                 Location/Qualifiers
source              1..1701
                    /mol_type=other RNA
                    /organism=synthetic construct

Sequence
atggagctgc ccatcctgaa gaccaacgcc attacaacca tcctggccgc cgtgacactg   60
tgttttgcca gcagccagaa catcaccgag gagttttacc agagcacctg cagcgccgtg   120
agcaagggat atctgggcgc cctgagaaca ggctggtaca caagcgtgat caccatcgag   180
ctgagcaaca tcaaggagaa caagtgcaac ggcaccgacg ccaaggtgaa gctgattaag   240
caggagctgg acaagtacaa gaacgccgtg accgatctgc agctgctgat gcagagcaca   300
ccatgtggcg gatctggagg aggatctgga tctgccatcg cttctggaat cgccgtgtgt   360
aaagtgctgc acctggaggg cgaagtgaac aagatcaaaa gcgccctgct gagcaccaac   420
aaggccgtgg tgagcctgag caatggcgtg agcgtgctga ccttcaaggt gctggacctg   480
aaaaactaca tcgacaagca gctgctcccc atcgtgaaca agcagagctg cagcatctcc   540
aacatcgaga cagtgatcga gtttcagcag aagaacaacc ggctgctgga gatcacccgg   600
gagtttagcg tgaacgccgg cgtgaccacc ccagtgagca cctacatgct gaccaatagc   660
gagctgctga gcctgatcaa cgacatgccc atcaccaacg accagaagaa gctcatgagc   720
agcaacgtgc agatcgtgag gcagcagagc tacagcatca tgtgcattat caaggaggag   780
gtgctggcct acgtggtgca gctgcctctg tacggcgtga tcgatacccc ctgctggaag   840
ctgcacacat ctcctctgtg cacaaccaac accaaagagg gcagcaatat ctgcctgacc   900
agaaccgata ggggctggta ctgtgacaat gccggcagcg tgtctttctt ccctcaggcc   960
gagacatgca aggtgcagtg caatagggtg ttctgcgaca ccatgaacag cctgaccctg   1020
cccagcgagg tgaacctgtg caacatcgac atcttcaacc ccaagtacga ctgcaagatc   1080
atgaccagca agaccgacgt gagcagctcc gtgatcacaa gcctgggcgc tatcgtgagc   1140
tgttacggca agaccaagtg caccgccagc aacaagaatc ggggcatcat caagaccttc   1200
agcaacggct gcgactacgt gagcaacaag ggcgtggata cagtgagcgt gggcaatacc   1260
ctgtactacg tgaacaaaca ggagggcaag agcctgtacg tgaagggcga gcccatcatc   1320
aacttctacg accctctggt gttccccagc tgcgagtttt gcgccagcat cagccaggtg   1380
aacgagaaga tcaaccagag cctggccttc atccggaaga gcgatgagct gctgcacaac   1440
gtggaggaca agatcgagga gatcctgagc aagatctacc acatcgagaa cgagatcgcc   1500
aggatcaaga agctgatcgg cgaggccaac gccggcaaga gcaccaccaa catcatgatc   1560
accaccatca tcatcgtgat cattgtgatc ctgctggccc tgatcgccgt gggcctgctg   1620
ctgtactgca aggccagaag cacaccagtg accctgtcca aggatcagct gtctggcatc   1680
aataacatcg ccttctccaa c                                             1701

SEQ ID NO:          65
length              1701
molecule type       RNA
division            PAT

Key                 Location/Qualifiers
source              1..1701
                    /mol_type=other RNA
                    /organism=synthetic construct

Sequence
atggagctgc ccatcctgaa gaccaacgcc attacaacca tcctggccgc cgtgacactg   60
tgttttgcca gcagccagaa catcaccgag gagttttacc agagcacctg cagcgccgtg   120
agcaagggat atctgggcgc cctgagaaca ggctggtaca caagcgtgat caccatcgag   180
ctgagcaaca tcaaggagaa caagtgcaac ggcaccgacg ccaaggtgaa gctgattaag   240
caggagctgg acaagtacaa gaacgccgtg accgatctgc agctgctgat gcagagcaca   300
ccttgtggcg gatctggagg aggatctgga tctgccatcg cttctggcat tgccgtgtgt   360
aaagtgctgc acctggaggg cgaagtgaac aagatcaaat ctgccctgct gagcaccaac   420
aaggccgtgg tgagcctgag caacggcgtg agcgtgctga ccttcaaggt gctggacctg   480
aaaaactaca tcgacaagca gctgctcccc atcgtgaaca agcagagctg cagcatctcc   540
aacatcgaga cagtgatcga gtttcagcag aagaacaacc ggctgctgga gatcacccgg   600
gagtttagcg tgaacgccgg cgtgaccacc cctgtgagca cctacatgct gaccaatagc   660
gagctgctga gcctgatcaa cgacatgccc atcaccaacg accagaagaa gctcatgagc   720
agcaacgtgc agatcgtgag gcagcagagc tacagcatca tgtgcattat caaggaggag   780

gtgctggcct acgtggtgca gctgcctctg tacggcgtga tcgatacccc ctgttggaag 840
ctgcacacat ctcctctgtg cacaaccaac accaaggagg gcagcaatat ctgcctgacc 900
agaaccgata ggggctggta ctgtgacaat gccggctctg tgtctttctt ccctcaggcc 960
gagacatgca aggtgcagtg caatagggtg ttctgcgaca ccatgaacag cctgaccctg 1020
cccagcgagg tgaacctgtg caacatcgac atcttcaacc ccaagtacga ctgcaagatc 1080
atgaccagca agaccgacgt gagcagctcc gtgatcacaa gcctgggcgc tatcgtgagc 1140
tgttacggca agaccaagtg caccgccagc aacaagaatc ggggcatcat caagaccttc 1200
agcaacggct gcgactacgt gagcaacaag ggcgtggata cagtgagcgt gggcaatacc 1260
ctgtactacg tgaacaaaca ggagggaaag agcctgtacg tgaagggcga gcccatcatc 1320
aacttctacg accccctggt gttccccagc tgcgagtttt gcgccagcat cagccaggtg 1380
aacgagaaga tcaaccagag cctggccttc atccggaaga gcgatgagct gctgcacaac 1440
gtggaggaca agatcgagga gatcctgagc aaaatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccagatc taccccagtg accctgagca aggatcagct gtctggcatc 1680
aataacatcg ccttctccaa c 1701

SEQ ID NO: 66
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgttttgcca gcagccagaa catcaccgag gagttttacc agagcacctg cagcgccgtg 120
agcaagggat atctgggcgc cctgagaaca ggctggtaca ctagcgtgat caccatcgag 180
ctgagcaaca tcaaggagaa caagtgcaac ggcaccgacg ccaaggtgaa gctgataaag 240
caggagctgg acaagtacaa gaacgccgtg actgacctgc agctgctgat gcagagcaca 300
ccttgtggcg gatctggcgg aggatctgga tctgccatcg cttctggcat tgccgtgtgt 360
aaagtgctgc acctggaggg cgaagtgaac aagatcaaat ctgccctgct gagcaccaac 420
aaggccgtgg tgagcctgag caatggcgtg agcgtgctga ccttcaaggt gctggacctg 480
aaaaactaca tcgacaagca gctgctcccc atcgtgaaca agcagagctg cagcatctcc 540
aacatcgaga cagtgatcga gtttcagcag aagaacaacc ggctgctgga gatcacccgg 600
gagtttagcg tgaacgccgg cgtgacaacc ccagtgagca cctacatgct gaccaacagc 660
gagctgctga gcctgatcaa cgacatgccc atcaccaacg accagaagaa gctcatgagc 720
agcaacgtgc agatcgtgag gcagcagagc tacagcatca tgtgcattat caaggaggag 780
gtgctggcct acgtggtgca gctgcctctg tacggcgtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tgaacctgtg caacatcgac atcttcaacc ccaagtacga ctgcaagatc 1080
atgaccagca agaccgacgt gtctagctcc gtgatcacaa gcctgggagc tatcgtgagc 1140
tgctacggca agaccaagtg caccgccagc aacaagaatc ggggcatcat caagaccttc 1200
agcaacggct gcgactacgt gagcaacaag ggggtggata cagtgagcgt gggcaatacc 1260
ctgtactacg tgaataagca ggagggcaag agcctgtacg tgaaaggcga gcccatcatc 1320
aacttctacg accccctggt gttccccagc tgcgagtttt gcgccagcat cagccaggtg 1380
aacgagaaga tcaatcagag cctggccttc atccggaaga gcgacgagct cctgcacaac 1440
gtggaggaca agatcgagga gatcctgagc aaaatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccagatc tacccccgtg accctgagca aggatcagct gagcggcatc 1680
aataatatcg ccttctccaa c 1701

SEQ ID NO: 67
length 1704

molecule type        RNA
division             PAT

Key                  Location/Qualifiers
source               1..1704
                     /mol_type=other RNA
                     /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggcgg aggatctggc agcgctatcg cctctggaat tgccgtgtgt  360
aaagtgctgc acctggaggg cgaagtgaac aagatcaaaa gcgccctgct gagcaccaac  420
aaggctgtgg tgagcctgag caatggcgtg agcgtgctga ccttcaaggt gctggacctg  480
aaaaactaca ttgacaagca gctgctcccc atcgtgaaca agcagagctg cagcatcagc  540
aacatcgaga cggtgatcga gtttcagcag aagaacaacc ggctgctgga gatcacccgg  600
gagtttagcg tgaacgctgg cgtgaccacc ccagtgagca cctatatgct gaccaacagc  660
gagctgctga gcctgatcaa cgacatgccc atcaccaacg atcagaagaa gctgatgagc  720
agcaacgtgc agatcgtgag gcagcagagc tacagcatca tgtgcatcat caaggaggag  780
gtgctggcct acgtggtgca gctgcctctg tacggagtga tcgacacccc ctgttggaaa  840
ctgcacacat ctcctctgtg cacaaccaac accaaggagg gcagcaacat ctgcctgaca  900
agaaccgata ggggctggta ctgtgacaat gccggctctg tgtctttctt tcctcaggcc  960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc gacgagtttg acgccagcat cagccaggtg 1380
aacgagaaaa tcaatcagag cctggccttc atcaggaaga gcgacgagct cctgcacaac 1440
gtggaagata aaattgaaga aattctctcc aaaatttatc atattgaaaa tgaaattgct 1500
agaattaaaa aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa ctga                                         1704

SEQ ID NO:           68
length               1704
molecule type        RNA
division             PAT

Key                  Location/Qualifiers
source               1..1704
                     /mol_type=other RNA
                     /organism=synthetic construct

Sequence
atggagttgc cgatattgaa gactaatgcc attacaacga tccttgctgc ggtcaccctg   60
tgcttcgcgt cttcgcagaa tatcacggag gagttctacc agtccacctg cagcgccgtg  120
tccaagggct accttggggc gctgcggacg ggctggtaca cctccgtgat aacgatcgag  180
ctcagcaaca tcaaggagaa caagtgtaac ggcactgatg ctaaggtcaa gctcatcaag  240
caggagcttg acaagtataa gaatgccgtt acagacctcc aactcctgat gcagagcacc  300
ccatgcggcg gttccggagg cggtagcggc agcgctatcg cctccggaat cgccgtatgc  360
aaggttctgc atctggaggg ggaggtcaac aagatcaaga gcgctctgct tagcacgaac  420
aaggcagtag ttagcttgtc gaatggggtt tcggtcctga ccttcaaggt cctggacctg  480
aaaaactata tcgacaagca gctgctgcct atcgtgaata agcagagctg ctctatctcg  540
aacatcgaga cggtgatcga gttccagcag aagaacaatc gtctgctgga gatcacccgt  600
gagttcagcg tgaacgccgg ggtgacaacc ccggtgtcca cgtacatgct cacgaactcc  660

gagttgctga gcttgatcaa cgatatgccc attactaatg atcagaagaa gctgatgagt 720
agtaatgtgc agatcgtgcg gcagcagtcc tacagcatca tgtgcataat caaggaggag 780
gtcttggcct acgttgtgca gcttccactg tacggggtga tcgacacccc gtgctggaag 840
ctgcacacga gccccctttg taccaccaat acaaaggagg gctcgaacat ctgcctgacg 900
aggacggacc ggggctggta ttgcgacaat gccggctccg tgtccttctt ccctcaggca 960
gagacttgta aggtgcagtg caaccgggtc ttctgtgaca ccatgaactc actaacgctc 1020
cccagcgagg tgaatctctg caacatcgac atattcaacc cgaagtatga ttgcaaaatc 1080
atgacttcga agacggatgt gtcgagttca gtgatcacct cgctgggggc gatagtgagt 1140
tgttacggca agaccaagtg cactgcatct aacaagaacc gtgggatcat caagaccttc 1200
tccaatggtt gtgactacgt gagcaacaag ggggtcgaca cagttagtgt gggcaacacc 1260
ctttactacg tgaacaagca ggagggcaag tcactgtacg tgaagggtga gcccatcatt 1320
aacttctacg acccccttgt gttcccctcc gatgagtttg atgcgagcat atctcaggtc 1380
aatgagaaga tcaatcagag tctcgcgttc atccgaaagt cggatgagct cctccacaat 1440
gtggaggaca agatcgagga gattctgagc aagatctatc acattgagaa tgagattgct 1500
cgcatcaaga agctcatcgg agaggccaac gccggcaaga gcacgacgaa cataatgata 1560
accaccatta tcattgtgat catcgtgatc ttgctggcgt tgattgctgt gggactgctg 1620
ctgtactgca aggcgcgaag cacaccggtg accctcagca aggatcagtt gagtggcatt 1680
aacaatatcg ccttctccaa ctga 1704

SEQ ID NO: 69
length 1704
molecule type RNA
division PAT

Key Location/Qualifiers
source 1..1704
/mol_type=other RNA
/organism=synthetic construct

Sequence
atggagctgc cgatattgaa gactaatgcc attacaacga tccttgctgc ggtcaccctg 60
tgcttcgcgt cttcgcagaa tatcacggag gagttctacc agtccacctg cagcgccgtg 120
tccaagggct accttggggc gctgcggacg ggctggtaca cctccgtgat aacgatcgag 180
ctcagcaaca tcaaggagaa caagtgtaac ggcactgatg ctaaggtcaa gctcatcaag 240
caggagcttg acaagtataa gaatgccgtt acagacctcc aactcctgat gcagagcacc 300
ccatgcggcg gttccggagg cggtagcggc agcgctatcg cctccggaat cgccgtgtgc 360
aaggtgctgc atctggaggg ggaggtcaac aagatcaaga gcgctctgct gtccacgaac 420
aaggcagtgg tgagcttgtc gaatggggtt tcggtcctga ccttcaaggt cctggacctg 480
aaaaactata tcgacaagca gctgctgcct atcgtgaaca agcagagctg ctctatctcg 540
aacatcgaga cggtgatcga gttccagcag aagaacaatc ggctgctgga gatcacccgt 600
gagttcagcg tgaacgccgg ggtgacaacc ccggtgtcca cgtacatgct cacgaactcc 660
gagttgctga gcttgatcaa cgatatgccc attactaatg atcagaagaa gctgatgagt 720
agtaatgtgc agatcgtgcg gcagcagtcc tacagcatca tgtgcatcat caaggaggag 780
gtgcttgctt acgtggtgca gctcccactg tacggggtga tcgacacccc ttgttggaag 840
ctgcacacct ctcctttgtg taccaccaac acaaaggagg ggagcaacat ctgcctgacg 900
aggacggacc ggggctggta ttgcgacaat gccggctccg tgtccttctt ccctcaggca 960
gagacttgta aggtgcagtg caaccgggtc ttctgtgaca ccatgaactc actgacgctg 1020
cccagcgagg tgaacctgtg caatattgac atcttcaacc cgaagtatga ttgcaagatc 1080
atgacttcga agacagatgt cagtagctca gtgatcacct cgctgggcgc gatagtgagt 1140
tgttacggca agaccaagtg cactgcatct aacaagaatc gcggcattat taagaccttt 1200
agtaatggct gcgattacgt ttccaacaag ggggtcgaca ccgtgtcagt gggaaacacg 1260
ctgtactacg tgaacaagca ggagggcaag tccttgtacg tcaagggcga gcccatcatc 1320
aacttctacg accccctggt gttcccctcc gatgagtttg atgcgagcat atctcaggtc 1380
aatgagaaga tcaaccagag tctggctttc atcagaaagt cagacgagct gctccacaat 1440
gtggaggaca agatcgagga gatcttgtcc aagatctatc acattgagaa tgagattgct 1500
cgcatcaaga agctcatcgg agaggcgaac gccgggaagt cgaccaccaa cataatgatc 1560
acgacgatca tcatcgtgat cattgtgatc ctccttgcac tgatcgctgt gggactgctg 1620
ctgtactgca aggcgcggag cacaccggtg accctcagca aggatcagtt gagtggcatt 1680
aacaatatcg ccttctccaa ctga 1704

SEQ ID NO:          70
length               1704
molecule type        RNA
division             PAT

Key                  Location/Qualifiers
source               1..1704
                     /mol_type=other RNA
                     /organism=synthetic construct

Sequence
atggagctgc ccatcctgaa gaccaacgcc atcaccacca tcctggctgc cgttacactg   60
tgcttcgcca gctcccagaa catcacagag gagttctacc agagcacctg ctccgcagtg  120
tccaagggct acctgggggc actgcggaca ggctggtaca cctctgtgat caccatcgag  180
ctgagcaaca tcaaggagaa caagtgtaac ggcaccgatg ccaaggtgaa gctgatcaag  240
caggagctgg acaagtacaa gaacgccgtg accgacctgc agctgctgat gcagagcacc  300
ccctgcgggg ggtccggcgg gggcagcggc tccgccattg ccagtggcat cgccgtctgc  360
aaggtgctgc acctggaggg cgaggtgaac aagatcaaga gcgccctcct gagcaccaac  420
aaggcggtgg tgtcactgag caatggcgtg tcagtgctga ccttcaaggt gctggacctg  480
aagaactaca ttgacaagca gctgctgccc attgtgaaca agcagtcctg ctccatcagc  540
aacatcgaga ccgtgatcga gttccagcag aagaacaatc ggctgctgga gatcaccagg  600
gagttctccg tgaacgccgg ggtgaccacc cccgtgagca catacatgct gaccaatagc  660
gagctgctgt ctctgatcaa cgacatgccc atcaccaacg accagaagaa gctgatgagc  720
agcaacgtgc agatcgtgag acagcagagc tacagcatta tgtgcatcat caaggaggag  780
gtgctggcct atgtggtgca gctgccactg tatggggtga tcgacacccc atgctggaag  840
ctgcacacct cccctctgtg taccaccaac accaaggagg ggagcaacat ctgcctgacc  900
aggacagacc ggggctggta ttgcgacaat gccggctccg tgtctttctt ccctcaggca  960
gagacttgta aggtgcagtg caaccgggtc ttctgtgaca ccatgaatag cctgaccctg 1020
cccagtgagg tgaacctgtg caacatcgac atcttcaatc ccaagtacga ttgcaagatc 1080
atgaccagca agaccgatgt gagcagcagc gtgatcacct cactgggcgc tatcgtgagc 1140
tgttacggca agaccaagtg cactgcatct aacaagaaca ggggcatcat caagaccttc 1200
tccaatggat gcgactacgt gtccaacaag ggggtggaca ccgtgagcgt ggggaacacc 1260
ctgtactacg tgaacaagca ggagggcaag tccctgtacg tcaaggggga gcccatcatc 1320
aacttctatg accccctggt gttcccctct gacgagttcg atgccagcat tagccaggtc 1380
aatgagaaga tcaaccagag cctggccttc attcgcaaga gcgacgagct gctgcacaat 1440
gtggaggaca agatcgagga gatcctgtcc aagatctacc acattgagaa tgagatcgcc 1500
aggatcaaga agctcattgg cgaggctaat gctggcaaga gcaccaccaa catcatgatc 1560
acaaccatca tcattgtgat catcgtgatc ctgctggcac tgattgctgt gggcctgctg 1620
ctgtactgca aggcccgcag caccccagtg accctgagca aggatcagct gagcggcatc 1680
aacaacattg ccttcagcaa ctga                                        1704

SEQ ID NO:          71
length               1701
molecule type        RNA
division             PAT

Key                  Location/Qualifiers
source               1..1701
                     /mol_type=other RNA
                     /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggcgg aggatctggc agcgctatcg cctctggaat tgccgtgtgt  360
aaagtgctgc acctggaggg cgaagtgaac aagatcaaaa gcgccctgct gagcaccaac  420
aaggctgtgg tgagcctgag caatggcgtg agcgtgctga ccttcaaggt gctggacctg  480
aaaaactaca ttgacaagca gctgctcccc atcgtgaaca agcagagctg cagcatcccc  540

aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaggaca agatcgagga gatcctgagc aagatctacc acatcgagaa cgagatcgcc 1500
aggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacccctgtg accctgagca aggatcagct gagcggcatc 1680
aataatatcg ccttctccaa c 1701

SEQ ID NO: 72
length 1599
molecule type RNA
division PAT

Key Location/Qualifiers
source 1..1599
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcccc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtgaacgccg gcaagagcac caccaactgg ccttggtaca tctggctggg cttcatcgcc 1500
ggcctgatcg ccatcgtgat ggtgacaatc atgctgtgct gcatgaccag ctgttgtagc 1560
tgcctgaagg gctgctgctc ctgtggatct tgctgcaag 1599

SEQ ID NO: 73
length 1683
molecule type RNA
division PAT

Key Location/Qualifiers
source 1..1683
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcccc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaggaca agatcgagga gatcctgagc aagatctacc acatcgagaa cgagatcgcc 1500
aggatcaaga agctgatcgg cgaggccaac gccggcaaga gcaccaccaa ctggccttgg 1560
tacatctggc tgggcttcat cgccggcctg atcgccatcg tgatggtgac aatcatgctg 1620
tgctgcatga ccagctgttg tagctgcctg aagggctgct gctcctgtgg atcttgctgc 1680
aag 1683

SEQ ID NO: 74
length 1620
molecule type RNA
division PAT

Key Location/Qualifiers
source 1..1620
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540

aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtgaacgccg gcaagagcac caccaacatc atgatcacca ccatcatcat cgtgatcatt 1500
gtgatcctgc tggccctgat cgccgtgggc ctgctgctgt actgtaaggc caggtctacc 1560
cctgtgaccc tgagcaagga tcagctgagc ggcatcaata atatcgcctt ctccaactga 1620

SEQ ID NO: 75
length 1620
molecule type RNA
division PAT

Key Location/Qualifiers
source 1..1620
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc gacgagtttg acgccagcat cagccaggtg 1380
aacgagaaaa tcaatcagag cctggccttc atcaggaaga gcgacgagct cctgcacaac 1440
gtgaacgccg gcaagagcac caccaacatc atgatcacca ccatcatcat cgtgatcatt 1500
gtgatcctgc tggccctgat cgccgtgggc ctgctgctgt actgtaaggc caggtctacc 1560
cctgtgaccc tgagcaagga tcagctgagc ggcatcaata atatcgcctt ctccaactga 1620

SEQ ID NO: 76
length 1683

molecule type        RNA
division             PAT

Key                  Location/Qualifiers
source               1..1683
                     /mol_type=other RNA
                     /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggcgg aggatctggc agcgctatcg cctctggaat tgccgtgtgt  360
aaagtgctgc acctggaggg cgaagtgaac aagatcaaaa gcgccctgct gagcaccaac  420
aaggctgtgg tgagcctgag caatggcgtg agcgtgctga ccttcaaggt gctggacctg  480
aaaaactaca ttgacaagca gctgctcccc atcgtgaaca agcagagctg cagcatcagc  540
aacatcgaga cggtgatcga gtttcagcag aagaacaacc ggctgctgga gatcacccgg  600
gagtttagcg tgaacgctgg cgtgaccacc ccagtgagca cctatatgct gaccaacagc  660
gagctgctga gcctgatcaa cgacatgccc atcaccaacg atcagaagaa gctgatgagc  720
agcaacgtgc agatcgtgag gcagcagagc tacagcatca tgtgcatcat caaggaggag  780
gtgctggcct acgtggtgca gctgcctctg tacggagtga tcgacacccc ctgttggaaa  840
ctgcacacat ctcctctgtg cacaaccaac accaaggagg gcagcaacat ctgcctgaca  900
agaaccgata ggggctggta ctgtgacaat gccggctctg tgtctttctt tcctcaggcc  960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cattcacaac 1440
gtgaacggca gcatcaacca gatcaatgcc tctatcagaa agatcaacga gtccatcagc 1500
cagatcaacg ccggcaagag caccaccaac atcatgatca ccaccatcat catcgtgatc 1560
attgtgatcc tgctggccct gatcgccgtg ggcctgctgc tgtactgtaa ggccaggtct 1620
acccctgtga ccctgagcaa ggatcagctg agcggcatca ataatatcgc cttctccaac 1680
tga                                                               1683

SEQ ID NO:           77
length               1683
molecule type        RNA
division             PAT

Key                  Location/Qualifiers
source               1..1683
                     /mol_type=other RNA
                     /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggcgg aggatctggc agcgctatcg cctctggaat tgccgtgtgt  360
aaagtgctgc acctggaggg cgaagtgaac aagatcaaaa gcgccctgct gagcaccaac  420
aaggctgtgg tgagcctgag caatggcgtg agcgtgctga ccttcaaggt gctggacctg  480
aaaaactaca ttgacaagca gctgctcccc atcgtgaaca agcagagctg cagcatcagc  540
aacatcgaga cggtgatcga gtttcagcag aagaacaacc ggctgctgga gatcacccgg  600
gagtttagcg tgaacgctgg cgtgaccacc ccagtgagca cctatatgct gaccaacagc  660

gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc gacgagtttg acgccagcat cagccaggtg 1380
aacgagaaaa tcaatcagag cctggccttc atcaggaaga gcgacgagct cattcacaac 1440
gtgaacggca gcatcaacca gatcaatgcc tctatcagaa agatcaacga gtccatcagc 1500
cagatcaacg ccggcaagag caccaccaac atcatgatca ccaccatcat catcgtgatc 1560
attgtgatcc tgctggccct gatcgccgtg ggcctgctgc tgtactgtaa ggccaggtct 1620
acccctgtga ccctgagcaa ggatcagctg agcggcatca ataatatcgc cttctccaac 1680
tga 1683

SEQ ID NO: 78
length 1704
molecule type RNA
division PAT

Key Location/Qualifiers
source 1..1704
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaagata aaattgaaga aattctctcc aaaatttatc atattgaaaa tgaaattgct 1500
agaattaaaa aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa ctga 1704

SEQ ID NO:            79
length                 1704
molecule type          RNA
division               PAT

Key                    Location/Qualifiers
source                 1..1704
                       /mol_type=other RNA
                       /organism=synthetic construct

Sequence
atggagctgc ccatcctgaa gacgaatgcc attaccacca tcctggccgc cgtcacgctg   60
tgctttgcca gcagccagaa catcaccgag gagttctacc agtccacctg ctccgcagtg  120
tccaagggct acctgggggc actgcggacc gggtggtaca ccagcgtcat taccattgag  180
ctctccaaca tcaaggagaa caagtgcaat ggtactgacg ctaaggtgaa gcttatcaag  240
caggagcttg ataagtacaa gaacgccgtg actgatctgc agctgctgat gcagagcaca  300
ccgtgcggcg gctcaggagg tggtagtggc agcgccattg ccagcggcat cgccgtctgc  360
aaggtgctgc acctggaggg cgaggtgaac aagatcaaga gcgccctcct gagcaccaac  420
aaggcggtgg tgtcgctgag caatggcgtg agcgtgttga ccttcaaggt gctggatctc  480
aagaactaca tcgataagca gctgctgccg attgtgaaca agcagtcgtg cagcatcagc  540
aatatcgaga cagtgattga gttccagcag aagaacaaca ggctcctgga gatcaccaga  600
gagttctcag tgaacgccgg ggtgaccact cctgtgagca catacatgct gaccaatagc  660
gagctgctgt ctctgatcaa cgatatgccc attactaatg atcagaagaa gctgatgagc  720
agtaatgtgc agatcgtgag acagcagagc tacagcatta tgtgcatcat caaggaggag  780
gtgcttgctt acgtggtgca gctgcccctg tacggggtga tcgacacccc ttgttggaag  840
ctgcacacct ctcctttgtg taccaccaac acaaaggagg ggagcaacat ctgcctgacg  900
aggacggacc ggggctggta ttgcgacaat gccggctccg tgtccttctt ccctcaggca  960
gagacttgta aggtgcagtg caaccgggtc ttctgtgaca ccatgaatag cctgaccctg 1020
cccagcgagg tgaacctgtg caacatcgac atcttcaacc cgaagtatga ttgcaagatc 1080
atgacttcga agaccgatgt gagcagcagc gtgatcacct cgctgggcgc tatcgtgagc 1140
tgttacggca agaccaagtg cactgcatct aacaagaatc gcggcattat taagaccttt 1200
agtaatggct gcgattacgt ttccaacaag ggggtcgaca ccgtgtcagt gggcaacacg 1260
ctgtactacg tgaacaagca ggaggggaag agcctgtacg tgaaggggga gcccatcatc 1320
aacttttacg acccactggt cttcccctcc gatgagttcg acgccagcat tagccaggtc 1380
aatgagaaga ttaatcagag cctggctttc attcgcaaga gcgacgagct cttgcacaat 1440
gtggaggaca agatcgagga gatcttgtcc aagatctacc acattgagaa tgagatcgcc 1500
aggattaaga agctcattgg cgaggctaat gctggcaagt cgaccactaa catcatgatc 1560
acgacgatca tcatcgtgat catcgtgatc ctcctggccc tgattgcagt ggggctgctg 1620
ctctactgca aggccaggag caccccggtg acactgagca aggaccagct ctctgggatc 1680
aacaacatcg ccttcagcaa ctga                                        1704

SEQ ID NO:            80
length                 574
molecule type          AA
division               PAT

Key                    Location/Qualifiers
source                 1..574
                       /mol_type=protein
                       /organism=synthetic construct

Sequence
MELLIHRSSA IFLTLAINAL YLTSSQNITE EFYQSTCSAV SRGYLSALRT GWYTSVITIE   60
LSNIKETKCN GTDTKVKLIK QELDKYKNAV TELQLLMQNT PAVNNRARRE APQYMNYTIN  120
TTKNLNVSIS KKRKRRFLGF LLGVGSAIAS GIAVSKVLHL EGEVNKIKNA LQLTNKAVVS  180
LSNGVSVLTS KVLDLKNYIN NQLLPIVNQQ SCRISNIETV IEFQQKNSRL LEITREFSVN  240
AGVTTPLSTY MLTNSELLSL INDMPITNDQ KKLMSSNVQI VRQQSYSIMS IIKEEVLAYV  300
VQLPIYGVID TPCWKLHTSP LCTTNIKEGS NICLTRTDRG WYCDNAGSVS FFPQADTCKV  360
QSNRVFCDTM NSLTLPSEVS LCNTDIFNSK YDCKIMTSKT DISSSVITSL GAIVSCYGKT  420
KCTASNKNRG IIKTFSNGCD YVSNKGVDTV SVGNTLYYVN KLEGKNLYVK GEPIINYYDP  480
LVFPSDEFDA SISQVNEKIN QSLAFIRRSD ELLHNVNTGK STTNIMITAI IIVIIVVLLS  540

LIAIGLLLYC KAKNTPVTLS KDQLSGINNI AFSK                                574

SEQ ID NO:          81
length              567
molecule type       AA
division            PAT

Key                 Location/Qualifiers
source              1..567
                    /mol_type=protein
                    /organism=synthetic construct

Sequence
MELLIHRSSA IFLTLAINAL YLTSSQNITE EFYQSTCSAV SRGYLGALRT GWYTSVITIE  60
LSNIKETKCN GTDTKVKLIK QELDKYKNAV TDLQLLMQNT PCGGSGGGSG SAIASGIAVC  120
KVLHLEGEVN KIKNALQLTN KAVVSLSNGV SVLTFKVLDL KNYINNQLLP IVNQQSCRIS  180
NIETVIEFQQ KNSRLLEITR EFSVNAGVTT PLSTYMLTNS ELLSLINDMP ITNDQKKLMS  240
SNVQIVRQQS YSIMCIIKEE VLAYVVQLPI YGVIDTPCWK LHTSPLCTTN IKEGSNICLT  300
RTDRGWYCDN AGSVSFFPQA DTCKVQCNRV FCDTMNSLTL PSEVSLCNTD IFNSKYDCKI  360
MTSKTDISSS VITSLGAIVS CYGKTKCTAS NKNRGIIKTF SNGCDYVSNK GVDTVSVGNT  420
LYYVNKLEGK NLYVKGEPII NYYDPLVFPS CEFCASISQV NEKINQSLAF IRRSDELLHN  480
VEDKIEEILS KIYHIENEIA RIKKLIGEAN TGKSTTNIMI TAIIIVIIVV LLSLIAIGLL  540
LYCKAKNTPV TLSKDQLSGI NNIAFSK                                       567

SEQ ID NO:          82
length              567
molecule type       AA
division            PAT

Key                 Location/Qualifiers
source              1..567
                    /mol_type=protein
                    /organism=synthetic construct

Sequence
MELLIHRSSA IFLTLAINAL YLTSSQNITE EFYQSTCSAV SRGYLSALRT GWYTSVITIE  60
LSNIKETKCN GTDTKVKLIK QELDKYKNAV TDLQLLMQNT PCGGSGGGSG SAIASGIAVC  120
KVLHLEGEVN KIKNALQLTN KAVVSLSNGV SVLTFKVLDL KNYINNQLLP IVNQQSCRIS  180
NIETVIEFQQ KNSRLLEITR EFSVNAGVTT PLSTYMLTNS ELLSLINDMP ITNDQKKLMS  240
SNVQIVRQQS YSIMCIIKEE VLAYVVQLPI YGVIDTPCWK LHTSPLCTTN IKEGSNICLT  300
RTDRGWYCDN AGSVSFFPQA DTCKVQCNRV FCDTMNSLTL PSEVSLCNTD IFNSKYDCKI  360
MTSKTDISSS VITSLGAIVS CYGKTKCTAS NKNRGIIKTF SNGCDYVSNK GVDTVSVGNT  420
LYYVNKLEGK NLYVKGEPII NYYDPLVFPS CEFCASISQV NEKINQSLAF IRRSDELLHN  480
VEDKIEEILS KIYHIENEIA RIKKLIGEAN TGKSTTNIMI TAIIIVIIVV LLSLIAIGLL  540
LYCKAKNTPV TLSKDQLSGI NNIAFSK                                       567

SEQ ID NO:          83
length              567
molecule type       AA
division            PAT

Key                 Location/Qualifiers
source              1..567
                    /mol_type=protein
                    /organism=synthetic construct

Sequence
MELLIHRSSA IFLTLAINAL YLTSSQNITE EFYQSTCSAV SRGYLGALRT GWYTSVITIE  60
LSNIKETKCN GTDTKVKLIK QELDKYKNAV TELQLLMQNT PCGGSGGGSG SAIASGIAVC  120
KVLHLEGEVN KIKNALQLTN KAVVSLSNGV SVLTFKVLDL KNYINNQLLP IVNQQSCRIS  180
NIETVIEFQQ KNSRLLEITR EFSVNAGVTT PLSTYMLTNS ELLSLINDMP ITNDQKKLMS  240

SNVQIVRQQS YSIMCIIKEE VLAYVVQLPI YGVIDTPCWK LHTSPLCTTN IKEGSNICLT 300
RTDRGWYCDN AGSVSFFPQA DTCKVQCNRV FCDTMNSLTL PSEVSLCNTD IFNSKYDCKI 360
MTSKTDISSS VITSLGAIVS CYGKTKCTAS NKNRGIIKTF SNGCDYVSNK GVDTVSVGNT 420
LYYVNKLEGK NLYVKGEPII NYYDPLVFPS CEFCASISQV NEKINQSLAF IRRSDELLHN 480
VEDKIEEILS KIYHIENEIA RIKKLIGEAN TGKSTTNIMI TAIIIVIIVV LLSLIAIGLL 540
LYCKAKNTPV TLSKDQLSGI NNIAFSK 567

SEQ ID NO: 84
length 567
molecule type AA
division PAT

Key Location/Qualifiers
source 1..567
/mol_type=protein
/organism=synthetic construct

Sequence
MELLIHRSSA IFLTLAINAL YLTSSQNITE EFYQSTCSAV SRGYLGALRT GWYTSVITIE 60
LSNIKETKCN GTDTKVKLIK QELDKYKNAV TDLQLLMQNT PCGGSGGGSG SAIASGIAVC 120
KVLHLEGEVN KIKNALQLTN KAVVSLSNGV SVLTSKVLDL KNYINNQLLP IVNQQSCRIS 180
NIETVIEFQQ KNSRLLEITR EFSVNAGVTT PLSTYMLTNS ELLSLINDMP ITNDQKKLMS 240
SNVQIVRQQS YSIMCIIKEE VLAYVVQLPI YGVIDTPCWK LHTSPLCTTN IKEGSNICLT 300
RTDRGWYCDN AGSVSFFPQA DTCKVQCNRV FCDTMNSLTL PSEVSLCNTD IFNSKYDCKI 360
MTSKTDISSS VITSLGAIVS CYGKTKCTAS NKNRGIIKTF SNGCDYVSNK GVDTVSVGNT 420
LYYVNKLEGK NLYVKGEPII NYYDPLVFPS CEFCASISQV NEKINQSLAF IRRSDELLHN 480
VEDKIEEILS KIYHIENEIA RIKKLIGEAN TGKSTTNIMI TAIIIVIIVV LLSLIAIGLL 540
LYCKAKNTPV TLSKDQLSGI NNIAFSK 567

SEQ ID NO: 85
length 567
molecule type AA
division PAT

Key Location/Qualifiers
source 1..567
/mol_type=protein
/organism=synthetic construct

Sequence
MELLIHRSSA IFLTLAINAL YLTSSQNITE EFYQSTCSAV SRGYLGALRT GWYTSVITIE 60
LSNIKETKCN GTDTKVKLIK QELDKYKNAV TDLQLLMQNT PCGGSGGGSG SAIASGIAVS 120
KVLHLEGEVN KIKNALQLTN KAVVSLSNGV SVLTFKVLDL KNYINNQLLP IVNQQSCRIS 180
NIETVIEFQQ KNSRLLEITR EFSVNAGVTT PLSTYMLTNS ELLSLINDMP ITNDQKKLMS 240
SNVQIVRQQS YSIMSIIKEE VLAYVVQLPI YGVIDTPCWK LHTSPLCTTN IKEGSNICLT 300
RTDRGWYCDN AGSVSFFPQA DTCKVQCNRV FCDTMNSLTL PSEVSLCNTD IFNSKYDCKI 360
MTSKTDISSS VITSLGAIVS CYGKTKCTAS NKNRGIIKTF SNGCDYVSNK GVDTVSVGNT 420
LYYVNKLEGK NLYVKGEPII NYYDPLVFPS CEFCASISQV NEKINQSLAF IRRSDELLHN 480
VEDKIEEILS KIYHIENEIA RIKKLIGEAN TGKSTTNIMI TAIIIVIIVV LLSLIAIGLL 540
LYCKAKNTPV TLSKDQLSGI NNIAFSK 567

SEQ ID NO: 86
length 567
molecule type AA
division PAT

Key Location/Qualifiers
source 1..567
/mol_type=protein
/organism=synthetic construct

Sequence
MELLIHRSSA IFLTLAINAL YLTSSQNITE EFYQSTCSAV SRGYLGALRT GWYTSVITIE 60
LSNIKETKCN GTDTKVKLIK QELDKYKNAV TDLQLLMQNT PCGGSGGGSG SAIASGIAVC 120
KVLHLEGEVN KIKNALQLTN KAVVSLSNGV SVLTFKVLDL KNYINNQLLP IVNQQSCRIS 180
NIETVIEFQQ KNSRLLEITR EFSVNAGVTT PLSTYMLTNS ELLSLINDMP ITNDQKKLMS 240
SNVQIVRQQS YSIMCIIKEE VLAYVVQLPI YGVIDTPCWK LHTSPLCTTN IKEGSNICLT 300
RTDRGWYCDN AGSVSFFPQA DTCKVQCNRV FCDTMNSLTL PSEVSLCNTD IFNSKYDCKI 360
MTSKTDISSS VITSLGAIVS CYGKTKCTAS NKNRGIIKTF SNGCDYVSNK GVDTVSVGNT 420
LYYVNKLEGK NLYVKGEPII NYYDPLVFPS DEFDASISQV NEKINQSLAF IRRSDELLHN 480
VEDKIEEILS KIYHIENEIA RIKKLIGEAN TGKSTTNIMI TAIIIVIIVV LLSLIAIGLL 540
LYCKAKNTPV TLSKDQLSGI NNIAFSK 567

SEQ ID NO: 87
length 602
molecule type AA
division PAT

Key Location/Qualifiers
source 1..602
/mol_type=protein
/organism=synthetic construct

Sequence
MELLIHRSSA IFLTLAINAL YLTSSQNITE EFYQSTCSAV SRGYLGALRT GWYTSVITIE 60
LSNIKETKCN GTDTKVKLIK QELDKYKNAV TDLQLLMQNT PCVNNRARRE APQYMNYTIN 120
TTKNLNVSIS KKRKRRFLGF LLGVGSAIAS GIAVCKVLHL EGEVNKIKNA LQLTNKAVVS 180
LSNGVSVLTF KVLDLKNYIN NQLLPIVNQQ SCRISNIETV IEFQQKNSRL LEITREFSVN 240
AGVTTPLSTY MLTNSELLSL INDMPITNDQ KKLMSSNVQI VRQQSYSIMC IIKEEVLAYV 300
VQLPIYGVID TPCWKLHTSP LCTTNIKEGS NICLTRTDRG WYCDNAGSVS FFPQADTCKV 360
QCNRVFCDTM NSLTLPSEVS LCNTDIFNSK YDCKIMTSKT DISSSVITSL GAIVSCYGKT 420
KCTASNKNRG IIKTFSNGCD YVSNKGVDTV SVGNTLYYVN KLEGKNLYVK GEPIINYYDP 480
LVFPSCEFCA SISQVNEKIN QSLAFIRRSD ELLHNVEDKI EEILSKIYHI ENEIARIKKL 540
IGEANTGKST TNIMITAIII VIIVVLLSLI AIGLLLYCKA KNTPVTLSKD QLSGINNIAF 600
SK 602

SEQ ID NO: 88
length 539
molecule type AA
division PAT

Key Location/Qualifiers
source 1..539
/mol_type=protein
/organism=synthetic construct

Sequence
MELLIHRSSA IFLTLAINAL YLTSSQNITE EFYQSTCSAV SRGYLGALRT GWYTSVITIE 60
LSNIKETKCN GTDTKVKLIK QELDKYKNAV TDLQLLMQNT PCGGSGGGSG SAIASGIAVC 120
KVLHLEGEVN KIKNALQLTN KAVVSLSNGV SVLTFKVLDL KNYINNQLLP IVNQQSCRIS 180
NIETVIEFQQ KNSRLLEITR EFSVNAGVTT PLSTYMLTNS ELLSLINDMP ITNDQKKLMS 240
SNVQIVRQQS YSIMCIIKEE VLAYVVQLPI YGVIDTPCWK LHTSPLCTTN IKEGSNICLT 300
RTDRGWYCDN AGSVSFFPQA DTCKVQCNRV FCDTMNSLTL PSEVSLCNTD IFNSKYDCKI 360
MTSKTDISSS VITSLGAIVS CYGKTKCTAS NKNRGIIKTF SNGCDYVSNK GVDTVSVGNT 420
LYYVNKLEGK NLYVKGEPII NYYDPLVFPS CEFCASISQV NEKINQSLAF IRRSDELLHN 480
VNTGKSTTNI MITAIIIVII VVLLSLIAIG LLLYCKAKNT PVTLSKDQLS GINNIAFSK 539

SEQ ID NO: 89
length 567
molecule type AA
division PAT

Key                 Location/Qualifiers
source              1..567
                    /mol_type=protein
                    /organism=synthetic construct

Sequence
MELLIHRSSA IFLTLAINAL YLTSSQNITE EFYQSTCSAV SRGYLGALRT GWYTSVITIE  60
LSNIKETKCN GTDTKVKLIK QELDKYKNAV TDLQLLMQNT PAGGSGGGSG SAIASGIAVC  120
KVLHLEGEVN KIKNALQLTN KAVVSLSNGV SVLTFKVLDL KNYINNQLLP IVNQQSCRIS  180
NIETVIEFQQ KNSRLLEITR EFSVNAGVTT PLSTYMLTNS ELLSLINDMP ITNDQKKLMS  240
SNVQIVRQQS YSIMCIIKEE VLAYVVQLPI YGVIDTPCWK LHTSPLCTTN IKEGSNICLT  300
RTDRGWYCDN AGSVSFFPQA DTCKVQSNRV FCDTMNSLTL PSEVSLCNTD IFNSKYDCKI  360
MTSKTDISSS VITSLGAIVS CYGKTKCTAS NKNRGIIKTF SNGCDYVSNK GVDTVSVGNT  420
LYYVNKLEGK NLYVKGEPII NYYDPLVFPS CEFCASISQV NEKINQSLAF IRRSDELLHN  480
VEDKIEEILS KIYHIENEIA RIKKLIGEAN TGKSTTNIMI TAIIIVIIVV LLSLIAIGLL  540
LYCKAKNTPV TLSKDQLSGI NNIAFSK                                    567

SEQ ID NO:          90
length              1722
molecule type       RNA
division            PAT

Key                 Location/Qualifiers
source              1..1722
                    /mol_type=other RNA
                    /organism=synthetic construct

Sequence
atggagctgc tgatccacag atccagcgcc attttcctga ccctggccat caatgccctg  60
tatctgacca gcagccagaa catcaccgag gagttttacc agagcacatg cagcgccgtg  120
agcaggggct atctgagcgc cctgaggacc ggctggtaca ctagcgtgat caccattgag  180
ctgagcaata tcaaggagac aaagtgcaac ggcaccgaca ccaaggtgaa gctgatcaag  240
caggagctgg acaagtacaa gaacgccgtg accgagctgc aactgctgat gcagaatact  300
cccgccgtga acaaccgggc caggcgggag gccccccagt acatgaatta caccatcaac  360
accaccaaga acctgaacgt gagcatttcc aagaagagga agaggagatt cctgggcttc  420
ctgctgggag tgggatctgc aatcgcctct ggcattgccg tgtctaaagt gctgcacctg  480
gagggcgagg tgaacaagat caagaacgct ctgcagctga ccaataaggc cgtcgtgagc  540
ctgagcaacg gcgtgagcgt gctgacaagc aaggtgctgg acctgaagaa ttacatcaac  600
aaccagctgc tgcccatcgt gaaccagcag agctgccgga tcagcaacat cgaaaccgtg  660
atcgagtttc agcagaagaa cagccggctg ctggagatca ccagggagtt tagcgtgaac  720
gctggcgtga ctacccctct gagcacctac atgctgacca acagcgagct gctgtccctg  780
atcaacgaca tgcccatcac caacgaccag aagaagctga tgagcagcaa cgtgcagatc  840
gtgaggcagc aaagctacag catcatgagc atcatcaagg aggaggtgct ggcctacgtg  900
gtgcagctgc ccatttacgg cgtgatcgac accccctgct ggaaactgca cacctctccc  960
ctgtgcacca caaacatcaa ggaaggcagc aacatttgcc tgacaaggac cgatagaggc  1020
tggtactgtg acaatgccgg cagcgtgagc tttttccctc aggccgatac ctgcaaggtg  1080
cagagcaatc gggtgttctg cgacaccatg aacagcctga ccctccccag cgaggtgagc  1140
ctgtgcaaca ccgacatctt caacagcaag tacgactgca agatcatgac cagcaagacc  1200
gatatcagca gcagcgtcat taccagcctg ggagccatcg tgagctgcta cggcaagacc  1260
aagtgtaccg ccagcaacaa gaaccggggc atcattaaga ccttcagcaa cggctgcgac  1320
tacgtgagca acaagggcgt ggacacagtg agcgtgggca acaccctgta ctacgtgaac  1380
aagctggaag gcaagaatct gtacgtgaag ggcgagccca tcatcaacta ctacgacccc  1440
ctggtgttcc ctagcgacga gtttgacgcc agcatcagcc aggtgaacga aaagatcaac  1500
cagagcctgg ccttcatcag gcggagcgac gagctgctcc acaacgtgaa caccggcaag  1560
agcaccacca acatcatgat caccgccatc atcatcgtga ttatcgtggt gctgctgagc  1620
ctgattgcca tcggcctgct gctgtactgt aaggccaaga acacacccgt gaccctgagc  1680
aaggatcagc tgagcggcat caataacatc gccttctcca ag                    1722

SEQ ID NO:          91
length              1722

molecule type        RNA
division             PAT

Key                  Location/Qualifiers
source               1..1722
                     /mol_type=other RNA
                     /organism=synthetic construct

Sequence
atggagctgc tgatccacag gtccagcgcc attttcctga ccctggccat caatgccctg   60
tatctgacca gcagccagaa catcaccgag gagttttacc agagcacatg cagcgccgtg   120
agcaggggct acctgagcgc cctgaggacc ggctggtaca cctccgtgat tactattgag   180
ctgtccaata tcaaggagac aaagtgcaac gggaccgaca ccaaggtgaa gctcatcaag   240
caggagctgg acaagtacaa gaacgccgtg accgagctgc agttgctgat gcagaacact   300
cccgccgtga acaaccgcgc cagacgggag gccccccagt acatgaatta caccatcaac   360
accaccaaga acctgaacgt gagcatctcc aagaagagaa agaggagatt cctgggcttt   420
ttactgggcg tgggcagcgc catcgccagt ggaattgccg tgtccaaagt gctgcacctg   480
gagggcgagg tgaacaagat caagaacgca ctgcagctga ccaataaggc cgtggtgagc   540
ctgagcaacg gcgtgagcgt gctgacaagc aaggtgctcg acctgaagaa ttacatcaac   600
aaccagctgc tgcccatcgt gaaccagcag agctgccgga tcagcaacat cgaaaccgtg   660
atcgagtttc agcagaaaaa ctctcggctc ctggagatca cccgggagtt tagcgtgaac   720
gccggggtga ccaccccact gagcacctac atgctgacca actccgagct gctgtccctg   780
atcaacgaca tgcccatcac caacgaccag aagaaactta tgagcagcaa cgtgcagatc   840
gtgcggcagc aaagctacag catcatgtcc atcatcaagg aggaggtgct ggcctacgtg   900
gtgcagctgc ccatttacgg ggtgatcgac accccctgct ggaaactgca cacctctccc   960
ctgtgcacca caaacatcaa ggaaggcagc aatatttgcc tcactaggac cgacagaggc   1020
tggtactgtg acaatgccgg cagcgtgagc ttttttccac aggccgacac ctgcaaggtg   1080
cagagcaatc gggtgttctg tgacaccatg aacagcctga ccctccccag cgaggtgagc   1140
ctctgcaaca ccgacatctt caacagcaag tacgactgca agatcatgac cagcaagacc   1200
gatatcagca gcagcgtcat taccagcctg ggcgccatcg tgagctgcta cggcaagacc   1260
aaatgtaccg ccagcaacaa gaaccggggc atcattaaga ccttcagcaa cggctgcgac   1320
tacgtgagca acaagggcgt ggacacagtg agcgtgggca acaccctgta ctacgtgaac   1380
aagctggaag gcaagaatct gtacgtgaag ggcgagccca tcattaacta ctacgacccc   1440
ctggtgttcc cttccgacga gtttgacgcc tccatcagcc aggtgaacga gaagatcaac   1500
caatccctgg ccttcatccg gcggagcgac gagctgctcc acaacgtgaa caccggcaag   1560
agcaccacca acatcatgat cacagccatc atcatcgtga ttatcgtggt gctgctgagc   1620
ctgatcgcca tcggcctgct gctgtattgt aaggccaaga acacacccgt gaccctgagc   1680
aaggatcagc tgagcggcat caataacatc gccttctcca ag                     1722

SEQ ID NO:           92
length               1701
molecule type        RNA
division             PAT

Key                  Location/Qualifiers
source               1..1701
                     /mol_type=other RNA
                     /organism=synthetic construct

Sequence
atggagctgc tgatccacag aagcagcgcc atctttctga ccctggccat caacgctctg   60
tatctgacct ctagccagaa catcaccgag gagttttacc agagcacctg cagcgccgtg   120
agcaggggct atctgggagc cctgaggacc ggctggtaca ccagcgtgat caccatcgag   180
ctgagcaata tcaaagagac aaagtgcaac ggcaccgaca ccaaggtcaa gctgatcaag   240
caggagctgg acaagtacaa gaacgctgtg accgatctgc agctcctgat gcagaatacc   300
ccttgtggag gctctggagg aggcagcggc tcagctatcg cctctggaat cgccgtgtgc   360
aaggtgctgc acctggaagg agaggtgaat aagatcaaga atgccctgca gctgaccaac   420
aaggccgtgg tgagcctgag caatggcgtg tctgtgctga ccttcaaggt gctcgacctg   480
aaaaactaca tcaacaacca gctgctgccc atcgtgaacc agcagagctg ccggatcagc   540
aacatcgaaa ccgtgatcga gtttcagcag aagaacagcc ggctgctgga gattacccgg   600
gagtttagcg tgaacgccgg cgtgacaacc cccctgagca cctacatgct gaccaatagc   660

gagctgctgt ccctgatcaa cgacatgccc atcaccaacg accagaaaaa gctgatgagc 720
agcaacgtgc agatcgtgcg gcaacagagc tacagcatca tgtgcatcat caaggaggag 780
gtgctggcct acgtggtgca gctgcctatc tacggcgtga tcgacacccc ctgctggaag 840
ctgcacacaa gccctctgtg cacaaccaac attaaggagg gctctaacat ctgcctgacc 900
agaaccgata gaggatggta ctgcgacaac gccggaagcg tgagcttttt ccctcaggcc 960
gatacctgta aggtgcagtg caatcgggtg ttctgcgaca ccatgaacag cctgaccctg 1020
cccagcgagg tgtccctgtg caacaccgac atcttcaaca gcaagtacga ctgcaagatc 1080
atgaccagca agaccgacat cagcagcagc gtgattacca gcctgggcgc tatcgtgagc 1140
tgctacggca agaccaagtg taccgccagc aataagaacc ggggcatcat taagaccttc 1200
agcaacggct gcgactacgt gagcaacaag ggcgtggaca ccgtgagcgt gggaaacacc 1260
ctgtactacg tgaataagct ggagggcaag aacctgtacg tgaagggcga gcccatcatc 1320
aactactacg accccctggt gttccccagc tgtgagtttt gcgcctctat cagccaggtg 1380
aacgagaaga tcaatcagag cctggccttc atccggagga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gcaccaccaa catcatgatc 1560
accgccatca tcatcgtgat catcgtggtg ctgctgtctc tgatcgccat cggcctgctg 1620
ctgtactgca aggccaagaa tacacccgtg accctgagca aagaccagct gagcggcatc 1680
aacaatatcg ccttctccaa g 1701

SEQ ID NO: 93
length 1701
molecule type RNA
division PAT

Key Location/Qualifiers
source 1..1701
/mol_type=other RNA
/organism=synthetic construct

Sequence
atggagctgc tgatccacag atccagcgcc atctttctga ccctggccat caatgccctg 60
tatctgacct ctagccagaa cattaccgag gagttttacc agagcacctg cagcgccgtg 120
agcaggggct atctgggagc cctgaggacc ggctggtaca ccagcgtgat caccatcgag 180
ctgagcaata tcaaagagac aaagtgcaac ggcaccgaca ccaaggtcaa gctgatcaag 240
caggagctgg acaagtacaa gaatgctgtg accgatctgc agctcctgat gcagaatacc 300
ccttgtggag gctctggagg aggcagcggc agcgctatcg cctctggaat cgccgtgtgc 360
aaggtgctgc acctggaagg agaggtgaat aagatcaaga atgccctgca gctgaccaac 420
aaggccgtgg tgagcctgag caatggcgtg tctgtgctga ccttcaaggt gctcgacctg 480
aaaaactaca tcaacaacca gctgctgccc atcgtgaacc agcagagctg ccggatcagc 540
aacatcgaaa ccgtgatcga gtttcagcag aagaacagcc ggctgctgga gattacccgg 600
gagtttagcg tgaacgccgg cgtgactacc cccctgagca cctacatgct gaccaatagc 660
gagctgctgt ccctgatcaa cgacatgccc atcaccaacg accagaaaaa gctgatgagc 720
agcaacgtgc agatcgtgcg gcaacagagc tacagcatca tgtgcatcat caaggaggag 780
gtgctggcct acgtggtgca gctgcctatc tacggcgtga tcgacacacc ctgctggaag 840
ctgcacacaa gccccctgtg tactaccaac attaaggagg gctctaacat ctgcctgacc 900
agaaccgata gaggctggta ctgcgacaac gccggaagcg tgagcttttt ccctcaggct 960
gatacctgta aggtgcagtg caatcgggtg ttctgcgaca ccatgaacag cctgaccctg 1020
cccagcgagg tgtccctgtg caacaccgac atcttcaaca gcaagtacga ctgcaagatc 1080
atgaccagca agaccgacat cagcagcagc gtgattacca gcctgggcgc catcgtgagc 1140
tgctacggca agaccaagtg taccgccagc aataagaacc ggggcatcat taagaccttc 1200
agcaacggct gcgactacgt gagcaacaag ggcgtggaca ccgtgagcgt gggaaacacc 1260
ctgtactacg tgaataagct ggagggcaag aacctgtacg tgaagggcga gcccatcatc 1320
aactactacg accccctggt gttccccagc tgtgagtttt gcgcctctat cagccaggtg 1380
aacgagaaga tcaaccagag cctggccttc attcggagga gcgacgagct gctccacaac 1440
gtggaggaca agatcgagga gatcctgagc aagatctacc acatcgagaa cgagatcgcc 1500
cgcatcaaga agctgatcgg cgaggccaac accggcaaga gcaccaccaa catcatgatc 1560
accgccatca tcatcgtgat catcgtggtg ctgctgtctc tgatcgccat cggcctgctg 1620
ctgtactgca aggccaagaa tacacccgtg accctgagca aagaccagct gagcggcatc 1680
aataatatcg ccttctccaa g 1701

SEQ ID NO:          94
length               1701
molecule type        RNA
division             PAT

Key                  Location/Qualifiers
source               1..1701
                     /mol_type=other RNA
                     /organism=synthetic construct

Sequence
atggagctgc tgattcacag aagctccgcc atctttctga ccctggccat taatgccctg  60
tacctgacct ctagccagaa catcaccgag gagttttacc agagcacctg cagcgccgtg  120
agcaggggct acctgggagc cctgaggacc ggctggtata cctccgtgat caccatcgag  180
ctgagcaata tcaaggaaac taaatgcaac ggcaccgaca ccaaggtcaa gctgatcaag  240
caggagctgg acaagtacaa gaatgcagtg accgatctgc agctcctgat gcagaatacc  300
ccctgtggag gctctggagg aggctccggc tctgctatcg cctctggaat cgccgtgtgc  360
aaggtgctgc acctggaggg agaggtgaat aagattaaga acgctctgca gctgaccaac  420
aaggctgtgg tgagcctgag caatggcgtg agtgtgctga ccttcaaggt gctcgacctg  480
aaaaactaca tcaacaacca gctgctgccc atcgtgaacc agcagagctg ccggatcagc  540
aacatcgaaa ccgtgatcga gtttcagcag aagaatagcc ggctcctgga gattacccgg  600
gagtttagcg tgaacgccgg ggtgaccacc cccctgagca cctacatgct gaccaattcc  660
gagctgctgt ccctcatcaa tgacatgccc atcaccaacg accagaaaaa gctgatgagc  720
agcaacgtgc agatcgtgcg gcagcaaagc tacagcatca tgtgcatcat caaggaggag  780
gtgctggcct acgtggtgca gctgccaatc tacggcgtga tcgacactcc ctgctggaag  840
ctgcacacca gccccctgtg cacaaccaac attaaggagg ggtctaatat ttgcctgacc  900
aggacagaca gaggctggta ctgcgacaac gccggaagcg tgagcttttt cccacaggct  960
gatacctgta aggtccagtg caatcgcgtg ttctgcgaca ccatgaacag cctgaccctg  1020
cccagcgagg tgagcctctg caacaccgac atcttcaact ccaagtacga ctgcaagatc  1080
atgaccagca agaccgacat cagcagcagc gtgattacca gcctgggcgc catcgtgagc  1140
tgttacggca agaccaagtg taccgccagc aataagaacc ggggcatcat taagaccttc  1200
agcaacggct gcgactacgt gagcaacaag ggggtggaca ccgtgagcgt gggaaacacc  1260
ctgtactacg tgaataagct ggagggcaag aacctgtacg tgaaggggga gcccatcatc  1320
aactactacg accccctggt gttccccagc tgcgagtttt gcgcttctat ttcccaggtg  1380
aacgagaaga tcaaccagag cctggccttc atccggagga gcgacgagct gctccacaac  1440
gtggaggaca agatcgagga gatcctgagc aagatctacc acatcgagaa cgagatcgcc  1500
cggatcaaga agctgatcgg cgaggccaac accggcaaga gcaccaccaa catcatgatc  1560
accgccatca tcatcgtgat catcgtggtg ttgctgagcc tgattgccat cggcctgctg  1620
ctgtactgca aggccaagaa cacacccgtg accctgagca aagaccagct gagcggcatc  1680
aacaatatcg ccttctccaa g                                            1701

SEQ ID NO:          95
length               1701
molecule type        RNA
division             PAT

Key                  Location/Qualifiers
source               1..1701
                     /mol_type=other RNA
                     /organism=synthetic construct

Sequence
atggagctgc tgatccacag aagcagcgcc atctttctga cactggccat caatgccctg  60
tacctgacca gcagccagaa catcaccgag gagttttatc agagcacctg cagcgccgtg  120
agcaggggct atctgagcgc cctgagaacc ggctggtaca cctccgtgat taccatcgag  180
ctgagcaaca tcaaggaaac caagtgcaac ggcaccgata ccaaggtgaa gctcatcaag  240
caggagctgg acaagtacaa gaacgccgtg accgacctgc aactgctgat gcagaataca  300
ccttgtggag gcagcggagg aggctctggc tctgccatcg cctctggcat tgccgtgtgt  360
aaagtgctgc atctggaggg cgaggtgaat aagatcaaga acgctctgca gctgaccaac  420
aaggccgtgg tgagcctgtc taacggcgtg agcgtgctga ccttcaaggt gctggacctg  480
aagaattaca tcaacaacca gctgctgccc atcgtgaacc agcagagctg ccggatcagc  540

aacatcgaga cagtgatcga gtttcagcag aagaacagcc ggctgctgga gatcaccagg 600
gagtttagcg tgaatgccgg cgtgaccacc cccctgagca cctacatgct gaccaatagc 660
gagctgctgt ccctgatcaa cgacatgccc atcaccaacg accagaaaaa gctgatgagc 720
agcaacgtgc agatcgtgcg gcagcaaagc tacagcatca tgtgcatcat caaggaggag 780
gtgctggcct acgtggtgca gctgcccatt tacggcgtga tcgacacccc ctgctggaag 840
ctgcatacct ctcctctgtg caccaccaat atcaaggaag gcagcaatat ctgcctgacc 900
agaacagatc ggggatggta ctgcgacaat gccggcagcg tgagcttctt tcctcaggct 960
gatacctgca aggtgcagtg taaccgggtg ttctgcgaca ccatgaacag cctgaccctg 1020
cccagcgagg tgtccctgtg caacaccgac atcttcaaca gcaagtacga ctgcaagatc 1080
atgaccagca agaccgacat cagctctagc gtgatcacca gcctgggcgc catcgtgagc 1140
tgttacggca agaccaaatg caccgccagc aacaagaacc ggggaatcat caagaccttc 1200
agcaacggct gcgactacgt gagcaacaag ggcgtggaca ccgtgtctgt gggcaatacc 1260
ctgtactacg tgaataagct ggagggcaag aacctgtacg tgaagggcga gcccatcatc 1320
aactactacg accccctggt gttccccagc tgtgagtttt gcgccagcat cagccaggtg 1380
aacgagaaga tcaaccagag cctggccttc atcaggagga gcgatgagct gctgcacaac 1440
gtggaggaca agatcgagga gatcctgagc aagatctacc acatcgagaa cgagatcgcc 1500
cggatcaaga agctgatcgg cgaggccaac accggcaaga gcaccacaaa catcatgatc 1560
accgccatca tcatcgtgat catcgtggtg ctgctgagcc tgatcgccat cggcctgctg 1620
ctgtactgca aggccaagaa cacacccgtg accctgagca aagatcagct gagcggcatc 1680
aataacatcg ccttctccaa g 1701

SEQ ID NO: 96
length 1701
molecule type RNA
division PAT

Key Location/Qualifiers
source 1..1701
/mol_type=other RNA
/organism=synthetic construct

Sequence
atggagctgc tgatccacag aagcagcgcc atctttctga cactggccat caatgccctg 60
tacctgacca gcagccagaa catcaccgag gagttttatc agagcacctg cagcgccgtg 120
agcaggggct atctgagcgc cctgaggacc ggctggtaca catccgtgat cacaatcgag 180
ctgagcaaca tcaaggaaac caagtgcaat ggcaccgaca ccaaggtgaa gctcatcaag 240
caggagctgg acaagtacaa gaacgccgtg accgacctgc aactgctgat gcagaataca 300
ccttgtggag gcagcggagg aggctctgga tctgccatcg ccagcggcat tgccgtgtgt 360
aaagtgctgc acctggaagg cgaggtgaat aagatcaaga acgctctgca gctgaccaac 420
aaggccgtgg tgagcctgtc taacggcgtg agcgtgctga ccttcaaggt gctggacctg 480
aagaattaca tcaacaacca gctgctgccc atcgtgaacc agcagagctg ccggatcagc 540
aacatcgaga cagtgatcga gtttcagcag aagaacagcc ggctgctgga gatcacaagg 600
gagtttagcg tgaacgccgg cgtgaccacc cccctgagca cctacatgct gaccaatagc 660
gagctgctgt ccctgatcaa cgacatgcct atcaccaacg accagaaaaa gctgatgagc 720
agcaacgtgc agatcgtgcg gcagcaaagc tacagcatca tgtgcatcat caaggaggag 780
gtgctggcct acgtggtgca gctgcccatt tacggcgtga tcgacacccc ctgctggaag 840
ctgcatacca gccctctgtg caccaccaat atcaaggaag gcagcaatat ctgcctgaca 900
agaacagatc ggggatggta ctgcgacaat gccggcagcg tgagcttctt tcctcaggct 960
gatacctgca aggtgcagtg taaccgggtg ttctgcgaca ccatgaacag cctgaccctg 1020
cccagcgagg tgtccctgtg caacaccgac atcttcaaca gcaagtacga ctgcaagatc 1080
atgaccagca agaccgacat cagctctagc gtgatcacca gcctgggcgc catcgtgagc 1140
tgttacggca agaccaaatg caccgccagc aacaagaacc ggggaatcat caagaccttc 1200
agcaacggct gcgactacgt gagcaacaag ggcgtggaca ccgtctctgt gggcaatacc 1260
ctgtactacg tgaataagct ggagggcaag aacctgtacg tgaagggcga gcccatcatc 1320
aactactacg accccctggt gttccccagc tgtgagtttt gcgcaagcat cagccaggtg 1380
aacgagaaga tcaaccagag cctggccttc atccggagga gcgatgagct gctgcacaac 1440
gtggaggaca agatcgagga gatcctgagc aagatctacc acatcgagaa cgagatcgcc 1500
cggatcaaga agctgatcgg cgaggccaac accggcaaga gcaccacaaa catcatgatc 1560
accgccatca tcatcgtgat catcgtggtg ctgctgagcc tgatcgccat cggcctgctg 1620
ctgtactgca aggccaagaa cacacccgtg accctgagca aagatcagct gtctggcatc 1680

aataacatcg ccttctccaa g                                          1701

SEQ ID NO:          97
length              1701
molecule type       RNA
division            PAT

Key                 Location/Qualifiers
source              1..1701
                    /mol_type=other RNA
                    /organism=synthetic construct

Sequence
atggagctgc tgatccacag aagctctgcc atctttctga cactggccat caacgccctg   60
tacctgacca gcagccagaa catcaccgaa gagttttatc agagcacctg cagcgccgtg  120
agccggggct acctgagcgc cctgcgcact gggtggtaca cttctgtgat cacaatcgag  180
ctgtccaaca tcaaggaaac caagtgcaat ggcaccgaca ccaaggtgaa gctcatcaag  240
caggagctgg acaagtacaa gaacgccgtg accgacctgc aactgctgat gcagaataca  300
ccatgtggag gcagcggagg aggctctgga tctgccatcg cctccgggat tgccgtgtgt  360
aaagtgctgc atctggaagg cgaggtgaac aagatcaaga acgctctgca gctgaccaac  420
aaggccgtgg tgagcctgtc taacggggtg agcgtgctga cattcaaggt gctggacctg  480
aagaattaca tcaacaacca gctgctgccc atcgtgaacc agcagagctg caggatcagc  540
aacatcgaaa ccgtgatcga gtttcagcag aagaacagcc ggctcctgga gatcacacgg  600
gagtttagcg tgaacgccgg ggtgaccacc cccctgagca cctacatgct gaccaattcc  660
gagctgctgt ccctgatcaa cgacatgcct atcaccaacg accagaaaaa gctgatgagc  720
agcaacgtgc agatcgtgcg gcagcaatcc tacagcatca tgtgcatcat caaggaggag  780
gtgctggcct acgtggtgca gctgcccatt tacggggtga tcgacacccc ctgctggaag  840
ctgcatacct ccccactgtg caccaccaat atcaaggaag gctccaatat ctgcctgaca  900
agaacagatc ggggatggta ctgcgacaat gccggcagcg tgagcttctt tcctcaggcc  960
gatacctgca aggtgcagtg taaccgggtg ttctgcgaca ccatgaatag cctgaccctg 1020
cccagcgagg tgtccctgtg caacaccgac atcttcaact ccaagtacga ctgcaagatc 1080
atgaccagca aaaccgatat cagctccagc gtgatcacca gcctgggcgc cattgtgagc 1140
tgctacggga agaccaaatg caccgccagc aacaagaacc ggggcatcat taagaccttc 1200
agcaacggct gcgactacgt gagcaacaag ggcgtggaca ccgtgtctgt gggcaatacc 1260
ctgtactacg tgaataagct ggagggcaag aacctgtacg tgaagggcga gcccatcatc 1320
aactactacg accccctggt gttccccagc tgtgagtttt gcgccagcat cagccaggtg 1380
aacgagaaga tcaaccaatc cctggccttc atccggaggt ccgacgagct gcttcacaac 1440
gtggaggaca agatcgagga gatcctgagc aagatctacc acatcgagaa cgagatcgcc 1500
cggatcaaga agctgatcgg cgaggccaac accggcaaga gcaccacgaa catcatgatc 1560
accgccatca tcatcgtgat catcgtggtg ttgctgagcc tgattgccat cggcctgctg 1620
ctgtactgca aggccaagaa cacacccgtg accctgagca aagaccagct gagcggcatc 1680
aataacatcg ccttctccaa g                                          1701

SEQ ID NO:          98
length              1701
molecule type       RNA
division            PAT

Key                 Location/Qualifiers
source              1..1701
                    /mol_type=other RNA
                    /organism=synthetic construct

Sequence
atggagctgc tgatccacag aagcagcgcc attttcctga cactggccat caatgccctg   60
tacctgacct ctagccagaa catcaccgag gagttttacc agagcacctg tagcgccgtg  120
tctaggggct atctgggcgc tctgcggacc ggctggtaca cctccgtgat caccatcgag  180
ctgagcaata tcaaggaaac caagtgcaac ggcaccgaca ccaaggtgaa gctgattaag  240
caggagctgg acaagtacaa gaacgccgtg accgagctgc agttgctgat gcagaataca  300
ccttgtggcg gatctggagg aggctctggc tctgctatcg ccagcggaat cgccgtgtgt  360

aaagtgctgc acctggaggg cgaggtgaat aagattaaga acgccctgca gctgacaaac 420
aaggccgtgg tgagcctgag caatggcgtg agcgtgctga ccttcaaggt gctggacctg 480
aagaattaca tcaacaacca gctgctgccc atcgtgaacc agcagagctg ccggatcagc 540
aacatcgaaa ccgtgatcga gtttcagcag aagaacagcc ggctgctgga gatcaccagg 600
gagtttagcg tgaacgccgg cgtgaccacc cctctgagca cctacatgct gaccaacagc 660
gagctgctgt ccctgatcaa cgatatgccc atcaccaacg accagaaaaa gctgatgagc 720
agcaacgtgc agatcgtgcg gcaacagagc tacagcatca tgtgcatcat caaggaggag 780
gtgctggcct acgtggtgca gctgcccatt tacggcgtga tcgacacccc ctgctggaag 840
ctgcacacaa gccctctgtg taccaccaac atcaaggaag gcagcaatat ctgtctgaca 900
cggacagatc ggggatggta ctgcgataat gccggcagcg tgagcttctt tcctcaggcc 960
gatacctgca aggtgcagtg caatagggtg ttctgcgaca ccatgaacag cctgaccctg 1020
cccagcgagg tgagcctctg caacaccgac atcttcaaca gcaagtacga ctgcaagatc 1080
atgaccagca agaccgacat cagcagcagc gtgatcacat ctctgggcgc catcgtgagc 1140
tgctacggca agaccaagtg taccgccagc aacaagaacc ggggcatcat taagaccttc 1200
agcaacggct gcgactacgt gagcaacaag ggcgtggaca cagtgagcgt gggaaacacc 1260
ctgtactacg tgaataagct ggagggcaag aacctgtacg tgaagggcga gcccatcatc 1320
aactactacg accccctggt gttccccagc tgcgaatttt gcgccagcat cagccaggtg 1380
aacgagaaga ttaaccagag cctggccttc atcagacgga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gcaccacaaa catcatgatc 1560
accgccatca tcatcgtgat catcgtggtg ctgctgagcc tgatcgccat cggcctgctg 1620
ctgtactgca aggccaagaa cacccctgtg accctgtcta aagaccagct gagcggcatc 1680
aacaatatcg ccttctccaa g 1701

SEQ ID NO: 99
length 1701
molecule type RNA
division PAT

Key Location/Qualifiers
source 1..1701
/mol_type=other RNA
/organism=synthetic construct

Sequence
atggagctgc tgatccacag aagctctgcc attttcctga cactggccat caatgccctg 60
tacctgacct ctagccagaa catcaccgag gagttttacc agagcacctg tagcgccgtg 120
tctcggggct atctgggcgc tctgcggacc ggctggtata cctccgtgat caccatcgag 180
ctgtccaata tcaaggaaac caagtgcaac ggcaccgaca ccaaggtgaa gctgattaag 240
caggagctgg acaagtacaa gaacgccgtg accgagctgc agttgctgat gcagaataca 300
ccctgtggcg gatcaggagg aggctctggc tctgctatcg ctagcggcat cgccgtgtgt 360
aaggtgctgc atctggaggg cgaggtgaat aagattaaga acgccctgca gctgacaaac 420
aaggccgtgg tgagcctgag caatggcgtg agcgtgctga ccttcaaggt gctggacctg 480
aagaattaca tcaacaacca gctgctgccc atcgtgaacc agcagagctg ccggatcagc 540
aacatcgaaa ccgtgatcga gtttcagcag aagaacagcc ggctgctgga gatcaccagg 600
gagtttagcg tgaacgccgg cgtgacaacc cccctgagca cctacatgct gaccaacagc 660
gagctgctgt ccctgatcaa cgatatgccc atcaccaacg accagaagaa actgatgagc 720
agcaacgtgc agatcgtgcg gcaacagagc tacagcatca tgtgcatcat caaggaggag 780
gtgctggcct acgtggtgca gctgcccatt tacggcgtga tcgacacccc ctgctggaag 840
ctgcacacaa gccctctgtg taccaccaac atcaaggaag gcagcaatat ctgtctgaca 900
cggacagatc ggggatggta ctgcgataat gccggcagcg tgagcttctt tcctcaggcc 960
gatacctgca aggtgcagtg caatagggtg ttctgcgaca ccatgaacag cctgaccctg 1020
cccagcgagg tgagcctctg caacaccgac atcttcaaca gcaagtacga ctgcaagatc 1080
atgaccagca agaccgacat cagcagcagc gtgatcacat ctctgggcgc catcgtgagc 1140
tgctacggca agaccaagtg taccgccagc aacaagaacc ggggcatcat taagaccttc 1200
agcaacggct gcgactacgt gagcaacaag ggcgtggaca cagtgagcgt gggaaacacc 1260
ctgtactacg tgaataagct ggagggcaag aacctgtacg tgaagggcga gcccatcatc 1320
aactactacg accccctggt gttccccagc tgcgaatttt gcgccagcat cagccaggtg 1380
aacgagaaga ttaaccagag cctggccttc atcaggcgga gcgacgagct gctccacaac 1440
gtggaggaca agatcgagga gatcctgagc aagatctacc acatcgagaa cgagatcgcc 1500

cggatcaaga agctgatcgg cgaggccaac accggcaaga gtaccacaaa catcatgatc 1560
accgccatca tcatcgtgat catcgtggtg ctgctgagcc tgatcgccat cggcctgctg 1620
ctgtactgca aggccaagaa cacccctgtg accctgagca aagaccagct gagcggcatc 1680
aacaatatcg ccttctccaa g 1701

SEQ ID NO: 100
length 1701
molecule type RNA
division PAT

Key Location/Qualifiers
source 1..1701
/mol_type=other RNA
/organism=synthetic construct

Sequence
atggagctgc tgatccacag aagctctgcc atcttcctga cactggccat caatgccctg 60
tacctgacca gtagccagaa catcaccgag gagttttacc agagcacctg cagcgccgtg 120
tctcgcggct atctgggcgc tctgcgcacc ggctggtaca cctctgtgat caccatcgag 180
ctgtctaata tcaaggaaac caagtgcaac ggcaccgaca ccaaggtgaa gctgattaag 240
caggagctgg acaagtacaa gaatgccgtg accgagctgc agttgctgat gcagaatact 300
ccctgtggcg gaagcggagg aggctctgga agcgcaattg ctagcggcat cgccgtgtgt 360
aaggtgctgc atctggaggg cgaggtgaat aagattaaga acgccctgca gctgacaaac 420
aaggccgtgg tgagcctgtc caacggcgtg agcgtgctga ccttcaaggt gctggacctg 480
aagaattaca tcaacaacca gctgctgccc atcgtgaacc agcagagctg ccggatctcc 540
aacatcgaaa ccgtgatcga atttcagcag aagaacagcc ggctgctgga gatcacacgg 600
gagtttagcg tgaacgccgg cgtgacaacc cccctgtcca cctacatgct gaccaactcc 660
gagctgctgt ccctgatcaa cgatatgccc atcaccaacg accagaagaa actgatgagc 720
agcaacgtgc agatcgtgcg gcagcaaagc tacagcatca tgtgcatcat caaggaggag 780
gtgctggcct acgtggtgca gctgcccatt tacggcgtga tcgacacccc ctgctggaag 840
ctgcacacat ctcctctgtg caccaccaac atcaaagaag gcagcaatat ctgtctgact 900
cgcacagatc ggggctggta ctgcgacaat gccggcagtg tgtcattttt ccctcaggcc 960
gatacctgca aggtgcagtg caaccgggtg ttttgcgaca ccatgaacag cctgaccctg 1020
cccagcgagg tgagcctctg caacaccgac atcttcaact ccaagtacga ctgcaagatc 1080
atgaccagca agaccgacat cagcagcagc gtgatcacaa gcctgggcgc tatcgtgtcc 1140
tgctacggca agaccaagtg taccgccagc aataagaacc ggggcatcat taagaccttc 1200
agcaacggct gcgactacgt gagcaacaag ggcgtggata ccgtgagcgt cggcaacacc 1260
ctgtattacg tgaacaagct ggagggcaag aacctgtacg tgaagggcga gcccatcatc 1320
aactactacg accccctggt gttccccagc tgcgagtttt gtgccagcat cagccaggtg 1380
aatgagaaga tcaaccaatc cctggccttc atcaggcgga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gtaccacgaa catcatgatc 1560
accgccatca tcatcgtgat catcgtggtg ctgctgagcc tgatcgccat cggcctgctg 1620
ctgtactgca aggccaagaa taccccagtg accctgagca aagaccagct gagcggcatc 1680
aacaatatcg ccttctccaa g 1701

SEQ ID NO: 101
length 1701
molecule type RNA
division PAT

Key Location/Qualifiers
source 1..1701
/mol_type=other RNA
/organism=synthetic construct

Sequence
atggagctgc tgatccacag aagctccgcc atctttctga cactggccat caatgccctg 60
tacctgacct ctagccagaa catcaccgag gagttttatc agagcacctg cagcgctgtg 120
agcaggggct acctgggcgc cctgaggacc ggctggtaca cctccgtgat caccatcgag 180

ctgagcaata ttaaggaaac caagtgcaat ggcaccgaca ccaaggtcaa gctgatcaag 240
caggagctgg acaagtacaa gaacgccgtg actgatctgc agctcctgat gcagaataca 300
ccttgtggcg gctctggagg aggctctggc agcgccattg cctctggcat cgctgtgtgt 360
aaggtgctgc acctggaggg cgaggtgaat aaaatcaaga atgccctgca gctgaccaac 420
aaagccgtgg tgagcctgtc taacggcgtg agcgtgctga caagcaaggt gctggacctg 480
aaaaactaca tcaacaacca gctgctgccc atcgtgaacc agcagagctg ccggatctcc 540
aacatcgaga cagtgatcga gtttcagcag aagaacagcc gcctgctgga gatcaccaga 600
gagtttagcg tgaacgccgg cgtgaccacc cccctgagca cctacatgct gaccaatagc 660
gagctgctgt ccctgatcaa cgacatgccc atcaccaacg accagaagaa actgatgagc 720
agcaacgtgc agatcgtgcg gcagcagagt tacagcatca tgtgcatcat caaggaggag 780
gtgctggcct acgtggtgca gctgcccatt tacggcgtga tcgacacccc ctgctggaag 840
ctgcacacaa gccctctgtg taccaccaat atcaaggaag gcagcaacat ctgtctgaca 900
aggaccgata gaggctggta ctgcgataac gccggcagcg tgagcttctt tcctcaggcc 960
gacacatgca aggtgcagtg taatcgggtg ttctgcgaca ccatgaacag cctgaccctg 1020
cccagcgagg tgtccctgtg caacaccgac atcttcaaca gcaagtacga ctgcaagatc 1080
atgaccagca agaccgacat cagcagcagc gtgattacct ctctgggcgc catcgtgagc 1140
tgctacggca agaccaagtg taccgccagc aacaagaacc ggggcattat caagaccttc 1200
agcaacggct gcgactacgt gagcaacaag ggcgtggaca cagtgagcgt gggcaatacc 1260
ctgtactacg tgaataagct ggagggcaag aacctgtacg tgaagggcga gcccatcatc 1320
aactactacg accccctggt gttccccagc tgtgagtttt gcgctagcat cagccaggtg 1380
aacgagaaga tcaaccagag cctggccttc attcggcgga gcgatgagct gctgcacaac 1440
gtggaggaca agatcgagga gatcctgagc aagatctacc acatcgagaa cgagatcgcc 1500
cggatcaaga agctgatcgg cgaggccaat accggcaaga gcaccaccaa catcatgatc 1560
accgccatca tcatcgtgat catcgtggtg ctgctgagcc tgatcgccat cggcctgctg 1620
ctgtactgca aggccaagaa cacccctgtg accctgtcta aagaccagct gagcggcatc 1680
aataacatcg ccttctccaa g 1701

SEQ ID NO: 102
length 1701
molecule type RNA
division PAT

Key Location/Qualifiers
source 1..1701
/mol_type=other RNA
/organism=synthetic construct

Sequence
atggagctgc tgatccacag aagctctgcc atctttctga cactggccat caatgccctg 60
tacctgacct ctagccagaa catcaccgag gagttttacc agagcacctg cagcgctgtg 120
agcaggggct acctgggcgc cctgaggacc ggctggtaca ccagcgtgat caccatcgag 180
ctgagcaata ttaaggaaac caagtgcaat ggcacagaca ccaaggtcaa gctgatcaag 240
caggagctgg acaagtacaa gaacgccgtg accgatctgc agctcctgat gcagaataca 300
ccttgtggag gaagcggagg aggcagcggc agcgccattg ccagcggcat cgctgtgtgt 360
aaggtgctgc acctggaagg cgaggtgaat aaaatcaaga atgccctgca gctgaccaac 420
aaagccgtgg tgagcctgtc taacggcgtg agcgtgctga caagcaaggt gctggacctg 480
aaaaactaca tcaacaacca gctgctgccc atcgtgaacc agcagagctg ccggatctcc 540
aacatcgaga cagtgatcga gtttcagcag aagaacagca ggctgctgga gatcaccagg 600
gagtttagcg tgaacgccgg cgtgaccacc cccctgagca cctacatgct gaccaatagc 660
gagctgctgt ccctgatcaa cgacatgccc atcaccaacg accagaagaa actgatgagc 720
agcaacgtgc agatcgtgcg gcagcaaagc tacagcatca tgtgcatcat caaggaggag 780
gtgctggcct acgtggtgca gctgcccatt tacggcgtga tcgacacccc ctgctggaag 840
ctgcacacaa gccctctgtg taccaccaat atcaaggaag gcagcaacat ctgtctgaca 900
aggaccgata gaggctggta ctgcgataac gccggcagcg tgagcttctt tcctcaggcc 960
gacacatgca aggtgcagtg taatcgggtg ttctgcgaca ccatgaacag cctgaccctg 1020
cccagcgagg tgtccctgtg caacaccgac atcttcaaca gcaagtacga ctgcaagatc 1080
atgaccagca agaccgacat cagcagcagc gtgattacct ctctgggagc catcgtgagc 1140
tgctacggca agaccaagtg taccgccagc aacaagaacc ggggcattat caagaccttc 1200
agcaacggct gcgactacgt gagcaacaag ggcgtggaca ccgtgagcgt cggcaataca 1260
ctgtactacg tgaacaagct ggagggcaag aacctgtacg tgaagggcga gccaatcatc 1320

aactactacg accccctggt gttccccagc tgcgaatttt gcgctagcat cagccaggtg 1380
aacgagaaga tcaaccagag cctggccttc attcggcgga gcgatgagct gctgcacaac 1440
gtggaggaca agatcgagga gatcctgagc aagatctacc acatcgagaa cgagatcgcc 1500
cggatcaaga agctgatcgg cgaggccaat accggcaaga gcaccaccaa catcatgatc 1560
accgccatca tcatcgtgat catcgtggtg ctgctgagcc tgatcgccat cggcctgctg 1620
ctgtactgca aggccaagaa cacacctgtg accctgagca aagaccagct gagcggaatc 1680
aataacatcg ccttctccaa g 1701

SEQ ID NO: 103
length 1701
molecule type RNA
division PAT

Key Location/Qualifiers
source 1..1701
/mol_type=other RNA
/organism=synthetic construct

Sequence
atggagctgc tgatccatag aagctctgcc atctttctga cactggccat caatgccctg 60
tacctgacca gcagccagaa catcaccgag gagttttacc agagcacctg cagcgctgtg 120
tcccggggct acctgggcgc cctgaggacc ggctggtaca caagcgtgat caccatcgag 180
ctgagcaata ttaaggaaac caagtgcaat ggcacagaca ccaaggtcaa gctgatcaag 240
caggagctgg acaagtacaa gaacgccgtg accgatctgc agctcctgat gcagaataca 300
ccttgtggag gaagcggagg aggctccggc agcgccattg ccagcgggat cgcagtgtgt 360
aaggtgctgc atctggaggg cgaggtgaat aaaatcaaga atgccctgca gctgaccaac 420
aaagccgtgg tgagcctgtc taacggcgtg agcgtgctga ctagcaaggt gctggacctg 480
aaaaactaca tcaacaacca gctgctgccc atcgtcaacc agcagagctg ccggatctcc 540
aacatcgaga cagtgatcga gtttcagcag aagaacagca ggctgctgga gatcaccagg 600
gagtttagcg tgaacgccgg ggtgaccacc cccctgtcca cctacatgct gaccaatagc 660
gagctgctgt cgctgatcaa tgacatgccc atcaccaacg accagaagaa actgatgagc 720
agcaacgtgc agatcgtgcg gcagcaaagc tactccatca tgtgcatcat caaggaggag 780
gtgctggcct acgtggtgca gctgcccatt tacggcgtga tcgacacccc ctgttggaag 840
ctgcacacaa gccctctgtg caccaccaac atcaaggaag gcagcaacat ctgtctgaca 900
cgcaccgata gaggatggta ctgcgataat gccgggtccg tgagcttttt tcctcaggcc 960
gacacctgca aggtgcagtg caatcgggtg ttttgcgaca ccatgaacag cctgaccctg 1020
cccagcgagg tgtccctgtg caacaccgac atcttcaaca gcaagtacga ctgcaagatc 1080
atgacctcca agaccgacat cagcagcagc gtgattacca gcctgggagc catcgtgagc 1140
tgctacggga agaccaagtg taccgccagc aacaagaacc gggggatcat taagaccttc 1200
agcaacggct gcgactacgt gagcaacaag ggcgtggata ccgtgtccgt cggcaataca 1260
ctgtactacg tgaacaagct ggagggcaag aacctgtacg tgaagggcga gcccatcatc 1320
aactactacg accccctggt gttccccagc tgcgaatttt gcgccagcat tagccaggtg 1380
aacgagaaga tcaaccaatc cctggccttc attcggcgga gcgacgagct gctccacaac 1440
gtggaggaca agatcgagga gatcctgtcc aagatctacc acatcgagaa cgagatcgcc 1500
cggatcaaga agctgatcgg cgaggccaac accggcaaga gcaccacgaa catcatgatc 1560
accgccatca tcatcgtgat catcgtggtg ttgctgagcc tgattgccat cggcctgctg 1620
ctgtactgca aggccaagaa cacacccgtg accctgagca aagaccagct gagcggcatc 1680
aacaatatcg ccttctccaa g 1701

SEQ ID NO: 104
length 1701
molecule type RNA
division PAT

Key Location/Qualifiers
source 1..1701
/mol_type=other RNA
/organism=synthetic construct

Sequence

atggagctgc tgatccacag aagcagcgcc atctttctga ccctggctat taatgccctg 60
tacctgacct ctagccagaa tatcaccgag gagttttacc agagcacctg tagcgccgtg 120
agcaggggct atctgggcgc cctgcggacc ggatggtaca ccagcgtgat caccatcgag 180
ctgagcaaca tcaaggaaac aaagtgcaac ggcaccgaca ccaaggtgaa gctcatcaag 240
caggagctgg acaagtacaa gaacgccgtg acagatctgc agcttctgat gcagaataca 300
ccatgtggcg gatcaggagg cggctctggc tctgctatcg ccagcggcat tgccgtgtcc 360
aaagtgctgc acctggaggg cgaagtgaat aagatcaaga atgccctgca gctgaccaac 420
aaggccgtgg tgagcctgtc taacggcgtg agcgtgctga cattcaaggt gctggacctg 480
aagaattaca tcaacaacca gctgctgccc atcgtgaacc agcagagctg ccggatcagc 540
aacatcgaaa ccgtgatcga gtttcagcag aagaacagcc ggctgctgga gatcacacgg 600
gagtttagcg tgaacgccgg cgtgaccaca cccctgagca cctacatgct gaccaatagc 660
gagctgctgt ccctgatcaa tgacatgccc atcaccaacg accagaagaa gctcatgagc 720
agcaacgtgc agatcgtgcg gcaacagagc tacagcatca tgagcatcat caaggaggag 780
gtgctggcct acgtggtgca gctgcccatt tacggcgtga tcgacacccc ctgctggaag 840
ctgcatacca gccctctgtg tacaaccaac atcaaagagg gcagcaatat ctgtctgacc 900
aggaccgata ggggctggta ctgtgacaat gccggcagcg tgagcttttt ccctcaggcc 960
gatacctgta aggtgcagtg caatcgggtg ttctgcgaca ccatgaacag cctgaccctg 1020
cccagcgagg tgagcctctg caacaccgac atcttcaaca gcaagtacga ctgcaagatc 1080
atgaccagca agaccgatat cagcagcagc gtgattacca gcctgggcgc cattgtgagc 1140
tgctacggca agaccaagtg taccgccagc aacaagaacc ggggcattat caagaccttc 1200
agcaacggct gcgactacgt gagcaacaag ggcgtggaca ccgtgtcagt gggcaacacc 1260
ctgtactacg tgaataagct ggagggcaag aacctgtacg tgaagggcga gcccatcatc 1320
aactactacg accctctggt gttccccagc tgcgagtttt gcgccagcat cagccaggtg 1380
aatgagaaga tcaaccagag cctggccttc atcagacgga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gcaccacaaa catcatgatc 1560
accgccatca tcatcgtgat catcgtggtg ctgctgagcc tgatcgccat cggcctgctg 1620
ctgtactgca aggccaagaa cacccctgtg acactgagca aggatcagct gtctggcatc 1680
aacaatatcg ccttctccaa g 1701

SEQ ID NO: 105
length 1701
molecule type RNA
division PAT

Key Location/Qualifiers
source 1..1701
/mol_type=other RNA
/organism=synthetic construct

Sequence
atggagctgc tgatccacag aagcagcgcc atttttctga ccctggctat taatgccctg 60
tacctgacct ctagccagaa catcaccgag gagttttacc agagcacctg tagcgccgtg 120
agcaggggct atctgggcgc cctgcggacc ggatggtaca ccagcgtgat caccatcgag 180
ctgagcaaca tcaaggaaac aaagtgcaac ggcaccgaca ccaaggtgaa gctcatcaag 240
caggagctgg acaagtacaa gaatgccgtg acagatctgc agcttctgat gcagaataca 300
ccatgtggcg gaagcggagg cggctctggc tctgctatcg ccagcggcat cgccgtgtcc 360
aaagtgctgc acctggaagg cgaggtgaat aagattaaga atgccctgca gctgaccaac 420
aaggccgtgg tgagcctgtc taacggcgtg agcgtgctga cattcaaggt gctggacctg 480
aagaattaca tcaacaacca gctgctgccc atcgtgaacc agcagagctg ccggatcagc 540
aacatcgaaa ccgtgatcga gtttcagcag aagaacagcc ggctgctgga gatcacacgg 600
gagtttagcg tgaacgccgg cgtgaccaca cccctgagca cctacatgct gaccaatagc 660
gagctgctgt ccctgatcaa tgacatgccc atcaccaacg accagaagaa gctcatgagc 720
agcaacgtgc agatcgtgcg gcaacagagc tacagcatca tgtccatcat caaggaggag 780
gtgctggcct acgtggtgca gctgcccatt tacggcgtga tcgacacccc ctgctggaag 840
ctgcatacca gccctctgtg tacaaccaac atcaaagagg gcagcaatat ctgtctgacc 900
aggaccgata ggggctggta ctgtgacaat gccggcagcg tgagcttttt ccctcaggcc 960
gatacctgta aggtgcagtg caatcgggtg ttctgcgaca ccatgaacag cctgaccctg 1020
cccagcgagg tgagcctctg caacaccgac atcttcaaca gcaagtacga ctgcaagatc 1080
atgaccagca agaccgacat cagcagcagc gtgattacca gcctgggcgc cattgtgagc 1140

tgctatggca agaccaagtg taccgccagc aacaagaacc ggggcattat caagaccttc 1200
agcaacggct gcgactacgt gagcaacaag ggcgtggaca ccgtgtccgt gggcaatacc 1260
ctgtactacg tgaacaagct ggagggcaag aacctgtacg tgaagggcga gcccatcatt 1320
aactactacg accctctggt gttccccagc tgcgagtttt gcgccagcat cagccaggtg 1380
aatgagaaga tcaaccagag cctggccttc atcagacgga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gcaccacaaa catcatgatc 1560
accgccatca tcatcgtgat catcgtggtg ctgctgagcc tgatcgccat cggcctgctg 1620
ctgtactgca aggccaagaa cacccctgtg acactgagca aagaccagct gagcggcatc 1680
aacaatatcg ccttctccaa g 1701

SEQ ID NO: 106
length 1701
molecule type RNA
division PAT

Key Location/Qualifiers
source 1..1701
/mol_type=other RNA
/organism=synthetic construct

Sequence
atggagctgc tgattcacag gagcagcgcc atttttctga ccctggctat taatgccctg 60
tacctgacct cttcccagaa catcaccgag gagttttacc agagcacctg tagcgccgtg 120
agcaggggct atctgggcgc cctgcgcacc ggttggtaca ccagcgtgat caccatcgag 180
ctgagcaaca tcaaggaaac aaagtgcaac ggcaccgaca ccaaggtgaa gctcatcaag 240
caggagctgg acaagtacaa gaatgccgtg actgatctgc agcttctgat gcagaataca 300
ccatgtggcg gaagcggagg cggcagtggc tctgctatcg ccagcggcat cgccgtgtcc 360
aaagtgctgc acctggaagg cgaggtgaat aagattaaga atgccctgca gctgaccaac 420
aaggccgtgg tgtccctgtc taacggcgtg agcgtgctga cattcaaggt gctcgacctg 480
aagaattaca tcaacaacca gctgctgccc atcgtgaacc agcagagctg ccggatcagc 540
aacatcgaaa ccgtgatcga gtttcagcag aagaacagcc ggctcctgga gatcacacgg 600
gagtttagcg tgaacgccgg ggtgaccact cccctgagca cctacatgct gaccaatagc 660
gagctgctgt ccctgatcaa tgacatgccc atcaccaacg accagaagaa gctcatgagc 720
agcaacgtgc agatcgtgcg ccaacagagc tacagcatca tgtccatcat caaggaggag 780
gtgctggcct acgtggtgca gctgcccatt tacggcgtga tcgacacccc ttgctggaag 840
ctgcacacct cccctctgtg tactaccaac atcaaagagg gaagcaatat ctgcttgaca 900
cgcaccgata ggggctggta ctgtgacaac gccggcagcg tgtctttttt cccacaggcc 960
gatacatgca aggtgcagtg caaccgggtg ttctgtgaca ccatgaacag cctgaccctg 1020
cccagcgagg tgagcctctg caacaccgac atcttcaact ccaagtacga ctgcaagatc 1080
atgaccagca agaccgatat cagcagcagc gtgattacca gcctgggcgc catcgtgagc 1140
tgctatggca agaccaagtg taccgccagc aacaagaacc ggggcatcat caaaaccttc 1200
agcaacggct gcgactacgt gagcaacaag ggggtggaca ccgtgtccgt gggcaatacc 1260
ctgtactacg tgaacaagct ggagggcaag aatctgtacg tgaagggcga gcccatcatt 1320
aactactacg accctctggt gttccccagc tgcgagtttt gcgccagcat cagccaggtg 1380
aatgagaaga ttaaccagag cctggccttc atccggcgga gcgacgagct gctccacaac 1440
gtggaggaca agatcgagga gatcctgagc aagatctacc acatcgagaa cgagatcgcc 1500
cggatcaaga agctgatcgg cgaggccaac accggaaaga gcaccacgaa catcatgatc 1560
accgccatca tcatcgtgat catcgtggtg ttgctgagcc tgatcgccat cggcctgctg 1620
ctgtactgca aggccaagaa cacccctgtg accctgagca aagaccagct gagcggcatc 1680
aacaatatcg ccttctccaa g 1701

SEQ ID NO: 107
length 1701
molecule type RNA
division PAT

Key Location/Qualifiers
source 1..1701
/mol_type=other RNA

/organism=synthetic construct

Sequence
atggagctgc tgatccacag gagctctgcc atctttctga cactggccat caatgccctg 60
tacctgacca gctctcagaa tatcaccgag gagttttacc agagcacctg cagcgccgtg 120
agcaggggat atctgggcgc cctgagaacc ggctggtaca ccagcgtgat caccatcgag 180
ctgagcaaca tcaaggaaac caagtgcaac ggcaccgaca ccaaggtgaa gctgattaag 240
caggagctgg acaagtacaa gaatgccgtg acagacctgc aactgctgat gcagaatacc 300
ccttgcggcg gatctggagg aggctctggc agcgccattg ctagcggaat cgccgtgtgt 360
aaagtgctgc acctggaagg cgaggtgaat aagatcaaga acgccctgca gctgaccaac 420
aaggccgtgg tgtccctgtc taatggcgtg agcgtgctga ccttcaaggt gctggacctg 480
aagaattaca tcaacaacca gctgctgccc atcgtgaacc agcagagctg ccggatcagc 540
aacatcgaaa ccgtgatcga gtttcagcag aagaacagca ggctgctgga gatcacccgg 600
gagtttagcg tgaacgccgg cgtgaccacc cccctgagca cctacatgct gaccaatagc 660
gagctgctgt ccctgatcaa tgacatgccc atcaccaacg accagaagaa actgatgagc 720
agcaacgtgc agatcgtgcg gcagcaaagc tacagcatca tgtgcatcat caaggaggag 780
gtgctggcct acgtggtgca gctgcccatt tacggcgtga tcgacacccc ctgctggaag 840
ctgcacacct ctcctctgtg cacaaccaat atcaaggaag gcagcaatat ctgcctgaca 900
cggacagata gaggctggta ctgcgacaat gccggcagcg tgagcttctt tcctcaggcc 960
gatacctgca aggtgcagtg caacagagtg ttctgcgaca ccatgaacag cctgaccctg 1020
cccagcgagg tgagcctgtg caacaccgac atcttcaaca gcaagtacga ctgcaagatc 1080
atgaccagca agaccgacat tagcagcagc gtcatcacca gcctgggcgc tatcgtgagc 1140
tgttacggca agaccaagtg taccgccagc aacaagaacc gggggatcat caagaccttc 1200
agcaacggct gcgactacgt gagcaacaag ggcgtggaca ccgtgagcgt cggcaataca 1260
ctgtactacg tgaataagct ggagggcaag aacctgtacg tgaagggcga gcccatcatc 1320
aactactacg accccctggt gttccctagc gacgagtttg acgcctccat tagccaggtg 1380
aacgagaaga tcaaccagag cctggccttc atccggagga gcgatgagct gctccacaac 1440
gtggaggaca agatcgagga gatcctgagc aagatctacc acatcgagaa cgagatcgcc 1500
cggatcaaga agctgatcgg cgaggccaat accggcaaga gcaccaccaa catcatgatc 1560
accgccatca tcatcgtgat catcgtggtg ctgctgagcc tgatcgccat cggcctgctg 1620
ctgtactgca aggccaagaa tacacccgtg accctgagca aagaccagct gagcggcatc 1680
aacaatatcg ccttctccaa g 1701

SEQ ID NO: 108
length 1701
molecule type RNA
division PAT

Key Location/Qualifiers
source 1..1701
/mol_type=other RNA
/organism=synthetic construct

Sequence
atggagctgc tgatccacag gagctctgcc atctttctga cactggccat caatgccctg 60
tacctgacca gctctcagaa cattaccgag gagttttacc agagcacatg cagcgccgtg 120
agccggggat atctgggcgc cctgagaacc ggctggtaca ccagcgtgat caccatcgag 180
ctgagcaaca tcaaggaaac caagtgcaac ggcaccgaca ccaaggtgaa gctgattaag 240
caggagctgg acaagtacaa gaatgccgtg acagacctgc aactgctgat gcagaatacc 300
ccttgcggcg gatctggagg aggctctggc agcgccattg ctagcggaat cgccgtgtgt 360
aaagtgctgc acctggaagg cgaggtgaat aagatcaaga acgccctgca gctgaccaac 420
aaggccgtgg tgtccctgtc aaatggcgtg agcgtgctga ccttcaaggt gctggacctg 480
aagaattaca tcaacaacca gctgctgccc atcgtgaacc agcagagctg ccgcatcagc 540
aacatcgaaa ccgtgatcga gtttcagcag aagaacagca ggctgctgga gatcacacgg 600
gagtttagcg tgaacgccgg cgtgaccacc cccctgagca cctacatgct gaccaatagc 660
gagctgctgt ccctgatcaa cgacatgcct atcaccaacg accagaagaa actgatgagc 720
agcaacgtgc agatcgtgcg gcagcaaagc tacagcatca tgtgcatcat caaggaggag 780
gtgctggcct acgtggtgca gctgcccatt tacggcgtga tcgacacccc ctgctggaag 840
ctgcacacct ctcctctgtg cacaaccaat atcaaggaag gcagcaatat ctgcctgaca 900
cggacagata gaggctggta ctgcgacaat gccggcagcg tgagcttctt tccacaggcc 960

gatacctgca aggtgcagtg caacagagtg ttctgcgaca ccatgaacag cctgaccctg 1020
cccagcgagg tgagcctgtg caacaccgac atcttcaaca gcaagtacga ctgcaagatc 1080
atgaccagca agaccgacat tagcagcagc gtcatcacca gcctgggcgc tatcgtgagc 1140
tgctacggca agaccaagtg taccgccagc aataagaacc gggggatcat caagaccttc 1200
agcaacggct gcgactacgt gagcaacaag ggcgtggaca ccgtgagcgt cggcaataca 1260
ctgtactacg tgaataagct ggagggcaag aacctgtacg tgaagggcga gcccatcatc 1320
aactactacg accccctggt gttccctagc gacgagtttg acgccagcat cagccaggtg 1380
aacgagaaga tcaatcagag cctggccttc atccggagga gcgacgagct gctccacaat 1440
gtggaggaca agatcgagga gatcctgagc aagatctacc acatcgagaa cgagatcgcc 1500
cggatcaaga agctgatcgg cgaggccaat accggcaaga gcaccaccaa catcatgatc 1560
accgccatca tcatcgtgat catcgtggtg ctgctgagcc tgatcgccat cggcctgctg 1620
ctgtactgca aggccaagaa tacacccgtg accctgagca aagaccagct gagcggcatc 1680
aacaatatcg ccttctccaa g 1701

SEQ ID NO: 109
length 1701
molecule type RNA
division PAT

Key Location/Qualifiers
source 1..1701
/mol_type=other RNA
/organism=synthetic construct

Sequence
atggagctgc tgatccacag aagctctgcc atctttctga cactggccat caatgccctg 60
tacctgacct cctctcagaa cattaccgag gagttttacc agagcacatg cagcgccgtg 120
agccgggggt acctgggcgc cctgagaacc ggctggtaca ccagcgtgat caccattgag 180
ctgagcaaca tcaaggaaac caagtgcaac ggcaccgaca ccaaggtgaa gctgattaag 240
caggagctgg acaagtacaa gaatgccgtg acagacctgc aactgctgat gcagaatacc 300
ccttgtggcg gatctggagg aggcagcggg agcgccatcg ctagcggaat cgccgtgtgt 360
aaagtgctgc atctggaagg cgaggtcaac aaaatcaaga acgccctgca gctgaccaac 420
aaggccgtgg tgtccctgtc caatggcgtg tccgtgctga ccttcaaggt gctggacctg 480
aagaattaca tcaacaacca gctgctgccc atcgtgaatc agcagagctg ccggatctcc 540
aacatcgaaa ccgtgatcga gtttcagcag aagaacagcc ggctgctgga gatcacacgc 600
gagttttccg tgaacgccgg ggtgaccacc cccctgagca cctacatgct gaccaatagc 660
gagctgctgt ccctgatcaa cgacatgcct atcaccaacg accagaagaa actgatgagc 720
agcaacgtgc agatcgtgcg ccagcaaagc tacagcatca tgtgcatcat caaggaggag 780
gtgctggcct acgtggtgca gctgcccatt tacggcgtga tcgacacccc ctgctggaag 840
ctgcacacct ctccactgtg cacaaccaat atcaaggaag ggagcaatat ctgcctgaca 900
cgcacagata gaggctggta ctgcgacaat gccggctccg tgagcttctt cccacaggcc 960
gatacatgca aggtgcagtg caacagagtg ttctgcgaca ccatgaacag cctgaccctg 1020
cccagcgagg tgagcctgtg caacaccgac atcttcaact ccaagtacga ctgcaagatc 1080
atgaccagca agaccgacat ttccagcagc gtcatcacca gcctgggcgc aatcgtgagc 1140
tgctacggga agaccaagtg taccgccagc aataagaacc gggggatcat caagaccttc 1200
agcaacggct gcgactacgt gtccaacaag ggcgtggaca ccgtgagcgt cggcaatacc 1260
ctgtactatg tgaataagct ggagggcaag aacctgtacg tgaagggcga gcccatcatc 1320
aactactacg accccctggt gttccctagc gacgagtttg acgccagcat cagccaggtg 1380
aacgagaaaa tcaaccaatc cctggccttc atccggagga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gcaccaccaa catcatgatc 1560
accgccatca tcatcgtgat tatcgtggtg ctgctgagcc tgatcgccat cggcctgctg 1620
ctgtactgca aggccaagaa tactcccgtg accctgagca aagaccagct gagcggcatc 1680
aacaatatcg ccttctccaa g 1701

SEQ ID NO: 110
length 1806
molecule type RNA
division PAT

Key                    Location/Qualifiers
source                 1..1806
                       /mol_type=other RNA
                       /organism=synthetic construct

Sequence
atggagctgc tgatccacag gagcagcgcc atctttctga ccctggccat caacgccctg   60
tacctgacca gcagccagaa cattacagag gagttttacc agagcacatg cagcgccgtg   120
agcaggggct atctgggcgc tctgaggacc ggatggtata caagcgtgat caccatcgag   180
ctgagcaaca tcaaggaaac caagtgcaac ggcaccgaca ccaaggtcaa gctgatcaag   240
caggagctgg acaagtacaa gaacgccgtg accgacctgc agctcctgat gcagaataca   300
ccatgcgtga acaaccgggc ccggcgggag gccccccagt acatgaatta caccatcaac   360
accaccaaga acctgaacgt gagcatctcc aagaagagaa agagaaggtt cctgggcttc   420
ctgctgggcg tgggatctgc cattgcttct ggaatcgctg tgtgtaaagt gctgcacctg   480
gagggcgagg tgaacaagat caagaatgcc ctgcagctga ccaacaaggc cgtggtgagc   540
ctgagcaacg gcgtgagcgt gctgaccttc aaggtgctgg acctgaaaaa ctacatcaac   600
aaccagctgc tgcccatcgt gaaccagcag agctgccgga tcagcaacat cgaaaccgtg   660
atcgagtttc agcagaagaa cagccggctg ctggagatca ccagggagtt ttccgtgaat   720
gccggcgtga ccacccccct gagcacctac atgctgacca atagcgagct gctgtccctg   780
atcaacgaca tgcccatcac caacgaccag aaaaagctga tgagcagcaa cgtgcagatc   840
gtgcggcagc aaagctacag catcatgtgc atcatcaagg aggaggtgct ggcctacgtg   900
gtgcagctgc ccatttacgg cgtgatcgac accccctgtt ggaagctgca cacaagccct   960
ctgtgcacca ccaacatcaa agagggcagc aatatctgcc tgacaagaac agaccgggga   1020
tggtactgcg acaatgccgg cagcgtgtct ttctttcctc aggccgacac ctgtaaggtg   1080
cagtgcaaca gagtgttctg cgacaccatg aacagcctga ccctgcccag cgaggtgagc   1140
ctctgcaaca ccgacatctt caacagcaag tacgactgca agatcatgac cagcaagacc   1200
gacatcagca gcagcgtgat taccagcctg ggcgccatcg tgagctgcta cggcaagacc   1260
aaatgcaccg cctctaacaa gaaccggggc atcattaaga ccttcagcaa cggctgcgac   1320
tacgtgagca acaagggcgt ggacaccgtg agcgtcggaa ataccctgta ctacgtgaac   1380
aagctggagg gcaagaatct gtacgtgaag ggcgagccca tcatcaacta ctacgacccc   1440
ctggtgttcc ccagctgtga gttttgcgcc agcatcagcc aggtgaacga gaagatcaac   1500
cagagcctgg ccttcatcag acggagcgac gagctgctcc acaacgtgga ggacaagatc   1560
gaggagatcc tgagcaagat ctaccacatc gagaacgaga tcgccaggat caagaagctg   1620
atcggcgagg ccaacaccgg caagagcacc acaaacatca tgatcaccgc catcatcatc   1680
gtgatcatcg tggtgctgct gagcctgatc gccatcggcc tgctgctgta ctgcaaggcc   1740
aagaacaccc ccgtgaccct gagcaaagat cagctgagcg gcatcaacaa tatcgccttc   1800
tccaag                                                              1806

SEQ ID NO:             111
length                 1806
molecule type          RNA
division               PAT

Key                    Location/Qualifiers
source                 1..1806
                       /mol_type=other RNA
                       /organism=synthetic construct

Sequence
atggagctgc tgatccacag aagcagcgcc atctttctga ccctggccat caatgccctg   60
tacctgacca gcagccagaa cattacagag gagttttacc agagcacatg cagcgccgtg   120
agcaggggct atctgggcgc tctgaggacc ggctggtata caagcgtgat caccatcgag   180
ctgagcaaca tcaaggaaac caagtgcaac ggcaccgaca ccaaggtcaa gctgatcaag   240
caggagctgg acaagtacaa gaacgccgtg accgacctgc agctcctgat gcagaataca   300
ccttgcgtga acaaccgggc ccggcgggag gccccccagt acatgaatta caccatcaac   360
accaccaaga acctgaacgt gagcatcagc aagaagagaa agagaaggtt cctgggcttc   420
ctgcttggcg tgggaagcgc cattgctagc ggaatcgctg tgtgtaaagt gctgcacctg   480
gagggagagg tgaacaagat caagaacgct ctgcagctga ccaacaaggc cgtggtgagc   540
ctgagcaacg gcgtgagcgt gctgaccttc aaggtgctgg acctgaaaaa ctacatcaac   600
aaccagctgc tgcccatcgt gaaccagcag agctgccgga tcagcaacat cgaaaccgtg   660

atcgagtttc agcagaagaa cagccggctg ctggagatca ccagggagtt ttctgtgaac 720
gccggcgtga ccacccccct gagcacctac atgctgacca atagcgagct gctgtccctg 780
atcaacgaca tgcccatcac caacgaccag aaaaagctga tgagcagcaa cgtgcagatc 840
gtgcggcagc aaagctacag catcatgtgc atcatcaagg aggaggtgct ggcctacgtg 900
gtgcagctgc ccatttacgg cgtgatcgac accccctgtt ggaagctgca cacaagccct 960
ctgtgcacca ccaacatcaa agagggcagc aatatctgcc tgactagaac agaccgggga 1020
tggtactgcg acaatgccgg cagcgtgtct ttctttcctc aggccgacac ctgtaaggtg 1080
cagtgcaaca gagtgttctg cgacaccatg aacagcctga ccctgcccag cgaggtgagc 1140
ctctgcaaca ccgacatctt caacagcaag tacgactgca agatcatgac cagcaagacc 1200
gacatcagca gcagcgtgat taccagcctg ggcgccatcg tgagctgcta cggcaagacc 1260
aaatgcaccg cctctaacaa gaaccggggc atcattaaga ccttcagcaa cggctgcgac 1320
tacgtgagca acaagggcgt ggacaccgtg agcgtcggaa ataccctgta ctacgtgaac 1380
aagctggagg gcaagaatct gtacgtgaag ggcgagccca tcatcaacta ctacgacccc 1440
ctggtgttcc ctagctgcga gttttgcgcc agcatcagcc aggtgaacga gaagatcaac 1500
cagagcctgg ccttcatcag acggagcgac gagctgctcc acaacgtgga ggacaagatc 1560
gaggagatcc tgagcaagat ctaccacatc gagaacgaga tcgccaggat caagaagctg 1620
atcggcgagg ccaacaccgg caagagcacc acgaacatca tgatcaccgc catcatcatc 1680
gtgatcatcg tggtgttgct gagcctgatc gccatcggcc tgctgctgta ctgcaaggcc 1740
aagaacaccc ccgtgaccct gagcaaagat cagctgtctg gcatcaacaa tatcgccttc 1800
tccaag 1806

SEQ ID NO: 112
length 1806
molecule type RNA
division PAT

Key Location/Qualifiers
source 1..1806
/mol_type=other RNA
/organism=synthetic construct

Sequence
atggagctgc tgatccacag aagctctgcc atctttctga ccctggccat caatgccctg 60
tacctgacca gcagccagaa cattacagag gagttttacc agagcacatg cagcgccgtg 120
agccggggct atctgggcgc tctgcgcacc ggctggtata caagcgtgat caccatcgag 180
ctgtctaaca tcaaggaaac caagtgcaac ggcaccgaca ccaaggtcaa gctgatcaag 240
caggagctgg acaagtacaa gaatgccgtg accgacctgc agctcctgat gcagaatact 300
ccttgcgtga acaaccgggc ccggcgggag gccccccagt acatgaatta caccatcaac 360
accaccaaga acctgaacgt gagcatctct aagaagagga agagacggtt tctgggcttc 420
ctgttgggcg tgggaagcgc cattgcaagc ggaatcgctg tgtgtaaagt gctgcacctg 480
gagggtgagg tgaacaagat caagaacgct ctgcagctga ccaacaaggc cgtggtgagc 540
ctgagcaacg gcgtgagcgt gctgaccttc aaggtgctcg acctgaaaaa ctacatcaac 600
aaccagctgc tgcccatcgt gaaccagcag agctgccgga tcagcaacat cgaaaccgtg 660
atcgagtttc agcagaagaa cagccggctc ctggagatca ccagggagtt ttctgtgaac 720
gccggggtga ccacccccct gtccacctac atgctgacca atagcgagct gctgtccctg 780
atcaacgaca tgcccatcac caacgaccag aaaaagctga tgagcagcaa cgtgcagatc 840
gtgcggcagc aaagctacag catcatgtgc atcatcaagg aggaggtgct ggcctacgtg 900
gtgcagctgc ccatttacgg cgtgatcgac accccctgtt ggaagctgca caccagccct 960
ctgtgcacaa ccaacatcaa agagggctcc aacatttgtc tgactaggac agaccgggga 1020
tggtattgcg acaacgccgg cagcgtgtct ttctttcctc aggccgacac ctgtaaggtg 1080
cagtgcaaca gagtgttctg cgacaccatg aacagcctga cactgcccag cgaggtgagc 1140
ctctgcaaca ccgacatctt caactccaag tacgactgca agatcatgac cagcaagacc 1200
gacatcagca gcagcgtgat taccagcctg ggcgccatcg tgtcctgcta cgggaagacc 1260
aaatgcaccg ccagtaacaa gaaccggggc atcattaaga ccttcagcaa cggctgcgac 1320
tacgtgagca acaagggcgt ggacaccgtg agcgtcggca ataccctgta ctatgtgaac 1380
aagctggagg gcaagaatct gtacgtgaag ggcgagccca tcatcaacta ctacgacccc 1440
ctggtgttcc ctagctgcga gttttgcgcc agcatctccc aggtcaacga gaagatcaac 1500
cagagcctgg ccttcatcag acggagcgac gagctgctcc acaacgtgga ggacaagatc 1560
gaggagatcc tgagcaagat ctaccacatc gagaacgaga tcgcccggat caagaagctg 1620
atcggcgagg ccaacaccgg caagagcacc acgaacatca tgatcaccgc catcatcatc 1680

gtgatcatcg tggtgttgct gagcctgatc gccatcggcc tgctgctgta ctgcaaggcc 1740
aagaacacac ccgtgaccct gagcaaagac cagctgtctg gcatcaacaa tatcgccttc 1800
tccaag 1806

SEQ ID NO: 113
length 1617
molecule type RNA
division PAT

Key Location/Qualifiers
source 1..1617
/mol_type=other RNA
/organism=synthetic construct

Sequence
atggagctgc tgatccacag aagctctgcc attttcctga ccctggccat caatgctctg 60
tacctgacca gcagccagaa catcaccgag gagttttacc agagcacctg tagcgccgtg 120
agcagaggct atctgggcgc cctgaggacc ggctggtaca ccagcgtgat caccatcgag 180
ctgagcaaca ttaaggagac aaagtgcaat ggcaccgaca ccaaggtgaa gctgatcaag 240
caggagctgg acaagtacaa gaacgccgtg accgatctgc agctcctgat gcagaataca 300
ccatgtgggg gatctggagg cggctctggc tctgctatcg cctctggaat tgccgtgtgc 360
aaagtgctgc acctggaggg cgaggtgaat aagatcaaga atgccctgca gctgaccaat 420
aaggccgtgg tgagcctgag caatggcgtg agcgtgctga ccttcaaggt gctcgacctg 480
aagaattaca tcaacaacca gctgctgccc atcgtgaacc agcagagctg ccggatcagc 540
aacatcgaaa ccgtgatcga gtttcagcag aagaacagcc ggctgctgga gatcaccagg 600
gagtttagcg tgaacgccgg cgtgaccacc ccactgagca cctacatgct gaccaacagc 660
gagctgctgt ccctgatcaa tgacatgcca atcaccaacg accagaagaa gctgatgagc 720
agcaacgtgc agatcgtgcg gcaacagagc tacagcatca tgtgcatcat caaggaggag 780
gtgctggcct acgtggtgca gctgcctatc tacggcgtga tcgacacccc ctgctggaag 840
ctgcatacca gccccctgtg tacaacaaac atcaaggaag gcagcaatat ctgcctgacc 900
aggacagaca gaggctggta ctgtgacaat gccggcagcg tgagcttctt ccctcaggct 960
gatacctgta aggtgcagtg caaccgggtg ttctgcgata ccatgaacag cctgaccctc 1020
cccagcgagg tgagcctctg caacaccgac atcttcaaca gcaagtacga ctgcaagatc 1080
atgaccagca agaccgacat cagcagcagc gtgattacca gcctgggcgc tatcgtgagc 1140
tgctacggca agaccaagtg caccgccagc aacaagaaca ggggcatcat taagaccttc 1200
agcaacggct gcgactacgt gagcaacaag ggcgtggaca cagtgagcgt gggcaacacc 1260
ctgtattacg tgaacaagct ggagggcaag aatctgtacg tgaagggcga gcccatcatc 1320
aactactacg accccctggt gttccctagc tgcgagtttt gcgccagcat cagccaggtg 1380
aacgagaaga ttaaccagag cctggccttt atccggcgga gcgatgagct gctgcacaac 1440
gtgaacaccg gcaagagcac caccaacatc atgatcaccg ccatcatcat cgtgatcatc 1500
gtggtgctgc tgagcctgat tgccatcggc ctgctgctgt actgtaaggc caagaacacc 1560
cctgtgaccc tgagcaagga tcagctgagc ggcatcaaca atatcgcctt ctccaag 1617

SEQ ID NO: 114
length 1617
molecule type RNA
division PAT

Key Location/Qualifiers
source 1..1617
/mol_type=other RNA
/organism=synthetic construct

Sequence
atggagctgc tgatccacag aagctctgcc atcttcctga ccctggccat caatgctctg 60
tacctgacca gcagccagaa catcaccgag gagttttacc agagcacctg tagcgccgtg 120
agccggggct atctgggcgc cctgaggacc ggatggtata ccagcgtgat caccatcgag 180
ctgtccaaca ttaaggagac aaagtgcaat ggcaccgaca ccaaggtgaa gctgatcaag 240
caggagctgg acaagtacaa gaacgccgtg accgatctgc agctcctgat gcagaataca 300
ccttgtggcg gatctggagg cggctctggc tctgctatcg cctctggaat tgccgtgtgc 360

aaggtgctgc acctggaggg agaggtgaat aagatcaaga atgccctgca gctgaccaat 420
aaggccgtgg tgagcctgag caacggcgtg agcgtgctga ccttcaaggt gctcgacctg 480
aagaattaca tcaacaacca gctgctgcca atcgtgaacc agcagagctg ccggatcagc 540
aacatcgaaa ccgtgatcga gtttcagcag aagaacagcc ggctgctgga gatcaccagg 600
gagtttagcg tgaacgccgg cgtgaccacc cctctgagca cctacatgct gaccaacagc 660
gagctgctgt ccctgatcaa tgacatgcca atcaccaacg accagaagaa gctgatgagc 720
agcaacgtgc agatcgtgcg gcaacagagc tacagcatca tgtgcatcat caaggaggag 780
gtgctggcct acgtggtgca gctgcctatc tacggcgtga tcgacacccc ctgctggaag 840
ctgcatacca gccccctgtg tacaacaaac atcaaagagg gcagcaatat ctgcctgacc 900
aggacagaca gaggctggta ctgtgacaat gccggcagcg tgagcttctt ccctcaggct 960
gatacctgta aagtgcagtg caaccgggtg ttctgcgaca ccatgaacag cctgaccctc 1020
cccagcgagg tgtccctctg caacaccgac atcttcaaca gcaagtacga ctgcaagatc 1080
atgaccagca agaccgacat cagcagcagc gtgattacca gcctgggcgc tatcgtgagc 1140
tgctacggca agaccaagtg caccgccagc aacaagaaca ggggcatcat taagaccttc 1200
agcaacggct gcgactacgt gagcaacaag ggcgtggaca cagtgagcgt gggcaacacc 1260
ctgtattacg tgaacaagct ggagggcaag aatctgtacg tgaagggcga gcccatcatc 1320
aactactacg accccctggt gttccctagc tgcgagtttt gcgccagcat cagccaggtg 1380
aacgagaaga ttaaccagag cctggccttt atccggcgga gcgatgagct gctgcacaac 1440
gtgaacaccg gcaagagcac caccaacatc atgatcaccg ccatcatcat cgtgatcatc 1500
gtggtgctgc tgagcctgat tgccatcggc ctgctgctgt actgtaaggc caagaacacc 1560
cctgtgaccc tgtctaagga ccagctgagc ggcatcaaca atatcgcctt ctccaag 1617

SEQ ID NO: 115
length 1617
molecule type RNA
division PAT

Key Location/Qualifiers
source 1..1617
/mol_type=other RNA
/organism=synthetic construct

Sequence
atggagctgc tgatccatag aagctctgcc attttcctga ccctggccat caatgccctg 60
tacctgacca gcagccagaa catcaccgag gagttttacc agagcacctg ctccgccgtg 120
tcccggggct accttggcgc cctgcgcacc ggatggtaca ccagcgtgat caccattgag 180
ctgagcaaca ttaaggaaac aaaatgcaat ggcaccgaca ccaaggtgaa gctgatcaag 240
caggagctgg acaagtacaa gaacgccgtg accgatctgc agctcctgat gcagaataca 300
ccttgtggcg gatctggcgg cggctctggc tctgctatcg ccagcggaat tgccgtgtgc 360
aaggtgctgc acctggaggg agaggtgaac aagatcaaga atgctctgca gctgacaaac 420
aaggccgtgg tgagcctgag caatggcgtg agcgtgctga ccttcaaggt gctcgatctg 480
aagaattaca tcaacaacca gctgctgcca atcgtgaacc agcagagctg ccggatcagc 540
aacatcgaaa ccgtgatcga gtttcagcag aagaacagcc ggctgctgga gatcaccagg 600
gagtttagcg tgaacgccgg cgtgaccacc cctctgagca cctacatgct gaccaacagc 660
gagctgctgt ccctgatcaa tgacatgccc atcaccaacg atcagaagaa gctgatgagc 720
agcaacgtgc agatcgtgcg gcagcaatcc tacagcatca tgtgcatcat caaggaggag 780
gtgctggcct acgtggtgca gctgcctatc tacggcgtga tcgacacccc ctgctggaag 840
ctgcacacaa gccccctgtg tactacaaac atcaaagagg gctccaacat ttgtctgacc 900
aggacagaca gaggctggta ctgtgacaac gccggcagcg tgtctttttt tccccaggct 960
gacacctgca aagtgcagtg caaccgggtg ttctgcgaca ccatgaatag cctgaccctc 1020
cccagcgagg tgagcctctg caacaccgac atcttcaaca gcaagtacga ctgcaagatc 1080
atgaccagca agaccgacat cagcagcagc gtgattacca gcctgggcgc aatcgtgagc 1140
tgctacggca agaccaagtg caccgcctcc aacaagaaca ggggcatcat taagaccttc 1200
agcaacggct gcgactacgt gagcaacaag ggcgtggaca cagtgagcgt gggcaacacc 1260
ctgtactatg tgaacaagct ggagggcaag aatctgtacg tgaagggcga gcccatcatc 1320
aactactacg accccctggt gttccccagc tgcgagtttt gcgccagcat tagccaggtg 1380
aacgagaaga tcaaccaatc cctggccttt atccggcgga gcgatgagct gctccacaac 1440
gtgaacaccg gcaagagcac caccaacatc atgatcaccg ccatcatcat cgtgatcatc 1500
gtggtgctgc tgagcctgat tgccatcggc ctgctgctgt actgcaaggc caagaacacc 1560
ccagtgaccc tgtcaaagga ccagctgagc ggcatcaaca atatcgcctt ctccaag 1617

SEQ ID NO:          116
length               1701
molecule type        RNA
division             PAT

Key                  Location/Qualifiers
source               1..1701
                     /mol_type=other RNA
                     /organism=synthetic construct

Sequence
atggagctgc tgatccacag aagcagcgcc atctttctga ccctggccat caacgctctg  60
tacctgacct ctagccagaa tatcaccgag gagttttacc agagcacatg cagcgccgtg  120
agcagaggct acctgggagc cctgaggacc ggctggtaca ccagcgtgat caccatcgag  180
ctgagcaata tcaaggaaac caagtgcaac ggcaccgaca ccaaggtcaa gctgatcaag  240
caggagctgg acaagtacaa gaatgccgtg accgatctgc agctcctgat gcagaataca  300
cctgctggag gatctggcgg aggaagcggc tctgctattg ccagcggcat cgccgtgtgt  360
aaagtgctgc acctggaggg agaagtgaat aagatcaaga acgccctgca gctgaccaac  420
aaggccgtgg tgagcctgag caacggcgtg tctgtgctga ccttcaaggt gctggacctg  480
aagaattaca tcaacaacca gctgctgccc atcgtgaacc agcagagctg ccggatcagc  540
aacatcgaaa ccgtgatcga gtttcagcag aagaacagcc ggctgctgga gatcacccgg  600
gagttttctg tgaacgccgg cgtgaccacc cccctgagca cctacatgct gaccaatagc  660
gagctgctgt ccctgatcaa tgacatgccc atcaccaacg accagaagaa actgatgagc  720
agcaacgtgc agatcgtgcg gcagcaaagc tacagcatca tgtgcatcat caaggaggag  780
gtgctggcct acgtggtgca gctgcctatc tacggcgtga tcgacacccc ctgctggaag  840
ctgcacacaa gccctctgtg cacaaccaac atcaaagagg gaagcaacat ctgtctgacc  900
aggaccgata gaggctggta ctgtgacaat gccggcagcg tgagcttctt tcctcaggcc  960
gatacctgca aagtgcagag caacagggtg ttctgcgaca ccatgaacag cctgaccctg  1020
cccagcgagg tcagcctgtg caacaccgac atcttcaaca gcaagtacga ctgcaagatc  1080
atgaccagca agaccgacat cagcagcagc gtgattacaa gcctgggcgc catcgtgagc  1140
tgctacggca agaccaaatg taccgccagc aacaagaacc ggggcatcat taagaccttc  1200
agcaacggct gcgactacgt gagcaacaag ggcgtggaca ccgtgagcgt gggcaatacc  1260
ctgtactacg tgaacaagct ggagggcaag aatctgtacg tgaagggcga gcccatcatc  1320
aactactacg accccctggt gttccccagc tgtgaatttt gcgccagcat cagccaggtg  1380
aacgagaaga tcaaccagag cctggccttc atcaggcgga gcgacgagct gctccacaac  1440
gtggaggaca agatcgagga gatcctgtcc aagatctacc acatcgagaa cgagatcgcc  1500
aggatcaaga agctgatcgg cgaggccaac accggcaaga gcaccaccaa tatcatgatc  1560
accgccatca tcatcgtgat catcgtggtg ctgctgagcc tgatcgccat cggcctgctg  1620
ctgtactgca aggccaagaa cacccccgtg acactgagca aggatcagct gagcggcatc  1680
aataatatcg ccttctccaa g                                          1701

SEQ ID NO:          117
length               1701
molecule type        RNA
division             PAT

Key                  Location/Qualifiers
source               1..1701
                     /mol_type=other RNA
                     /organism=synthetic construct

Sequence
atggagctgc tgatccacag aagcagcgcc atctttctga ccctggccat caacgctctg  60
tatctgacct ctagccagaa tatcaccgag gagttttacc agagcacatg cagcgccgtg  120
agccggggct acctgggagc cctgaggacc gggtggtaca ccagcgtgat cacaatcgag  180
ctgagcaata tcaaggaaac caagtgcaac ggcaccgaca ccaaggtcaa gctgatcaag  240
caggagctgg ataagtacaa gaatgccgtg accgatctgc agctcctgat gcagaataca  300
ccagccggag gatctggcgg aggaagcggc tctgctattg ccagcggcat cgccgtgtgt  360
aaggtgctgc atctggaggg agaggtgaat aagatcaaga acgccctgca gctgaccaac  420
aaggccgtgg tgagcctgag caacggcgtg tctgtgctga ccttcaaggt gctcgacctg  480
aagaattaca tcaataacca gctgctgccc atcgtgaacc agcagagctg ccggatcagc  540

aacatcgaaa ccgtgatcga gtttcagcag aagaacagcc ggctgctgga gatcacccgg 600
gagttttctg tgaacgccgg cgtgaccacc cccctgagca cctacatgct gaccaatagc 660
gagctgctgt ccctgatcaa tgacatgccc atcaccaacg accagaagaa actgatgagc 720
tccaacgtgc agatcgtgcg gcagcaaagc tacagcatca tgtgcatcat caaggaggag 780
gtgctggcct acgtggtgca gctgccaatc tacggcgtga tcgacacccc ctgctggaag 840
ctgcacacaa gccctctgtg cacaaccaac atcaaagagg gaagcaacat ctgcctgaca 900
aggaccgata gaggctggta ctgtgacaat gccggcagcg tgagcttctt tcctcaggcc 960
gatacctgca aagtgcagag caacagggtg ttctgcgaca ccatgaacag cctgaccctg 1020
cccagcgagg tcagcctgtg caacaccgac atcttcaaca gcaagtacga ctgcaagatc 1080
atgaccagca agaccgacat cagcagcagc gtgattacaa gcctgggcgc catcgtgagc 1140
tgctacggca agaccaaatg taccgccagc aacaagaacc ggggcatcat taagaccttc 1200
agcaacggct gcgactacgt gagcaacaag ggcgtggaca ccgtgagcgt gggcaatacc 1260
ctgtactacg tgaacaagct ggagggcaag aatctgtacg tgaagggcga gcccatcatc 1320
aactactacg accccctggt gtttcccagc tgtgagtttt gcgccagcat cagccaggtg 1380
aacgagaaga tcaaccagag cctggccttc atcaggcgga gcgacgagct gctccacaac 1440
gtggaggaca agatcgagga gatcctgtcc aagatctacc acatcgagaa cgagatcgcc 1500
aggatcaaga agctgatcgg cgaggccaac accggcaaga gcaccaccaa tatcatgatc 1560
accgccatca tcatcgtgat catcgtggtg ctgctgagcc tgatcgccat cggcctgctg 1620
ctgtactgca aggccaagaa cacacccgtg accctgagca aggatcagct gagcggcatc 1680
aataatatcg ccttctccaa g 1701

SEQ ID NO: 118
length 1701
molecule type RNA
division PAT

Key Location/Qualifiers
source 1..1701
/mol_type=other RNA
/organism=synthetic construct

Sequence
atggagctgc tgatccacag aagctctgcc atttttctga ccctggccat caacgcactg 60
tatctgacct ccagccagaa catcaccgag gagttttacc agagcacatg cagcgccgtg 120
agccggggct acctgggagc cctgcgcacc ggatggtaca ccagcgtgat cacaatcgag 180
ctgagcaata tcaaggaaac caagtgcaat ggcaccgaca ccaaggtcaa gctgatcaag 240
caggagctgg ataagtacaa gaacgccgtg acagatctgc agctcctgat gcagaatact 300
cctgccggag gatcaggcgg aggcagcggc agcgctattg cctccggaat cgccgtgtgc 360
aaggtgctgc atcttgaggg agaagtgaac aagattaaga acgccctgca gctgaccaac 420
aaggctgtgg tgagcctgag caacggcgtg tcagtgctga ccttcaaggt gctcgacctg 480
aaaaactaca tcaataacca gctgctgccc atcgtgaacc agcagagctg ccggatcagc 540
aacatcgaaa ccgtgatcga gtttcagcag aagaacagcc ggctcctgga gatcacccgg 600
gagttttctg tgaacgccgg cgtgaccacc cccctgagca cctacatgct gaccaatagc 660
gagctgctgt ccctgatcaa cgacatgccc atcaccaacg accagaagaa actgatgagc 720
tctaacgtgc agatcgtgcg gcagcaaagc tactccatca tgtgcatcat caaggaggag 780
gtgctggcct acgtggtgca gctgccaatc tacggcgtga tcgacacccc ctgctggaag 840
ctgcacacaa gccctctgtg taccaccaac atcaaagagg gaagcaacat ctgcctgacc 900
cgaacagata gaggctggta ctgcgacaat gccggcagcg tgagcttttt tcctcaggcc 960
gacacctgca aagtgcagtc aaaccgcgtg ttctgcgaca ccatgaacag cctgaccctg 1020
cctagcgagg tcagcctgtg caacaccgac atcttcaact ccaagtacga ctgcaagatc 1080
atgaccagca agaccgacat cagcagcagc gtgattacat ccctgggcgc catcgtgagc 1140
tgctacggca agaccaaatg caccgccagc aataagaacc ggggcatcat taagaccttc 1200
agcaacggct gcgactacgt gagcaacaag ggggtggaca ccgtgagcgt gggcaacacc 1260
ctgtactacg tgaataagct ggagggcaag aatctgtacg tgaagggcga gcccatcatc 1320
aactactacg accccctggt gttccccagc tgtgagtttt gcgccagcat cagccaggtg 1380
aatgagaaaa tcaaccaatc cctggccttc atccggcgga gcgacgagct gctccacaac 1440
gtggaggaca agatcgagga gatcctgagc aagatctacc acatcgagaa cgagatcgcc 1500
cggatcaaga agctgatcgg cgaggccaac accggcaaga gcaccaccaa tatcatgatc 1560
accgccatca tcatcgtgat catcgtggtg ctgctgagcc tgattgccat cggcctgctg 1620
ctgtactgca aggccaagaa cacacccgtg accctgagca aagaccagct gagcggcatc 1680

aataatatcg ccttctccaa g                                          1701

SEQ ID NO:          119
length              1722
molecule type       RNA
division            PAT

Key                 Location/Qualifiers
source              1..1722
                    /mol_type=other RNA
                    /organism=synthetic construct

Sequence
atggagctgc tgatccacag aagcagcgcc atcttcctga ccctggccat caatgccctg  60
tatctgacca gcagccagaa catcaccgag gagttttacc agagcacatg cagcgccgtg  120
agcaggggct atctgagcgc cctgaggacc ggctggtaca caagcgtgat caccattgag  180
ctgagcaata tcaaggagac aaagtgcaac ggcaccgaca ccaaggtgaa gctgatcaag  240
caggagctgg acaagtacaa gaacgccgtg accgagctgc aactgctgat gcagaatacc  300
cctgccgtga acaaccgggc ccggcgggag gccccccagt acatgaatta caccatcaac  360
accaccaaga acctgaacgt gagcattagc aagaagagga agaggagatt cctgggcttc  420
ctgctgggag tgggatctgc tatcgcctct ggcattgccg tgtctaaagt gctgcacctg  480
gagggcgagg tgaacaagat caagaacgct ctgcagctga ccaataaggc cgtcgtgagc  540
ctgagcaacg gcgtgagcgt gctgacaagc aaggtgctgg acctgaagaa ttacatcaac  600
aaccagctgc tgcccatcgt gaaccagcag agctgccgga tcagcaacat cgaaaccgtg  660
atcgagtttc agcagaagaa cagccggctg ctggagatca ccagggagtt tagcgtgaac  720
gccggcgtga caacccctct gagcacctac atgctgacca acagcgagct gctgtctctg  780
atcaacgaca tgcccatcac caacgaccag aagaagctga tgagcagcaa cgtgcagatc  840
gtgcggcagc aaagctacag catcatgagc atcatcaagg aggaggtgct ggcctacgtg  900
gtgcagctgc caatttacgg cgtgatcgac accccctgct ggaagctgca cacctctccc  960
ctgtgtacca caaacatcaa agagggcagc aacatttgtc tgaccagaac cgacagaggc  1020
tggtactgtg acaatgccgg cagcgtgagc tttttccctc aggccgatac ctgcaaggtg  1080
cagagcaata gggtgttctg cgacaccatg aacagcctga ccctccccag cgaggtgagc  1140
ctgtgcaaca ccgacatctt caacagcaag tacgactgca agatcatgac cagcaagacc  1200
gacatcagca gcagcgtgat tacctctctg ggagccatcg tgagctgcta cggcaagacc  1260
aagtgtaccg ccagcaacaa gaaccggggc atcattaaga ccttcagcaa cggctgcgac  1320
tacgtgagca acaagggcgt ggacacagtg agcgtgggca acaccctgta ctacgtgaac  1380
aagctggagg gcaagaatct gtacgtgaag ggcgagccca tcatcaacta ctacgacccc  1440
ctggtgtttc ctagcgacga gtttgacgcc agcatcagcc aggtgaatga gaagatcaac  1500
cagagcctgg ccttcatcag gcggagcgac gagctgctcc acaacgtgaa caccggcaag  1560
agcaccacca acatcatgat caccgccatc atcatcgtga ttatcgtggt gctgctgagc  1620
ctgatcgcca ttggcctgct gctgtactgc aaggccaaga atacacccgt gaccctgagc  1680
aaggatcagc tgagcggcat caataacatc gccttctcca ag                    1722

SEQ ID NO:          120
length              567
molecule type       AA
division            PAT

Key                 Location/Qualifiers
source              1..567
                    /mol_type=protein
                    /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIS  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360

MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLED 480
KIEEILSKIY HIENEIARIK KLIGEAHNVN AGKSTTNIMI TTIIIVIIVI LLALIAVGLL 540
LYCKARSTPV TLSKDQLSGI NNIAFSN 567

SEQ ID NO: 121
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctggaagat 1440
aaaattgaag aaattctctc caaaatttat catattgaaa atgaaattgc tagaattaaa 1500
aaactcattg gagaagctca caacgtgaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 122
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180

NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VNAGEDKIEE ILSKIYHIEN EIARIKKLIG EAKSTTNIMI TTIIIVIIVI LLALIAVGLL 540
LYCKARSTPV TLSKDQLSGI NNIAFSN 567

SEQ ID NO: 123
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtgaacgccg gcgaagataa aattgaagaa attctctcca aaatttatca tattgaaaat 1500
gaaattgcta gaattaaaaa actcattgga gaagctaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 124
length 567
molecule type AA
division PAT

Key Location/Qualifiers
source 1..567
/mol_type=protein
/organism=synthetic construct

Sequence

MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VNAGKSTEDK IEEILSKIYH IENEIARIKK LIGEATNIMI TTIIIVIIVI LLALIAVGLL 540
LYCKARSTPV TLSKDQLSGI NNIAFSN 567

SEQ ID NO: 125
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtgaacgccg gcaagagcac cgaagataaa attgaagaaa ttctctccaa aatttatcat 1500
attgaaaatg aaattgctag aattaaaaaa ctcattggag aagctaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 126
length 566
molecule type AA
division PAT

Key Location/Qualifiers
source 1..566
/mol_type=protein

/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGSGS AIASGIAVCK 120
VLHLEGEVNK IKSALLSTNK AVVSLSNGVS VLTFKVLDLK NYIDKQLLPI VNKQSCSISN 180
IETVIEFQQK NNRLLEITRE FSVNAGVTTP VSTYMLTNSE LLSLINDMPI TNDQKKLMSS 240
NVQIVRQQSY SIMCIIKEEV LAYVVQLPLY GVIDTPCWKL HTSPLCTTNT KEGSNICLTR 300
TDRGWYCDNA GSVSFFPQAE TCKVQCNRVF CDTMNSLTLP SEVNLCNIDI FNPKYDCKIM 360
TSKTDVSSSV ITSLGAIVSC YGKTKCTASN KNRGIIKTFS NGCDYVSNKG VDTVSVGNTL 420
YYVNKQEGKS LYVKGEPIIN FYDPLVFPSC EFCASISQVN EKINQSLAFI RKSDELLHNV 480
EDKIEEILSK IYHIENEIAR IKKLIGEANA GKSTTNIMIT TIIIVIIVIL LALIAVGLLL 540
YCKARSTPVT LSKDQLSGIN NIAFSN 566

SEQ ID NO: 127
length 1698
molecule type RNA
division PAT

Key Location/Qualifiers
source 1..1698
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg atctggcagc gctatcgcct ctggaattgc cgtgtgtaaa 360
gtgctgcacc tggagggcga agtgaacaag atcaaaagcg ccctgctgag caccaacaag 420
gctgtggtga gcctgagcaa tggcgtgagc gtgctgacct tcaaggtgct ggacctgaaa 480
aactacattg acaagcagct gctccccatc gtgaacaagc agagctgcag catcagcaac 540
atcgagacgg tgatcgagtt tcagcagaag aacaaccggc tgctggagat cacccgggag 600
tttagcgtga acgctggcgt gaccacccca gtgagcacct atatgctgac caacagcgag 660
ctgctgagcc tgatcaacga catgcccatc accaacgatc agaagaagct gatgagcagc 720
aacgtgcaga tcgtgaggca gcagagctac agcatcatgt gcatcatcaa ggaggaggtg 780
ctggcctacg tggtgcagct gcctctgtac ggagtgatcg acaccccctg ttggaaactg 840
cacacatctc ctctgtgcac aaccaacacc aaggagggca gcaacatctg cctgacaaga 900
accgataggg gctggtactg tgacaatgcc ggctctgtgt ctttctttcc tcaggccgag 960
acatgcaagg tgcagtgcaa ccgggtgttc tgcgacacca tgaacagcct gaccctgccc 1020
agcgaggtca acctgtgcaa catcgacatc ttcaacccca agtacgactg caagatcatg 1080
accagcaaga ccgacgtgtc tagctccgtg atcacaagcc tgggagccat cgtgagctgc 1140
tacggcaaga ccaagtgcac cgccagcaac aagaatcggg gcatcattaa gaccttcagc 1200
aacggctgcg actacgtgag caacaagggc gtggatacag tgagcgtggg caacaccctg 1260
tactacgtga ataagcagga gggcaagagc ctgtacgtga agggcgagcc catcatcaac 1320
ttctacgacc ccctggtgtt tcccagctgc gagttttgcg ccagcatcag ccaggtgaac 1380
gagaaaatca atcagagcct ggccttcatc aggaagagcg acgagctcct gcacaacgtg 1440
gaagataaaa ttgaagaaat tctctccaaa atttatcata ttgaaaatga aattgctaga 1500
attaaaaaac tcattggaga agctaacgcc ggcaagagca ccaccaacat catgatcacc 1560
accatcatca tcgtgatcat tgtgatcctg ctggccctga tcgccgtggg cctgctgctg 1620
tactgcaagg ctaggagcac ccctgtgacc ctgagcaagg atcagctgag cggcatcaat 1680
aacatcgcct tcagcaac 1698

SEQ ID NO: 128
length 565
molecule type AA
division PAT

Key                 Location/Qualifiers
source              1..565
                    /mol_type=protein
                    /organism=synthetic construct

Sequence
MELPILKTNA ITTILAAVTL CFASSQNITE EFYQSTCSAV SKGYLGALRT GWYTSVITIE   60
LSNIKENKCN GTDAKVKLIK QELDKYKNAV TDLQLLMQST PCGGSGSGSA IASGIAVCKV  120
LHLEGEVNKI KSALLSTNKA VVSLSNGVSV LTFKVLDLKN YIDKQLLPIV NKQSCSISNI  180
ETVIEFQQKN NRLLEITREF SVNAGVTTPV STYMLTNSEL LSLINDMPIT NDQKKLMSSN  240
VQIVRQQSYS IMCIIKEEVL AYVVQLPLYG VIDTPCWKLH TSPLCTTNTK EGSNICLTRT  300
DRGWYCDNAG SVSFFPQAET CKVQCNRVFC DTMNSLTLPS EVNLCNIDIF NPKYDCKIMT  360
SKTDVSSSVI TSLGAIVSCY GKTKCTASNK NRGIIKTFSN GCDYVSNKGV DTVSVGNTLY  420
YVNKQEGKSL YVKGEPIINF YDPLVFPSCE FCASISQVNE KINQSLAFIR KSDELLHNVE  480
DKIEEILSKI YHIENEIARI KKLIGEANAG KSTTNIMITT IIIVIIVILL ALIAVGLLLY  540
CKARSTPVTL SKDQLSGINN IAFSN                                        565

SEQ ID NO:          129
length              1695
molecule type       RNA
division            PAT

Key                 Location/Qualifiers
source              1..1695
                    /mol_type=other RNA
                    /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggctc tggcagcgct atcgcctctg gaattgccgt gtgtaaagtg  360
ctgcacctgg agggcgaagt gaacaagatc aaaagcgccc tgctgagcac caacaaggct  420
gtggtgagcc tgagcaatgg cgtgagcgtg ctgaccttca aggtgctgga cctgaaaaac  480
tacattgaca agcagctgct ccccatcgtg aacaagcaga gctgcagcat cagcaacatc  540
gagacggtga tcgagtttca gcagaagaac aaccggctgc tggagatcac ccgggagttt  600
agcgtgaacg ctggcgtgac caccccagtg agcacctata tgctgaccaa cagcgagctg  660
ctgagcctga tcaacgacat gcccatcacc aacgatcaga agaagctgat gagcagcaac  720
gtgcagatcg tgaggcagca gagctacagc atcatgtgca tcatcaagga ggaggtgctg  780
gcctacgtgg tgcagctgcc tctgtacgga gtgatcgaca ccccctgttg gaaactgcac  840
acatctcctc tgtgcacaac caacaccaag gagggcagca acatctgcct gacaagaacc  900
gataggggct ggtactgtga caatgccggc tctgtgtctt tctttcctca ggccgagaca  960
tgcaaggtgc agtgcaaccg ggtgttctgc gacaccatga acagcctgac cctgcccagc 1020
gaggtcaacc tgtgcaacat cgacatcttc aaccccaagt acgactgcaa gatcatgacc 1080
agcaagaccg acgtgtctag ctccgtgatc acaagcctgg gagccatcgt gagctgctac 1140
ggcaagacca agtgcaccgc cagcaacaag aatcggggca tcattaagac cttcagcaac 1200
ggctgcgact acgtgagcaa caagggcgtg gatacagtga gcgtgggcaa caccctgtac 1260
tacgtgaata agcaggaggg caagagcctg tacgtgaagg gcgagcccat catcaacttc 1320
tacgaccccc tggtgtttcc cagctgcgag ttttgcgcca gcatcagcca ggtgaacgag 1380
aaaatcaatc agagcctggc cttcatcagg aagagcgacg agctcctgca caacgtggaa 1440
gataaaattg aagaaattct ctccaaaatt tatcatattg aaaatgaaat tgctagaatt 1500
aaaaaactca ttggagaagc taacgccggc aagagcacca ccaacatcat gatcaccacc 1560
atcatcatcg tgatcattgt gatcctgctg gccctgatcg ccgtgggcct gctgctgtac 1620
tgcaaggcta ggagcacccc tgtgaccctg agcaaggatc agctgagcgg catcaataac 1680
atcgccttca gcaac                                                  1695

SEQ ID NO:          130
length              564

molecule type AA
division PAT

Key Location/Qualifiers
source 1..564
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGSGSGSAI ASGIAVCKVL 120
HLEGEVNKIK SALLSTNKAV VSLSNGVSVL TFKVLDLKNY IDKQLLPIVN KQSCSISNIE 180
TVIEFQQKNN RLLEITREFS VNAGVTTPVS TYMLTNSELL SLINDMPITN DQKKLMSSNV 240
QIVRQQSYSI MCIIKEEVLA YVVQLPLYGV IDTPCWKLHT SPLCTTNTKE GSNICLTRTD 300
RGWYCDNAGS VSFFPQAETC KVQCNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSCEF CASISQVNEK INQSLAFIRK SDELLHNVED 480
KIEEILSKIY HIENEIARIK KLIGEANAGK STTNIMITTI IIVIIVILLA LIAVGLLLYC 540
KARSTPVTLS KDQLSGINNI AFSN 564

SEQ ID NO: 131
length 1692
molecule type RNA
division PAT

Key Location/Qualifiers
source 1..1692
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggat ctggctctgg cagcgctatc gcctctggaa ttgccgtgtg taaagtgctg 360
cacctggagg gcgaagtgaa caagatcaaa agcgccctgc tgagcaccaa caaggctgtg 420
gtgagcctga gcaatggcgt gagcgtgctg accttcaagg tgctggacct gaaaaactac 480
attgacaagc agctgctccc catcgtgaac aagcagagct gcagcatcag caacatcgag 540
acggtgatcg agtttcagca gaagaacaac cggctgctgg agatcacccg ggagtttagc 600
gtgaacgctg gcgtgaccac cccagtgagc acctatatgc tgaccaacag cgagctgctg 660
agcctgatca acgacatgcc catcaccaac gatcagaaga agctgatgag cagcaacgtg 720
cagatcgtga ggcagcagag ctacagcatc atgtgcatca tcaaggagga ggtgctggcc 780
tacgtggtgc agctgcctct gtacggagtg atcgacaccc cctgttggaa actgcacaca 840
tctcctctgt gcacaaccaa caccaaggag ggcagcaaca tctgcctgac aagaaccgat 900
aggggctggt actgtgacaa tgccggctct gtgtctttct ttcctcaggc cgagacatgc 960
aaggtgcagt gcaaccgggt gttctgcgac accatgaaca gcctgaccct gcccagcgag 1020
gtcaacctgt gcaacatcga catcttcaac cccaagtacg actgcaagat catgaccagc 1080
aagaccgacg tgtctagctc cgtgatcaca agcctgggag ccatcgtgag ctgctacggc 1140
aagaccaagt gcaccgccag caacaagaat cggggcatca ttaagacctt cagcaacggc 1200
tgcgactacg tgagcaacaa gggcgtggat acagtgagcg tgggcaacac cctgtactac 1260
gtgaataagc aggagggcaa gagcctgtac gtgaagggcg agcccatcat caacttctac 1320
gaccccctgg tgtttcccag ctgcgagttt tgcgccagca tcagccaggt gaacgagaaa 1380
atcaatcaga gcctggcctt catcaggaag agcgacgagc tcctgcacaa cgtggaagat 1440
aaaattgaag aaattctctc caaaatttat catattgaaa atgaaattgc tagaattaaa 1500
aaactcattg gagaagctaa cgccggcaag agcaccacca acatcatgat caccaccatc 1560
atcatcgtga tcattgtgat cctgctggcc ctgatcgccg tgggcctgct gctgtactgc 1620
aaggctagga gcacccctgt gaccctgagc aaggatcagc tgagcggcat caataacatc 1680
gccttcagca ac 1692

SEQ ID NO:          132
length               563
molecule type        AA
division             PAT

Key                  Location/Qualifiers
source               1..563
                     /mol_type=protein
                     /organism=synthetic construct

Sequence
MELPILKTNA ITTILAAVTL CFASSQNITE EFYQSTCSAV SKGYLGALRT GWYTSVITIE   60
LSNIKENKCN GTDAKVKLIK QELDKYKNAV TDLQLLMQST PCGGSGSAIA SGIAVCKVLH  120
LEGEVNKIKS ALLSTNKAVV SLSNGVSVLT FKVLDLKNYI DKQLLPIVNK QSCSISNIET  180
VIEFQQKNNR LLEITREFSV NAGVTTPVST YMLTNSELLS LINDMPITND QKKLMSSNVQ  240
IVRQQSYSIM CIIKEEVLAY VVQLPLYGVI DTPCWKLHTS PLCTTNTKEG SNICLTRTDR  300
GWYCDNAGSV SFFPQAETCK VQCNRVFCDT MNSLTLPSEV NLCNIDIFNP KYDCKIMTSK  360
TDVSSSVITS LGAIVSCYGK TKCTASNKNR GIIKTFSNGC DYVSNKGVDT VSVGNTLYYV  420
NKQEGKSLYV KGEPIINFYD PLVFPSCEFC ASISQVNEKI NQSLAFIRKS DELLHNVEDK  480
IEEILSKIYH IENEIARIKK LIGEANAGKS TTNIMITTII IVIIVILLAL IAVGLLLYCK  540
ARSTPVTLSK DQLSGINNIA FSN                                          563

SEQ ID NO:          133
length               1689
molecule type        RNA
division             PAT

Key                  Location/Qualifiers
source               1..1689
                     /mol_type=other RNA
                     /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggag gctctggcag cgctatcgcc tctggaattg ccgtgtgtaa agtgctgcac  360
ctggagggcg aagtgaacaa gatcaaaagc gccctgctga gcaccaacaa ggctgtggtg  420
agcctgagca atggcgtgag cgtgctgacc ttcaaggtgc tggacctgaa aaactacatt  480
gacaagcagc tgctccccat cgtgaacaag cagagctgca gcatcagcaa catcgagacg  540
gtgatcgagt ttcagcagaa gaacaaccgg ctgctggaga tcacccggga gtttagcgtg  600
aacgctggcg tgaccacccc agtgagcacc tatatgctga ccaacagcga gctgctgagc  660
ctgatcaacg acatgcccat caccaacgat cagaagaagc tgatgagcag caacgtgcag  720
atcgtgaggc agcagagcta cagcatcatg tgcatcatca aggaggaggt gctggcctac  780
gtggtgcagc tgcctctgta cggagtgatc gacaccccct gttggaaact gcacacatct  840
cctctgtgca caaccaacac caaggagggc agcaacatct gcctgacaag aaccgatagg  900
ggctggtact gtgacaatgc cggctctgtg tctttctttc ctcaggccga gacatgcaag  960
gtgcagtgca accgggtgtt ctgcgacacc atgaacagcc tgaccctgcc cagcgaggtc 1020
aacctgtgca acatcgacat cttcaacccc aagtacgact gcaagatcat gaccagcaag 1080
accgacgtgt ctagctccgt gatcacaagc ctgggagcca tcgtgagctg ctacggcaag 1140
accaagtgca ccgccagcaa caagaatcgg ggcatcatta agaccttcag caacggctgc 1200
gactacgtga gcaacaaggg cgtggataca gtgagcgtgg gcaacaccct gtactacgtg 1260
aataagcagg agggcaagag cctgtacgtg aagggcgagc ccatcatcaa cttctacgac 1320
cccctggtgt ttcccagctg cgagttttgc gccagcatca gccaggtgaa cgagaaaatc 1380
aatcagagcc tggccttcat caggaagagc gacgagctcc tgcacaacgt ggaagataaa 1440
attgaagaaa ttctctccaa aatttatcat attgaaaatg aaattgctag aattaaaaaa 1500
ctcattggag aagctaacgc cggcaagagc accaccaaca tcatgatcac caccatcatc 1560
atcgtgatca ttgtgatcct gctggccctg atcgccgtgg gcctgctgct gtactgcaag 1620
gctaggagca cccctgtgac cctgagcaag gatcagctga gcggcatcaa taacatcgcc 1680

ttcagcaac                                                          1689

SEQ ID NO:          134
length              562
molecule type       AA
division            PAT

Key                 Location/Qualifiers
source              1..562
                    /mol_type=protein
                    /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGSGSAIAS GIAVCKVLHL  120
EGEVNKIKSA LLSTNKAVVS LSNGVSVLTF KVLDLKNYID KQLLPIVNKQ SCSISNIETV  180
IEFQQKNNRL LEITREFSVN AGVTTPVSTY MLTNSELLSL INDMPITNDQ KKLMSSNVQI  240
VRQQSYSIMC IIKEEVLAYV VQLPLYGVID TPCWKLHTSP LCTTNTKEGS NICLTRTDRG  300
WYCDNAGSVS FFPQAETCKV QCNRVFCDTM NSLTLPSEVN LCNIDIFNPK YDCKIMTSKT  360
DVSSSVITSL GAIVSCYGKT KCTASNKNRG IIKTFSNGCD YVSNKGVDTV SVGNTLYYVN  420
KQEGKSLYVK GEPIINFYDP LVFPSCEFCA SISQVNEKIN QSLAFIRKSD ELLHNVEDKI  480
EEILSKIYHI ENEIARIKKL IGEANAGKST TNIMITTIII VIIVILLALI AVGLLLYCKA  540
RSTPVTLSKD QLSGINNIAF SN                                          562

SEQ ID NO:          135
length              1686
molecule type       RNA
division            PAT

Key                 Location/Qualifiers
source              1..1686
                    /mol_type=other RNA
                    /organism=synthetic construct

Sequence
atggagctgc ccatcctgaa gaccaatgcc attacaacca tcctggccgc cgtgacactg  60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggct ctggcagcgc tatcgcctct ggaattgccg tgtgtaaagt gctgcacctg  360
gagggcgaag tgaacaagat caaaagcgcc ctgctgagca ccaacaaggc tgtggtgagc  420
ctgagcaatg gcgtgagcgt gctgaccttc aaggtgctgg acctgaaaaa ctacattgac  480
aagcagctgc tccccatcgt gaacaagcag agctgcagca tcagcaacat cgagacggtg  540
atcgagtttc agcagaagaa caaccggctg ctggagatca cccgggagtt tagcgtgaac  600
gctggcgtga ccaccccagt gagcacctat atgctgacca acagcgagct gctgagcctg  660
atcaacgaca tgcccatcac caacgatcag aagaagctga tgagcagcaa cgtgcagatc  720
gtgaggcagc agagctacag catcatgtgc atcatcaagg aggaggtgct ggcctacgtg  780
gtgcagctgc ctctgtacgg agtgatcgac accccctgtt ggaaactgca cacatctcct  840
ctgtgcacaa ccaacaccaa ggagggcagc aacatctgcc tgacaagaac cgataggggc  900
tggtactgtg acaatgccgg ctctgtgtct ttctttcctc aggccgagac atgcaaggtg  960
cagtgcaacc gggtgttctg cgacaccatg aacagcctga ccctgcccag cgaggtcaac  1020
ctgtgcaaca tcgacatctt caaccccaag tacgactgca agatcatgac cagcaagacc  1080
gacgtgtcta gctccgtgat cacaagcctg ggagccatcg tgagctgcta cggcaagacc  1140
aagtgcaccg ccagcaacaa gaatcggggc atcattaaga ccttcagcaa cggctgcgac  1200
tacgtgagca acaagggcgt ggatacagtg agcgtgggca acaccctgta ctacgtgaat  1260
aagcaggagg gcaagagcct gtacgtgaag ggcgagccca tcatcaactt ctacgacccc  1320
ctggtgtttc ccagctgcga gttttgcgcc agcatcagcc aggtgaacga gaaaatcaat  1380
cagagcctgg ccttcatcag gaagagcgac gagctcctgc acaacgtgga agataaaatt  1440
gaagaaattc tctccaaaat ttatcatatt gaaaatgaaa ttgctagaat taaaaaactc  1500

attggagaag ctaacgccgg caagagcacc accaacatca tgatcaccac catcatcatc 1560
gtgatcattg tgatcctgct ggccctgatc gccgtgggcc tgctgctgta ctgcaaggct 1620
aggagcaccc ctgtgaccct gagcaaggat cagctgagcg gcatcaataa catcgccttc 1680
agcaac 1686

SEQ ID NO: 136
length 568
molecule type AA
division PAT

Key Location/Qualifiers
source 1..568
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGGSGGGS GSAIASGIAV 120
CKVLHLEGEV NKIKSALLST NKAVVSLSNG VSVLTFKVLD LKNYIDKQLL PIVNKQSCSI 180
SNIETVIEFQ QKNNRLLEIT REFSVNAGVT TPVSTYMLTN SELLSLINDM PITNDQKKLM 240
SSNVQIVRQQ SYSIMCIIKE EVLAYVVQLP LYGVIDTPCW KLHTSPLCTT NTKEGSNICL 300
TRTDRGWYCD NAGSVSFFPQ AETCKVQCNR VFCDTMNSLT LPSEVNLCNI DIFNPKYDCK 360
IMTSKTDVSS SVITSLGAIV SCYGKTKCTA SNKNRGIIKT FSNGCDYVSN KGVDTVSVGN 420
TLYYVNKQEG KSLYVKGEPI INFYDPLVFP SCEFCASISQ VNEKINQSLA FIRKSDELLH 480
NVEDKIEEIL SKIYHIENEI ARIKKLIGEA NAGKSTTNIM ITTIIIVIIV ILLALIAVGL 540
LLYCKARSTP VTLSKDQLSG INNIAFSN 568

SEQ ID NO: 137
length 1704
molecule type RNA
division PAT

Key Location/Qualifiers
source 1..1704
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gaggatctgg cggaggatct ggcagcgcta tcgcctctgg aattgccgtg 360
tgtaaagtgc tgcacctgga gggcgaagtg aacaagatca aaagcgccct gctgagcacc 420
aacaaggctg tggtgagcct gagcaatggc gtgagcgtgc tgaccttcaa ggtgctggac 480
ctgaaaaact acattgacaa gcagctgctc cccatcgtga acaagcagag ctgcagcatc 540
agcaacatcg agacggtgat cgagtttcag cagaagaaca accggctgct ggagatcacc 600
cgggagttta gcgtgaacgc tggcgtgacc accccagtga gcacctatat gctgaccaac 660
agcgagctgc tgagcctgat caacgacatg cccatcacca acgatcagaa gaagctgatg 720
agcagcaacg tgcagatcgt gaggcagcag agctacagca tcatgtgcat catcaaggag 780
gaggtgctgg cctacgtggt gcagctgcct ctgtacggag tgatcgacac cccctgttgg 840
aaactgcaca catctcctct gtgcacaacc aacaccaagg agggcagcaa catctgcctg 900
acaagaaccg ataggggctg gtactgtgac aatgccggct ctgtgtcttt ctttcctcag 960
gccgagacat gcaaggtgca gtgcaaccgg gtgttctgcg acaccatgaa cagcctgacc 1020
ctgcccagcg aggtcaacct gtgcaacatc gacatcttca accccaagta cgactgcaag 1080
atcatgacca gcaagaccga cgtgtctagc tccgtgatca caagcctggg agccatcgtg 1140
agctgctacg gcaagaccaa gtgcaccgcc agcaacaaga atcggggcat cattaagacc 1200
ttcagcaacg gctgcgacta cgtgagcaac aagggcgtgg atacagtgag cgtgggcaac 1260
accctgtact acgtgaataa gcaggagggc aagagcctgt acgtgaaggg cgagcccatc 1320

atcaacttct acgaccccct ggtgtttccc agctgcgagt tttgcgccag catcagccag 1380
gtgaacgaga aaatcaatca gagcctggcc ttcatcagga agagcgacga gctcctgcac 1440
aacgtggaag ataaaattga agaaattctc tccaaaattt atcatattga aaatgaaatt 1500
gctagaatta aaaaactcat tggagaagct aacgccggca agagcaccac caacatcatg 1560
atcaccacca tcatcatcgt gatcattgtg atcctgctgg ccctgatcgc cgtgggcctg 1620
ctgctgtact gcaaggctag gagcacccct gtgaccctga gcaaggatca gctgagcggc 1680
atcaataaca tcgccttcag caac 1704

SEQ ID NO: 138
length 569
molecule type AA
division PAT

Key Location/Qualifiers
source 1..569
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGSGG SGSAIASGIA 120
VCKVLHLEGE VNKIKSALLS TNKAVVSLSN GVSVLTFKVL DLKNYIDKQL LPIVNKQSCS 180
ISNIETVIEF QQKNNRLLEI TREFSVNAGV TTPVSTYMLT NSELLSLIND MPITNDQKKL 240
MSSNVQIVRQ QSYSIMCIIK EEVLAYVVQL PLYGVIDTPC WKLHTSPLCT TNTKEGSNIC 300
LTRTDRGWYC DNAGSVSFFP QAETCKVQCN RVFCDTMNSL TLPSEVNLCN IDIFNPKYDC 360
KIMTSKTDVS SSVITSLGAI VSCYGKTKCT ASNKNRGIIK TFSNGCDYVS NKGVDTVSVG 420
NTLYYVNKQE GKSLYVKGEP IINFYDPLVF PSCEFCASIS QVNEKINQSL AFIRKSDELL 480
HNVEDKIEEI LSKIYHIENE IARIKKLIGE ANAGKSTTNI MITTIIIVII VILLALIAVG 540
LLLYCKARST PVTLSKDQLS GINNIAFSN 569

SEQ ID NO: 139
length 1707
molecule type RNA
division PAT

Key Location/Qualifiers
source 1..1707
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggag gatctggagg atctggagga tctggcagcg ctatcgcctc tggaattgcc 360
gtgtgtaaag tgctgcacct ggagggcgaa gtgaacaaga tcaaaagcgc cctgctgagc 420
accaacaagg ctgtggtgag cctgagcaat ggcgtgagcg tgctgacctt caaggtgctg 480
gacctgaaaa actacattga caagcagctg ctccccatcg tgaacaagca gagctgcagc 540
atcagcaaca tcgagacggt gatcgagttt cagcagaaga acaaccggct gctggagatc 600
acccgggagt ttagcgtgaa cgctggcgtg accaccccag tgagcaccta tatgctgacc 660
aacagcgagc tgctgagcct gatcaacgac atgcccatca ccaacgatca gaagaagctg 720
atgagcagca acgtgcagat cgtgaggcag cagagctaca gcatcatgtg catcatcaag 780
gaggaggtgc tggcctacgt ggtgcagctg cctctgtacg gagtgatcga caccccctgt 840
tggaaactgc acacatctcc tctgtgcaca accaacacca aggagggcag caacatctgc 900
ctgacaagaa ccgatagggg ctggtactgt gacaatgccg gctctgtgtc tttctttcct 960
caggccgaga catgcaaggt gcagtgcaac cgggtgttct gcgacaccat gaacagcctg 1020
accctgccca gcgaggtcaa cctgtgcaac atcgacatct tcaaccccaa gtacgactgc 1080
aagatcatga ccagcaagac cgacgtgtct agctccgtga tcacaagcct gggagccatc 1140

gtgagctgct acggcaagac caagtgcacc gccagcaaca agaatcgggg catcattaag 1200
accttcagca acggctgcga ctacgtgagc aacaagggcg tggatacagt gagcgtgggc 1260
aacaccctgt actacgtgaa taagcaggag ggcaagagcc tgtacgtgaa gggcgagccc 1320
atcatcaact tctacgaccc cctggtgttt cccagctgcg agttttgcgc cagcatcagc 1380
caggtgaacg agaaaatcaa tcagagcctg gccttcatca ggaagagcga cgagctcctg 1440
cacaacgtgg aagataaaat tgaagaaatt ctctccaaaa tttatcatat tgaaaatgaa 1500
attgctagaa ttaaaaaact cattggagaa gctaacgccg gcaagagcac caccaacatc 1560
atgatcacca ccatcatcat cgtgatcatt gtgatcctgc tggccctgat cgccgtgggc 1620
ctgctgctgt actgcaaggc taggagcacc cctgtgaccc tgagcaagga tcagctgagc 1680
ggcatcaata acatcgcctt cagcaac 1707

SEQ ID NO: 140
length 37
molecule type AA
division PAT

Key Location/Qualifiers
source 1..37
/mol_type=protein
/organism=synthetic construct

Sequence
SAIGGYIPEA PRDGQAYVRK DGEWVLLSTF LGGLVPR 37

SEQ ID NO: 141
length 576
molecule type AA
division PAT

Key Location/Qualifiers
source 1..576
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLSA 480
IGGYIPEAPR DGQAYVRKDG EWVLLSTFLG GLVPRHNVNA GKSTTNIMIT TIIIVIIVIL 540
LALIAVGLLL YCKARSTPVT LSKDQLSGIN NIAFSN 576

SEQ ID NO: 142
length 1728
molecule type RNA
division PAT

Key Location/Qualifiers
source 1..1728
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240

caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgagcgcc 1440
atcggcggct acatccccga ggcccccaga gacggccagg cctacgtgag gaaggacggc 1500
gagtgggtgc tgctgagcac cttcctgggc ggcctggtgc ccagacacaa cgtgaacgcc 1560
ggcaagagca ccaccaacat catgatcacc accatcatca tcgtgatcat tgtgatcctg 1620
ctggccctga tcgccgtggg cctgctgctg tactgcaagg ctaggagcac ccctgtgacc 1680
ctgagcaagg atcagctgag cggcatcaat aacatcgcct tcagcaac 1728

SEQ ID NO: 143
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIENITS KIYNITNEIA NITKLIGEAN AGKSTTNIMI TTIIIVIIVI LLALIAVGLL 540
LYCKARSTPV TLSKDQLSGI NNIAFSN 567

SEQ ID NO: 144
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60

tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaagata aaattgaaaa cattacctcc aaaatttata acattaccaa tgaaattgct 1500
aacattacca aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 145
length 576
molecule type AA
division PAT

Key Location/Qualifiers
source 1..576
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLSA 480
IGGYIPEAPN DTQAYVRKDG EWVLLSTFLG GLVPRHNVNA GKSTTNIMIT TIIIVIIVIL 540
LALIAVGLLL YCKARSTPVT LSKDQLSGIN NIAFSN 576

SEQ ID NO: 146
length 1728
molecule type RNA
division PAT

Key Location/Qualifiers
source 1..1728
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgagcgcc 1440
atcggcggct acatccccga ggcccccaac gacacccagg cctacgtgag gaaggacggc 1500
gagtgggtgc tgctgagcac cttcctgggc ggcctggtgc ccagacacaa cgtgaacgcc 1560
ggcaagagca ccaccaacat catgatcacc accatcatca tcgtgatcat tgtgatcctg 1620
ctggccctga tcgccgtggg cctgctgctg tactgcaagg ctaggagcac ccctgtgacc 1680
ctgagcaagg atcagctgag cggcatcaat aacatcgcct tcagcaac 1728

SEQ ID NO: 147
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIENITS KIYHIENEIA RIKKLIGEAN AGKSTTNIMI TTIIIVIIVI LLALIAVGLL 540
LYCKARSTPV TLSKDQLSGI NNIAFSN 567

SEQ ID NO: 148
length 1701
molecule type RNA
division PAT

Key Location/Qualifiers
source 1..1701

/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaagata aaattgaaaa cattacctcc aaaatttatc atattgaaaa tgaaattgct 1500
agaattaaaa aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 149
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360
MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIEEILS KIYNITNEIA RIKKLIGEAN AGKSTTNIMI TTIIIVIIVI LLALIAVGLL 540
LYCKARSTPV TLSKDQLSGI NNIAFSN 567

SEQ ID NO: 150
length 1701
molecule type RNA
division PAT

Key                    Location/Qualifiers
source                 1..1701
                       /mol_type=other RNA
                       /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg   120
agcaagggct acctgggcgc cctgaggacc ggctggtaca caagcgtgat caccatcgag   180
ctgagcaaca tcaaagaaaa caagtgcaac ggcacagacg ccaaagtgaa gctgatcaag   240
caggagctgg acaagtacaa gaacgccgtg actgacctgc agctgctgat gcagtcaaca   300
ccttgtggcg gatctggcgg aggatctggc agcgctatcg cctctggaat tgccgtgtgt   360
aaagtgctgc acctggaggg cgaagtgaac aagatcaaaa gcgccctgct gagcaccaac   420
aaggctgtgg tgagcctgag caatggcgtg agcgtgctga ccttcaaggt gctggacctg   480
aaaaactaca ttgacaagca gctgctcccc atcgtgaaca agcagagctg cagcatcagc   540
aacatcgaga cggtgatcga gtttcagcag aagaacaacc ggctgctgga gatcacccgg   600
gagtttagcg tgaacgctgg cgtgaccacc ccagtgagca cctatatgct gaccaacagc   660
gagctgctga gcctgatcaa cgacatgccc atcaccaacg atcagaagaa gctgatgagc   720
agcaacgtgc agatcgtgag gcagcagagc tacagcatca tgtgcatcat caaggaggag   780
gtgctggcct acgtggtgca gctgcctctg tacggagtga tcgacacccc ctgttggaaa   840
ctgcacacat ctcctctgtg cacaaccaac accaaggagg gcagcaacat ctgcctgaca   900
agaaccgata ggggctggta ctgtgacaat gccggctctg tgtctttctt tcctcaggcc   960
gagacatgca aggtgcagtg caaccgggtg ttctgcgaca ccatgaacag cctgaccctg   1020
cccagcgagg tcaacctgtg caacatcgac atcttcaacc ccaagtacga ctgcaagatc   1080
atgaccagca agaccgacgt gtctagctcc gtgatcacaa gcctgggagc catcgtgagc   1140
tgctacggca agaccaagtg caccgccagc aacaagaatc ggggcatcat taagaccttc   1200
agcaacggct gcgactacgt gagcaacaag ggcgtggata cagtgagcgt gggcaacacc   1260
ctgtactacg tgaataagca ggagggcaag agcctgtacg tgaagggcga gcccatcatc   1320
aacttctacg accccctggt gtttcccagc tgcgagtttt gcgccagcat cagccaggtg   1380
aacgagaaaa tcaatcagag cctggccttc atcaggaaga gcgacgagct cctgcacaac   1440
gtggaagata aaattgaaga aattctctcc aaaatttata acattaccaa tgaaattgct   1500
agaattaaaa aactcattgg agaagctaac gccggcaaga gcaccaccaa catcatgatc   1560
accaccatca tcatcgtgat cattgtgatc ctgctggccc tgatcgccgt gggcctgctg   1620
ctgtactgca aggctaggag cacccctgtg accctgagca aggatcagct gagcggcatc   1680
aataacatcg ccttcagcaa c                                              1701

SEQ ID NO:             151
length                 567
molecule type          AA
division               PAT

Key                    Location/Qualifiers
source                 1..567
                       /mol_type=protein
                       /organism=synthetic construct

Sequence
MELPILKTNA ITTILAAVTL CFASSQNITE EFYQSTCSAV SKGYLGALRT GWYTSVITIE   60
LSNIKENKCN GTDAKVKLIK QELDKYKNAV TDLQLLMQST PCGGSGGGSG SAIASGIAVC   120
KVLHLEGEVN KIKSALLSTN KAVVSLSNGV SVLTFKVLDL KNYIDKQLLP IVNKQSCSIS   180
NIETVIEFQQ KNNRLLEITR EFSVNAGVTT PVSTYMLTNS ELLSLINDMP ITNDQKKLMS   240
SNVQIVRQQS YSIMCIIKEE VLAYVVQLPL YGVIDTPCWK LHTSPLCTTN TKEGSNICLT   300
RTDRGWYCDN AGSVSFFPQA ETCKVQCNRV FCDTMNSLTL PSEVNLCNID IFNPKYDCKI   360
MTSKTDVSSS VITSLGAIVS CYGKTKCTAS NKNRGIIKTF SNGCDYVSNK GVDTVSVGNT   420
LYYVNKQEGK SLYVKGEPII NFYDPLVFPS CEFCASISQV NEKINQSLAF IRKSDELLHN   480
VEDKIEEILS KIYHIENEIA NITKLIGEAN AGKSTTNIMI TTIIIVIIVI LLALIAVGLL   540
LYCKARSTPV TLSKDQLSGI NNIAFSN                                        567

SEQ ID NO:             152
length                 1701

molecule type        RNA
division             PAT

Key                  Location/Qualifiers
source               1..1701
                     /mol_type=other RNA
                     /organism=synthetic construct

Sequence
atggagctgc ccatcctgaa gaccaatgcc attacaacca tcctggccgc cgtgacactg   60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggcgg aggatctggc agcgctatcg cctctggaat tgccgtgtgt  360
aaagtgctgc acctggaggg cgaagtgaac aagatcaaaa gcgccctgct gagcaccaac  420
aaggctgtgg tgagcctgag caatggcgtg agcgtgctga ccttcaaggt gctggacctg  480
aaaaactaca ttgacaagca gctgctcccc atcgtgaaca agcagagctg cagcatcagc  540
aacatcgaga cggtgatcga gtttcagcag aagaacaacc ggctgctgga gatcacccgg  600
gagtttagcg tgaacgctgg cgtgaccacc ccagtgagca cctatatgct gaccaacagc  660
gagctgctga gcctgatcaa cgacatgccc atcaccaacg atcagaagaa gctgatgagc  720
agcaacgtgc agatcgtgag gcagcagagc tacagcatca tgtgcatcat caaggaggag  780
gtgctggcct acgtggtgca gctgcctctg tacggagtga tcgacacccc ctgttggaaa  840
ctgcacacat ctcctctgtg cacaaccaac accaaggagg gcagcaacat ctgcctgaca  900
agaaccgata ggggctggta ctgtgacaat gccggctctg tgtctttctt tcctcaggcc  960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaagata aaattgaaga aattctctcc aaaatttatc atattgaaaa tgaaattgct 1500
aacattacca aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c                                           1701

SEQ ID NO:           153
length               567
molecule type        AA
division             PAT

Key                  Location/Qualifiers
source               1..567
                     /mol_type=protein
                     /organism=synthetic construct

Sequence
MELPILKTNA ITTILAAVTL CFASSQNITE EFYQSTCSAV SKGYLGALRT GWYTSVITIE   60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIS  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360
MTSKTDVSSS VITSLGAIVS CYGKTKCTAS NKNRGIIKTF SNGCDYVSNK GVDTVSVGNT  420
LYYVNKQEGK SLYVKGEPII NFYDPLVFPS CEFCASISQV NEKINQSLAF IRKSDELLHN  480
VEDKIENITS KIYNITNEIA RIKKLIGEAN AGKSTTNIMI TTIIIVIIVI LLALIAVGLL  540
LYCKARSTPV TLSKDQLSGI NNIAFSN                                     567

SEQ ID NO:          154
length               1701
molecule type        RNA
division             PAT

Key                  Location/Qualifiers
source               1..1701
                     /mol_type=other RNA
                     /organism=synthetic construct

Sequence
atggagctgc ccatcctgaa gaccaatgcc attacaacca tcctggccgc cgtgacactg  60
tgtttcgcca gctctcagaa catcaccgag gaattttacc agagcacctg cagcgccgtg  120
agcaagggct acctgggcgc cctgaggacc ggctggtaca caagcgtgat caccatcgag  180
ctgagcaaca tcaaagaaaa caagtgcaac ggcacagacg ccaaagtgaa gctgatcaag  240
caggagctgg acaagtacaa gaacgccgtg actgacctgc agctgctgat gcagtcaaca  300
ccttgtggcg gatctggcgg aggatctggc agcgctatcg cctctggaat tgccgtgtgt  360
aaagtgctgc acctggaggg cgaagtgaac aagatcaaaa gcgccctgct gagcaccaac  420
aaggctgtgg tgagcctgag caatggcgtg agcgtgctga ccttcaaggt gctggacctg  480
aaaaactaca ttgacaagca gctgctcccc atcgtgaaca agcagagctg cagcatcagc  540
aacatcgaga cggtgatcga gtttcagcag aagaacaacc ggctgctgga gatcacccgg  600
gagtttagcg tgaacgctgg cgtgaccacc ccagtgagca cctatatgct gaccaacagc  660
gagctgctga gcctgatcaa cgacatgccc atcaccaacg atcagaagaa gctgatgagc  720
agcaacgtgc agatcgtgag gcagcagagc tacagcatca tgtgcatcat caaggaggag  780
gtgctggcct acgtggtgca gctgcctctg tacggagtga tcgacacccc ctgttggaaa  840
ctgcacacat ctcctctgtg cacaaccaac accaaggagg gcagcaacat ctgcctgaca  900
agaaccgata ggggctggta ctgtgacaat gccggctctg tgtctttctt tcctcaggcc  960
gagacatgca aggtgcagtg caaccgggtg ttctgcgaca ccatgaacag cctgaccctg  1020
cccagcgagg tcaacctgtg caacatcgac atcttcaacc ccaagtacga ctgcaagatc  1080
atgaccagca agaccgacgt gtctagctcc gtgatcacaa gcctgggagc catcgtgagc  1140
tgctacggca agaccaagtg caccgccagc aacaagaatc ggggcatcat taagaccttc  1200
agcaacggct gcgactacgt gagcaacaag ggcgtggata cagtgagcgt gggcaacacc  1260
ctgtactacg tgaataagca ggagggcaag agcctgtacg tgaagggcga gcccatcatc  1320
aacttctacg accccctggt gtttcccagc tgcgagtttt gcgccagcat cagccaggtg  1380
aacgagaaaa tcaatcagag cctggccttc atcaggaaga gcgacgagct cctgcacaac  1440
gtggaagata aaattgaaaa cattacctcc aaaatttata acattaccaa tgaaattgct  1500
agaattaaaa aactcattgg agaagctaac gccggcaaga gcaccaccaa catcatgatc  1560
accaccatca tcatcgtgat cattgtgatc ctgctggccc tgatcgccgt gggcctgctg  1620
ctgtactgca aggctaggag cacccctgtg accctgagca aggatcagct gagcggcatc  1680
aataacatcg ccttcagcaa c                                            1701

SEQ ID NO:          155
length               567
molecule type        AA
division             PAT

Key                  Location/Qualifiers
source               1..567
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIS  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360
MTSKTDVSSS VITSLGAIVS CYGKTKCTAS NKNRGIIKTF SNGCDYVSNK GVDTVSVGNT  420
LYYVNKQEGK SLYVKGEPII NFYDPLVFPS CEFCASISQV NEKINQSLAF IRKSDELLHN  480
VEDKIENITS KIYHIENEIA NITKLIGEAN AGKSTTNIMI TTIIIVIIVI LLALIAVGLL  540

LYCKARSTPV TLSKDQLSGI NNIAFSN 567

SEQ ID NO: 156
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaagata aaattgaaaa cattacctcc aaaatttatc atattgaaaa tgaaattgct 1500
aacattacca aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 157
length 567
molecule type AA
division PAT

Key Location/Qualifiers
source 1..567
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGGSG SAIASGIAVC 120
KVLHLEGEVN KIKSALLSTN KAVVSLSNGV SVLTFKVLDL KNYIDKQLLP IVNKQSCSIS 180
NIETVIEFQQ KNNRLLEITR EFSVNAGVTT PVSTYMLTNS ELLSLINDMP ITNDQKKLMS 240
SNVQIVRQQS YSIMCIIKEE VLAYVVQLPL YGVIDTPCWK LHTSPLCTTN TKEGSNICLT 300
RTDRGWYCDN AGSVSFFPQA ETCKVQCNRV FCDTMNSLTL PSEVNLCNID IFNPKYDCKI 360

MTSKTDVSSS VITSLGAIVS CYGKTKCTAS NKNRGIIKTF SNGCDYVSNK GVDTVSVGNT 420
LYYVNKQEGK SLYVKGEPII NFYDPLVFPS CEFCASISQV NEKINQSLAF IRKSDELLHN 480
VEDKIEEILS KIYNITNEIA NITKLIGEAN AGKSTTNIMI TTIIIVIIVI LLALIAVGLL 540
LYCKARSTPV TLSKDQLSGI NNIAFSN 567

SEQ ID NO: 158
length 1701
molecule type RNA
division PAT

Key Location/Qualifiers
source 1..1701
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcagc 540
aacatcgaga cggtgatcga gtttcagcag aagaacaacc ggctgctgga gatcacccgg 600
gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaagata aaattgaaga aattctctcc aaaatttata acattaccaa tgaaattgct 1500
aacattacca aactcattgg agaagctaac gccggcaaga gcaccaccaa catcatgatc 1560
accaccatca tcatcgtgat cattgtgatc ctgctggccc tgatcgccgt gggcctgctg 1620
ctgtactgca aggctaggag cacccctgtg accctgagca aggatcagct gagcggcatc 1680
aataacatcg ccttcagcaa c 1701

SEQ ID NO: 159
length 37
molecule type AA
division PAT

Key Location/Qualifiers
source 1..37
/mol_type=protein
/organism=synthetic construct

Sequence
SAIGGYIPEA PNDTQAYVRK DGEWVLLSTF LGGLVPR 37

SEQ ID NO: 160

length 46
molecule type AA
division PAT

Key Location/Qualifiers
source 1..46
/mol_type=protein
/organism=synthetic construct

Sequence
EDKIEEILSK IYHIENEIAR IKKLIGEAIY HIENEIARIK KLIGEA 46

SEQ ID NO: 161
length 574
molecule type AA
division PAT

Key Location/Qualifiers
source 1..574
/mol_type=protein
/organism=synthetic construct

Sequence
MELLILKANA ITTILTAVTF CFASGQNITE EFYQSTCSAV SKGYLSALRT GWYTSVITIE 60
LSNIKENKCN GTDAKVKLIK QELDKYKNAV TELQLLMQST PATNNRARRE LPRFMNYTLN 120
NAKKTNVTLS KKRKRRFLGF LLGVGSAIAS GVAVSKVLHL EGEVNKIKSA LLSTNKAVVS 180
LSNGVSVLTS KVLDLKNYID KQLLPIVNKQ SCSISNIETV IEFQQKNNRL LEITREFSVN 240
AGVTTPVSTY MLTNSELLSL INDMPITNDQ KKLMSNNVQI VRQQSYSIMS IIKEEVLAYV 300
VQLPLYGVID TPCWKLHTSP LCTTNTKEGS NICLTRTDRG WYCDNAGSVS FFPQAETCKV 360
QSNRVFCDTM NSLTLPSEVN LCNVDIFNPK YDCKIMTSKT DVSSSVITSL GAIVSCYGKT 420
KCTASNKNRG IIKTFSNGCD YVSNKGVDTV SVGNTLYYVN KQEGKSLYVK GEPIINFYDP 480
LVFPSDEFDA SISQVNEKIN QSLAFIRKSD ELLHNVNAGK STTNIMITTI IIVIIVILLS 540
LIAVGLLLYC KARSTPVTLS KDQLSGINNI AFSN 574

SEQ ID NO: 162
length 574
molecule type AA
division PAT

Key Location/Qualifiers
source 1..574
/mol_type=protein
/organism=synthetic construct

Sequence
MELLILKANA ITTILTAVTF CFASGQNITE EFYQSTCSAV SKGYLSALRT GWYTSVITIE 60
LSNIKENKCN GTDAKVKLIK QELDKYKNAV TELQLLMQST PATNNRARRE LPRFMNYTLN 120
NAKKTNVTLS KKRKRRFLGF LLGVGSAIAS GVAVSKVLHL EGEVNKIKSA LLSTNKAVVS 180
LSNGVSVLTS KVLDLKNYID KQLLPIVNKQ SCSISNIETV IEFQQKNNRL LEITREFSVN 240
AGVTTPVSTY MLTNSELLSL INDMPITNDQ KKLMSNNVQI VRQQSYSIMS IIKEEVLAYV 300
VQLPLYGVID TPCWKLHTSP LCTTNTKEGS NICLTRTDRG WYCDNAGSVS FFPQAETCKV 360
QSNRVFCDTM NSLTLPSEVN LCNVDIFNPK YDCKIMTSKT DVSSSVITSL GAIVSCYGKT 420
KCTASNKNRG IIKTFSNGCD YVSNKGVDTV SVGNTLYYVN KQEGKSLYVK GEPIINFYDP 480
LVFPSDEFDA SISQVNEKIN QSLAFIRKSD ELLHNVNAGK STTNIMITTI IIVIIVILLS 540
LIAVGLLLYC KARSTPVTLS KDQLSGINNI AFSN 574

SEQ ID NO: 163
length 602
molecule type AA
division PAT

Key                 Location/Qualifiers
source              1..602
                    /mol_type=protein
                    /organism=synthetic construct

Sequence
MELLILKANA ITTILTAVTF CFASGQNITE EFYQSTCSAV SKGYLSALRT GWYTSVITIE  60
LSNIKENKCN GTDAKVKLIK QELDKYKNAV TELQLLMQST PATNNRARRE LPRFMNYTLN  120
NAKKTNVTLS KKRKRRFLGF LLGVGSAIAS GVAVCKVLHL EGEVNKIKSA LLSTNKAVVS  180
LSNGVSVLTF KVLDLKNYID KQLLPILNKQ SCSISNIETV IEFQQKNNRL LEITREFSVN  240
AGVTTPVSTY MLTNSELLSL INDMPITNDQ KKLMSNNVQI VRQQSYSIMC IIKEEVLAYV  300
VQLPLYGVID TPCWKLHTSP LCTTNTKEGS NICLTRTDRG WYCDNAGSVS FFPQAETCKV  360
QSNRVFCDTM NSLTLPSEVN LCNVDIFNPK YDCKIMTSKT DVSSSVITSL GAIVSCYGKT  420
KCTASNKNRG IIKTFSNGCD YVSNKGVDTV SVGNTLYYVN KQEGKSLYVK GEPIINFYDP  480
LVFPSDEFDA SISQVNEKIN QSLAFIRKSD ELLHNVEDKI EEILSKIYHI ENEIARIKKL  540
IGEANAGKST TNIMITTIII VIIVILLSLI AVGLLLYCKA RSTPVTLSKD QLSGINNIAF  600
SN                                                                602

SEQ ID NO:          164
length              620
molecule type       AA
division            PAT

Key                 Location/Qualifiers
source              1..620
                    /mol_type=protein
                    /organism=synthetic construct

Sequence
MELLILKANA ITTILTAVTF CFASGQNITE EFYQSTCSAV SKGYLSALRT GWYTSVITIE  60
LSNIKENKCN GTDAKVKLIK QELDKYKNAV TELQLLMQST PATNNRARRE LPRFMNYTLN  120
NAKKTNVTLS KKRKRRFLGF LLGVGSAIAS GVAVCKVLHL EGEVNKIKSA LLSTNKAVVS  180
LSNGVSVLTF KVLDLKNYID KQLLPILNKQ SCSISNIETV IEFQQKNNRL LEITREFSVN  240
AGVTTPVSTY MLTNSELLSL INDMPITNDQ KKLMSNNVQI VRQQSYSIMC IIKEEVLAYV  300
VQLPLYGVID TPCWKLHTSP LCTTNTKEGS NICLTRTDRG WYCDNAGSVS FFPQAETCKV  360
QSNRVFCDTM NSLTLPSEVN LCNVDIFNPK YDCKIMTSKT DVSSSVITSL GAIVSCYGKT  420
KCTASNKNRG IIKTFSNGCD YVSNKGVDTV SVGNTLYYVN KQEGKSLYVK GEPIINFYDP  480
LVFPSDEFDA SISQVNEKIN QSLAFIRKSD ELLHNVEDKI EEILSKIYHI ENEIARIKKL  540
IGEAIYHIEN EIARIKKLIG EANAGKSTTN IMITTIIIVI IVILLSLIAV GLLLYCKARS  600
TPVTLSKDQL SGINNIAFSN                                             620

SEQ ID NO:          165
length              511
molecule type       AA
division            PAT

Key                 Location/Qualifiers
source              1..511
                    /mol_type=protein
                    /organism=synthetic construct

Sequence
MELLILKANA ITTILTAVTF CFASGQNITE EFYQSTCSAV SKGYLSALRT GWYTSVITIE  60
LSNIKENKCN GTDAKVKLIK QELDKYKNAV TELQLLMQST PATGSGSAIC SGVAVCKVLH  120
LEGEVNKIKS ALLSTNKAVV SLSNGVSVLT FKVLDLKNYI DKQLLPILNK QSCSISNIET  180
VIEFQQKNNR LLEITREFSV NAGVTTPVST YMLTNSELLS LINDMPITND QKKLMSNNVQ  240
IVRQQSYSIM CIIKEEVLAY VVQLPLYGVI DTPCWKLHTS PLCTTNTKEG SNICLTRTDR  300
GWYCDNAGSV SFFPQAETCK VQSNRVFCDT MNSRTLPSEV NLCNVDIFNP KYDCKIMTSK  360
TDVSSSVITS LGAIVSCYGK TKCTASNKNR GIIKTFSNGC DYVSNKGVDT VSVGNTLYCV  420
NKQEGKSLYV KGEPIINFYD PLVFPSDEFD ASISQVNEKI NQSLAFIRKS DELLHNVNAG  480
KSTTNIMITT IIIVIIVILL SLIAVGLLLY C                                511

SEQ ID NO:          166
length               585
molecule type        AA
division             PAT

Key                  Location/Qualifiers
source               1..585
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIP  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360
MTSKTDVSSS VITSLGAIVS CYGKTKCTAS NKNRGIIKTF SNGCDYVSNK GVDTVSVGNT  420
LYYVNKQEGK SLYVKGEPII NFYDPLVFPS CEFCASISQV NEKINQSLAF IRKSDELLHN  480
VEDKIEEILS KIYHIENEIA RIKKLIGEAI YHIENEIARI KKLIGEANAG KSTTNIMITT  540
IIIVIIVILL ALIAVGLLLY CKARSTPVTL SKDQLSGINN IAFSN                585

SEQ ID NO:          167
length               478
molecule type        AA
division             PAT

Key                  Location/Qualifiers
source               1..478
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIP  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360
MTSKTDVSSS VITSLGAIVS CYGKTKCTAS NKNRGIIKTF SNGCDYVSNK GVDTVSVGNT  420
LYYVNKQEGK SLYVKGEPII NFYDPLVFPS CEFCASISQV NEKINQSLAF IRKSDELL    478

SEQ ID NO:          168
length               539
molecule type        AA
division             PAT

Key                  Location/Qualifiers
source               1..539
                     /mol_type=protein
                     /organism=synthetic construct

Sequence
MELPILKTNA ITTILAAVTL CFASSQNITE EFYQSTCSAV SKGYLGALRT GWYTSVITIE  60
LSNIKENKCN GTDAKVKLIK QELDKYKNAV TDLQLLMQST PCGGSGGGSG SAIASGIAVC  120
KVLHLEGEVN KIKSALLSTN KAVVSLSNGV SVLTFKVLDL KNYIDKQLLP IVNKQSCSIP  180
NIETVIEFQQ KNNRLLEITR EFSVNAGVTT PVSTYMLTNS ELLSLINDMP ITNDQKKLMS  240
SNVQIVRQQS YSIMCIIKEE VLAYVVQLPL YGVIDTPCWK LHTSPLCTTN TKEGSNICLT  300
RTDRGWYCDN AGSVSFFPQA ETCKVQCNRV FCDTMNSLTL PSEVNLCNID IFNPKYDCKI  360
MTSKTDVSSS VITSLGAIVS CYGKTKCTAS NKNRGIIKTF SNGCDYVSNK GVDTVSVGNT  420
LYYVNKQEGK SLYVKGEPII NFYDPLVFPS DEFDASISQV NEKINQSLAF IRKSDELLHN  480

VNAGKSTTNI MITTIIIVII VILLALIAVG LLLYCKARST PVTLSKDQLS GINNIAFSN 539

SEQ ID NO: 169
length 538
molecule type AA
division PAT
Key Location/Qualifiers
source 1..538
/mol_type=protein
/organism=synthetic construct
Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGGSGS AIASGIAVCK 120
VLHLEGEVNK IKSALLSTNK AVVSLSNGVS VLTFKVLDLK NYIDKQLLPI VNKQSCSIPN 180
IETVIEFQQK NNRLLEITRE FSVNAGVTTP VSTYMLTNSE LLSLINDMPI TNDQKKLMSS 240
NVQIVRQQSY SIMCIIKEEV LAYVVQLPLY GVIDTPCWKL HTSPLCTTNT KEGSNICLTR 300
TDRGWYCDNA GSVSFFPQAE TCKVQCNRVF CDTMNSLTLP SEVNLCNIDI FNPKYDCKIM 360
TSKTDVSSSV ITSLGAIVSC YGKTKCTASN KNRGIIKTFS NGCDYVSNKG VDTVSVGNTL 420
YYVNKQEGKS LYVKGEPIIN FYDPLVFPSD EFDASISQVN EKINQSLAFI RKSDELLHNV 480
NAGKSTTNIM ITTIIIVIIV ILLALIAVGL LLYCKARSTP VTLSKDQLSG INNIAFSN 538

SEQ ID NO: 170
length 538
molecule type AA
division PAT
Key Location/Qualifiers
source 1..538
/mol_type=protein
/organism=synthetic construct
Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PAGGSGGSGS AIASGIAVCK 120
VLHLEGEVNK IKSALLSTNK AVVSLSNGVS VLTFKVLDLK NYIDKQLLPI VNKQSCSIPN 180
IETVIEFQQK NNRLLEITRE FSVNAGVTTP VSTYMLTNSE LLSLINDMPI TNDQKKLMSS 240
NVQIVRQQSY SIMCIIKEEV LAYVVQLPLY GVIDTPCWKL HTSPLCTTNT KEGSNICLTR 300
TDRGWYCDNA GSVSFFPQAE TCKVQSNRVF CDTMNSLTLP SEVNLCNIDI FNPKYDCKIM 360
TSKTDVSSSV ITSLGAIVSC YGKTKCTASN KNRGIIKTFS NGCDYVSNKG VDTVSVGNTL 420
YYVNKQEGKS LYVKGEPIIN FYDPLVFPSC EFCASISQVN EKINQSLAFI RKSDELLHNV 480
NAGKSTTNIM ITTIIIVIIV ILLALIAVGL LLYCKARSTP VTLSKDQLSG INNIAFSN 538

SEQ ID NO: 171
length 537
molecule type AA
division PAT
Key Location/Qualifiers
source 1..537
/mol_type=protein
/organism=synthetic construct
Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSGSA IASGIAVCKV 120
LHLEGEVNKI KSALLSTNKA VVSLSNGVSV LTFKVLDLKN YIDKQLLPIV NKQSCSIPNI 180
ETVIEFQQKN NRLLEITREF SVNAGVTTPV STYMLTNSEL LSLINDMPIT NDQKKLMSSN 240
VQIVRQQSYS IMCIIKEEVL AYVVQLPLYG VIDTPCWKLH TSPLCTTNTK EGSNICLTRT 300
DRGWYCDNAG SVSFFPQAET CKVQCNRVFC DTMNSLTLPS EVNLCNIDIF NPKYDCKIMT 360

SKTDVSSSVI TSLGAIVSCY GKTKCTASNK NRGIIKTFSN GCDYVSNKGV DTVSVGNTLY 420
YVNKQEGKSL YVKGEPIINF YDPLVFPSDE FDASISQVNE KINQSLAFIR KSDELLHNVN 480
AGKSTTNIMI TTIIIVIIVI LLALIAVGLL LYCKARSTPV TLSKDQLSGI NNIAFSN 537

SEQ ID NO: 172
length 537
molecule type AA
division PAT

Key Location/Qualifiers
source 1..537
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PAGGSGSGSA IASGIAVCKV 120
LHLEGEVNKI KSALLSTNKA VVSLSNGVSV LTFKVLDLKN YIDKQLLPIV NKQSCSIPNI 180
ETVIEFQQKN NRLLEITREF SVNAGVTTPV STYMLTNSEL LSLINDMPIT NDQKKLMSSN 240
VQIVRQQSYS IMCIIKEEVL AYVVQLPLYG VIDTPCWKLH TSPLCTTNTK EGSNICLTRT 300
DRGWYCDNAG SVSFFPQAET CKVQSNRVFC DTMNSLTLPS EVNLCNIDIF NPKYDCKIMT 360
SKTDVSSSVI TSLGAIVSCY GKTKCTASNK NRGIIKTFSN GCDYVSNKGV DTVSVGNTLY 420
YVNKQEGKSL YVKGEPIINF YDPLVFPSCE FCASISQVNE KINQSLAFIR KSDELLHNVN 480
AGKSTTNIMI TTIIIVIIVI LLALIAVGLL LYCKARSTPV TLSKDQLSGI NNIAFSN 537

SEQ ID NO: 173
length 536
molecule type AA
division PAT

Key Location/Qualifiers
source 1..536
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGSGSGSAI ASGIAVCKVL 120
HLEGEVNKIK SALLSTNKAV VSLSNGVSVL TFKVLDLKNY IDKQLLPIVN KQSCSIPNIE 180
TVIEFQQKNN RLLEITREFS VNAGVTTPVS TYMLTNSELL SLINDMPITN DQKKLMSSNV 240
QIVRQQSYSI MCIIKEEVLA YVVQLPLYGV IDTPCWKLHT SPLCTTNTKE GSNICLTRTD 300
RGWYCDNAGS VSFFPQAETC KVQCNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSDEF DASISQVNEK INQSLAFIRK SDELLHNVNA 480
GKSTTNIMIT TIIIVIIVIL LALIAVGLLL YCKARSTPVT LSKDQLSGIN NIAFSN 536

SEQ ID NO: 174
length 536
molecule type AA
division PAT

Key Location/Qualifiers
source 1..536
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PAGSGSGSAI ASGIAVCKVL 120
HLEGEVNKIK SALLSTNKAV VSLSNGVSVL TFKVLDLKNY IDKQLLPIVN KQSCSIPNIE 180
TVIEFQQKNN RLLEITREFS VNAGVTTPVS TYMLTNSELL SLINDMPITN DQKKLMSSNV 240

QIVRQQSYSI MCIIKEEVLA YVVQLPLYGV IDTPCWKLHT SPLCTTNTKE GSNICLTRTD 300
RGWYCDNAGS VSFFPQAETC KVQSNRVFCD TMNSLTLPSE VNLCNIDIFN PKYDCKIMTS 360
KTDVSSSVIT SLGAIVSCYG KTKCTASNKN RGIIKTFSNG CDYVSNKGVD TVSVGNTLYY 420
VNKQEGKSLY VKGEPIINFY DPLVFPSCEF CASISQVNEK INQSLAFIRK SDELLHNVNA 480
GKSTTNIMIT TIIIVIIVIL LALIAVGLLL YCKARSTPVT LSKDQLSGIN NIAFSN 536

SEQ ID NO: 175
length 535
molecule type AA
division PAT

Key Location/Qualifiers
source 1..535
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PCGGSGSAIA SGIAVCKVLH 120
LEGEVNKIKS ALLSTNKAVV SLSNGVSVLT FKVLDLKNYI DKQLLPIVNK QSCSIPNIET 180
VIEFQQKNNR LLEITREFSV NAGVTTPVST YMLTNSELLS LINDMPITND QKKLMSSNVQ 240
IVRQQSYSIM CIIKEEVLAY VVQLPLYGVI DTPCWKLHTS PLCTTNTKEG SNICLTRTDR 300
GWYCDNAGSV SFFPQAETCK VQCNRVFCDT MNSLTLPSEV NLCNIDIFNP KYDCKIMTSK 360
TDVSSSVITS LGAIVSCYGK TKCTASNKNR GIIKTFSNGC DYVSNKGVDT VSVGNTLYYV 420
NKQEGKSLYV KGEPIINFYD PLVFPSDEFD ASISQVNEKI NQSLAFIRKS DELLHNVNAG 480
KSTTNIMITT IIIVIIVILL ALIAVGLLLY CKARSTPVTL SKDQLSGINN IAFSN 535

SEQ ID NO: 176
length 535
molecule type AA
division PAT

Key Location/Qualifiers
source 1..535
/mol_type=protein
/organism=synthetic construct

Sequence
MELPILKTNA ITTILAAVTL CFASSQNITE EFYQSTCSAV SKGYLGALRT GWYTSVITIE 60
LSNIKENKCN GTDAKVKLIK QELDKYKNAV TDLQLLMQST PAGGSGSAIA SGIAVCKVLH 120
LEGEVNKIKS ALLSTNKAVV SLSNGVSVLT FKVLDLKNYI DKQLLPIVNK QSCSIPNIET 180
VIEFQQKNNR LLEITREFSV NAGVTTPVST YMLTNSELLS LINDMPITND QKKLMSSNVQ 240
IVRQQSYSIM CIIKEEVLAY VVQLPLYGVI DTPCWKLHTS PLCTTNTKEG SNICLTRTDR 300
GWYCDNAGSV SFFPQAETCK VQSNRVFCDT MNSLTLPSEV NLCNIDIFNP KYDCKIMTSK 360
TDVSSSVITS LGAIVSCYGK TKCTASNKNR GIIKTFSNGC DYVSNKGVDT VSVGNTLYYV 420
NKQEGKSLYV KGEPIINFYD PLVFPSCEFC ASISQVNEKI NQSLAFIRKS DELLHNVNAG 480
KSTTNIMITT IIIVIIVILL ALIAVGLLLY CKARSTPVTL SKDQLSGINN IAFSN 535

SEQ ID NO: 177
length 1722
molecule type RNA
division PAT

Key Location/Qualifiers
source 1..1722
/mol_type=other RNA
/organism=synthetic construct

Sequence
atggagctgc tgatcctgaa ggccaacgcc atcacaacaa tcctgaccgc tgtgaccttc 60
tgtttcgcca gcggccagaa catcaccgag gagttttatc agagcacctg tagcgccgtg 120

agcaagggat acctgtctgc cctgagaacc ggctggtaca ccagcgtgat caccatcgag 180
ctgagcaaca tcaaggagaa caagtgcaac ggcaccgacg ccaaggtgaa gctgatcaaa 240
caggagctgg acaagtacaa gaacgccgtg accgagctgc agctgctcat gcagagcacc 300
cctgctacca acaatcgggc tagacgggag ctgccccggt tcatgaatta caccctgaac 360
aacgccaaga agaccaacgt gacactgtct aaaaagagga agcggagatt cctgggcttc 420
ctgctgggag tgggctctgc tattgcttct ggagtggccg tgtctaaggt gctgcacctg 480
gagggagagg tgaacaagat caagagcgcc ctgctgtcca ccaataaggc cgtggtgagc 540
ctgagcaatg gcgtgagcgt gctgacctct aaggtcctgg acctgaagaa ttacatcgac 600
aagcagctgc tgcccatcgt gaacaagcag tcatgcagca tcagcaacat cgagacggtg 660
atcgagtttc agcagaagaa caaccggctg ctggagatca cccgggagtt tagcgtgaac 720
gccggcgtga ccacacccgt gagcacctac atgctgacca acagcgagct gctgtccctg 780
atcaacgaca tgcccatcac caacgaccag aaaaagctga tgagcaacaa cgtgcagatc 840
gtgaggcagc agagctacag catcatgagc atcatcaaag aggaggtgct ggcctacgtg 900
gtgcagctgc ctctgtacgg cgtgatcgac accccttgct ggaagctgca caccagccct 960
ctgtgcacaa ccaataccaa ggagggcagc aatatctgtc tgaccagaac cgataggggc 1020
tggtattgtg acaatgccgg cagcgtgtct ttcttcccac aggccgaaac ctgcaaggtg 1080
cagagcaaca gagtgttctg cgacaccatg aacagcctga ccctgcccag cgaggtcaac 1140
ctgtgcaatg tggacatctt caaccccaag tacgactgca agatcatgac cagcaagacc 1200
gacgtgagca gcagcgtgat taccagcctg ggcgccatcg tgagctgcta cggaaaaacc 1260
aagtgcaccg ccagcaacaa gaacaggggc atcattaaga ccttcagcaa cggctgcgac 1320
tacgtgagca ataagggcgt ggacaccgtg tccgtgggca acacactgta ctacgtgaat 1380
aagcaggagg gcaagagcct gtacgtgaag ggcgagccca tcatcaactt ctacgacccc 1440
ctggtgttcc cctctgacga gtttgacgcc agcatctccc aggtgaacga gaagatcaac 1500
cagagcctgg ccttcatccg gaaaagcgat gagctgctgc acaacgtcaa cgccggcaag 1560
agcaccacca acatcatgat caccaccatc atcatcgtga tcattgtgat cctgctgagc 1620
ctgatcgccg tgggcctgct gctgtactgc aaggccaggt ctacccccgt gacactcagc 1680
aaggatcagc tgagcggcat caacaatatc gccttctcca ac 1722

SEQ ID NO: 178
length 1722
molecule type RNA
division PAT

Key Location/Qualifiers
source 1..1722
/mol_type=other RNA
/organism=synthetic construct

Sequence
atggagctgc tgatcctgaa ggccaacgcc atcacaacaa tcctgaccgc tgtgaccttc 60
tgtttcgcca gcggccagaa catcaccgag gagttttatc agagcacctg tagcgccgtg 120
agcaagggat acctgtctgc cctgagaacc ggctggtaca ccagcgtgat caccatcgag 180
ctgagcaaca tcaaggagaa caagtgcaac ggcaccgacg ccaaggtgaa gctgatcaaa 240
caggagctgg acaagtacaa gaacgccgtg accgagctgc agctgctcat gcagagcacc 300
cctgctacca acaatcgggc tagacgggag ctgccccggt tcatgaatta caccctgaac 360
aacgccaaga agaccaacgt gacactgtct aaaaagagga agcggagatt cctgggcttc 420
ctgctgggag tgggctctgc tattgcttct ggagtggccg tgtctaaggt gctgcacctg 480
gagggagagg tgaacaagat caagagcgcc ctgctgtcca ccaataaggc cgtggtgagc 540
ctgagcaatg gcgtgagcgt gctgacctct aaggtcctgg acctgaagaa ttacatcgac 600
aagcagctgc tgcccatcgt gaacaagcag tcatgcagca tcagcaacat cgagacggtg 660
atcgagtttc agcagaagaa caaccggctg ctggagatca cccgggagtt tagcgtgaac 720
gccggcgtga ccacacccgt gagcacctac atgctgacca acagcgagct gctgtccctg 780
atcaacgaca tgcccatcac caacgaccag aaaaagctga tgagcaacaa cgtgcagatc 840
gtgaggcagc agagctacag catcatgagc atcatcaaag aggaggtgct ggcctacgtg 900
gtgcagctgc ctctgtacgg cgtgatcgac accccttgct ggaagctgca caccagccct 960
ctgtgcacaa ccaataccaa ggagggcagc aatatctgtc tgaccagaac cgataggggc 1020
tggtattgtg acaatgccgg cagcgtgtct ttcttcccac aggccgaaac ctgcaaggtg 1080
cagagcaaca gagtgttctg cgacaccatg aacagcctga ccctgcccag cgaggtcaac 1140
ctgtgcaatg tggacatctt caaccccaag tacgactgca agatcatgac cagcaagacc 1200
gacgtgagca gcagcgtgat taccagcctg ggcgccatcg tgagctgcta cggaaaaacc 1260

aagtgcaccg ccagcaacaa gaacaggggc atcattaaga ccttcagcaa cggctgcgac 1320
tacgtgagca ataagggcgt ggacaccgtg tccgtgggca acacactgta ctacgtgaat 1380
aagcaggagg gcaagagcct gtacgtgaag ggcgagccca tcatcaactt ctacgacccc 1440
ctggtgttcc cctctgacga gtttgacgcc agcatctccc aggtgaacga gaagatcaac 1500
cagagcctgg ccttcatccg gaaaagcgat gagctgctgc acaacgtcaa cgccggcaag 1560
agcaccacca acatcatgat caccaccatc atcatcgtga tcattgtgat cctgctgagc 1620
ctgatcgccg tgggcctgct gctgtactgc aaggccaggt ctacccccgt gacactcagc 1680
aaggatcagc tgagcggcat caacaatatc gccttctcca ac 1722

SEQ ID NO: 179
length 1806
molecule type RNA
division PAT

Key Location/Qualifiers
source 1..1806
/mol_type=other RNA
/organism=synthetic construct

Sequence
atggagctgc tcatcctgaa ggccaacgcc atcaccacca tcctgaccgc cgtgacattt 60
tgtttcgcca gcggccagaa catcaccgag gagttttacc agagcacctg ctctgccgtg 120
agcaagggct acctgtctgc cctgagaaca ggctggtaca ccagcgtgat taccatcgag 180
ctgagcaaca tcaaggagaa caagtgcaac ggcaccgacg ccaaggtgaa gctgatcaag 240
caggagctgg acaagtacaa gaatgccgtg accgagctgc agctgctgat gcagagcaca 300
cctgccacca acaacagggc caggagagag ctgccccggt tcatgaatta caccctgaac 360
aacgccaaga agaccaacgt gaccctgagc aagaagagaa agaggcggtt cctgggattt 420
ctgctgggag tgggctctgc tattgcttct ggagtggctg tgtgtaaagt gctgcacctg 480
gagggagagg tgaacaagat caagagcgcc ctgctgtcta ccaacaaggc cgtggtgagc 540
ctgagcaacg gcgtgagcgt gctgaccttc aaggtgctgg acctgaagaa ttacatcgac 600
aagcagctgc tccccatcct gaacaagcag agctgctcca tcagcaacat cgagacagtg 660
atcgagtttc agcagaagaa caaccggctg ctggagatca cccgggagtt tagcgtgaac 720
gctggcgtga ccacccccgt gagcacctac atgctgacca actctgagct gctctccctg 780
atcaacgaca tgcccatcac caacgaccag aagaagctga tgtccaacaa cgtgcagatc 840
gtgcggcagc agagctacag catcatgtgt atcatcaaag aggaggtgct ggcctacgtg 900
gtgcagctgc ctctgtacgg cgtgatcgac accccatgct ggaagctgca cacctctcct 960
ctgtgtacca caaacaccaa ggagggcagc aatatctgcc tgaccagaac cgatagaggc 1020
tggtattgcg acaatgccgg cagcgtgagc ttctttcctc aggccgagac atgcaaggtg 1080
cagagcaaca gggtgttttg cgacaccatg aacagcctga ccctgcccag cgaggtgaac 1140
ctgtgcaacg tggacatctt caaccccaag tacgactgca agatcatgac cagcaagacc 1200
gacgtgtcta gctctgtgat cacctctctg ggcgccatcg tgagctgtta cggcaagacc 1260
aagtgcaccg ccagcaacaa gaatcggggc atcatcaaga ccttcagcaa cggctgcgac 1320
tacgtgagca acaagggcgt ggataccgtg tctgtgggca ataccctgta ctacgtgaat 1380
aagcaggagg gcaagagcct gtacgtgaag ggcgagccca tcatcaactt ctacgacccc 1440
ctggtgttcc ccagcgacga gtttgacgcc agcatcagcc aggtgaatga gaaaatcaac 1500
cagagcctgg ccttcatccg caagagcgac gagctgctgc acaacgtgga ggacaagatc 1560
gaggagatcc tgagcaagat ctaccacatc gagaacgaga tcgccaggat caagaagctg 1620
atcggcgagg ccaacgccgg caagagcacc accaacatca tgatcacaac catcatcatc 1680
gtgattatcg tgatcctgct gagcctgatc gccgtgggcc tgctgctgta ctgtaaggcc 1740
agaagcaccc ctgtcacact gagcaaggat cagctgagcg gcatcaataa catcgccttc 1800
tccaac 1806

SEQ ID NO: 180
length 1860
molecule type RNA
division PAT

Key Location/Qualifiers
source 1..1860
/mol_type=other RNA

/organism=synthetic construct

Sequence
atggagctgc tcatcctgaa ggccaacgcc atcaccacca tcctgaccgc cgtgacattt 60
tgtttcgcca gcggccagaa catcaccgag gagttttacc agagcacctg ctctgccgtg 120
agcaagggct acctgtctgc cctgagaaca ggctggtaca ccagcgtgat taccatcgag 180
ctgagcaaca tcaaggagaa caagtgcaac ggcaccgacg ccaaggtgaa gctgatcaag 240
caggagctgg acaagtacaa gaatgccgtg accgagctgc agctgctgat gcagagcaca 300
cctgccacca acaacagggc caggagagag ctgccccggt tcatgaatta caccctgaac 360
aacgccaaga agaccaacgt gaccctgagc aagaagagaa agaggcggtt cctgggattt 420
ctgctgggag tgggctctgc tattgcttct ggagtggctg tgtgtaaagt gctgcacctg 480
gagggagagg tgaacaagat caagagcgcc ctgctgtcta ccaacaaggc cgtggtgagc 540
ctgagcaacg gcgtgagcgt gctgaccttc aaggtgctgg acctgaagaa ttacatcgac 600
aagcagctgc tccccatcct gaacaagcag agctgctcca tcagcaacat cgagacagtg 660
atcgagtttc agcagaagaa caaccggctg ctggagatca cccgggagtt tagcgtgaac 720
gctggcgtga ccacccccgt gagcacctac atgctgacca actctgagct gctctccctg 780
atcaacgaca tgcccatcac caacgaccag aagaagctga tgtccaacaa cgtgcagatc 840
gtgcggcagc agagctacag catcatgtgt atcatcaaag aggaggtgct ggcctacgtg 900
gtgcagctgc ctctgtacgg cgtgatcgac accccatgct ggaagctgca cacctctcct 960
ctgtgtacca caaacaccaa ggagggcagc aatatctgcc tgaccagaac cgatagaggc 1020
tggtattgcg acaatgccgg cagcgtgagc ttctttcctc aggccgagac atgcaaggtg 1080
cagagcaaca gggtgttttg cgacaccatg aacagcctga ccctgcccag cgaggtgaac 1140
ctgtgcaacg tggacatctt caaccccaag tacgactgca agatcatgac cagcaagacc 1200
gacgtgtcta gctctgtgat cacctctctg ggcgccatcg tgagctgtta cggcaagacc 1260
aagtgcaccg ccagcaacaa gaatcggggc atcatcaaga ccttcagcaa cggctgcgac 1320
tacgtgagca acaagggcgt ggataccgtg tctgtgggca ataccctgta ctacgtgaat 1380
aagcaggagg gcaagagcct gtacgtgaag ggcgagccca tcatcaactt ctacgacccc 1440
ctggtgttcc ccagcgacga gtttgacgcc agcatcagcc aggtgaatga gaaaatcaac 1500
cagagcctgg ccttcatccg caagagcgac gagctgctgc acaacgtgga ggacaagatc 1560
gaggagatcc tgagcaagat ctaccacatc gagaacgaga tcgccaggat caagaagctg 1620
atcggcgagg ccatctacca catcgagaac gagatcgcca ggatcaagaa gctgatcggc 1680
gaggccaacg ccggcaagag caccaccaac atcatgatca caaccatcat catcgtgatt 1740
atcgtgatcc tgctgagcct gatcgccgtg ggcctgctgc tgtactgtaa ggccagaagc 1800
acccctgtca cactgagcaa ggatcagctg agcggcatca ataacatcgc cttctccaac 1860

SEQ ID NO: 181
length 1533
molecule type RNA
division PAT

Key Location/Qualifiers
source 1..1533
/mol_type=other RNA
/organism=synthetic construct

Sequence
atggagctgc tgatcctgaa ggccaacgcc attacaacca tcctgaccgc cgtgaccttc 60
tgcttcgcta gcgggcagaa catcaccgag gagttctatc agagcacctg cagcgccgtg 120
agcaagggct acctgagcgc cctgagaacc ggctggtaca caagcgtgat taccatcgag 180
ctgagcaaca tcaaggagaa caagtgcaac ggcaccgacg ccaaggtgaa gctgatcaag 240
caagagctgg acaagtacaa gaacgccgtg accgagctgc agctgctgat gcagagcacc 300
cccgccaccg gcagcgggag cgccatctgc agcggcgtgg ccgtgtgcaa ggtgctgcac 360
ctggagggcg aggtgaacaa gatcaagagc gccctgctga gcaccaacaa ggccgtggtg 420
agcctgagca acggcgtgag cgtgctgacc ttcaaggtgc tggacctgaa gaactacatc 480
gacaagcagc tgctgcccat cctgaacaag cagagctgca gcatcagcaa catcgagacc 540
gtgatcgagt ttcagcagaa gaacaacaga ctgctggaga tcacaagaga gttcagcgtc 600
aatgccggcg tgaccacccc cgtgagcacc tacatgctga ccaacagcga gctgctctcc 660
ctgattaacg acatgcccat caccaacgat cagaagaagc tgatgagcaa caacgtgcag 720
atcgtgagac agcagagcta cagcatcatg tgcatcatca aggaagaggt gctggcctac 780
gtggtgcagc tgcccctgta cggcgtgatc gacaccccct gctggaagct gcacacaagc 840

cccctgtgca ccaccaacac caaggagggc agcaacatct gcctgacaag aaccgacaga 900
ggctggtact gcgacaacgc cggcagcgtg agcttcttcc cccaagccga gacctgcaag 960
gtgcagagca acagagtgtt ctgcgacacc atgaacagcc gtaccctgcc tagcgaggtg 1020
aacctgtgca acgtggacat cttcaacccc aagtacgact gcaagatcat gacaagcaag 1080
accgacgtga gcagctccgt gatcacaagc ctcggcgcca tcgtgagctg ctacggcaag 1140
accaagtgca ccgctagcaa caagaacaga ggcatcatca agaccttcag caacggctgc 1200
gactacgtga gcaacaaggg cgtggacacc gtgagcgtgg gcaacaccct gtactgcgtg 1260
aacaagcaag agggcaagag cctgtacgtg aagggcgagc ccatcatcaa cttctacgac 1320
cccctggtgt tccctagcga cgagttcgac gctagcatca gccaagtgaa cgagaagatc 1380
aatcagagcc tggccttcat cagaaagagc gacgagctgc tgcacaacgt gaatgctggc 1440
aaaagcacca caaacatcat gatcacaaca atcattatcg tgatcatcgt gatcctcctg 1500
agcctgatcg ccgtgggcct gctcctgtac tgc 1533

SEQ ID NO: 182
length 1701
molecule type RNA
division PAT

Key Location/Qualifiers
source 1..1701
/mol_type=other RNA
/organism=synthetic construct

Sequence
atggagctgc ccatcctgaa gaccaatgcc atcacaacca tcctggccgc cgtgacactg 60
tgtttcgcca gcagccagaa tatcaccgag gagttttacc agagcacctg cagcgccgtg 120
agcaagggct atctgggcgc tctgaggaca ggctggtaca ccagcgtgat taccatcgag 180
ctgagcaata tcaaggagaa caagtgcaac ggcaccgacg ccaaggtgaa gctgattaag 240
caggagctgg acaagtacaa gaacgccgtg accgatctgc agctgctgat gcagagcaca 300
ccttgtggcg gatctggagg aggatctggc tctgctatcg ccagcggaat tgccgtgtgt 360
aaagtgctgc acctggaggg agaggtgaat aagatcaaaa gcgccctgct gagcaccaac 420
aaggccgtgg tgagcctgag caatggcgtg agcgtgctga ccttcaaggt gctggacctg 480
aagaattaca tcgacaagca gctgctcccc atcgtgaaca agcagagctg cagcatcccc 540
aacatcgaga cagtgatcga gtttcagcag aagaacaacc ggctgctgga gatcacccgg 600
gagtttagcg tgaatgccgg cgtgaccacc ccagtgagca cctacatgct gaccaacagc 660
gagctgctga gcctgatcaa cgacatgcct atcaccaacg accagaagaa gctcatgagc 720
agcaacgtgc agatcgtgag gcagcagagc tacagcatca tgtgcatcat caaggaggag 780
gtgctggcct atgtggtgca gctgcctctg tacggcgtga tcgatacccc ctgctggaag 840
ctgcacacat ctcctctgtg cacaaccaat accaaggagg gcagcaatat ctgcctgacc 900
agaaccgata ggggctggta ctgtgacaat gccggcagcg tgagcttttt ccctcaggcc 960
gagacatgta aggtgcagtg caatcgggtg ttctgcgaca ccatgaacag cctgaccctg 1020
cccagcgagg tgaacctgtg caacatcgac atcttcaacc ccaagtacga ctgcaagatc 1080
atgaccagca agaccgatgt gagcagcagc gtgatcacaa gcctgggagc catcgtgagc 1140
tgctacggca agaccaagtg caccgccagc aacaagaacc ggggcatcat taagaccttc 1200
agcaacggct gcgactacgt gagcaacaag ggcgtggata cagtgagcgt gggcaatacc 1260
ctgtactacg tgaataagca ggaaggcaag agcctgtacg tgaagggcga gcccatcatc 1320
aacttctacg accccctggt gttccccagc tgcgagtttt gcgccagcat cagccaggtg 1380
aacgagaaga tcaaccagag cctggccttc atcaggaaga gcgatgagct gctgcacaac 1440
gtggaggaca agatcgagga gatcctgagc aagatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg acactgagca aggatcagct gagcggcatc 1680
aacaatatcg ccttctccaa c 1701

SEQ ID NO: 183
length 1701
molecule type RNA
division PAT

Key Location/Qualifiers

source                1..1701
                      /mol_type=other RNA
                      /organism=synthetic construct

Sequence
atggagctgc ccatcctgaa gaccaatgcc atcacaacca tcctggccgc cgtgacactg   60
tgtttcgcca gcagccagaa tatcaccgag gagttttacc agagcacctg cagcgccgtg   120
agcaagggct atctgggcgc tctgaggaca ggctggtaca ccagcgtgat taccatcgag   180
ctgagcaata tcaaggagaa caagtgcaac ggcaccgacg ccaaggtgaa gctgattaag   240
caggagctgg acaagtacaa gaacgccgtg accgatctgc agctgctgat gcagagcaca   300
ccttgtggcg gatctggagg aggatctggc tctgctatcg ccagcggaat tgccgtgtgt   360
aaagtgctgc acctggaggg agaggtgaat aagatcaaaa gcgccctgct gagcaccaac   420
aaggccgtgg tgagcctgag caatggcgtg agcgtgctga ccttcaaggt gctggacctg   480
aagaattaca tcgacaagca gctgctcccc atcgtgaaca agcagagctg cagcatcccc   540
aacatcgaga cagtgatcga gtttcagcag aagaacaacc ggctgctgga gatcacccgg   600
gagtttagcg tgaatgccgg cgtgaccacc ccagtgagca cctacatgct gaccaatagc   660
gagctgctga gcctgatcaa cgacatgccc atcaccaacg accagaagaa gctcatgagc   720
agcaacgtgc agatcgtgag gcagcagagc tacagcatca tgtgcatcat caaggaggag   780
gtgctggcct atgtggtgca gctgcctctg tacggcgtga tcgatacccc ctgctggaag   840
ctgcacacat ctcctctgtg cacaaccaat accaaggagg gcagcaatat ctgcctgacc   900
agaaccgata ggggctggta ctgtgacaat gccggcagcg tgagcttttt ccctcaggcc   960
gagacatgta aggtgcagtg caatcgggtg ttctgcgaca ccatgaacag cctgaccctg   1020
cccagcgagg tgaacctgtg caacatcgac atcttcaacc ccaagtacga ctgcaagatc   1080
atgaccagca agaccgatgt gagcagcagc gtgatcacaa gcctgggagc catcgtgagc   1140
tgctacggca agaccaagtg caccgccagc aacaagaacc ggggcatcat taagaccttc   1200
agcaacggct gcgactacgt gagcaacaag ggcgtggata cagtgagcgt gggcaatacc   1260
ctgtactacg tgaataagca ggaaggcaag agcctgtacg tgaagggcga gcccatcatc   1320
aacttctacg accccctggt gttccccagc tgcgagtttt gcgccagcat cagccaggtg   1380
aacgagaaga tcaaccagag cctggccttc atcaggaaga gcgatgagct gctgcacaac   1440
gtggaggaca agatcgagga gatcctgagc aagatctacc acatcgagaa cgagatcgcc   1500
cggatcaaga agctgatcgg cgaggccaac gccggcaaga gcaccaccaa catcatgatc   1560
accaccatca tcatcgtgat cattgtgatc ctgctggccc tgatcgccgt gggcctgctg   1620
ctgtactgta aggccaggtc tacacctgtg acactgagca aggatcagct gagcggcatc   1680
aacaatatcg ccttctccaa c                                              1701

SEQ ID NO:            184
length                1701
molecule type         RNA
division              PAT

Key                   Location/Qualifiers
source                1..1701
                      /mol_type=other RNA
                      /organism=synthetic construct

Sequence
atggagctgc ccatcctgaa gaccaatgcc atcacaacca tcctggccgc cgtgacactg   60
tgtttcgcca gcagccagaa tatcaccgag gagttttacc agagcacctg cagcgccgtg   120
agcaagggct atctgggcgc tctgaggaca ggctggtaca ccagcgtgat taccatcgag   180
ctgagcaata tcaaggagaa caagtgcaac ggcaccgacg ccaaggtgaa gctgattaag   240
caggagctgg acaagtacaa gaacgccgtg accgatctgc agctgctgat gcagagcaca   300
ccttgtggcg gatctggagg aggatctggc agcgctatcg cctctggcat tgccgtgtgt   360
aaagtgctgc acctggaagg cgaagtgaat aagatcaaaa gcgccctgct gagcaccaac   420
aaggccgtgg tgagcctgag caatggcgtg agcgtgctga ccttcaaggt gctggacctg   480
aagaattaca tcgacaagca gctgctcccc atcgtgaaca agcagagctg cagcatcccc   540
aacatcgaga cagtgatcga gtttcagcag aagaacaacc ggctgctgga gatcacccgg   600
gagtttagcg tgaatgccgg cgtgaccacc ccagtgagca cctacatgct gaccaatagc   660
gagctgctga gcctgatcaa cgacatgccc atcaccaacg accagaagaa gctcatgagc   720
agcaacgtgc agatcgtgag gcagcagagc tacagcatca tgtgcatcat caaggaggag   780
gtgctggcct atgtggtgca gctgcctctg tacggcgtga tcgatacccc ctgctggaag   840

ctgcacacat ctcctctgtg cacaaccaat accaaggagg gcagcaatat ctgcctgacc 900
agaaccgata ggggctggta ctgtgacaat gccggcagcg tgagcttttt ccctcaggcc 960
gagacatgta aggtgcagtg caatcgggtg ttctgcgaca ccatgaacag cctgaccctg 1020
cccagcgagg tgaacctgtg caacatcgac atcttcaacc ccaagtacga ctgcaagatc 1080
atgaccagca agaccgatgt gagcagcagc gtgatcacaa gcctgggagc catcgtgagc 1140
tgctacggca agaccaagtg caccgccagc aacaagaacc ggggcatcat taagaccttc 1200
agcaacggct gcgactacgt gagcaacaag ggcgtggata cagtgagcgt gggcaatacc 1260
ctgtactacg tgaataagca ggaaggcaag agcctgtacg tgaagggcga gcccatcatc 1320
aacttctacg accccctggt gttccccagc tgcgagtttt gcgccagcat cagccaggtg 1380
aacgagaaga tcaaccagag cctggccttc atcaggaaga gcgatgagct gctgcacaac 1440
gtggaggaca agatcgagga gatcctgagc aagatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg acactgagca aggatcagct gagcggcatc 1680
aacaatatcg ccttctccaa c 1701

SEQ ID NO: 185
length 1701
molecule type RNA
division PAT

Key Location/Qualifiers
source 1..1701
/mol_type=other RNA
/organism=synthetic construct

Sequence
atggagctgc ccatcctgaa gaccaatgcc atcacaacca tcctggccgc cgtgacactg 60
tgtttcgcca gcagccagaa tatcaccgag gagttttacc agagcacctg cagcgccgtg 120
agcaagggct atctgggcgc tctgaggaca ggctggtaca ccagcgtgat taccatcgag 180
ctgagcaata tcaaggagaa caagtgcaac ggcaccgacg ccaaggtgaa gctgattaag 240
caggagctgg acaagtacaa gaacgccgtg accgatctgc agctgctgat gcagagcaca 300
ccttgtggcg gatctggagg aggatctggc agcgctatcg cctctggcat tgccgtgtgt 360
aaagtgctgc acctggaagg cgaagtgaat aagatcaaat ccgccctgct gagcaccaac 420
aaggccgtgg tgagcctgag caatggcgtg agcgtgctga ccttcaaggt gctggacctg 480
aagaattaca tcgacaagca gctgctcccc atcgtgaaca agcagagctg cagcatcccc 540
aacatcgaga cagtgatcga gtttcagcag aagaacaacc ggctgctgga gatcacccgg 600
gagtttagcg tgaatgccgg cgtgaccacc ccagtgagca cctacatgct gaccaatagc 660
gagctgctga gcctgatcaa cgacatgccc atcaccaacg accagaagaa gctcatgagc 720
agcaacgtgc agatcgtgag gcagcagagc tacagcatca tgtgcatcat caaggaggag 780
gtgctggcct atgtggtgca gctgcctctg tacggcgtga tcgatacccc ctgctggaag 840
ctgcacacat ctcctctgtg cacaaccaat accaaggagg gcagcaatat ctgcctgacc 900
agaaccgata ggggctggta ctgtgacaat gccggcagcg tgagcttttt ccctcaggcc 960
gagacatgta aggtgcagtg caatcgggtg ttctgcgaca ccatgaacag cctgaccctg 1020
cccagcgagg tgaacctgtg caacatcgac atcttcaacc ccaagtacga ctgcaagatc 1080
atgaccagca agaccgatgt gagcagctcc gtgatcacaa gcctgggagc catcgtgagc 1140
tgctacggca agaccaagtg caccgccagc aacaagaacc ggggcatcat taagaccttc 1200
agcaacggct gcgactacgt gagcaacaag ggcgtggata cagtgagcgt gggcaatacc 1260
ctgtactacg tgaataagca ggaaggcaag agcctgtacg tgaagggcga gcccatcatc 1320
aacttctacg accccctggt gttccccagc tgcgagtttt gcgccagcat cagccaggtg 1380
aacgagaaga tcaaccagag cctggccttc atccggaaga gcgatgagct gctgcacaac 1440
gtggaggaca agatcgagga gatcctgagc aagatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg acactgagca aggatcagct gagcggcatc 1680
aataatatcg ccttctccaa c 1701

SEQ ID NO: 186
length 1701
molecule type RNA

division PAT

Key Location/Qualifiers
source 1..1701
/mol_type=other RNA
/organism=synthetic construct

Sequence
atggagctgc ccatcctgaa gaccaatgcc atcacaacca tcctggccgc cgtgacactg 60
tgtttcgcca gcagccagaa tatcaccgag gagttttacc agagcacctg cagcgccgtg 120
agcaagggct atctgggcgc tctgaggaca ggctggtaca ccagcgtgat caccattgag 180
ctgagcaaca tcaaagagaa caagtgcaac ggcaccgacg ccaaggtgaa gctgattaag 240
caggagctgg acaagtacaa gaacgccgtg accgatctgc agctgctgat gcagagcaca 300
ccttgtggcg gatctggagg aggatctggc agcgctatcg cctctggcat tgccgtgtgt 360
aaagtgctgc acctggaagg cgaagtgaac aagatcaaat ctgccctgct gagcaccaac 420
aaggccgtgg tgagcctgag caatggcgtg agcgtgctga ccttcaaggt gctggacctg 480
aagaattaca tcgacaagca gctgctcccc atcgtgaaca agcagagctg cagcatcccc 540
aacatcgaga cagtgatcga gtttcagcag aagaacaacc ggctgctgga gatcacccgg 600
gagtttagcg tgaatgccgg cgtgaccacc ccagtgagca cctacatgct gaccaatagc 660
gagctgctga gcctgatcaa cgacatgccc atcaccaacg accagaagaa gctcatgagc 720
agcaacgtgc agatcgtgag gcagcagagc tacagcatca tgtgcatcat caaggaggag 780
gtgctggcct atgtggtgca gctgcctctg tacggcgtga tcgatacccc ctgctggaag 840
ctgcacacat ctcctctgtg cacaaccaat accaaggagg gcagcaatat ctgcctgacc 900
agaaccgata ggggctggta ctgtgacaat gccggcagcg tgagcttttt ccctcaggcc 960
gagacatgta aggtgcagtg caatcgggtg ttctgcgaca ccatgaacag cctgaccctg 1020
cccagcgagg tgaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaacc ggggcatcat taagaccttc 1200
agcaacggct gcgactacgt gagcaacaag ggcgtggata cagtgagcgt gggcaatacc 1260
ctgtactacg tgaataagca ggaaggcaag agcctgtacg tgaagggcga gcccatcatc 1320
aacttctacg accccctggt gttccccagc tgcgagtttt gcgccagcat cagccaggtg 1380
aacgagaaga tcaaccagag cctggccttc atccggaaga gcgatgagct gctgcacaac 1440
gtggaggaca agatcgagga gatcctgagc aaaatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg accctgagca aggatcagct gagcggcatc 1680
aataatatcg ccttctccaa c 1701

SEQ ID NO: 187
length 1701
molecule type RNA
division PAT

Key Location/Qualifiers
source 1..1701
/mol_type=other RNA
/organism=synthetic construct

Sequence
atggagctgc ccatcctgaa gaccaatgcc atcacaacca tcctggccgc cgtgacactg 60
tgtttcgcca gcagccagaa tatcaccgag gagttttacc agagcacctg cagcgccgtg 120
agcaagggct atctgggcgc tctgaggaca ggctggtaca ccagcgtgat caccattgag 180
ctgagcaaca tcaaagagaa caagtgcaac ggcaccgacg ccaaggtgaa gctgattaag 240
caggagctgg acaagtacaa gaacgccgtg accgatctgc agctgctgat gcagagcaca 300
ccttgtggcg gatctggagg aggatctggc agcgctatcg cctctggcat tgccgtgtgt 360
aaagtgctgc acctggaagg cgaagtgaac aagatcaaat ctgccctgct gagcaccaac 420
aaggccgtgg tgagcctgag caatggcgtg agcgtgctga ccttcaaggt gctggacctg 480
aagaattaca tcgacaagca gctgctcccc atcgtgaaca agcagagctg cagcatcccc 540
aacatcgaga cagtgatcga gtttcagcag aagaacaacc ggctgctgga gatcacccgg 600
gagtttagcg tgaatgccgg cgtgaccacc ccagtgagca cctacatgct gaccaatagc 660
gagctgctga gcctgatcaa cgacatgccc atcaccaacg accagaagaa gctcatgagc 720

agcaacgtgc agatcgtgag gcagcagagc tacagcatca tgtgcatcat caaggaggag 780
gtgctggcct acgtggtgca gctgcctctg tacggcgtga tcgatacccc atgctggaag 840
ctgcacacat ctcctctgtg cacaaccaat accaaggagg gcagcaacat ctgtctgacc 900
agaaccgata ggggctggta ctgtgacaat gccggcagcg tgtctttctt tcctcaggcc 960
gagacatgca aggtgcagtg caatcgggtg ttctgcgaca ccatgaacag cctgaccctg 1020
cccagcgagg tgaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaacc ggggcatcat taagaccttc 1200
agcaacggct gcgactacgt gagcaacaag ggcgtggata cagtgagcgt gggcaatacc 1260
ctgtactacg tgaataagca ggaaggcaag agcctgtacg tgaagggcga gcccatcatc 1320
aacttctacg accccctggt gttccccagc tgcgagtttt gcgccagcat cagccaggtg 1380
aacgagaaga tcaaccagag cctggccttc atccggaaga gcgatgagct gctgcacaac 1440
gtggaggaca agatcgagga gatcctgagc aaaatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg accctgagca aggatcagct gagcggcatc 1680
aataatatcg ccttctccaa c 1701

SEQ ID NO: 188
length 1701
molecule type RNA
division PAT

Key Location/Qualifiers
source 1..1701
/mol_type=other RNA
/organism=synthetic construct

Sequence
atggagctgc caatcctgaa gaccaatgcc atcacaacca tcctggccgc cgtgacactg 60
tgctttgcct ccagccagaa tatcaccgag gagttttacc agagcacctg cagcgccgtg 120
agcaagggct atctgggcgc tctgaggaca ggctggtaca ccagcgtgat caccattgag 180
ctgagcaaca tcaaagagaa caagtgcaac ggcaccgacg ccaaggtgaa gctgattaag 240
caggagctgg acaagtacaa gaacgccgtg actgacctgc agctgctgat gcagagcaca 300
ccttgtggcg gatctggagg aggatctggc agcgctatcg cctctggcat tgccgtgtgt 360
aaagtgctgc acctggaagg cgaagtgaac aagatcaaat ctgccctgct gagcaccaac 420
aaggccgtgg tgagcctgag caatggcgtg agcgtgctga ccttcaaggt gctggacctg 480
aagaattaca tcgacaagca gctgctcccc atcgtgaaca agcagagctg cagcatcccc 540
aacatcgaga cagtgatcga gtttcagcag aagaacaacc ggctgctgga gatcacccgg 600
gagtttagcg tgaatgccgg cgtgaccacc cccgtgagca cctacatgct gaccaatagc 660
gagctgctga gcctgatcaa cgacatgcca atcaccaacg accagaagaa gctcatgagc 720
agcaacgtgc agatcgtgag acagcagagc tacagcatca tgtgcatcat caaggaggag 780
gtgctggcct acgtggtgca gctgcctctg tacggcgtga tcgacacccc atgctggaag 840
ctgcacacat ctcctctgtg cacaaccaat accaaggagg gcagcaacat ctgtctgacc 900
agaaccgata ggggctggta ctgtgacaat gccggcagcg tgtctttctt tcctcaggcc 960
gagacatgca aagtgcagtg caaccgggtg ttctgcgaca ccatgaacag cctgaccctg 1020
cccagcgagg tgaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaacc ggggcatcat taagaccttc 1200
agcaacggct gcgactacgt gagcaacaag ggcgtggata cagtgagcgt gggcaatacc 1260
ctgtactacg tgaataagca ggaaggcaag agcctgtacg tgaagggcga gcccatcatc 1320
aacttctacg accccctggt gttccccagc tgcgagtttt gcgcctctat cagccaggtg 1380
aacgagaaga tcaaccagag cctggccttc atccggaaga gcgatgagct gctgcacaac 1440
gtggaggaca agatcgagga gatcctgagc aaaatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg accctgagca aggatcagct gagcggcatc 1680
aataatatcg ccttctccaa c 1701

SEQ ID NO: 189

length 1701
molecule type RNA
division PAT

Key Location/Qualifiers
source 1..1701
/mol_type=other RNA
/organism=synthetic construct

Sequence
atggagctgc ccatcctgaa gaccaatgcc atcacaacca tcctggctgc cgtgacactg 60
tgctttgcta gctcccagaa catcaccgag gagttttacc agagcacctg cagcgccgtg 120
agcaagggct atctgggcgc cctgaggaca ggctggtaca ccagcgtgat caccatcgag 180
ctgtctaaca tcaaggaaaa caagtgcaac ggcactgacg ccaaggtgaa gctgattaag 240
caggagctgg acaagtacaa gaacgccgtg actgacctgc agctgctgat gcagagcaca 300
ccttgtggcg gatctggcgg aggatctggc agcgctatcg cctctggcat tgccgtgtgt 360
aaagtgctgc acctggaagg cgaagtgaac aagatcaaat ctgccctgct gagcaccaac 420
aaggccgtgg tgagcctgag caatggcgtg agcgtgctga ccttcaaggt gctggacctg 480
aagaattaca tcgacaagca gctgctcccc atcgtgaaca agcagagctg cagcatcccc 540
aacatcgaga cagtgatcga gtttcagcag aagaacaacc ggctgctgga gatcacccgg 600
gagtttagcg tgaacgccgg cgtgaccacc cctgtgagca cctatatgct gaccaacagc 660
gagctgctga gcctgatcaa cgacatgccc atcaccaacg accagaagaa gctcatgagc 720
agcaacgtgc agatcgtgag acagcagagc tacagcatca tgtgcatcat caaggaggag 780
gtgctggcct acgtggtgca gctgcccctg tacggagtga tcgacacccc ctgttggaag 840
ctgcacacat ctcctctgtg cacaaccaat accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tgaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggaaggcaag agcctgtacg tgaagggcga gcccatcatc 1320
aacttctacg accccctggt gttccccagc tgcgagtttt gcgcctctat cagccaggtg 1380
aacgagaaga tcaaccagag cctggccttc atccggaaga gcgacgagct cctgcacaac 1440
gtggaggaca agatcgagga gatcctgagc aaaatctacc acatcgagaa cgagatcgcc 1500
cggatcaaga agctgatcgg cgaggccaac gccggcaaga gcaccaccaa catcatgatc 1560
accaccatca tcatcgtgat cattgtgatc ctgctggccc tgatcgccgt gggcctgctg 1620
ctgtactgta aggccaggtc tacacctgtg accctgagca aggatcagct gagcggcatc 1680
aataatatcg ccttctccaa c 1701

SEQ ID NO: 190
length 1755
molecule type RNA
division PAT

Key Location/Qualifiers
source 1..1755
/mol_type=other RNA
/organism=synthetic construct

Sequence
atggagctgc ccatcctgaa gaccaatgcc attacaacca tcctggccgc cgtgacactg 60
tgtttcgcca gctctcagaa catcaccgag gaattttacc agagcacctg cagcgccgtg 120
agcaagggct acctgggcgc cctgaggacc ggctggtaca caagcgtgat caccatcgag 180
ctgagcaaca tcaaagaaaa caagtgcaac ggcacagacg ccaaagtgaa gctgatcaag 240
caggagctgg acaagtacaa gaacgccgtg actgacctgc agctgctgat gcagtcaaca 300
ccttgtggcg gatctggcgg aggatctggc agcgctatcg cctctggaat tgccgtgtgt 360
aaagtgctgc acctggaggg cgaagtgaac aagatcaaaa gcgccctgct gagcaccaac 420
aaggctgtgg tgagcctgag caatggcgtg agcgtgctga ccttcaaggt gctggacctg 480
aaaaactaca ttgacaagca gctgctcccc atcgtgaaca agcagagctg cagcatcccc 540
aacatcgaga cggtgatcga gtttcagcag aagaacaacc ggctgctgga gatcacccgg 600

gagtttagcg tgaacgctgg cgtgaccacc ccagtgagca cctatatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaggag 780
gtgctggcct acgtggtgca gctgcctctg tacggagtga tcgacacccc ctgttggaaa 840
ctgcacacat ctcctctgtg cacaaccaac accaaggagg gcagcaacat ctgcctgaca 900
agaaccgata ggggctggta ctgtgacaat gccggctctg tgtctttctt tcctcaggcc 960
gagacatgca aggtgcagtg caaccgggtg ttctgcgaca ccatgaacag cctgaccctg 1020
cccagcgagg tcaacctgtg caacatcgac atcttcaacc ccaagtacga ctgcaagatc 1080
atgaccagca agaccgacgt gtctagctcc gtgatcacaa gcctgggagc catcgtgagc 1140
tgctacggca agaccaagtg caccgccagc aacaagaatc ggggcatcat taagaccttc 1200
agcaacggct gcgactacgt gagcaacaag ggcgtggata cagtgagcgt gggcaacacc 1260
ctgtactacg tgaataagca ggagggcaag agcctgtacg tgaagggcga gcccatcatc 1320
aacttctacg accccctggt gtttcccagc tgcgagtttt gcgccagcat cagccaggtg 1380
aacgagaaaa tcaatcagag cctggccttc atcaggaaga gcgacgagct cctgcacaac 1440
gtggaggaca agatcgagga gatcctgagc aagatctacc acatcgagaa cgagatcgcc 1500
aggatcaaga agctgatcgg cgaggccatc taccacatcg agaacgagat cgccaggatc 1560
aagaagctga tcggcgaggc caacgccggc aagagcacca ccaacatcat gatcaccacc 1620
atcatcatcg tgatcattgt gatcctgctg gccctgatcg ccgtgggcct gctgctgtac 1680
tgtaaggcca ggtctacccc tgtgaccctg agcaaggatc agctgagcgg catcaataat 1740
atcgccttct ccaac 1755

SEQ ID NO: 191
length 1434
molecule type RNA
division PAT

Key Location/Qualifiers
source 1..1434
/mol_type=other RNA
/organism=synthetic construct

Sequence
atggagctgc ctatcctgaa gaccaatgcc attacaacaa tcctggccgc cgtgacactg 60
tgtttcgcca gcagccagaa catcaccgag gagttttacc agagcacctg ctctgccgtg 120
agcaagggct acctgggcgc cctgagaaca ggctggtaca ccagcgtgat caccatcgag 180
ctgagcaaca tcaaggagaa caagtgcaat ggcaccgacg ctaaggtgaa gctgatcaaa 240
caggagctgg acaagtacaa gaacgctgtg accgatctgc agctgctgat gcagtcaacc 300
ccttgtggag gatctggcgg cggaagcgga tctgccattg cttctggcat cgccgtgtgt 360
aaagtgctgc acctggaggg agaggtgaac aagattaaga gcgccctgct gagcaccaac 420
aaggccgtgg tgtctctgag caacggcgtg agcgtcctga ccttcaaggt gctggacctg 480
aagaattaca tcgacaaaca gctgctgccc atcgtgaaca agcagagctg cagcatcccc 540
aacatcgaaa ccgtgatcga gtttcagcag aagaacaacc ggctgctgga gatcacccgg 600
gagtttagcg tgaacgccgg cgtgaccaca cccgtgagca cctacatgct gaccaacagc 660
gagctgctga gcctgatcaa cgacatgcct atcaccaacg atcagaagaa gctgatgagc 720
agcaacgtgc agatcgtgcg gcagcagagc tatagcatca tgtgcatcat caaggaagag 780
gtgctggcct acgtggtgca gctgcccctg tacggcgtga ttgacacacc ttgctggaag 840
ctgcacacaa gccccctgtg cacaaccaac accaaagagg gaagcaatat ctgcctgacc 900
aggacagaca ggggatggta ctgtgataac gccggcagcg tgtctttctt ccctcaggcc 960
gaaacctgca aggtgcagtg caacagggtg ttctgcgaca ccatgaacag cctgaccctg 1020
cccagcgagg tgaacctgtg caacatcgac atcttcaacc ccaagtacga ctgcaagatc 1080
atgaccagca agaccgatgt gagctccagc gtcatcacct ctctgggcgc catcgtgagc 1140
tgctatggca agaccaagtg caccgccagc aacaagaaca gggggatcat caagaccttc 1200
agcaacggct gcgactacgt gagcaacaaa ggcgtggaca ccgtgagcgt gggcaatacc 1260
ctgtactacg tgaataagca ggagggcaag agcctgtatg tgaagggcga gcccatcatc 1320
aacttctacg accccctggt gttccccagc tgcgagtttt gcgccagcat cagccaggtg 1380
aatgagaaga tcaaccagag cctggccttt atcaggaaga gcgatgagct gctc 1434

SEQ ID NO: 192
length 1617
molecule type RNA

division PAT

Key Location/Qualifiers
source 1..1617
/mol_type=other RNA
/organism=synthetic construct

Sequence
atggagctgc ccatcctgaa gaccaacgcc atcaccacaa ttctggctgc cgtgaccctg 60
tgcttcgcca gctctcagaa tatcaccgag gagttttacc agagcacctg ctctgccgtg 120
tctaagggct acctgggagc cctgaggacc ggctggtaca ccagcgtgat caccatcgag 180
ctgtctaaca ttaaggagaa caagtgcaac ggcaccgacg ccaaggtgaa gctgatcaag 240
caggagctgg acaagtacaa gaatgccgtg acagatctgc agctgctgat gcagagcaca 300
ccttgtggag gctctggagg cggatctgga agcgccatcg cttctggaat tgccgtgtgt 360
aaggtgctgc acctggaagg cgaggtgaac aaaatcaaaa gcgccctgct gagcaccaac 420
aaggccgtgg tgagcctgag caacggcgtg agcgtgctga ccttcaaggt gctggacctg 480
aaaaactaca tcgacaaaca gctgctgccc attgtgaaca agcagagctg cagcatcccc 540
aacatcgaga cagtgatcga gtttcagcag aagaacaacc ggctgctgga gatcaccaga 600
gagtttagcg tgaacgccgg cgtgaccacc cccgtgagca cctacatgct gaccaacagc 660
gagctgctga gcctgatcaa cgacatgccc atcaccaacg atcagaagaa gctgatgagc 720
agcaacgtgc agatcgtgag gcagcagagc tacagcatca tgtgcatcat caaggaagag 780
gtgctggcct atgtggtgca gctgcctctg tacggcgtga tcgatacccc ttgctggaag 840
ctgcacacaa gccccctgtg tacaacaaac accaaggagg gcagcaatat ctgcctgaca 900
aggaccgata ggggctggta ctgcgacaat gccggctctg tgtctttctt cccccaggcc 960
gaaacctgca aggtgcagtg caatagggtg ttttgcgaca ccatgaacag cctgaccctg 1020
cccagcgagg tcaacctgtg caacatcgac atcttcaacc ccaagtacga ctgcaagatc 1080
atgaccagca agacagacgt gtcctccagc gtcatcacaa gcctcggcgc catcgtgagc 1140
tgttacggca agaccaagtg caccgccagc aacaagaacc ggggcattat caagaccttc 1200
agcaacggct gcgactatgt gagcaacaag ggcgtggaca ccgtgtctgt gggcaacacc 1260
ctgtattacg tgaataaaca ggagggcaag agcctgtacg tgaagggcga gcccatcatc 1320
aacttctacg accccctggt gttccccagc gacgagtttg acgccagcat cagccaggtg 1380
aacgagaaga tcaatcagag cctggctttc atccggaaga gcgacgagct gctccacaac 1440
gtgaatgccg gcaagagcac caccaacatc atgatcacca ccatcatcat cgttatcatc 1500
gtgatcctgc tggccctgat cgccgtgggc ctgctgctgt attgtaaggc ccggagcaca 1560
ccagtgaccc tgagcaagga tcagctgagc ggcatcaaca acatcgcctt ctccaac 1617

SEQ ID NO: 193
length 1614
molecule type RNA
division PAT

Key Location/Qualifiers
source 1..1614
/mol_type=other RNA
/organism=synthetic construct

Sequence
atggagctgc ccatcctgaa gaccaatgcc attaccacaa tcctggccgc cgtgacactg 60
tgtttcgcta gcagccagaa catcaccgag gagttttatc agagcacctg ctctgccgtg 120
tctaagggct acctgggcgc cctgaggacc ggctggtaca ccagcgtgat caccatcgag 180
ctgagcaata tcaaggagaa caagtgcaac ggcacagatg ccaaggtgaa gctgatcaag 240
caggagctgg acaagtacaa gaacgccgtg actgatctgc agctgctgat gcagagcaca 300
ccttgtggag gatctggagg ttctggcagc gctattgcca gcggcatcgc tgtgtgcaag 360
gtgctgcacc tggaaggcga agtgaacaag atcaagagtg ctctgctgag caccaacaag 420
gctgtggtga gcctgagcaa cggcgtgagc gtgctgacct tcaaggtcct ggacctgaag 480
aattacatcg acaaacagct gctccccatc gtgaacaagc agagctgcag catccccaac 540
atcgagacag tgatcgagtt tcagcagaag aacaaccggc tgctggagat caccagggag 600
tttagcgtga acgccggcgt gaccaccccc gtgagcacct acatgctgac caacagcgag 660
ctgctcagcc tgatcaacga catgcctatc accaacgatc agaagaagct gatgagcagc 720
aacgtgcaga tcgtgaggca gcagagctac agcatcatgt gtatcatcaa ggaggaggtg 780
ctggcctacg tggtgcagct gcctctgtac ggcgtgatcg acaccccatg ttggaagctg 840

cacacaagcc ccctgtgtac caccaacacc aaagagggct ctaacatctg tctgaccagg 900
acagataggg gatggtactg cgacaatgcc ggaagcgtga gctttttccc tcaggccgag 960
acatgcaagg tccagtgcaa cagggtgttc tgcgacacca tgaacagcct gaccctgcca 1020
agcgaggtga acctgtgcaa catcgacatc ttcaacccca agtacgactg caagatcatg 1080
accagcaaga ccgacgtgag ctctagcgtc atcaccagcc tgggcgctat cgtgtcttgc 1140
tacggcaaga ccaaatgcac cgccagcaac aagaaccggg gcatcatcaa gaccttcagc 1200
aacggctgcg actacgtgag caacaagggc gtggacaccg ttagcgtggg caatacactg 1260
tactacgtga ataagcagga aggcaagagc ctgtacgtga agggcgagcc catcatcaac 1320
ttctacgacc ccctggtgtt ccccagcgac gagtttgacg ccagcatcag ccaggtgaac 1380
gagaagatca atcagagcct ggccttcatc cggaagagcg acgagctgct gcacaacgtc 1440
aacgccggca agagcacaac caacatcatg atcaccacca ttatcatcgt catcatcgtg 1500
atcctgctgg ccctgatcgc cgtgggcctg ctgctgtact gcaaagccag aagcacccct 1560
gtgactctga gcaaggacca gctgagcggc atcaataaca tcgccttctc caac 1614

SEQ ID NO: 194
length 1614
molecule type RNA
division PAT

Key Location/Qualifiers
source 1..1614
/mol_type=other RNA
/organism=synthetic construct

Sequence
atggagctgc ccatcctgaa gaccaatgcc attaccacaa tcctggccgc cgtgacactg 60
tgtttcgcta gcagccagaa catcaccgag gagttttatc agagcacctg ctctgccgtg 120
tctaagggct acctgggcgc cctgaggacc ggctggtaca ccagcgtgat caccatcgag 180
ctgagcaata tcaaggagaa caagtgcaac ggcacagatg ccaaggtgaa gctgatcaag 240
caggagctgg acaagtacaa gaacgccgtg actgatctgc agctgctgat gcagagcaca 300
cctgccggag gatctggagg ttctggcagc gctattgcca gcggcatcgc tgtgtgcaag 360
gtgctgcacc tggaaggcga agtgaacaag atcaagagtg ctctgctgag caccaacaag 420
gctgtggtga gcctgagcaa cggcgtgagc gtgctgacct tcaaggtcct ggacctgaag 480
aattacatcg acaaacagct gctccccatc gtgaacaagc agagctgcag catccccaac 540
atcgagacag tgatcgagtt tcagcagaag aacaaccggc tgctggagat caccagggag 600
tttagcgtga acgccggcgt gaccaccccc gtgagcacct acatgctgac caacagcgag 660
ctgctcagcc tgatcaacga catgcctatc accaacgatc agaagaagct gatgagcagc 720
aacgtgcaga tcgtgaggca gcagagctac agcatcatgt gtatcatcaa ggaggaggtg 780
ctggcctacg tggtgcagct gcctctgtac ggcgtgatcg acaccccatg ttggaagctg 840
cacacaagcc ccctgtgtac caccaacacc aaagagggct ctaacatctg tctgaccagg 900
acagataggg gatggtactg cgacaatgcc ggaagcgtga gctttttccc tcaggccgag 960
acatgcaagg tccagagcaa cagggtgttc tgcgacacca tgaacagcct gaccctgcca 1020
agcgaggtga acctgtgcaa catcgacatc ttcaacccca agtacgactg caagatcatg 1080
accagcaaga ccgacgtgag ctctagcgtc atcaccagcc tgggcgctat cgtgtcttgc 1140
tacggcaaga ccaaatgcac cgccagcaac aagaaccggg gcatcatcaa gaccttcagc 1200
aacggctgcg actacgtgag caacaagggc gtggacaccg ttagcgtggg caatacactg 1260
tactacgtga ataagcagga aggcaagagc ctgtacgtga agggcgagcc catcatcaac 1320
ttctacgacc ccctggtgtt ccccagctgc gagttttgcg ccagcatcag ccaggtgaac 1380
gagaagatca atcagagcct ggccttcatc cggaagagcg acgagctgct gcacaacgtc 1440
aacgccggca agagcacaac caacatcatg atcaccacca ttatcatcgt catcatcgtg 1500
atcctgctgg ccctgatcgc cgtgggcctg ctgctgtact gcaaagccag aagcacccct 1560
gtgactctga gcaaggacca gctgagcggc atcaataaca tcgccttctc caac 1614

SEQ ID NO: 195
length 1611
molecule type RNA
division PAT

Key Location/Qualifiers
source 1..1611

/mol_type=other RNA
/organism=synthetic construct

Sequence
atggagctgc ccatcctgaa gaccaatgcc attaccacca ttctggccgc cgtgaccctg 60
tgttttgcct ctagccagaa catcaccgag gaattttacc agagcacctg cagcgccgtg 120
agcaagggct acctgggcgc cctgagaacc ggctggtata cctccgtgat caccatcgag 180
ctgagcaata tcaaggagaa caagtgcaac ggcaccgatg ccaaggtgaa gctgattaag 240
caggagctgg acaagtacaa gaacgctgtg accgatctgc agctgctgat gcagagcaca 300
ccttgcggag gaagcggatc tggatctgcc attgccagcg gcatcgccgt gtgtaaggtg 360
ctgcacctgg aaggcgaggt gaacaagatt aagtcagccc tgctgtctac caacaaggcc 420
gtggtgtctc tgagcaacgg cgtgagcgtc ctgaccttca aggtgctgga cctgaagaat 480
tacatcgaca agcagctgct ccccatcgtc aacaagcaga gctgcagcat ccccaacatc 540
gagacagtga tcgagtttca gcagaagaac aaccggctgc tggagatcac cagggagttt 600
agcgtgaacg ccggcgtgac cacccccgtg agcacctaca tgctgaccaa cagcgagctg 660
ctcagcctga tcaacgacat gcctatcacc aacgaccaga agaagctgat gagctctaac 720
gtgcagatcg tgaggcagca gagctacagc atcatgtgta tcatcaagga ggaggtgctg 780
gcctacgtgg tgcagctgcc tctgtacggc gtgattgaca ccccctgctg gaagctgcat 840
accagcccac tgtgcaccac caataccaaa gagggctcta atatctgcct gacaaggacc 900
gacagaggct ggtactgtga taacgccggc agcgtgagct tctttcccca ggccgaaacc 960
tgtaaggtcc agtgcaacag ggtgttctgc gacaccatga acagcctgac actgccaagc 1020
gaggtcaacc tgtgcaacat cgacatcttc aaccccaagt acgactgcaa gatcatgacc 1080
agcaagaccg acgtgagcag cagcgtgatc acatctctgg gcgccatcgt gagctgctac 1140
ggcaagacca agtgcaccgc cagcaacaag aacaggggca tcatcaagac cttcagcaac 1200
ggctgcgact acgtgagcaa caagggcgtg gataccgtga gcgtgggaaa taccctgtat 1260
tacgtgaaca agcaggaggg caagagcctg tacgtgaagg gagagcccat catcaacttc 1320
tacgaccccc tggtgttccc cagcgacgag tttgacgcca gcatcagcca ggtgaacgag 1380
aagatcaacc agagcctggc tttcatccgg aagagcgacg agctgctgca caacgtcaac 1440
gccggaaaga gcacaaccaa catcatgatc accaccatca tcatcgtgat cattgtgatc 1500
ctgctggccc tgatcgccgt gggcctgctg ctgtactgta aggccaggag cacccctgtg 1560
accctgagca aggatcagct gagcggcatt aacaatatcg ccttctccaa c 1611

SEQ ID NO: 196
length 1611
molecule type RNA
division PAT

Key Location/Qualifiers
source 1..1611
/mol_type=other RNA
/organism=synthetic construct

Sequence
atggagctgc ccatcctgaa gaccaatgcc attaccacca ttctggccgc cgtgaccctg 60
tgttttgcct ctagccagaa catcaccgag gaattttacc agagcacctg cagcgccgtg 120
agcaagggct acctgggcgc cctgagaacc ggctggtata cctccgtgat caccatcgag 180
ctgagcaata tcaaggagaa caagtgcaac ggcaccgatg ccaaggtgaa gctgattaag 240
caggagctgg acaagtacaa gaacgctgtg accgatctgc agctgctgat gcagagcaca 300
cctgccggag gaagcggatc tggatctgcc attgccagcg gcatcgccgt gtgtaaggtg 360
ctgcacctgg aaggcgaggt gaacaagatt aagtcagccc tgctgtctac caacaaggcc 420
gtggtgtctc tgagcaacgg cgtgagcgtc ctgaccttca aggtgctgga cctgaagaat 480
tacatcgaca agcagctgct ccccatcgtc aacaagcaga gctgcagcat ccccaacatc 540
gagacagtga tcgagtttca gcagaagaac aaccggctgc tggagatcac cagggagttt 600
agcgtgaacg ccggcgtgac cacccccgtg agcacctaca tgctgaccaa cagcgagctg 660
ctcagcctga tcaacgacat gcctatcacc aacgaccaga agaagctgat gagctctaac 720
gtgcagatcg tgaggcagca gagctacagc atcatgtgta tcatcaagga ggaggtgctg 780
gcctacgtgg tgcagctgcc tctgtacggc gtgattgaca ccccctgctg gaagctgcat 840
accagcccac tgtgcaccac caataccaaa gagggctcta atatctgcct gacaaggacc 900
gacagaggct ggtactgtga taacgccggc agcgtgagct tctttcccca ggccgaaacc 960
tgtaaggtcc agagcaacag ggtgttctgc gacaccatga acagcctgac actgccaagc 1020

gaggtcaacc tgtgcaacat cgacatcttc aaccccaagt acgactgcaa gatcatgacc 1080
agcaagaccg acgtgagcag cagcgtgatc acatctctgg gcgccatcgt gagctgctac 1140
ggcaagacca agtgcaccgc cagcaacaag aacaggggca tcatcaagac cttcagcaac 1200
ggctgcgact acgtgagcaa caagggcgtg gataccgtga gcgtgggaaa taccctgtat 1260
tacgtgaaca agcaggaggg caagagcctg tacgtgaagg gagagcccat catcaacttc 1320
tacgaccccc tggtgttccc cagctgcgag ttttgcgcca gcatcagcca ggtgaacgag 1380
aagatcaacc agagcctggc tttcatccgg aagagcgacg agctgctgca caacgtcaac 1440
gccggaaaga gcacaaccaa catcatgatc accaccatca tcatcgtgat cattgtgatc 1500
ctgctggccc tgatcgccgt gggcctgctg ctgtactgta aggccaggag cacccctgtg 1560
accctgagca aggatcagct gagcggcatt aacaatatcg ccttctccaa c 1611

SEQ ID NO: 197
length 1608
molecule type RNA
division PAT

Key Location/Qualifiers
source 1..1608
/mol_type=other RNA
/organism=synthetic construct

Sequence
atggagctgc ctatcctgaa gaccaacgcc attaccacca ttctggccgc tgtgacactg 60
tgctttgcct ctagccagaa catcacagag gagttttatc agagcacctg cagcgccgtg 120
agcaagggct acctgggcgc cctgagaacc ggctggtaca ccagcgtgat caccatcgag 180
ctgtccaaca tcaaggaaaa caagtgcaac ggcaccgacg ccaaggtgaa gctgatcaag 240
caggagctgg ataaatataa gaacgccgtg accgatctgc agctgctgat gcagagtaca 300
ccttgtggct ctggcagtgg cagcgccatc gcctctggca tcgctgtgtg taaggtgctc 360
cacctggagg gcgaagtgaa taagatcaaa agcgccctgc tgtctaccaa caaggccgtg 420
gtgagcctgt ctaacggcgt gagcgtgctg accttcaagg tgctggacct gaaaaactac 480
atcgacaaac agctgctgcc catcgtgaac aagcagagct gcagcatccc caacatcgag 540
acagtgatcg agtttcagca gaagaacaac cggctgctgg agatcacccg ggagtttagc 600
gtgaacgccg gcgtgaccac ccctgtgagc acctacatgc tgaccaacag cgagctgctg 660
agcctgatca acgacatgcc tatcaccaac gatcagaaga agctgatgag cagcaacgtg 720
cagatcgtgc ggcagcagag ctacagcatt atgtgcatca ttaaggagga ggtgctggcc 780
tacgtggtgc agctgcccct gtacggcgtg atcgacacac cttgctggaa gctgcacaca 840
agcccactgt gcaccacaaa cacaaaagag ggcagcaaca tctgtctgac acggacagac 900
cggggctggt attgtgataa tgccggcagc gtgagcttct ttccccaggc tgaaacctgc 960
aaggtgcagt gcaatcgggt gttctgcgac accatgaata gcctgaccct gcccagcgag 1020
gtgaacctgt gcaacatcga catcttcaac cccaagtacg actgcaagat catgaccagc 1080
aagaccgacg tgagcagctc tgtgattacc agcctgggcg cgatcgtgtc ttgctacggc 1140
aagaccaagt gcaccgccag caacaagaac aggggcatca tcaagacatt cagcaacggc 1200
tgcgactacg tgagcaataa gggcgtggac accgtgagcg tcggcaacac actgtactac 1260
gtgaacaaac aggagggcaa aagcctgtac gtgaagggcg agcccatcat caacttctac 1320
gaccccctgg tgttccccag cgacgagttt gacgccagca tcagccaggt gaacgagaag 1380
atcaaccaga gcctggcctt tatcaggaag agcgacgagc tcctgcacaa cgtcaacgcc 1440
ggcaagagca ccaccaacat catgatcacc accatcatca ttgtgatcat cgtgatcctg 1500
ctggccctga ttgccgtggg cctgctgctg tactgcaagg ccagaagcac acccgtgaca 1560
ctgagcaagg atcagctgag cggcatcaac aacatcgcct tcagcaac 1608

SEQ ID NO: 198
length 1608
molecule type RNA
division PAT

Key Location/Qualifiers
source 1..1608
/mol_type=other RNA
/organism=synthetic construct

Sequence
atggagctgc ctatcctgaa gaccaacgcc attaccacca ttctggccgc tgtgacactg 60
tgctttgcct ctagccagaa catcacagag gagttttatc agagcacctg cagcgccgtg 120
agcaagggct acctgggcgc cctgagaacc ggctggtaca ccagcgtgat caccatcgag 180
ctgtccaaca tcaaggaaaa caagtgcaac ggcaccgacg ccaaggtgaa gctgatcaag 240
caggagctgg ataaatataa gaacgccgtg accgatctgc agctgctgat gcagagtaca 300
cctgccggct ctggcagtgg cagcgccatc gcctctggca tcgctgtgtg taaggtgctc 360
cacctggagg gcgaagtgaa taagatcaaa agcgccctgc tgtctaccaa caaggccgtg 420
gtgagcctgt ctaacggcgt gagcgtgctg accttcaagg tgctggacct gaaaaactac 480
atcgacaaac agctgctgcc catcgtgaac aagcagagct gcagcatccc caacatcgag 540
acagtgatcg agtttcagca gaagaacaac cggctgctgg agatcacccg ggagtttagc 600
gtgaacgccg gcgtgaccac ccctgtgagc acctacatgc tgaccaacag cgagctgctg 660
agcctgatca acgacatgcc tatcaccaac gatcagaaga agctgatgag cagcaacgtg 720
cagatcgtgc ggcagcagag ctacagcatt atgtgcatca ttaaggagga ggtgctggcc 780
tacgtggtgc agctgcccct gtacggcgtg atcgacacac cttgctggaa gctgcacaca 840
agcccactgt gcaccacaaa cacaaaagag ggcagcaaca tctgtctgac acggacagac 900
cggggctggt attgtgataa tgccggcagc gtgagcttct ttccccaggc tgaaacctgc 960
aaggtgcaga gcaatcgggt gttctgcgac accatgaata gcctgaccct gcccagcgag 1020
gtgaacctgt gcaacatcga catcttcaac cccaagtacg actgcaagat catgaccagc 1080
aagaccgacg tgagcagctc tgtgattacc agcctgggcg cgatcgtgtc ttgctacggc 1140
aagaccaagt gcaccgccag caacaagaac aggggcatca tcaagacatt cagcaacggc 1200
tgcgactacg tgagcaataa gggcgtggac accgtgagcg tcggcaacac actgtactac 1260
gtgaacaaac aggagggcaa aagcctgtac gtgaagggcg agcccatcat caacttctac 1320
gaccccctgg tgttccccag ctgcgagttt tgcgccagca tcagccaggt gaacgagaag 1380
atcaaccaga gcctggcctt tatcaggaag agcgacgagc tcctgcacaa cgtcaacgcc 1440
ggcaagagca ccaccaacat catgatcacc accatcatca ttgtgatcat cgtgatcctg 1500
ctggccctga ttgccgtggg cctgctgctg tactgcaagg ccagaagcac acccgtgaca 1560
ctgagcaagg atcagctgag cggcatcaac aacatcgcct tcagcaac 1608

SEQ ID NO: 199
length 1605
molecule type RNA
division PAT

Key Location/Qualifiers
source 1..1605
/mol_type=other RNA
/organism=synthetic construct

Sequence
atggagctgc ccatcctgaa gaccaacgcc atcacaacaa tcctggccgc cgtgaccctg 60
tgcttcgcct ctagccagaa cattaccgag gagttttatc agagcacatg cagcgccgtg 120
agcaagggct acctgggcgc cctgaggacc ggctggtaca caagcgtgat tacaatcgag 180
ctgagcaaca ttaaggagaa caagtgcaac ggcaccgacg ccaaggtgaa gctgatcaag 240
caggagctgg acaagtacaa gaatgccgtg accgatctgc agctgctgat gcagagcaca 300
ccttgtggag gatctggcag cgccatcgct tctggcatcg ctgtgtgtaa ggtgctgcac 360
ctggagggag aggtgaataa gatcaagagc gccctgctga gcacaaacaa agccgtggtg 420
agcctgagca acggagtgag cgtgctgacc ttcaaggtgc tggacctgaa aaactacatc 480
gacaagcagc tgctccccat cgtgaacaag cagagctgca gcatccccaa catcgaaacc 540
gtgatcgagt ttcagcagaa gaacaacagg ctgctggaga tcacccggga gtttagcgtc 600
aacgccggcg tgaccacccc tgtgtccacc tacatgctga ccaacagcga gctgctgagc 660
ctgatcaacg acatgcccat caccaacgat cagaagaagc tgatgtctag caacgtgcag 720
atcgtgcggc agcagagcta cagcatcatg tgcatcatca aggaggaggt gctggcctat 780
gtggtgcagc tgcctctgta cggcgtgatc gacaccccct gctggaagtt gcatacaagc 840
cccctgtgca ccaccaatac caaggaaggc agcaatatct gcctgacaag aaccgataga 900
ggctggtatt gcgacaatgc cggcagcgtg agcttcttcc ctcaggctga aacatgcaag 960
gtgcagtgca accgggtgtt ctgcgacacc atgaacagcc tgaccctgcc cagcgaggtg 1020
aacctgtgca acatcgacat cttcaatccc aagtacgact gcaagatcat gaccagcaag 1080
accgacgtga gcagctctgt gatcaccagc ctgggagcca tcgtgagctg ctacggcaag 1140
accaagtgca ccgccagcaa taagaaccgg gggatcatta agaccttcag caacggctgc 1200

gactatgtga gcaacaaggg cgtggacacc gtgagcgtgg gcaacacact gtactacgtg 1260
aacaaacagg agggaaagag cctgtacgtg aagggcgagc ccatcatcaa cttctacgac 1320
cccctggtgt tccccagcga cgagtttgac gccagcatca gccaggtgaa cgagaagatc 1380
aatcagagcc tggccttcat caggaagagc gatgagctgc tgcacaacgt gaacgccggc 1440
aagagcacga ccaacatcat gatcaccacc atcattatcg tgatcatcgt gatcctgctg 1500
gccctgattg ccgtgggcct gctgctgtac tgcaaggcca ggtctacccc tgtgaccctg 1560
agcaaggacc agctgtctgg catcaacaac atcgccttct ctaat 1605

SEQ ID NO: 200
length 1605
molecule type RNA
division PAT

Key Location/Qualifiers
source 1..1605
/mol_type=other RNA
/organism=synthetic construct

Sequence
atggagctgc ccatcctgaa gaccaacgcc atcacaacaa tcctggccgc cgtgaccctg 60
tgcttcgcct ctagccagaa cattaccgag gagttttatc agagcacatg cagcgccgtg 120
agcaagggct acctgggcgc cctgaggacc ggctggtaca caagcgtgat tacaatcgag 180
ctgagcaaca ttaaggagaa caagtgcaac ggcaccgacg ccaaggtgaa gctgatcaag 240
caggagctgg acaagtacaa gaatgccgtg accgatctgc agctgctgat gcagagcaca 300
cctgccggag gatctggcag cgccatcgct tctggcatcg ctgtgtgtaa ggtgctgcac 360
ctggagggag aggtgaataa gatcaagagc gccctgctga gcacaaacaa agccgtggtg 420
agcctgagca acggagtgag cgtgctgacc ttcaaggtgc tggacctgaa aaactacatc 480
gacaagcagc tgctccccat cgtgaacaag cagagctgca gcatccccaa catcgaaacc 540
gtgatcgagt ttcagcagaa gaacaacagg ctgctggaga tcacccggga gtttagcgtc 600
aacgccggcg tgaccacccc tgtgtccacc tacatgctga ccaacagcga gctgctgagc 660
ctgatcaacg acatgcccat caccaacgat cagaagaagc tgatgtctag caacgtgcag 720
atcgtgcggc agcagagcta cagcatcatg tgcatcatca aggaggaggt gctggcctat 780
gtggtgcagc tgcctctgta cggcgtgatc gacaccccct gctggaagtt gcatacaagc 840
cccctgtgca ccaccaatac caaggaaggc agcaatatct gcctgacaag aaccgataga 900
ggctggtatt gcgacaatgc cggcagcgtg agcttcttcc ctcaggctga aacatgcaag 960
gtgcagagca accgggtgtt ctgcgacacc atgaacagcc tgaccctgcc cagcgaggtg 1020
aacctgtgca acatcgacat cttcaatccc aagtacgact gcaagatcat gaccagcaag 1080
accgacgtga gcagctctgt gatcaccagc ctgggagcca tcgtgagctg ctacggcaag 1140
accaagtgca ccgccagcaa taagaaccgg gggatcatta agaccttcag caacggctgc 1200
gactatgtga gcaacaaggg cgtggacacc gtgagcgtgg gcaacacact gtactacgtg 1260
aacaaacagg agggaaagag cctgtacgtg aagggcgagc ccatcatcaa cttctacgac 1320
cccctggtgt tccccagctg cgagttttgc gccagcatca gccaggtgaa cgagaagatc 1380
aatcagagcc tggccttcat caggaagagc gatgagctgc tgcacaacgt gaacgccggc 1440
aagagcacga ccaacatcat gatcaccacc atcattatcg tgatcatcgt gatcctgctg 1500
gccctgattg ccgtgggcct gctgctgtac tgcaaggcca ggtctacccc tgtgaccctg 1560
agcaaggacc agctgtctgg catcaacaac atcgccttct ctaat 1605

SEQ ID NO: 201
length 564
molecule type AA
division PAT

Key Location/Qualifiers
source 1..564
/mol_type=protein
/organism=synthetic construct

Sequence
MELLILKANA ITTILTAVTF CFASGQNITE EFYQSTCSAV SKGYLSALRT GWYTSVITIE 60
LSNIKENKCN GTDAKVKLIK QELDKYKNAV TELQLLMQST PATNNRARRE LPRFMNYTLN 120

NAKKTNVTLS KKRKRRAIAS GVAVSKVLHL EGEVNKIKSA LLSTNKAVVS LSNGVSVLTS 180
KVLDLKNYID KQLLPIVNKQ SCSISNIETV IEFQQKNNRL LEITREFSVN AGVTTPVSTY 240
MLTNSELLSL INDMPITNDQ KKLMSNNVQI VRQQSYSIMS IIKEEVLAYV VQLPLYGVID 300
TPCWKLHTSP LCTTNTKEGS NICLTRTDRG WYCDNAGSVS FFPQAETCKV QSNRVFCDTM 360
NSLTLPSEVN LCNVDIFNPK YDCKIMTSKT DVSSSVITSL GAIVSCYGKT KCTASNKNRG 420
IIKTFSNGCD YVSNKGVDTV SVGNTLYYVN KQEGKSLYVK GEPIINFYDP LVFPSDEFDA 480
SISQVNEKIN QSLAFIRKSD ELLHNVNAGK STTNIMITTI IIVIIVILLS LIAVGLLLYC 540
KARSTPVTLS KDQLSGINNI AFSN 564

SEQ ID NO: 202
length 1692
molecule type RNA
division PAT

Key Location/Qualifiers
source 1..1692
/mol_type=other RNA
/organism=synthetic construct

Sequence
atggaactgc ttatcctgaa agctaatgct attacaacca ttctgactgc cgttacattc 60
tgctttgcca gtggccagaa tatcacggag gaattctacc agagcacctg cagcgcagtt 120
tccaaaggat atctttcagc attgcggact ggctggtata cctcagttat tacaattgag 180
ctgagcaaca ttaaggaaaa taagtgcaac ggaacagacg ccaaagtcaa gctgatcaag 240
caggagctgg ataagtacaa aaatgctgtc acagagcttc agctcctcat gcagagcact 300
cccgccacca ataaccgagc tcgacgagag ctgcctaggt tcatgaacta cactcttaat 360
aatgctaaaa aaactaacgt gacactttca aagaagcgaa agcggagagc aatcgcaagc 420
ggagtggccg tgagtaaggt gttgcatttg gaaggcgagg tgaataaaat taagagcgca 480
ctgctcagca ccaacaaggc cgttgtgagc ctgagcaatg gagtcagtgt gcttacctct 540
aaggtcctgg acctcaagaa ctacatcgat aagcagctgc tcccaattgt gaataagcaa 600
tcctgttcta tctcaaacat cgagaccgta atcgagttcc aacaaaaaaa caacaggctg 660
ctggaaatca ccagggagtt ctccgttaac gcaggggtga ccacccccgt ctctacctac 720
atgctgacaa acagcgaatt gctgtcattg attaacgata tgccgattac caatgatcag 780
aaaaaactca tgtccaacaa tgtgcaaatt gtgcggcagc agtcctactc catcatgagt 840
attatcaaag aggaagtgct cgcttacgtg gttcagcttc ccctgtatgg cgttatcgat 900
acaccctgct ggaaattgca cacatcaccc ctgtgcacca ccaatacgaa ggagggaagc 960
aacatctgtc tgactaggac cgatcgcggc tggtactgcg acaatgcagg tagtgtgtca 1020
ttttttcctc aggcagaaac atgcaaagtc cagtccaatc gcgtgttctg cgataccatg 1080
aacagtttga cgctgccctc cgaggtgaat ctctgcaatg tagatatctt taatcctaag 1140
tatgattgca agatcatgac ttccaaaacc gacgtgtcat cttctgttat tactagcctt 1200
ggcgcgattg tcagctgtta cggtaagacc aagtgcaccg cctccaataa gaatcgcgga 1260
atcattaaga ctttttcaaa tggatgcgac tacgtgagta ataaaggagt tgacacggtg 1320
tcagtgggca atacgttgta ttacgtcaac aagcaggagg ggaagagcct ctacgtgaag 1380
ggagaaccga taattaactt ctacgaccct ctggtgttcc cctcagatga atttgacgca 1440
tccatttccc aggtaaatga gaagatcaac cagagtctgg cgtttattcg aaaatctgac 1500
gaactgcttc acaatgtgaa tgctggcaaa agtactacga acatcatgat cactaccatt 1560
attatcgtaa ttatcgtgat tttgctgagc ctgatcgctg tgggcctgtt gctctattgt 1620
aaggccagat ccaccccagt gactctttca aaggaccagc tgagtggcat caacaacatt 1680
gccttcagta at 1692

### CROSS-REFERENCE TO RELATED APPLICATIONS

This application claims priority to Chinese Patent Application No. 2023114397970, filed on October 31, 2023; Chinese Patent Application No. 2024100528143, filed on January 12, 2024; Chinese Patent Application No. 2024101736872, filed on February 6, 2024; and Chinese Patent Application No. 2024113315075, filed on September 23, 2024.

The entire contents of each of the above-identified applications are hereby incorporated by reference in their entirety for all purposes.

### TECHNICAL FIELD

The present invention relates to the field of vaccines. More specifically, the present invention relates to a respiratory syncytial virus (RSV) antigen, a nucleic acid encoding the same, a recombinant expression vector comprising the nucleic acid, a pharmaceutical composition comprising the antigen or nucleic acid, and uses thereof.

### BACKGROUND

Respiratory syncytial virus (RSV) is a major cause of respiratory tract infections in infants, children, adolescents, and the elderly. In pediatric patients, RSV infection may result in severe disease, including serious lower respiratory tract infections and long-term respiratory sequelae.

RSV belongs to the family *Pneumoviridae,* genus *Orthopneumovirus,* and has a single serotype. The RSV genome is approximately 15.2 kb in length and encodes ten proteins. RSV infection primarily causes lower respiratory tract infections, such as bronchiolitis and pneumonia, particularly in infants younger than six months of age. In older children and adults, RSV infection typically results in upper respiratory tract infections, including rhinitis and common cold symptoms.

RSV contains three surface membrane proteins: fusion glycoprotein (F protein), attachment glycoprotein (G protein), and small hydrophobic protein (SH protein).

The infection process of RSV primarily depends on the function of the F protein. During infection, the G protein mediates viral attachment to host cells by interacting with host attachment factors. The RSV F protein undergoes a conformational transition from a prefusion conformation to a postfusion conformation, thereby driving fusion between the viral membrane and the host cell membrane and initiating the viral infection cycle.

Neutralizing antibodies induced by natural RSV infection are primarily directed against the RSV F protein. Accordingly, most current RSV vaccine designs are based on the RSV F protein. In addition, three candidate vaccines based on non-F viral antigens are currently under clinical development.

RSV is the most common viral pathogen responsible for acute lower respiratory tract infections (ALRTI) in children under five years of age worldwide and represents the leading cause of hospitalization due to viral respiratory infection in infants. Epidemiological data indicate that RSV-associated ALRTI accounts for approximately 28% of all ALRTI cases. Among fatal ALRTI cases, RSV-associated deaths account for approximately 13%-22% (Lancet, 2017, 390:946).

Existing RSV vaccines are summarized in Table 1.

**Table 1. Existing RSV Vaccines**

| Name | Status | Advantages | Disadvantages |
|---|---|---|---|
| SYNAGIS (Palivizumab) | Approved in 1999 | In premature infants born at less than 35 weeks of gestation, SYNAGIS significantly reduced hospitalization associated with severe RSV disease by more than 50%. In children under 24 months of age with congenital heart disease, SYNAGIS demonstrated a significant reduction in RSV-related hospitalization. In premature infants born at ≤32 weeks or at 32-35 weeks of gestation without bronchopulmonary dysplasia, RSV-related hospitalization was markedly reduced. In patients under 24 months of age with bronchopulmonary dysplasia, SYNAGIS also reduced RSV-related hospitalization to a certain extent. | Its use is limited to high-risk infants, provides protection for only approximately one month per dose, and requires five injections to cover a typical RSV season. |
| Arexvy (GSK) | Approved in 2023 | Overall vaccine efficacy of 82.6%. | Cases of Guillain-Barré syndrome (GBS) have been reported. As a recombinant protein vaccine, it presents certain limitations, including the difficulty of identifying an optimal expression system. In addition, its antigenicity may be influenced by the selected expression system; therefore, careful selection and optimization of the expression system are required during vaccine development. |
| Abrysvo (PFizer) | Approved in 2023 | 66.7% efficacy against RSV-associated lower respiratory tract disease defined by ≥2 symptoms; 85.7% efficacy when defined by ≥3 symptoms. | Cases of Guillain-Barré syndrome (GBS) have been reported. As a recombinant protein vaccine, it presents certain limitations, including the difficulty of identifying an optimal expression system. In addition, its antigenicity may be influenced by the selected expression system; therefore, careful selection and optimization of the expression system are required during vaccine development. |
| nirsevimab | Approved in 2023 | Nirsevimab reduced hospitalization due to RSV-associated lower respiratory tract infection (LRTI) by 83%. In the nirsevimab group, all-cause LRTI hospitalization in infants was reduced by 58%. It also reduced hospitalization due to severe RSV-associated LRTI (defined as percutaneous oxygen saturation <90% requiring oxygen therapy) by 75%. Compared with placebo, nirsevimab significantly reduced medically attended RSV-associated lower respiratory tract infection by 76%. | |
| mRNA-1345 (Moderna) | Under regulatory review | 83.7% efficacy against RSV-associated disease defined by ≥2 symptoms. | |

However, the protective efficacy of currently available RSV antigen-based vaccines remains limited, particularly with respect to their ability to elicit robust neutralizing antibody responses. In addition, the presence of excessive postfusion conformations of the RSV antigen may be associated with increased immunogenic side effects, thereby raising safety concerns.

### SUMMARY OF THE INVENTION

The present invention addresses the technical problem of overcoming the deficiencies in the prior art, namely that currently available RSV antigen-based vaccines exhibit relatively low protective efficacy, particularly due to insufficient induction of neutralizing antibodies, and that excessive postfusion conformations of RSV antigens may lead to increased immunogenic side effects.

To solve the foregoing problems, the present invention provides an RSV antigen, a nucleic acid encoding the same, a recombinant expression vector comprising the nucleic acid, a pharmaceutical composition comprising the antigen or nucleic acid, and uses thereof. The RSV antigen provided herein

exhibits enhanced immunogenicity compared with RSV antigens known in the prior art, is capable of inducing higher neutralizing antibody titers, and confers improved protective efficacy.

### First Aspect

In a first aspect, the present invention provides an RSV antigen comprising mutations A102C and S362C relative to the amino acid sequence set forth in SEQ ID NO: 1 or SEQ ID NO: 80.

### Additional Embodiments

In certain embodiments, the RSV antigen further comprises one or more mutations selected from S46G, E92D, S155C, S190F, S215P, S290C, D486C, D489C, A149C, and Y458C.

In certain preferred embodiments, the RSV antigen comprises mutations D486C, D489C, A 149C, and Y458C.

In certain embodiments, the RSV antigen further comprises a linker.

In certain preferred embodiments, the linker comprises an amino acid sequence selected from SEQ ID NOs: 2-5 or GGS, and/or the linker replaces amino acid residues 103-144 of SEQ ID NO: 1.

In certain embodiments, the RSV antigen further comprises an inserted fragment positioned between amino acid residues 516 and 517 of SEQ ID NO: 1.

In certain preferred embodiments, the inserted fragment comprises the amino acid sequence set forth in SEQ ID NO: 6 or SEQ ID NO: 7.

In certain embodiments of the present invention, the RSV antigen satisfies any one of the following:
**(I)** The RSV antigen differs from SEQ ID NO: 1 in that it comprises mutations S155C, S290C, S190F, S215P, S46G, E92D, D486C, D489C, A102C, and S362C; and amino acid residues 103-144 of SEQ ID NO: 1 are replaced with a linker comprising an amino acid sequence set forth in any one of SEQ ID NO: 2, SEQ ID NO: 3, SEQ ID NO: 4, SEQ ID NO: 5, or GGS;
   and the RSV antigen further comprises one or more of the following:
   - a reversion mutation P215S;
   - an inserted fragment comprising an amino acid sequence set forth in SEQ ID NO: 6, SEQ ID NO: 8, or SEQ ID NO: 140; and/or
   - a replacement fragment comprising an amino acid sequence set forth in SEQ ID NO: 7.
**(II)** The RSV antigen differs from SEQ ID NO: 80 in that it comprises mutations S155C, S290C, S190F, S46G, E92D, D486C, D489C, A102C, and S362C; and amino acid residues 103-144 of SEQ ID NO: 80 are replaced with a linker comprising the amino acid sequence set forth in SEQ ID NO: 2;
and the RSV antigen further comprises an inserted fragment comprising the amino acid sequence set forth in SEQ ID NO: 6.

### Preferred Embodiments

In certain preferred embodiments, the RSV antigen further satisfies one or more of the following conditions:
1. In the RSV antigen according to (I) or (II), the inserted fragment is positioned between amino acid residues 516 and 517 of SEQ ID NO: 1 or SEQ ID NO: 80.
2. In the RSV antigen according to (I), the replacement fragment replaces amino acid residues 525-574 of SEQ ID NO: 1.
3. In the RSV antigen according to (I), the RSV antigen further comprises mutation L513I relative to SEQ ID NO: 1.
4. In the RSV antigen according to (I), the inserted fragment is positioned between residues 513 and 514, between residues 519 and 520, or between residues 522 and 523 of SEQ ID NO: 1.

### Post-Translational Modifications

In certain embodiments, the RSV antigen further comprises a post-translational modification.

In certain embodiments of (I), the RSV antigen further comprises reversion mutation C486D and/or C489D.

In certain embodiments of (II), the RSV antigen further comprises one or more reversion mutations selected from:
(a) G46S; (b) D92E; (c) F190S; (d) C155S and C290S; (e) C486D and C489D; or (f) C102A and C362S.

In certain preferred embodiments, the post-translational modification is glycosylation.

In certain more preferred embodiments, the glycosylation is N-linked glycosylation, and/or the glycosylation occurs on the inserted fragment.

### Specific Sequence Embodiments

In certain specific embodiments, the RSV antigen comprises an amino acid sequence set forth in any one of:
SEQ ID NO: 11, SEQ ID NO: 12, SEQ ID NO: 16, SEQ ID NO: 20, SEQ ID NO: 24, SEQ ID NO: 28, SEQ ID NO: 33, SEQ ID NOs: 34-40, SEQ ID NOs: 81-89, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, or SEQ ID NOs: 166-176.

### Second Aspect

In a second aspect, the present invention provides an isolated nucleic acid comprising a nucleotide sequence encoding the RSV antigen according to the first aspect.

In certain embodiments, the isolated nucleic acid is mRNA.

In certain preferred embodiments, the mRNA is codon-optimized.

In certain more preferred embodiments, the mRNA further comprises one or more of the following elements: a promoter region, a 5' cap structure, a 5' untranslated region (5' UTR), a protein tag, and a 3' untranslated region (3' UTR) and poly(A) tail.

In certain specific embodiments, the promoter is a T7 promoter, and/or the protein tag is HA-HiBiT.

In certain specific embodiments, the nucleotide sequence encoding the RSV antigen comprises a nucleotide sequence set forth in any one of SEQ ID NO: 42, 43, 47, 51, 55, 59, 64-79, 92-118, 121, 123, 125, 127, 129, 131, 133, 135, 137, 139, 142, 144, 146, 148, 150, 152, 154, 156, 158, or 182-200.

In certain embodiments, the nucleic acid is DNA.

In certain preferred embodiments, the DNA further comprises one or more sequences encoding: a 5' cap structure, a 5' UTR, a 3' UTR, a 3' poly(A) sequence, and/or a protein tag.

### Third Aspect

In a third aspect, the present invention provides a recombinant expression vector comprising a starting plasmid and the isolated nucleic acid according to the second aspect.

### Fourth Aspect

In a fourth aspect, the present invention provides a transformed cell comprising the isolated nucleic acid according to the second aspect or the recombinant expression vector according to the third aspect.

### Fifth Aspect

In a fifth aspect, the present invention provides a method for preparing the isolated nucleic acid according to the second aspect, wherein the method comprises subjecting the recombinant expression vector according to the third aspect to in vitro transcription.

### Sixth Aspect

In a sixth aspect, the present invention provides a method for producing the RSV antigen, the method comprising culturing the transformed cell according to the fourth aspect under conditions suitable for expression of the RSV antigen.

### Seventh Aspect

In a seventh aspect, the present invention provides a pharmaceutical composition comprising:
(1) the nucleic acid according to the second aspect; and
(2) a delivery vehicle.

In certain embodiments, the delivery vehicle comprises a lipid nanoparticle (LNP).

In certain preferred embodiments, the LNP comprises:
(A) an ionizable or cationic lipid selected from SM-102, cationic lipid RL151, LQ104-54, LQ104-56, LQ104-E16b-2, or Compound 6; (B) cholesterol; (C) DSPC; and (D) a PEGylated lipid. 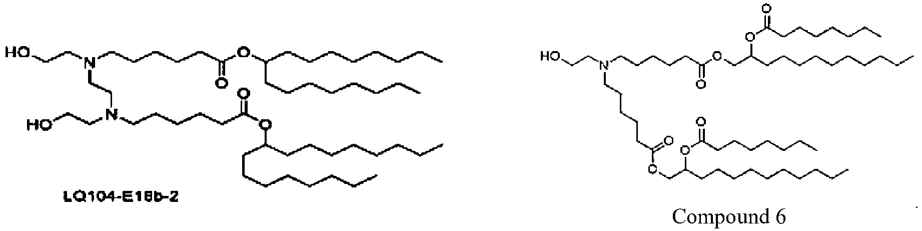 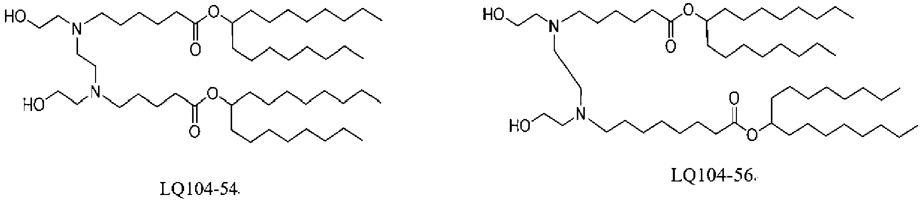 ∘

In certain more preferred embodiments, the molar ratio of cationic lipid RL151, cholesterol, DSPC, and PEGylated lipid is 50:38.5:10:1.5.

In certain specific embodiments:
- the molar ratio of cationic lipid RL151, cholesterol, DSPC, and DMG-PEG2000 is 50:38.5:10:1.5;
- or the molar ratio of LQ104-E16b-2, cholesterol, DSPC, and DMG-PEG2000 is 50:38.5:10:1.5;
- or the molar ratio of Compound 6, cholesterol, DSPC, and DMG-PEG2000 is 50:38.5:10:1.5;
- or the molar ratio of LQ104-54, cholesterol, DSPC, and DMG-PEG2000 is 50:38.5:10:1.5;
- or the molar ratio of LQ104-56, cholesterol, DSPC, and DMG-PEG2000 is 50:38.5:10:1.5.

In certain embodiments, the pharmaceutical composition is a vaccine formulation.

In certain optional embodiments, the pharmaceutical composition further comprises a pharmaceutically acceptable carrier and/or excipient.

In certain embodiments, the pharmaceutical composition further comprises an adjuvant.

### Eighth Aspect

In an eighth aspect, the present invention provides a kit or pharmaceutical package comprising one or more of:
- the RSV antigen according to the first aspect;
- the isolated nucleic acid according to the second aspect;
- the recombinant expression vector according to the third aspect;
- the transformed cell according to the fourth aspect; and
- the pharmaceutical composition according to the seventh aspect.

### Ninth Aspect

In a ninth aspect, the present invention provides the use of one or more of:
- the RSV antigen according to the first aspect;
- the isolated nucleic acid according to the second aspect;
- the recombinant expression vector according to the third aspect;
- the transformed cell according to the fourth aspect; and
- the pharmaceutical composition according to the seventh aspect,
in the manufacture of a medicament for alleviating, preventing, and/or treating a disease caused by RSV.

### BRIEF DESCRIPTION OF THE DRAWINGS

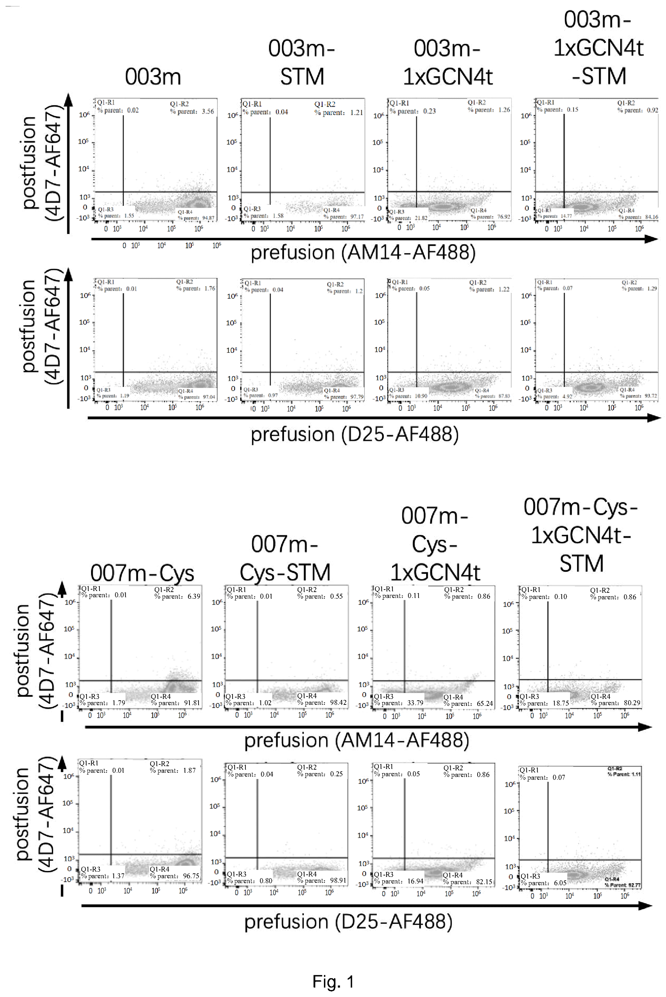

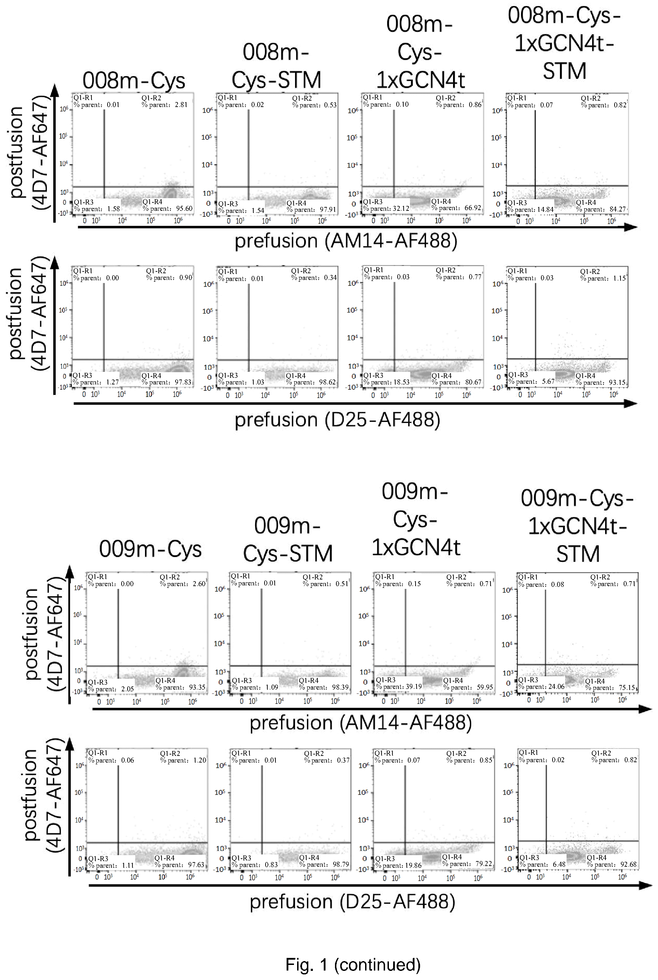

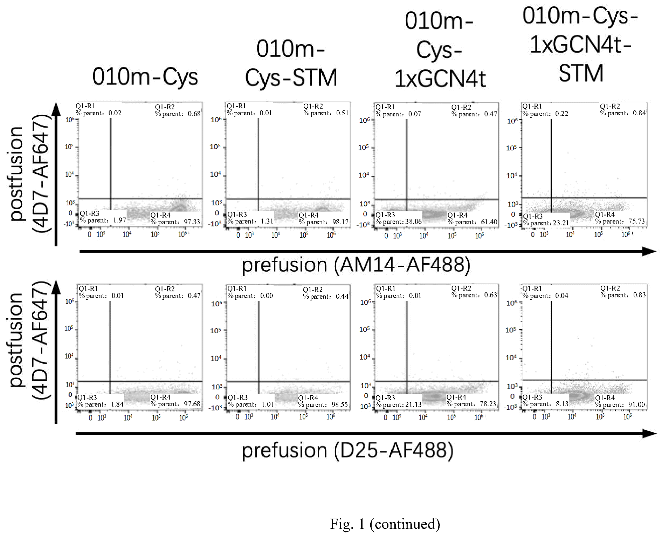

FIG. 1 shows in vitro flow cytometry analysis results of differently designed candidate RSV F antigens.

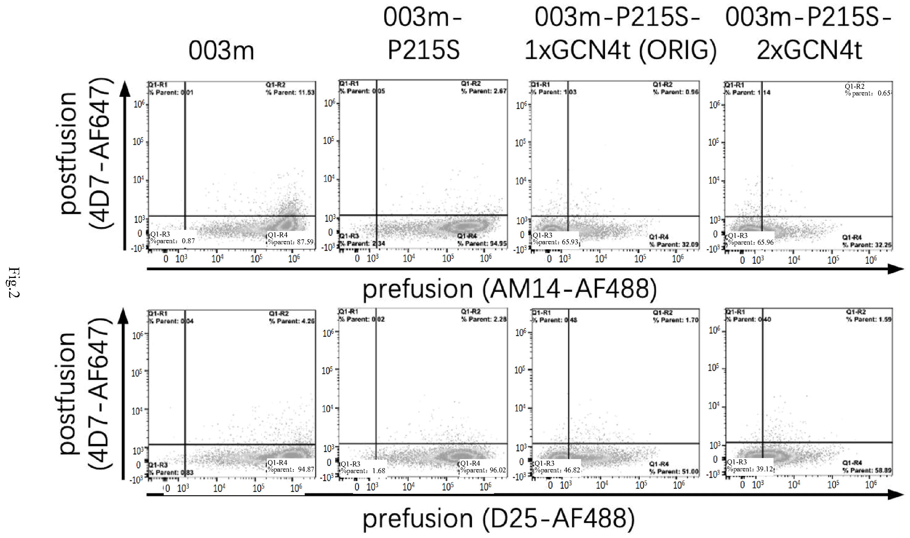

FIG. 2 shows in vitro flow cytometry analysis of RSV F antigen comprising the P215S reversion mutation.
FIG. 3 shows in vitro flow cytometry analysis of RSV F antigens with different extended designs and sequence optimizations.
FIG. 4 shows in vitro flow cytometry analysis of RSV F antigens optimized using different codon optimization strategies.
FIG. 5 shows the effect of an intermolecular disulfide bond (C486 and C489, hereinafter designated ΔF111) on membrane surface expression and conformation of RSV F antigen.
FIG. 6 shows the effect of the intermolecular disulfide bond (C486 and C489, ΔF111) on membrane surface expression and conformation of RSV F antigen.
FIG. 7 shows in vitro flow cytometry analysis after optimization of RNA secondary structure (mRNA-Lipofectamine 2000).
FIG. 8 shows in vitro flow cytometry analysis after optimization of RNA secondary structure (mRNA-RL151).

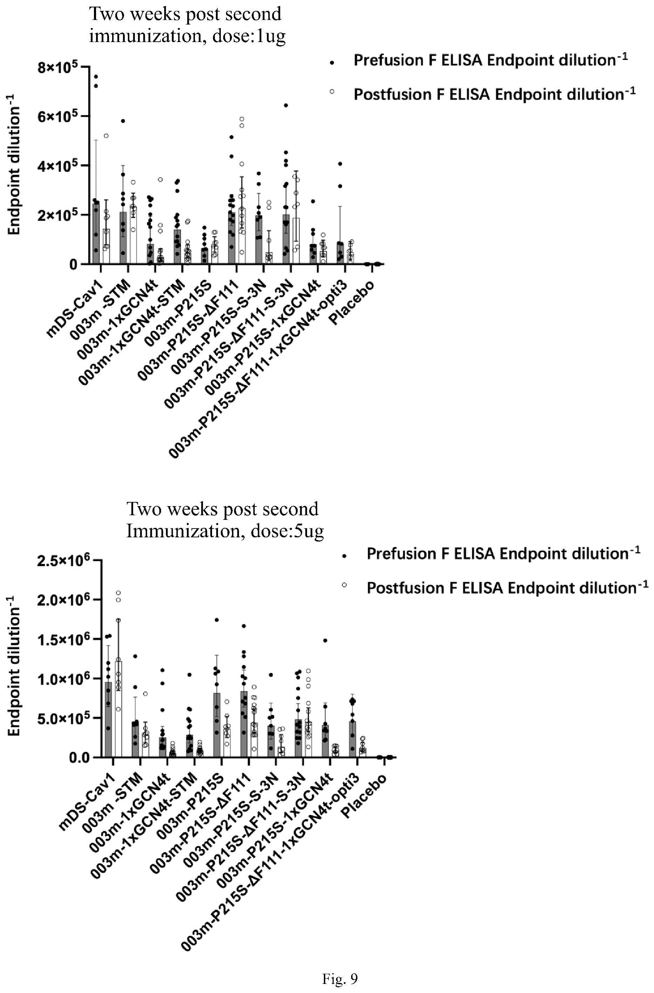

FIG. 9 shows total serum antibody titers in the 5 µg group at two weeks after the second immunization.
FIG. 10 shows neutralizing antibody titers in sera from immunized BALB/c mice.
FIG. 11 shows single-cytokine levels of antigen-specific CD4⁺ T cell immune responses in spleens of BALB/c mice induced by different antigens.
FIG. 12 shows polyfunctional cytokine levels of antigen-specific CD4⁺ T cell immune responses in spleens of BALB/c mice induced by different antigens.
FIG. 13 shows single-cytokine levels of antigen-specific CD8⁺ T cell immune responses in spleens of BALB/c mice induced by different antigens.
FIG. 14 shows polyfunctional cytokine levels of antigen-specific CD8⁺ T cell immune responses in spleens of BALB/c mice induced by different antigens.

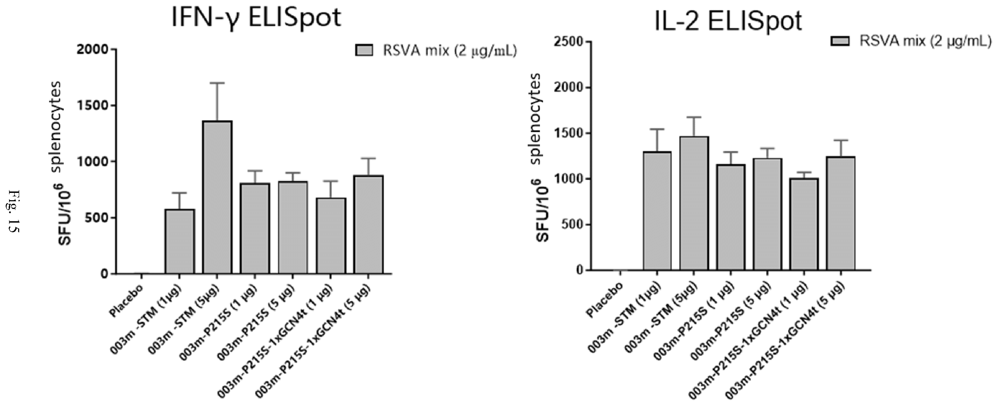

FIG. 15 shows ELISpot analysis of antigen-specific IFN-γ and IL-2 responses in spleens of BALB/c mice induced by different antigens.
FIG. 16 shows percentage body weight change (%) in BALB/c mice following viral challenge.
FIG. 17 shows viral titers and viral copy numbers in BALB/c mice following viral challenge.

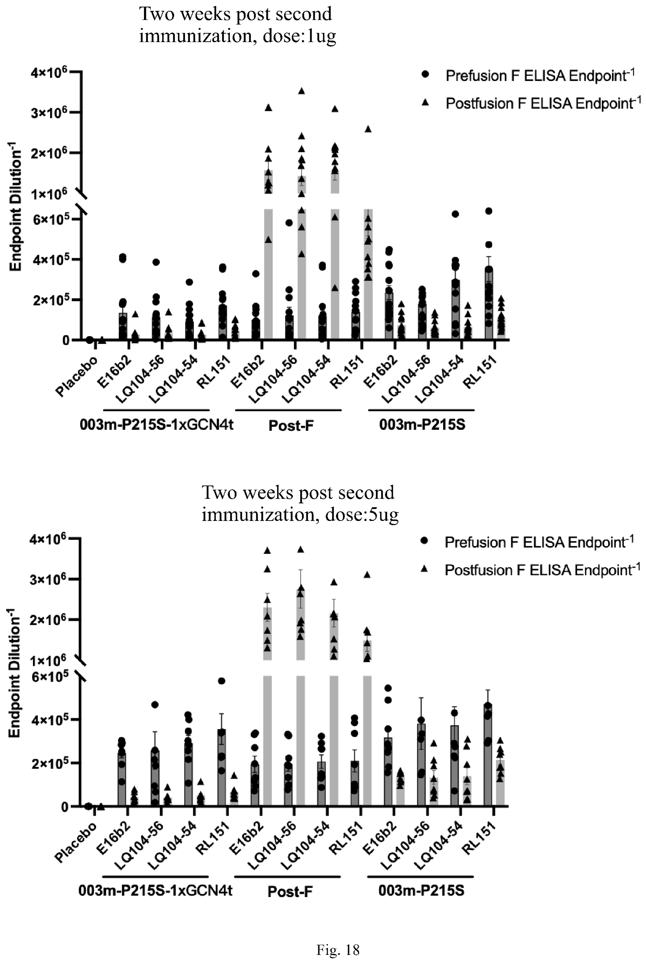

FIG. 18 shows antigen-specific IgG antibody levels induced in BALB/c mice by candidate antigens delivered using different delivery systems.
FIG. 19 shows neutralizing antibody levels induced in BALB/c mice by candidate antigens delivered using different delivery systems.
FIG. 20 shows single-cytokine levels of antigen-specific CD4⁺ T cell immune responses in spleens of BALB/c mice.
FIG. 21 shows polyfunctional cytokine levels of antigen-specific CD4⁺ T cell immune responses in spleens of BALB/c mice.
FIG. 22 shows single-cytokine levels of antigen-specific CD8⁺ T cell immune responses in spleens of BALB/c mice.
FIG. 23 shows polyfunctional cytokine levels of antigen-specific CD8⁺ T cell immune responses in spleens of BALB/c mice.

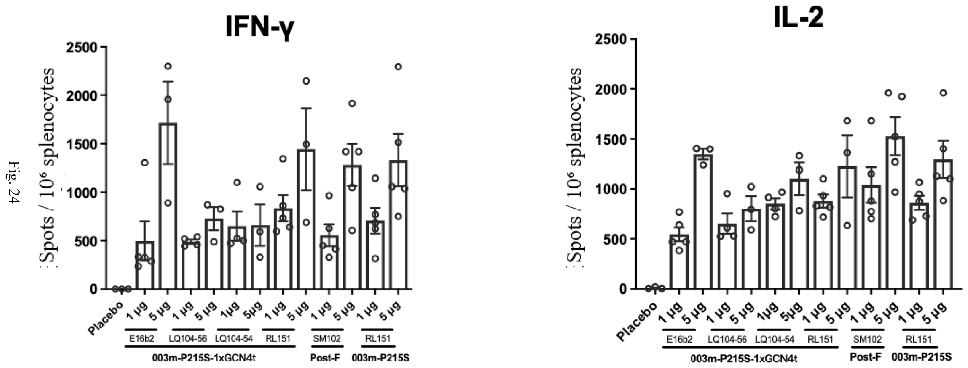

FIG. 24 shows ELISpot analysis of splenocyte immune responses in BALB/c mice.

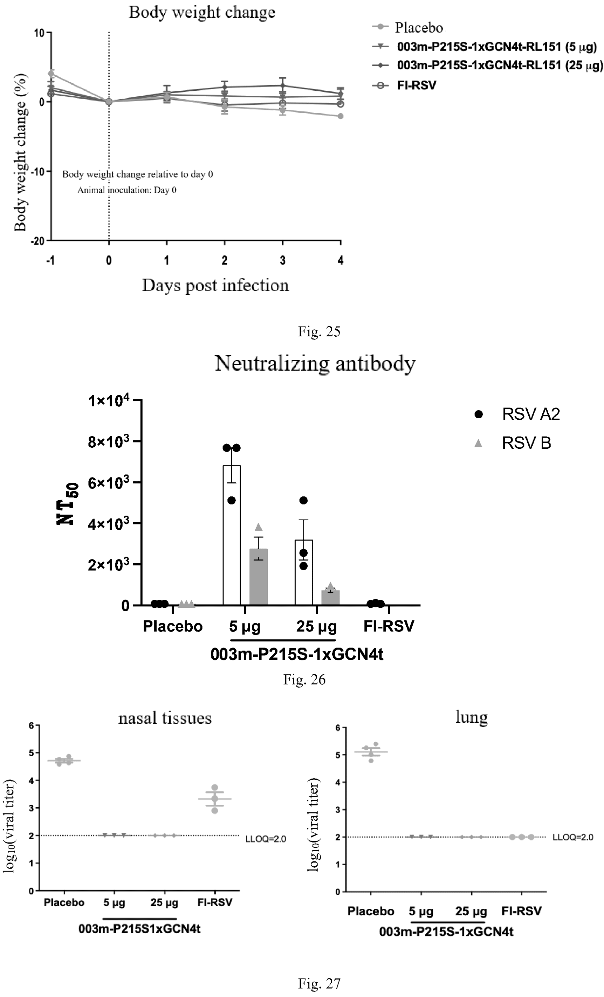

FIG. 25 shows percentage body weight change (%) in cotton rats following viral challenge.
FIG. 26 shows neutralizing antibody levels in cotton rats induced by candidate antigens.
FIG. 27 shows viral titers in lung and nasal tissues of cotton rats following viral challenge.

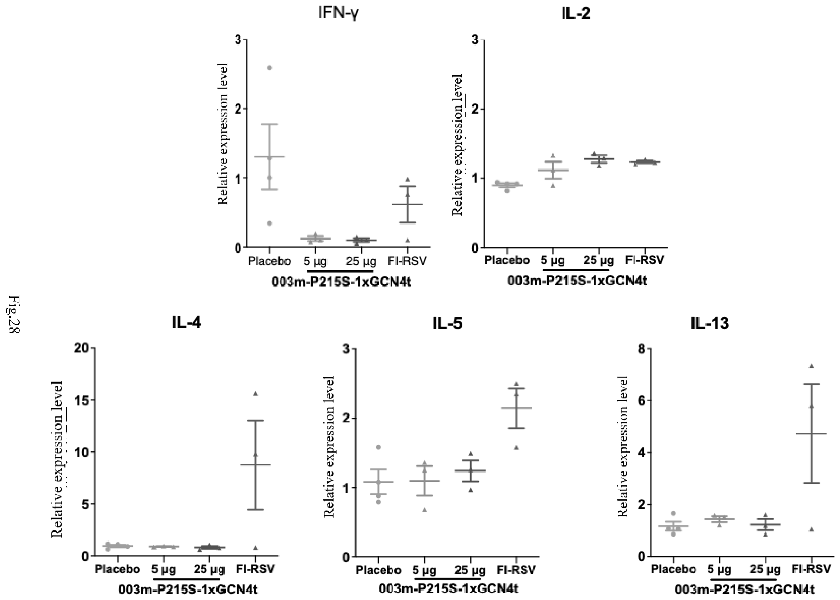

FIG. 28 shows cytokine levels in lung tissues of cotton rats following viral challenge.

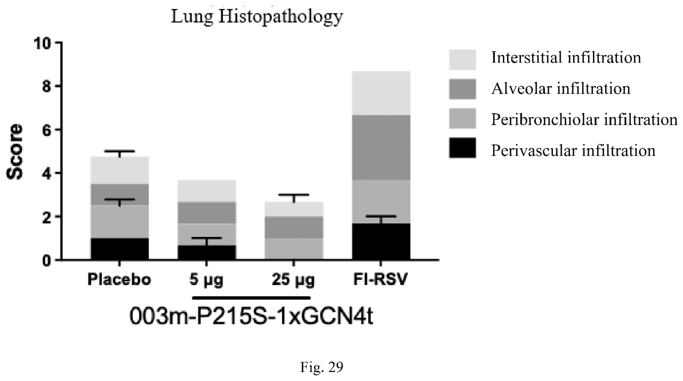

FIG. 29 shows lung histopathology in cotton rats following viral challenge.

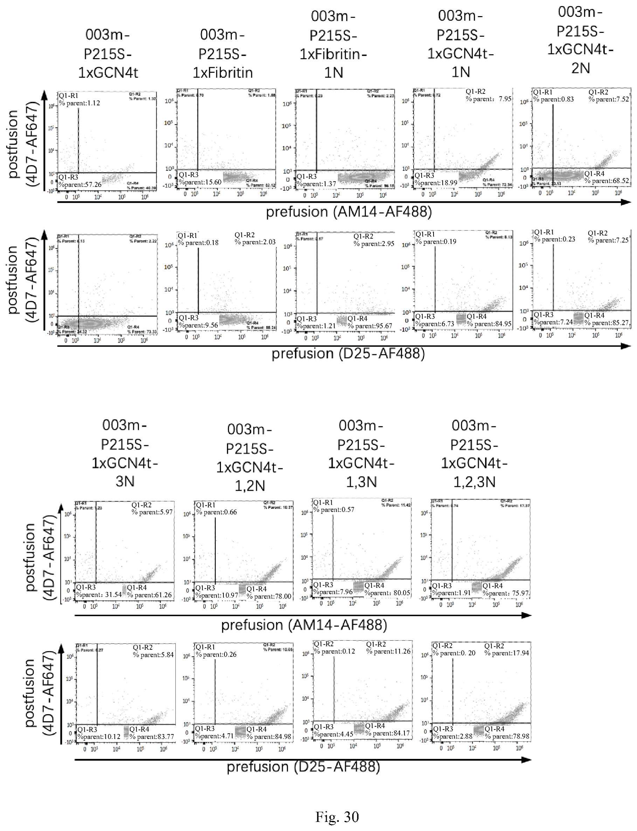

FIG. 30 shows in vitro flow cytometry analysis of RSV F antigens comprising extended sequences with different glycosylation modifications.

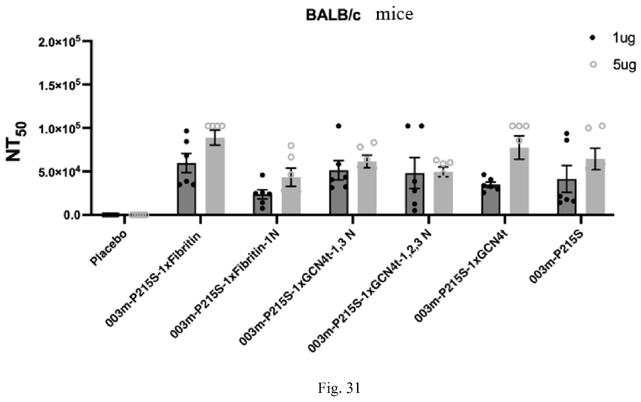

FIG. 31 shows neutralizing antibody levels induced by RSV F antigens comprising extended sequences with different glycosylation modifications.

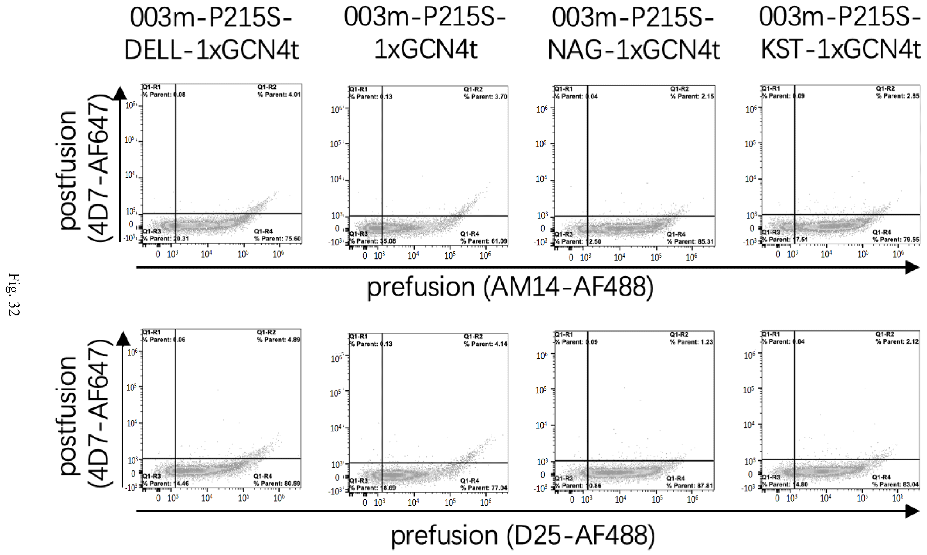

FIG. 32 shows in vitro flow cytometry analysis of RSV F mutants with different 1×GCN4t insertion sites.

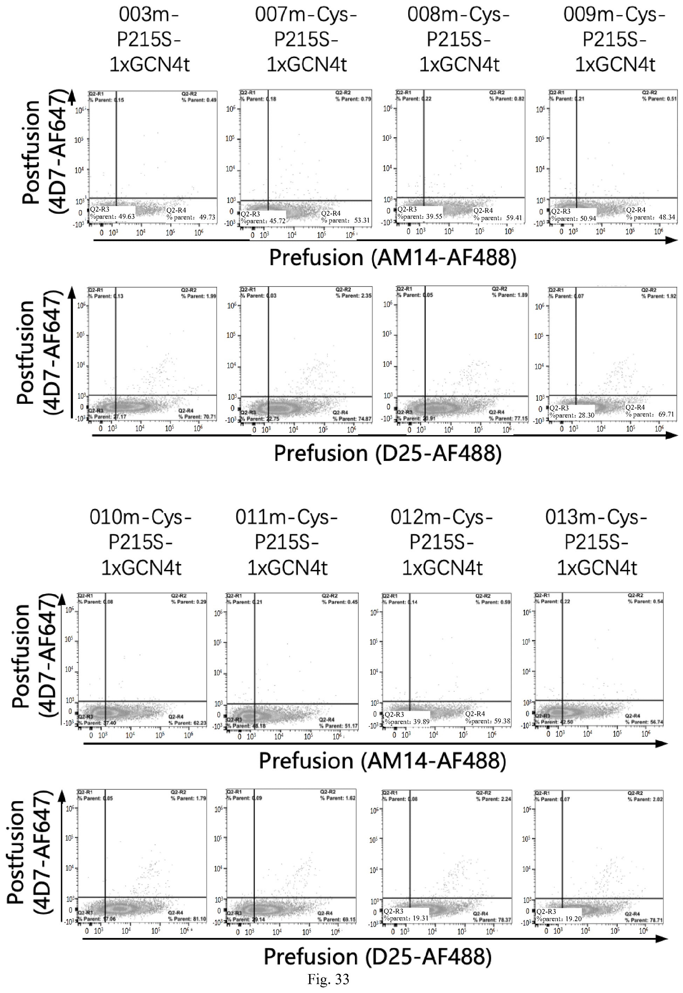

FIG. 33 shows in vitro flow cytometry analysis of RSV F mutants with different linker lengths.

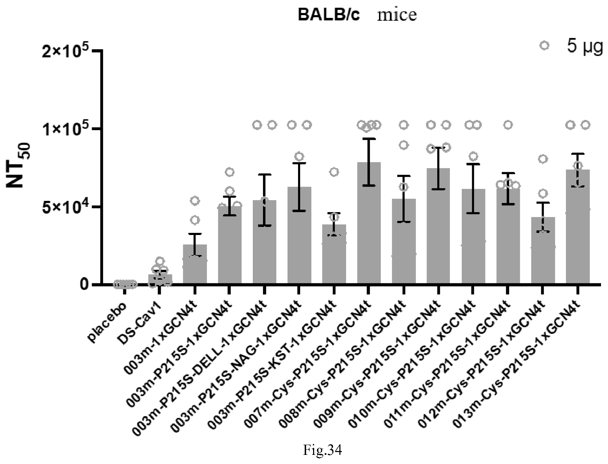

FIG. 34 shows neutralizing antibody levels induced by RSV F antigens comprising different linker lengths.

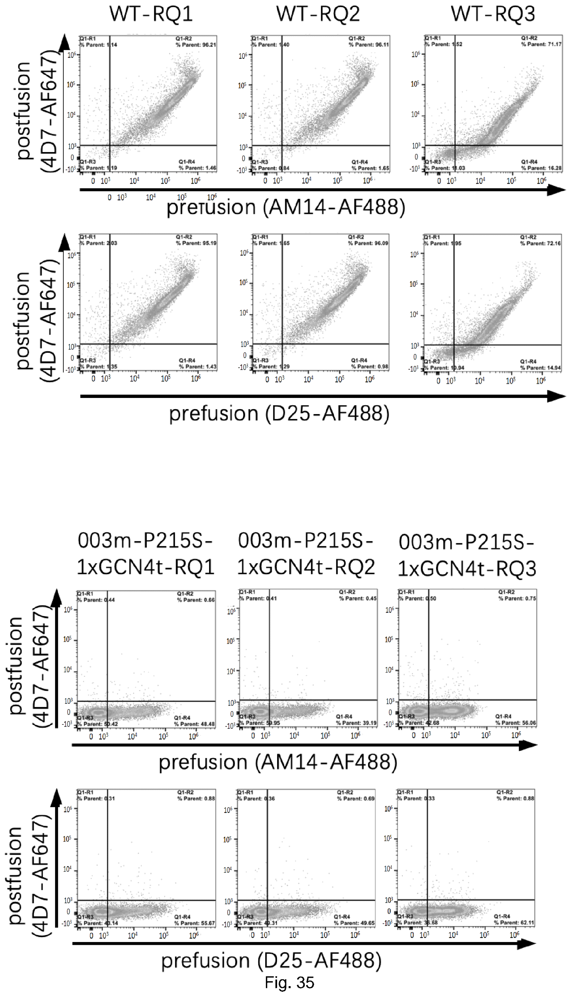

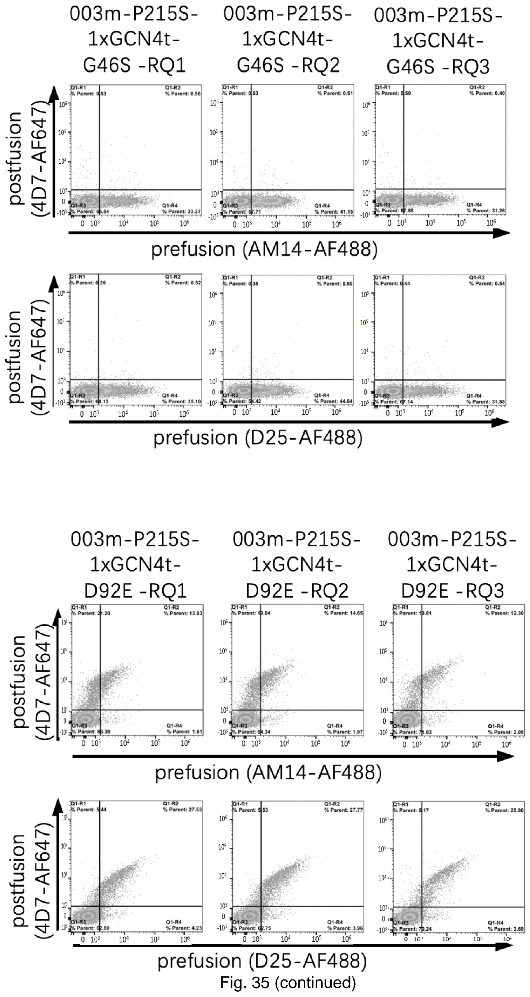

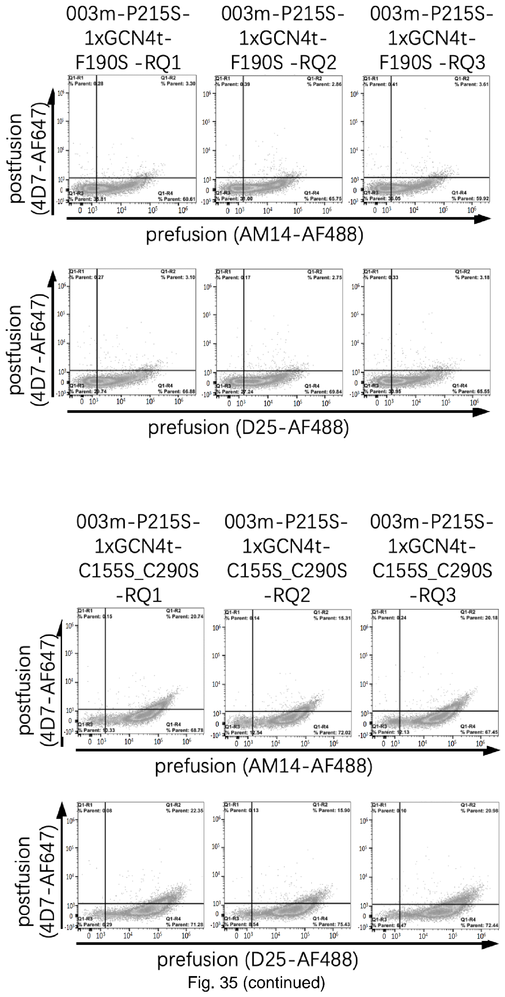

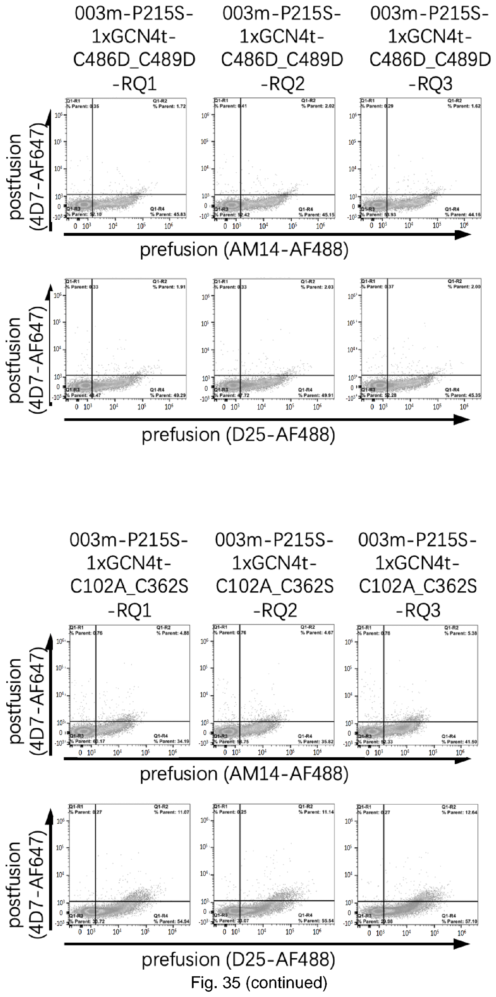

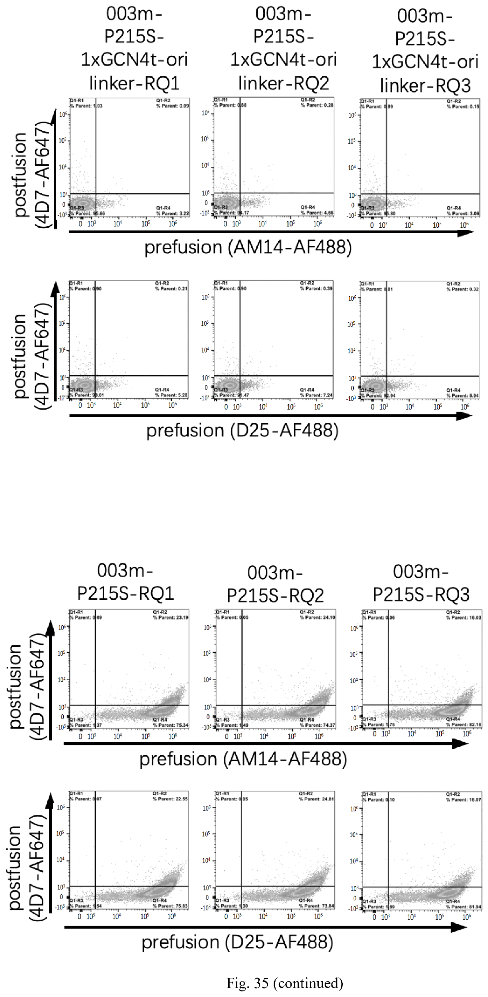

FIG. 35 shows in vitro flow cytometry analysis of RSV B mutants.
FIG. 36 shows neutralizing antibody levels induced by RSV B candidate vaccines.
FIG. 37 shows Western blot (WB) results of different antigen designs following in vitro transfection; "negative" indicates a negative control.
FIG. 38 shows flow cytometry analysis results of different antigen designs following in vitro transfection.
FIG. 39 shows Western blot (WB) results of different antigen designs with various disulfide bond combinations following in vitro transfection; "negative" indicates a negative control.
FIG. 40 shows flow cytometry analysis results of different antigen designs with various disulfide bond combinations following in vitro transfection.
FIG. 41 shows Western blot (WB) results of extended antigen designs following in vitro transfection; "negative" indicates a negative control.
FIG. 42 shows flow cytometry analysis results of extended antigen designs following in vitro transfection.
FIG. 43 shows integrity analysis of mRNA and mRNA-LNP in mouse immunization samples.
FIG. 44 shows flow cytometry analysis results following in vitro transfection of mouse immunization samples.
FIG. 45 shows total antibody levels in sera of antigen-immunized mice.
FIG. 46 shows single-cytokine levels of antigen-specific CD8⁺ T cell immune responses in spleens of BALB/c mice induced by different antigens.
FIG. 47 shows polyfunctional cytokine levels of antigen-specific CD8⁺ T cell immune responses in spleens of BALB/c mice induced by different antigens.
FIG. 48 shows single-cytokine levels of antigen-specific CD4⁺ T cell immune responses in spleens of BALB/c mice induced by different antigens.
FIG. 49 shows polyfunctional cytokine levels of antigen-specific CD4⁺ T cell immune responses in spleens of BALB/c mice induced by different antigens.
FIG. 50 shows ELISpot analysis of antigen-specific IFN-γ and IL-2 responses in spleens of BALB/c mice induced by different antigens.
FIG. 51 shows the structure of the mRNA.

### DETAILED DESCRIPTION OF THE INVENTION

The present invention is further illustrated by the following Examples. However, the scope of the present invention is not limited to the specific embodiments described herein.

Unless otherwise specified, experimental procedures not specifically described in the following Examples were performed according to conventional methods and conditions known in the art or in accordance with the manufacturer's instructions.

Unless otherwise expressly indicated, identical sequence designations differing only in capitalization refer to the same sequence. For example, "003m-P215S-1×GCN4t" and "003M-P215S-1×GCN4t" refer to the same sequence, and the same applies to other similar cases.

### Example 1

### Experimental Methods

### I. In Vitro Transcription and mRNA Transfection

### 1. PCR Amplification to Obtain Linearized Target Fragment

To generate a linearized template fragment for in vitro transcription, a PCR reaction was prepared as follows. In a 1.5 mL centrifuge tube, the following components were added:
- 8.7 µL ddH₂O;
- 0.5 µL template DNA (pcDNA(+) backbone with conventional modifications introduced into the 3'UTR and 5'UTR of the open reading frame; synthesized by GenScript);
- 0.4 µL forward primer (synthesized by GenScript);
- 0.4 µL reverse primer (synthesized by GenScript); and
- 10 µL PrimerSTAR^{®} MAX DNA Polymerase (Takara, Cat. No. R450A).

The reaction mixture was gently mixed to ensure homogeneity.

The PCR reaction system is shown in Table 2.

**Table 2. PCR Reaction System**

| | |
|---|---|
| Plasmid (pcDNA3.1(+)) | 0.5 µL |
| Primer F: T7-mRNA-pCDNA-F | 0.4 µL |
| Primer R: Human-HBA-UTR-100T-R | 0.4 µL |
| H₂O | 8.7 µL |
| PrimerSTAR Max | 10 µL |
| Total | 20 µL |

The final PCR cycling conditions are shown in Table 3 below.

**Table 3. PCR Conditions**

| | | |
|---|---|---|
| 98°C | 3 min | |
| 98°C | 15 s | 34× |
| 60°C | 10 s | |
| 72°C | 1 min (30 s/kb) | |
| 72°C | 10 min | |
| 10°C | ∞ | |

Upon completion of the PCR reaction, agarose gel electrophoresis was performed to verify the amplification product. If a single, uniform nucleic acid band was observed, the product was used directly for the subsequent in vitro transcription (IVT) reaction. If the amplification did not yield a uniform band, the PCR was repeated.

### 2. In Vitro Transcription (IVT)

A small-scale IVT reaction was performed according to the formulation shown in Table 4 (total reaction volume: 20 µL).

**Table 4. IVT Reaction System**

| | |
|---|---|
| ddH₂O | 11 µL |
| 10×HYB (ApexBio, Cat. No. K1083) | 2 µL |
| Cap1 (B8176, 100 mM) (ApexBio, Cat. No. B8176) | 0.4 µL |
| GTP (100 mM) (Hongene Biotech, Cat. No. R2331) | 0.4 µL |
| ATP (100 mM) (Hongene Biotech, Cat. No. R1331) | 0.4 µL |
| CTP (100 mM) (Hongene Biotech, Cat. No. R3331) | 0.4 µL |
| Pseudo-U (100 mM) (Hongene Biotech, Cat. No. R5331) | 0.4 µL |
| T7 mix (ApexBio, Cat. No. K1083) | 2 µL |
| PCR Product (Linearized DNA Template) | 3 µL |
| Total Volume | 20 µL |

After preparation of the IVT reaction mixture, the entire reaction system was incubated in a 37°C water bath for 2 hours. Upon completion of the transcription reaction, digestion of the DNA template was performed by adding DNase I.

Specifically, 1 µL DNase I (Vazyme, Cat. No. EN401-01) was added to the 20 µL IVT reaction mixture (35 µL DNase I per 1 mL IVT reaction), followed by incubation at 37°C for 15 minutes. The IVT product was then purified according to the following procedure:

### IVT Product Purification

### Step I:

Add 80 µL ddH₂O to the 20 µL digestion product to bring the total volume to 100 µL.

### Step II:

Add 350 µL Solution D and 250 µL absolute ethanol (Hushi, Cat. No. 801769722), mix thoroughly, and transfer the mixture to a nucleic acid purification column (Solarbio, Cat. No. N1012). Centrifuge at 10,000 × g for 1 minute.

### Step III:

Add 500 µL of 70% ethanol and centrifuge at 10,000 × g for 1 minute. Repeat this wash step once, followed by centrifugation at 10,000 × g for an additional 2 minutes.

### Step IV:

Add 70 µL sodium citrate solution (Sigma, Cat. No. C8532) to the purification column. After standing at room temperature for 1 minute, centrifuge at 10,000 × g for 2 minutes. The flow-through was collected as the purified RNA product.

RNA quality was assessed by electrophoresis on a 1% agarose gel prepared according to standard procedures. Electrophoresis was performed at 160 V for 20 minutes. After completion, the gel was visualized using a gel imaging system (Tanon, Cat. No. Tanon 4600 SF). If a single, distinct band was observed, the RNA concentration was subsequently determined.

### 3. Cell Transfection

For transfection, 1 µg of mRNA sample was mixed with 3 µL Lipofectamine^{™} 2000 or RL151 and incubated at room temperature for 20 minutes.

The resulting mixture was added to 1 × 10⁶ Expi293F cells (Thermo Fisher Scientific, Cat. No. A39241). The cells were cultured at 37°C in a 5% CO₂ incubator for 18-20 hours, after which the cells were harvested for subsequent analysis.

### II. In Vitro Flow Cytometry Analysis

### 1. Cell Preparation

Transfected cells were washed three times with 1× PBS (Sangon Biotech, Cat. No. B548117-0500). The cells were then resuspended in 500 µL of 1× PBS.

### 2. Sample Aliquoting

From each single-cell suspension, three aliquots of 100 µL were transferred into separate 1.5 mL EP tubes (Shanghai Yueyi Biotechnology, Cat. No. YB-1.5s). The tubes were labeled as:
- Unstained group
- T1 group
- T2 group

### 3. Preparation of Single-Stain and LIVE/DEAD Control Groups

The RSV F protein exists in postfusion and prefusion conformations. The prefusion conformation further includes monomeric and trimeric forms. Accordingly, three antibodies were used for detection:
- 4D7 (Biointron, Lot No. 20220808M011)
- D25 (Biointron, Lot No. 20220808M010)
- AM14 (Biointron, Lot No. 20220808M009)

Based on literature references, the above antibodies were conjugated with fluorescent dyes:
- Alexa Fluor^{®} 647 (Thermo Fisher Scientific, Cat. No. A20186)
- Alexa Fluor^{®} 488 (Thermo Fisher Scientific, Cat. No. A20181)

The conjugates were designated as follows:
- 4D7-AF647
- D25-AF488
- AM14-AF488

For single-stain controls, 4D7-AF647 and D25-AF488 were used individually for staining.

For preparation of the LIVE/DEAD control group, cells were incubated in a 65°C metal bath (Hangzhou Ruicheng Instrument Co., Ltd., Cat. No. DH300) for 30 minutes, followed by immediate placement on ice. Prior to acquisition, SYTOX^{™} (Thermo Fisher Scientific, Cat. No. S34861) was added to a final dilution of 1:5000 for signal detection.

### 4. Experimental Group Preparation

For the T1 group, 50 µL of antibody mixture containing AM14-AF488 and 4D7-AF647 was added.

For the T2 group, 50 µL of antibody mixture containing D25-AF488 and 4D7-AF647 was added.

The samples were incubated at 4°C for 1 hour. Following incubation, samples were centrifuged at 500 × g for 4 minutes at 4°C, and the supernatant was discarded. Cells were washed three times with 150 µL of 1× PBS and resuspended in 75 µL of 1× PBS.

### 5. Flow Cytometry Acquisition

Prior to analysis, 75 µL of SYTOX^{™} at a final dilution of 1:5000 was added to the LIVE/DEAD group, T1 group, and T2 group. After incubation at room temperature for 5 minutes, samples were analyzed using a flow cytometer (Cytek, Cat. No. NL-CLC V16 B14 R8).

### III. Western Blot Analysis

### 1. Preparation of Cell Lysates

Transfected cells were lysed by adding cell lysis buffer supplemented with a protease inhibitor cocktail (ApexBio, Cat. No. K1012). The lysates were incubated on ice for 10 minutes and then centrifuged at 12,000 × g for 10 minutes. The supernatant was collected as the total protein extract.

### 2. Preparation of Electrophoresis Samples

The collected lysates were divided into two equal portions. One portion was mixed with 4× LDS loading buffer containing 40 mM DTT (Invitrogen, Cat. No. NP0008), and the other portion was mixed with 4× LDS loading buffer without DTT.

Samples containing DTT were subjected to heat denaturation at 95°C for 8 minutes, followed by immediate cooling on ice. Samples without DTT were not subjected to heat treatment.

### 3. SDS-PAGE Electrophoresis

Prepared samples were loaded into the wells of a polyacrylamide gel (Tanno, Cat. No. 180-9115H).

Electrophoresis was performed using 1× MOPS running buffer (Tanno, Cat. No. BT8100-2002H) in a compatible electrophoresis chamber (Tanno, Cat. No. VE-180).

### 4. Protein Transfer and Detection

Following electrophoresis, proteins were transferred to a membrane using a semi-dry transfer apparatus (Bio-Rad, Cat. No. 1704150EDU). After transfer, the membrane was incubated sequentially with a primary antibody (Promega, Cat. No. CS2006A01) and a corresponding secondary antibody (Abcam, Cat. No. ab6728).

After incubation and washing, protein bands were visualized using an enhanced chemiluminescence (ECL) detection reagent (ApexBio, Cat. No. K1200) and analyzed using a gel imaging system.

### 5. Membrane Stripping and Reprobing

After detection of the target protein, the PVDF membrane (Cytiva, Cat. No. 10600023) was treated with 1× stripping buffer to remove bound primary and secondary antibodies.

The membrane was then re-blocked and incubated with an appropriate concentration of a different primary antibody (Proteintech, Cat. No. 60004-1-Ig), followed by incubation with secondary antibody (Abcam, Cat. No. ab6728).

After washing, the membrane was again developed using ECL reagent (ApexBio, Cat. No. K1200) and analyzed using the gel imaging system.

### IV. mRNA-LNP Integrity Analysis

### 1. LNP Disruption

Ten (10) µL of mRNA-LNP sample (200 ng/µL) was transferred into a new EP tube. Subsequently, 0.4 µL of 10% Triton^{™} X-100 was added. The mixture was heated at 70°C for 5 minutes and immediately placed on ice.

### 2. RNA Electrophoresis

A total of 400 ng of the treated sample was transferred to a new tube, followed by addition of 1 µL RNA loading buffer and 1 µL ddH₂O. The mixture was heated at 70°C for 8 minutes and immediately placed on ice. After cooling, the sample was briefly centrifuged, and an appropriate volume was loaded onto an agarose gel for electrophoresis to assess RNA integrity.

### V. Total Antibody Level Detection

### 1. Antigen Coating and ELISA

### (1) Total Antigen ELISA

The target antigen (Shanghai Blue Magpie Biotechnology, Lot No. rq20221031) was diluted to 1 µg/mL. A volume of 100 µL per well was added to a 96-well ELISA plate (Nest, Cat. No. 514201) and incubated overnight at 4°C in the dark.

The plate was then blocked at room temperature for 2 hours using 1× PBST containing 3% bovine serum albumin (BSA).

Immune mouse serum was diluted 1:100 as the starting concentration and subjected to 4-fold serial dilutions in PBS for a total of 11 dilution points.

### (2) Prefusion and Postfusion Antigen ELISA

The prefusion conformation antigen (Shanghai Biointron Biological Technology Co., Ltd., Lot No. M-202309120335) and the postfusion conformation antigen (Shanghai Biointron Biological Technology Co., Ltd., Lot No. M-202309160679) were diluted to 400 ng/mL and 300 ng/mL, respectively.

One hundred (100) µL of each diluted antigen was added per well to a 96-well ELISA plate (Nest, Cat. No. 514201) and incubated overnight at 4°C in the dark.

The plate was blocked at room temperature for 2 hours using 1× PBST containing 3% BSA.

Immune mouse serum was diluted 1:200 as the starting concentration and subjected to 4-fold serial dilutions in PBS for a total of 10 dilution points.

After coating and blocking, plates were washed with 1× PBST. Serially diluted serum samples were added and incubated at room temperature for 2 hours.

To determine RSV F-specific antibody responses, the plates were incubated with HRP-conjugated rabbit anti-mouse IgG (Abcam, Cat. No. ab6728) at 25°C for 1 hour. Following washing, tetramethylbenzidine (TMB) substrate solution (Invitrogen, Cat. No. 00-4201-56) was added for color development.

After approximately 12 minutes, the reaction was stopped by adding 1 M sulfuric acid. Absorbance was measured at 450 nm using a microplate reader (Synergy^{™} H1, BioTek).

### VI. Neutralizing Antibody Assay

### 1. Vazyme-Based Neutralization Assay

### 1) Cell Preparation

Hep-2 cells (ATCC, Cat. No. CCL-23) were seeded into 96-well plates (Nest, Cat. No. 701001) at an appropriate density and cultured overnight in a CO₂ incubator (Thermo Scientific, Cat. No. 3111) at 37°C with 5% CO₂. On the following day, cells reached approximately 90% confluence and were used for the assay.

### 2) Serial Dilution of Serum Samples

Serum samples were initially diluted 1:20 and subjected to 3-fold serial dilutions for a total of 8 dilution points (including the initial dilution), tested in single or duplicate wells.

### 3) Virus Dilution

RSV virus was diluted to an appropriate concentration according to its PFU value.

### 4) Neutralization Reaction

Diluted virus was added to serum sample wells and virus control wells. For back-titration wells, virus was serially diluted 2-fold across four dilution levels. The plate was incubated at 37°C with 5% CO₂ for approximately 1 hour to allow neutralization.

### 5) Virus Adsorption

Fifty (50) µL of the serum-virus mixture and control virus were added to pre-seeded Hep-2 cells (two replicates per condition). After incubation at 37°C with 5% CO₂ for approximately 2 hours, the medium was replaced with 100 µL fresh culture medium and incubated for approximately 22 hours.

### 6) Plate Reading

The supernatant was discarded, cells were fixed, and fluorescently labeled detection antibody was added. The plate was read using a CTL instrument.

### Data Interpretation

Neutralization activity was determined by comparison with the RSV virus control group. The ID₅₀ value (serum dilution at which 50% of RSV infection is inhibited) was calculated to represent neutralizing antibody potency.

### 2. Guangzhou Huawell Testing Co., Ltd. Method

**1)** Hep-2 cells were seeded into 96-well plates one day prior to the assay and grown to a confluent monolayer.
**2)** Heat-inactivated mouse sera were subjected to 2-fold serial dilutions in DMEM from 1:20 to 1:5120.
**3)** Frozen RSV stock was thawed and diluted in serum-free DMEM to the desired concentration. Fifty (50) µL of diluted virus was added to each diluted serum well and incubated at room temperature for 2 hours.
**4)** Virus dilutions (1×, 10×, 100×, and 1000×) were added to 96-well plates at 100 µL per well in 12 replicates per dilution for viral titration.
**5)** Cells were trypsinized, counted, and resuspended in DMEM containing 4% FBS and 1% antibiotics to a density of 1 × 10⁵ cells/mL.
6)After neutralization incubation, 100 µL of prepared cell suspension was added per well and cultured at 37°C, 5% CO₂ for 5 days. Cytopathic effects (CPE) were monitored and recorded.

### Acceptance Criteria

(1) Cell control (CC) wells must show no cytopathic effect.
(2) Virus control (VC) wells must exhibit cytopathic effect.
(3) Negative serum control (NC) neutralization titer must be <1:40.
(4) Positive serum control (PC) must fall within the specified range.
(5) Viral back-titration must yield 630-6310 TCID₅₀/mL (log TCID₅₀/mL within range).

Failure to meet any criterion invalidates the experiment.

### 3. Zhongke Guobang Testing Method

**1) Sample Preparation:** Serum samples were thawed at room temperature and heat-inactivated at 56°C for 30 minutes, then cooled to room temperature.
**2) Sample Dilution:** Initial dilution was 1:40, followed by 4-fold serial dilutions for six dilution levels. In a 96-well plate, column 2 served as cell control (CC), and column 3 as virus control (VC). Diluted samples were added to designated wells and serially diluted 4-fold. Positive and negative reference controls were processed in parallel.
**3) Virus Dilution:** Virus was diluted on ice to 800-1200 FFU per well and added to all wells except CC wells.
**4) Neutralization Incubation:** Plates were incubated at 37°C with 5% CO₂ for 1 hour.
**5) Cell Addition:** Hep-2 cells were prepared at 2.0 × 10⁵ cells/mL in complete medium and added at 100 µL per well.
**6) Detection:** After 24-28 hours incubation, GFP fluorescent foci were counted using a cell imaging multimode plate detection system.

### Data Analysis

Fluorescent foci counts were analyzed using a data analysis template, and 50% neutralization titers (ND₅₀) were calculated using the Reed-Muench method.

### 4. Fluorescence-Based Neutralization Assay

**1) Cell Preparation:** A549 cells were seeded into 96-well plates one day prior to assay and grown to confluence.
**2) Serum Dilution:** Heat-inactivated mouse sera were diluted 4-fold serially in DMEM from 1:25 to 1:1,638,400.
**3) Virus Preparation:** RSV-GFP virus was thawed from -80°C storage and diluted in serum-free DMEM to the desired concentration. Equal volumes of diluted virus were added to each serum dilution and incubated at room temperature for 1 hour.

### 4) Infection

Following neutralization incubation, plates were incubated at 37°C, 5% CO₂ for 2 days.

### 5) Detection

Green fluorescence signals were detected using an automated live-cell imaging system.

### Data Analysis

Cells expressing GFP were considered RSV-GFP infection-positive cells. Based on the proportion of GFP-positive cell area and serum dilution, NT₅₀ values were calculated by nonlinear regression.

### VII. Detection of Antigen-Specific T Cell Responses

Antigen-specific T cell responses in mouse splenocytes were evaluated by intracellular cytokine staining (ICS).

Briefly, RSV F peptide pools were added to wells of a 96-well plate. Culture medium containing an equivalent amount of DMSO was used as a negative control. Mouse splenocytes were resuspended in RPMI-1640 complete medium and added to the wells.

Cells were incubated at 37°C for 1 hour, followed by addition of a protein transport inhibitor, and further incubated for an additional 5 hours at 37°C.

Cells were washed once with PBS and stained with Fixable Viability Stain 510 (BD Biosciences, Cat. No. 564406) for 10 minutes. After washing, cells were incubated with anti-mouse CD16/CD32 antibody (BD Biosciences, Cat. No. 553142) at 4°C for 10 minutes to block Fc receptors.

Cells were then stained with surface marker antibodies, including anti-mouse CD3-FITC (Cat. No. 553061), CD4-APC (Cat. No. 553051), and CD8-PerCP-Cy5.5 (Cat. No. 551162) (all from BD Biosciences). After 30 minutes of incubation, cells were washed twice.

Subsequently, cells were fixed and permeabilized for 20 minutes, washed once, and stained intracellularly with a cytokine antibody mixture comprising anti-mouse IFN-γ-PE-Cy7 (Cat. No. 557649), IL-2-BV605 (Cat. No. 563943), TNF-α-BV650 (Cat. No. 563911), IL-4-BV711 (Cat. No. 564005), and IL-5-PE (Cat. No. 562049) (all from BD Biosciences).

After 30 minutes of incubation, cells were washed twice and resuspended in 200 µL PBS.

Fluorescence signals were acquired and analyzed using a CYTEK Aurora/NL flow cytometer (Model NL-CLC V16B14R8, Cytek Biosciences).

### VIII. Detection of Antigen-Specific T Cell Responses (ELISpot Assay)

### Day 1

### 1.Activation of Pre-Coated ELISpot Plates

For Dakewe (Dakewe Biotech) plates:
Add 200 µL per well of Dakewe ELISpot serum-free medium. Incubate at room temperature for 5-10 minutes and discard the medium.

For Mabtech plates:
Wash the plate four times with sterile 1× PBS. Add 200 µL per well of serum-free medium and incubate at room temperature for at least 30 minutes.

### 2. Cell Seeding and Stimulation

Add prepared splenocytes to the wells, followed by addition of the corresponding stimulant (e.g., RSV F peptide pool). Cover the plate and incubate at 37°C in a 5% CO₂ incubator for 23 hours. Avoid disturbance during incubation.

### Day 2

(Post-incubation procedures; sterile technique is no longer required.)

### 1. Cell Lysis

Discard the culture medium from the wells. Add 200 µL per well of pre-chilled deionized water and incubate at 4°C for 10 minutes to induce hypotonic cell lysis.

### 2. Preparation of Washing Buffer

Dilute the stock washing buffer 1:50 with deionized water to prepare 1× washing buffer. Prepare fresh buffer as needed and store at 4°C.

### 3. Plate Washing

Discard the liquid in the wells. Wash the plate six times with 250 µL per well of 1× washing buffer, allowing each wash to stand for 1 minute. After each wash, tap the plate dry on absorbent paper.

### 4. Detection Antibody Incubation

Add 100 µL per well of diluted biotinylated detection antibody working solution. Incubate at 37°C for 1 hour.

Preparation of reagents:
- Dilution Buffer R (10×) was diluted 1:9 with 1× PBS to obtain 1× Dilution Buffer R working solution.
- Biotinylated antibody was diluted 1:100 in 1× Dilution Buffer R to obtain the working solution.

### 5. Plate Washing

Wash the plate six times with 250 µL per well of 1× washing buffer, allowing each wash to stand for 1 minute. Tap dry after each wash.

### 6. Streptavidin-HRP Incubation

Add 100 µL per well of diluted streptavidin-HRP working solution and incubate at 37°C for 1 hour. Preparation of streptavidin-HRP working solution:
- Streptavidin-HRP was diluted 1:100 in 1× Dilution Buffer R.

### 7. Plate Washing

Wash the plate six times with 250 µL per well of 1× washing buffer, allowing each wash to stand for 1 minute. Tap dry after each wash.

### 8. Color Development

Add 100 µL per well of freshly prepared ACE substrate solution. Incubate at room temperature in the dark for 5-30 minutes. The reaction time was determined based on spot development.

If room temperature was below 20°C, color development was performed in a 37°C incubator, and spot formation was monitored every 5-10 minutes.

### Preparation of ACE Substrate Solution:

In a clean container, mix ACE dilution, ACE solution I (20×), ACE solution II (20×), and ACE solution III (200×) in a ratio of 180:10:10:1 to prepare the working solution (see Table 5, if applicable). The ACE substrate solution has a half-life of approximately 30 minutes at room temperature and should be prepared fresh immediately before use.

**Table 5. ACE Substrate Reaction System**

| 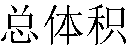 mL | ACE dilution/mL | ACE solution I (20×) /µL | ACE solution II (20×) /µL | ACE solution III (200×) /µL |
|---|---|---|---|---|
| 1 | 0.9 | 50 | 50 | 5 |
| 2 | 1.8 | 100 | 100 | 10 |
| 3 | 2.7 | 150 | 150 | 15 |
| 4 | 3.6 | 200 | 200 | 20 |
| 5 | 4.5 | 250 | 250 | 25 |
| 6 | 5.4 | 300 | 300 | 30 |
| 8 | 7.2 | 400 | 400 | 40 |
| 10 | 9 | 500 | 500 | 50 |

### 9. Termination of Color Development

The substrate solution was discarded from the wells. Each well, including the front and back surfaces of the membrane and the plate bottom, was washed three times with deionized water to terminate the color development reaction.

The plate was placed in a shaded area at room temperature and allowed to air dry completely. After drying, the plate bottom was reassembled.

### 10. ELISpot Spot Counting and Data Analysis

Spots on the ELISpot plate were counted using an ELISpot reader. Various spot parameters were recorded and subjected to statistical analysis.

If immediate reading was not possible, the plate strips were sealed and stored in the dark. It is recommended that plates be read within one week.

### IX. Preparation of FI-RSV (Formalin-Inactivated RSV) Vaccine

### 1. Cell and Reagent Preparation

### 1) Hep-2 Cells

Prepare ten (10) T75 culture flasks of Hep-2 cells at full confluence. Seed thirty (30) 10 cm culture dishes with 1.2 × 10⁷ cells per dish. When cell confluence exceeds 90%, proceed to the next step.

### 2) D10 Medium

1× DMEM supplemented with 10% fetal bovine serum (FBS) and 1% penicillin-streptomycin (PS).

### 3) SF-DMEM

1× DMEM supplemented with 1% penicillin-streptomycin (serum-free).

### 4) 10% Formalin Solution

Neutral formalin solution at 10%.

### 5) Aluminum Hydroxide Adjuvant

Al(OH)₃ adjuvant at 40 mg/mL.

### 2. Equipment

### I. Temperature-controlled shaking incubator

### II. Hitachi ultracentrifuge CP100NX

### 3. Experimental Procedure

1) When Hep-2 cells reached >90% confluence, wash once with SF-DMEM.
2) Dilute RSV-A2 (GenBank: KT992094) in SF-DMEM to achieve a multiplicity of infection (MOI) of 0.1.
3) Add 3 mL of diluted virus to each 10 cm dish and incubate at 37°C with 5% CO₂ for 1 hour to allow viral adsorption.
4) Remove the viral supernatant and wash cells once with SF-DMEM.
5) Add 10 mL SF-DMEM per 10 cm dish.
6) Incubate at 37°C with 5% CO₂ for 3 days until approximately 50% cytopathic effect (CPE) was observed.

(Example timeline: infection initiated on Aug. 10, 2024 at 15:30; amplification completed on Aug. 13, 2024 at 16:30.)

### 7) Virus Collection

Collect cell culture supernatant into 50 mL centrifuge tubes and centrifuge at 1,000 × g at 4°C for 10 minutes. Collect the clarified supernatant. Reserve 120 µL of viral suspension for viral titration.

### 8) Formalin Inactivation

Add 10% neutral formalin at a 1:400 ratio to the viral suspension in a shaking flask. Mix thoroughly, seal, and incubate on a shaking platform at 37°C, 50 rpm for 3 days to inactivate the virus.

### 9) Virus Concentration

After inactivation, collect the virus by ultracentrifugation at room temperature and resuspend in 10 mL PBS.

### 10) Adjuvant Adsorption

Add aluminum hydroxide adjuvant to a final concentration of 4 mg/mL and incubate at room temperature overnight to allow adsorption.

### 11) Final Preparation

Centrifuge at 1,000 × g for 10 minutes. Resuspend the pellet in 3 mL PBS containing 1% penicillin-streptomycin.

### Antigen Design

The wild-type RSV sequence (SEQ ID NO: 1) is as follows:

MELPILKTNAITTILAAVTLCFASSQNITEEFYQSTCSAVSKGYLSALRTGWYTSVITIELSNIKE

NKCNGTDAKVKLIKQELDKYKNAVTELQLLMQSTPAANSRARRELPRFMNYTLNNTKNTNVTL
SKKRKRRFLGFLLGVGSAIASGIAVSKVLHLEGEVNKIKSALLSTNKAVVSLSNGVSVLTSKVLDL
KNYIDKQLLPIVNKQSCSISNIETVIEFQQKNNRLLEITREFSVNAGVTTPVSTYMLTNSELLSLIND
MPITNDQKKLMSSNVQIVRQQSYSIMSIIKEEVLAYVVQLPLYGVIDTPCWKLHTSPLCTTNTKEG
SNICLTRTDRGWYCDNAGSVSFFPQAETCKVQSNRVFCDTMNSLTLPSEVNLCNIDIFNPKYDCKI
MTSKTDVSSSVITSLGAIVSCYGKTKCTASNKNRGIIKTFSNGCDYVSNKGVDTVSVGNTLYYVN
KQEGKSLYVKGEPIINFYDPLVFPSDEFDASISQVNEKINQSLAFIRKSDELLHNVNAGKSTTNIMIT
TIIIVIIVILLALIAVGLLLYCKARSTPVTLSKDQLSGINNIAFSN

The mutation sites involved in the 003m construct and extended variants are shown in Table 6 below.

**Table 6. Mutation Sites and Linker Sequences of 003m Construct**

| sequence | Mutations | **Linker Sequence** |
|---|---|---|
| 003m | S155C, S290C, S190F, S215P, S46G, E92D, D486C, D489C, A102C, S362C | GGSGGGS (SEQ ID NO: 2) |

In certain embodiments, the 003m-1×GCN4t construct comprises an insertion of the amino ac id sequence EDKIEEILSKIYHIENEIARIKKLIGEA (SEQ ID NO: 6) between amino acid residues 516 and 517 of SEQ ID NO: 14.

In certain embodiments, the 003m-2×GCN4t construct comprises an insertion of the amino ac id sequence EDKIEEILSKIYHIENEIARIKKLIGEATYHIENEIARIKKLIGEA (SEQ ID NO: 160) be tween amino acid residues 516 and 517 of SEQ ID NO: 14.

The extended sequences are shown in Table 7 below.

**Table 7. Extended Sequences**

| Sequence | Insertion site | Inserted Sequence (SEQ ID NO) |
|---|---|---|
| 1xGCN4t | V516 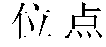 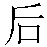 | EDKIEEILSKIYHIENEIARIKKLIGEA (SEQ ID NO: 6) |
| 2xGCN4t | V516 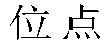 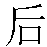 | EDKIEEILSKIYHIENEIARIKKLIGEATYHIENEIARIKKLIGEA (SEQ ID NO: 160) |

The mutation sites involved in the 006m construct and its derivative variants are shown in Table 8 below.

| 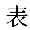 **Table 8. Mutation Sites and Linker Sequences of 006m Construct** | | |
|---|---|---|
| Sequence | Known Engineered (Artificial) Mutations | **Linker Sequence** |
| 006m | S155C, S290C, S190F, S215P, S46G, E92D, A102C, S362C | GGSGGGS (SEQ ID NO: 2) |

The mutation sites involved in the 007m construct and its derivative variants are shown in Table 9 below.

**Table 9. Mutation Sites and Linker Sequences of 007m Construct**

| Sequence | Known Engineered (Artificial) Mutations | **Linker Sequence** |
|---|---|---|
| 007m | S155C, S290C, S190F, S215P, S46G, E92D, D486C, D489C, A102C, S362C | GGSGGS (SEQ ID NO: 3) |

The mutation sites involved in the 008m construct and its derivative variants are shown in Table 10 below.

**Table 10. Mutation Sites and Linker Sequences of 008m Construct**

| Sequence | Known Engineered (Artificial) Mutations | **Linker Sequence** |
|---|---|---|
| 008m | S155C, S290C, S190F, S215P, S46G, E92D, D486C, D489C, A102C, S362C | GGSGS (SEQ ID NO: 4) |

The mutation sites involved in the 009m construct and its derivative variants are shown in Table 11 below.

**Table 11. Mutation Sites and Linker Sequences of 009m Construct**

| Sequence | Known Engineered (Artificial) Mutations | **Linker Sequence** |
|---|---|---|
| 009m | S155C, S290C, S190F, S215P, S46G, E92D, D486C, D489C, A102C, S362C | GSGS (SEQ ID NO: 5) |

The mutation sites involved in the 010m construct and its derivative variants are shown in Table 12 below.

**Table 12. Mutation Sites and Linker Sequences of 010m Construct**

| Sequence | Known Engineered (Artificial) Mutations | **Linker Sequence** |
|---|---|---|
| 010m | S155C, S290C, S190F, S215P, S46G, E92D, D486C, D489C, A102C, S362C | GGS |

DS-Cav1 and its derivative variants involve the mutation sites shown in Table 13 below.

**Table 13. Mutation Sites and Linker Sequences of DS-Cav1**

| Sequence | Known Engineered (Artificial) Mutations | **Linker Sequence** |
|---|---|---|
| DS-Cav1 | S155C, S290C, S190F, V207L | 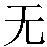 linker |

In certain embodiments, the DS-Cav1-1×GCNt construct comprises an insertion of the amino acid sequence EDKIEEILSKIYHIENEIARIKKLIGEA (SEQ ID NO: 6) between amino acid residues 516 and 517 of SEQ ID NO: 10.

In certain embodiments, the DS-Cav1-2×GCNt construct comprises an insertion of the amino acid sequence EDKIEEILSKIYHIENEIARIKKLIGEATYHIENEIARIKKLIGEA (SEQ ID NO: 160) between amino acid residues 516 and 517 of SEQ ID NO: 10.

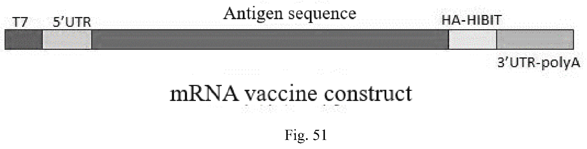

In the experiments described herein, the structure of all mRNA constructs is shown in FIG. 51.

The explanations of the sequence identifiers are provided in Table 14 below.

**Table 14. Explanation of Sequence Identifiers**

| **Type** | **Mutation Site** |
|---|---|
| _Cys | D486C/D489C |
| -ΔCys | C102A/C362A |
| -DS | A149C/Y458C |

### Example 2: Preliminary Antigen Screening

This example was conducted using the procedures described in Example 1 under Sections I (In Vitro Transcription and mRNA Transfection), II (In Vitro Flow Cytometry Analysis), and III (Western Blot Analysis).

The expression constructs encoding the respective antigens were synthesized by GenScript.

The nucleotide sequences encoding the respective antigens are set forth in SEQ ID NOs: 42, 43, 47, 51, 55, 71, and 182-200. The corresponding amino acid sequences are set forth in SEQ ID NOs: 10, 11, 16, 20, 24, 34, 161-169, 12, and 170-176.

(For the 003m construct, the coding nucleic acid sequence corresponds to SEQ ID NO: 42.)

### Results

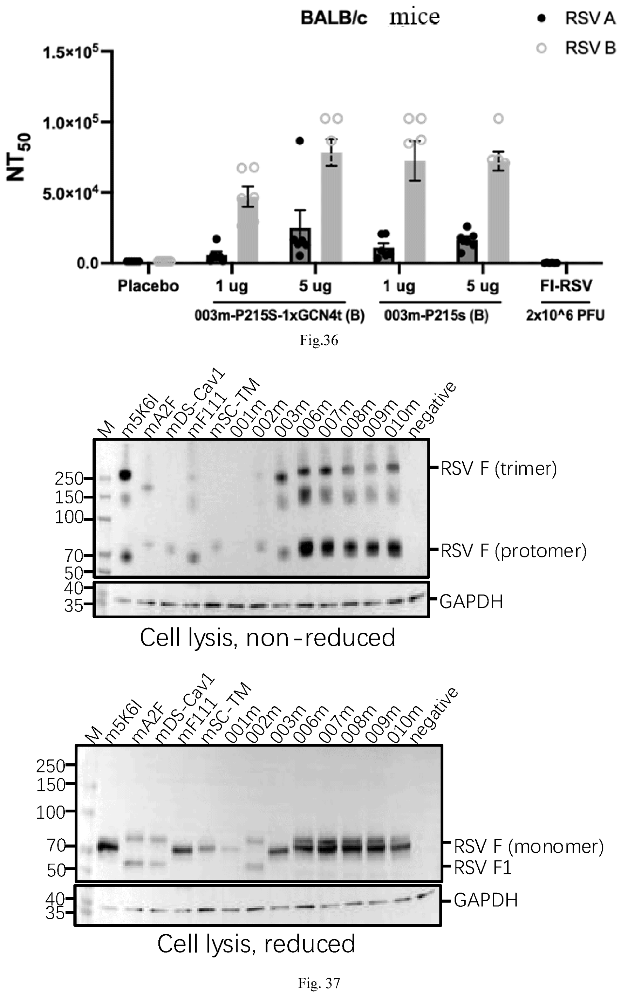

### Western Blot Analysis (FIG. 37)

Based on the Western blot results following in vitro transfection, constructs m5K6I, 006m-010m, and 003m exhibited relatively strong protein expression.

Due to the presence of the engineered linker in these mutant constructs, reducing gel electrophoresis demonstrated a predominant single-chain form. In contrast, in constructs lacking the linker, the F1 and F2 domains were clearly separated, indicating cleavage into distinct fragments.

### Flow Cytometry Analysis

Based on mean fluorescence intensity (MFI) values (see Table 15), membrane surface expression levels of m5K6I and 006m-010m antigens were relatively high. The membrane surface expression level of 003m was slightly lower than that of m5K6I and 006m-010m but remained substantial.

**Table 15. Flow Cytometry Fluorescence Data**

| | MFI | |
|---|---|---|
| | AM14-AF488 | D25-AF488 |
| | MFI | MFI |
| m5K6I | 722,697 | 807,465 |
| mA2F | 174,430 | 180,681 |
| DS-Cav1 | 121,804 | 158,906 |
| 001m | 4,277 | 7,748 |
| 002m | 162,155 | 172,409 |
| 003m | 343,168 | 310,437 |
| 006m | 1,414,101 | 1,265,475 |
| 007m | 1,223,057 | 1,097,166 |
| 008m | 1,282,164 | 1,153,472 |
| 009m | 1,104,242 | 994,374 |
| 010m | 863,866 | 801,590 |

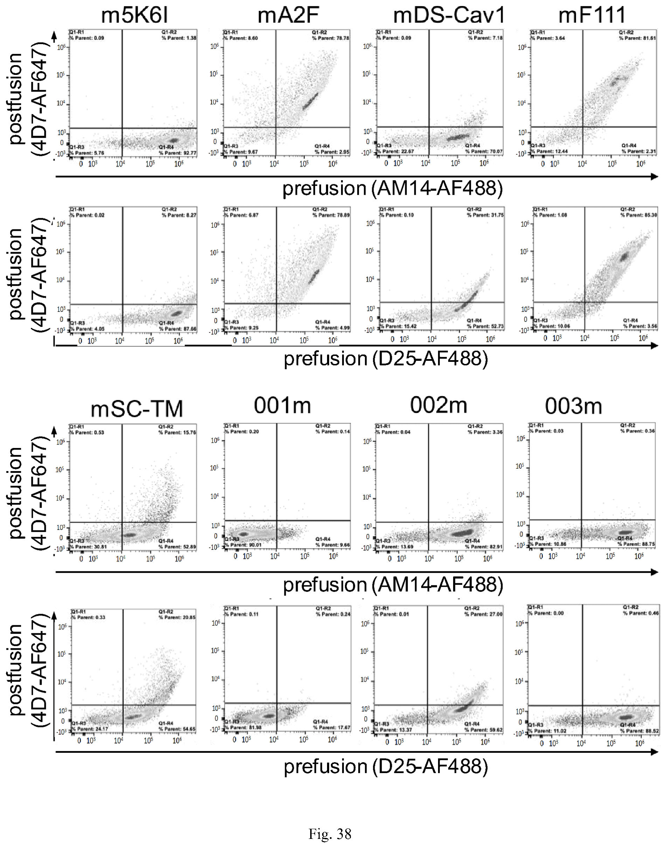

### Flow Cytometry Conformational Analysis (FIG. 38)

Three monoclonal antibodies were used for conformational analysis by flow cytometry: 4D7 (conjugated with Alexa Fluor^{®} 647), which specifically recognizes the postfusion conformation of RSV F protein; AM14 (conjugated with Alexa Fluor^{®} 488), which specifically recognizes the trimeric prefusion conformation; and

D25 (conjugated with Alexa Fluor^{®} 488), which recognizes the prefusion conformation.

The results demonstrate that mA2F (wild-type, WT) exhibits approximately comparable proportions of postfusion and prefusion conformations on the cell surface. In the DS-Cav1 construct, the majority of surface-expressed antigen is in the prefusion conformation; however, a detectable proportion of postfusion conformation remains present. In contrast, among the tested variants, the 003m construct displays surface expression predominantly in the prefusion conformation, with no appreciable postfusion conformation detected. By comparison, constructs 006m-010m, m5K6I, mF111, and mSC-TM exhibit a substantial proportion of postfusion conformation at the membrane surface.

### Example 3: Disulfide Bond Engineering of Antigen Constructs

This example was conducted using the procedures described in Example 1 under Sections I (In Vitro Transcription and mRNA Transfection), II (In Vitro Flow Cytometry Analysis), and III (Western Blot Analysis).

### Results

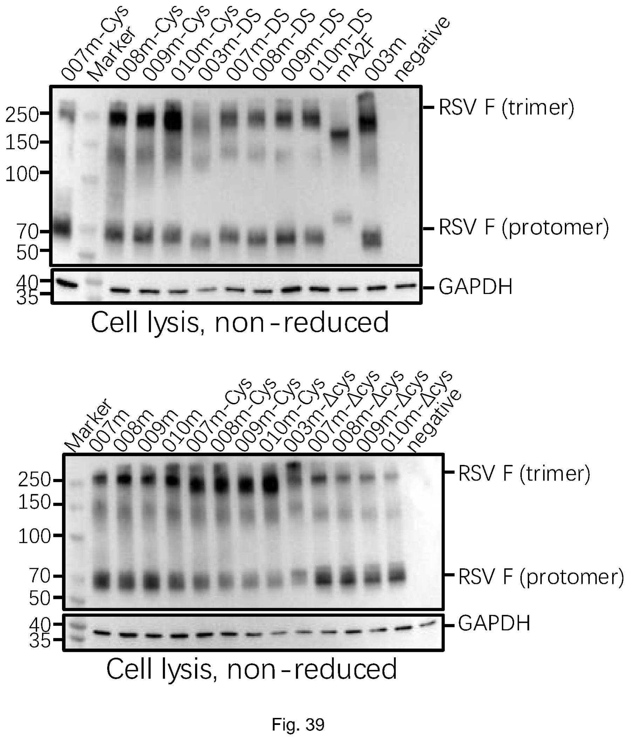

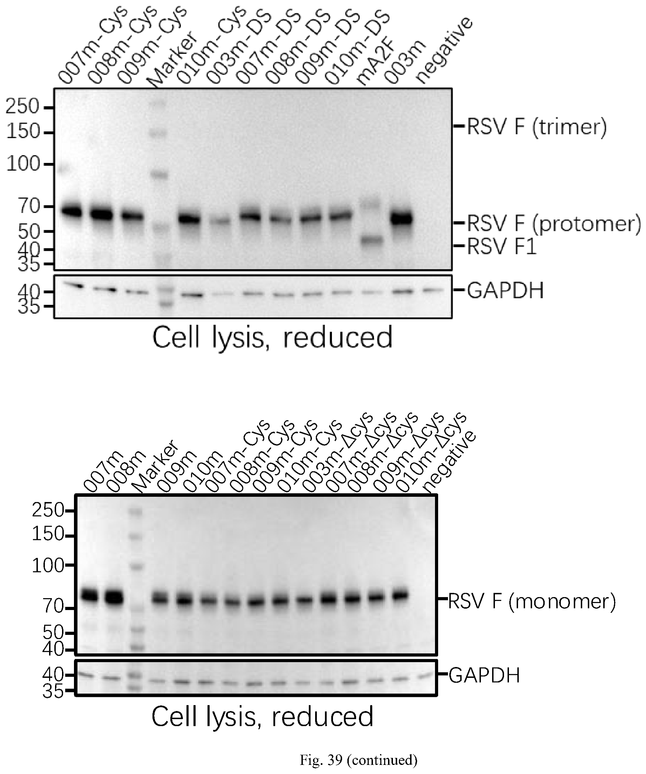

### Western Blot Analysis (FIG. 39)

Based on Western blot analysis, introduction of the D486C/D489C cysteine pair (forming an engineered disulfide bond) into constructs 007m-010m (renamed 007m_Cys-010m_Cys) did not result in a significant decrease in overall protein expression levels. In contrast, introduction of the A149C/Y458C cysteine pair into 003m and 007m-010m constructs (renamed 003m_DS and 007m_DS-010m_DS, respectively) led to a marked reduction in protein expression levels. Furthermore, removal of the A102C/S362C cysteine pair from 003m and 007m_Cys-010m_Cys constructs (renamed 003m_ΔCys and 007m_Cys_ΔCys-010m_Cys_ΔCys, respectively) also resulted in a significant decrease in expression levels.

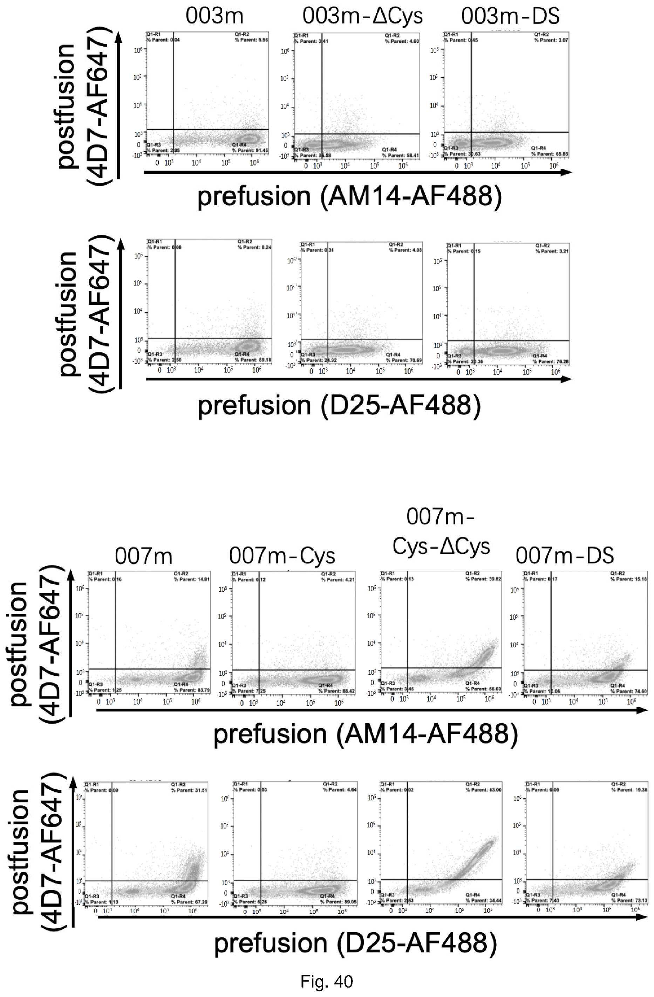

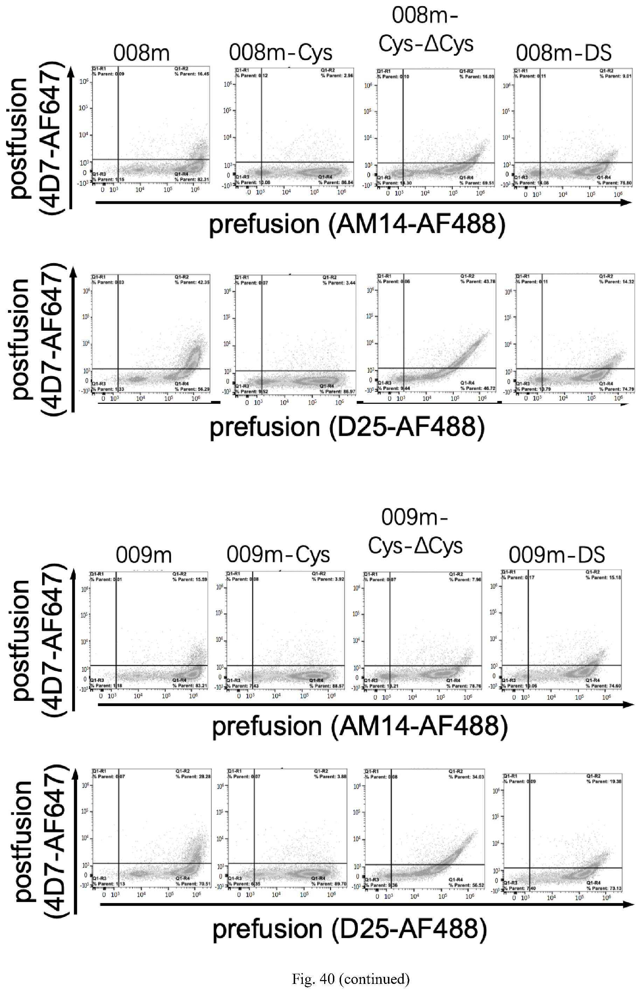

### Flow Cytometry Analysis (FIG. 40)

Flow cytometry results demonstrated that both 003m and 007m_Cys-010m_Cys maintained the prefusion conformation in a relatively stable manner. By contrast, constructs 007m-010m, 007m_DS-010m_DS, and 007m_Cys_ΔCys-010m_Cys_ΔCys failed to stably maintain the prefusion conformation. Although 003m_DS maintained the prefusion conformation, its membrane surface expression level was significantly reduced. Consistent with Western blot results, total protein expression was also substantially decreased. Quantitative flow cytometry data reflecting cell surface fluorescence intensity (see Table 16) correlate with relative levels of surface-expressed target protein.

### Technical Interpretation

None of the tested disulfide bond combinations resulted in a meaningful improvement in protein expression levels. These findings further demonstrate that the structural design of 003m achieves a favorable balance between prefusion stabilization and protein expression, indicating that the 003m construct represents an optimized configuration among the tested variants.

**Table 16. Flow Cytometry Fluorescence Data**

| | MFI | |
|---|---|---|
| | AM14-AF488 | D25- AF488 |
| mA2F | 122,346 | 146,362 |
| 003m | 589,889 | 450,784 |
| 007m | 664,232 | 610,001 |
| 008m | 792,760 | 523,303 |
| 009m | 787,108 | 530,344 |
| 010m | 603,389 | 410,226 |
| 007m-Cys | 610,001 | 229,085 |
| 008m-Cys | 257,869 | 196,326 |
| 009m-Cys | 247,491 | 199,337 |
| 010m-Cys | 333,282 | 252,137 |
| 003m-ΔCys | 8,385 | 16,226 |
| 007m-Cys-ΔCys | 429,540 | 369,002 |
| 008m-Cys-ΔCys | 181,415 | 173,009 |
| 009m-Cys-ΔCys | 148,302 | 135,832 |
| 010m-Cys-ΔCys | 153,323 | 135,924 |
| 003m-DS | 10,113 | 37,110 |
| 007m-DS | 143,885 | 159,057 |
| 008m-DS | 138,530 | 149,901 |
| 009m-DS | 146,853 | 154,418 |
| 010m-DS | 106,897 | 118,110 |

### Example 4: Extended Antigen Design

This example was conducted using the procedures described in Example 1 under Sections I (In Vitro Transcription and mRNA Transfection), II (In Vitro Flow Cytometry Analysis), and III (Western Blot Analysis).

In this study, extended variants were generated based on 003m and DS-Cav1 constructs by inserting either 1×GCN4t or 2×GCN4t sequences between amino acid residues 516 and 517 of the respective parent constructs.

### Results

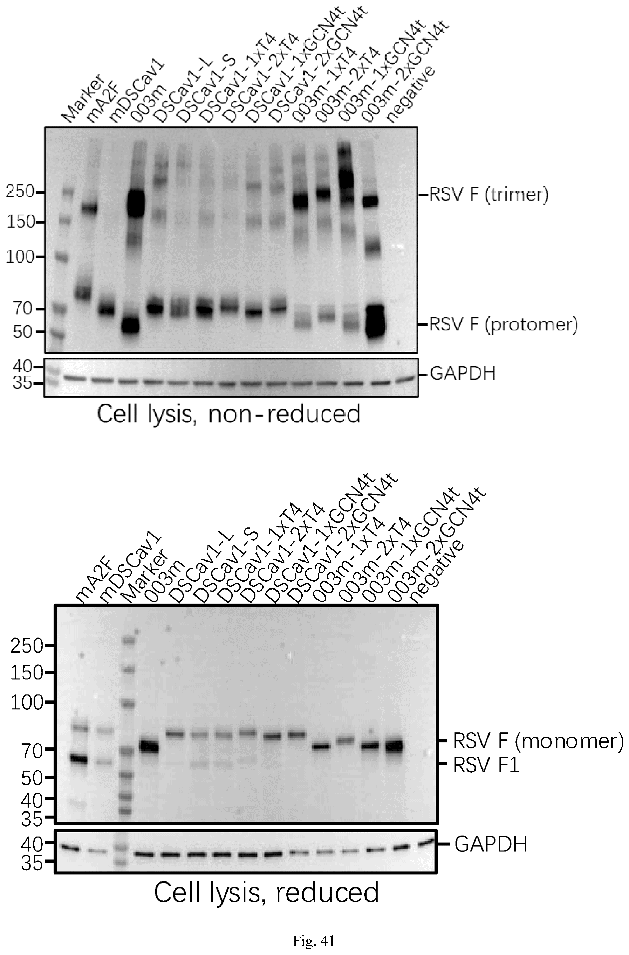

Western Blot Analysis (FIG. 41)

Western blot results indicated that, in the context of the 003m construct, the extended variants (1×GCN4t and 2×GCN4t insertions) exhibited a noticeable reduction in overall protein expression compared to the non-extended parent construct.

### Flow Cytometry Analysis

Based on mean fluorescence intensity (MFI) values (see Table 17), membrane surface antigen expression levels showed a clear downward trend following the extended design modifications.

### Technical Interpretation

These results indicate that insertion of additional GCN4t sequences at the 516/517 position may negatively affect antigen expression levels, particularly in the 003m background, suggesting that excessive structural extension can compromise expression efficiency despite potential trimer stabilization effects.

**Table 17. Flow Cytometry Fluorescence Data**

| | MFI | |
|---|---|---|
| | AM14-AF488 | D25- AF488 |
| | MFI | MFI |
| DS-Cav1 | 107,571 | 145,078 |
| DS-Cav1-1×GCN4t | 923 | 1,786 |
| DS-Cav1-2×GCN4t | 437 | 1,172 |
| 003m | 402,564 | 193,769 |
| 003m-1×GCN4t | 6,546 | 12,062 |
| 003m-2×GCN4t | 434,174 | 172,853 |

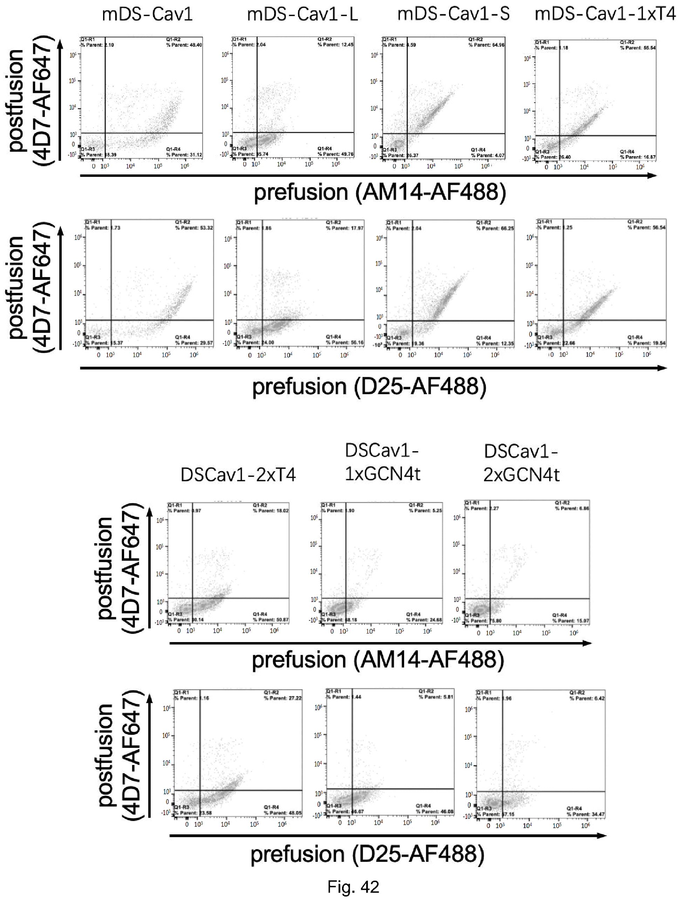

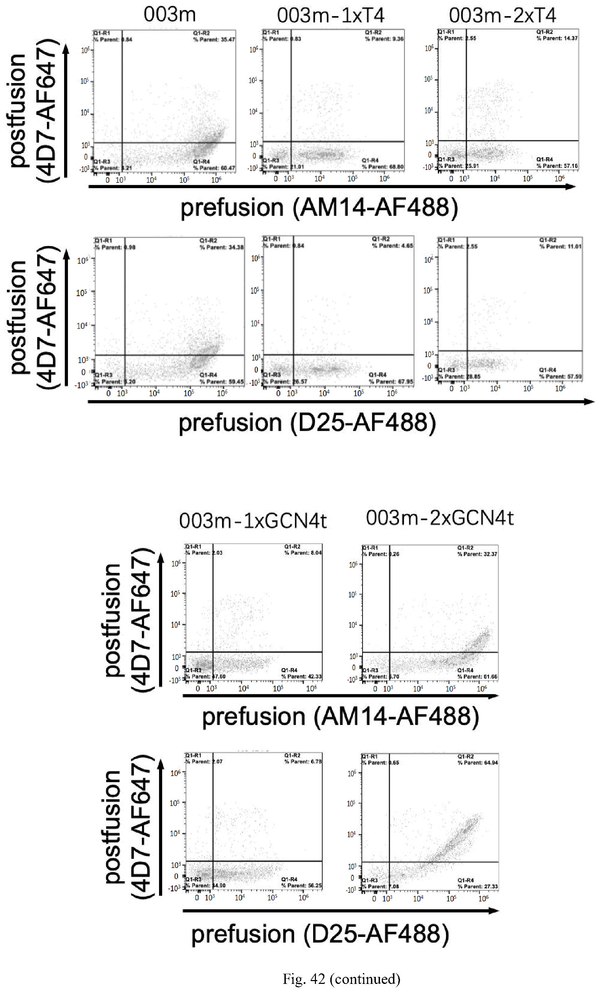

### Flow Cytometry Analysis (FIG. 42)

Flow cytometry results demonstrated that, following extension of the 003m construct, all variants except the 2×GCN4t insertion were able to maintain the prefusion conformation under in vitro expression conditions.

### Conclusion

The comparative results described above indicate that extension with GCN4t does not universally yield favorable outcomes across different antigen constructs, either in terms of conformational stability or expression level. In particular, certain constructs exhibited reduced membrane expression and/or compromised prefusion stability following extension.

Taken together, these findings demonstrate that the 003m construct provides a more suitable structural background for GCN4t-based extension design, achieving a favorable balance between maintenance of the prefusion conformation and preservation of expression levels.

### Example 5: Preparation of Lipid Nanoparticles (LNPs)

The mRNA encoding the selected antigen sequences described in the foregoing Examples was formulated into lipid nanoparticles (LNPs) under acidic conditions, wherein the mRNA was ionized (cationic state).The LNP formulation comprised two helper lipids, DSPC (1,2-distearoyl-sn-glycero-3-phosphocholine; Cat. No. B90536; Nippon Fine Chemical Co., Ltd.) and cholesterol (Cat. No. C00373; Nippon Fine Chemical Co., Ltd.), together with the ionizable lipid RL151 (Cat. No. W211-YB211202; Zhejiang Shenzhou Pharmaceutical Co., Ltd.) and a PEGylated lipid DMG-PEG2000 (Cat. No. M-DMG-2000; JenKem Technology).

### Preparation of mRNAAqueous Solution

mRNA dissolved in ultrapure water was mixed at a volume ratio of 1:1 with 100 mM citrate buffer (pH 4.0) to prepare the aqueous mRNA solution.

### Preparation of Lipid Solution

The four lipid components were dissolved in 99.5% ethanol to form a lipid solution. The molar ratio of the lipid components was adjusted as required. In certain embodiments, the molar ratio of ionizable lipid RL151 : cholesterol : DSPC : DMG-PEG2000 was 50 : 38.5 : 10 : 1.5.

### Microfluidic Mixing and Nanoparticle Formation

The aqueous mRNA solution and the lipid solution were mixed using a NanoAssemblr^{®} microfluidic mixing system (Precision Nanosystems). The mixing conditions included:
- Volume ratio (H₂O : EtOH) = 3 : 1
- Total flow rate = 12 mL/min

Lipid nanoparticles were prepared at a nitrogen-to-phosphate (N/P) ratio of ionizable lipid to mRNA ranging from 3:1 to 15:1.

The resulting formulation comprised mRNA encapsulated in lipid nanoparticles (mRNA-LNP).

### Post-Processing

The lipid nanoparticles were subjected to dialysis, concentration, and sterile filtration prior to storage, thereby yielding a vaccine formulation comprising the target antigen.

### Results

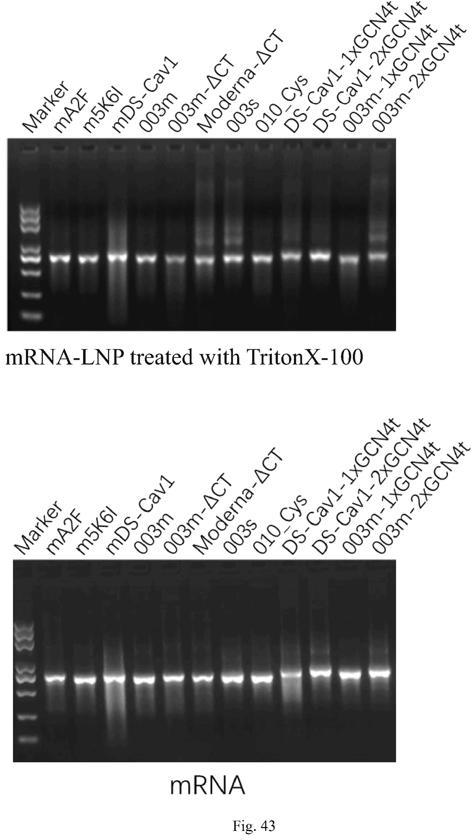

FIG. 43: Agarose gel electrophoresis analysis demonstrated that both purified mRNA and LNP-encapsulated mRNA exhibited no observable degradation, indicating that RNA integrity was preserved during formulation.

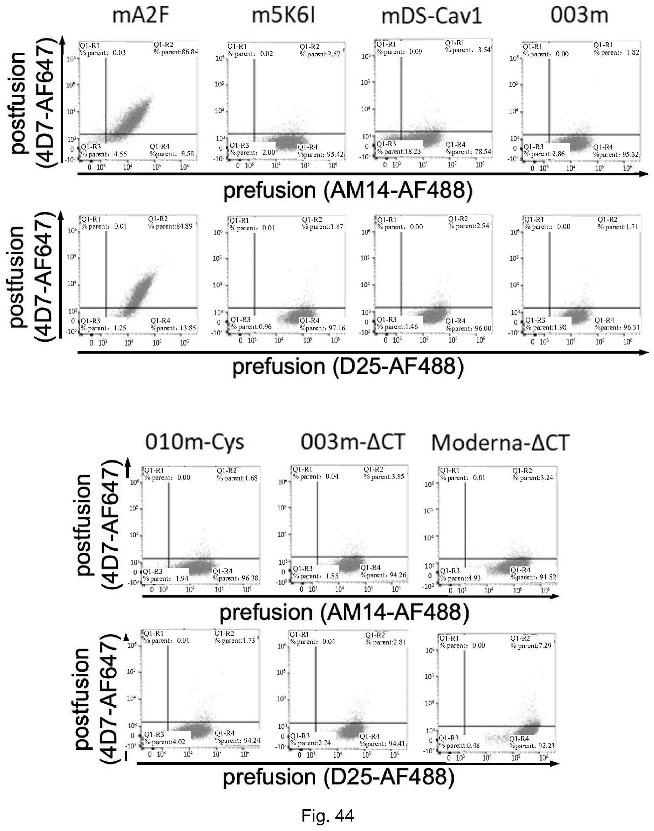

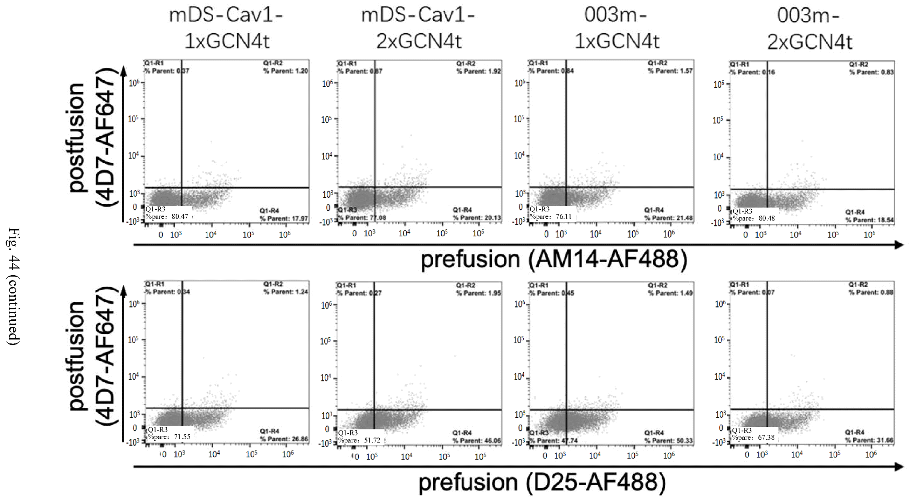

FIG. 44: Flow cytometry analysis was performed 20 hours after in vitro transfection of mRNA-LNP formulations. The results demonstrated that the membrane surface expression level of 003m-1×GCN4t was significantly lower than that of 003m, and was also markedly lower than that of m5K6I and Moderna-ΔCT. For both 003m and DS-Cav1 constructs, the 1×GCN4t extended variants exhibited slightly lower membrane surface expression levels compared to the corresponding 2×GCN4t extended variants. Notably, all extended variants of 003m and DS-Cav1 were able to effectively maintain the trimeric prefusion conformation. In addition, the membrane surface expression level of 10m-Cys was substantially comparable to that of 003m.

### Example 6: First Round of Animal Immunization

### I. Animal Vaccination and Serum Collection

### BALB/c Mice

For mouse immunization studies, the vaccine formulations comprising the target antigens prepared in Example 5 were administered by intramuscular injection to female BALB/c mice aged 5 to 7 weeks (purchased from Hangzhou Ziyuan Laboratory Animal Technology Co., Ltd.). A prime immunization was performed on Day 0, followed by a booster immunization on Day 21 to enhance the immune response. Each experimental group included six BALB/c mice (n = 6).

The immunization regimens are summarized in Table 18.

**Table 18. Immunization Regimen**

| **Group** | **Antigen Name** | **Dose (ug)** | **Administration Volume mL** | **Number of Animals (Male/Female)** | **Route of Administration** |
|---|---|---|---|---|---|
| A | mA2F | 0.5 ug | 0.05 | 6 (Female) | Intramuscular injection |
| A | mA2F | 2.5 ug | 0.05 | 6 (Female) | Intramuscular injection |
| B | m5K6I | 0.5 ug | 0.05 | 6 (Female) | Intramuscular injection |
| B | m5K6I | 2.5 ug | 0.05 | 6 (Female) | Intramuscular injection |
| C | DS-Cav1 | 0.5 ug | 0.05 | 6 (Female) | Intramuscular injection |
| C | DS-Cav1 | 2.5 ug | 0.05 | 6 (Female) | Intramuscular injection |
| D | 003m | 0.5 ug | 0.05 | 6 (Female) | Intramuscular injection |
| D | 003m | 2.5 ug | 0.05 | 6 (Female) | Intramuscular injection |
| E | 010m-Cys | 0.5 ug | 0.05 | 6 (Female) | Intramuscular injection |
| E | 010m-Cys | 2.5 ug | 0.05 | 6 (Female) | Intramuscular injection |
| F | 003m-ΔCT | 0.5 ug | 0.05 | 6 (Female) | Intramuscular injection |
| F | 003m-ΔCT | 2.5 ug | 0.05 | 6 (Female) | Intramuscular injection |
| G | Moderna-ΔCT | 0.5 ug | 0.05 | 6 (Female) | Intramuscular injection |
| G | Moderna-ΔCT | 2.5 ug | 0.05 | 6 (Female) | Intramuscular injection |
| H | 003S | 0.5 ug | 0.05 | 6 (Female) | Intramuscular injection |
| H | 003S | 2.5 ug | 0.05 | 6 (Female) | Intramuscular injection |
| K | Empty-LNP | NA | 0.05 | 6 (Female) | Intramuscular injection |
| L | PBS | NA | 0.05 | 6 (Female) | Intramuscular injection |
| Q | DS-Cav1-1×GCN4t | 0.5 ug | 0.05 | 6 (Female) | Intramuscular injection |
| Q | DS-Cav1-1×GCN4t | 2.5 ug | 0.05 | 6 (Female) | Intramuscular injection |
| R | DS-Cav1-2×GCN4t | 0.5 ug | 0.05 | 6 (Female) | Intramuscular injection |
| R | DS-Cav1-2×GCN4t | 2.5 ug | 0.05 | 6 (Female) | Intramuscular injection |
| S | 003m-1×GCN4t | 0.5 ug | 0.05 | 6 (Female) | Intramuscular injection |
| S | 003m-1×GCN4t | 2.5 ug | 0.05 | 6 (Female) | Intramuscular injection |
| T | 003m-2×GCN4t | 0.5 ug | 0.05 | 6 (Female) | Intramuscular injection |
| T | 003m-2×GCN4t | 2.5 ug | 0.05 | 6 (Female) | Intramuscular injection |
| U | Natural breeding | NA | 0.05 | 6 (Female) | Intramuscular injection |

A total of two immunizations were administered at an interval of three weeks. The dose for each administration is as set forth in Table 18.

An appropriately sized sterile insulin syringe was used to accurately withdraw 400 µL of the test article. Prior to withdrawal, the formulation was gently inverted 5-10 times to ensure homogeneity. Intramuscular injection was performed in the right hind limb of each animal, with a single injection volume of 50 µL per injection site.

Serum samples were collected from the immunized mice and heat-inactivated at 56°C for 30 minutes prior to analysis of RSV F protein-specific IgG antibodies and neutralizing antibody titers.

### II. Antibody Level Analysis

The total antibody level was determined using the method described in Example 1, Section V ("Total Antibody Level Detection"), specifically following the antigen-coating procedure described in Section 1.(1).

The neutralizing antibody assay was performed according to the method described in Example 1, Section VI ("Neutralizing Antibody Detection").

### Results

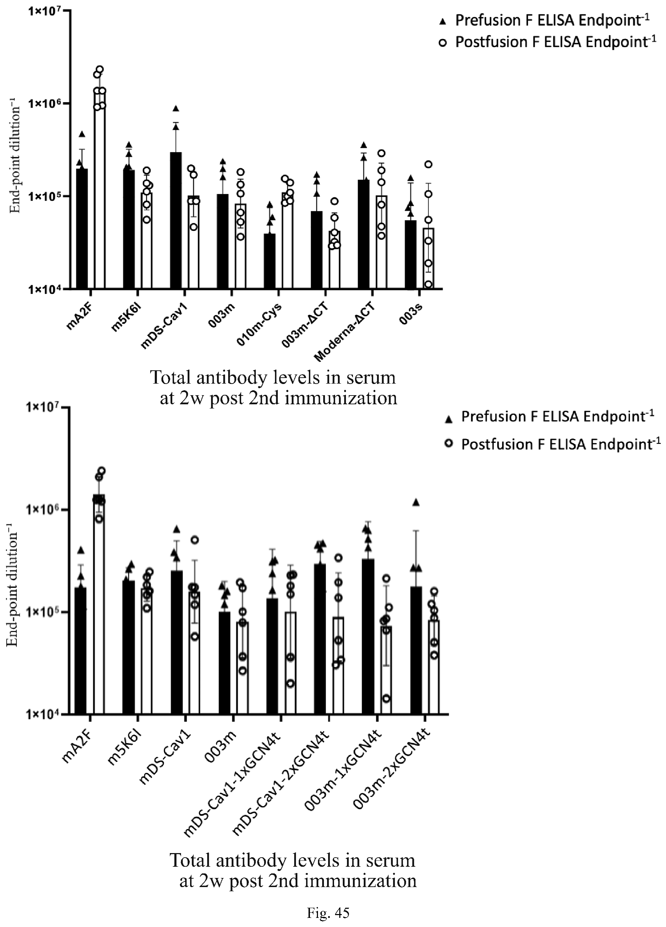

### Total Antibody Levels (FIG. 45)

FIG.45: Analysis of total serum antibody levels showed that mice immunized with mA2F (wild-type) predominantly produced antibodies directed against the postfusion conformation. In mice immunized with DS-Cav1, the highest total antibody levels were directed against the prefusion conformation, followed by m5K6I and Moderna-ΔCT, and then 003m. Notably, mice immunized with 003m produced the lowest total antibody levels against the postfusion conformation. The 003m-1×GCN4t construct induced a markedly higher level of total antibodies targeting the prefusion conformation compared to 003m and 003m-2×GCN4t. Meanwhile, the total antibody level directed against the postfusion conformation remained at a relatively low level.

### Neutralizing Antibody Results

Neutralization assay results (see Tables 19 and 20) demonstrated that 003m-1×GCN4t induced relatively high neutralizing antibody titers.

**Table 19. Neutralizing Antibody Titers in Serum Two Weeks After the Second Immunization**

| Sample name | HWAM (CPE Method) | Vazyme (Fluorescence Method) |
|---|---|---|
| DS-Cav1 | 320 | 115 |
| 003m | 640 | 1671 |
| DS-Cav1-2×GCN4t | 60 | 205 |
| 003m-1×GCN4t | 5120 | 6009 |
| Placebo | <40 | <20 |

**Table 20. Neutralizing Antibody Titers Against Different RSV Subtypes in Serum Two Weeks After the Second Immunization**

| Sample name | RSV A2 Subtype Neutralizing Antibody Titer (ND50, FRNT Assay) | RSV B Subtype Neutralizing Antibody Titer (ND50, FRNT Assay) |
|---|---|---|
| m5K6I | 5224 | 1757 |
| 010m-Cys | 4018 | 1952 |
| 003S | 1313 | 932 |
| Moderna-ΔCT | 6607 | 1542 |
| 003m-1xGCN4t | 9558 | 1929 |
| Placebo | <160 | <160 |

### III. Cellular Immune Responses in BALB/c Mice

The detection procedure was performed according to the method described in Example 1, Section VII ("Antigen-Specific T Cell Response Detection").

The intracellular cytokine staining (ICS) results were generally consistent with the serum antibody titer results. Across all experimental groups, the expression levels of IL-4 and IL-5 were relatively low, suggesting no apparent risk associated with Th2-skewed immune responses.

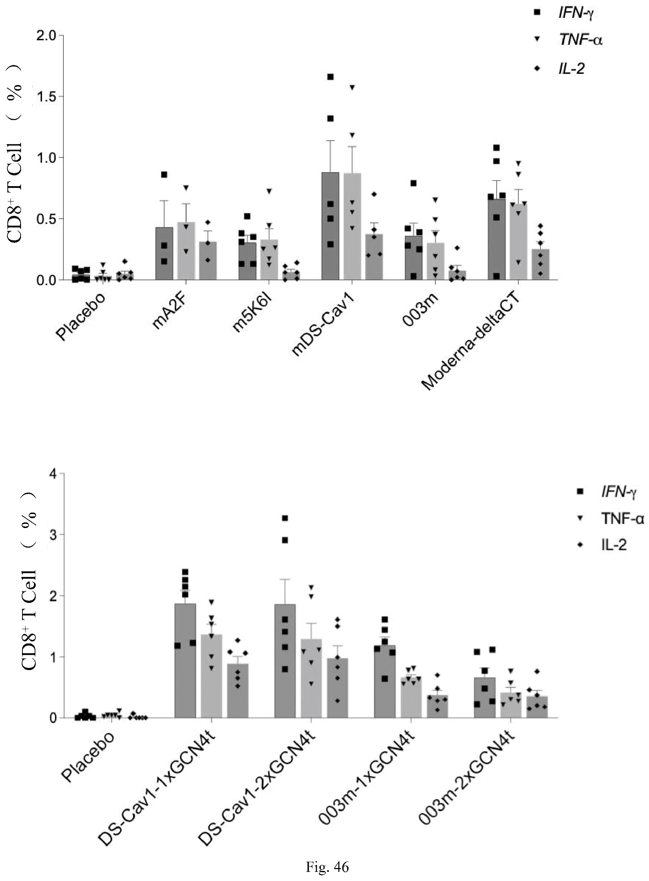

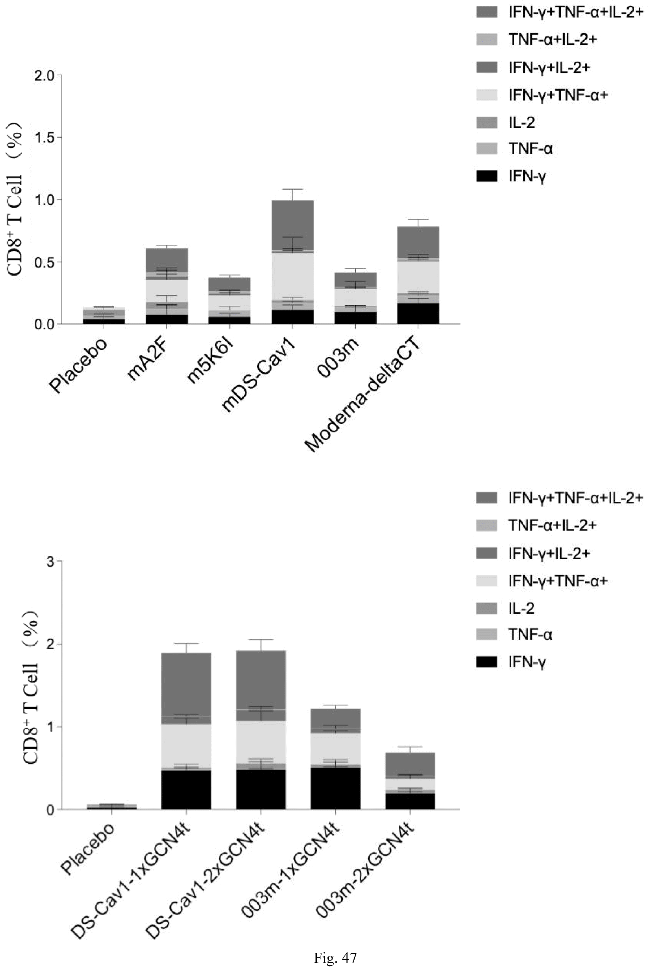

CD8⁺ T Cell Responses (FIGS. 46 and 47)

Analysis of single-cytokine and polyfunctional CD8⁺ T cell responses showed trends generally consistent with total antibody levels.

The cellular immune response induced by 003m was lower than that induced by m5K6I, DS-Cav1, and Moderna-ΔCT. In contrast, 003m-1×GCN4t induced a higher cellular immune response compared to 003m-2×GCN4t.

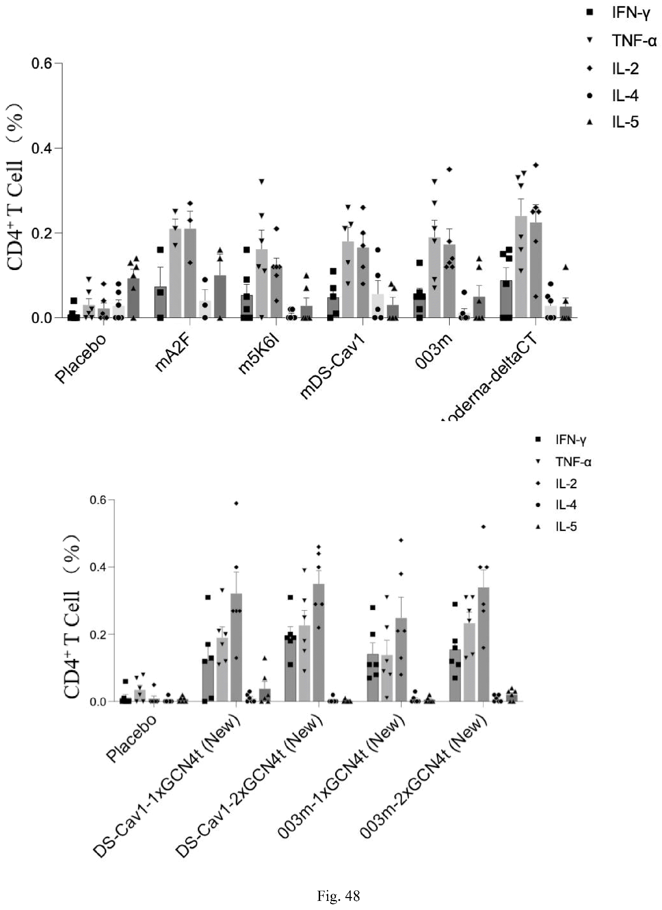

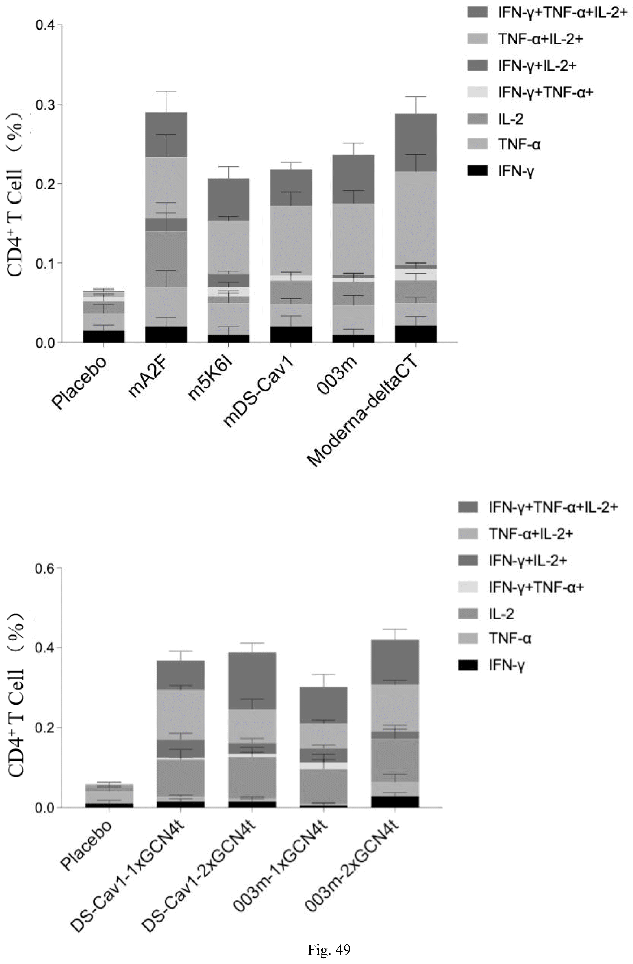

CD4⁺ T Cell Responses (FIGS. 48 and 49)

Analysis of single-cytokine and polyfunctional CD4⁺ T cell responses demonstrated that mA2F induced relatively higher levels of IL-4 and IL-5 compared to the other groups. The remaining groups exhibited lower IL-4 and IL-5 levels.

Among the extended variants, IL-4 and IL-5 levels were consistently low, indicating a reduced likelihood of vaccine-enhanced disease (VED).

### IV. Cellular Immune Responses in BALB/c Mice (ELISpot)

The detection procedure was performed in accordance with the method described in Example 1, Section VIII ("Antigen-Specific T Cell Response Detection (ELISpot)").

The analysis was conducted 95 days after the second immunization.

The immunization dose was 2.5 µg per mouse.

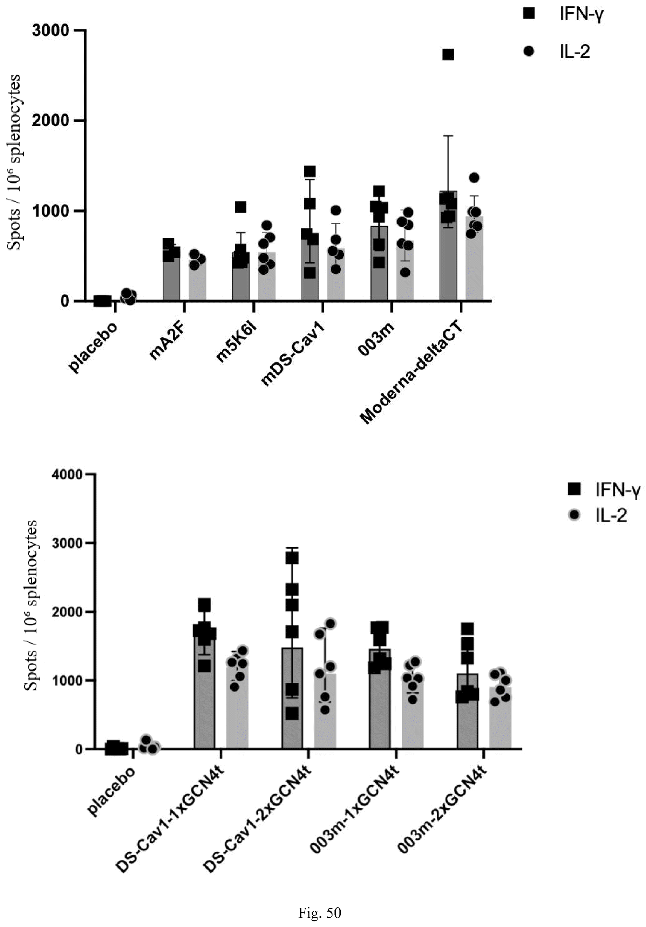

### Results (FIG. 50)

The ELISpot results were generally consistent with the intracellular cytokine staining (ICS) results.

Among the tested constructs, Moderna-ΔCT induced the highest levels of IFN-γ and IL-2.

For the extended variants of DS-Cav1, the two mutant constructs exhibited comparable results.

In contrast, among the extended variants of 003m, the 1×GCN4t construct showed slightly higher IFN-γ and IL-2 responses compared to the 2×GCN4t construct.

### Examples 7-18: Second-Round Screening and Optimization of RSV Antigens and Applications

### Example 7

### Effect of the P215S Reversion Mutation on Membrane Surface Conformation and Expression of RSV F Antigen

The following constructs were evaluated: 003m-STM, 007m-Cys-STM, 008m-Cys-STM, 009m-Cys-STM, 010m-Cys-STM, and related variants.

In these constructs, the STM modification refers to replacement of amino acid residues I525-N574 with the sequence WPWYIWLGFIAGLIAIVMVTIMLCCMTSCCSCLKGCCSCGSCCK (SEQ ID NO: 7).

### Results

### FIG. 1

The results demonstrated that insertion of 1×GCN4t and 1×GCN4t-STM extension designs led to a significant decrease in membrane surface expression of the RSV F antigen. In contrast, the STM modification alone did not result in a noticeable change in overall protein expression levels, and the modified proteins maintained the trimeric prefusion conformation. Additionally, variations in linker length did not produce significant differences in membrane surface expression levels. In all cases, the RSV F antigen maintained a stable trimeric prefusion conformation.

### FIG. 2

For the 003m construct, a P215S reversion mutation was introduced. Based on this background, further extended variants incorporating 1×GCN4t and 2×GCN4t were generated. Flow cytometry analysis showed that the P215S reversion mutation, as well as the 1×GCN4t and 2×GCN4t extensions, significantly reduced membrane surface expression of the RSV F antigen. The reduction in membrane expression was more pronounced in the extended variants (see Table 21).

Importantly, none of these modifications altered the prefusion trimeric conformation of the antigen.

**Table 21. Mean Fluorescence Intensity (MFI) of Different P215S Variant Designs Determined by In Vitro Flow Cytometry**

| Lipofectamine 2000-mRNA | | |
|---|---|---|
| Group | MFI | |
| | AM14-AF488 | D25-AF488 |
| 003m | 524529 | 805191 |
| 003m-P215S | 338141 | 270066 |
| 003m-P215S-1×GCN4t (ORIG) | 4151 | 8389 |
| 003m-P215S-2×GCN4t | 2681 | 5115 |

### Example 8

### Effect of Different Extension Designs and Sequence Optimization on Antigen Expression and Conformation

The S-modified and L-modified constructs were generated by introducing mutation L513I together with insertion of specific extension sequences.
- 003M-P215S-S: insertion of the sequence NEKINQISASIRKIDESISQI (SEQ ID NO: 8) between residues V516 and N517.
- 003M-P215S-L: insertion of the sequence
   NEKINQISASIRKIDESINEKINQISASIRKIDESISQI (SEQ ID NO: 9) between residues V516 and N517.

Based on these constructs, additional variants were generated: 003M-P215S-S-3N and 003M-P215S-L-3N, in which selected amino acid residues within the inserted sequences were modified to introduce N-linked glycosylation sites.

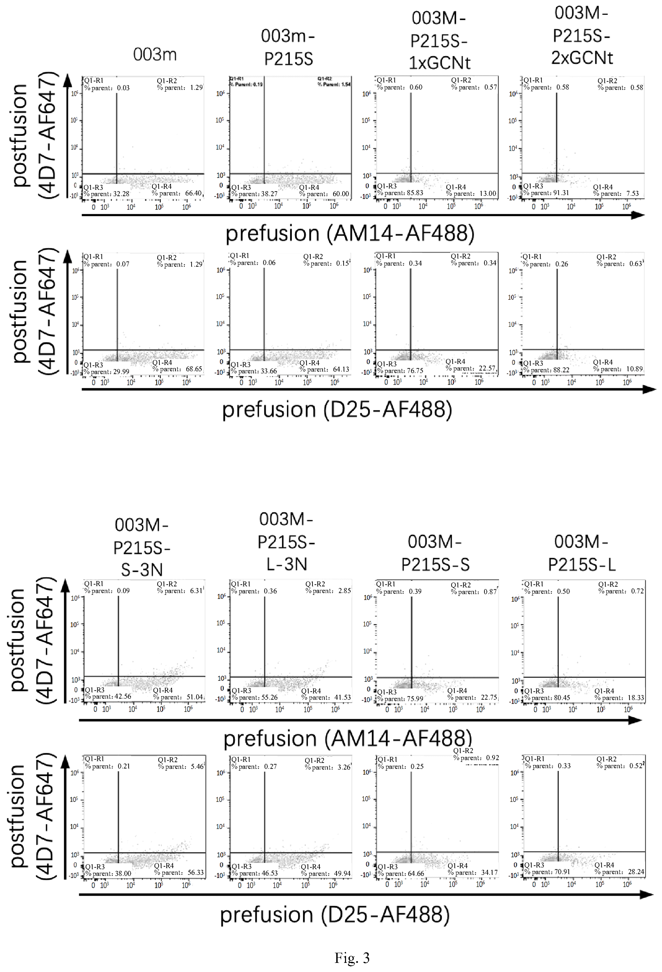

### Results (FIG. 3)

Comparative analysis of 003M-P215S-S versus 003M-P215S-S-3N, and 003M-P215S-L versus 003M-P215S-L-3N, demonstrated that introduction of additional N-linked glycosylation sites significantly increased membrane surface expression of the RSV F antigen. The membrane surface expression levels of the glycosylation-modified constructs were substantially higher than those observed for the 1×GCN4t and 2×GCN4t extended variants (see Table 22). Importantly, the trimeric prefusion conformation of the RSV F antigen was not noticeably altered by the glycosylation modifications.

**Table 22. Mean Fluorescence Intensity (MFI) of Different Extension Designs Determined by In Vitro Flow Cytometry**

| Lipofectamine 2000-mRNA | | |
|---|---|---|
| Group | MFI | |
| | AM14-AF488 | D25-AF488 |
| 003m | 155700 | 127563 |
| 003m-P215S | 90092 | 77226 |
| 003M-P215S-S-3N | 42395 | 48842 |
| 003M-P215S-L-3N | 17463 | 23442 |
| 003M-P215S-S | 5616 | 8917 |
| 003M-P215S-L | 4540 | 5815 |
| 003M-P215S-1×GCNt (ORIG) | 2562 | 3840 |
| 003M-P215S-2×GCNt | 1887 | 2151 |

### Example 9

### Screening of Codon Optimization Strategies

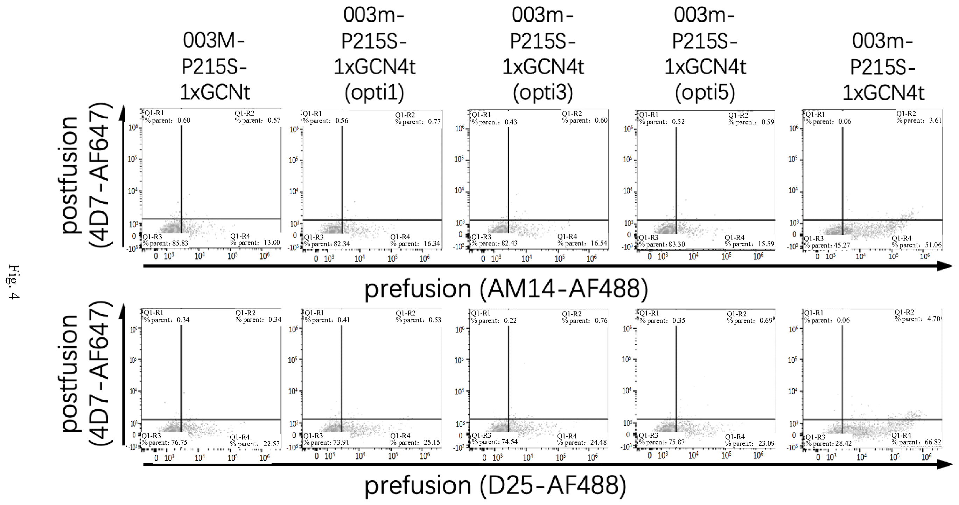

### Results (FIG. 4)

Based on the in vitro flow cytometry analysis and corresponding mean fluorescence intensity (MFI) values (see Table 23), transfection of cells with codon-optimized 003m-P215S-1×GCN4t resulted in a significant increase in membrane surface antigen expression compared to the non-optimized construct.

These results demonstrate that codon optimization effectively enhances antigen expression without adversely affecting membrane localization.

**Table 23. Mean Fluorescence Intensity (MFI) of 003m-P215S-1×GCN4t Constructs with Different Codon Optimization Strategies Determined by In Vitro Flow Cytometry**

| Lipofectamine 2000-mRNA | | |
|---|---|---|
| Group | MFI | |
| | AM14-AF488 | D25-AF488 |
| 003m-P215S-1×GCN4t | 30070 | 54218 |
| 003m-P215S-1×GCN4t-opti1 | 3382 | 5685 |
| 003m-P215S-1×GCN4t-opti3 | 3426 | 4992 |
| 003m-P215S-1×GCN4t-opti5 | 2976 | 4620 |
| 003M-P215S-1×GCN4t (ORIG) | 2562 | 3840 |

### Example 10

### Screening of Intermolecular Disulfide Bond Designs

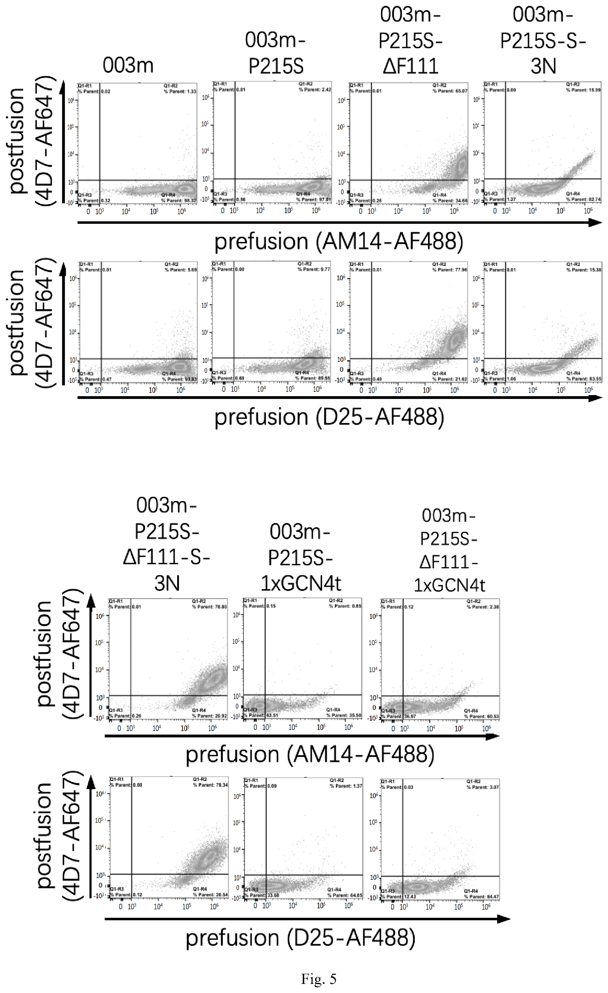

### Results (FIG. 5)

In vitro transfection was performed using Lipofectamine^{®} 2000. Upon introduction of reversion mutations at residues C486 and C489, thereby disrupting the intermolecular disulfide bond (hereinafter referred to as ΔF111), a significant increase in membrane surface expression of the RSV F antigen was observed (see Table 24).

However, the ΔF111 modification also resulted in the generation of a portion of antigen in the postfusion conformation.

In addition, all extended constructs exhibited a noticeable decrease in membrane surface expression levels under these conditions.

**Table 24. Mean Fluorescence Intensity (MFI) of Intermolecular Disulfide Bond Designs in Different Mutant Constructs Determined by In Vitro Flow Cytometry (Lipofectamine Transfection)**

| Lipofectamine 2000-mRNA | | |
|---|---|---|
| Group | MFI | |
| | AM14-AF488 | D25-AF488 |
| 003m-P215S-ΔF111 | 1517578 | 1020133 |
| 003m | 1190743 | 740253 |
| 003m-P215S-ΔF111-S-3N | 1083345 | 704379 |
| 003m-P215S | 759207 | 485917 |
| 003m-P215S-S-3N | 127325 | 148175 |
| 003m-P215S-ΔF111-1×GCN4t | 12459 | 14664 |
| 003m-P215S-1×GCN4t | 5651 | 8628 |

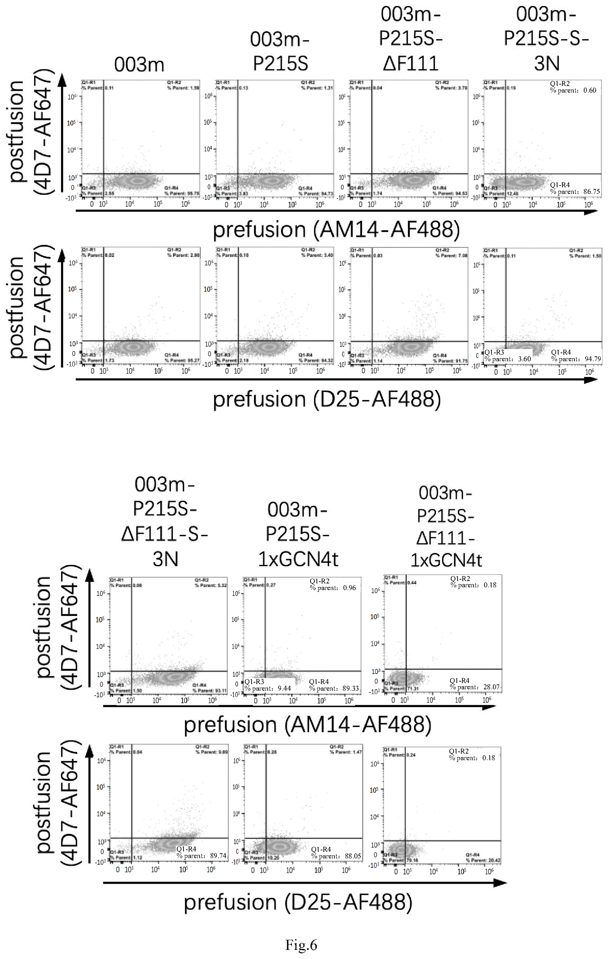

### Results (FIG. 6)

In vitro transfection was performed using mRNA-LNP formulated with RL151 (Cat. No. W211-YB211202; Zhejiang Shenzhou Pharmaceutical Co., Ltd.). The preparation method of the mRNA-LNP formulation was as described in Example 8, except that LQ104-E16b-2 was replaced with RL151.

Compared to Lipofectamine^{®} 2000-mediated transfection, the mRNA-LNP transfection resulted in a relatively lower level of membrane surface protein expression (see Table 25).

Nevertheless, all antigen designs maintained a stable trimeric prefusion conformation.

Following introduction of the ΔF111 reversion mutation, membrane surface expression levels of the RSV F antigen were still significantly increased relative to the corresponding non-reverted constructs in most designs, with the exception of 003m-P215S-ΔF111-1×GCN4t.

**Table 25. Mean Fluorescence Intensity (MFI) of Intermolecular Disulfide Bond Designs in Different Mutant Constructs Determined by In Vitro Flow Cytometry (mRNA-LNP Transfection)**

| mRNA-LNP (RL151) | | |
|---|---|---|
| Group | MFI | |
| | AM14-AF488 | D25-AF488 |
| 003m-P215S-ΔF111-S-3N | 49133 | 44838 |
| 003m-P215S-ΔF111 | 45151 | 37304 |
| 003m-P215S | 19984 | 16422 |
| 003m | 20082 | 14298 |
| 003m-P215S-S-3N | 5920 | 6733 |
| 003m-P215S-1×GCN4t | 4179 | 3883 |
| 003m-P215S-ΔF111-1×GCN4t | 821 | 682 |

### Example 11

### Optimization of RNA Secondary Structure

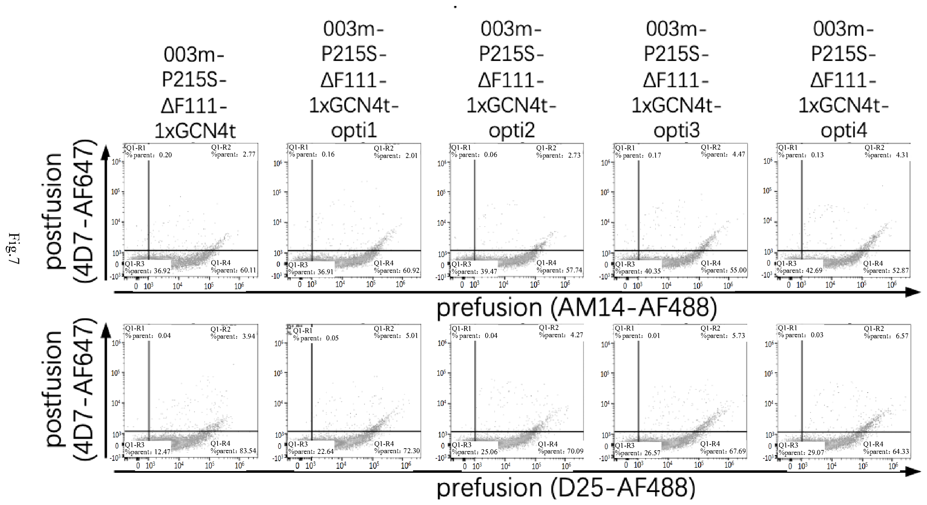

### Results (FIG. 7)

When Lipofectamine^{®} 2000-mediated mRNA transfection was employed, optimization of RNA secondary structure had a limited effect on membrane surface expression levels (see Table 26).

In addition, a portion of the RSV F antigen remained in the postfusion conformation despite RNA secondary structure optimization.

**Table 26. Mean Fluorescence Intensity (MFI) of Different RNA Secondary Structure Optimization Designs Determined by In Vitro Flow Cytometry (Lipofectamine Transfection)**

| Lipofectamine 2000-mRNA | | |
|---|---|---|
| Group | MFI | |
| | AM14-AF488 | D25-AF488 |
| 003m-P215S-ΔF111-1×GCN4t-opti3 | 15747 | 20368 |
| 003m-P215S-ΔF111-1×GCN4t-opti4 | 15740 | 19445 |
| 003m-P215S-ΔF111-1×GCN4t-opti2 | 14738 | 17215 |
| 003m-P215S-ΔF111-1×GCN4t-opti1 | 12739 | 14886 |
| 003m-P215S-ΔF111-1×GCN4t | 12472 | 14632 |

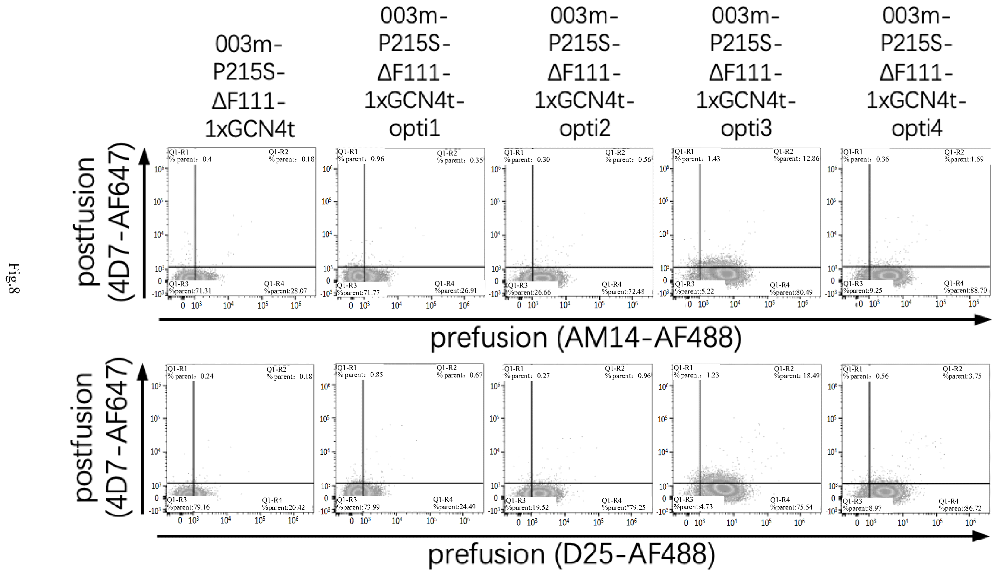

### Results (FIG. 8)

When in vitro transfection was performed using mRNA-LNP formulated with RL151, optimization of RNA secondary structure had a pronounced effect on membrane surface expression levels.

Specifically, the high-expression group exhibited approximately an eight-fold increase in membrane surface expression compared to the low-expression group (see Table 27).

The antigen protein maintained a stable trimeric prefusion conformation under these conditions. However, overall membrane surface expression levels were substantially lower than those observed with Lipofectamine^{®} 2000-mediated transfection.

**Table 27. Mean Fluorescence Intensity (MFI) of Different RNA Secondary Structure Optimization Designs Determined by In Vitro Flow Cytometry (mRNA-LNP Transfection)**

| mRNA-LNP (RL151) | | |
|---|---|---|
| Group | MFI | |
| | AM14-AF488 | D25-AF488 |
| 003m-P215S-ΔF111-1×GCN4t-opti3 | 6084 | 5832 |
| 003m-P215S-ΔF111-1×GCN4t-opti4 | 4132 | 3705 |
| 003m-P215S-ΔF111-1×GCN4t-opti2 | 2366 | 2193 |
| 003m-P215 S-ΔF111-1×GCN4t-opti1 | 844 | 797 |
| 003m-P215S-ΔF111-1×GCN4t | 821 | 682 |

### Example 12

### Preparation of Lipid Nanoparticles for BALB/c Mouse Immunization Samples

The mRNA sequences selected from Examples 7-11, including mDS-Cav1, 003m-1×GCN4t, 003m-1×GCN4t-STM, 003m-STM, 003m-P215S, 003m-P215S-ΔF111, 003m-P215S-S-3N, 003m-P215S-ΔF111-S-3N, 003m-P215S-1×GCN4t, and 003m-P215S-ΔF111-1×GCN4t-opti3, were formulated into lipid nanoparticles (LNPs) under acidic conditions.

Under low pH conditions, the mRNA was ionized (cationic state) and encapsulated using two helper lipids, DSPC (1,2-distearoyl-sn-glycero-3-phosphocholine; Cat. No. B90536; Nippon Fine Chemical Co., Ltd.) and cholesterol (Cat. No. C00373; Nippon Fine Chemical Co., Ltd.), together with the ionizable lipid LQ104-E16b-2 (as described in CN117534584A) and a PEGylated lipid DMG-PEG2000 (Cat. No. M-DMG-2000; JenKem Technology).

### Preparation of mRNAAqueous Solution

mRNA dissolved in ultrapure water was mixed at a 1:1 (v/v) ratio with 100 mM citrate buffer (pH 4.0) to form the aqueous mRNA solution.

### Preparation of Lipid Solution

The lipid components were dissolved in 99.5% ethanol to form a lipid solution. In certain embodiments, the molar ratio of LQ104-E16b-2 : cholesterol : DSPC : DMG-PEG2000 was 50 : 38.5 : 10 : 1.5.

### Microfluidic Mixing and LNP Formation

The aqueous mRNA solution and the ethanol lipid solution were mixed using a NanoAssemblr^{®} microfluidic mixing system (Precision Nanosystems) under the following conditions:
- Volume ratio (H₂O : EtOH) = 3 : 1
- Total flow rate = 12 mL/min

Lipid nanoparticles were prepared at a nitrogen-to-phosphate (N/P) ratio of ionizable lipid to mRNA ranging from 3:1 to 15:1.

The resulting formulation comprised mRNA encapsulated within lipid nanoparticles (mRNA-LNP).

### Post-Processing

The lipid nanoparticles were subjected to dialysis, concentration, and sterile filtration prior to storage, thereby yielding vaccine formulations containing the target antigen.

### Example 13

### Immunization of BALB/c Mice

### I. Animal Vaccination and Serum Collection

### BALB/c Mice

For mouse immunization studies, the vaccine formulations comprising the target antigens prepared in Example 12 were administered by intramuscular injection to female BALB/c mice aged 5 to 7 weeks (purchased from SPF (Suzhou) Biotechnology Co., Ltd.).

A prime immunization was performed on Day 0, followed by a booster immunization on Day 21 to enhance the immune response.

Each experimental group consisted of 8 or 14 BALB/c mice (n = 8 or 14).

The immunization regimens are summarized in Table 28.

**Table 28. Immunization Schedule for BALB/c Mice**

| Antigen Name | Immunization Dose (µg | Number of Animals (Female) | Administration Volume (mL) | Route of Administration |
|---|---|---|---|---|
| Placebo | NA | 14 | 0.1 | Intramuscular injection |
| mDS-Cav 1 | 1 µg | 8 | 0.1 | Intramuscular injection |
| | 5 µg | 8 | 0.1 | Intramuscular injection |
| 003m -STM | 1 µg | 8 | 0.1 | Intramuscular injection |
| | 5 µg | 8 | 0.1 | Intramuscular injection |
| 003m-1×GCN4t | 1 µg | 14 | 0.1 | Intramuscular injection |
| | 5 µg | 14 | 0.1 | Intramuscular injection |
| 003m-1×GCN4t-STM | 1 µg | 14 | 0.1 | Intramuscular injection |
| | 5 µg | 14 | 0.1 | Intramuscular injection |
| 003m-P215S | 1 µg | 8 | 0.1 | Intramuscular injection |
| | 5 µg | 8 | 0.1 | Intramuscular injection |
| 003m-P215S-ΔF111 | 1 µg | 14 | 0.1 | Intramuscular injection |
| | 5 µg | 14 | 0.1 | Intramuscular injection |
| 003m-P215S-S-3N | 1 µg | 8 | 0.1 | Intramuscular injection |
| | 5 µg | 8 | 0.1 | Intramuscular injection |
| 003m-P215S-ΔF111-S-3N | 1 µg | 14 | 0.1 | Intramuscular injection |
| | 5 µg | 14 | 0.1 | Intramuscular injection |
| 003m-P215S-1×GCN4t | 1 µg | 8 | 0.1 | Intramuscular injection |
| | 5 µg | 8 | 0.1 | Intramuscular injection |
| 003m-P215S-ΔF111-1×GCN4t-opti3 | 1 µg | 8 | 0.1 | Intramuscular injection |
| | 5 µg | 8 | 0.1 | Intramuscular injection |

A total of two immunizations were administered at an interval of three weeks. The dose for each administration is as set forth in Table 28.

An appropriately sized sterile insulin syringe was used to accurately withdraw 400 µL of the test article. Prior to withdrawal, the formulation was gently inverted 5-10 times to ensure homogeneity. Intramuscular injection was performed in the right hind limb of each animal, with a single injection volume of 100 µL per injection site.

Serum samples were collected from the immunized mice and heat-inactivated at 56°C for 30 minutes prior to analysis of RSV F protein-specific IgG antibodies and neutralizing antibody titers.

### II. Antibody Level Analysis

### 1. Total Antibody Levels

The total antibody assay was performed according to the method described in Example 1, Section III ("Total Antibody Level Detection"). The neutralizing antibody assay was conducted according to Example 1, Section IV ("Neutralizing Antibody Detection").

### FIG. 9

Analysis of total serum antibody levels showed that, among all antigen designs containing 1×GCN4t, the levels of antibodies directed against the postfusion conformation were relatively low.

Among these constructs, 003m-1×GCN4t and 003m-P215S-1×GCN4t produced the highest ratios of prefusion-specific antibodies to postfusion-specific antibodies.

Although antigen designs incorporating the ΔF111 reversion mutation exhibited relatively high expression levels in vitro, they did not induce correspondingly high total antibody levels in vivo. In addition, because ΔF111-containing antigens displayed a portion of postfusion conformation protein in vitro, the in vivo total antibody results also indicated increased production of antibodies directed against the postfusion conformation.

### 2. Neutralizing Antibody Titers

### FIG. 10

Neutralization assay results demonstrated that, in the high-dose group, mice immunized with 003m-P215S-1×GCN4t generated the highest neutralizing antibody titers. This was followed by 003m-P215S, 003m-P215S-S-3N, and 003m-P215S-ΔF111-S-3N.

### III. Cellular Immune Responses in BALB/c Mice

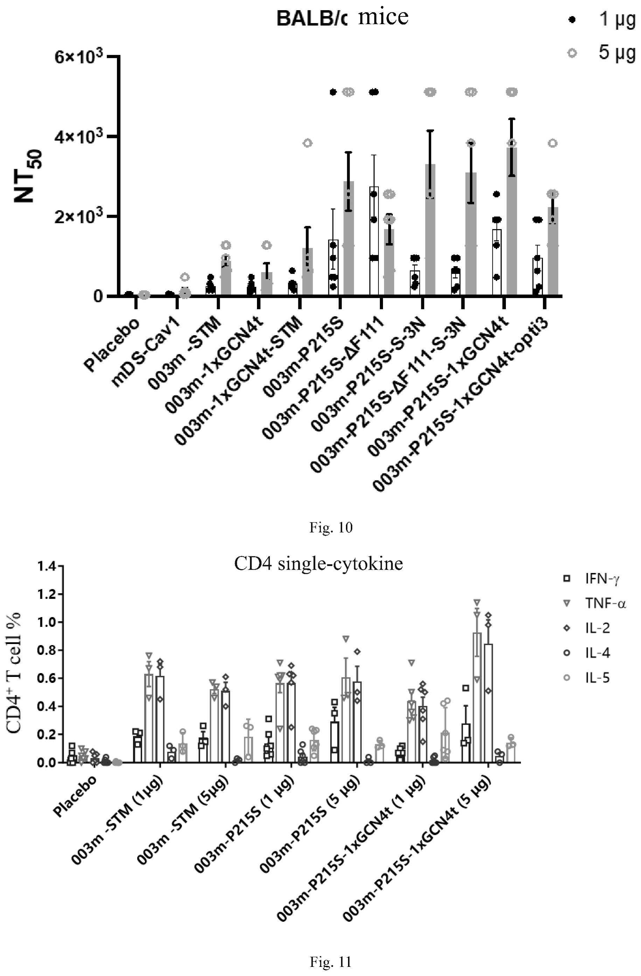

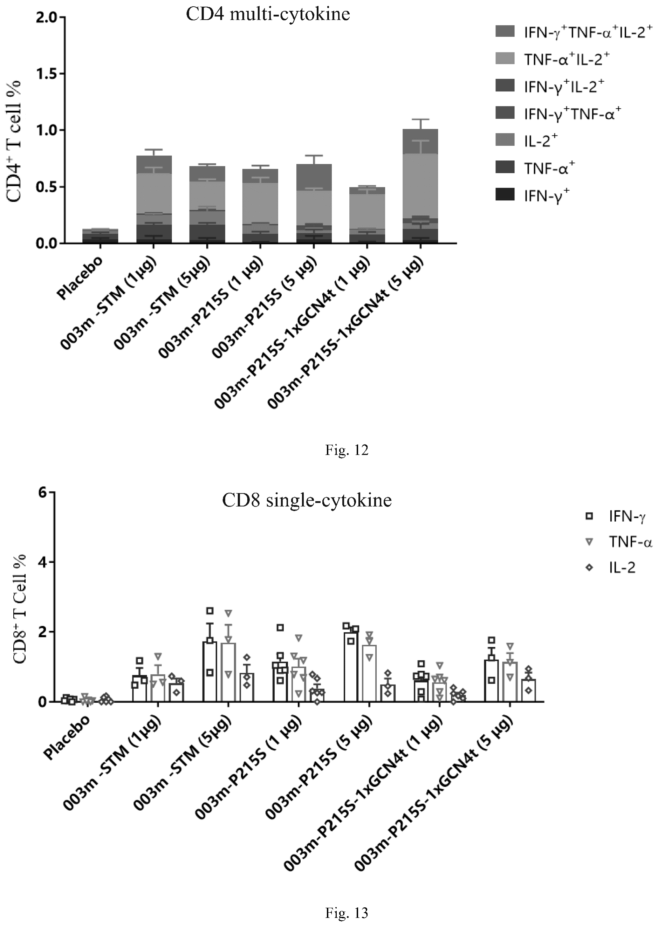

### FIGS. 11 and 12 (CD4⁺ T Cell Responses)

Analysis of CD4⁺ T cell single-cytokine and polyfunctional responses, together with total antibody levels (FIG. 10), indicated that the high-dose group of 003m-P215S-1×GCN4t induced the strongest cellular immune response. This trend was consistent with the observed ratio of prefusion-specific to postfusion-specific antibodies in the total antibody analysis.

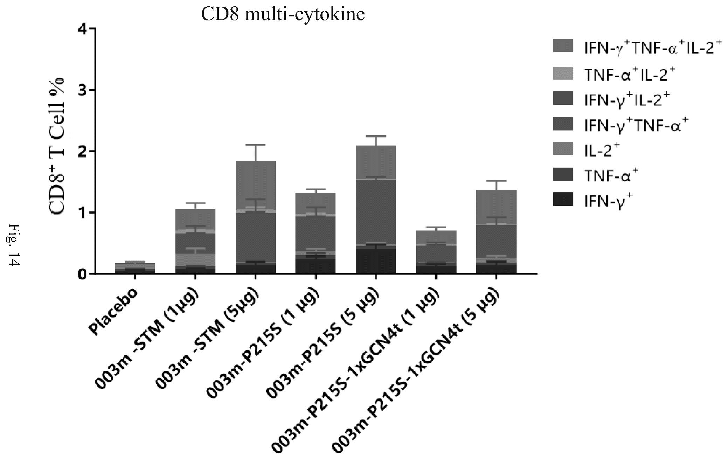

### FIGS. 13 and 14 (CD8⁺ T Cell Responses)

Analysis of CD8⁺ T cell single-cytokine and polyfunctional responses, in conjunction with total antibody levels (FIG. 10), showed that 003m-P215S induced the highest cellular immune response overall. However, no significant differences were observed among the different dose groups.

### IV. Cellular Immune Responses in BALB/c Mice (ELISpot)

### FIG. 15

ELISpot analysis demonstrated that the high-dose group of 003m-P215S-1×GCN4t exhibited slightly superior responses compared to 003m-P215S. This finding was generally consistent with the CD4⁺ T cell response results.

If desired, I can draft a concise integrated efficacy conclusion paragraph summarizing why 003m-P215S-1×GCN4t represents the optimal candidate based on humoral, neutralizing, and cellular immunity data-useful for strengthening inventive-step positioning.

### Example 14

### RSV Challenge Study in BALB/c Mice

### I. Body Weight Changes

### FIG. 16

The results demonstrated that the 5 µg vaccine groups effectively mitigated body weight loss induced by RSV A2 viral infection. Among these, the 003m-P215S-1×GCN4t group showed the most favorable outcome.

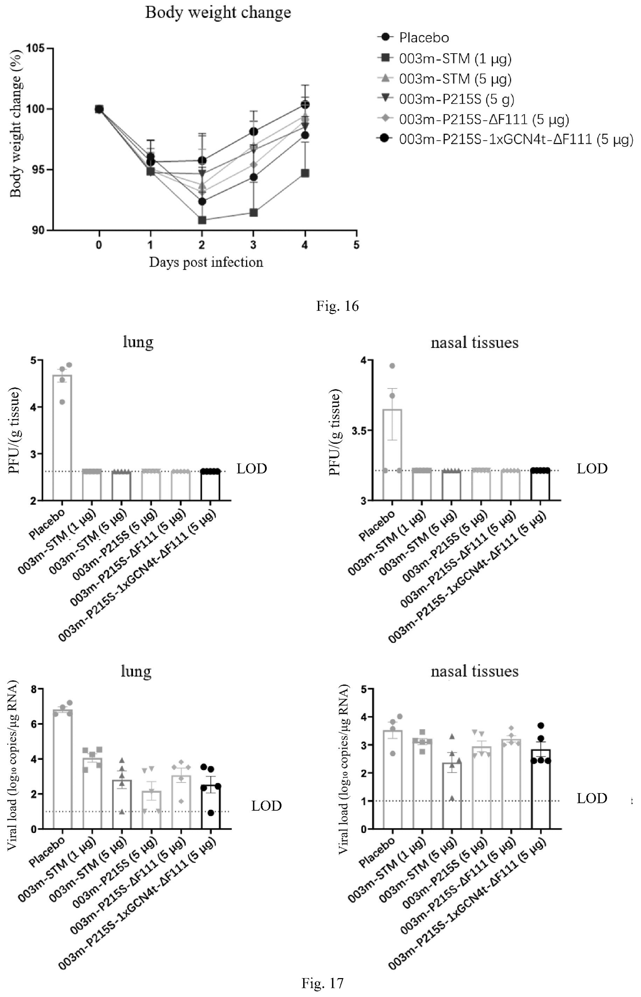

In contrast, the 003m-STM group at 1 µg exhibited more severe weight loss compared to the placebo group (FIG. 16), suggesting a potential risk of vaccine-enhanced disease (VED).

### II. Viral Titers and Viral Copy Numbers

### FIG. 17

The results showed that all vaccine groups significantly reduced viral titers and viral copy numbers in both lung and nasal tissues.

In vaccinated animals, viral titers in lung and nasal tissues decreased by more than two orders of magnitude compared to the control group, indicating that the candidate antigens provided robust protective efficacy.

Limits of Detection:
- Viral titer detection limit:
   o Lung tissue: 417 PFU/g tissue
   o Nasal tissue: 1,630 PFU/g tissue
- Viral copy number detection limit:
   o 1 Log10 copies/µg RNA

### Example 15

### Immunogenicity of Candidate Antigens Using Different Delivery Systems

### I. Animal Vaccination and Serum Collection

### BALB/c Mice

For mouse immunization studies, vaccine formulations comprising the target antigens were prepared according to the mRNA-LNP preparation methods described in Examples 12 and 17. The resulting formulations were administered by intramuscular injection to female BALB/c mice aged 5 to 7 weeks (purchased from SPF (Suzhou) Biotechnology Co., Ltd.).

A prime immunization was performed on Day 0, followed by a booster immunization on Day 21 to enhance the immune response.

Each experimental group consisted of 8 BALB/c mice (n = 8).

The immunization regimens are summarized in Table 29.

### Preparation of LQ104-54

### Step 1: Preparation of Compound 54-1

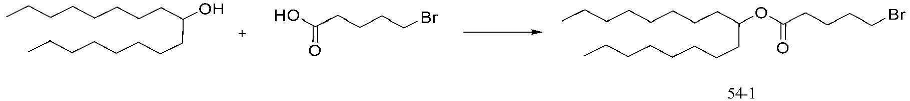

5-Bromovaleric acid (10 g, 55 mmol, 1.1 equiv), dicyclohexylcarbodiimide (DCC) (20.6 g, 100 mmol, 2.0 equiv), 4-dimethylaminopyridine (DMAP) (610 mg, 5 mmol, 0.1 equiv), and dichloromethane (DCM) (300 mL) were added to a reaction flask. 9-Heptadecanol (12.8 g, 50 mmol, 1.0 equiv) was then added to the reaction mixture.

The reaction mixture was stirred at room temperature for 12 hours. After completion of the reaction, the mixture was filtered to remove insoluble by-products, and the filtrate was concentrated under reduced pressure. The residue was purified by column chromatography to afford 14 g of a colorless oil.

Thin-layer chromatography (TLC) analysis (petroleum ether:ethyl acetate = 20:1) showed the product with an Rf value of 0.6.

### Step 2: Preparation of Compound 16-3

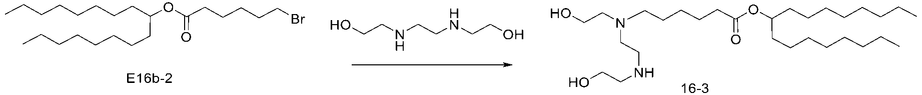

E16b-2 (5 g, 11.5 mmol, 0.8 equiv; as described in CN117534584A), N,N'-bis(2-hydroxyethyl)ethylenediamine (2.2 g, 14.4 mmol, 1.0 equiv), potassium carbonate (K₂CO₃) (3 g, 21.6 mmol,

1.5 equiv), and potassium iodide (KI) (2.4 g, 14.4 mmol, 1.0 equiv) were added to a reaction flask containing acetonitrile (80 mL).

The reaction mixture was heated to 70°C and stirred for 16 hours. Upon completion, the reaction mixture was filtered to remove inorganic salts, and the filtrate was concentrated under reduced pressure. The residue was purified by column chromatography to afford 4 g of a colorless oil.

Thin-layer chromatography (TLC) analysis (dichloromethane:methanol = 10:1) showed the product with an Rf value of 0.5.

### Step 3: Preparation of LQ104-54

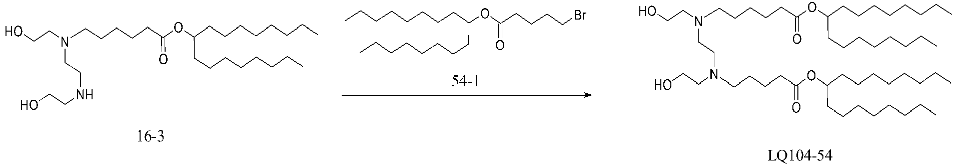

Compound 16-3 (1.6 g, 3.2 mmol, 1.1 equiv), compound 54-1 (1.63 g, 3.9 mmol, 1.2 equiv), potassium carbonate (K₂CO₃) (1.0 g, 6.4 mmol, 2.0 equiv), and potassium iodide (KI) (0.6 g, 3.2 mmol, 1.0 equiv) were added to a reaction flask containing acetonitrile (40 mL).

The reaction mixture was heated to 70°C and stirred for 16 hours. After completion of the reaction, the mixture was filtered to remove inorganic salts, and the filtrate was concentrated under reduced pressure. The residue was purified by column chromatography to afford 700 mg of a colorless oil.

Thin-layer chromatography (TLC) analysis (dichloromethane:methanol = 10:1) showed the product with an Rf value of 0.4.

### Characterization Data

¹H NMR (600 MHz, Chloroform-d) δ 4.86 (p, *J* = 6.3 Hz, 2H), 3.63 (q, *J* = 5.0 Hz, 4H), 2.70 - 2.55 (m, 12H), 2.30 (dt, *J=* 15.2, 7.3 Hz, 4H), 1.62 (dt, *J=* 18.1, 7.7 Hz, 4H), 1.55 - 1.47 (m, 12H), 1.30 - 1.22 (m, 50H), 0.87 (t, *J* = 6.9 Hz, 12H).

MS (ES+) *m*/*z*): 839 (M),840 (M+H).

### Preparation of LQ104-56

### Step 1: Preparation of Compound 56-1

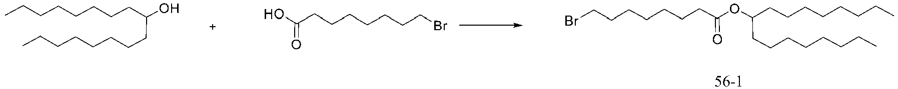

8-Bromooctanoic acid (12.2 g, 55 mmol, 1.1 equiv), dicyclohexylcarbodiimide (DCC) (20.6 g, 100 mmol, 2.0 equiv), 4-dimethylaminopyridine (DMAP) (610 mg, 5 mmol, 0.1 equiv), and dichloromethane (DCM) (300 mL) were added to a reaction flask. 9-Heptadecanol (12.8 g, 50 mmol, 1.0 equiv) was then added to the reaction mixture.

The reaction mixture was stirred at room temperature for 12 hours. Upon completion of the reaction, the mixture was filtered to remove insoluble by-products, and the filtrate was concentrated under reduced pressure. The residue was purified by column chromatography to afford 15.5 g of a colorless oil.

Thin-layer chromatography (TLC) analysis (petroleum ether:ethyl acetate = 20:1) showed the product with an Rf value of 0.6.

### Step 2: Preparation of LQ104-56

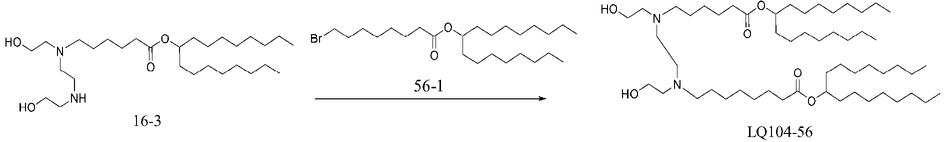

Compound 16-3 (1.6 g, 3.2 mmol, 1.1 equiv), compound 20-1 (1.8 g, 3.9 mmol, 1.2 equiv), potassium carbonate (K₂CO₃) (1.0 g, 6.4 mmol, 2.0 equiv), and potassium iodide (KI) (0.6 g, 3.2 mmol, 1.0 equiv) were added to a reaction flask containing acetonitrile (40 mL).

The reaction mixture was heated to 70°C and stirred for 16 hours. Upon completion of the reaction, the mixture was filtered to remove inorganic salts, and the filtrate was concentrated under reduced pressure. The residue was purified by column chromatography to afford 1.2 g of a colorless oil.

Thin-layer chromatography (TLC) analysis (dichloromethane:methanol = 10:1) showed the product with an Rf value of 0.4.

### Characterization Data

¹H NMR (400 MHz, Chloroform-d) δ 4.85 (p, *J =* 6.2 Hz, 2H), 3.64 - 3.57 (m, 4H), 2.67 - 2.57 (m, 8H), 2.52 (t, *J* = 7.9 Hz, 4H), 2.27 (td, *J* = 7.5, 4.7 Hz, 4H), 1.68 - 1.57 (m, 4H), 1.49 (t, *J* = 6.2 Hz, 12H), 1.33 - 1.22 (m, 56H), 0.90 - 0.84 (m, 12H).

MS (ES+) *m*/*z* ): 882 (M),883 (M+H).

**Table 29. Immunization Schedule for BALB/c Mice**

| Antigen Name | Lipid Formulation | Immunization Dose (µg) | Number of Animals (Female) | Route of Administration |
|---|---|---|---|---|
| Placebo | Tris-AC | NA | 8 | Intramuscular injection |
| 003m-P215S-1×GCN4t | E16b2 | 1 µg | 8 | Intramuscular injection |
| | | 5 µg | 8 | Intramuscular injection |
| | LQ104-56 | 1 µg | 8 | Intramuscular injection |
| | | 5 µg | 8 | Intramuscular injection |
| | LQ104-54 | 1 µg | 8 | Intramuscular injection |
| | | 5 µg | 8 | Intramuscular injection |
| | RL151 | 1 µg | 8 | Intramuscular injection |
| | | 5 µg | 8 | Intramuscular injection |
| Post-F | E16b2 | 1 µg | 8 | Intramuscular injection |
| | | 5 µg | 8 | Intramuscular injection |
| | LQ104-56 | 1 µg | 8 | Intramuscular injection |
| | | 5 µg | 8 | Intramuscular injection |
| | LQ104-54 | 1 µg | 8 | Intramuscular injection |
| | | 5 µg | 8 | Intramuscular injection |
| | RL151 | 1 µg | 8 | Intramuscular injection |
| | | 5 µg | 8 | Intramuscular injection |
| 003m-P215S | E16b2 | 1 µg | 8 | Intramuscular injection |
| | | 5 µg | 8 | Intramuscular injection |
| | LQ104-56 | 1 µg | 8 | Intramuscular injection |
| | | 5 µg | 8 | Intramuscular injection |
| | LQ104-54 | 1 µg | 8 | Intramuscular injection |
| | | 5 µg | 8 | Intramuscular injection |
| | RL151 | 1 µg | 8 | Intramuscular injection |
| | | 5 µg | 8 | Intramuscular injection |

A total of two immunizations were administered at an interval of three weeks. The dose for each administration is as set forth in Table 29.

An appropriately sized sterile insulin syringe was used to accurately withdraw 400 µL of the test article. Prior to withdrawal, the formulation was gently inverted 5-10 times to ensure homogeneity. Intramuscular injection was performed in the right hind limb of each animal, with a single injection volume of 100 µL per injection site.

Serum samples were collected from the immunized mice and heat-inactivated at 56°C for 30 minutes prior to analysis of RSV F protein-specific IgG antibodies and neutralizing antibody titers.

### II. Antibody Level Analysis

### 1. Total Antibody Levels

The total antibody assay was performed according to the method described in Example 1, Section V ("Total Antibody Level Detection"), specifically following the antigen-coating method described in Section 1.(2). The neutralizing antibody assay was conducted in accordance with Example 1, Section VI ("Neutralizing Antibody Detection").

### FIG. 18

The results demonstrated the following:
1. Compared with the 1 µg group, the 5 µg group induced higher total antibody levels, showing an overall dose-dependent trend.
2. Following immunization with 003m-P215S-1×GCN4t using different lipid formulations, low levels of antibodies directed against the postfusion conformation were observed. Moreover, the level of postfusion-specific antibodies induced by 003m-P215S-1×GCN4t was significantly lower than that induced by 003m-P215S.
3. When delivered using the same lipid formulation, 003m-P215S induced higher total antibody titers than 003m-P215S-1×GCN4t.
4. The majority of antibodies induced by Post-F were directed against the postfusion conformation.
5. Compared with the other three lipid formulations, RL151 induced higher levels of prefusion-specific antibodies in both the 003m-P215S and 003m-P215S-1×GCN4t groups.

Overall, both 003m-P215S and 003m-P215S-1×GCN4t induced substantial levels of antibodies directed against the prefusion conformation. Notably, 003m-P215S-1×GCN4t exhibited a more favorable ratio of prefusion-specific to postfusion-specific antibodies compared to 003m-P215S.

### 2. Neutralizing Antibody Titers

### FIG. 19

The neutralizing antibody results were generally consistent with the total antibody results:
1. Post-F induced relatively low neutralizing antibody titers.
2. The 5 µg group generated higher neutralizing antibody titers than the 1 µg group, indicating dose dependency.
3. Compared with 003m-P215S, 003m-P215S-1×GCN4t induced higher neutralizing antibody titers in BALB/c mice.
4. Both RL151 and iPLX delivery systems induced relatively high neutralizing antibody titers.

### III. Splenocyte Immune Responses in BALB/c Mice

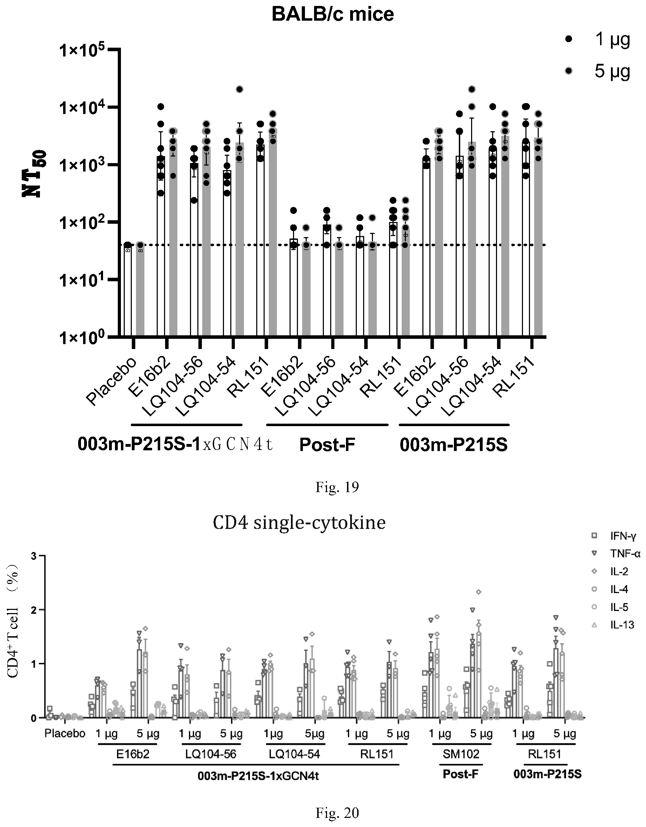

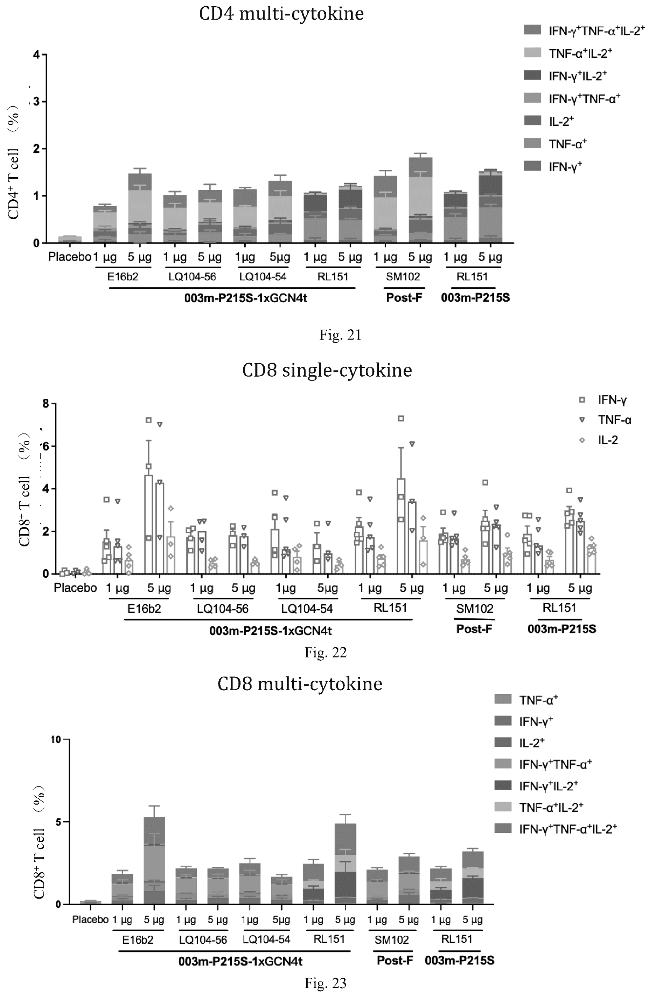

### CD4⁺ T Cell Responses (FIGS. 20 and 21)

1. 003m-P215S, 003m-P215S-1×GCN4t, and Post-F all induced robust cellular immune responses, with the 5 µg groups showing slightly higher responses than the 1 µg groups.
2. Compared with E16b2, RL151, LQ104-56, and LQ104-54 induced lower levels of Th2-associated cytokines, suggesting a potential risk of vaccine-enhanced disease (VED) when E16b2 is used as the delivery system.
3. Even when delivered using RL151, Post-F still exhibited a potential risk of VED.

### CD8⁺ T Cell Responses (FIGS. 22 and 23)

1. Polyfunctional cellular immune responses showed a linear correlation with dose, with the 5 µg groups slightly higher than the 1 µg groups.
2. RL151-mediated delivery induced significantly higher cellular immune responses compared to LQ104-56 and LQ104-54.
3. 003m-P215S-1×GCN4t induced higher cellular immune responses than 003m-P215S, indicating that 003m-P215S-1×GCN4t is superior to 003m-P215S in BALB/c mice.

### IV. Splenocyte Immune Responses in BALB/c Mice (ELISpot)

### FIG. 24

The ELISpot results were consistent with the intracellular cytokine staining results:
1. RL151-mediated delivery induced significantly higher cellular immune responses than LQ104-56 and LQ104-54, although slightly lower than E16b2.
2. Compared with 003m-P215S, 003m-P215S-1×GCN4t induced higher cellular immune responses.

### Example 16: Cotton Rat Challenge Study

### I. Animal Immunization and Serum Collection

### Cotton rats:

For cotton rat immunization, vaccine formulations comprising the target antigen prepared according to the mRNA-LNP (RL151) preparation method described in Example 17 were administered intramuscularly to 6-8-week-old female cotton rats (purchased from SPF (Suzhou) Biotechnology Co., Ltd.) (hereinafter referred to as "immunization"). A booster dose was administered on Day 21 following the initial immunization to enhance the immune response.

In the placebo group, Tris-Ac (pH 7.5) was used as the control formulation. The specific composition of Tris-Ac was:
- 20 mM Tris
- 1.42 mM glacial acetic acid
- 2.89 mM sodium acetate

Each group included 3 or 4 cotton rats (n = 3/4).

The immunization schedule is shown in Table 30.

**Table 30. Immunization Schedule for Cotton Rats**

| Antigen Name | Lipid Formulation | Immunization Dose (µg) | Number of Animals (Female) | Route of Administration |
|---|---|---|---|---|
| Placebo | Tris-AC | NA | 4 | Intramuscular injection |
| 003m-P215S-1xGCN4t | RL151 | 5 µg | 3 | Intramuscular injection |
| | | 25 µg | 3 | Intramuscular injection |
| FI-RSV | | | 3 | Intramuscular injection |

### II. Body Weight Changes

### FIG. 25

Compared with the placebo group, all vaccinated groups alleviated body weight loss caused by RSV A2 virus (GenBank: KT992094) infection. Among them, the 003m-P215S-1×GCN4t (25 µg) group demonstrated superior protective performance.

### III. Neutralizing Antibodies

### FIG. 26

Compared with the inactivated vaccine group (FI-RSV), 003m-P215S-1×GCN4t induced relatively high neutralizing antibody titers at a dose of 5 µg. The induced antibodies were capable of neutralizing both RSV A2 and RSV B strains. However, the 25 µg group did not induce higher neutralizing antibody titers than the lower-dose group.

### IV. Viral Titers

### FIG. 27

1. Compared with the placebo group, the inactivated virus group (FI-RSV) significantly reduced viral titers in lung tissue; however, virus remained detectable in nasal tissues.
2. 003m-P215S-1×GCN4t significantly reduced viral titers in both lung and nasal tissues, with reductions exceeding two orders of magnitude. These results indicate that the antigen provides robust protective efficacy.

### V. Lung Cytokine Analysis

### FIG. 28

Cytokine analysis showed that the inactivated virus group (FI-RSV) exhibited significantly elevated expression of Th2-associated cytokines (IL-4, IL-2, IL-3) compared with both the 003m-P215S-1×GCN4t group and the placebo group. In contrast, cytokine levels in the 003m-P215S-1×GCN4t group were comparable to those in the placebo group. These findings suggest that the inactivated virus group (FI-RSV) presents a higher risk of vaccine-enhanced disease (VED).

### VI. Lung Histopathology

### FIG. 29

RSV A2 infection resulted in pathological changes including inflammation in the alveolar spaces, peribronchiolar regions, perivascular regions, and alveolar interstitium. Immunization with 003m-P215S-1×GCN4t markedly reduced pulmonary inflammation in a dose-dependent manner.

In contrast, the inactivated virus group (FI-RSV) exhibited more severe pulmonary inflammation, indicating that FI-RSV induced pronounced vaccine-enhanced disease (VED).

### Example 17: Screening of Extended Sequences with Different Glycosylation Modifications

According to published literature, in addition to GCN4t, which promotes trimer formation, T4 foldon (Fibritin) can also facilitate trimerization. Accordingly, an extended construct incorporating Fibritin was designed.

The 1×Fibritin extension was generated by inserting the amino acid sequence SAIGGYIPEAPRDGQAYVRKDGEWVLLSTFLGGLVPR (SEQ ID NO: 140) between residues L513 and H514 of the amino acid sequence shown in SEQ ID NO: 1.

Because both GCN4t and T4 foldon possess a certain degree of intrinsic immunogenicity, glycosylation modifications were introduced to partially mask their immunogenic epitopes and achieve immune silencing.

For 1×GCN4t, three glycosylation sites were introduced and designated sequentially as site 1 (1N), site 2 (2N), and site 3 (3N). The designation "1,2N" indicates glycosylation modifications at both site 1 and site 2. Similarly, "1,3N" indicates glycosylation at sites 1 and 3, and so forth.

For 1×Fibritin, glycosylation modification was also performed. The modified 1×Fibritin-1N construct was generated by inserting the sequence SAIGGYIPEAPNDTQAYVRKDGEWVLLSTFLGGLVPR (SEQ ID NO: 159) between residues L513 and H514 of SEQ ID NO: 1.

### I. In Vitro Flow Cytometry Analysis

### FIG. 30

In vitro flow cytometry results demonstrated that both the 1×Fibritin extension and glycosylation-modified variants of 1×Fibritin and 1×GCN4t significantly increased membrane surface expression of RSV F protein in the monomeric or trimeric prefusion conformation (see Table 31).

Compared with other glycosylation combinations of 1×GCN4t, dual glycosylation modification (003m-P215S-1×GCN4t-1,3N) and triple glycosylation modification (003m-P215S-1×GCN4t-1,2,3N) produced particularly pronounced increases in membrane surface protein expression.

Accordingly, the following constructs were selected for subsequent animal immunization studies:
- 003m-P215S-1×Fibritin
- 003m-P215S-1×Fibritin-1N
- 003m-P215S-1×GCN4t-1,3N
- 003m-P215S-1×GCN4t-1,2,3N
- 003m-P215S-1×GCN4t
- 003m-P215S

**Table 31. Mean Fluorescence Intensity (MFI) of In Vitro Flow Cytometry for Extended Sequences with Different Glycosylation Modifications**

| Lipofectamine 2000-mRNA | | |
|---|---|---|
| Group | MFI | |
| | AM14-AF488 | D25-AF488 |
| 003m-P215S-1×GCN4t | 4866 | 10370 |
| 003m-P215S-1×Fibritin | 22339 | 22988 |
| 003m-P215S-1×Fibritin-1N | 241784 | 235078 |
| 003m-P215S-1×GCN4t-1N | 37370 | 59392 |
| 003m-P215S-1×GCN4t-2N | 28844 | 54007 |
| 003m-P215S-1×GCN4t-3N | 25142 | 43404 |
| 003m-P215S-1×GCN4t-1,2N | 58380 | 100099 |
| 003m-P215S-1×GCN4t-1,3 N | 71022 | 122310 |
| 003m-P215S-1×GCN4t-2,3 N | 39113 | 70747 |
| 003m-P215S-1×GCN4t-1,2,3 N | 89637 | 147330 |

### II. Animal Immunization and Serum Collection

### BALB/c mice:

For mouse immunization, the lipid component LQ104-E16b-2 was prepared in accordance with the method described in CN117534584A.

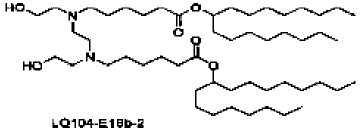

Vaccine formulations comprising the target antigen were prepared according to Example 8 and administered intramuscularly to 5-7-week-old female BALB/c mice (purchased from SPF (Suzhou) Biotechnology Co., Ltd.) (hereinafter referred to as "immunization"). A booster dose was administered on Day 21 following the initial immunization to enhance the immune response.

Each experimental group included 6 or 12 BALB/c mice (n = 6/12).

The immunization schedule is shown in Table 32.

**Table 32. Immunization Schedule for BALB/c Mice**

| Antigen Name | Lipid Formulation | Immunization Dose (µg) | Number of Animals (Female) | Route of Administration |
|---|---|---|---|---|
| Placebo | NA | 12 | 0.1 | Intramuscular injection |
| 003m-P215S-1×Fibritin | 1 µg | 6 | 0.1 | Intramuscular injection |
| | 5 µg | 6 | 0.1 | Intramuscular injection |
| 003m-P215S-1×Fibritin-1N | 1 µg | 6 | 0.1 | Intramuscular injection |
| | 5 µg | 6 | 0.1 | Intramuscular injection |
| 003m-P215S-1×GCN4t-1,3 N | 1 µg | 6 | 0.1 | Intramuscular injection |
| | 5 µg | 6 | 0.1 | Intramuscular injection |
| 003m-P215S-1×GCN4t-1,2,3 N | 1 µg | 6 | 0.1 | Intramuscular injection |
| | 5 µg | 6 | 0.1 | Intramuscular injection |
| 003m-P215S-1×GCN4t | 1 µg | 6 | 0.1 | Intramuscular injection |
| | 5 µg | 6 | 0.1 | Intramuscular injection |
| 003m-P215S | 1 µg | 6 | 0.1 | Intramuscular injection |
| | 5 µg | 6 | 0.1 | Intramuscular injection |

Two doses were administered at an interval of three weeks, with the dose for each administration as shown in Table 32. An appropriate sterile insulin syringe was used to accurately withdraw 400 µL of the test article (the test article was gently inverted 5-10 times prior to withdrawal to ensure homogeneity). Intramuscular injection was performed in the right hind limb of each animal, with 100 µL administered per injection site.

Serum samples were collected from the immunized mice, heat-inactivated at 56°C for 30 minutes, and stored at -80°C for subsequent analysis.

### III. Neutralizing Antibody Analysis

Neutralizing antibody titers were determined using the fluorescence-based assay described in Example 1, Section VI ("Neutralizing Antibody Detection").

### FIG. 31

The neutralizing antibody results demonstrated the following:
1. For both 1×Fibritin and 1×GCN4t extension formats, glycosylation modification reduced neutralizing antibody titers.
2. Compared with the 1×GCN4t extension, the 1×Fibritin extension induced higher neutralizing antibody titers.
3. Within the 1×GCN4t extension format, increasing the degree of glycosylation modification resulted in a greater reduction in neutralizing antibody titers.

### Example 18: Effect of 1×GCN4t Insertion Site and Linker Length on Antigen Immunogenicity

To investigate whether the insertion position of 1×GCN4t (SEQ ID NO: 6) exhibits positional flexibility, multiple extended constructs were designed with 1×GCN4t inserted at different positions. Specifically, based on the insertion site used in 003m-P215S-1×GCN4t, additional constructs were generated by inserting 1×GCN4t at intervals of three amino acids along the sequence. The results are summarized in Table 18.

In addition, to evaluate the impact of linker length on RSV F mutants, constructs containing linkers of varying lengths were designed. The corresponding results are provided in Table 19.

### I. In Vitro Flow Cytometry Analysis

### FIGS. 32 and 33

In vitro flow cytometry analysis demonstrated that different insertion sites of 1×GCN4t and varying linker lengths did not significantly affect membrane surface expression of RSV F protein in the monomeric prefusion conformation (see Tables 33 and 34).

**Table 33. Mean Fluorescence Intensity (MFI) of In Vitro Flow Cytometry for RSV F Mutants with Different 1×GCN4t Insertion Sites**

| Lipofectamine 2000-mRNA | | |
|---|---|---|
| Group | MFI | |
| | AM14-AF488 | D25-AF488 |
| 003m-P215S-DELL-1×GCN4t | 31396 | 35666 |
| 003m-P215S-1×GCN4t | 21630 | 32789 |
| 003m-P215S-NAG-1×GCN4t | 45748 | 33859 |
| 003m-P215S-KST-1×GCN4t | 33830 | 31561 |

**Table 34. Mean Fluorescence Intensity (MFI) of In Vitro Flow Cytometry for RSV F Mutants with Different Linker Lengths**

| Lipofectamine 2000-mRNA | | |
|---|---|---|
| Group | MFI | |
| | AM14-AF488 | D25-AF488 |
| 003m-P215S-1×GCN4t | 6705 | 12208 |
| 007m-Cys-P215S-1×GCN4t | 7676 | 12818 |
| 008m-Cys-P215S-1×GCN4t | 9785 | 15737 |
| 009m-Cys-P215S-1×GCN4t | 6454 | 11496 |
| 010m-Cys-P215S-1×GCN4t | 8647 | 13363 |
| 011m-Cys-P215S-1×GCN4t | 7712 | 10292 |
| 012m-Cys-P215S-1×GCN4t | 9388 | 15764 |
| 013m-Cys-P215S-1×GCN4t | 7926 | 14575 |

### II. Animal Immunization and Serum Collection

### BALB/c mice:

For mouse immunization, vaccine formulations comprising the target antigens listed in Table 20 were prepared in accordance with the procedures described in Example 17, Section II ("Animal Immunization and Serum Collection"). The resulting vaccine formulations were administered intramuscularly to 5-7-week-old female BALB/c mice (purchased from SPF (Suzhou) Biotechnology Co., Ltd.) (hereinafter referred to as "immunization"). A booster dose was administered on Day 21 following the initial immunization to enhance the immune response.

Each experimental group consisted of six BALB/c mice (n = 6).

The immunization schedule is shown in Table 35.

**Table 35. Immunization Schedule for Evaluation of 1×GCN4t Insertion Sites and Linker Length Variants**

| Antigen Name | Immunization Dose (µg) | Number of Animals (Female) | Administration Volume (mL) | Route of Administration |
|---|---|---|---|---|
| Placebo | NA | 6 | 0.1 | Intramuscular injection |
| 003m-P215S-DELL-1×GCN4t | 5 µg | 6 | 0.1 | Intramuscular injection |
| 003m-P215S-1×GCN4t | 5 µg | 6 | 0.1 | Intramuscular injection |
| 003m-P215S-NAG-1×GCN4t | 5 µg | 6 | 0.1 | Intramuscular injection |
| 003m-P215S-KST-1×GCN4t | 5 µg | 6 | 0.1 | Intramuscular injection |
| 003m-P215S-1×GCN4t | 5 µg | 6 | 0.1 | Intramuscular injection |
| 007m-Cys-P215S-1×GCN4t | 5 µg | 6 | 0.1 | Intramuscular injection |
| 008m-Cys-P215S-1×GCN4t | 5 µg | 6 | 0.1 | Intramuscular injection |
| 009m-Cys-P215S-1×GCN4t | 5 µg | 6 | 0.1 | Intramuscular injection |
| 010m-Cys-P215S-1×GCN4t | 5 µg | 6 | 0.1 | Intramuscular injection |
| 011m-Cys-P215S-1×GCN4t | 5 µg | 6 | 0.1 | Intramuscular injection |
| 012m-Cys-P215S-1×GCN4t | 5 µg | 6 | 0.1 | Intramuscular injection |
| 013m-Cys-P215S-1×GCN4t | 5 µg | 6 | 0.1 | Intramuscular injection |

Two doses were administered at an interval of three weeks, with the dose for each administration as shown in Table 35. An appropriate sterile insulin syringe was used to accurately withdraw 400 µL of the test article (the test article was gently inverted 5-10 times prior to withdrawal to ensure homogeneity). Intramuscular injection was performed in the right hind limb of each animal, with 100 µL administered at a single injection site.

Serum samples were collected from the immunized mice, heat-inactivated at 56°C for 30 minutes, and stored at -80°C for subsequent analysis.

### III. Neutralizing Antibody Analysis

Neutralizing antibody titers were determined using the fluorescence-based method described in Example 1, Section VI ("Neutralizing Antibody Detection").

### FIG. 34

The neutralizing antibody results demonstrated the following:
1. The insertion position of 1×GCN4t had a measurable impact on neutralizing antibody titers. As the insertion site moved further toward the C-terminus, neutralizing antibody titers tended to decrease; however, all variants induced higher titers than 003m-1×GCN4t and DS-Cav1.
2. Variations in linker length among the RSV F mutants also influenced neutralizing antibody titers to some extent, but no significant differences were observed compared with 003m-P215S-1×GCN4t.

Overall, different insertion sites and linker lengths of 1×GCN4t were all capable of inducing relatively high neutralizing antibody titers, indicating that the selection of insertion position and linker length for 1×GCN4t exhibits positional flexibility and general applicability.

### Example 19: Engineering of Candidate RSV B Antigens

Three antibodies were used for flow cytometry analysis:
- 4D7, labeled with Alexa Fluor 647 (AF647), for detection of the postfusion conformation;
- AM14, labeled with Alexa Fluor 488 (AF488), for detection of the trimeric prefusion conformation;
- D25, labeled with Alexa Fluor 488 (AF488), for detection of the prefusion conformation.

The amino acid sequence of wild-type RSV B (SEQ ID NO: 80) is provided below:

MELLIHRSSAIFLTLAINALYLTSSQNITEEFYQSTCSAVSRGYLSALRTGWYTSVITIELSNIKE
TKCNGTDTKVKLIKQELDKYKNAVTELQLLMQNTPAVNNRARREAPQYMNYTINTTKNLNVSIS
KKRKRRFLGFLLGVGSAIASGIAVSKVLHLEGEVNKIKNALQLTNKAVVSLSNGVSVLTSKVLDL

KNYINNQLLPIVNQQSCRISNIETVIEFQQKNSRLLEITREFSVNAGVTTPLSTYMLTNSELLSLIND
MPITNDQKKLMSSNVQIVRQQSYSIMSIIKEEVLAYVVQLPIYGVIDTPCWKLHTSPLCTTNIKEGS
NICLTRTDRGWYCDNAGSVSFFPQADTCKVQSNRVFCDTMNSLTLPSEVSLCNTDIFNSKYDCKI
MTSKTDISSSVITSLGAIVSCYGKTKCTASNKNRGIIKTFSNGCDYVSNKGVDTVSVGNTLYYVNK
LEGKNLYVKGEPIINYYDPLVFPSDEFDASISQVNEKINQSLAFIRRSDELLHNVNTGKSTTNIMITA
IIIVIIVVLLSLIAIGLLLYCKAKNTPVTLSKDQLSGINNIAFSK

### Modifications Based on RSV B

Modifications were introduced based on RSV B.

Compared with the wild-type RSV B amino acid sequence (SEQ ID NO: 80), the mutation sites and linker sequences involved in 003m are as described in Table 6 of Example 1.

The 1×GCN4t extension was generated by inserting the sequence EDKIEEILSKIYHIENEIARIKKLIGEA (SEQ ID NO: 6) between residues V516 and N517.

In addition, the following revertant mutations, linker replacements, and dGCN4t modifications were introduced:
- RSV B-003m-P215S-1×GCN4t-G46S
- RSV B-003m-P215S-1×GCN4t-D92E
- RSV B-003m-P215S-1×GCN4t-F190S
- RSV B-003m-P215S-1×GCN4t-C155S-C290S
- RSV B-003m-P215S-1×GCN4t-C486D-C489D
- RSV B-003m-P215S-1×GCN4t-C102A-C362S
- RSV B-003m-P215S-1×GCN4t-ori_linker
- RSV B-003m-P215S

For each mutant construct, three different codon-optimized nucleotide sequences (RQ1-RQ3) were designed. The corresponding mutant amino acid sequences are shown in SEQ ID NOs: 82-89.

These modifications may be combined. For example, RSV B-003m was first subjected to the P215S revertant mutation, and subsequently further modified by insertion of 1×GCN4t. The amino acid sequence of RSV B-003m-P215S-1×GCN4t is shown in SEQ ID NO: 81.

### In Vitro Flow Cytometry Analysis

### FIG. 35

In vitro flow cytometry results demonstrated that:
1. Introduction of single-point mutations or disulfide bond pair mutations resulted in the presence of partial postfusion F protein on the cell surface.
2. In the absence of a linker, membrane surface protein expression was severely reduced.
3. Removal of 1×GCN4t increased membrane surface protein expression; however, this also led to an increase in postfusion-conformation F protein.
4. Among the three codon optimization strategies, RSV B-003m-P215S-1×GCN4t-RQ1 exhibited improved expression compared with the other two optimized sequences.

Overall, RSV B-003m-P215S-1×GCN4t was able to maintain both monomeric and trimeric prefusion conformations in the RSV B subtype. Among the three codon-optimized variants, RQ1 was optimal (see Table 36).

**Table 36. Mean Fluorescence Intensity (MFI) of In Vitro Flow Cytometry for RSV B-Type F Mutants**

| Lipofectamine 2000-mRNA | | |
|---|---|---|
| Group (RSV B) | MFI | |
| | AM14-AF488 | D25-AF488 |
| WT-RQ1 | 201129 | 132275 |
| WT-RQ2 | 206181 | 138271 |
| WT-RQ3 | 55835 | 36939 |
| 003m-P215S-1×GCN4t-RQ1 | 7337 | 8773 |
| 003m-P215S-1×GCN4t-RQ2 | 5296 | 7114 |
| 003m-P215S-1×GCN4t-RQ3 | 5056 | 6192 |
| 003m-P215S-1×GCN4t-G46S-RQ1 | 4135 | 4571 |
| 003m-P215S-1×GCN4t-G46S-RQ2 | 3667 | 4106 |
| 003m-P215S-1×GCN4t-G46S-RQ3 | 4098 | 4219 |
| 003m-P215S-1×GCN4t-D92E-RQ1 | 763 | 2250 |
| 003m-P215S-1×GCN4t-D92E-RQ2 | 792 | 2483 |
| 003m-P215S-1×GCN4t-D92E-RQ3 | 814 | 2109 |
| 003m-P215S-1×GCN4t-F190S-RQ1 | 11135 | 13463 |
| 003m-P215S-1×GCN4t-F190S-RQ2 | 11306 | 13143 |
| 003m-P215S-1×GCN4t-F190S-RQ3 | 11250 | 13099 |
| 003m-P215S-1×GCN4t-C155S-C290S_RQ1 | 48411 | 94595 |
| 003m-P215S-1×GCN4t-C155S-C290S_RQ2 | 36262 | 67294 |
| 003m-P215S-1×GCN4t-C155S-C290S_RQ3 | 47567 | 93499 |
| 003m-P215S-1×GCN4t-C486D-C489D_RQ1 | 8131 | 8174 |
| 003m-P215S-1×GCN4t-C486D-C489D_RQ2 | 8082 | 8129 |
| 003m-P215S-1×GCN4t-C486D-C489D_RQ3 | 7588 | 7160 |
| 003m-P215S-1×GCN4t-ori_linker-RQ1 | 175 | 480 |
| 003m-P215S-1×GCN4t-ori-linker-RQ2 | 286 | 556 |
| 003m-P215S-1×GCN4t-ori-linker-RQ3 | 182 | 515 |
| 003m-P215S-RQ1 | 496209 | 364297 |
| 003m-P215S -RQ2 | 520970 | 379002 |
| 003m-P215S -RQ3 | 353345 | 264518 |
| 003m-P215S-1×GCN4t-C102A_C362S-RQ1 | 3644 | 13936 |
| 003m-P215S-1×GCN4t-C102A_C362S-RQ2 | 3945 | 15731 |
| 003m-P215S-1×GCN4t-C102A_C362S-RQ3 | 4434 | 16010 |

### Example 20: Immunization and Neutralization Analysis of Candidate RSV B Antigens in BALB/c Mice

### I. Animal Immunization and Serum Collection

### BALB/c mice:

For mouse immunization, vaccine formulations were prepared by encapsulating the mRNA encoding the target sequences (RSV B-003m-P215S-1×GCN4t-RQ1 and RSV B-003m-P215S-RQ1) with Lipid Compound 6.

### Preparation of Lipid Compound 6

### Step 1: Preparation of Intermediate 6-1

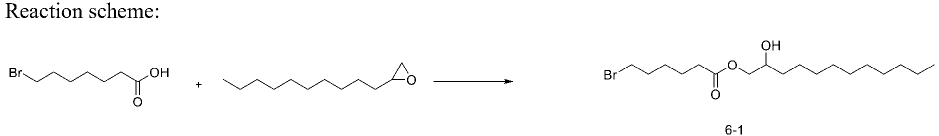

The material ratios are shown in Table 37.

**Table 37. Material Ratio Table for the Preparation of 6-1**

| Material Name | Molecular Weight | eeding Ratio (Equivalents) | Amount Charged | mmol |
|---|---|---|---|---|
| 1,2-Epoxydodecane | 184 | 1 eq | 5g | 27 |
| 6-Bromohexanoic acid | 209 | 1.1 eq | 5.85 g | 30 |
| Ferric chloride (FeCl₃) | 162 | 0.025 eq | 220 mg | 1.35 |
| Pyridine | 79 | 0.0125 eq | 53 mg | 0.68 |

### Procedure:

6-Bromohexanoic acid, 1,2-epoxydodecane, ferric chloride, and pyridine were added to a 250 mL reaction flask in the proportions shown in Table 37. The reaction mixture was stirred at room temperature for 16 hours.

Thin-layer chromatography (TLC) analysis (petroleum ether:ethyl acetate = 4:1) indicated completion of the reaction (Rf of the product = 0.5). The reaction mixture was purified by column chromatography to afford 6.8 g of a colorless oily product.

### Step 2: Preparation of 6-2

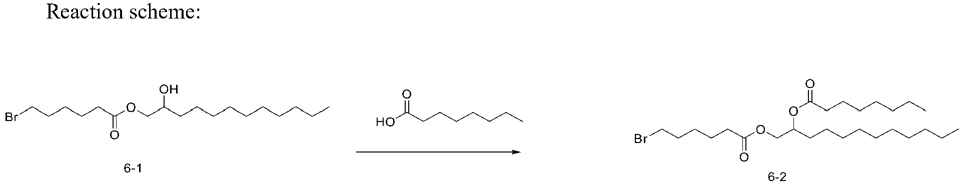

The material ratios are shown in Table 38.

**Table 38. Material Ratio Table for the Preparation of 6-2**

| Material Name | Molecular Weight | eeding Ratio (Equivalents) | Amount Charged | mmol |
|---|---|---|---|---|
| 6-1 | 379.38 | 1 eq | 6.6 g | 17.5 |
| Octanoic acid | 144 | 1.1 eq | 2.8 g | 19.2 |
| EDCI | 192 | 2 eq | 6.7 g | 35 |
| DMAP | 122 | 0.2 eq | 420 mg | 3.5 |
| Dichloromethane | - | - | 80 mL | - |

### Procedure:

Intermediate 6-1, octanoic acid, EDCI, DMAP, and dichloromethane were added to a reaction flask. The reaction mixture was stirred at room temperature for 16 hours.

Thin-layer chromatography (TLC) analysis (petroleum ether:ethyl acetate = 20:1) indicated completion of the reaction (Rf of the product = 0.6). The reaction mixture was washed twice with 100 mL of water. The organic phase was collected and dried over anhydrous sodium sulfate. After filtration and concentration under reduced pressure, the residue was purified by column chromatography to afford 6.5 g of a colorless oily product.

### Step 3: Preparation of Compound 6

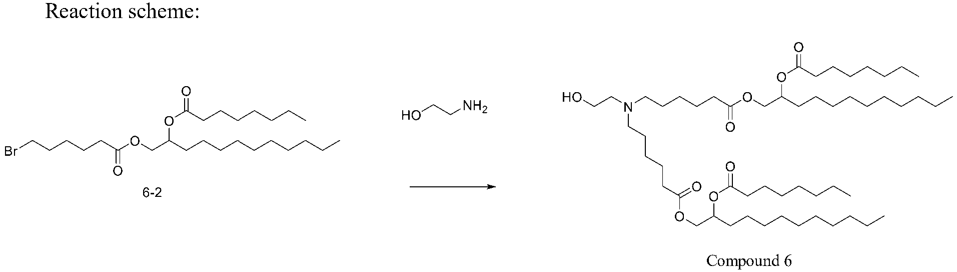

The material ratios are shown in Table 39.

**Table 39. Material Ratio Table for the Preparation of Compound 6**

| Material Name | Molecular Weight | eeding Ratio (Equivalents) | Amount Charged | mmol |
|---|---|---|---|---|
| 6-2 | 505.58 | 2.2 eq | 4.3 g | 8.55 |
| Ethanolamine | 61 | 1 eq | 230 mg | 3.9 |
| K₂CO₃ | 138 | 2 eq | 1.1 g | 7.8 |
| KI | 166 | 2 eq | 1.3 g | 7.8 |
| Acetonitrile | - | - | 40 mL | - |

### Procedure:

Intermediate 6-2, ethanolamine, K₂CO₃, KI, and acetonitrile were added to a reaction flask. The reaction mixture was heated to 65°C and stirred for 16 hours.

Thin-layer chromatography (TLC) analysis (DCM:MeOH = 10:1) indicated completion of the reaction (Rf of the product = 0.5). The reaction mixture was filtered and concentrated under reduced pressure. The residue was purified by column chromatography to afford 2.8 g of a colorless oily product.

**¹H NMR** (600 MHz, Chloroform-d) δ 5.10 - 5.05 (m, 2H), 4.22 (dd, *J* = 11.8, 3.3 Hz, 2H), 4.01 (dd, J = 11.8, 6.8 Hz, 2H), 3.61 (d, *J* = 5.1 Hz, 2H), 2.73 - 2.49 (m, 6H), 2.30 (td, J = 7.5, 5.1 Hz, 8H), 1.65 - 1.50 (m, 16H), 1.33 - 1.23 (m, 52H), 0.91 - 0.85 (m, 12H).

**MS (ES+) m/z ):** 910.7(M+H)⁺.

A control FI-RSV inactivated vaccine was prepared according to Example 1, Section IX ("Preparation of FI-RSV Inactivated Vaccine").

Vaccine formulations comprising the target antigens listed in Table 25 were prepared according to Example 12, using Compound 6:cholesterol:DSPC:DMG-PEG2000 at a molar ratio of 50:38.5:10:1.5 to encapsulate the corresponding mRNA.

The prepared vaccine formulations were administered intramuscularly to 5-7-week-old female BALB/c mice (purchased from SPF (Suzhou) Biotechnology Co., Ltd.) (hereinafter referred to as "immunization"). A booster dose was administered on Day 21 following the initial immunization to enhance the immune response.

Each experimental group consisted of six BALB/c mice (n = 6).

The immunization schedule is shown below.

**Table 40. Immunization Schedule for RSV B-Type F Mutants in BALB/c Mice**

| Antigen Name | Immunization Dose (µg) | Number of Animals (Female) | Administration Volume (mL) | Route of Administration |
|---|---|---|---|---|
| Placebo | NA | 12 | 0.1 | Intramuscular injection |
| 003m-P215S-1×GCN4t (B) | 1 µg | 6 | 0.1 | Intramuscular injection |
| | 5 µg | 6 | 0.1 | Intramuscular injection |
| 003m-P215S (B) | 1 µg | 6 | 0.1 | Intramuscular injection |
| | 5 µg | 6 | 0.1 | Intramuscular injection |
| FI-RSV | 2x10⁶ PFU | 6 | 0.1 | Intramuscular injection |

Two doses were administered at an interval of three weeks, with the dose for each administration as shown in Table 40. An appropriate sterile insulin syringe was used to accurately withdraw 400 µL of the test article (the test article was gently inverted 5-10 times prior to withdrawal to ensure homogeneity). Intramuscular injection was performed in the right hind limb of each animal, with 100 µL administered at a single injection site.

Serum samples were collected from the immunized mice, heat-inactivated at 56°C for 30 minutes, and stored at -80°C for subsequent analysis.

### II. Neutralizing Antibody Analysis

Neutralizing antibody titers were determined using the fluorescence-based method described in Example 1, Section VI ("Neutralizing Antibody Detection").

### FIG. 36

The neutralizing antibody results demonstrated the following:
1. Compared with FI-RSV, both mutant constructs induced higher neutralizing antibody titers.
2. At the 1 µg immunization dose, the mutant lacking 1×GCN4t induced higher neutralizing antibody titers; however, at the 5 µg immunization dose, the RSV B construct containing 1×GCN4t induced higher neutralizing antibody titers.
3. Compared with RSV A strains, both RSV B antigen designs induced higher neutralizing antibody titers against the RSV B strain.

Overall, both RSV B mutant constructs were capable of inducing robust neutralizing antibody responses against the RSV B strain.

The sequences involved in the present invention are set forth in Table 41 and in the sequence listings provided below.

### Nucleotide Sequences

**The nucleotide sequence encoding mA2F** (**SEQ ID NO: 177**) 
ATGGAGCTGCTGATCCTGAAGGCCAACGCCATCACAACAATCCTGACCGCTGTGACCTTC
TGTTTCGCCAGCGGCCAGAACATCACCGAGGAGTTTTATCAGAGCACCTGTAGCGCCGTGAG
CAAGGGATACCTGTCTGCCCTGAGAACCGGCTGGTACACCAGCGTGATCACCATCGAGCTGA
GCAACATCAAGGAGAACAAGTGCAACGGCACCGACGCCAAGGTGAAGCTGATCAAACAGGA
GCTGGACAAGTACAAGAACGCCGTGACCGAGCTGCAGCTGCTCATGCAGAGCACCCCTGCTA
CCAACAATCGGGCTAGACGGGAGCTGCCCCGGTTCATGAATTACACCCTGAACAACGCCAAG
AAGACCAACGTGACACTGTCTAAAAAGAGGAAGCGGAGATTCCTGGGCTTCCTGCTGGGAGT
GGGCTCTGCTATTGCTTCTGGAGTGGCCGTGTCTAAGGTGCTGCACCTGGAGGGAGAGGTGA
ACAAGATCAAGAGCGCCCTGCTGTCCACCAATAAGGCCGTGGTGAGCCTGAGCAATGGCGTG
AGCGTGCTGACCTCTAAGGTCCTGGACCTGAAGAATTACATCGACAAGCAGCTGCTGCCCATC
GTGAACAAGCAGTCATGCAGCATCAGCAACATCGAGACGGTGATCGAGTTTCAGCAGAAGAA
CAACCGGCTGCTGGAGATCACCCGGGAGTTTAGCGTGAACGCCGGCGTGACCACACCCGTGA
GCACCTACATGCTGACCAACAGCGAGCTGCTGTCCCTGATCAACGACATGCCCATCACCAACG
ACCAGAAAAAGCTGATGAGCAACAACGTGCAGATCGTGAGGCAGCAGAGCTACAGCATCATG
 
AGCATCATCAAAGAGGAGGTGCTGGCCTACGTGGTGCAGCTGCCTCTGTACGGCGTGATCGA
CACCCCTTGCTGGAAGCTGCACACCAGCCCTCTGTGCACAACCAATACCAAGGAGGGCAGCA
ATATCTGTCTGACCAGAACCGATAGGGGCTGGTATTGTGACAATGCCGGCAGCGTGTCTTTCTT
CCCACAGGCCGAAACCTGCAAGGTGCAGAGCAACAGAGTGTTCTGCGACACCATGAACAGC
CTGACCCTGCCCAGCGAGGTCAACCTGTGCAATGTGGACATCTTCAACCCCAAGTACGACTGC
AAGATCATGACCAGCAAGACCGACGTGAGCAGCAGCGTGATTACCAGCCTGGGCGCCATCGT
GAGCTGCTACGGAAAAACCAAGTGCACCGCCAGCAACAAGAACAGGGGCATCATTAAGACC
TTCAGCAACGGCTGCGACTACGTGAGCAATAAGGGCGTGGACACCGTGTCCGTGGGCAACAC
ACTGTACTACGTGAATAAGCAGGAGGGCAAGAGCCTGTACGTGAAGGGCGAGCCCATCATCA
ACTTCTACGACCCCCTGGTGTTCCCCTCTGACGAGTTTGACGCCAGCATCTCCCAGGTGAACG
AGAAGATCAACCAGAGCCTGGCCTTCATCCGGAAAAGCGATGAGCTGCTGCACAACGTCAAC
GCCGGCAAGAGCACCACCAACATCATGATCACCACCATCATCATCGTGATCATTGTGATCCTGC
TGAGCCTGATCGCCGTGGGCCTGCTGCTGTACTGCAAGGCCAGGTCTACCCCCGTGACACTCA
GCAAGGATCAGCTGAGCGGCATCAACAATATCGCCTTCTCCAAC
**The nucleotide sequence encoding m5K6I SEQ ID NO: 178**) 
ATGGAGCTGCTGATCCTGAAGGCCAACGCCATCACAACAATCCTGACCGCTGTGACCTTC
TGTTTCGCCAGCGGCCAGAACATCACCGAGGAGTTTTATCAGAGCACCTGTAGCGCCGTGAG
CAAGGGATACCTGTCTGCCCTGAGAACCGGCTGGTACACCAGCGTGATCACCATCGAGCTGA
GCAACATCAAGGAGAACAAGTGCAACGGCACCGACGCCAAGGTGAAGCTGATCAAACAGGA
GCTGGACAAGTACAAGAACGCCGTGACCGAGCTGCAGCTGCTCATGCAGAGCACCCCTGCTA
CCAACAATCGGGCTAGACGGGAGCTGCCCCGGTTCATGAATTACACCCTGAACAACGCCAAG
AAGACCAACGTGACACTGTCTAAAAAGAGGAAGCGGAGATTCCTGGGCTTCCTGCTGGGAGT
GGGCTCTGCTATTGCTTCTGGAGTGGCCGTGTCTAAGGTGCTGCACCTGGAGGGAGAGGTGA
ACAAGATCAAGAGCGCCCTGCTGTCCACCAATAAGGCCGTGGTGAGCCTGAGCAATGGCGTG
AGCGTGCTGACCTCTAAGGTCCTGGACCTGAAGAATTACATCGACAAGCAGCTGCTGCCCATC
GTGAACAAGCAGTCATGCAGCATCAGCAACATCGAGACGGTGATCGAGTTTCAGCAGAAGAA
CAACCGGCTGCTGGAGATCACCCGGGAGTTTAGCGTGAACGCCGGCGTGACCACACCCGTGA
GCACCTACATGCTGACCAACAGCGAGCTGCTGTCCCTGATCAACGACATGCCCATCACCAACG
ACCAGAAAAAGCTGATGAGCAACAACGTGCAGATCGTGAGGCAGCAGAGCTACAGCATCATG
AGCATCATCAAAGAGGAGGTGCTGGCCTACGTGGTGCAGCTGCCTCTGTACGGCGTGATCGA
CACCCCTTGCTGGAAGCTGCACACCAGCCCTCTGTGCACAACCAATACCAAGGAGGGCAGCA
ATATCTGTCTGACCAGAACCGATAGGGGCTGGTATTGTGACAATGCCGGCAGCGTGTCTTTCTT
CCCACAGGCCGAAACCTGCAAGGTGCAGAGCAACAGAGTGTTCTGCGACACCATGAACAGC
CTGACCCTGCCCAGCGAGGTCAACCTGTGCAATGTGGACATCTTCAACCCCAAGTACGACTGC
AAGATCATGACCAGCAAGACCGACGTGAGCAGCAGCGTGATTACCAGCCTGGGCGCCATCGT
GAGCTGCTACGGAAAAACCAAGTGCACCGCCAGCAACAAGAACAGGGGCATCATTAAGACC
TTCAGCAACGGCTGCGACTACGTGAGCAATAAGGGCGTGGACACCGTGTCCGTGGGCAACAC
 
ACTGTACTACGTGAATAAGCAGGAGGGCAAGAGCCTGTACGTGAAGGGCGAGCCCATCATCA
ACTTCTACGACCCCCTGGTGTTCCCCTCTGACGAGTTTGACGCCAGCATCTCCCAGGTGAACG
AGAAGATCAACCAGAGCCTGGCCTTCATCCGGAAAAGCGATGAGCTGCTGCACAACGTCAAC
GCCGGCAAGAGCACCACCAACATCATGATCACCACCATCATCATCGTGATCATTGTGATCCTGC
TGAGCCTGATCGCCGTGGGCCTGCTGCTGTACTGCAAGGCCAGGTCTACCCCCGTGACACTCA
GCAAGGATCAGCTGAGCGGCATCAACAATATCGCCTTCTCCAAC
**The nucleotide sequence encoding DS-Cav1-1xGCN4t** (**SEQ ID NO: 179**) 
atggagctgctcatcctgaaggccaacgccatcaccaccatcctgaccgccgtgacattttgtttcgccagcggccagaacatcaccgaggagttt
taccagagcacctgctctgccgtgagcaagggctacctgtctgccctgagaacaggctggtacaccagcgtgattaccatcgagctgagcaacatcaag
gagaacaagtgcaacggcaccgacgccaaggtgaagctgatcaagcaggagctggacaagtacaagaatgccgtgaccgagctgcagctgctgatg
cagagcacacctgccaccaacaacagggccaggagagagctgccccggttcatgaattacaccctgaacaacgccaagaagaccaacgtgaccctga
gcaagaagagaaagaggcggttcctgggatttctgctgggagtgggctctgctattgcttctggagtggctgtgtgtaaagtgctgcacctggagggaga
ggtgaacaagatcaagagcgccctgctgtctaccaacaaggccgtggtgagcctgagcaacggcgtgagcgtgctgaccttcaaggtgctggacctga
agaattacatcgacaagcagctgctccccatcctgaacaagcagagctgctccatcagcaacatcgagacagtgatcgagtttcagcagaagaacaacc
ggctgctggagatcacccgggagtttagcgtgaacgctggcgtgaccacccccgtgagcacctacatgctgaccaactctgagctgctctccctgatcaa
cgacatgcccatcaccaacgaccagaagaagctgatgtccaacaacgtgcagatcgtgcggcagcagagctacagcatcatgtgtatcatcaaagagg
aggtgctggcctacgtggtgcagctgcctctgtacggcgtgatcgacaccccatgctggaagctgcacacctctcctctgtgtaccacaaacaccaagga
gggcagcaatatctgcctgaccagaaccgatagaggctggtattgcgacaatgccggcagcgtgagcttctttcctcaggccgagacatgcaaggtgca
gagcaacagggtgttttgcgacaccatgaacagcctgaccctgcccagcgaggtgaacctgtgcaacgtggacatcttcaaccccaagtacgactgcaa
gatcatgaccagcaagaccgacgtgtctagctctgtgatcacctctctgggcgccatcgtgagctgttacggcaagaccaagtgcaccgccagcaacaa
gaatcggggcatcatcaagaccttcagcaacggctgcgactacgtgagcaacaagggcgtggataccgtgtctgtgggcaataccctgtactacgtgaa
taagcaggagggcaagagcctgtacgtgaagggcgagcccatcatcaacttctacgacccccctggtgttccccagcgacgagtttgacgccagcatca
gccaggtgaatgagaaaatcaaccagagcctggccttcatccgcaagagcgacgagctgctgcacaacgtgGAGGACAAGATCGAGG
AGATCCTGAGCAAGATCTACCACATCGAGAACGAGATCGCCAGGATCAAGAAGCTGATCGGC
GAGGCCaacgccggcaagagcaccaccaacatcatgatcacaaccatcatcatcgtgattatcgtgatcctgctgagcctgatcgccgtgggcctg
ctgctgtactgtaaggccagaagcacccctgtcacactgagcaaggatcagctgagcggcatcaataacatcgccttctccaac
**The nucleotide sequence encoding DS-Cav1-2xGCN4t** (**SEQ ID NO: 180**) 
atggagctgctcatcctgaaggccaacgccatcaccaccatcctgaccgccgtgacattttgtttcgccagcggccagaacatcaccgaggagttt
taccagagcacctgctctgccgtgagcaagggctacctgtctgccctgagaacaggctggtacaccagcgtgattaccatcgagctgagcaacatcaag
gagaacaagtgcaacggcaccgacgccaaggtgaagctgatcaagcaggagctggacaagtacaagaatgccgtgaccgagctgcagctgctgatg
cagagcacacctgccaccaacaacagggccaggagagagctgccccggttcatgaattacaccctgaacaacgccaagaagaccaacgtgaccctga
gcaagaagagaaagaggcggttcctgggatttctgctgggagtgggctctgctattgcttctggagtggctgtgtgtaaagtgctgcacctggagggaga
ggtgaacaagatcaagagcgccctgctgtctaccaacaaggccgtggtgagcctgagcaacggcgtgagcgtgctgaccttcaaggtgctggacctga
agaattacatcgacaagcagctgctccccatcctgaacaagcagagctgctccatcagcaacatcgagacagtgatcgagtttcagcagaagaacaacc
ggctgctggagatcacccgggagtttagcgtgaacgctggcgtgaccacccccgtgagcacctacatgctgaccaactctgagctgctctccctgatcaa
cgacatgcccatcaccaacgaccagaagaagctgatgtccaacaacgtgcagatcgtgcggcagcagagctacagcatcatgtgtatcatcaaagagg
 
aggtgctggcctacgtggtgcagctgcctctgtacggcgtgatcgacaccccatgctggaagctgcacacctctcctctgtgtaccacaaacaccaagga
gggcagcaatatctgcctgaccagaaccgatagaggctggtattgcgacaatgccggcagcgtgagcttctttcctcaggccgagacatgcaaggtgca
gagcaacagggtgttttgcgacaccatgaacagcctgaccctgcccagcgaggtgaacctgtgcaacgtggacatcttcaaccccaagtacgactgcaa
gatcatgaccagcaagaccgacgtgtctagctctgtgatcacctctctgggcgccatcgtgagctgttacggcaagaccaagtgcaccgccagcaacaa
gaatcggggcatcatcaagaccttcagcaacggctgcgactacgtgagcaacaagggcgtggataccgtgtctgtgggcaataccctgtactacgtgaa
taagcaggagggcaagagcctgtacgtgaagggcgagcccatcatcaacttctacgacccccctggtgttccccagcgacgagtttgacgccagcatca
gccaggtgaatgagaaaatcaaccagagcctggccttcatccgcaagagcgacgagctgctgcacaacgtgGAGGACAAGATCGAGG
AGATCCTGAGCAAGATCTACCACATCGAGAACGAGATCGCCAGGATCAAGAAGCTGATCGGC
GAGGCCATCTACCACATCGAGAACGAGATCGCCAGGATCAAGAAGCTGATCGGCGAGGCCaac
gccggcaagagcaccaccaacatcatgatcacaaccatcatcatcgtgattatcgtgatcctgctgagcctgatcgccgtgggcctgctgctgtactgtaa
ggccagaagcacccctgtcacactgagcaaggatcagctgagcggcatcaataacatcgccttctccaac
**The nucleotide sequence encoding Moderna-ACT** (**SEQ ID NO: 181**) 
atggagctgctgatcctgaaggccaacgccattacaaccatcctgaccgccgtgaccttctgcttcgctagcgggcagaacatcaccgaggagtt
ctatcagagcacctgcagcgccgtgagcaagggctacctgagcgccctgagaaccggctggtacacaagcgtgattaccatcgagctgagcaacatca
aggagaacaagtgcaacggcaccgacgccaaggtgaagctgatcaagcaagagctggacaagtacaagaacgccgtgaccgagctgcagctgctga
tgcagagcacccccgccaccggcagcgggagcgccatctgcagcggcgtggccgtgtgcaaggtgctgcacctggagggcgaggtgaacaagatc
aagagcgccctgctgagcaccaacaaggccgtggtgagcctgagcaacggcgtgagcgtgctgaccttcaaggtgctggacctgaagaactacatcg
acaagcagctgctgcccatcctgaacaagcagagctgcagcatcagcaacatcgagaccgtgatcgagtttcagcagaagaacaacagactgctggag
atcacaagagagttcagcgtcaatgccggcgtgaccacccccgtgagcacctacatgctgaccaacagcgagctgctctccctgattaacgacatgccc
atcaccaacgatcagaagaagctgatgagcaacaacgtgcagatcgtgagacagcagagctacagcatcatgtgcatcatcaaggaagaggtgctggc
ctacgtggtgcagctgcccctgtacggcgtgatcgacacccccctgctggaagctgcacacaagcccccctgtgcaccaccaacaccaaggagggcagc
aacatctgcctgacaagaaccgacagaggctggtactgcgacaacgccggcagcgtgagcttcttcccccaagccgagacctgcaaggtgcagagca
acagagtgttctgcgacaccatgaacagccgtaccctgcctagcgaggtgaacctgtgcaacgtggacatcttcaaccccaagtacgactgcaagatcat
gacaagcaagaccgacgtgagcagctccgtgatcacaagcctcggcgccatcgtgagctgctacggcaagaccaagtgcaccgctagcaacaagaa
cagaggcatcatcaagaccttcagcaacggctgcgactacgtgagcaacaagggcgtggacaccgtgagcgtgggcaacaccctgtactgcgtgaac
aagcaagagggcaagagcctgtacgtgaagggcgagcccatcatcaacttctacgacccccctggtgttccctagcgacgagttcgacgctagcatcagc
caagtgaacgagaagatcaatcagagcctggccttcatcagaaagagcgacgagctgctgcacaacgtgaatgctggcaaaagcaccacaaacatcat
gatcacaacaatcattatcgtgatcatcgtgatcctcctgagcctgatcgccgtgggcctgctcctgtactgc
**The optimized nucleotide sequence encoding 003m-1xGCN4t** (**optimize 1**) (**SEQ ID NO: 182**) 
ATGGAGCTGCCCATCCTGAAGACCAATGCCATCACAACCATCCTGGCCGCCGTGACACTG
TGTTTCGCCAGCAGCCAGAATATCACCGAGGAGTTTTACCAGAGCACCTGCAGCGCCGTGAG
CAAGGGCTATCTGGGCGCTCTGAGGACAGGCTGGTACACCAGCGTGATTACCATCGAGCTGA
GCAATATCAAGGAGAACAAGTGCAACGGCACCGACGCCAAGGTGAAGCTGATTAAGCAGGA
GCTGGACAAGTACAAGAACGCCGTGACCGATCTGCAGCTGCTGATGCAGAGCACACCTTGTG
GCGGATCTGGAGGAGGATCTGGCTCTGCTATCGCCAGCGGAATTGCCGTGTGTAAAGTGCTGC
 
ACCTGGAGGGAGAGGTGAATAAGATCAAAAGCGCCCTGCTGAGCACCAACAAGGCCGTGGT
GAGCCTGAGCAATGGCGTGAGCGTGCTGACCTTCAAGGTGCTGGACCTGAAGAATTACATCG
ACAAGCAGCTGCTCCCCATCGTGAACAAGCAGAGCTGCAGCATCCCCAACATCGAGACAGTG
ATCGAGTTTCAGCAGAAGAACAACCGGCTGCTGGAGATCACCCGGGAGTTTAGCGTGAATGC
CGGCGTGACCACCCCAGTGAGCACCTACATGCTGACCAACAGCGAGCTGCTGAGCCTGATCA
ACGACATGCCTATCACCAACGACCAGAAGAAGCTCATGAGCAGCAACGTGCAGATCGTGAGG
CAGCAGAGCTACAGCATCATGTGCATCATCAAGGAGGAGGTGCTGGCCTATGTGGTGCAGCTG
CCTCTGTACGGCGTGATCGATACCCCCTGCTGGAAGCTGCACACATCTCCTCTGTGCACAACC
AATACCAAGGAGGGCAGCAATATCTGCCTGACCAGAACCGATAGGGGCTGGTACTGTGACAAT
GCCGGCAGCGTGAGCTTTTTCCCTCAGGCCGAGACATGTAAGGTGCAGTGCAATCGGGTGTTC
TGCGACACCATGAACAGCCTGACCCTGCCCAGCGAGGTGAACCTGTGCAACATCGACATCTT
CAACCCCAAGTACGACTGCAAGATCATGACCAGCAAGACCGATGTGAGCAGCAGCGTGATCA
CAAGCCTGGGAGCCATCGTGAGCTGCTACGGCAAGACCAAGTGCACCGCCAGCAACAAGAA
CCGGGGCATCATTAAGACCTTCAGCAACGGCTGCGACTACGTGAGCAACAAGGGCGTGGATA
CAGTGAGCGTGGGCAATACCCTGTACTACGTGAATAAGCAGGAAGGCAAGAGCCTGTACGTG
AAGGGCGAGCCCATCATCAACTTCTACGACCCCCTGGTGTTCCCCAGCTGCGAGTTTGCGCC
AGCATCAGCCAGGTGAACGAGAAGATCAACCAGAGCCTGGCCTTCATCAGGAAGAGCGATGA
GCTGCTGCACAACGTGGAGGACAAGATCGAGGAGATCCTGAGCAAGATCTACCACATCGAGA
ACGAGATCGCCCGGATCAAGAAGCTGATCGGCGAGGCCAACGCCGGCAAGAGCACCACCAA
CATCATGATCACCACCATCATCATCGTGATCATTGTGATCCTGCTGGCCCTGATCGCCGTGGGC
CTGCTGCTGTACTGTAAGGCCAGGTCTACACCTGTGACACTGAGCAAGGATCAGCTGAGCGG
CATCAACAATATCGCCTTCTCCAAC
**The optimized nucleotide sequence encoding 003m-1xGCN4t** (**optimize 2**) (**SEQ ID NO: 183**) 
ATGGAGCTGCCCATCCTGAAGACCAATGCCATCACAACCATCCTGGCCGCCGTGACACTG
TGTTTCGCCAGCAGCCAGAATATCACCGAGGAGTTTTACCAGAGCACCTGCAGCGCCGTGAG
CAAGGGCTATCTGGGCGCTCTGAGGACAGGCTGGTACACCAGCGTGATTACCATCGAGCTGA
GCAATATCAAGGAGAACAAGTGCAACGGCACCGACGCCAAGGTGAAGCTGATTAAGCAGGA
GCTGGACAAGTACAAGAACGCCGTGACCGATCTGCAGCTGCTGATGCAGAGCACACCTTGTG
GCGGATCTGGAGGAGGATCTGGCTCTGCTATCGCCAGCGGAATTGCCGTGTGTAAAGTGCTGC
ACCTGGAGGGAGAGGTGAATAAGATCAAAAGCGCCCTGCTGAGCACCAACAAGGCCGTGGT
GAGCCTGAGCAATGGCGTGAGCGTGCTGACCTTCAAGGTGCTGGACCTGAAGAATTACATCG
ACAAGCAGCTGCTCCCCATCGTGAACAAGCAGAGCTGCAGCATCCCCAACATCGAGACAGTG
ATCGAGTTTCAGCAGAAGAACAACCGGCTGCTGGAGATCACCCGGGAGTTTAGCGTGAATGC
 
CGGCGTGACCACCCCAGTGAGCACCTACATGCTGACCAATAGCGAGCTGCTGAGCCTGATCA
ACGACATGCCCATCACCAACGACCAGAAGAAGCTCATGAGCAGCAACGTGCAGATCGTGAGG
CAGCAGAGCTACAGCATCATGTGCATCATCAAGGAGGAGGTGCTGGCCTATGTGGTGCAGCTG
CCTCTGTACGGCGTGATCGATACCCCCTGCTGGAAGCTGCACACATCTCCTCTGTGCACAACC
AATACCAAGGAGGGCAGCAATATCTGCCTGACCAGAACCGATAGGGGCTGGTACTGTGACAAT
GCCGGCAGCGTGAGCTTTTTCCCTCAGGCCGAGACATGTAAGGTGCAGTGCAATCGGGTGTTC
TGCGACACCATGAACAGCCTGACCCTGCCCAGCGAGGTGAACCTGTGCAACATCGACATCTT
CAACCCCAAGTACGACTGCAAGATCATGACCAGCAAGACCGATGTGAGCAGCAGCGTGATCA
CAAGCCTGGGAGCCATCGTGAGCTGCTACGGCAAGACCAAGTGCACCGCCAGCAACAAGAA
CCGGGGCATCATTAAGACCTTCAGCAACGGCTGCGACTACGTGAGCAACAAGGGCGTGGATA
CAGTGAGCGTGGGCAATACCCTGTACTACGTGAATAAGCAGGAAGGCAAGAGCCTGTACGTG
AAGGGCGAGCCCATCATCAACTTCTACGACCCCCTGGTGTTCCCCAGCTGCGAGTTTTGCGCC
AGCATCAGCCAGGTGAACGAGAAGATCAACCAGAGCCTGGCCTTCATCAGGAAGAGCGATGA
GCTGCTGCACAACGTGGAGGACAAGATCGAGGAGATCCTGAGCAAGATCTACCACATCGAGA
ACGAGATCGCCCGGATCAAGAAGCTGATCGGCGAGGCCAACGCCGGCAAGAGCACCACCAA
CATCATGATCACCACCATCATCATCGTGATCATTGTGATCCTGCTGGCCCTGATCGCCGTGGGC
CTGCTGCTGTACTGTAAGGCCAGGTCTACACCTGTGACACTGAGCAAGGATCAGCTGAGCGG
CATCAACAATATCGCCTTCTCCAAC
**The optimized nucleotide sequence encoding 003m-1xGCN4t** (**optimize 3**) (**SEQ ID NO: 184**) 
ATGGAGCTGCCCATCCTGAAGACCAATGCCATCACAACCATCCTGGCCGCCGTGACACTG
TGTTTCGCCAGCAGCCAGAATATCACCGAGGAGTTTTACCAGAGCACCTGCAGCGCCGTGAG
CAAGGGCTATCTGGGCGCTCTGAGGACAGGCTGGTACACCAGCGTGATTACCATCGAGCTGA
GCAATATCAAGGAGAACAAGTGCAACGGCACCGACGCCAAGGTGAAGCTGATTAAGCAGGA
GCTGGACAAGTACAAGAACGCCGTGACCGATCTGCAGCTGCTGATGCAGAGCACACCTTGTG
GCGGATCTGGAGGAGGATCTGGCAGCGCTATCGCCTCTGGCATTGCCGTGTGTAAAGTGCTGC
ACCTGGAAGGCGAAGTGAATAAGATCAAAAGCGCCCTGCTGAGCACCAACAAGGCCGTGGT
GAGCCTGAGCAATGGCGTGAGCGTGCTGACCTTCAAGGTGCTGGACCTGAAGAATTACATCG
ACAAGCAGCTGCTCCCCATCGTGAACAAGCAGAGCTGCAGCATCCCCAACATCGAGACAGTG
ATCGAGTTTCAGCAGAAGAACAACCGGCTGCTGGAGATCACCCGGGAGTTTAGCGTGAATGC
CGGCGTGACCACCCCAGTGAGCACCTACATGCTGACCAATAGCGAGCTGCTGAGCCTGATCA
ACGACATGCCCATCACCAACGACCAGAAGAAGCTCATGAGCAGCAACGTGCAGATCGTGAGG
CAGCAGAGCTACAGCATCATGTGCATCATCAAGGAGGAGGTGCTGGCCTATGTGGTGCAGCTG
CCTCTGTACGGCGTGATCGATACCCCCTGCTGGAAGCTGCACACATCTCCTCTGTGCACAACC
 
AATACCAAGGAGGGCAGCAATATCTGCCTGACCAGAACCGATAGGGGCTGGTACTGTGACAAT
GCCGGCAGCGTGAGCTTTTTCCCTCAGGCCGAGACATGTAAGGTGCAGTGCAATCGGGTGTTC
TGCGACACCATGAACAGCCTGACCCTGCCCAGCGAGGTGAACCTGTGCAACATCGACATCTT
CAACCCCAAGTACGACTGCAAGATCATGACCAGCAAGACCGATGTGAGCAGCAGCGTGATCA
CAAGCCTGGGAGCCATCGTGAGCTGCTACGGCAAGACCAAGTGCACCGCCAGCAACAAGAA
CCGGGGCATCATTAAGACCTTCAGCAACGGCTGCGACTACGTGAGCAACAAGGGCGTGGATA
CAGTGAGCGTGGGCAATACCCTGTACTACGTGAATAAGCAGGAAGGCAAGAGCCTGTACGTG
AAGGGCGAGCCCATCATCAACTTCTACGACCCCCTGGTGTTCCCCAGCTGCGAGTTTTGCGCC
AGCATCAGCCAGGTGAACGAGAAGATCAACCAGAGCCTGGCCTTCATCAGGAAGAGCGATGA
GCTGCTGCACAACGTGGAGGACAAGATCGAGGAGATCCTGAGCAAGATCTACCACATCGAGA
ACGAGATCGCCCGGATCAAGAAGCTGATCGGCGAGGCCAACGCCGGCAAGAGCACCACCAA
CATCATGATCACCACCATCATCATCGTGATCATTGTGATCCTGCTGGCCCTGATCGCCGTGGGC
CTGCTGCTGTACTGTAAGGCCAGGTCTACACCTGTGACACTGAGCAAGGATCAGCTGAGCGG
CATCAACAATATCGCCTTCTCCAAC
**The optimized nucleotide sequence encoding 003m-1xGCN4t** (**optimize 4**) (**SEQ ID NO: 185**) 
ATGGAGCTGCCCATCCTGAAGACCAATGCCATCACAACCATCCTGGCCGCCGTGACACTG
TGTTTCGCCAGCAGCCAGAATATCACCGAGGAGTTTTACCAGAGCACCTGCAGCGCCGTGAG
CAAGGGCTATCTGGGCGCTCTGAGGACAGGCTGGTACACCAGCGTGATTACCATCGAGCTGA
GCAATATCAAGGAGAACAAGTGCAACGGCACCGACGCCAAGGTGAAGCTGATTAAGCAGGA
GCTGGACAAGTACAAGAACGCCGTGACCGATCTGCAGCTGCTGATGCAGAGCACACCTTGTG
GCGGATCTGGAGGAGGATCTGGCAGCGCTATCGCCTCTGGCATTGCCGTGTGTAAAGTGCTGC
ACCTGGAAGGCGAAGTGAATAAGATCAAATCCGCCCTGCTGAGCACCAACAAGGCCGTGGTG
AGCCTGAGCAATGGCGTGAGCGTGCTGACCTTCAAGGTGCTGGACCTGAAGAATTACATCGA
CAAGCAGCTGCTCCCCATCGTGAACAAGCAGAGCTGCAGCATCCCCAACATCGAGACAGTGA
TCGAGTTTCAGCAGAAGAACAACCGGCTGCTGGAGATCACCCGGGAGTTTAGCGTGAATGCC
GGCGTGACCACCCCAGTGAGCACCTACATGCTGACCAATAGCGAGCTGCTGAGCCTGATCAA
CGACATGCCCATCACCAACGACCAGAAGAAGCTCATGAGCAGCAACGTGCAGATCGTGAGGC
AGCAGAGCTACAGCATCATGTGCATCATCAAGGAGGAGGTGCTGGCCTATGTGGTGCAGCTGC
CTCTGTACGGCGTGATCGATACCCCCTGCTGGAAGCTGCACACATCTCCTCTGTGCACAACCA
ATACCAAGGAGGGCAGCAATATCTGCCTGACCAGAACCGATAGGGGCTGGTACTGTGACAATG
CCGGCAGCGTGAGCTTTTTCCCTCAGGCCGAGACATGTAAGGTGCAGTGCAATCGGGTGTTCT
GCGACACCATGAACAGCCTGACCCTGCCCAGCGAGGTGAACCTGTGCAACATCGACATCTTC
AACCCCAAGTACGACTGCAAGATCATGACCAGCAAGACCGATGTGAGCAGCTCCGTGATCAC
 
AAGCCTGGGAGCCATCGTGAGCTGCTACGGCAAGACCAAGTGCACCGCCAGCAACAAGAAC
CGGGGCATCATTAAGACCTTCAGCAACGGCTGCGACTACGTGAGCAACAAGGGCGTGGATAC
AGTGAGCGTGGGCAATACCCTGTACTACGTGAATAAGCAGGAAGGCAAGAGCCTGTACGTGA
AGGGCGAGCCCATCATCAACTTCTACGACCCCCTGGTGTTCCCCAGCTGCGAGTTTTGCGCCA
GCATCAGCCAGGTGAACGAGAAGATCAACCAGAGCCTGGCCTTCATCCGGAAGAGCGATGAG
CTGCTGCACAACGTGGAGGACAAGATCGAGGAGATCCTGAGCAAGATCTACCACATCGAGAA
CGAGATCGCCCGGATCAAGAAGCTGATCGGCGAGGCCAACGCCGGCAAGAGCACCACCAAC
ATCATGATCACCACCATCATCATCGTGATCATTGTGATCCTGCTGGCCCTGATCGCCGTGGGCC
TGCTGCTGTACTGTAAGGCCAGGTCTACACCTGTGACACTGAGCAAGGATCAGCTGAGCGGC
ATCAATAATATCGCCTTCTCCAAC
**The optimized nucleotide sequence encoding 003m-1xGCN4t** (**optimize 5**) (**SEQ ID NO: 186**) 
ATGGAGCTGCCCATCCTGAAGACCAATGCCATCACAACCATCCTGGCCGCCGTGACACTG
TGTTTCGCCAGCAGCCAGAATATCACCGAGGAGTTTTACCAGAGCACCTGCAGCGCCGTGAG
CAAGGGCTATCTGGGCGCTCTGAGGACAGGCTGGTACACCAGCGTGATCACCATTGAGCTGA
GCAACATCAAAGAGAACAAGTGCAACGGCACCGACGCCAAGGTGAAGCTGATTAAGCAGGA
GCTGGACAAGTACAAGAACGCCGTGACCGATCTGCAGCTGCTGATGCAGAGCACACCTTGTG
GCGGATCTGGAGGAGGATCTGGCAGCGCTATCGCCTCTGGCATTGCCGTGTGTAAAGTGCTGC
ACCTGGAAGGCGAAGTGAACAAGATCAAATCTGCCCTGCTGAGCACCAACAAGGCCGTGGT
GAGCCTGAGCAATGGCGTGAGCGTGCTGACCTTCAAGGTGCTGGACCTGAAGAATTACATCG
ACAAGCAGCTGCTCCCCATCGTGAACAAGCAGAGCTGCAGCATCCCCAACATCGAGACAGTG
ATCGAGTTTCAGCAGAAGAACAACCGGCTGCTGGAGATCACCCGGGAGTTTAGCGTGAATGC
CGGCGTGACCACCCCAGTGAGCACCTACATGCTGACCAATAGCGAGCTGCTGAGCCTGATCA
ACGACATGCCCATCACCAACGACCAGAAGAAGCTCATGAGCAGCAACGTGCAGATCGTGAGG
CAGCAGAGCTACAGCATCATGTGCATCATCAAGGAGGAGGTGCTGGCCTATGTGGTGCAGCTG
CCTCTGTACGGCGTGATCGATACCCCCTGCTGGAAGCTGCACACATCTCCTCTGTGCACAACC
AATACCAAGGAGGGCAGCAATATCTGCCTGACCAGAACCGATAGGGGCTGGTACTGTGACAAT
GCCGGCAGCGTGAGCTTTTTCCCTCAGGCCGAGACATGTAAGGTGCAGTGCAATCGGGTGTTC
TGCGACACCATGAACAGCCTGACCCTGCCCAGCGAGGTGAACCTGTGCAACATCGACATCTT
CAACCCCAAGTACGACTGCAAGATCATGACCAGCAAGACCGACGTGTCTAGCTCCGTGATCA
CAAGCCTGGGAGCCATCGTGAGCTGCTACGGCAAGACCAAGTGCACCGCCAGCAACAAGAA
CCGGGGCATCATTAAGACCTTCAGCAACGGCTGCGACTACGTGAGCAACAAGGGCGTGGATA
CAGTGAGCGTGGGCAATACCCTGTACTACGTGAATAAGCAGGAAGGCAAGAGCCTGTACGTG
AAGGGCGAGCCCATCATCAACTTCTACGACCCCCTGGTGTTCCCCAGCTGCGAGTTTTGCGCC
 
AGCATCAGCCAGGTGAACGAGAAGATCAACCAGAGCCTGGCCTTCATCCGGAAGAGCGATGA
GCTGCTGCACAACGTGGAGGACAAGATCGAGGAGATCCTGAGCAAAATCTACCACATCGAGA
ACGAGATCGCCCGGATCAAGAAGCTGATCGGCGAGGCCAACGCCGGCAAGAGCACCACCAA
CATCATGATCACCACCATCATCATCGTGATCATTGTGATCCTGCTGGCCCTGATCGCCGTGGGC
CTGCTGCTGTACTGTAAGGCCAGGTCTACACCTGTGACCCTGAGCAAGGATCAGCTGAGCGG
CATCAATAATATCGCCTTCTCCAAC
**The optimized nucleotide sequence encoding 003m-1xGCN4t** (**optimize 6**) (**SEQ ID NO: 187**) 
ATGGAGCTGCCCATCCTGAAGACCAATGCCATCACAACCATCCTGGCCGCCGTGACACTG
TGTTTCGCCAGCAGCCAGAATATCACCGAGGAGTTTTACCAGAGCACCTGCAGCGCCGTGAG
CAAGGGCTATCTGGGCGCTCTGAGGACAGGCTGGTACACCAGCGTGATCACCATTGAGCTGA
GCAACATCAAAGAGAACAAGTGCAACGGCACCGACGCCAAGGTGAAGCTGATTAAGCAGGA
GCTGGACAAGTACAAGAACGCCGTGACCGATCTGCAGCTGCTGATGCAGAGCACACCTTGTG
GCGGATCTGGAGGAGGATCTGGCAGCGCTATCGCCTCTGGCATTGCCGTGTGTAAAGTGCTGC
ACCTGGAAGGCGAAGTGAACAAGATCAAATCTGCCCTGCTGAGCACCAACAAGGCCGTGGT
GAGCCTGAGCAATGGCGTGAGCGTGCTGACCTTCAAGGTGCTGGACCTGAAGAATTACATCG
ACAAGCAGCTGCTCCCCATCGTGAACAAGCAGAGCTGCAGCATCCCCAACATCGAGACAGTG
ATCGAGTTTCAGCAGAAGAACAACCGGCTGCTGGAGATCACCCGGGAGTTTAGCGTGAATGC
CGGCGTGACCACCCCAGTGAGCACCTACATGCTGACCAATAGCGAGCTGCTGAGCCTGATCA
ACGACATGCCCATCACCAACGACCAGAAGAAGCTCATGAGCAGCAACGTGCAGATCGTGAGG
CAGCAGAGCTACAGCATCATGTGCATCATCAAGGAGGAGGTGCTGGCCTACGTGGTGCAGCT
GCCTCTGTACGGCGTGATCGATACCCCATGCTGGAAGCTGCACACATCTCCTCTGTGCACAAC
CAATACCAAGGAGGGCAGCAACATCTGTCTGACCAGAACCGATAGGGGCTGGTACTGTGACA
ATGCCGGCAGCGTGTCTTTCTTTCCTCAGGCCGAGACATGCAAGGTGCAGTGCAATCGGGTGT
TCTGCGACACCATGAACAGCCTGACCCTGCCCAGCGAGGTGAACCTGTGCAACATCGACATC
TTCAACCCCAAGTACGACTGCAAGATCATGACCAGCAAGACCGACGTGTCTAGCTCCGTGATC
ACAAGCCTGGGAGCCATCGTGAGCTGCTACGGCAAGACCAAGTGCACCGCCAGCAACAAGA
ACCGGGGCATCATTAAGACCTTCAGCAACGGCTGCGACTACGTGAGCAACAAGGGCGTGGAT
ACAGTGAGCGTGGGCAATACCCTGTACTACGTGAATAAGCAGGAAGGCAAGAGCCTGTACGT
GAAGGGCGAGCCCATCATCAACTTCTACGACCCCCTGGTGTTCCCCAGCTGCGAGTTTTGCGC
CAGCATCAGCCAGGTGAACGAGAAGATCAACCAGAGCCTGGCCTTCATCCGGAAGAGCGATG
AGCTGCTGCACAACGTGGAGGACAAGATCGAGGAGATCCTGAGCAAAATCTACCACATCGAG
AACGAGATCGCCCGGATCAAGAAGCTGATCGGCGAGGCCAACGCCGGCAAGAGCACCACCA
ACATCATGATCACCACCATCATCATCGTGATCATTGTGATCCTGCTGGCCCTGATCGCCGTGGG
 
CCTGCTGCTGTACTGTAAGGCCAGGTCTACACCTGTGACCCTGAGCAAGGATCAGCTGAGCG
GCATCAATAATATCGCCTTCTCCAAC
**The optimized nucleotide sequence encoding 003m-1xGCN4t** (**optimize 7**) (**SEQ ID NO: 188**) 
ATGGAGCTGCCAATCCTGAAGACCAATGCCATCACAACCATCCTGGCCGCCGTGACACTG
TGCTTTGCCTCCAGCCAGAATATCACCGAGGAGTTTTACCAGAGCACCTGCAGCGCCGTGAGC
AAGGGCTATCTGGGCGCTCTGAGGACAGGCTGGTACACCAGCGTGATCACCATTGAGCTGAG
CAACATCAAAGAGAACAAGTGCAACGGCACCGACGCCAAGGTGAAGCTGATTAAGCAGGAG
CTGGACAAGTACAAGAACGCCGTGACTGACCTGCAGCTGCTGATGCAGAGCACACCTTGTGG
CGGATCTGGAGGAGGATCTGGCAGCGCTATCGCCTCTGGCATTGCCGTGTGTAAAGTGCTGCA
CCTGGAAGGCGAAGTGAACAAGATCAAATCTGCCCTGCTGAGCACCAACAAGGCCGTGGTG
AGCCTGAGCAATGGCGTGAGCGTGCTGACCTTCAAGGTGCTGGACCTGAAGAATTACATCGA
CAAGCAGCTGCTCCCCATCGTGAACAAGCAGAGCTGCAGCATCCCCAACATCGAGACAGTGA
TCGAGTTTCAGCAGAAGAACAACCGGCTGCTGGAGATCACCCGGGAGTTTAGCGTGAATGCC
GGCGTGACCACCCCCGTGAGCACCTACATGCTGACCAATAGCGAGCTGCTGAGCCTGATCAA
CGACATGCCAATCACCAACGACCAGAAGAAGCTCATGAGCAGCAACGTGCAGATCGTGAGAC
AGCAGAGCTACAGCATCATGTGCATCATCAAGGAGGAGGTGCTGGCCTACGTGGTGCAGCTG
CCTCTGTACGGCGTGATCGACACCCCATGCTGGAAGCTGCACACATCTCCTCTGTGCACAACC
AATACCAAGGAGGGCAGCAACATCTGTCTGACCAGAACCGATAGGGGCTGGTACTGTGACAA
TGCCGGCAGCGTGTCTTTCTTTCCTCAGGCCGAGACATGCAAAGTGCAGTGCAACCGGGTGT
TCTGCGACACCATGAACAGCCTGACCCTGCCCAGCGAGGTGAACCTGTGCAACATCGACATC
TTCAACCCCAAGTACGACTGCAAGATCATGACCAGCAAGACCGACGTGTCTAGCTCCGTGATC
ACAAGCCTGGGAGCCATCGTGAGCTGCTACGGCAAGACCAAGTGCACCGCCAGCAACAAGA
ACCGGGGCATCATTAAGACCTTCAGCAACGGCTGCGACTACGTGAGCAACAAGGGCGTGGAT
ACAGTGAGCGTGGGCAATACCCTGTACTACGTGAATAAGCAGGAAGGCAAGAGCCTGTACGT
GAAGGGCGAGCCCATCATCAACTTCTACGACCCCCTGGTGTTCCCCAGCTGCGAGTTTTGCGC
CTCTATCAGCCAGGTGAACGAGAAGATCAACCAGAGCCTGGCCTTCATCCGGAAGAGCGATG
AGCTGCTGCACAACGTGGAGGACAAGATCGAGGAGATCCTGAGCAAAATCTACCACATCGAG
AACGAGATCGCCCGGATCAAGAAGCTGATCGGCGAGGCCAACGCCGGCAAGAGCACCACCA
ACATCATGATCACCACCATCATCATCGTGATCATTGTGATCCTGCTGGCCCTGATCGCCGTGGG
CCTGCTGCTGTACTGTAAGGCCAGGTCTACACCTGTGACCCTGAGCAAGGATCAGCTGAGCG
GCATCAATAATATCGCCTTCTCCAAC
**The optimized nucleotide sequence encoding 003m-1xGCN4t** (**optimize 8**) (**SEQ ID NO: 189**) 
ATGGAGCTGCCCATCCTGAAGACCAATGCCATCACAACCATCCTGGCTGCCGTGACACTG
TGCTTTGCTAGCTCCCAGAACATCACCGAGGAGTTTTACCAGAGCACCTGCAGCGCCGTGAG
CAAGGGCTATCTGGGCGCCCTGAGGACAGGCTGGTACACCAGCGTGATCACCATCGAGCTGT
CTAACATCAAGGAAAACAAGTGCAACGGCACTGACGCCAAGGTGAAGCTGATTAAGCAGGA
GCTGGACAAGTACAAGAACGCCGTGACTGACCTGCAGCTGCTGATGCAGAGCACACCTTGTG
GCGGATCTGGCGGAGGATCTGGCAGCGCTATCGCCTCTGGCATTGCCGTGTGTAAAGTGCTGC
ACCTGGAAGGCGAAGTGAACAAGATCAAATCTGCCCTGCTGAGCACCAACAAGGCCGTGGT
GAGCCTGAGCAATGGCGTGAGCGTGCTGACCTTCAAGGTGCTGGACCTGAAGAATTACATCG
ACAAGCAGCTGCTCCCCATCGTGAACAAGCAGAGCTGCAGCATCCCCAACATCGAGACAGTG
ATCGAGTTTCAGCAGAAGAACAACCGGCTGCTGGAGATCACCCGGGAGTTTAGCGTGAACGC
CGGCGTGACCACCCCTGTGAGCACCTATATGCTGACCAACAGCGAGCTGCTGAGCCTGATCAA
CGACATGCCCATCACCAACGACCAGAAGAAGCTCATGAGCAGCAACGTGCAGATCGTGAGAC
AGCAGAGCTACAGCATCATGTGCATCATCAAGGAGGAGGTGCTGGCCTACGTGGTGCAGCTG
CCCCTGTACGGAGTGATCGACACCCCCTGTTGGAAGCTGCACACATCTCCTCTGTGCACAACC
AATACCAAGGAGGGCAGCAACATCTGCCTGACAAGAACCGATAGGGGCTGGTACTGTGACAA
TGCCGGCTCTGTGTCTTTCTTTCCTCAGGCCGAGACATGCAAGGTGCAGTGCAACCGGGTGTT
CTGCGACACCATGAACAGCCTGACCCTGCCCAGCGAGGTGAACCTGTGCAACATCGACATCT
TCAACCCCAAGTACGACTGCAAGATCATGACCAGCAAGACCGACGTGTCTAGCTCCGTGATC
ACAAGCCTGGGAGCCATCGTGAGCTGCTACGGCAAGACCAAGTGCACCGCCAGCAACAAGA
ATCGGGGCATCATTAAGACCTTCAGCAACGGCTGCGACTACGTGAGCAACAAGGGCGTGGAT
ACAGTGAGCGTGGGCAACACCCTGTACTACGTGAATAAGCAGGAAGGCAAGAGCCTGTACGT
GAAGGGCGAGCCCATCATCAACTTCTACGACCCCCTGGTGTTCCCCAGCTGCGAGTTTTGCGC
CTCTATCAGCCAGGTGAACGAGAAGATCAACCAGAGCCTGGCCTTCATCCGGAAGAGCGACG
AGCTCCTGCACAACGTGGAGGACAAGATCGAGGAGATCCTGAGCAAAATCTACCACATCGAG
AACGAGATCGCCCGGATCAAGAAGCTGATCGGCGAGGCCAACGCCGGCAAGAGCACCACCA
ACATCATGATCACCACCATCATCATCGTGATCATTGTGATCCTGCTGGCCCTGATCGCCGTGGG
CCTGCTGCTGTACTGTAAGGCCAGGTCTACACCTGTGACCCTGAGCAAGGATCAGCTGAGCG
GCATCAATAATATCGCCTTCTCCAAC
**The nucleotide sequence encoding 003m-2xGCN4t** (**SEQ ID NO: 190**) 
ATGGAGCTGCCCATCCTGAAGACCAATGCCATTACAACCATCCTGGCCGCCGTGACACTG
TGTTTCGCCAGCTCTCAGAACATCACCGAGGAATTTTACCAGAGCACCTGCAGCGCCGTGAGC
AAGGGCTACCTGGGCGCCCTGAGGACCGGCTGGTACACAAGCGTGATCACCATCGAGCTGAG
CAACATCAAAGAAAACAAGTGCAACGGCACAGACGCCAAAGTGAAGCTGATCAAGCAGGAG
CTGGACAAGTACAAGAACGCCGTGACTGACCTGCAGCTGCTGATGCAGTCAACACCTTGTGG
 
CGGATCTGGCGGAGGATCTGGCAGCGCTATCGCCTCTGGAATTGCCGTGTGTAAAGTGCTGCA
CCTGGAGGGCGAAGTGAACAAGATCAAAAGCGCCCTGCTGAGCACCAACAAGGCTGTGGTG
AGCCTGAGCAATGGCGTGAGCGTGCTGACCTTCAAGGTGCTGGACCTGAAAAACTACATTGA
CAAGCAGCTGCTCCCCATCGTGAACAAGCAGAGCTGCAGCATCCCCAACATCGAGACGGTGA
TCGAGTTTCAGCAGAAGAACAACCGGCTGCTGGAGATCACCCGGGAGTTTAGCGTGAACGCT
GGCGTGACCACCCCAGTGAGCACCTATATGCTGACCAACAGCGAGCTGCTGAGCCTGATCAA
CGACATGCCCATCACCAACGATCAGAAGAAGCTGATGAGCAGCAACGTGCAGATCGTGAGGC
AGCAGAGCTACAGCATCATGTGCATCATCAAGGAGGAGGTGCTGGCCTACGTGGTGCAGCTG
CCTCTGTACGGAGTGATCGACACCCCCTGTTGGAAACTGCACACATCTCCTCTGTGCACAACC
AACACCAAGGAGGGCAGCAACATCTGCCTGACAAGAACCGATAGGGGCTGGTACTGTGACA
ATGCCGGCTCTGTGTCTTTCTTTCCTCAGGCCGAGACATGCAAGGTGCAGTGCAACCGGGTGT
TCTGCGACACCATGAACAGCCTGACCCTGCCCAGCGAGGTCAACCTGTGCAACATCGACATC
TTCAACCCCAAGTACGACTGCAAGATCATGACCAGCAAGACCGACGTGTCTAGCTCCGTGATC
ACAAGCCTGGGAGCCATCGTGAGCTGCTACGGCAAGACCAAGTGCACCGCCAGCAACAAGA
ATCGGGGCATCATTAAGACCTTCAGCAACGGCTGCGACTACGTGAGCAACAAGGGCGTGGAT
ACAGTGAGCGTGGGCAACACCCTGTACTACGTGAATAAGCAGGAGGGCAAGAGCCTGTACGT
GAAGGGCGAGCCCATCATCAACTTCTACGACCCCCTGGTGTTCCCCAGCTGCGAGTTTTGCGC
CAGCATCAGCCAGGTGAACGAGAAAATCAATCAGAGCCTGGCCTTCATCAGGAAGAGCGACG
AGCTCctgcacaacgtgGAGGACAAGATCGAGGAGATCCTGAGCAAGATCTACCACATCGAGAACG
AGATCGCCAGGATCAAGAAGCTGATCGGCGAGGCCATCTACCACATCGAGAACGAGATCGCC
AGGATCAAGAAGCTGATCGGCGAGGCCAACGCCGGCAAGAGCACCACCAACATCATGATCAC
CACCATCATCATCGTGATCATTGTGATCCTGCTGGCCCTGATCGCCGTGGGCCTGCTGCTGTAC
TGTAAGGCCAGGTCTACCCCTGTGACCCTGAGCAAGGATCAGCTGAGCGGCATCAATAATATC
GCCTTCTCCAAC
**The nucleotide sequence encoding 003s** (**SEQ ID NO: 191**) 
ATGGAGCTGCCTATCCTGAAGACCAATGCCATTACAACAATCCTGGCCGCCGTGACACTG
TGTTTCGCCAGCAGCCAGAACATCACCGAGGAGTTTTACCAGAGCACCTGCTCTGCCGTGAG
CAAGGGCTACCTGGGCGCCCTGAGAACAGGCTGGTACACCAGCGTGATCACCATCGAGCTGA
GCAACATCAAGGAGAACAAGTGCAATGGCACCGACGCTAAGGTGAAGCTGATCAAACAGGA
GCTGGACAAGTACAAGAACGCTGTGACCGATCTGCAGCTGCTGATGCAGTCAACCCCTTGTG
GAGGATCTGGCGGCGGAAGCGGATCTGCCATTGCTTCTGGCATCGCCGTGTGTAAAGTGCTGC
ACCTGGAGGGAGAGGTGAACAAGATTAAGAGCGCCCTGCTGAGCACCAACAAGGCCGTGGT
GTCTCTGAGCAACGGCGTGAGCGTCCTGACCTTCAAGGTGCTGGACCTGAAGAATTACATCG
ACAAACAGCTGCTGCCCATCGTGAACAAGCAGAGCTGCAGCATCCCCAACATCGAAACCGTG
ATCGAGTTTCAGCAGAAGAACAACCGGCTGCTGGAGATCACCCGGGAGTTTAGCGTGAACGC
CGGCGTGACCACACCCGTGAGCACCTACATGCTGACCAACAGCGAGCTGCTGAGCCTGATCA
ACGACATGCCTATCACCAACGATCAGAAGAAGCTGATGAGCAGCAACGTGCAGATCGTGCGG
 
CAGCAGAGCTATAGCATCATGTGCATCATCAAGGAAGAGGTGCTGGCCTACGTGGTGCAGCTG
CCCCTGTACGGCGTGATTGACACACCTTGCTGGAAGCTGCACACAAGCCCCCTGTGCACAAC
CAACACCAAAGAGGGAAGCAATATCTGCCTGACCAGGACAGACAGGGGATGGTACTGTGATA
ACGCCGGCAGCGTGTCTTTCTTCCCTCAGGCCGAAACCTGCAAGGTGCAGTGCAACAGGGTG
TTCTGCGACACCATGAACAGCCTGACCCTGCCCAGCGAGGTGAACCTGTGCAACATCGACAT
CTTCAACCCCAAGTACGACTGCAAGATCATGACCAGCAAGACCGATGTGAGCTCCAGCGTCAT
CACCTCTCTGGGCGCCATCGTGAGCTGCTATGGCAAGACCAAGTGCACCGCCAGCAACAAGA
ACAGGGGGATCATCAAGACCTTCAGCAACGGCTGCGACTACGTGAGCAACAAAGGCGTGGA
CACCGTGAGCGTGGGCAATACCCTGTACTACGTGAATAAGCAGGAGGGCAAGAGCCTGTATGT
GAAGGGCGAGCCCATCATCAACTTCTACGACCCCCTGGTGTTCCCCAGCTGCGAGTTTTGCGC
CAGCATCAGCCAGGTGAATGAGAAGATCAACCAGAGCCTGGCCTTTATCAGGAAGAGCGATG
AGCTGCTC
**The nucleotide sequence encoding 006m** (**SEQ ID NO: 192**) 
ATGGAGCTGCCCATCCTGAAGACCAACGCCATCACCACAATTCTGGCTGCCGTGACCCTG
TGCTTCGCCAGCTCTCAGAATATCACCGAGGAGTTTTACCAGAGCACCTGCTCTGCCGTGTCT
AAGGGCTACCTGGGAGCCCTGAGGACCGGCTGGTACACCAGCGTGATCACCATCGAGCTGTC
TAACATTAAGGAGAACAAGTGCAACGGCACCGACGCCAAGGTGAAGCTGATCAAGCAGGAG
CTGGACAAGTACAAGAATGCCGTGACAGATCTGCAGCTGCTGATGCAGAGCACACCTTGTGG
AGGCTCTGGAGGCGGATCTGGAAGCGCCATCGCTTCTGGAATTGCCGTGTGTAAGGTGCTGCA
CCTGGAAGGCGAGGTGAACAAAATCAAAAGCGCCCTGCTGAGCACCAACAAGGCCGTGGTG
AGCCTGAGCAACGGCGTGAGCGTGCTGACCTTCAAGGTGCTGGACCTGAAAAACTACATCGA
CAAACAGCTGCTGCCCATTGTGAACAAGCAGAGCTGCAGCATCCCCAACATCGAGACAGTGA
TCGAGTTTCAGCAGAAGAACAACCGGCTGCTGGAGATCACCAGAGAGTTTAGCGTGAACGCC
GGCGTGACCACCCCCGTGAGCACCTACATGCTGACCAACAGCGAGCTGCTGAGCCTGATCAA
CGACATGCCCATCACCAACGATCAGAAGAAGCTGATGAGCAGCAACGTGCAGATCGTGAGGC
AGCAGAGCTACAGCATCATGTGCATCATCAAGGAAGAGGTGCTGGCCTATGTGGTGCAGCTGC
CTCTGTACGGCGTGATCGATACCCCTTGCTGGAAGCTGCACACAAGCCCCCTGTGTACAACAA
ACACCAAGGAGGGCAGCAATATCTGCCTGACAAGGACCGATAGGGGCTGGTACTGCGACAAT
GCCGGCTCTGTGTCTTTCTTCCCCCAGGCCGAAACCTGCAAGGTGCAGTGCAATAGGGTGTTT
TGCGACACCATGAACAGCCTGACCCTGCCCAGCGAGGTCAACCTGTGCAACATCGACATCTT
CAACCCCAAGTACGACTGCAAGATCATGACCAGCAAGACAGACGTGTCCTCCAGCGTCATCA
CAAGCCTCGGCGCCATCGTGAGCTGTTACGGCAAGACCAAGTGCACCGCCAGCAACAAGAAC
CGGGGCATTATCAAGACCTTCAGCAACGGCTGCGACTATGTGAGCAACAAGGGCGTGGACAC
CGTGTCTGTGGGCAACACCCTGTATTACGTGAATAAACAGGAGGGCAAGAGCCTGTACGTGA
AGGGCGAGCCCATCATCAACTTCTACGACCCCCTGGTGTTCCCCAGCGACGAGTTTGACGCCA
GCATCAGCCAGGTGAACGAGAAGATCAATCAGAGCCTGGCTTTCATCCGGAAGAGCGACGAG
CTGCTCCACAACGTGAATGCCGGCAAGAGCACCACCAACATCATGATCACCACCATCATCATC
 
GTTATCATCGTGATCCTGCTGGCCCTGATCGCCGTGGGCCTGCTGCTGTATTGTAAGGCCCGGA
GCACACCAGTGACCCTGAGCAAGGATCAGCTGAGCGGCATCAACAACATCGCCTTCTCCAAC
**The nucleotide sequence encoding 007m** (**SEQ ID NO: 193**) 
ATGGAGCTGCCCATCCTGAAGACCAATGCCATTACCACAATCCTGGCCGCCGTGACACTG
TGTTTCGCTAGCAGCCAGAACATCACCGAGGAGTTTTATCAGAGCACCTGCTCTGCCGTGTCT
AAGGGCTACCTGGGCGCCCTGAGGACCGGCTGGTACACCAGCGTGATCACCATCGAGCTGAG
CAATATCAAGGAGAACAAGTGCAACGGCACAGATGCCAAGGTGAAGCTGATCAAGCAGGAG
CTGGACAAGTACAAGAACGCCGTGACTGATCTGCAGCTGCTGATGCAGAGCACACCTTGTGG
AGGATCTGGAGGTTCTGGCAGCGCTATTGCCAGCGGCATCGCTGTGTGCAAGGTGCTGCACCT
GGAAGGCGAAGTGAACAAGATCAAGAGTGCTCTGCTGAGCACCAACAAGGCTGTGGTGAGC
CTGAGCAACGGCGTGAGCGTGCTGACCTTCAAGGTCCTGGACCTGAAGAATTACATCGACAA
ACAGCTGCTCCCCATCGTGAACAAGCAGAGCTGCAGCATCCCCAACATCGAGACAGTGATCG
AGTTTCAGCAGAAGAACAACCGGCTGCTGGAGATCACCAGGGAGTTTAGCGTGAACGCCGGC
GTGACCACCCCCGTGAGCACCTACATGCTGACCAACAGCGAGCTGCTCAGCCTGATCAACGA
CATGCCTATCACCAACGATCAGAAGAAGCTGATGAGCAGCAACGTGCAGATCGTGAGGCAGC
AGAGCTACAGCATCATGTGTATCATCAAGGAGGAGGTGCTGGCCTACGTGGTGCAGCTGCCTC
TGTACGGCGTGATCGACACCCCATGTTGGAAGCTGCACACAAGCCCCCTGTGTACCACCAAC
ACCAAAGAGGGCTCTAACATCTGTCTGACCAGGACAGATAGGGGATGGTACTGCGACAATGC
CGGAAGCGTGAGCTTTTTCCCTCAGGCCGAGACATGCAAGGTCCAGTGCAACAGGGTGTTCT
GCGACACCATGAACAGCCTGACCCTGCCAAGCGAGGTGAACCTGTGCAACATCGACATCTTC
AACCCCAAGTACGACTGCAAGATCATGACCAGCAAGACCGACGTGAGCTCTAGCGTCATCAC
CAGCCTGGGCGCTATCGTGTCTTGCTACGGCAAGACCAAATGCACCGCCAGCAACAAGAACC
GGGGCATCATCAAGACCTTCAGCAACGGCTGCGACTACGTGAGCAACAAGGGCGTGGACACC
GTTAGCGTGGGCAATACACTGTACTACGTGAATAAGCAGGAAGGCAAGAGCCTGTACGTGAA
GGGCGAGCCCATCATCAACTTCTACGACCCCCTGGTGTTCCCCAGCGACGAGTTTGACGCCAG
CATCAGCCAGGTGAACGAGAAGATCAATCAGAGCCTGGCCTTCATCCGGAAGAGCGACGAGC
TGCTGCACAACGTCAACGCCGGCAAGAGCACAACCAACATCATGATCACCACCATTATCATCG
TCATCATCGTGATCCTGCTGGCCCTGATCGCCGTGGGCCTGCTGCTGTACTGCAAAGCCAGAA
GCACCCCTGTGACTCTGAGCAAGGACCAGCTGAGCGGCATCAATAACATCGCCTTCTCCAAC
**The nucleotide sequence encoding 007m-Cys-Δcys** (**SEQ ID NO: 194**) 
atggagctgcccatcctgaagaccaatgccattaccacaatcctggccgccgtgacactgtgtttcgctagcagccagaacatcaccgaggagttt
tatcagagcacctgctctgccgtgtctaagggctacctgggcgccctgaggaccggctggtacaccagcgtgatcaccatcgagctgagcaatatcaag
gagaacaagtgcaacggcacagatgccaaggtgaagctgatcaagcaggagctggacaagtacaagaacgccgtgactgatctgcagctgctgatgc
agagcacacctgccggaggatctggaggttctggcagcgctattgccagcggcatcgctgtgtgcaaggtgctgcacctggaaggcgaagtgaacaag
atcaagagtgctctgctgagcaccaacaaggctgtggtgagcctgagcaacggcgtgagcgtgctgaccttcaaggtcctggacctgaagaattacatc
gacaaacagctgctccccatcgtgaacaagcagagctgcagcatccccaacatcgagacagtgatcgagtttcagcagaagaacaaccggctgctgga
 
gatcaccagggagtttagcgtgaacgccggcgtgaccaccccgtgagcacctacatgctgaccaacagcgagctgctcagcctgatcaacgacatgc
ctatcaccaacgatcagaagaagctgatgagcagcaacgtgcagatcgtgaggcagcagagctacagcatcatgtgtatcatcaaggaggaggtgctg
gcctacgtggtgcagctgcctctgtacggcgtgatcgacaccccatgttggaagctgcacacaagcccctgtgtaccaccaacaccaaagagggctct
aacatctgtctgaccaggacagataggggatggtactgcgacaatgccggaagcgtgagctttttccctcaggccgagacatgcaaggtccagagcaac
agggtgttctgcgacaccatgaacagcctgaccctgccaagcgaggtgaacctgtgcaacatcgacatcttcaaccccaagtacgactgcaagatcatg
accagcaagaccgacgtgagctctagcgtcatcaccagcctgggcgctatcgtgtcttgctacggcaagaccaaatgcaccgccagcaacaagaaccg
gggcatcatcaagaccttcagcaacggctgcgactacgtgagcaacaagggcgtggacaccgttagcgtgggcaatacactgtactacgtgaataagc
aggaaggcaagagcctgtacgtgaagggcgagcccatcatcaacttctacgacccccctggtgttccccagctgcgagttttgcgccagcatcagccagg
tgaacgagaagatcaatcagagcctggccttcatccggaagagcgacgagctgctgcacaacgtcaacgccggcaagagcacaaccaacatcatgat
caccaccattatcatcgtcatcatcgtgatcctgctggccctgatcgccgtgggcctgctgctgtactgcaaagccagaagcacccctgtgactctgagca
aggaccagctgagcggcatcaataacatcgccttctccaac
**The nucleotide sequence encoding 008m** (**SEQ ID NO: 195**) 
ATGGAGCTGCCCATCCTGAAGACCAATGCCATTACCACCATTCTGGCCGCCGTGACCCTG
TGTTTTGCCTCTAGCCAGAACATCACCGAGGAATTTTACCAGAGCACCTGCAGCGCCGTGAGC
AAGGGCTACCTGGGCGCCCTGAGAACCGGCTGGTATACCTCCGTGATCACCATCGAGCTGAGC
AATATCAAGGAGAACAAGTGCAACGGCACCGATGCCAAGGTGAAGCTGATTAAGCAGGAGCT
GGACAAGTACAAGAACGCTGTGACCGATCTGCAGCTGCTGATGCAGAGCACACCTTGCGGAG
GAAGCGGATCTGGATCTGCCATTGCCAGCGGCATCGCCGTGTGTAAGGTGCTGCACCTGGAAG
GCGAGGTGAACAAGATTAAGTCAGCCCTGCTGTCTACCAACAAGGCCGTGGTGTCTCTGAGC
AACGGCGTGAGCGTCCTGACCTTCAAGGTGCTGGACCTGAAGAATTACATCGACAAGCAGCT
GCTCCCCATCGTCAACAAGCAGAGCTGCAGCATCCCCAACATCGAGACAGTGATCGAGTTTC
AGCAGAAGAACAACCGGCTGCTGGAGATCACCAGGGAGTTTAGCGTGAACGCCGGCGTGAC
CACCCCCGTGAGCACCTACATGCTGACCAACAGCGAGCTGCTCAGCCTGATCAACGACATGC
CTATCACCAACGACCAGAAGAAGCTGATGAGCTCTAACGTGCAGATCGTGAGGCAGCAGAGC
TACAGCATCATGTGTATCATCAAGGAGGAGGTGCTGGCCTACGTGGTGCAGCTGCCTCTGTAC
GGCGTGATTGACACCCCCTGCTGGAAGCTGCATACCAGCCCACTGTGCACCACCAATACCAAA
GAGGGCTCTAATATCTGCCTGACAAGGACCGACAGAGGCTGGTACTGTGATAACGCCGGCAG
CGTGAGCTTCTTTCCCCAGGCCGAAACCTGTAAGGTCCAGTGCAACAGGGTGTTCTGCGACA
CCATGAACAGCCTGACACTGCCAAGCGAGGTCAACCTGTGCAACATCGACATCTTCAACCCC
AAGTACGACTGCAAGATCATGACCAGCAAGACCGACGTGAGCAGCAGCGTGATCACATCTCT
GGGCGCCATCGTGAGCTGCTACGGCAAGACCAAGTGCACCGCCAGCAACAAGAACAGGGGC
ATCATCAAGACCTTCAGCAACGGCTGCGACTACGTGAGCAACAAGGGCGTGGATACCGTGAG
CGTGGGAAATACCCTGTATTACGTGAACAAGCAGGAGGGCAAGAGCCTGTACGTGAAGGGAG
AGCCCATCATCAACTTCTACGACCCCCTGGTGTTCCCCAGCGACGAGTTTGACGCCAGCATCA
GCCAGGTGAACGAGAAGATCAACCAGAGCCTGGCTTTCATCCGGAAGAGCGACGAGCTGCT
GCACAACGTCAACGCCGGAAAGAGCACAACCAACATCATGATCACCACCATCATCATCGTGAT
CATTGTGATCCTGCTGGCCCTGATCGCCGTGGGCCTGCTGCTGTACTGTAAGGCCAGGAGCAC
 CCCTGTGACCCTGAGCAAGGATCAGCTGAGCGGCATTAACAATATCGCCTTCTCCAAC
**The nucleotide sequence encoding 008m-Cys-Δcys** (**SEQ ID NO: 196**) 
atggagctgcccatcctgaagaccaatgccattaccaccattctggccgccgtgaccctgtgttttgcctctagccagaacatcaccgaggaattttta
ccagagcacctgcagcgccgtgagcaagggctacctgggcgccctgagaaccggctggtatacctccgtgatcaccatcgagctgagcaatatcaagg
agaacaagtgcaacggcaccgatgccaaggtgaagctgattaagcaggagctggacaagtacaagaacgctgtgaccgatctgcagctgctgatgca
gagcacacctgccggaggaagcggatctggatctgccattgccagcggcatcgccgtgtgtaaggtgctgcacctggaaggcgaggtgaacaagatta
agtcagccctgctgtctaccaacaaggccgtggtgtctctgagcaacggcgtgagcgtcctgaccttcaaggtgctggacctgaagaattacatcgacaa
gcagctgctccccatcgtcaacaagcagagctgcagcatccccaacatcgagacagtgatcgagtttcagcagaagaacaaccggctgctggagatca
ccagggagtttagcgtgaacgccggcgtgaccacccccgtgagcacctacatgctgaccaacagcgagctgctcagcctgatcaacgacatgcctatc
accaacgaccagaagaagctgatgagctctaacgtgcagatcgtgaggcagcagagctacagcatcatgtgtatcatcaaggaggaggtgctggccta
cgtggtgcagctgcctctgtacggcgtgattgacacccccctgctggaagctgcataccagcccactgtgcaccaccaataccaaagagggctctaatatc
tgcctgacaaggaccgacagaggctggtactgtgataacgccggcagcgtgagcttctttccccaggccgaaacctgtaaggtccagagcaacagggt
gttctgcgacaccatgaacagcctgacactgccaagcgaggtcaacctgtgcaacatcgacatcttcaaccccaagtacgactgcaagatcatgaccag
caagaccgacgtgagcagcagcgtgatcacatctctgggcgccatcgtgagctgctacggcaagaccaagtgcaccgccagcaacaagaacagggg
catcatcaagaccttcagcaacggctgcgactacgtgagcaacaagggcgtggataccgtgagcgtgggaaataccctgtattacgtgaacaagcagg
agggcaagagcctgtacgtgaagggagagcccatcatcaacttctacgacccccctggtgttccccagctgcgagttttgcgccagcatcagccaggtga
acgagaagatcaaccagagcctggctttcatccggaagagcgacgagctgctgcacaacgtcaacgccggaaagagcacaaccaacatcatgatcac
caccatcatcatcgtgatcattgtgatcctgctggccctgatcgccgtgggcctgctgctgtactgtaaggccaggagcacccctgtgaccctgagcaagg
atcagctgagcggcattaacaatatcgccttctccaac
**The nucleotide sequence encoding 009m** (**SEQ ID NO: 197**) 
ATGGAGCTGCCTATCCTGAAGACCAACGCCATTACCACCATTCTGGCCGCTGTGACACTG
TGCTTTGCCTCTAGCCAGAACATCACAGAGGAGTTTTATCAGAGCACCTGCAGCGCCGTGAGC
AAGGGCTACCTGGGCGCCCTGAGAACCGGCTGGTACACCAGCGTGATCACCATCGAGCTGTC
CAACATCAAGGAAAACAAGTGCAACGGCACCGACGCCAAGGTGAAGCTGATCAAGCAGGAG
CTGGATAAATATAAGAACGCCGTGACCGATCTGCAGCTGCTGATGCAGAGTACACCTTGTGGC
TCTGGCAGTGGCAGCGCCATCGCCTCTGGCATCGCTGTGTGTAAGGTGCTCCACCTGGAGGGC
GAAGTGAATAAGATCAAAAGCGCCCTGCTGTCTACCAACAAGGCCGTGGTGAGCCTGTCTAA
CGGCGTGAGCGTGCTGACCTTCAAGGTGCTGGACCTGAAAAACTACATCGACAAACAGCTGC
TGCCCATCGTGAACAAGCAGAGCTGCAGCATCCCCAACATCGAGACAGTGATCGAGTTTCAG
CAGAAGAACAACCGGCTGCTGGAGATCACCCGGGAGTTTAGCGTGAACGCCGGCGTGACCA
CCCCTGTGAGCACCTACATGCTGACCAACAGCGAGCTGCTGAGCCTGATCAACGACATGCCTA
TCACCAACGATCAGAAGAAGCTGATGAGCAGCAACGTGCAGATCGTGCGGCAGCAGAGCTAC
AGCATTATGTGCATCATTAAGGAGGAGGTGCTGGCCTACGTGGTGCAGCTGCCCCTGTACGGC
GTGATCGACACACCTTGCTGGAAGCTGCACACAAGCCCACTGTGCACCACAAACACAAAAG
AGGGCAGCAACATCTGTCTGACACGGACAGACCGGGGCTGGTATTGTGATAATGCCGGCAGC
GTGAGCTTCTTTCCCCAGGCTGAAACCTGCAAGGTGCAGTGCAATCGGGTGTTCTGCGACAC
 
CATGAATAGCCTGACCCTGCCCAGCGAGGTGAACCTGTGCAACATCGACATCTTCAACCCCAA
GTACGACTGCAAGATCATGACCAGCAAGACCGACGTGAGCAGCTCTGTGATTACCAGCCTGG
GCGCGATCGTGTCTTGCTACGGCAAGACCAAGTGCACCGCCAGCAACAAGAACAGGGGCATC
ATCAAGACATTCAGCAACGGCTGCGACTACGTGAGCAATAAGGGCGTGGACACCGTGAGCGT
CGGCAACACACTGTACTACGTGAACAAACAGGAGGGCAAAAGCCTGTACGTGAAGGGCGAG
CCCATCATCAACTTCTACGACCCCCTGGTGTTCCCCAGCGACGAGTTTGACGCCAGCATCAGC
CAGGTGAACGAGAAGATCAACCAGAGCCTGGCCTTTATCAGGAAGAGCGACGAGCTCCTGCA
CAACGTCAACGCCGGCAAGAGCACCACCAACATCATGATCACCACCATCATCATTGTGATCAT
CGTGATCCTGCTGGCCCTGATTGCCGTGGGCCTGCTGCTGTACTGCAAGGCCAGAAGCACACC
CGTGACACTGAGCAAGGATCAGCTGAGCGGCATCAACAACATCGCCTTCAGCAAC
**The nucleotide sequence encoding 009m-Cys-Δcys** (**SEQ ID NO: 198**) 
atggagctgcctatcctgaagaccaacgccattaccaccattctggccgctgtgacactgtgctttgcctctagccagaacatcacagaggagtttt
atcagagcacctgcagcgccgtgagcaagggctacctgggcgccctgagaaccggctggtacaccagcgtgatcaccatcgagctgtccaacatcaa
ggaaaacaagtgcaacggcaccgacgccaaggtgaagctgatcaagcaggagctggataaatataagaacgccgtgaccgatctgcagctgctgatg
cagagtacacctgccggctctggcagtggcagcgccatcgcctctggcatcgctgtgtgtaaggtgctccacctggagggcgaagtgaataagatcaaa
agcgccctgctgtctaccaacaaggccgtggtgagcctgtctaacggcgtgagcgtgctgaccttcaaggtgctggacctgaaaaactacatcgacaaa
cagctgctgcccatcgtgaacaagcagagctgcagcatccccaacatcgagacagtgatcgagtttcagcagaagaacaaccggctgctggagatcac
ccgggagtttagcgtgaacgccggcgtgaccacccctgtgagcacctacatgctgaccaacagcgagctgctgagcctgatcaacgacatgcctatcac
caacgatcagaagaagctgatgagcagcaacgtgcagatcgtgcggcagcagagctacagcattatgtgcatcattaaggaggaggtgctggcctacg
tggtgcagctgcccctgtacggcgtgatcgacacaccttgctggaagctgcacacaagcccactgtgcaccacaaacacaaaagagggcagcaacatc
tgtctgacacggacagaccggggctggtattgtgataatgccggcagcgtgagcttctttccccaggctgaaacctgcaaggtgcagagcaatcgggtgt
tctgcgacaccatgaatagcctgaccctgcccagcgaggtgaacctgtgcaacatcgacatcttcaaccccaagtacgactgcaagatcatgaccagca
agaccgacgtgagcagctctgtgattaccagcctgggcgcgatcgtgtcttgctacggcaagaccaagtgcaccgccagcaacaagaacaggggcatc
atcaagacattcagcaacggctgcgactacgtgagcaataagggcgtggacaccgtgagcgtcggcaacacactgtactacgtgaacaaacaggagg
gcaaaagcctgtacgtgaagggcgagcccatcatcaacttctacgacccccctggtgttccccagctgcgagttttgcgccagcatcagccaggtgaacg
agaagatcaaccagagcctggcctttatcaggaagagcgacgagctcctgcacaacgtcaacgccggcaagagcaccaccaacatcatgatcaccac
catcatcattgtgatcatcgtgatcctgctggccctgattgccgtgggcctgctgctgtactgcaaggccagaagcacacccgtgacactgagcaaggatc
agctgagcggcatcaacaacatcgccttcagcaac
**The nucleotide sequence encoding 010m** (**SEQ ID NO: 199**) 
ATGGAGCTGCCCATCCTGAAGACCAACGCCATCACAACAATCCTGGCCGCCGTGACCCTG
TGCTTCGCCTCTAGCCAGAACATTACCGAGGAGTTTTATCAGAGCACATGCAGCGCCGTGAGC
AAGGGCTACCTGGGCGCCCTGAGGACCGGCTGGTACACAAGCGTGATTACAATCGAGCTGAG
CAACATTAAGGAGAACAAGTGCAACGGCACCGACGCCAAGGTGAAGCTGATCAAGCAGGAG
CTGGACAAGTACAAGAATGCCGTGACCGATCTGCAGCTGCTGATGCAGAGCACACCTTGTGG
AGGATCTGGCAGCGCCATCGCTTCTGGCATCGCTGTGTGTAAGGTGCTGCACCTGGAGGGAG
AGGTGAATAAGATCAAGAGCGCCCTGCTGAGCACAAACAAAGCCGTGGTGAGCCTGAGCAA
 
CGGAGTGAGCGTGCTGACCTTCAAGGTGCTGGACCTGAAAAACTACATCGACAAGCAGCTGC
TCCCCATCGTGAACAAGCAGAGCTGCAGCATCCCCAACATCGAAACCGTGATCGAGTTTCAG
CAGAAGAACAACAGGCTGCTGGAGATCACCCGGGAGTTTAGCGTCAACGCCGGCGTGACCA
CCCCTGTGTCCACCTACATGCTGACCAACAGCGAGCTGCTGAGCCTGATCAACGACATGCCCA
TCACCAACGATCAGAAGAAGCTGATGTCTAGCAACGTGCAGATCGTGCGGCAGCAGAGCTAC
AGCATCATGTGCATCATCAAGGAGGAGGTGCTGGCCTATGTGGTGCAGCTGCCTCTGTACGGC
GTGATCGACACCCCCTGCTGGAAGTTGCATACAAGCCCCCTGTGCACCACCAATACCAAGGA
AGGCAGCAATATCTGCCTGACAAGAACCGATAGAGGCTGGTATTGCGACAATGCCGGCAGCGT
GAGCTTCTTCCCTCAGGCTGAAACATGCAAGGTGCAGTGCAACCGGGTGTTCTGCGACACCA
TGAACAGCCTGACCCTGCCCAGCGAGGTGAACCTGTGCAACATCGACATCTTCAATCCCAAG
TACGACTGCAAGATCATGACCAGCAAGACCGACGTGAGCAGCTCTGTGATCACCAGCCTGGG
AGCCATCGTGAGCTGCTACGGCAAGACCAAGTGCACCGCCAGCAATAAGAACCGGGGATCA
TTAAGACCTTCAGCAACGGCTGCGACTATGTGAGCAACAAGGGCGTGGACACCGTGAGCGTG
GGCAACACACTGTACTACGTGAACAAACAGGAGGGAAAGAGCCTGTACGTGAAGGGCGAGC
CCATCATCAACTTCTACGACCCCCTGGTGTTCCCCAGCGACGAGTTTGACGCCAGCATCAGCC
AGGTGAACGAGAAGATCAATCAGAGCCTGGCCTTCATCAGGAAGAGCGATGAGCTGCTGCAC
AACGTGAACGCCGGCAAGAGCACGACCAACATCATGATCACCACCATCATTATCGTGATCATC
GTGATCCTGCTGGCCCTGATTGCCGTGGGCCTGCTGCTGTACTGCAAGGCCAGGTCTACCCCT
GTGACCCTGAGCAAGGACCAGCTGTCTGGCATCAACAACATCGCCTTCTCTAAT
**The nucleotide sequence encoding 010m-Cys-Δcys** (**SEQ ID NO: 200**)

atggagctgcccatcctgaagaccaacgccatcacaacaatcctggccgccgtgaccctgtgcttcgcctctagccagaacattaccgaggagttt
tatcagagcacatgcagcgccgtgagcaagggctacctgggcgccctgaggaccggctggtacacaagcgtgattacaatcgagctgagcaacattaa
ggagaacaagtgcaacggcaccgacgccaaggtgaagctgatcaagcaggagctggacaagtacaagaatgccgtgaccgatctgcagctgctgatg
cagagcacacctgccggaggatctggcagcgccatcgcttctggcatcgctgtgtgtaaggtgctgcacctggagggagaggtgaataagatcaagag
cgccctgctgagcacaaacaaagccgtggtgagcctgagcaacggagtgagcgtgctgaccttcaaggtgctggacctgaaaaactacatcgacaagc
agctgctccccatcgtgaacaagcagagctgcagcatccccaacatcgaaaccgtgatcgagtttcagcagaagaacaacaggctgctggagatcacc
cgggagtttagcgtcaacgccggcgtgaccacccctgtgtccacctacatgctgaccaacagcgagctgctgagcctgatcaacgacatgcccatcacc
aacgatcagaagaagctgatgtctagcaacgtgcagatcgtgcggcagcagagctacagcatcatgtgcatcatcaaggaggaggtgctggcctatgtg
gtgcagctgcctctgtacggcgtgatcgacacccccctgctggaagttgcatacaagcccccctgtgcaccaccaataccaaggaaggcagcaatatctgc
ctgacaagaaccgatagaggctggtattgcgacaatgccggcagcgtgagcttcttccctcaggctgaaacatgcaaggtgcagagcaaccgggtgttc
tgcgacaccatgaacagcctgaccctgcccagcgaggtgaacctgtgcaacatcgacatcttcaatcccaagtacgactgcaagatcatgaccagcaag
accgacgtgagcagctctgtgatcaccagcctgggagccatcgtgagctgctacggcaagaccaagtgcaccgccagcaataagaaccgggggatca
ttaagaccttcagcaacggctgcgactatgtgagcaacaagggcgtggacaccgtgagcgtgggcaacacactgtactacgtgaacaaacaggaggg
aaagagcctgtacgtgaagggcgagcccatcatcaacttctacgacccccctggtgttccccagctgcgagttttgcgccagcatcagccaggtgaacga
gaagatcaatcagagcctggccttcatcaggaagagcgatgagctgctgcacaacgtgaacgccggcaagagcacgaccaacatcatgatcaccacc
atcattatcgtgatcatcgtgatcctgctggccctgattgccgtgggcctgctgctgtactgcaaggccaggtctacccctgtgaccctgagcaaggacca
gctgtctggcatcaacaacatcgccttctctaat

### The amino acid sequence

**The amino acid sequence of mA2F** (**SEQ ID NO: 161**) 
MELLILKANAITTILTAVTFCFASGQNITEEFYQSTCSAVSKGYLSALRTGWYTSVITIELSNIKE
NKCNGTDAKVKLIKQELDKYKNAVTELQLLMQSTPATNNRARRELPRFMNYTLNNAKKTNVTL
SKKRKRRFLGFLLGVGSAIASGVAVSKVLHLEGEVNKIKSALLSTNKAVVSLSNGVSVLTSKVLDL
KNYIDKQLLPIVNKQSCSISNIETVIEFQQKNNRLLEITREFSVNAGVTTPVSTYMLTNSELLSLIND
MPITNDQKKLMSNNVQIVRQQSYSIMSIIKEEVLAYVVQLPLYGVIDTPCWKLHTSPLCTTNTKEG
SNICLTRTDRGWYCDNAGSVSFFPQAETCKVQSNRVFCDTMNSLTLPSEVNLCNVDIFNPKYDCK
IMTSKTDVSSSVITSLGAIVSCYGKTKCTASNKNRGIIKTFSNGCDYVSNKGVDTVSVGNTLYYVN
KQEGKSLYVKGEPIINFYDPLVFPSDEFDASISQVNEKINQSLAFIRKSDELLHNVNAGKSTTNIMIT
TIIIVIIVILLSLIAVGLLLYCKARSTPVTLSKDQLSGINNIAFSN
**The amino acid sequence of m5K6I** (**SEQ ID NO: 162**) 
MELLILKANAITTILTAVTFCFASGQNITEEFYQSTCSAVSKGYLSALRTGWYTSVITIELSNIKE
NKCNGTDAKVKLIKQELDKYKNAVTELQLLMQSTPATNNRARRELPRFMNYTLNNAKKTNVTL
SKKRKRRFLGFLLGVGSAIASGVAVSKVLHLEGEVNKIKSALLSTNKAVVSLSNGVSVLTSKVLDL
KNYIDKQLLPIVNKQSCSISNIETVIEFQQKNNRLLEITREFSVNAGVTTPVSTYMLTNSELLSLIND
MPITNDQKKLMSNNVQIVRQQSYSIMSIIKEEVLAYVVQLPLYGVIDTPCWKLHTSPLCTTNTKEG
SNICLTRTDRGWYCDNAGSVSFFPQAETCKVQSNRVFCDTMNSLTLPSEVNLCNVDIFNPKYDCK
IMTSKTDVSSSVITSLGAIVSCYGKTKCTASNKNRGIIKTFSNGCDYVSNKGVDTVSVGNTLYYVN
KQEGKSLYVKGEPIINFYDPLVFPSDEFDASISQVNEKINQSLAFIRKSDELLHNVNAGKSTTNIMIT
TIIIVIIVILLSLIAVGLLLYCKARSTPVTLSKDQLSGINNIAFSN
**The amino acid sequence of DS-Cav1-1xGCN4t** (**SEQ ID NO: 163**) 
MELLILKANAITTILTAVTFCFASGQNITEEFYQSTCSAVSKGYLSALRTGWYTSVITIELSNIKE
NKCNGTDAKVKLIKQELDKYKNAVTELQLLMQSTPATNNRARRELPRFMNYTLNNAKKTNVTL
SKKRKRRFLGFLLGVGSAIASGVAVCKVLHLEGEVNKIKSALLSTNKAVVSLSNGVSVLTFKVLD
LKNYIDKQLLPILNKQSCSISNIETVIEFQQKNNRLLEITREFSVNAGVTTPVSTYMLTNSELLSLIN
DMPITNDQKKLMSNNVQIVRQQSYSIMCIIKEEVLAYVVQLPLYGVIDTPCWKLHTSPLCTTNTKE
GSNICLTRTDRGWYCDNAGSVSFFPQAETCKVQSNRVFCDTMNSLTLPSEVNLCNVDIFNPKYDC
KIMTSKTDVSSSVITSLGAIVSCYGKTKCTASNKNRGIIKTFSNGCDYVSNKGVDTVSVGNTLYYV
NKQEGKSLYVKGEPIINFYDPLVFPSDEFDASISQVNEKINQSLAFIRKSDELLHNVEDKIEEILSKIY
HIENEIARIKKLIGEANAGKSTTNIMITTIIIVIIVILLSLIAVGLLLYCKARSTPVTLSKDQLSGINNIA
FSN
**The amino acid sequence of DS-Cav1-2xGCN4t** (**SEQ ID NO: 164**) MELLILKANAITTILTAVTFCFASGQNITEEFYQSTCSAVSKGYLSALRTGWYTSVITIELSNIKE 
NKCNGTDAKVKLIKQELDKYKNAVTELQLLMQSTPATNNRARRELPRFMNYTLNNAKKTNVTL
SKKRKRRFLGFLLGVGSAIASGVAVCKVLHLEGEVNKIKSALLSTNKAVVSLSNGVSVLTFKVLD
LKNYIDKQLLPILNKQSCSISNIETVIEFQQKNNRLLEITREFSVNAGVTTPVSTYMLTNSELLSLIN
DMPITNDQKKLMSNNVQIVRQQSYSIMCIIKEEVLAYVVQLPLYGVIDTPCWKLHTSPLCTTNTKE
GSNICLTRTDRGWYCDNAGSVSFFPQAETCKVQSNRVFCDTMNSLTLPSEVNLCNVDIFNPKYDC
KIMTSKTDVSSSVITSLGAIVSCYGKTKCTASNKNRGIIKTFSNGCDYVSNKGVDTVSVGNTLYYV
NKQEGKSLYVKGEPIINFYDPLVFPSDEFDASISQVNEKINQSLAFIRKSDELLHNVEDKIEEILSKIY
HIENEIARIKKLIGEAIYHIENEIARIKKLIGEANAGKSTTNIMITTIIIVIIVILLSLIAVGLLLYCKARS
TPVTLSKDQLSGINNIAFSN
**The amino acid sequence of Moderna-ΔCT** (**SEQ ID NO: 165**) 
MELLILKANAITTILTAVTFCFASGQNITEEFYQSTCSAVSKGYLSALRTGWYTSVITIELSNIKE
NKCNGTDAKVKLIKQELDKYKNAVTELQLLMQSTPATGSGSAICSGVAVCKVLHLEGEVNKIKSA
LLSTNKAVVSLSNGVSVLTFKVLDLKNYIDKQLLPILNKQSCSISNIETVIEFQQKNNRLLEITREFS
VNAGVTTPVSTYMLTNSELLSLINDMPITNDQKKLMSNNVQIVRQQSYSIMCIIKEEVLAYVVQLP
LYGVIDTPCWKLHTSPLCTTNTKEGSNICLTRTDRGWYCDNAGSVSFFPQAETCKVQSNRVFCDT
MNSRTLPSEVNLCNVDIFNPKYDCKIMTSKTDVSSSVITSLGAIVSCYGKTKCTASNKNRGIIKTFS
NGCDYVSNKGVDTVSVGNTLYCVNKQEGKSLYVKGEPIINFYDPLVFPSDEFDASISQVNEKINQS
LAFIRKSDELLHNVNAGKSTTNIMITTIIIVIIVILLSLIAVGLLLYC
**The amino acid sequence of 003m-2xGCN4t** (**SEQ ID NO: 166**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPCGGSGGGSGSAIASGIAVCKVLHLEGEV
NKIKSALLSTNKAVVSLSNGVSVLTFKVLDLKNYIDKQLLPIVNKQSCSIPNIETVIEFQQKNNRLL
EITREFSVNAGVTTPVSTYMLTNSELLSLINDMPITNDQKKLMSSNVQIVRQQSYSIMCIIKEEVLA
YVVQLPLYGVIDTPCWKLHTSPLCTTNTKEGSNICLTRTDRGWYCDNAGSVSFFPQAETCKVQCN
RVFCDTMNSLTLPSEVNLCNIDIFNPKYDCKIMTSKTDVSSSVITSLGAIVSCYGKTKCTASNKNRG
IIKTFSNGCDYVSNKGVDTVSVGNTLYYVNKQEGKSLYVKGEPIINFYDPLVFPSCEFCASISQVNE
KINQSLAFIRKSDELLHNVEDKIEEILSKIYHIENEIARIKKLIGEAIYHIENEIARIKKLIGEANAGKS
TTNIMITTIIIVIIVILLALIAVGLLLYCKARSTPVTLSKDQLSGINNIAFSN
**The amino acid sequence of 003s** (**SEQ ID NO: 167**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPCGGSGGGSGSAIASGIAVCKVLHLEGEV
NKIKSALLSTNKAVVSLSNGVSVLTFKVLDLKNYIDKQLLPIVNKQSCSIPNIETVIEFQQKNNRLL
EITREFSVNAGVTTPVSTYMLTNSELLSLINDMPITNDQKKLMSSNVQIVRQQSYSIMCIIKEEVLA
YVVQLPLYGVIDTPCWKLHTSPLCTTNTKEGSNICLTRTDRGWYCDNAGSVSFFPQAETCKVQCN
RVFCDTMNSLTLPSEVNLCNIDIFNPKYDCKIMTSKTDVSSSVITSLGAIVSCYGKTKCTASNKNRG
 
IIKTFSNGCDYVSNKGVDTVSVGNTLYYVNKQEGKSLYVKGEPIINFYDPLVFPSCEFCASISQVNE
KINQSLAFIRKSDELL
**The amino acid sequence of 006m** (**SEQ ID NO: 168**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPCGGSGGGSGSAIASGIAVCKVLHLEGEV
NKIKSALLSTNKAVVSLSNGVSVLTFKVLDLKNYIDKQLLPIVNKQSCSIPNIETVIEFQQKNNRLL
EITREFSVNAGVTTPVSTYMLTNSELLSLINDMPITNDQKKLMSSNVQIVRQQSYSIMCIIKEEVLA
YVVQLPLYGVIDTPCWKLHTSPLCTTNTKEGSNICLTRTDRGWYCDNAGSVSFFPQAETCKVQCN
RVFCDTMNSLTLPSEVNLCNIDIFNPKYDCKIMTSKTDVSSSVITSLGAIVSCYGKTKCTASNKNRG
IIKTFSNGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLYCKARSTPVTLSKDQLSGI
NNIAFSN
**The amino acid sequence of 007m** (**SEQ ID NO: 169**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPCGGSGGSGSAIASGIAVCKVLHLEGEVN
KIKSALLSTNKAVVSLSNGVSVLTFKVLDLKNYIDKQLLPIVNKQSCSIPNIETVIEFQQKNNRLLEI
TREFSVNAGVTTPVSTYMLTNSELLSLINDMPITNDQKKLMSSNVQIVRQQSYSIMCIIKEEVLAY
VVQLPLYGVIDTPCWKLHTSPLCTTNTKEGSNICLTRTDRGWYCDNAGSVSFFPQAETCKVQCNR
VFCDTMNSLTLPSEVNLCNIDIFNPKYDCKIMTSKTDVSSSVITSLGAIVSCYGKTKCTASNKNRGI
IKTFSNGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLYCKARSTPVTLSKDQLSGI
NNIAFSN
**The amino acid sequence of 007m-Cys-Δcys** (**SEQ ID NO: 170**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPAGGSGGSGSAIASGIAVCKVLHLEGEVN
KIKSALLSTNKAVVSLSNGVSVLTFKVLDLKNYIDKQLLPIVNKQSCSIPNIETVIEFQQKNNRLLEI
TREFSVNAGVTTPVSTYMLTNSELLSLINDMPITNDQKKLMSSNVQIVRQQSYSIMCIIKEEVLAY
VVQLPLYGVIDTPCWKLHTSPLCTTNTKEGSNICLTRTDRGWYCDNAGSVSFFPQAETCKVQSNR
VFCDTMNSLTLPSEVNLCNIDIFNPKYDCKIMTSKTDVSSSVITSLGAIVSCYGKTKCTASNKNRGI
IKTFSNGCDYVSNKGVDTVSVGNTLYYVNKQEGKSLYVKGEPIINFYDPLVFPSCEFCASISQVNE
KINQSLAFIRKSDELLHNVNAGKSTTNIMITTIIIVIIVILLALIAVGLLLYCKARSTPVTLSKDQLSGI
NNIAFSN
**The amino acid sequence of 008m** (**SEQ ID NO: 171**) MELPILKTNAITTILAAVTLCFASSQNITEEFYQSTCSAVSKGYLGALRTGWYTSVITIELSNIK 
ENKCNGTDAKVKLIKQELDKYKNAVTDLQLLMQSTPCGGSGSGSAIASGIAVCKVLHLEGEVNKI
KSALLSTNKAVVSLSNGVSVLTFKVLDLKNYIDKQLLPIVNKQSCSIPNIETVIEFQQKNNRLLEIT
REFSVNAGVTTPVSTYMLTNSELLSLINDMPITNDQKKLMSSNVQIVRQQSYSIMCIIKEEVLAYV
VQLPLYGVIDTPCWKLHTSPLCTTNTKEGSNICLTRTDRGWYCDNAGSVSFFPQAETCKVQCNRV
FCDTMNSLTLPSEVNLCNIDIFNPKYDCKIMTSKTDVSSSVITSLGAIVSCYGKTKCTASNKNRGII
KTFSNGCDYVSNKGVDTVSVGNTLYYVNKQEGKSLYVKGEPIINFYDPLVFPSDEFDASISQVNE
KINQSLAFIRKSDELLHNVNAGKSTTNIMITTIIIVIIVILLALIAVGLLLYCKARSTPVTLSKDQLSGI
NNIAFSN
**The amino acid sequence of 008m-Cys-Δcys** (**SEQ ID NO: 172**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPAGGSGSGSAIASGIAVCKVLHLEGEVNKI
KSALLSTNKAVVSLSNGVSVLTFKVLDLKNYIDKQLLPIVNKQSCSIPNIETVIEFQQKNNRLLEIT
REFSVNAGVTTPVSTYMLTNSELLSLINDMPITNDQKKLMSSNVQIVRQQSYSIMCIIKEEVLAYV
VQLPLYGVIDTPCWKLHTSPLCTTNTKEGSNICLTRTDRGWYCDNAGSVSFFPQAETCKVQSNRV
FCDTMNSLTLPSEVNLCNIDIFNPKYDCKIMTSKTDVSSSVITSLGAIVSCYGKTKCTASNKNRGII
KTFSNGCDYVSNKGVDTVSVGNTLYYVNKQEGKSLYVKGEPIINFYDPLVFPSCEFCASISQVNEK
INQSLAFIRKSDELLHNVNAGKSTTNIMITTIIIVIIVILLALIAVGLLLYCKARSTPVTLSKDQLSGIN
NIAFSN
**The amino acid sequence of 009m** (**SEQ ID NO: 173**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPCGSGSGSAIASGIAVCKVLHLEGEVNKIK
SALLSTNKAVVSLSNGVSVLTFKVLDLKNYIDKQLLPIVNKQSCSIPNIETVIEFQQKNNRLLEITRE
FSVNAGVTTPVSTYMLTNSELLSLINDMPITNDQKKLMSSNVQIVRQQSYSIMCIIKEEVLAYVVQ
LPLYGVIDTPCWKLHTSPLCTTNTKEGSNICLTRTDRGWYCDNAGSVSFFPQAETCKVQCNRVFC
DTMNSLTLPSEVNLCNIDIFNPKYDCKIMTSKTDVSSSVITSLGAIVSCYGKTKCTASNKNRGIIKT
FSNGCDYVSNKGVDTVSVGNTLYYVNKQEGKSLYVKGEPIINFYDPLVFPSDEFDASISQVNEKIN
QSLAFIRKSDELLHNVNAGKSTTNIMITTIIIVIIVILLALIAVGLLLYCKARSTPVTLSKDQLSGINNI
AFSN
**The amino acid sequence of 009m-Cys-Δcys** (**SEQ ID NO: 174**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPAGSGSGSAIASGIAVCKVLHLEGEVNKIK
SALLSTNKAVVSLSNGVSVLTFKVLDLKNYIDKQLLPIVNKQSCSIPNIETVIEFQQKNNRLLEITRE
FSVNAGVTTPVSTYMLTNSELLSLINDMPITNDQKKLMSSNVQIVRQQSYSIMCIIKEEVLAYVVQ
LPLYGVIDTPCWKLHTSPLCTTNTKEGSNICLTRTDRGWYCDNAGSVSFFPQAETCKVQSNRVFC
DTMNSLTLPSEVNLCNIDIFNPKYDCKIMTSKTDVSSSVITSLGAIVSCYGKTKCTASNKNRGIIKT
 
FSNGCDYVSNKGVDTVSVGNTLYYVNKQEGKSLYVKGEPIINFYDPLVFPSCEFCASISQVNEKIN
QSLAFIRKSDELLHNVNAGKSTTNIMITTIIIVIIVILLALIAVGLLLYCKARSTPVTLSKDQLSGINNI
AFSN
**The amino acid sequence of 010m** (**SEQ ID NO: 175**) 
MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPCGGSGSAIASGIAVCKVLHLEGEVNKIKS
ALLSTNKAVVSLSNGVSVLTFKVLDLKNYIDKQLLPIVNKQSCSIPNIETVIEFQQKNNRLLEITREF
SVNAGVTTPVSTYMLTNSELLSLINDMPITNDQKKLMSSNVQIVRQQSYSIMCIIKEEVLAYVVQL
PLYGVIDTPCWKLHTSPLCTTNTKEGSNICLTRTDRGWYCDNAGSVSFFPQAETCKVQCNRVFCD
TMNSLTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASISQVNEKINQS
LAFIRKSDELLHNVNAGKSTTNIMITTIIIVIIVILLALIAVGLLLYCKARSTPVTLSKDQLSGINNIAF
SN
**The amino acid sequence of OlOm-Cys-Δcys** (**SEQ ID NO: 176**)

MELPILKTNAITTILAAVTLCFASSQNITEEFYQSTCSAVSKGYLGALRTGWYTSVITIELSNIK
ENKCNGTDAKVKLIKQELDKYKNAVTDLQLLMQSTPAGGSGSAIASGIAVCKVLHLEGEVNKIKS
ALLSTNKAVVSLSNGVSVLTFKVLDLKNYIDKQLLPIVNKQSCSIPNIETVIEFQQKNNRLLEITREF
SVNAGVTTPVSTYMLTNSELLSLINDMPITNDQKKLMSSNVQIVRQQSYSIMCIIKEEVLAYVVQL
PLYGVIDTPCWKLHTSPLCTTNTKEGSNICLTRTDRGWYCDNAGSVSFFPQAETCKVQSNRVFCD
TMNSLTLPSEVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSCEFCASISQVNEKINQS
LAFIRKSDELLHNVNAGKSTTNIMITTIIIVIIVILLALIAVGLLLYCKARSTPVTLSKDQLSGINNIAF
SN

**Table 41. Sequence Information**

| **Sequence Name** | **Amino Acid Sequence** | **Nucleic Acid Sequence** |
|---|---|---|
| WT | (SEQ ID NO: 1) | - |
| 003m linker | GGSGGGS (SEQ ID NO: 2) | - |
| 007m linker | GGSGGS (SEQ ID NO: 3) | - |
| 008m linker | GGSGS (SEQ ID NO: 4) | - |
| 009m linker | GSGS (SEQ ID NO: 5) | - |
| 010m linker | GGS | - |
| 1×GCN4tInsertion Sequence | EDKIEEILSKIYHIENEIARIKK LIGEA（SEQ ID NO: 6） | - |
| STM Replacement Sequence | WPWYIWLGFIAGLIAIVMVTI MLCCMTSCCSCLKGCCSCGS CCK（SEQ ID NO: 7） | - |
| S-Modified Insertion Sequence | NEKINQISASIRKIDESISQI (SEQ ID NO: 8) | - |
| L-Modified Insertion Sequence | NEKINQISASIRKIDESINEKIN QISASIRKIDESISQI （ SEQ ID NO: 9） | - |
| mDS-Cav1 | MELLILKANAITTILTAVTFCF ASGQNITEEFYQSTCSAVSKG YLSALRTGWYTSVITIELSNIK ENKCNGTDAKVKLIKQELDK YKNAVTELQLLMQSTPATNN RARRELPRFMNYTLNNAKKT NVTLSKKRKRRFLGFLLGVG SAIASGVAVCKVLHLEGEVNK IKSALLSTNKAVVSLSNGVSV LTFKVLDLKNYIDKQLLPILN KQSCSISNIETVIEFQQKNNRL LEITREFSVNAGVTTPVSTYM LTNSELLSLINDMPITNDQKK LMSNNVQIVRQQSYSIMCIIK EEVLAYVVQLPLYGVIDTPCW KLHTSPLCTTNTKEGSNICLTR TDRGWYCDNAGSVSFFPQAE TCKVQSNRVFCDTMNSLTLPS EVNLCNVDIFNPKYDCKIMTS KTDVSSSVITSLGAIVSCYGK TKCTASNKNRGIIKTFSNGCD YVSNKGVDTVSVGNTLYYVN KQEGKSLYVKGEPIINFYDPL VFPSDEFDASISQVNEKINQSL AFIRKSDELLHNVNAGKSTTN IMITTIIIVIIVILLSLIAVGLLLY CKARSTPVTLSKDQLSGINNI AFSN （SEQ ID NO: 10） | ATGGAGCTGCTGATCCTGAAGGCCAACGCCATCACC ACAATCCTGACCGCCGTGACATTTTGCTTCGCTAGCG GCCAGAACATCACAGAGGAATTTTACCAGAGCACCT GCAGCGCTGTGAGCAAGGGCTACCTGTCTGCTCTGC GGACCGGCTGGTACACCAGCGTGATCACAATCGAGC TGAGCAACATCAAGGAGAACAAGTGCAACGGCACC GACGCCAAGGTGAAGCTGATCAAGCAGGAGCTGGA CAAGTACAAGAACGCCGTCACAGAGCTTCAGCTCCT GATGCAGAGCACCCCTGCCACCAATAACCGGGCCCG GAGAGAGCTGCCCAGGTTCATGAATTACACCCTGAA CAACGCCAAGAAGACCAACGTGACCCTGTCTAAGA AGAGAAAGCGGAGATTCCTGGGCTTCCTGCTGGGAG TGGGATCTGCTATTGCTTCTGGCGTGGCTGTGTGCAA AGTGCTGCACCTGGAGGGAGAGGTGAACAAGATCA AGAGCGCCCTCCTGAGCACCAACAAAGCCGTGGTG AGCCTGAGCAACGGAGTGAGCGTGCTGACCTTCAA GGTGCTGGACCTGAAGAATTACATCGACAAGCAGCT GCTGCCTATCCTGAACAAGCAGAGCTGCAGCATCAG CAACATCGAGACGGTGATCGAGTTTCAGCAGAAGAA CAACCGGCTGCTGGAGATCACCCGGGAGTTTAGCGT GAACGCCGGCGTGACCACACCCGTGAGCACCTACAT GCTGACCAACAGCGAGCTGCTGTCTCTGATCAACGA CATGCCCATCACCAACGATCAGAAGAAGCTGATGAG CAACAACGTGCAGATCGTGCGGCAGCAGAGCTACA GCATCATGTGCATCATCAAGGAAGAGGTGCTGGCCT ACGTGGTGCAGCTGCCCCTGTACGGCGTGATCGATA CCCCATGCTGGAAGCTGCACACCAGCCCACTGTGTA CCACCAACACCAAAGAGGGAAGCAACATTTGTCTGA CACGGACAGATAGGGGCTGGTACTGTGATAATGCCG GCAGCGTCTCCTTCTTTCCCCAGGCCGAGACATGCA AGGTGCAGAGCAACCGGGTGTTCTGCGACACCATGA ACAGCCTGACACTGCCCAGCGAGGTGAATCTGTGCA ACGTGGACATCTTCAACCCCAAGTACGACTGCAAGA TCATGACCAGCAAGACAGACGTGTCTAGCAGCGTCA TTACCAGCCTGGGCGCTATCGTGAGCTGCTACGGCA AGACCAAGTGCACAGCCAGCAACAAGAACCGGGGG ATCATCAAGACCTTCTCCAACGGCTGCGACTACGTG AGCAATAAGGGCGTGGACACCGTGAGCGTGGGCAAT ACCCTGTACTACGTGAACAAACAGGAGGGCAAGAG CCTGTACGTGAAGGGCGAGCCCATCATCAACTTCTA CGATCCTCTGGTGTTCCCCAGCGACGAGTTTGACGC CAGCATCTCCCAGGTGAACGAGAAGATCAACCAGAG CCTGGCCTTCATCAGGAAGAGCGACGAGCTCCTGCA CAACGTCAACGCCGGCAAAAGCACCACAAACATCAT GATCACCACCATCATCATCGTGATTATCGTGATCCTGC TGAGCCTGATCGCCGTGGGCCTGCTGCTGTATTGCAA GGCCAGGAGCACCCCCGTGACACTGAGCAAGGACC AGCTGTCTGGCATCAACAACATCGCCTTCAGCAAC （SEQ ID NO: 41） |
| 003m | (SEQ ID NO: 11) | (SEQ ID NO: 42) |
| 007m-Cys | (SEQ ID NO: 12) | (SEQ ID NO: 43) |
| 007m-Cys-STM | (SEQ ID NO: 13) | (SEQ ID NO: 44) |
| 007m-Cys-1×GCN4t | (SEQ ID NO: 14) | (SEQ ID NO: 45) |
| 007m-Cys-1×GCN4t-STM | (SEQ ID NO: 15) | (SEQ ID NO: 46) |
| 008m-Cys | (SEQ ID NO: 16) | (SEQ ID NO: 47) |
| 008m-Cys-STM | (SEQ ID NO: 17) | (SEQ ID NO: 48) |
| 008m-Cys-1×GCN4t | (SEQ ID NO: 18) | (SEQ ID NO: 49) |
| 008m-Cys-1×GCN4t-STM | (SEQ ID NO: 19) | (SEQ ID NO: 50) |
| 009m-Cys | (SEQ ID NO: 20) | (SEQ ID NO: 51) |
| 009m-Cys-STM | (SEQ ID NO: 21) | (SEQ ID NO: 52) |
| 009m-Cys-1×GCN4t | (SEQ ID NO: 22) | (SEQ ID NO: 53) |
| 009m-Cys-1×GCN4t-STM | (SEQ ID NO: 23) | (SEQ ID NO: 54) |
| 010m-Cys | (SEQ ID NO: 24) | (SEQ ID NO: 55) |
| 010m-Cys-STM | (SEQ ID NO: 25) | (SEQ ID NO: 56) |
| 010m-Cys-1×GCN4t | (SEQ ID NO: 26) | (SEQ ID NO: 57) |
| 010m-Cys-1×GCN4t-STM | (SEQ ID NO: 27) | (SEQ ID NO: 58) |
| 003m-P215S-1×GCN4t (ORIG) | (SEQ ID NO: 28) | (SEQ ID NO: 59) |
| 003m-P215S-2xGCN4t | (SEQ ID NO: 29) | (SEQ ID NO: 60) |
| 003M-P215S-L-3N | (SEQ ID NO: 30) | (SEQ ID NO: 61) |
| 003M-P215S-S | (SEQ ID NO: 31) | (SEQ ID NO: 62) |
| 003M-P215S-L | (SEQ ID NO: 32) | (SEQ ID NO: 63) |
| 003m-P215S-1×GCN4t (optil) | (SEQ ID NO: 28) | (SEQ ID NO: 64) |
| 003m-P215S-1×GCN4t (opti3) | ((SEQ ID NO: 28) | (SEQ ID NO: 65) |
| 003m-P215S-1×GCN4t (opti5) | (SEQ ID NO: 28) | (SEQ ID NO: 66) |
| 003m-P215S-ΔF111-1×GCN4t | (SEQ ID NO: 33) | (SEQ ID NO: 67) |
| 003m-P215S-ΔF111-1×GCN4t-opti1 | (SEQ ID NO: 33) | (SEQ ID NO: 68) |
| 003m-P215S-ΔF111-1×GCN4t-opti2 | (SEQ ID NO: 33) | (SEQ ID NO: 69) |
| 003m-P215S-ΔF111-1×GCN4t-opti4 | (SEQ ID NO: 33) | (SEQ ID NO: 70) |
| 003m-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSIPNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYHIENEIARIKKLIGE ANAGKSTTNIMITTIIIVIIVILL ALIAVGLLLYCKARSTPVTLS KDQLSGINNIAFSN （SEQ ID NO: 34） | ATGGAGCTGCCCATCCTGAAGACCAATGCCATTACA ACCATCCTGGCCGCCGTGACACTGTGTTTCGCCAGC TCTCAGAACATCACCGAGGAATTTTACCAGAGCACC TGCAGCGCCGTGAGCAAGGGCTACCTGGGCGCCCTG AGGACCGGCTGGTACACAAGCGTGATCACCATCGAG CTGAGCAACATCAAAGAAAACAAGTGCAACGGCAC AGACGCCAAAGTGAAGCTGATCAAGCAGGAGCTGG ACAAGTACAAGAACGCCGTGACTGACCTGCAGCTGC TGATGCAGTCAACACCTTGTGGCGGATCTGGCGGAG GATCTGGCAGCGCTATCGCCTCTGGAATTGCCGTGTG TAAAGTGCTGCACCTGGAGGGCGAAGTGAACAAGA TCAAAAGCGCCCTGCTGAGCACCAACAAGGCTGTG GTGAGCCTGAGCAATGGCGTGAGCGTGCTGACCTTC AAGGTGCTGGACCTGAAAAACTACATTGACAAGCAG CTGCTCCCCATCGTGAACAAGCAGAGCTGCAGCATC CCCAACATCGAGACGGTGATCGAGTTTCAGCAGAAG AACAACCGGCTGCTGGAGATCACCCGGGAGTTTAGC GTGAACGCTGGCGTGACCACCCCAGTGAGCACCTAT ATGCTGACCAACAGCGAGCTGCTGAGCCTGATCAAC GACATGCCCATCACCAACGATCAGAAGAAGCTGATG AGCAGCAACGTGCAGATCGTGAGGCAGCAGAGCTA CAGCATCATGTGCATCATCAAGGAGGAGGTGCTGGC CTACGTGGTGCAGCTGCCTCTGTACGGAGTGATCGA CACCCCCTGTTGGAAACTGCACACATCTCCTCTGTGC ACAACCAACACCAAGGAGGGCAGCAACATCTGCCT GACAAGAACCGATAGGGGCTGGTACTGTGACAATGC CGGCTCTGTGTCTTTCTTTCCTCAGGCCGAGACATGC AAGGTGCAGTGCAACCGGGTGTTCTGCGACACCATG AACAGCCTGACCCTGCCCAGCGAGGTCAACCTGTGC AACATCGACATCTTCAACCCCAAGTACGACTGCAAG ATCATGACCAGCAAGACCGACGTGTCTAGCTCCGTG ATCACAAGCCTGGGAGCCATCGTGAGCTGCTACGGC AAGACCAAGTGCACCGCCAGCAACAAGAATCGGGG CATCATTAAGACCTTCAGCAACGGCTGCGACTACGT GAGCAACAAGGGCGTGGATACAGTGAGCGTGGGCA ACACCCTGTACTACGTGAATAAGCAGGAGGGCAAGA GCCTGTACGTGAAGGGCGAGCCCATCATCAACTTCT ACGACCCCCTGGTGTTTCCCAGCTGCGAGTTTTGCG CCAGCATCAGCCAGGTGAACGAGAAAATCAATCAGA GCCTGGCCTTCATCAGGAAGAGCGACGAGCTCctgcac aacgtgGAGGACAAGATCGAGGAGATCCTGAGCAAGAT CTACCACATCGAGAACGAGATCGCCAGGATCAAGAA GCTGATCGGCGAGGCCAACGCCGGCAAGAGCACCA CCAACATCATGATCACCACCATCATCATCGTGATCATT GTGATCCTGCTGGCCCTGATCGCCGTGGGCCTGCTGC TGTACTGTAAGGCCAGGTCTACCCCTGTGACCCTGA GCAAGGATCAGCTGAGCGGCATCAATAATATCGCCTT CTCCAAC （SEQ ID NO: 71） |
| 003m-STM | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSIPNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVNAG KSTTNWPWYIWLGFIAGLIAI VMVTIMLCCMTSCCSCLKGC CSCGSCCK （SEQ ID NO: 35） | atggagctgcccatcctgaagaccaatgccattacaaccatcctggccgccgtgacac tgtgtttcgccagctctcagaacatcaccgaggaattttaccagagcacctgcagcgcc gtgagcaagggctacctgggcgccctgaggaccggctggtacacaagcgtgatcac catcgagctgagcaacatcaaagaaaacaagtgcaacggcacagacgccaaagtga agctgatcaagcaggagctggacaagtacaagaacgccgtgactgacctgcagctg ctgatgcagtcaacacccttgtggcggatctggcggaggatctggcagcgctatcgcct ctggaattgccgtgtgtaaagtgctgcacctggagggcgaagtgaacaagatcaaaa gcgccctgctgagcaccaacaaggctgtggtgagcctgagcaatggcgtgagcgtg ctgaccttcaaggtgctggacctgaaaaactacattgacaagcagctgctccccatcgt gaacaagcagagctgcagcatccccaacatcgagacggtgatcgagtttcagcaga agaacaaccggctgctggagatcacccgggagtttagcgtgaacgctggcgtgacc accccagtgagcacctatatgctgaccaacagcgagctgctgagcctgatcaacgac atgcccatcaccaacgatcagaagaagctgatgagcagcaacgtgcagatcgtgag gcagcagagctacagcatcatgtgcatcatcaaggaggaggtgctggcctacgtggt gcagctgcctctgtacggagtgatcgacacccccctgttggaaactgcacacatctcctc tgtgcacaaccaacaccaaggagggcagcaacatctgcctgacaagaaccgatagg ggctggtactgtgacaatgccggctctgtgtctttctttcctcaggccgagacatgcaag gtgcagtgcaaccgggtgttctgcgacaccatgaacagcctgaccctgcccagcgag gtcaacctgtgcaacatcgacatcttcaaccccaagtacgactgcaagatcatgacca gcaagaccgacgtgtctagctccgtgatcacaagcctgggagccatcgtgagctgct acggcaagaccaagtgcaccgccagcaacaagaatcggggcatcattaagaccttc agcaacggctgcgactacgtgagcaacaagggcgtggatacagtgagcgtgggca acaccctgtactacgtgaataagcaggagggcaagagcctgtacgtgaagggcgag cccatcatcaacttctacgacccccctggtgtttcccagctgcgagttttgcgccagcatc agccaggtgaacgagaaaatcaatcagagcctggccttcatcaggaagagcgacga gctcctgcacaacgtgaacgccggcaagagcaccaccaacTGGCCTTGGT ACATCTGGCTGGGCTTCATCGCCGGCCTGATCGCCAT CGTGATGGTGACAATCATGCTGTGCTGCATGACCAGC TGTTGTAGCTGCCTGAAGGGCTGCTGCTCCTGTGGAT CTTGCTGCAAG （SEQ ID NO: 72） |
| 003m-1×GCN4t-STM | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSIPNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYHIENEIARIKKLIGE ANAGKSTTNWPWYIWLGFIA GLIAIVMVTIMLCCMTSCCSC LKGCCSCGSCCK （SEQ ID NO: 36） | ATGGAGCTGCCCATCCTGAAGACCAATGCCATTACA ACCATCCTGGCCGCCGTGACACTGTGTTTCGCCAGC TCTCAGAACATCACCGAGGAATTTTACCAGAGCACC TGCAGCGCCGTGAGCAAGGGCTACCTGGGCGCCCTG AGGACCGGCTGGTACACAAGCGTGATCACCATCGAG CTGAGCAACATCAAAGAAAACAAGTGCAACGGCAC AGACGCCAAAGTGAAGCTGATCAAGCAGGAGCTGG ACAAGTACAAGAACGCCGTGACTGACCTGCAGCTGC TGATGCAGTCAACACCTTGTGGCGGATCTGGCGGAG GATCTGGCAGCGCTATCGCCTCTGGAATTGCCGTGTG TAAAGTGCTGCACCTGGAGGGCGAAGTGAACAAGA TCAAAAGCGCCCTGCTGAGCACCAACAAGGCTGTG GTGAGCCTGAGCAATGGCGTGAGCGTGCTGACCTTC AAGGTGCTGGACCTGAAAAACTACATTGACAAGCAG CTGCTCCCCATCGTGAACAAGCAGAGCTGCAGCATC CCCAACATCGAGACGGTGATCGAGTTTCAGCAGAAG AACAACCGGCTGCTGGAGATCACCCGGGAGTTTAGC GTGAACGCTGGCGTGACCACCCCAGTGAGCACCTAT ATGCTGACCAACAGCGAGCTGCTGAGCCTGATCAAC GACATGCCCATCACCAACGATCAGAAGAAGCTGATG AGCAGCAACGTGCAGATCGTGAGGCAGCAGAGCTA CAGCATCATGTGCATCATCAAGGAGGAGGTGCTGGC CTACGTGGTGCAGCTGCCTCTGTACGGAGTGATCGA CACCCCCTGTTGGAAACTGCACACATCTCCTCTGTGC ACAACCAACACCAAGGAGGGCAGCAACATCTGCCT GACAAGAACCGATAGGGGCTGGTACTGTGACAATGC CGGCTCTGTGTCTTTCTTTCCTCAGGCCGAGACATGC AAGGTGCAGTGCAACCGGGTGTTCTGCGACACCATG AACAGCCTGACCCTGCCCAGCGAGGTCAACCTGTGC AACATCGACATCTTCAACCCCAAGTACGACTGCAAG ATCATGACCAGCAAGACCGACGTGTCTAGCTCCGTG ATCACAAGCCTGGGAGCCATCGTGAGCTGCTACGGC AAGACCAAGTGCACCGCCAGCAACAAGAATCGGGG CATCATTAAGACCTTCAGCAACGGCTGCGACTACGT GAGCAACAAGGGCGTGGATACAGTGAGCGTGGGCA ACACCCTGTACTACGTGAATAAGCAGGAGGGCAAGA GCCTGTACGTGAAGGGCGAGCCCATCATCAACTTCT ACGACCCCCTGGTGTTTCCCAGCTGCGAGTTTTGCG CCAGCATCAGCCAGGTGAACGAGAAAATCAATCAGA GCCTGGCCTTCATCAGGAAGAGCGACGAGCTCctgcac aacgtgGAGGACAAGATCGAGGAGATCCTGAGCAAGAT CTACCACATCGAGAACGAGATCGCCAGGATCAAGAA GCTGATCGGCGAGGCCAACGCCGGCAAGAGCACCA CCAACTGGCCTTGGTACATCTGGCTGGGCTTCATCGC CGGCCTGATCGCCATCGTGATGGTGACAATCATGCTG TGCTGCATGACCAGCTGTTGTAGCTGCCTGAAGGGC TGCTGCTCCTGTGGATCTTGCTGCAAG （SEQ ID NO: 73） |
| 003m-P215S | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVNAG KSTTNIMITTIIIVIIVILLALIAV GLLLYCKARSTPVTLSKDQLS GINNIAFSN （SEQ ID NO: 37） | ATGGAGCTGCCCATCCTGAAGACCAATGCCATTACA ACCATCCTGGCCGCCGTGACACTGTGTTTCGCCAGC TCTCAGAACATCACCGAGGAATTTTACCAGAGCACC TGCAGCGCCGTGAGCAAGGGCTACCTGGGCGCCCTG AGGACCGGCTGGTACACAAGCGTGATCACCATCGAG CTGAGCAACATCAAAGAAAACAAGTGCAACGGCAC AGACGCCAAAGTGAAGCTGATCAAGCAGGAGCTGG ACAAGTACAAGAACGCCGTGACTGACCTGCAGCTGC TGATGCAGTCAACACCTTGTGGCGGATCTGGCGGAG GATCTGGCAGCGCTATCGCCTCTGGAATTGCCGTGTG TAAAGTGCTGCACCTGGAGGGCGAAGTGAACAAGA TCAAAAGCGCCCTGCTGAGCACCAACAAGGCTGTG GTGAGCCTGAGCAATGGCGTGAGCGTGCTGACCTTC AAGGTGCTGGACCTGAAAAACTACATTGACAAGCAG CTGCTCCCCATCGTGAACAAGCAGAGCTGCAGCATC agcAACATCGAGACGGTGATCGAGTTTCAGCAGAAGA ACAACCGGCTGCTGGAGATCACCCGGGAGTTTAGCG TGAACGCTGGCGTGACCACCCCAGTGAGCACCTATA TGCTGACCAACAGCGAGCTGCTGAGCCTGATCAACG ACATGCCCATCACCAACGATCAGAAGAAGCTGATGA GCAGCAACGTGCAGATCGTGAGGCAGCAGAGCTAC AGCATCATGTGCATCATCAAGGAGGAGGTGCTGGCC TACGTGGTGCAGCTGCCTCTGTACGGAGTGATCGAC ACCCCCTGTTGGAAACTGCACACATCTCCTCTGTGC ACAACCAACACCAAGGAGGGCAGCAACATCTGCCT GACAAGAACCGATAGGGGCTGGTACTGTGACAATGC CGGCTCTGTGTCTTTCTTTCCTCAGGCCGAGACATGC AAGGTGCAGTGCAACCGGGTGTTCTGCGACACCATG AACAGCCTGACCCTGCCCAGCGAGGTCAACCTGTGC AACATCGACATCTTCAACCCCAAGTACGACTGCAAG ATCATGACCAGCAAGACCGACGTGTCTAGCTCCGTG ATCACAAGCCTGGGAGCCATCGTGAGCTGCTACGGC AAGACCAAGTGCACCGCCAGCAACAAGAATCGGGG CATCATTAAGACCTTCAGCAACGGCTGCGACTACGT GAGCAACAAGGGCGTGGATACAGTGAGCGTGGGCA ACACCCTGTACTACGTGAATAAGCAGGAGGGCAAGA GCCTGTACGTGAAGGGCGAGCCCATCATCAACTTCT ACGACCCCCTGGTGTTTCCCAGCTGCGAGTTTTGCG CCAGCATCAGCCAGGTGAACGAGAAAATCAATCAGA GCCTGGCCTTCATCAGGAAGAGCGACGAGCTCCTGC ACAACGTGAACGCCGGCAAGAGCACCACCAACATC ATGATCACCACCATCATCATCGTGATCATTGTGATCCT GCTGGCCCTGATCGCCGTGGGCCTGCTGCTGTACTGT AAGGCCAGGTCTACCCCTGTGACCCTGAGCAAGGAT CAGCTGAGCGGCATCAATAATATCGCCTTCTCCAACtg a （SEQ ID NO: 74） |
| 003m-P215S-ΔF111 | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSDEFDASISQVNEK INQSLAFIRKSDELLHNVNAG KSTTNIMITTIIIVIIVILLALIAV GLLLYCKARSTPVTLSKDQLS GINNIAFSN （SEQ ID NO: 38） | ATGGAGCTGCCCATCCTGAAGACCAATGCCATTACA ACCATCCTGGCCGCCGTGACACTGTGTTTCGCCAGC TCTCAGAACATCACCGAGGAATTTTACCAGAGCACC TGCAGCGCCGTGAGCAAGGGCTACCTGGGCGCCCTG AGGACCGGCTGGTACACAAGCGTGATCACCATCGAG CTGAGCAACATCAAAGAAAACAAGTGCAACGGCAC AGACGCCAAAGTGAAGCTGATCAAGCAGGAGCTGG ACAAGTACAAGAACGCCGTGACTGACCTGCAGCTGC TGATGCAGTCAACACCTTGTGGCGGATCTGGCGGAG GATCTGGCAGCGCTATCGCCTCTGGAATTGCCGTGTG TAAAGTGCTGCACCTGGAGGGCGAAGTGAACAAGA TCAAAAGCGCCCTGCTGAGCACCAACAAGGCTGTG GTGAGCCTGAGCAATGGCGTGAGCGTGCTGACCTTC AAGGTGCTGGACCTGAAAAACTACATTGACAAGCAG CTGCTCCCCATCGTGAACAAGCAGAGCTGCAGCATC agcAACATCGAGACGGTGATCGAGTTTCAGCAGAAGA ACAACCGGCTGCTGGAGATCACCCGGGAGTTTAGCG TGAACGCTGGCGTGACCACCCCAGTGAGCACCTATA TGCTGACCAACAGCGAGCTGCTGAGCCTGATCAACG ACATGCCCATCACCAACGATCAGAAGAAGCTGATGA GCAGCAACGTGCAGATCGTGAGGCAGCAGAGCTAC AGCATCATGTGCATCATCAAGGAGGAGGTGCTGGCC TACGTGGTGCAGCTGCCTCTGTACGGAGTGATCGAC ACCCCCTGTTGGAAACTGCACACATCTCCTCTGTGC ACAACCAACACCAAGGAGGGCAGCAACATCTGCCT GACAAGAACCGATAGGGGCTGGTACTGTGACAATGC CGGCTCTGTGTCTTTCTTTCCTCAGGCCGAGACATGC AAGGTGCAGTGCAACCGGGTGTTCTGCGACACCATG AACAGCCTGACCCTGCCCAGCGAGGTCAACCTGTGC AACATCGACATCTTCAACCCCAAGTACGACTGCAAG ATCATGACCAGCAAGACCGACGTGTCTAGCTCCGTG ATCACAAGCCTGGGAGCCATCGTGAGCTGCTACGGC AAGACCAAGTGCACCGCCAGCAACAAGAATCGGGG CATCATTAAGACCTTCAGCAACGGCTGCGACTACGT GAGCAACAAGGGCGTGGATACAGTGAGCGTGGGCA ACACCCTGTACTACGTGAATAAGCAGGAGGGCAAGA GCCTGTACGTGAAGGGCGAGCCCATCATCAACTTCT ACGACCCCCTGGTGTTTCCCAGCgacGAGTTTgacGCC AGCATCAGCCAGGTGAACGAGAAAATCAATCAGAGC CTGGCCTTCATCAGGAAGAGCGACGAGCTCCTGCAC AACGTGAACGCCGGCAAGAGCACCACCAACATCATG ATCACCACCATCATCATCGTGATCATTGTGATCCTGCT GGCCCTGATCGCCGTGGGCCTGCTGCTGTACTGTAA GGCCAGGTCTACCCCTGTGACCCTGAGCAAGGATCA GCTGAGCGGCATCAATAATATCGCCTTCTCCAACtga （SEQ ID NO: 75） |
| 003m-P215S-S-3N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELIHNVNGSI NQINASIRKINESISQINAGKST TNIMITTIIIVIIVILLALIAVGLL LYCKARSTPVTLSKDQLSGIN NIAFSN （SEQ ID NO: 39） | ATGGAGCTGCCCATCCTGAAGACCAATGCCATTACA ACCATCCTGGCCGCCGTGACACTGTGTTTCGCCAGC TCTCAGAACATCACCGAGGAATTTTACCAGAGCACC TGCAGCGCCGTGAGCAAGGGCTACCTGGGCGCCCTG AGGACCGGCTGGTACACAAGCGTGATCACCATCGAG CTGAGCAACATCAAAGAAAACAAGTGCAACGGCAC AGACGCCAAAGTGAAGCTGATCAAGCAGGAGCTGG ACAAGTACAAGAACGCCGTGACTGACCTGCAGCTGC TGATGCAGTCAACACCTTGTGGCGGATCTGGCGGAG GATCTGGCAGCGCTATCGCCTCTGGAATTGCCGTGTG TAAAGTGCTGCACCTGGAGGGCGAAGTGAACAAGA TCAAAAGCGCCCTGCTGAGCACCAACAAGGCTGTG GTGAGCCTGAGCAATGGCGTGAGCGTGCTGACCTTC AAGGTGCTGGACCTGAAAAACTACATTGACAAGCAG CTGCTCCCCATCGTGAACAAGCAGAGCTGCAGCATC agcAACATCGAGACGGTGATCGAGTTTCAGCAGAAGA ACAACCGGCTGCTGGAGATCACCCGGGAGTTTAGCG TGAACGCTGGCGTGACCACCCCAGTGAGCACCTATA TGCTGACCAACAGCGAGCTGCTGAGCCTGATCAACG ACATGCCCATCACCAACGATCAGAAGAAGCTGATGA GCAGCAACGTGCAGATCGTGAGGCAGCAGAGCTAC AGCATCATGTGCATCATCAAGGAGGAGGTGCTGGCC TACGTGGTGCAGCTGCCTCTGTACGGAGTGATCGAC ACCCCCTGTTGGAAACTGCACACATCTCCTCTGTGC ACAACCAACACCAAGGAGGGCAGCAACATCTGCCT GACAAGAACCGATAGGGGCTGGTACTGTGACAATGC CGGCTCTGTGTCTTTCTTTCCTCAGGCCGAGACATGC AAGGTGCAGTGCAACCGGGTGTTCTGCGACACCATG AACAGCCTGACCCTGCCCAGCGAGGTCAACCTGTGC AACATCGACATCTTCAACCCCAAGTACGACTGCAAG ATCATGACCAGCAAGACCGACGTGTCTAGCTCCGTG ATCACAAGCCTGGGAGCCATCGTGAGCTGCTACGGC AAGACCAAGTGCACCGCCAGCAACAAGAATCGGGG CATCATTAAGACCTTCAGCAACGGCTGCGACTACGT GAGCAACAAGGGCGTGGATACAGTGAGCGTGGGCA ACACCCTGTACTACGTGAATAAGCAGGAGGGCAAGA GCCTGTACGTGAAGGGCGAGCCCATCATCAACTTCT ACGACCCCCTGGTGTTTCCCAGCTGCGAGTTTTGCG CCAGCATCAGCCAGGTGAACGAGAAAATCAATCAGA GCCTGGCCTTCATCAGGAAGAGCGACGAGCTCattcaca acgtgaacggcagcatcaaccagatcaatgcctctatcagaaagatcaacgagtccat cagccagatcAACGCCGGCAAGAGCACCACCAACATCAT GATCACCACCATCATCATCGTGATCATTGTGATCCTGC TGGCCCTGATCGCCGTGGGCCTGCTGCTGTACTGTAA GGCCAGGTCTACCCCTGTGACCCTGAGCAAGGATCA GCTGAGCGGCATCAATAATATCGCCTTCTCCAACtga （SEQ ID NO: 76） |
| 003m-P215S-ΔF111-S-3N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSDEFDASISQVNEK INQSLAFIRKSDELIHNVNGSI NQINASIRKINESISQINAGKST TNIMITTIIIVIIVILLALIAVGLL LYCKARSTPVTLSKDQLSGIN NIAFSN （SEQ ID NO: 40） | ATGGAGCTGCCCATCCTGAAGACCAATGCCATTACA ACCATCCTGGCCGCCGTGACACTGTGTTTCGCCAGC TCTCAGAACATCACCGAGGAATTTTACCAGAGCACC TGCAGCGCCGTGAGCAAGGGCTACCTGGGCGCCCTG AGGACCGGCTGGTACACAAGCGTGATCACCATCGAG CTGAGCAACATCAAAGAAAACAAGTGCAACGGCAC AGACGCCAAAGTGAAGCTGATCAAGCAGGAGCTGG ACAAGTACAAGAACGCCGTGACTGACCTGCAGCTGC TGATGCAGTCAACACCTTGTGGCGGATCTGGCGGAG GATCTGGCAGCGCTATCGCCTCTGGAATTGCCGTGTG TAAAGTGCTGCACCTGGAGGGCGAAGTGAACAAGA TCAAAAGCGCCCTGCTGAGCACCAACAAGGCTGTG GTGAGCCTGAGCAATGGCGTGAGCGTGCTGACCTTC AAGGTGCTGGACCTGAAAAACTACATTGACAAGCAG CTGCTCCCCATCGTGAACAAGCAGAGCTGCAGCATC agcAACATCGAGACGGTGATCGAGTTTCAGCAGAAGA ACAACCGGCTGCTGGAGATCACCCGGGAGTTTAGCG TGAACGCTGGCGTGACCACCCCAGTGAGCACCTATA TGCTGACCAACAGCGAGCTGCTGAGCCTGATCAACG ACATGCCCATCACCAACGATCAGAAGAAGCTGATGA GCAGCAACGTGCAGATCGTGAGGCAGCAGAGCTAC AGCATCATGTGCATCATCAAGGAGGAGGTGCTGGCC TACGTGGTGCAGCTGCCTCTGTACGGAGTGATCGAC ACCCCCTGTTGGAAACTGCACACATCTCCTCTGTGC ACAACCAACACCAAGGAGGGCAGCAACATCTGCCT GACAAGAACCGATAGGGGCTGGTACTGTGACAATGC CGGCTCTGTGTCTTTCTTTCCTCAGGCCGAGACATGC AAGGTGCAGTGCAACCGGGTGTTCTGCGACACCATG AACAGCCTGACCCTGCCCAGCGAGGTCAACCTGTGC AACATCGACATCTTCAACCCCAAGTACGACTGCAAG ATCATGACCAGCAAGACCGACGTGTCTAGCTCCGTG ATCACAAGCCTGGGAGCCATCGTGAGCTGCTACGGC AAGACCAAGTGCACCGCCAGCAACAAGAATCGGGG CATCATTAAGACCTTCAGCAACGGCTGCGACTACGT GAGCAACAAGGGCGTGGATACAGTGAGCGTGGGCA ACACCCTGTACTACGTGAATAAGCAGGAGGGCAAGA GCCTGTACGTGAAGGGCGAGCCCATCATCAACTTCT ACGACCCCCTGGTGTTTCCCAGCgacGAGTTTgacGCC AGCATCAGCCAGGTGAACGAGAAAATCAATCAGAGC CTGGCCTTCATCAGGAAGAGCGACGAGCTCattcacaacg tgaacggcagcatcaaccagatcaatgcctctatcagaaagatcaacgagtccatcag ccagatcAACGCCGGCAAGAGCACCACCAACATCATGA TCACCACCATCATCATCGTGATCATTGTGATCCTGCTG GCCCTGATCGCCGTGGGCCTGCTGCTGTACTGTAAG GCCAGGTCTACCCCTGTGACCCTGAGCAAGGATCAG CTGAGCGGCATCAATAATATCGCCTTCTCCAACtga （SEQ ID NO: 77） |
| 003m-P215S-1×GCN4t | (SEQ ID NO: 28) | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAGAAAtTCtCtCCAAAAtttAtCATAttGAA AATGAAAttGCtAGAAttaaaaaactcattggagaagctAACGCCG GCAAGAGCACCACCAACAtCAtGAtCACCACCAtCAtC AtCGtGAtCAttGtGAtCCtGCtGgccctgatcgccgtgggcctgctgctg tactgcaaggctaggagcacccctgtgaccctgagcaaggatcagctgagcggcatc aataacatcgccttcagcaactGA （SEQ ID NO: 78） |
| 003m-P215S-ΔF111-1×GCN4t-opti3 | (SEQ ID NO: 33) | ATGGAGCTGCCCATCCTGAAGACGAATGCCATTACC ACCATCCTGGCCGCCGTCACGCTGTGCTTTGCCAGC AGCCAGAACATCACCGAGGAGTTCTACCAGTCCACC TGCTCCGCAGTGTCCAAGGGCTACCTGGGGGCACTG CGGACCGGGTGGTACACCAGCGTCATTACCATTGAG CTCTCCAACATCAAGGAGAACAAGTGCAATGGTACT GACGCTAAGGTGAAGCTTATCAAGCAGGAGCTTGAT AAGTACAAGAACGCCGTGACTGATCTGCAGCTGCTG ATGCAGAGCACACCGTGCGGCGGCTCAGGAGGTGG TAGTGGCAGCGCCATTGCCAGCGGCATCGCCGTCTG CAAGGTGCTGCACCTGGAGGGCGAGGTGAACAAGA TCAAGAGCGCCCTCCTGAGCACCAACAAGGCGGTG GTGTCGCTGAGCAATGGCGTGAGCGTGTTGACCTTC AAGGTGCTGGATCTCAAGAACTACATCGATAAGCAG CTGCTGCCGATTGTGAACAAGCAGTCGTGCAGCATC AGCAATATCGAGACAGTGATTGAGTTCCAGCAGAAG AACAACAGGCTCCTGGAGATCACCAGAGAGTTCTCA GTGAACGCCGGGGTGACCACTCCTGTGAGCACATAC ATGCTGACCAATAGCGAGCTGCTGTCTCTGATCAACG ATATGCCCATTACTAATGATCAGAAGAAGCTGATGAG CAGTAATGTGCAGATCGTGAGACAGCAGAGCTACAG CATTATGTGCATCATCAAGGAGGAGGTGCTTGCTTAC GTGGTGCAGCTGCCCCTGTACGGGGTGATCGACACC CCTTGTTGGAAGCTGCACACCTCTCCTTTGTGTACCA CCAACACAAAGGAGGGGAGCAACATCTGCCTGACG AGGACGGACCGGGGCTGGTATTGCGACAATGCCGGC TCCGTGTCCTTCTTCCCTCAGGCAGAGACTTGTAAG GTGCAGTGCAACCGGGTCTTCTGTGACACCATGAAT AGCCTGACCCTGCCCAGCGAGGTGAACCTGTGCAAC ATCGACATCTTCAACCCGAAGTATGATTGCAAGATCA TGACTTCGAAGACCGATGTGAGCAGCAGCGTGATCA CCTCGCTGGGCGCTATCGTGAGCTGTTACGGCAAGA CCAAGTGCACTGCATCTAACAAGAATCGCGGCATTAT TAAGACCTTTAGTAATGGCTGCGATTACGTTTCCAAC AAGGGGGTCGACACCGTGTCAGTGGGCAACACGCT GTACTACGTGAACAAGCAGGAGGGGAAGAGCCTGT ACGTGAAGGGGGAGCCCATCATCAACTTTTACGACC CACTGGTCTTCCCCTCCGATGAGTTCGACGCCAGCAT TAGCCAGGTCAATGAGAAGATTAATCAGAGCCTGGC TTTCATTCGCAAGAGCGACGAGCTCTTGCACAATGT GGAGGACAAGATCGAGGAGATCTTGTCCAAGATCTA CCACATTGAGAATGAGATCGCCAGGATTAAGAAGCT CATTGGCGAGGCTAATGCTGGCAAGTCGACCACTAA CATCATGATCACGACGATCATCATCGTGATCATCGTG ATCCTCCTGGCCCTGATTGCAGTGGGGCTGCTGCTCT ACTGCAAGGCCAGGAGCACCCCGGTGACACTGAGC AAGGACCAGCTCTCTGGGATCAACAACATCGCCTTC AGCAACtga （SEQ ID NO: 79） |
| 003m-P215S-DELL-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLEDKIEEIL SKIYHIENEIARIKKLIGEAHN VNAGKSTTNIMITTIIIVIIVILL ALIAVGLLLYCKARSTPVTLS KDQLSGINNIAFSN （SEQ ID NO: 120） | atggagctgcccatcctgaagaccaatgccattacaaccatcctggccgccgtgacac tgtgtttcgccagctctcagaacatcaccgaggaattttaccagagcacctgcagcgcc gtgagcaagggctacctgggcgccctgaggaccggctggtacacaagcgtgatcac catcgagctgagcaacatcaaagaaaacaagtgcaacggcacagacgccaaagtga agctgatcaagcaggagctggacaagtacaagaacgccgtgactgacctgcagctg ctgatgcagtcaacacccttgtggcggatctggcggaggatctggcagcgctatcgcct ctggaattgccgtgtgtaaagtgctgcacctggagggcgaagtgaacaagatcaaaa gcgccctgctgagcaccaacaaggctgtggtgagcctgagcaatggcgtgagcgtg ctgaccttcaaggtgctggacctgaaaaactacattgacaagcagctgctccccatcgt gaacaagcagagctgcagcatcagcaacatcgagacggtgatcgagtttcagcaga agaacaaccggctgctggagatcacccgggagtttagcgtgaacgctggcgtgacc accccagtgagcacctatatgctgaccaacagcgagctgctgagcctgatcaacgac atgcccatcaccaacgatcagaagaagctgatgagcagcaacgtgcagatcgtgag gcagcagagctacagcatcatgtgcatcatcaaggaggaggtgctggcctacgtggt gcagctgcctctgtacggagtgatcgacacccccctgttggaaactgcacacatctcctc tgtgcacaaccaacaccaaggagggcagcaacatctgcctgacaagaaccgatagg ggctggtactgtgacaatgccggctctgtgtctttctttcctcaggccgagacatgcaag gtgcagtgcaaccgggtgttctgcgacaccatgaacagcctgaccctgcccagcgag gtcaacctgtgcaacatcgacatcttcaaccccaagtacgactgcaagatcatgacca gcaagaccgacgtgtctagctccgtgatcacaagcctgggagccatcgtgagctgct acggcaagaccaagtgcaccgccagcaacaagaatcggggcatcattaagaccttc agcaacggctgcgactacgtgagcaacaagggcgtggatacagtgagcgtgggca acaccctgtactacgtgaataagcaggagggcaagagcctgtacgtgaagggcgag cccatcatcaacttctacgacccccctggtgtttcccagctgcgagttttgcgccagcatc agccaggtgaacgagaaaatcaatcagagcctggccttcatcaggaagagcgacga gctcctggaagataaaatTGAAGAAAtTCtCtCCAAAAtttATCAT AttGAAAATGAAAttGCtAGAAttaaaaaactcattggagaagctcaca acgtgaacgccggcaagagcaccaccaacatcatgatcaccaccatcatcatcgtga tcattgtgatcctgctggccctgatcgccgtgggcctgctgctgtactgcaaggctagg agcacccctgtgaccctgagcaaggatcagctgagcggcatcaataacatcgccttc agcaac （SEQ ID NO: 121） |
| 003m-P215S-NAG-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVNAG EDKIEEILSKIYHIENEIARIKK LIGEAKSTTNIMITTIIIVIIVILL ALIAVGLLLYCKARSTPVTLS KDQLSGINNIAFSN （SEQ ID NO: 122） | atggagctgcccatcctgaagaccaatgccattacaaccatcctggccgccgtgacac tgtgtttcgccagctctcagaacatcaccgaggaattttaccagagcacctgcagcgcc gtgagcaagggctacctgggcgccctgaggaccggctggtacacaagcgtgatcac catcgagctgagcaacatcaaagaaaacaagtgcaacggcacagacgccaaagtga agctgatcaagcaggagctggacaagtacaagaacgccgtgactgacctgcagctg ctgatgcagtcaacaccttgtggcggatctggcggaggatctggcagcgctatcgcct ctggaattgccgtgtgtaaagtgctgcacctggagggcgaagtgaacaagatcaaaa gcgccctgctgagcaccaacaaggctgtggtgagcctgagcaatggcgtgagcgtg ctgaccttcaaggtgctggacctgaaaaactacattgacaagcagctgctccccatcgt gaacaagcagagctgcagcatcagcaacatcgagacggtgatcgagtttcagcaga agaacaaccggctgctggagatcaccccgggagtttagcgtgaacgctggcgtgacc accccagtgagcacctatatgctgaccaacagcgagctgctgagcctgatcaacgac atgcccatcaccaacgatcagaagaagctgatgagcagcaacgtgcagatcgtgag gcagcagagctacagcatcatgtgcatcatcaaggaggaggtgctggcctacgtggt gcagctgcctctgtacggagtgatcgacacccccctgttggaaactgcacacatctcctc tgtgcacaaccaacaccaaggagggcagcaacatctgcctgacaagaaccgatagg ggctggtactgtgacaatgccggctctgtgtctttctttcctcaggccgagacatgcaag gtgcagtgcaaccgggtgttctgcgacaccatgaacagcctgaccctgcccagcgag gtcaacctgtgcaacatcgacatcttcaaccccaagtacgactgcaagatcatgacca gcaagaccgacgtgtctagctccgtgatcacaagcctgggagccatcgtgagctgct acggcaagaccaagtgcaccgccagcaacaagaatcggggcatcattaagaccttc agcaacggctgcgactacgtgagcaacaagggcgtggatacagtgagcgtgggca acaccctgtactacgtgaataagcaggagggcaagagcctgtacgtgaagggcgag cccatcatcaacttctacgacccccctggtgtttcccagctgcgagttttgcgccagcatc agccaggtgaacgagaaaatcaatcagagcctggccttcatcaggaagagcgacga gctcctgcacaacgtgaacgccggcgaagataaaatTGAAGAAAtTCtCtC CAAAAtttATCATAttGAAAATGAAAttGCtAGAAttaaaaaact cattggagaagctaagagcaccaccaacatcatgatcaccaccatcatcatcgtgatc attgtgatcctgctggccctgatcgccgtgggcctgctgctgtactgcaaggctagga gcacccctgtgaccctgagcaaggatcagctgagcggcatcaataacatcgccttca gcaac （SEQ ID NO: 123） |
| 003m-P215S-KST-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVNAG KSTEDKIEEILSKIYHIENEIAR IKKLIGEATNIMITTIIIVIIVILL ALIAVGLLLYCKARSTPVTLS KDQLSGINNIAFSN （SEQ ID NO: 124） | atggagctgcccatcctgaagaccaatgccattacaaccatcctggccgccgtgacac tgtgtttcgccagctctcagaacatcaccgaggaattttaccagagcacctgcagcgcc gtgagcaagggctacctgggcgccctgaggaccggctggtacacaagcgtgatcac catcgagctgagcaacatcaaagaaaacaagtgcaacggcacagacgccaaagtga agctgatcaagcaggagctggacaagtacaagaacgccgtgactgacctgcagctg ctgatgcagtcaacacccttgtggcggatctggcggaggatctggcagcgctatcgcct ctggaattgccgtgtgtaaagtgctgcacctggagggcgaagtgaacaagatcaaaa gcgccctgctgagcaccaacaaggctgtggtgagcctgagcaatggcgtgagcgtg ctgaccttcaaggtgctggacctgaaaaactacattgacaagcagctgctccccatcgt gaacaagcagagctgcagcatcagcaacatcgagacggtgatcgagtttcagcaga agaacaaccggctgctggagatcacccgggagtttagcgtgaacgctggcgtgacc accccagtgagcacctatatgctgaccaacagcgagctgctgagcctgatcaacgac atgcccatcaccaacgatcagaagaagctgatgagcagcaacgtgcagatcgtgag gcagcagagctacagcatcatgtgcatcatcaaggaggaggtgctggcctacgtggt gcagctgcctctgtacggagtgatcgacacccccctgttggaaactgcacacatctcctc tgtgcacaaccaacaccaaggagggcagcaacatctgcctgacaagaaccgatagg ggctggtactgtgacaatgccggctctgtgtctttctttcctcaggccgagacatgcaag gtgcagtgcaaccgggtgttctgcgacaccatgaacagcctgaccctgcccagcgag gtcaacctgtgcaacatcgacatcttcaaccccaagtacgactgcaagatcatgacca gcaagaccgacgtgtctagctccgtgatcacaagcctgggagccatcgtgagctgct acggcaagaccaagtgcaccgccagcaacaagaatcggggcatcattaagaccttc agcaacggctgcgactacgtgagcaacaagggcgtggatacagtgagcgtgggca acaccctgtactacgtgaataagcaggagggcaagagcctgtacgtgaagggcgag cccatcatcaacttctacgacccccctggtgtttcccagctgcgagttttgcgccagcatc agccaggtgaacgagaaaatcaatcagagcctggccttcatcaggaagagcgacga gctcctgcacaacgtgaacgccggcaagagcaccgaagataaaatTGAAGAA AtTCtCtCCAAAAtttATCATAttGAAAATGAAAttGCtAGA Attaaaaaactcattggagaagctaccaacatcatgatcaccaccatcatcatcgtgat cattgtgatcctgctggccctgatcgccgtgggcctgctgctgtactgcaaggctagga gcacccctgtgaccctgagcaaggatcagctgagcggcatcaataacatcgccttca gcaac （SEQ ID NO: 125） |
| 007m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGSGSAIASGIAVCKVLHLEG EVNKIKSALLSTNKAVVSLSN GVSVLTFKVLDLKNYIDKQLL PIVNKQSCSISNIETVIEFQQK NNRLLEITREFSVNAGVTTPV STYMLTNSELLSLINDMPITN DQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYHIENEIARIKKLIGE ANAGKSTTNIMITTIIIVIIVILL ALIAVGLLLYCKARSTPVTLS KDQLSGINNIAFSN （SEQ ID NO: 126） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAtCtGGCAGCGCtAtCGCCtCtGGAAttGC CGtGtGtAAAGtGCtGCACCtGGAGGGCGAAGtGAACAA GAtCAAAAGCGCCCtGCtGAGCACCAACAAGGCtGtGG tGAGCCtGAGCAAtGGCGtGAGCGtGCtGACCttCAAGGt GCtGGACCtGAAAAACtACAttGACAAGCAGCtGCtCCC CAtCGtGAACAAGCAGAGCtGCAGCAtCagcAACAtCGA GACGGtGAtCGAGtttCAGCAGAAGAACAACCGGCtGCt GGAGAtCACCCGGGAGtttAGCGtGAACGCtGGCGtGAC CACCCCAGtGAGCACCtAtAtGCtGACCAACAGCGAGCt GCtGAGCCtGAtCAACGACAtGCCCAtCACCAACGAtCA GAAGAAGCtGAtGAGCAGCAACGtGCAGAtCGtGAGG CAGCAGAGCtACAGCAtCAtGtGCAtCAtCAAGGAGGA GGtGCtGGCCtACGtGGtGCAGCtGCCtCtGtACGGAGtGA tCGACACCCCCtGttGGAAACtGCACACAtCtCCtCtGtGC ACAACCAACACCAAGGAGGGCAGCAACAtCtGCCtGA CAAGAACCGAtAGGGGCtGGtACtGtGACAAtGCCGGCt CtGtGtCtttCtttCCtCAGGCCGAGACAtGCAAGGtGCAGtG CAACCGGGtGttCtGCGACACCAtGAACAGCCtGACCCt GCCCAGCGAGGtCAACCtGtGCAACAtCGACAtCttCAA CCCCAAGtACGACtGCAAGAtCAtGACCAGCAAGACC GACGtGtCtAGCtCCGtGAtCACAAGCCtGGGAGCCAtCG tGAGCtGCtACGGCAAGACCAAGtGCACCGCCAGCAA CAAGAAtCGGGGCAtCAttAAGACCttCAGCAACGGCtG CGACtACGtGAGCAACAAGGGCGtGGAtACAGtGAGCG tGGGCAACACCCtGtACtACGtGAAtAAGCAGGAGGGC AAGAGCCtGtACGtGAAGGGCGAGCCCAtCAtCAACttCt ACGACCCCCtGGtGtttCCCAGCtGCGAGttttGCGCCAGC AtCAGCCAGGtGAACGAGAAAAtCAAtCAGAGCCtGGC CttCAtCAGGAAGAGCGACGAGCtCctgcacaacgtggaagataa aatTGAAGAAAtTCtCtCCAAAAtttATCATAttGAAAATGA AAttGCtAGAAttaaaaaactcattggagaagctAACGCCGGCAAG AGCACCACCAACAtCAtGAtCACCACCAtCAtCAtCGtG AtCAttGtGAtCCtGCtGgccctgatcgccgtgggcctgctgctgtactgcaa ggctaggagcacccctgtgaccctgagcaaggatcagctgagcggcatcaataacat cgccttcagcaac （SEQ ID NO: 127） |
| 008m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GSGSAIASGIAVCKVLHLEGE VNKIKSALLSTNKAVVSLSNG VSVLTFKVLDLKNYIDKQLLP IVNKQSCSISNIETVIEFQQKN NRLLEITREFSVNAGVTTPVS TYMLTNSELLSLINDMPITND QKKLMSSNVQIVRQQSYSIM CIIKEEVLAYVVQLPLYGVIDT PCWKLHTSPLCTTNTKEGSNI CLTRTDRGWYCDNAGSVSFF PQAETCKVQCNRVFCDTMNS LTLPSEVNLCNIDIFNPKYDCK IMTSKTDVSSSVITSLGAIVSC YGKTKCTASNKNRGIIKTFSN GCDYVSNKGVDTVSVGNTLY YVNKQEGKSLYVKGEPIINFY DPLVFPSCEFCASISQVNEKIN QSLAFIRKSDELLHNVEDKIE EILSKIYHIENEIARIKKLIGEA NAGKSTTNIMITTIIIVIIVILLA LIAVGLLLYCKARSTPVTLSK DQLSGINNIAFSN （SEQ ID NO: 128） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCtCtGGCAGCGCtAtCGCCtCtGGAAttGCCGtG tGtAAAGtGCtGCACCtGGAGGGCGAAGtGAACAAGAtC AAAAGCGCCCtGCtGAGCACCAACAAGGCtGtGGtGAG CCtGAGCAAtGGCGtGAGCGtGCtGACCttCAAGGtGCtG GACCtGAAAAACtACAttGACAAGCAGCtGCtCCCCAtC GtGAACAAGCAGAGCtGCAGCAtCagcAACAtCGAGAC GGtGAtCGAGtttCAGCAGAAGAACAACCGGCtGCtGGA GAtCACCCGGGAGtttAGCGtGAACGCtGGCGtGACCAC CCCAGtGAGCACCtAtAtGCtGACCAACAGCGAGCtGCt GAGCCtGAtCAACGACAtGCCCAtCACCAACGAtCAGA AGAAGCtGAtGAGCAGCAACGtGCAGAtCGtGAGGCA GCAGAGCtACAGCAtCAtGtGCAtCAtCAAGGAGGAGGt GCtGGCCtACGtGGtGCAGCtGCCtCtGtACGGAGtGAtCG ACACCCCCtGttGGAAACtGCACACAtCtCCtCtGtGCACA ACCAACACCAAGGAGGGCAGCAACAtCtGCCtGACAA GAACCGAtAGGGGCtGGtACtGtGACAAtGCCGGCtCtGt GtCtttCtttCCtCAGGCCGAGACAtGCAAGGtGCAGtGCA ACCGGGtGttCtGCGACACCAtGAACAGCCtGACCCtGC CCAGCGAGGtCAACCtGtGCAACAtCGACAtCttCAACC CCAAGtACGACtGCAAGAtCAtGACCAGCAAGACCGA CGtGtCtAGCtCCGtGAtCACAAGCCtGGGAGCCAtCGtG AGCtGCtACGGCAAGACCAAGtGCACCGCCAGCAACA AGAAtCGGGGCAtCAttAAGACCttCAGCAACGGCtGCG ACtACGtGAGCAACAAGGGCGtGGAtACAGtGAGCGtG GGCAACACCCtGtACtACGtGAAtAAGCAGGAGGGCAA GAGCCtGtACGtGAAGGGCGAGCCCAtCAtCAACttCtAC GACCCCCtGGtGtttCCCAGCtGCGAGttttGCGCCAGCAtC AGCCAGGtGAACGAGAAAAtCAAtCAGAGCCtGGCCtt CAtCAGGAAGAGCGACGAGCtCctgcacaacgtggaagataaaat TGAAGAAAtTCtCtCCAAAAtttATCATAttGAAAATGAA AttGCtAGAAttaaaaaactcattggagaagctAACGCCGGCAAGA GCACCACCAACAtCAtGAtCACCACCAtCAtCAtCGtGAt CAttGtGAtCCtGCtGgccctgatcgccgtgggcctgctgctgtactgcaagg ctaggagcacccctgtgaccctgagcaaggatcagctgagcggcatcaataacatcg ccttcagcaac （SEQ ID NO: 129） |
| 009m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGSG SGSAIASGIAVCKVLHLEGEV NKIKSALLSTNKAVVSLSNGV SVLTFKVLDLKNYIDKQLLPI VNKQSCSISNIETVIEFQQKNN RLLEITREFSVNAGVTTPVST YMLTNSELLSLINDMPITNDQ KKLMSSNVQIVRQQSYSIMCI IKEEVLAYVVQLPLYGVIDTP CWKLHTSPLCTTNTKEGSNIC LTRTDRGWYCDNAGSVSFFP QAETCKVQCNRVFCDTMNSL TLPSEVNLCNIDIFNPKYDCKI MTSKTDVSSSVITSLGAIVSC YGKTKCTASNKNRGIIKTFSN GCDYVSNKGVDTVSVGNTLY YVNKQEGKSLYVKGEPIINFY DPLVFPSCEFCASISQVNEKIN QSLAFIRKSDELLHNVEDKIE EILSKIYHIENEIARIKKLIGEA NAGKSTTNIMITTIIIVIIVILLA LIAVGLLLYCKARSTPVTLSK DQLSGINNIAFSN （SEQ ID NO: 130） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGAt CtGGCtCtGGCAGCGCtAtCGCCtCtGGAAttGCCGtGtGtA AAGtGCtGCACCtGGAGGGCGAAGtGAACAAGAtCAA AAGCGCCCtGCtGAGCACCAACAAGGCtGtGGtGAGCC tGAGCAAtGGCGtGAGCGtGCtGACCttCAAGGtGCtGGA CCtGAAAAACtACAttGACAAGCAGCtGCtCCCCAtCGtG AACAAGCAGAGCtGCAGCAtCagcAACAtCGAGACGGt GAtCGAGtttCAGCAGAAGAACAACCGGCtGCtGGAGAt CACCCGGGAGtttAGCGtGAACGCtGGCGtGACCACCCC AGtGAGCACCtAtAtGCtGACCAACAGCGAGCtGCtGAG CCtGAtCAACGACAtGCCCAtCACCAACGAtCAGAAGA AGCtGAtGAGCAGCAACGtGCAGAtCGtGAGGCAGCAG AGCtACAGCAtCAtGtGCAtCAtCAAGGAGGAGGtGCtG GCCtACGtGGtGCAGCtGCCtCtGtACGGAGtGAtCGACA CCCCCtGttGGAAACtGCACACAtCtCCtCtGtGCACAACC AACACCAAGGAGGGCAGCAACAtCtGCCtGACAAGA ACCGAtAGGGGCtGGtACtGtGACAAtGCCGGCtCtGtGtC tttCtttCCtCAGGCCGAGACAtGCAAGGtGCAGtGCAACC GGGtGttCtGCGACACCAtGAACAGCCtGACCCtGCCCA GCGAGGtCAACCtGtGCAACAtCGACAtCttCAACCCCA AGtACGACtGCAAGAtCAtGACCAGCAAGACCGACGtG tCtAGCtCCGtGAtCACAAGCCtGGGAGCCAtCGtGAGCt GCtACGGCAAGACCAAGtGCACCGCCAGCAACAAGA AtCGGGGCAtCAttAAGACCttCAGCAACGGCtGCGACtA CGtGAGCAACAAGGGCGtGGAtACAGtGAGCGtGGGC AACACCCtGtACtACGtGAAtAAGCAGGAGGGCAAGAG CCtGtACGtGAAGGGCGAGCCCAtCAtCAACttCtACGAC CCCCtGGtGtttCCCAGCtGCGAGttttGCGCCAGCAtCAGC CAGGtGAACGAGAAAAtCAAtCAGAGCCtGGCCttCAtC AGGAAGAGCGACGAGCtCctgcacaacgtggaagataaaatTGA AGAAAtTCtCtCCAAAAtttATCATAttGAAAATGAAAttGC tAGAAttaaaaaactcattggagaagctAACGCCGGCAAGAGCAC CACCAACAtCAtGAtCACCACCAtCAtCAtCGtGAtCAttGt GAtCCtGCtGgccctgatcgccgtgggcctgctgctgtactgcaaggctaggag cacccctgtgaccctgagcaaggatcagctgagcggcatcaataacatcgccttcag caac （SEQ ID NO: 131） |
| 010m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GSAIASGIAVCKVLHLEGEVN KIKSALLSTNKAVVSLSNGVS VLTFKVLDLKNYIDKQLLPIV NKQSCSISNIETVIEFQQKNNR LLEITREFSVNAGVTTPVSTY MLTNSELLSLINDMPITNDQK KLMSSNVQIVRQQSYSIMCII KEEVLAYVVQLPLYGVIDTPC WKLHTSPLCTTNTKEGSNICL TRTDRGWYCDNAGSVSFFPQ AETCKVQCNRVFCDTMNSLT LPSEVNLCNIDIFNPKYDCKI MTSKTDVSSSVITSLGAIVSC YGKTKCTASNKNRGIIKTFSN GCDYVSNKGVDTVSVGNTLY YVNKQEGKSLYVKGEPIINFY DPLVFPSCEFCASISQVNEKIN QSLAFIRKSDELLHNVEDKIE EILSKIYHIENEIARIKKLIGEA NAGKSTTNIMITTIIIVIIVILLA LIAVGLLLYCKARSTPVTLSK DQLSGINNIAFSN （SEQ ID NO: 132） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGA GGCtCtGGCAGCGCtAtCGCCtCtGGAAttGCCGtGtAA AGtGCtGCACCtGGAGGGCGAAGtGAACAAGAtCAAA AGCGCCCtGCtGAGCACCAACAAGGCtGtGGtGAGCCt GAGCAAtGGCGtGAGCGtGCtGACCttCAAGGtGCtGGA CCtGAAAAACtACAttGACAAGCAGCtGCtCCCCAtCGtG AACAAGCAGAGCtGCAGCAtCagcAACAtCGAGACGGt GAtCGAGtttCAGCAGAAGAACAACCGGCtGCtGGAGAt CACCCGGGAGtttAGCGtGAACGCtGGCGtGACCACCCC AGtGAGCACCtAtAtGCtGACCAACAGCGAGCtGCtGAG CCtGAtCAACGACAtGCCCAtCACCAACGAtCAGAAGA AGCtGAtGAGCAGCAACGtGCAGAtCGtGAGGCAGCAG AGCtACAGCAtCAtGtGCAtCAtCAAGGAGGAGGtGCtG GCCtACGtGGtGCAGCtGCCtCtGtACGGAGtGAtCGACA CCCCCtGttGGAAACtGCACACAtCtCCtCtGtGCACAACC AACACCAAGGAGGGCAGCAACAtCtGCCtGACAAGA ACCGAtAGGGGCtGGtACtGtGACAAtGCCGGCtCtGtGtC tttCtttCCtCAGGCCGAGACAtGCAAGGtGCAGtGCAACC GGGtGttCtGCGACACCAtGAACAGCCtGACCCtGCCCA GCGAGGtCAACCtGtGCAACAtCGACAtCttCAACCCCA AGtACGACtGCAAGAtCAtGACCAGCAAGACCGACGtG tCtAGCtCCGtGAtCACAAGCCtGGGAGCCAtCGtGAGCt GCtACGGCAAGACCAAGtGCACCGCCAGCAACAAGA AtCGGGGCAtCAttAAGACCttCAGCAACGGCtGCGACtA CGtGAGCAACAAGGGCGtGGAtACAGtGAGCGtGGGC AACACCCtGtACtACGtGAAtAAGCAGGAGGGCAAGAG CCtGtACGtGAAGGGCGAGCCCAtCAtCAACttCtACGAC CCCCtGGtGtttCCCAGCtGCGAGttttGCGCCAGCAtCAGC CAGGtGAACGAGAAAAtCAAtCAGAGCCtGGCCttCAtC AGGAAGAGCGACGAGCtCctgcacaacgtggaagataaaatTGA AGAAAtTCtCtCCAAAAtttATCATAttGAAAATGAAAttGC tAGAAttaaaaaactcattggagaagctAACGCCGGCAAGAGCAC CACCAACAtCAtGAtCACCACCAtCAtCAtCGtGAtCAttGt GAtCCtGCtGgccctgatcgccgtgggcctgctgctgtactgcaaggctaggag cacccctgtgaccctgagcaaggatcagctgagcggcatcaataacatcgccttcag caac （SEQ ID NO: 133） |
| 011m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGSG SAIASGIAVCKVLHLEGEVNKI KSALLSTNKAVVSLSNGVSVL TFKVLDLKNYIDKQLLPIVNK QSCSISNIETVIEFQQKNNRLL EITREFSVNAGVTTPVSTYML TNSELLSLINDMPITNDQKKL MSSNVQIVRQQSYSIMCIIKEE VLAYVVQLPLYGVIDTPCWK LHTSPLCTTNTKEGSNICLTRT DRGWYCDNAGSVSFFPQAET CKVQCNRVFCDTMNSLTLPSE VNLCNIDIFNPKYDCKIMTSK TDVSSSVITSLGAIVSCYGKT KCTASNKNRGIIKTFSNGCDY VSNKGVDTVSVGNTLYYVNK QEGKSLYVKGEPIINFYDPLVF PSCEFCASISQVNEKINQSLAF IRKSDELLHNVEDKIEEILSKI YHIENEIARIKKLIGEANAGKS TTNIMITTIIIVIIVILLALIAVGL LLYCKARSTPVTLSKDQLSGI NNIAFSN （SEQ ID NO: 134） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGCt CtGGCAGCGCtAtCGCCtCtGGAAttGCCGtGtGtAAAGtG CtGCACCtGGAGGGCGAAGtGAACAAGAtCAAAAGCG CCCtGCtGAGCACCAACAAGGCtGtGGtGAGCCtGAGC AAtGGCGtGAGCGtGCtGACCttCAAGGtGCtGGACCtGA AAAACtACAttGACAAGCAGCtGCtCCCCAtCGtGAACA AGCAGAGCtGCAGCAtCagcAACAtCGAGACGGtGAtCG AGtttCAGCAGAAGAACAACCGGCtGCtGGAGAtCACC CGGGAGtttAGCGtGAACGCtGGCGtGACCACCCCAGtG AGCACCtAtAtGCtGACCAACAGCGAGCtGCtGAGCCtG AtCAACGACAtGCCCAtCACCAACGAtCAGAAGAAGCt GAtGAGCAGCAACGtGCAGAtCGtGAGGCAGCAGAGCt ACAGCAtCAtGtGCAtCAtCAAGGAGGAGGtGCtGGCCt ACGtGGtGCAGCtGCCtCtGtACGGAGtGAtCGACACCCC CtGttGGAAACtGCACACAtCtCCtCtGtGCACAACCAAC ACCAAGGAGGGCAGCAACAtCtGCCtGACAAGAACCG AtAGGGGCtGGtACtGtGACAAtGCCGGCtCtGtGtCtttCttt CCtCAGGCCGAGACAtGCAAGGtGCAGtGCAACCGGGt GttCtGCGACACCAtGAACAGCCtGACCCtGCCCAGCGA GGtCAACCtGtGCAACAtCGACAtCttCAACCCCAAGtAC GACtGCAAGAtCAtGACCAGCAAGACCGACGtGtCtAG CtCCGtGAtCACAAGCCtGGGAGCCAtCGtGAGCtGCtAC GGCAAGACCAAGtGCACCGCCAGCAACAAGAAtCGG GGCAtCAttAAGACCttCAGCAACGGCtGCGACtACGtGA GCAACAAGGGCGtGGAtACAGtGAGCGtGGGCAACAC CCtGtACtACGtGAAtAAGCAGGAGGGCAAGAGCCtGtA CGtGAAGGGCGAGCCCAtCAtCAACttCtACGACCCCCt GGtGtttCCCAGCtGCGAGttttGCGCCAGCAtCAGCCAGGt GAACGAGAAAAtCAAtCAGAGCCtGGCCttCAtCAGGA AGAGCGACGAGCtCctgcacaacgtggaagataaaatTGAAGAA AtTCtCtCCAAAAtttATCATAttGAAAATGAAAttGCtAGA Attaaaaaactcattggagaagct AACGCCGGCAAGAGCACCAC CAACAtCAtGAtCACCACCAtCAtCAtCGtGAtCAttGtGAt CCtGCtGgccctgatcgccgtgggcctgctgctgtactgcaaggctaggagcacc cctgtgaccctgagcaaggatcagctgagcggcatcaataacatcgccttcagcaac （SEQ ID NO: 135） |
| 012m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGG SGGGSGSAIASGIAVCKVLHL EGEVNKIKSALLSTNKAVVSL SNGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYHIENEIARIKKLIGE ANAGKSTTNIMITTIIIVIIVILL ALIAVGLLLYCKARSTPVTLS KDQLSGINNIAFSN （SEQ ID NO: 136） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAGGAtCtGGCGGAGGAtCtGGCAGCGCtAtCGCCtCtG GAAttGCCGtGtGtAAAGtGCtGCACCtGGAGGGCGAAGt GAACAAGAtCAAAAGCGCCCtGCtGAGCACCAACAAG GCtGtGGtGAGCCtGAGCAAtGGCGtGAGCGtGCtGACCtt CAAGGtGCtGGACCtGAAAAACtACAttGACAAGCAGCt GCtCCCCAtCGtGAACAAGCAGAGCtGCAGCAtCagcAA CAtCGAGACGGtGAtCGAGtttCAGCAGAAGAACAACC GGCtGCtGGAGAtCACCCGGGAGtttAGCGtGAACGCtG GCGtGACCACCCCAGtGAGCACCtAtAtGCtGACCAACA GCGAGCtGCtGAGCCtGAtCAACGACAtGCCCAtCACCA ACGAtCAGAAGAAGCtGAtGAGCAGCAACGtGCAGAtC GtGAGGCAGCAGAGCtACAGCAtCAtGtGCAtCAtCAAG GAGGAGGtGCtGGCCtACGtGGtGCAGCtGCCtCtGtACG GAGtGAtCGACACCCCCtGttGGAAACtGCACACAtCtCC tCtGtGCACAACCAACACCAAGGAGGGCAGCAACAtCt GCCtGACAAGAACCGAtAGGGGCtGGtACtGtGACAAtG CCGGCtCtGtGtCtttCtttCCtCAGGCCGAGACAtGCAAGGt GCAGtGCAACCGGGtGttCtGCGACACCAtGAACAGCCt GACCCtGCCCAGCGAGGtCAACCtGtGCAACAtCGACAt CttCAACCCCAAGtACGACtGCAAGAtCAtGACCAGCAA GACCGACGtGtCtAGCtCCGtGAtCACAAGCCtGGGAGC CAtCGtGAGCtGCtACGGCAAGACCAAGtGCACCGCCA GCAACAAGAAtCGGGGCAtCAttAAGACCttCAGCAAC GGCtGCGACtACGtGAGCAACAAGGGCGtGGAtACAGt GAGCGtGGGCAACACCCtGtACtACGtGAAtAAGCAGG AGGGCAAGAGCCtGtACGtGAAGGGCGAGCCCAtCAtC AACttCtACGACCCCCtGGtGtttCCCAGCtGCGAGttttGCG CCAGCAtCAGCCAGGtGAACGAGAAAAtCAAtCAGAG CCtGGCCttCAtCAGGAAGAGCGACGAGCtCctgcacaacgtg gaagataaaatTGAAGAAAtTCtCtCCAAAAtttATCATAttGAA AATGAAAttGCtAGAAttaaaaaactcattggagaagctAACGCCG GCAAGAGCACCACCAACAtCAtGAtCACCACCAtCAtC AtCGtGAtCAttGtGAtCCtGCtGgccctgatcgccgtgggcctgctgctg tactgcaaggctaggagcacccctgtgaccctgagcaaggatcagctgagcggcatc aataacatcgccttcagcaac （SEQ ID NO: 137） |
| 013m-Cys-P215S-1×GCN4t | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGSGGSGSAIASGIAVCKVLH LEGEVNKIKSALLSTNKAVVS LSNGVSVLTFKVLDLKNYIDK QLLPIVNKQSCSISNIETVIEFQ QKNNRLLEITREFSVNAGVTT PVSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYHIENEIARIKKLIGE ANAGKSTTNIMITTIIIVIIVILL ALIAVGLLLYCKARSTPVTLS KDQLSGINNIAFSN （SEQ ID NO: 138） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGA GGAtCtGGAGGAtCtGGAGGAtCtGGCAGCGCtAtCGCCt CtGGAAttGCCGtGtGtAAAGtGCtGCACCtGGAGGGCGA AGtGAACAAGAtCAAAAGCGCCCtGCtGAGCACCAAC AAGGCtGtGGtGAGCCtGAGCAAtGGCGtGAGCGtGCtG ACCttCAAGGtGCtGGACCtGAAAAACtACAttGACAAGC AGCtGCtCCCCAtCGtGAACAAGCAGAGCtGCAGCAtCa gcAACAtCGAGACGGtGAtCGAGtttCAGCAGAAGAACA ACCGGCtGCtGGAGAtCACCCGGGAGtttAGCGtGAACG CtGGCGtGACCACCCCAGtGAGCACCtAtAtGCtGACCA ACAGCGAGCtGCtGAGCCtGAtCAACGACAtGCCCAtCA CCAACGAtCAGAAGAAGCtGAtGAGCAGCAACGtGCA GAtCGtGAGGCAGCAGAGCtACAGCAtCAtGtGCAtCAtC AAGGAGGAGGtGCtGGCCtACGtGGtGCAGCtGCCtCtGt ACGGAGtGAtCGACACCCCCtGttGGAAACtGCACACAt CtCCtCtGtGCACAACCAACACCAAGGAGGGCAGCAA CAtCtGCCtGACAAGAACCGAtAGGGGCtGGtACtGtGAC AAtGCCGGCtCtGtGtCtttCtttCCtCAGGCCGAGACAtGCA AGGtGCAGtGCAACCGGGtGttCtGCGACACCAtGAACA GCCtGACCCtGCCCAGCGAGGtCAACCtGtGCAACAtCG ACAtCttCAACCCCAAGtACGACtGCAAGAtCAtGACCA GCAAGACCGACGtGtCtAGCtCCGtGAtCACAAGCCtGG GAGCCAtCGtGAGCtGCtACGGCAAGACCAAGtGCACC GCCAGCAACAAGAAtCGGGGCAtCAttAAGACCttCAG CAACGGCtGCGACtACGtGAGCAACAAGGGCGtGGAtA CAGtGAGCGtGGGCAACACCCtGtACtACGtGAAtAAGC AGGAGGGCAAGAGCCtGtACGtGAAGGGCGAGCCCAt CAtCAACttCtACGACCCCCtGGtGtttCCCAGCtGCGAGtttt GCGCCAGCAtCAGCCAGGtGAACGAGAAAAtCAAtCA GAGCCtGGCCttCAtCAGGAAGAGCGACGAGCtCctgcaca acgtggaagataaaatTGAAGAAAtTCtCtCCAAAAtttATCATAtt GAAAATGAAAttGCtAGAAttaaaaaactcattggagaagctAACG CCGGCAAGAGCACCACCAACAtCAtGAtCACCACCAtC AtCAtCGtGAtCAttGtGAtCCtGCtGgccctgatcgccgtgggcctgct gctgtactgcaaggctaggagcacccctgtgaccctgagcaaggatcagctgagcg gcatcaataacatcgccttcagcaac （SEQ ID NO: 139） |
| 003m-P215S- | MELPILKTNAITTILAAVTLCF | AtGGAGCtGCCCAtCCtGAAGACCAAtGCCAttACAACC |
| 1×Fibritin | ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLSAIGGYIP EAPRDGQAYVRKDGEWVLLS TFLGGLVPRHNVNAGKSTTNI MITTIIIVIIVILLALIAVGLLLY CKARSTPVTLSKDQLSGINNI AFSN （SEQ ID NO: 141） | AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgAGCGCC ATCGGCGGCTACATCCCCGAGGCCCCCAGAGACGGC CAGGCCTACGTGAGGAAGGACGGCGAGTGGGTGCT GCTGAGCACCTTCCTGGGCGGCCTGGTGCCCAGAcac aacgtgAACGCCGGCAAGAGCACCACCAACAtCAtGAtC ACCACCAtCAtCAtCGtGAtCAttGtGAtCCtGCtGgccctgatcg ccgtgggcctgctgctgtactgcaaggctaggagcacccctgtgaccctgagcaagg atcagctgagcggcatcaataacatcgccttcagcaac （SEQ ID NO: 142） |
| 003m-P215S-1×Fibritin-1N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLSAIGGYIP EAPNDTQAYVRKDGEWVLLS TFLGGLVPRHNVNAGKSTTNI MITTIIIVIIVILLALIAVGLLLY CKARSTPVTLSKDQLSGINNI AFSN（SEQ ID NO: 145） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgAGCGCC ATCGGCGGCTACATCCCCGAGGCCCCCaacGAcaccCA GGCCTACGTGAGGAAGGACGGCGAGTGGGTGCTGC TGAGCACCTTCCTGGGCGGCCTGGTGCCCAGAcacaac gtgAACGCCGGCAAGAGCACCACCAACAtCAtGAtCAC CACCAtCAtCAtCGtGAtCAttGtGAtCCtGCtGgccctgatcgccg tgggcctgctgctgtactgcaaggctaggagcacccctgtgaccctgagcaaggatc agctgagcggcatcaataacatcgccttcagcaac （SEQ ID NO: 146） |
| 003m-P215S-1×GCN4t-1N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI ENITSKIYHIENEIARIKKLIGE ANAGKSTTNIMITTIIIVIIVILL ALIAVGLLLYCKARSTPVTLS KDQLSGINNIAFSN （SEQ ID NO: 147） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAaacAtTacctCCAAAAtttATCATAttGAAAA TGAAAttGCtAGAAttaaaaaactcattggagaagctAACGCCGGC AAGAGCACCACCAACAtCAtGAtCACCACCAtCAtCAtC GtGAtCAttGtGAtCCtGCtGgccctgatcgccgtgggcctgctgctgtact gcaaggctaggagcacccctgtgaccctgagcaaggatcagctgagcggcatcaat aacatcgccttcagcaac （SEQ ID NO: 148） |
| 003m-P215S-1×GCN4t-2N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYNITNEIARIKKLIGE ANAGKSTTNIMITTIIIVIIVILL ALIAVGLLLYCKARSTPVTLS KDQLSGINNIAFSN （SEQ ID NO: 149） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAGAAAtTCtCtCCAAAAtttATaacAttaccAAT GAAAttGCtAGAAttaaaaaactcattggagaagctAACGCCGGCA AGAGCACCACCAACAtCAtGAtCACCACCAtCAtCAtCG tGAtCAttGtGAtCCtGCtGgccctgatcgccgtgggcctgctgctgtactgc aaggctaggagcacccctgtgaccctgagcaaggatcagctgagcggcatcaataa catcgccttcagcaac （SEQ ID NO: 150） |
| 003m-P215S-1×GCN4t-3N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYHIENEIANITKLIGE ANAGKSTTNIMITTIIIVIIVILL ALIAVGLLLYCKARSTPVTLS KDQLSGINNIAFSN （SEQ ID NO: 151） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAGAAAtTCtCtCCAAAAtttATCATAttGAA AATGAAAttGCtaacAttaccaaactcattggagaagctAACGCCGG CAAGAGCACCACCAACAtCAtGAtCACCACCAtCAtCAt CGtGAtCAttGtGAtCCtGCtGgccctgatcgccgtgggcctgctgctgtac tgcaaggctaggagcacccctgtgaccctgagcaaggatcagctgagcggcatcaat aacatcgccttcagcaac （SEQ ID NO: 152） |
| 003m-P215S-1×GCN4t-1, 2N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI ENITSKIYNITNEIARIKKLIGE ANAGKSTTNIMITTIIIVIIVILL ALIAVGLLLYCKARSTPVTLS KDQLSGINNIAFSN （SEQ ID NO: 153） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAaacAtTacctCCAAAAtttATaacAttaccAATG AAAttGCtAGAAttaaaaaactcattggagaagctAACGCCGGCAA GAGCACCACCAACAtCAtGAtCACCACCAtCAtCAtCGt GAtCAttGtGAtCCtGCtGgccctgatcgccgtgggcctgctgctgtactgca aggctaggagcacccctgtgaccctgagcaaggatcagctgagcggcatcaataac atcgccttcagcaac （SEQ ID NO: 154） |
| 003m-P215S-1×GCN4t-1,3N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI ENITSKIYHIENEIANITKLIGE ANAGKSTTNIMITTIIIVIIVILL ALIAVGLLLYCKARSTPVTLS KDQLSGINNIAFSN （SEQ ID NO: 155） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAaacAtTacctCCAAAAtttATCATAttGAAAA TGAAAttGCtaacAttaccaaactcattggagaagctAACGCCGGCA AGAGCACCACCAACAtCAtGAtCACCACCAtCAtCAtCG tGAtCAttGtGAtCCtGCtGgccctgatcgccgtgggcctgctgctgtactgc aaggctaggagcacccctgtgaccctgagcaaggatcagctgagcggcatcaataa catcgccttcagcaac （SEQ ID NO: 156） |
| 003m-P215S-1×GCN4t-2,3N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI EEILSKIYNITNEIANITKLIGE ANAGKSTTNIMITTIIIVIIVILL ALIAVGLLLYCKARSTPVTLS KDQLSGINNIAFSN （SEQ ID NO: 157） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAAtCAAtCAGAGC CtGGCCttCAtCAGGAAGAGCGACGAGCtCctgcacaacgtgg aagataaaatTGAAGAAAtTCtCtCCAAAAtttATaacAttaccAAT GAAAttGCtaacAttaccaaactcattggagaagctAACGCCGGCAA GAGCACCACCAACAtCAtGAtCACCACCAtCAtCAtCGt GAtCAttGtGAtCCtGCtGgccctgatcgccgtgggcctgctgctgtactgca aggctaggagcacccctgtgaccctgagcaaggatcagctgagcggcatcaataac atcgccttcagcaac （SEQ ID NO: 158） |
| 003m-P215S-1×GCN4t-1,2,3N | MELPILKTNAITTILAAVTLCF ASSQNITEEFYQSTCSAVSKG YLGALRTGWYTSVITIELSNIK ENKCNGTDAKVKLIKQELDK YKNAVTDLQLLMQSTPCGGS GGGSGSAIASGIAVCKVLHLE GEVNKIKSALLSTNKAVVSLS NGVSVLTFKVLDLKNYIDKQ LLPIVNKQSCSISNIETVIEFQQ KNNRLLEITREFSVNAGVTTP VSTYMLTNSELLSLINDMPIT NDQKKLMSSNVQIVRQQSYSI MCIIKEEVLAYVVQLPLYGVI DTPCWKLHTSPLCTTNTKEGS NICLTRTDRGWYCDNAGSVS FFPQAETCKVQCNRVFCDTM NSLTLPSEVNLCNIDIFNPKYD CKIMTSKTDVSSSVITSLGAIV SCYGKTKCTASNKNRGIIKTF SNGCDYVSNKGVDTVSVGNT LYYVNKQEGKSLYVKGEPIIN FYDPLVFPSCEFCASISQVNEK INQSLAFIRKSDELLHNVEDKI ENITSKIYNITNEIANITKLIGE ANAGKSTTNIMITTIIIVIIVILL ALIAVGLLLYCKARSTPVTLS KDQLSGINNIAFSN （SEQ ID NO: 143） | AtGGAGCtGCCCAtCCtGAAGACCAAtGCCAttACAACC AtCCtGGCCGCCGtGACACtGtGtttCGCCAGCtCtCAGAA CAtCACCGAGGAAttttACCAGAGCACCtGCAGCGCCGt GAGCAAGGGCtACCtGGGCGCCCtGAGGACCGGCtGGt ACACAAGCGtGAtCACCAtCGAGCtGAGCAACAtCAAA GAAAACAAGtGCAACGGCACAGACGCCAAAGtGAAG CtGAtCAAGCAGGAGCtGGACAAGtACAAGAACGCCGt GACtGACCtGCAGCtGCtGAtGCAGtCAACACCttGtGGC GGAtCtGGCGGAGGAtCtGGCAGCGCtAtCGCCtCtGGAA ttGCCGtGtGtAAAGtGCtGCACCtGGAGGGCGAAGtGAA CAAGAtCAAAAGCGCCCtGCtGAGCACCAACAAGGCt GtGGtGAGCCtGAGCAAtGGCGtGAGCGtGCtGACCttCA AGGtGCtGGACCtGAAAAACtACAttGACAAGCAGCtGC tCCCCAtCGtGAACAAGCAGAGCtGCAGCAtCagcAACAt CGAGACGGtGAtCGAGtttCAGCAGAAGAACAACCGGC tGCtGGAGAtCACCCGGGAGtttAGCGtGAACGCtGGCGt GACCACCCCAGtGAGCACCtAtAtGCtGACCAACAGCG AGCtGCtGAGCCtGAtCAACGACAtGCCCAtCACCAACG AtCAGAAGAAGCtGAtGAGCAGCAACGtGCAGAtCGtG AGGCAGCAGAGCtACAGCAtCAtGtGCAtCAtCAAGGA GGAGGtGCtGGCCtACGtGGtGCAGCtGCCtCtGtACGGA GtGAtCGACACCCCCtGttGGAAACtGCACACAtCtCCtCt GtGCACAACCAACACCAAGGAGGGCAGCAACAtCtGC CtGACAAGAACCGAtAGGGGCtGGtACtGtGACAAtGCC GGCtCtGtGtCtttCtttCCtCAGGCCGAGACAtGCAAGGtGC AGtGCAACCGGGtGttCtGCGACACCAtGAACAGCCtGA CCCtGCCCAGCGAGGtCAACCtGtGCAACAtCGACAtCtt CAACCCCAAGtACGACtGCAAGAtCAtGACCAGCAAG ACCGACGtGtCtAGCtCCGtGAtCACAAGCCtGGGAGCC AtCGtGAGCtGCtACGGCAAGACCAAGtGCACCGCCAG CAACAAGAAtCGGGGCAtCAttAAGACCttCAGCAACG GCtGCGACtACGtGAGCAACAAGGGCGtGGAtACAGtG AGCGtGGGCAACACCCtGtACtACGtGAAtAAGCAGGA GGGCAAGAGCCtGtACGtGAAGGGCGAGCCCAtCAtCA ACttCtACGACCCCCtGGtGtttCCCAGCtGCGAGttttGCGC CAGCAtCAGCCAGGtGAACGAGAAAtCAAtCAGAGC CtGGCCttCAtCAGGAAGAGCGACGAGCtCctgcacaacgtgg aagataaaatTGAAaacAtTacctCCAAAAtttATaacAttaccAATG AAAttGCtaacAttaccaaactcattggagaagctAACGCCGGCAAG AGCACCACCAACAtCAtGAtCACCACCAtCAtCAtCGtG AtCAttGtGAtCCtGCtGgccctgatcgccgtgggcctgctgctgtactgcaa ggctaggagcacccctgtgaccctgagcaaggatcagctgagcggcatcaataacat cgccttcagcaac （SEQ ID NO: 144） |
| WT-RQ1 | MELLIHRSSAIFLTLAINALYL TSSQNITEEFYQSTCSAVSRGY LSALRTGWYTSVITIELSNIKE TKCNGTDTKVKLIKQELDKY KNAVTELQLLMQNTPAVNNR ARREAPQYMNYTINTTKNLN VSISKKRKRRFLGFLLGVGSA IASGIAVSKVLHLEGEVNKIK NALQLTNKAVVSLSNGVSVLT SKVLDLKNYINNQLLPIVNQQ SCRISNIETVIEFQQKNSRLLEI TREFSVNAGVTTPLSTYMLTN SELLSLINDMPITNDQKKLMS SNVQIVRQQSYSIMSIIKEEVL AYVVQLPIYGVIDTPCWKLHT SPLCTTNIKEGSNICLTRTDRG WYCDNAGSVSFFPQADTCKV QSNRVFCDTMNSLTLPSEVSL CNTDIFNSKYDCKIMTSKTDI SSSVITSLGAIVSCYGKTKCTA SNKNRGIIKTFSNGCDYVSNK GVDTVSVGNTLYYVNKLEGK NLYVKGEPIINYYDPLVFPSDE FDASISQVNEKINQSLAFIRRS DELLHNVNTGKSTTNIMITAII IVIIVVLLSLIAIGLLLYCKAKN TPVTLSKDQLSGINNIAFSK （SEQ ID NO: 80） | ATGGAGCTGCTGATCCACAGAAGCAGCGCCATCTTC CTGACCCTGGCCATCAATGCCCTGTATCTGACCAGCA GCCAGAACATCACCGAGGAGTTTTACCAGAGCACAT GCAGCGCCGTGAGCAGGGGCTATCTGAGCGCCCTGA GGACCGGCTGGTACACAAGCGTGATCACCATTGAGC TGAGCAATATCAAGGAGACAAAGTGCAACGGCACC GACACCAAGGTGAAGCTGATCAAGCAGGAGCTGGA CAAGTACAAGAACGCCGTGACCGAGCTGCAACTGCT GATGCAGAATACCCCTGCCGTGAACAACCGGGCCCG GCGGGAGGCCCCCCAGTACATGAATTACACCATCAA CACCACCAAGAACCTGAACGTGAGCATTAGCAAGA AGAGGAAGAGGAGATTCCTGGGCTTCCTGCTGGGAG TGGGATCTGCTATCGCCTCTGGCATTGCCGTGTCTAA AGTGCTGCACCTGGAGGGCGAGGTGAACAAGATCA AGAACGCTCTGCAGCTGACCAATAAGGCCGTCGTGA GCCTGAGCAACGGCGTGAGCGTGCTGACAAGCAAG GTGCTGGACCTGAAGAATTACATCAACAACCAGCTG CTGCCCATCGTGAACCAGCAGAGCTGCCGGATCAGC AACATCGAAACCGTGATCGAGTTTCAGCAGAAGAAC AGCCGGCTGCTGGAGATCACCAGGGAGTTTAGCGTG AACGCCGGCGTGACAACCCCTCTGAGCACCTACATG CTGACCAACAGCGAGCTGCTGTCTCTGATCAACGAC ATGCCCATCACCAACGACCAGAAGAAGCTGATGAGC AGCAACGTGCAGATCGTGCGGCAGCAAAGCTACAG CATCATGAGCATCATCAAGGAGGAGGTGCTGGCCTA CGTGGTGCAGCTGCCAATTTACGGCGTGATCGACAC CCCCTGCTGGAAGCTGCACACCTCTCCCCTGTGTAC CACAAACATCAAAGAGGGCAGCAACATTTGTCTGAC CAGAACCGACAGAGGCTGGTACTGTGACAATGCCGG CAGCGTGAGCTTTTTCCCTCAGGCCGATACCTGCAA GGTGCAGAGCAATAGGGTGTTCTGCGACACCATGAA CAGCCTGACCCTCCCCAGCGAGGTGAGCCTGTGCAA CACCGACATCTTCAACAGCAAGTACGACTGCAAGAT CATGACCAGCAAGACCGACATCAGCAGCAGCGTGAT TACCTCTCTGGGAGCCATCGTGAGCTGCTACGGCAA GACCAAGTGTACCGCCAGCAACAAGAACCGGGGCA TCATTAAGACCTTCAGCAACGGCTGCGACTACGTGA GCAACAAGGGCGTGGACACAGTGAGCGTGGGCAAC ACCCTGTACTACGTGAACAAGCTGGAGGGCAAGAAT CTGTACGTGAAGGGCGAGCCCATCATCAACTACTAC GACCCCCTGGTGTTTCCTAGCGACGAGTTTGACGCC AGCATCAGCCAGGTGAATGAGAAGATCAACCAGAGC CTGGCCTTCATCAGGCGGAGCGACGAGCTGCTCCAC AACGTGAACACCGGCAAGAGCACCACCAACATCATG ATCACCGCCATCATCATCGTGATTATCGTGGTGCTGCT GAGCCTGATCGCCATTGGCCTGCTGCTGTACTGCAA GGCCAAGAATACACCCGTGACCCTGAGCAAGGATCA GCTGAGCGGCATCAATAACATCGCCTTCTCCAAG （SEQ ID NO: 119） |
| WT-RQ2 | (SEQ ID NO: 80) | ATGGAGCTGCTGATCCACAGATCCAGCGCCATTTTCC TGACCCTGGCCATCAATGCCCTGTATCTGACCAGCAG CCAGAACATCACCGAGGAGTTTTACCAGAGCACATG CAGCGCCGTGAGCAGGGGCTATCTGAGCGCCCTGAG GACCGGCTGGTACACTAGCGTGATCACCATTGAGCT GAGCAATATCAAGGAGACAAAGTGCAACGGCACCG ACACCAAGGTGAAGCTGATCAAGCAGGAGCTGGAC AAGTACAAGAACGCCGTGACCGAGCTGCAACTGCT GATGCAGAATACTCCCGCCGTGAACAACCGGGCCAG GCGGGAGGCCCCCCAGTACATGAATTACACCATCAA CACCACCAAGAACCTGAACGTGAGCATTTCCAAGAA GAGGAAGAGGAGATTCCTGGGCTTCCTGCTGGGAGT GGGATCTGCAATCGCCTCTGGCATTGCCGTGTCTAAA GTGCTGCACCTGGAGGGCGAGGTGAACAAGATCAA GAACGCTCTGCAGCTGACCAATAAGGCCGTCGTGAG CCTGAGCAACGGCGTGAGCGTGCTGACAAGCAAGG TGCTGGACCTGAAGAATTACATCAACAACCAGCTGC TGCCCATCGTGAACCAGCAGAGCTGCCGGATCAGCA ACATCGAAACCGTGATCGAGTTTCAGCAGAAGAACA GCCGGCTGCTGGAGATCACCAGGGAGTTTAGCGTGA ACGCTGGCGTGACTACCCCTCTGAGCACCTACATGC TGACCAACAGCGAGCTGCTGTCCCTGATCAACGACA TGCCCATCACCAACGACCAGAAGAAGCTGATGAGCA GCAACGTGCAGATCGTGAGGCAGCAAAGCTACAGC ATCATGAGCATCATCAAGGAGGAGGTGCTGGCCTAC GTGGTGCAGCTGCCCATTTACGGCGTGATCGACACC CCCTGCTGGAAACTGCACACCTCTCCCCTGTGCACC ACAAACATCAAGGAAGGCAGCAACATTTGCCTGACA AGGACCGATAGAGGCTGGTACTGTGACAATGCCGGC AGCGTGAGCTTTTTCCCTCAGGCCGATACCTGCAAG GTGCAGAGCAATCGGGTGTTCTGCGACACCATGAAC AGCCTGACCCTCCCCAGCGAGGTGAGCCTGTGCAAC ACCGACATCTTCAACAGCAAGTACGACTGCAAGATC ATGACCAGCAAGACCGATATCAGCAGCAGCGTCATT ACCAGCCTGGGAGCCATCGTGAGCTGCTACGGCAAG ACCAAGTGTACCGCCAGCAACAAGAACCGGGGCAT CATTAAGACCTTCAGCAACGGCTGCGACTACGTGAG CAACAAGGGCGTGGACACAGTGAGCGTGGGCAACA CCCTGTACTACGTGAACAAGCTGGAAGGCAAGAATC TGTACGTGAAGGGCGAGCCCATCATCAACTACTACG ACCCCCTGGTGTTCCCTAGCGACGAGTTTGACGCCA GCATCAGCCAGGTGAACGAAAAGATCAACCAGAGC CTGGCCTTCATCAGGCGGAGCGACGAGCTGCTCCAC AACGTGAACACCGGCAAGAGCACCACCAACATCATG ATCACCGCCATCATCATCGTGATTATCGTGGTGCTGCT GAGCCTGATTGCCATCGGCCTGCTGCTGTACTGTAAG GCCAAGAACACACCCGTGACCCTGAGCAAGGATCA GCTGAGCGGCATCAATAACATCGCCTTCTCCAAG （SEQ ID NO: 90） |
| WT-RQ3 | (SEQ ID NO: 80) | ATGGAGCTGCTGATCCACAGGTCCAGCGCCATTTTCC TGACCCTGGCCATCAATGCCCTGTATCTGACCAGCAG CCAGAACATCACCGAGGAGTTTTACCAGAGCACATG CAGCGCCGTGAGCAGGGGCTACCTGAGCGCCCTGA GGACCGGCTGGTACACCTCCGTGATTACTATTGAGCT GTCCAATATCAAGGAGACAAAGTGCAACGGGACCG ACACCAAGGTGAAGCTCATCAAGCAGGAGCTGGAC AAGTACAAGAACGCCGTGACCGAGCTGCAGTTGCTG ATGCAGAACACTCCCGCCGTGAACAACCGCGCCAGA CGGGAGGCCCCCCAGTACATGAATTACACCATCAAC ACCACCAAGAACCTGAACGTGAGCATCTCCAAGAA GAGAAAGAGGAGATTCCTGGGCTTTTTACTGGGCGT GGGCAGCGCCATCGCCAGTGGAATTGCCGTGTCCAA AGTGCTGCACCTGGAGGGCGAGGTGAACAAGATCA AGAACGCACTGCAGCTGACCAATAAGGCCGTGGTGA GCCTGAGCAACGGCGTGAGCGTGCTGACAAGCAAG GTGCTCGACCTGAAGAATTACATCAACAACCAGCTG CTGCCCATCGTGAACCAGCAGAGCTGCCGGATCAGC AACATCGAAACCGTGATCGAGTTTCAGCAGAAAAAC TCTCGGCTCCTGGAGATCACCCGGGAGTTTAGCGTG AACGCCGGGGTGACCACCCCACTGAGCACCTACATG CTGACCAACTCCGAGCTGCTGTCCCTGATCAACGAC ATGCCCATCACCAACGACCAGAAGAAACTTATGAGC AGCAACGTGCAGATCGTGCGGCAGCAAAGCTACAG CATCATGTCCATCATCAAGGAGGAGGTGCTGGCCTAC GTGGTGCAGCTGCCCATTTACGGGGTGATCGACACC CCCTGCTGGAAACTGCACACCTCTCCCCTGTGCACC ACAAACATCAAGGAAGGCAGCAATATTTGCCTCACT AGGACCGACAGAGGCTGGTACTGTGACAATGCCGGC AGCGTGAGCTTTTTCCACAGGCCGACACCTGCAAG GTGCAGAGCAATCGGGTGTTCTGTGACACCATGAAC AGCCTGACCCTCCCCAGCGAGGTGAGCCTCTGCAAC ACCGACATCTTCAACAGCAAGTACGACTGCAAGATC ATGACCAGCAAGACCGATATCAGCAGCAGCGTCATT ACCAGCCTGGGCGCCATCGTGAGCTGCTACGGCAAG ACCAAATGTACCGCCAGCAACAAGAACCGGGGCATC ATTAAGACCTTCAGCAACGGCTGCGACTACGTGAGC AACAAGGGCGTGGACACAGTGAGCGTGGGCAACAC CCTGTACTACGTGAACAAGCTGGAAGGCAAGAATCT GTACGTGAAGGGCGAGCCCATCATTAACTACTACGA CCCCCTGGTGTTCCCTTCCGACGAGTTTGACGCCTCC ATCAGCCAGGTGAACGAGAAGATCAACCAATCCCTG GCCTTCATCCGGCGGAGCGACGAGCTGCTCCACAAC GTGAACACCGGCAAGAGCACCACCAACATCATGATC ACAGCCATCATCATCGTGATTATCGTGGTGCTGCTGA GCCTGATCGCCATCGGCCTGCTGCTGTATTGTAAGGC CAAGAACACACCCGTGACCCTGAGCAAGGATCAGCT GAGCGGCATCAATAACATCGCCTTCTCCAAG （SEQ ID NO: 91） |
| RSV B-003m-P215S-1×GCN4t_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPCGGSG GGSGSAIASGIAVCKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQCNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSCEFCASISQVNEKIN QSLAFIRRSDELLHNVEDKIEE ILSKIYHIENEIARIKKLIGEAN TGKSTTNIMITAIIIVIIVVLLSL IAIGLLLYCKAKNTPVTLSKD QLSGINNIAFSK （SEQ ID NO: 81） | ATGGAGCTGCTGATCCACAGAAGCAGCGCCATCTTT CTGACCCTGGCCATCAACGCTCTGTATCTGACCTCTA GCCAGAACATCACCGAGGAGTTTTACCAGAGCACCT GCAGCGCCGTGAGCAGGGGCTATCTGGGAGCCCTGA GGACCGGCTGGTACACCAGCGTGATCACCATCGAGC TGAGCAATATCAAAGAGACAAAGTGCAACGGCACC GACACCAAGGTCAAGCTGATCAAGCAGGAGCTGGA CAAGTACAAGAACGCTGTGACCGATCTGCAGCTCCT GATGCAGAATACCCCTTGTGGAGGCTCTGGAGGAGG CAGCGGCTCAGCTATCGCCTCTGGAATCGCCGTGTG CAAGGTGCTGCACCTGGAAGGAGAGGTGAATAAGAT CAAGAATGCCCTGCAGCTGACCAACAAGGCCGTGGT GAGCCTGAGCAATGGCGTGTCTGTGCTGACCTTCAA GGTGCTCGACCTGAAAAACTACATCAACAACCAGCT GCTGCCCATCGTGAACCAGCAGAGCTGCCGGATCAG CAACATCGAAACCGTGATCGAGTTTCAGCAGAAGAA CAGCCGGCTGCTGGAGATTACCCGGGAGTTTAGCGT GAACGCCGGCGTGACAACCCCCCTGAGCACCTACAT GCTGACCAATAGCGAGCTGCTGTCCCTGATCAACGA CATGCCCATCACCAACGACCAGAAAAAGCTGATGAG CAGCAACGTGCAGATCGTGCGGCAACAGAGCTACA GCATCATGTGCATCATCAAGGAGGAGGTGCTGGCCT ACGTGGTGCAGCTGCCTATCTACGGCGTGATCGACA CCCCCTGCTGGAAGCTGCACACAAGCCCTCTGTGCA CAACCAACATTAAGGAGGGCTCTAACATCTGCCTGA CCAGAACCGATAGAGGATGGTACTGCGACAACGCCG GAAGCGTGAGCTTTTTCCCTCAGGCCGATACCTGTA AGGTGCAGTGCAATCGGGTGTTCTGCGACACCATGA ACAGCCTGACCCTGCCCAGCGAGGTGTCCCTGTGCA ACACCGACATCTTCAACAGCAAGTACGACTGCAAGA TCATGACCAGCAAGACCGACATCAGCAGCAGCGTGA TTACCAGCCTGGGCGCTATCGTGAGCTGCTACGGCA AGACCAAGTGTACCGCCAGCAATAAGAACCGGGGC ATCATTAAGACCTTCAGCAACGGCTGCGACTACGTG AGCAACAAGGGCGTGGACACCGTGAGCGTGGGAAA CACCCTGTACTACGTGAATAAGCTGGAGGGCAAGAA CCTGTACGTGAAGGGCGAGCCCATCATCAACTACTA CGACCCCCTGGTGTTCCCCAGCTGTGAGTTTTGCGC CTCTATCAGCCAGGTGAACGAGAAGATCAATCAGAG CCTGGCCTTCATCCGGAGGAGCGACGAGCTGCTCCA CAACGTGGAGGACAAGATCGAGGAGATCCTGAGCA AGATCTACCACATCGAGAACGAGATCGCCCGGATCA AGAAGCTGATCGGCGAGGCCAACACCGGCAAGAGC ACCACCAACATCATGATCACCGCCATCATCATCGTGA TCATCGTGGTGCTGCTGTCTCTGATCGCCATCGGCCT GCTGCTGTACTGCAAGGCCAAGAATACACCCGTGAC CCTGAGCAAAGACCAGCTGAGCGGCATCAACAATAT CGCCTTCTCCAAG （SEQ ID NO: 92） |
| RSV B-003m-P215S-1×GCN4t_RQ2 | (SEQ ID NO: 81) | ATGGAGCTGCTGATCCACAGATCCAGCGCCATCTTTC TGACCCTGGCCATCAATGCCCTGTATCTGACCTCTAG CCAGAACATTACCGAGGAGTTTTACCAGAGCACCTG CAGCGCCGTGAGCAGGGGCTATCTGGGAGCCCTGAG GACCGGCTGGTACACCAGCGTGATCACCATCGAGCT GAGCAATATCAAAGAGACAAAGTGCAACGGCACCG ACACCAAGGTCAAGCTGATCAAGCAGGAGCTGGAC AAGTACAAGAATGCTGTGACCGATCTGCAGCTCCTG ATGCAGAATACCCCTTGTGGAGGCTCTGGAGGAGGC AGCGGCAGCGCTATCGCCTCTGGAATCGCCGTGTGC AAGGTGCTGCACCTGGAAGGAGAGGTGAATAAGATC AAGAATGCCCTGCAGCTGACCAACAAGGCCGTGGTG AGCCTGAGCAATGGCGTGTCTGTGCTGACCTTCAAG GTGCTCGACCTGAAAAACTACATCAACAACCAGCTG CTGCCCATCGTGAACCAGCAGAGCTGCCGGATCAGC AACATCGAAACCGTGATCGAGTTTCAGCAGAAGAAC AGCCGGCTGCTGGAGATTACCCGGGAGTTTAGCGTG AACGCCGGCGTGACTACCCCCCTGAGCACCTACATG CTGACCAATAGCGAGCTGCTGTCCCTGATCAACGAC ATGCCCATCACCAACGACCAGAAAAAGCTGATGAGC AGCAACGTGCAGATCGTGCGGCAACAGAGCTACAG CATCATGTGCATCATCAAGGAGGAGGTGCTGGCCTA CGTGGTGCAGCTGCCTATCTACGGCGTGATCGACAC ACCCTGCTGGAAGCTGCACACAAGCCCCCTGTGTAC TACCAACATTAAGGAGGGCTCTAACATCTGCCTGACC AGAACCGATAGAGGCTGGTACTGCGACAACGCCGG AAGCGTGAGCTTTTTCCCTCAGGCTGATACCTGTAAG GTGCAGTGCAATCGGGTGTTCTGCGACACCATGAAC AGCCTGACCCTGCCCAGCGAGGTGTCCCTGTGCAAC ACCGACATCTTCAACAGCAAGTACGACTGCAAGATC ATGACCAGCAAGACCGACATCAGCAGCAGCGTGATT ACCAGCCTGGGCGCCATCGTGAGCTGCTACGGCAAG ACCAAGTGTACCGCCAGCAATAAGAACCGGGGCATC ATTAAGACCTTCAGCAACGGCTGCGACTACGTGAGC AACAAGGGCGTGGACACCGTGAGCGTGGGAAACAC CCTGTACTACGTGAATAAGCTGGAGGGCAAGAACCT GTACGTGAAGGGCGAGCCCATCATCAACTACTACGA CCCCCTGGTGTTCCCCAGCTGTGAGTTTTGCGCCTCT ATCAGCCAGGTGAACGAGAAGATCAACCAGAGCCT GGCCTTCATTCGGAGGAGCGACGAGCTGCTCCACAA CGTGGAGGACAAGATCGAGGAGATCCTGAGCAAGAT CTACCACATCGAGAACGAGATCGCCCGCATCAAGAA GCTGATCGGCGAGGCCAACACCGGCAAGAGCACCA CCAACATCATGATCACCGCCATCATCATCGTGATCATC GTGGTGCTGCTGTCTCTGATCGCCATCGGCCTGCTGC TGTACTGCAAGGCCAAGAATACACCCGTGACCCTGA GCAAAGACCAGCTGAGCGGCATCAATAATATCGCCT TCTCCAAG （SEQ ID NO: 93） |
| RSV B-003m-P215S-1×GCN4t_RQ3 | (SEQ ID NO: 81) | ATGGAGCTGCTGATTCACAGAAGCTCCGCCATCTTTC TGACCCTGGCCATTAATGCCCTGTACCTGACCTCTAG CCAGAACATCACCGAGGAGTTTTACCAGAGCACCTG CAGCGCCGTGAGCAGGGGCTACCTGGGAGCCCTGA GGACCGGCTGGTATACCTCCGTGATCACCATCGAGCT GAGCAATATCAAGGAAACTAAATGCAACGGCACCGA CACCAAGGTCAAGCTGATCAAGCAGGAGCTGGACA AGTACAAGAATGCAGTGACCGATCTGCAGCTCCTGA TGCAGAATACCCCCTGTGGAGGCTCTGGAGGAGGCT CCGGCTCTGCTATCGCCTCTGGAATCGCCGTGTGCAA GGTGCTGCACCTGGAGGGAGAGGTGAATAAGATTAA GAACGCTCTGCAGCTGACCAACAAGGCTGTGGTGA GCCTGAGCAATGGCGTGAGTGTGCTGACCTTCAAGG TGCTCGACCTGAAAAACTACATCAACAACCAGCTGC TGCCCATCGTGAACCAGCAGAGCTGCCGGATCAGCA ACATCGAAACCGTGATCGAGTTTCAGCAGAAGAATA GCCGGCTCCTGGAGATTACCCGGGAGTTTAGCGTGA ACGCCGGGGTGACCACCCCCCTGAGCACCTACATGC TGACCAATTCCGAGCTGCTGTCCCTCATCAATGACAT GCCCATCACCAACGACCAGAAAAAGCTGATGAGCA GCAACGTGCAGATCGTGCGGCAGCAAAGCTACAGC ATCATGTGCATCATCAAGGAGGAGGTGCTGGCCTAC GTGGTGCAGCTGCCAATCTACGGCGTGATCGACACT CCCTGCTGGAAGCTGCACACCAGCCCCCTGTGCACA ACCAACATTAAGGAGGGGTCTAATATTTGCCTGACCA GGACAGACAGAGGCTGGTACTGCGACAACGCCGGA AGCGTGAGCTTTTTCCCACAGGCTGATACCTGTAAG GTCCAGTGCAATCGCGTGTTCTGCGACACCATGAAC AGCCTGACCCTGCCCAGCGAGGTGAGCCTCTGCAAC ACCGACATCTTCAACTCCAAGTACGACTGCAAGATC ATGACCAGCAAGACCGACATCAGCAGCAGCGTGATT ACCAGCCTGGGCGCCATCGTGAGCTGTTACGGCAAG ACCAAGTGTACCGCCAGCAATAAGAACCGGGGCATC ATTAAGACCTTCAGCAACGGCTGCGACTACGTGAGC AACAAGGGGGTGGACACCGTGAGCGTGGGAAACAC CCTGTACTACGTGAATAAGCTGGAGGGCAAGAACCT GTACGTGAAGGGGGAGCCCATCATCAACTACTACGA CCCCCTGGTGTTCCCCAGCTGCGAGTTTTGCGCTTCT ATTTCCCAGGTGAACGAGAAGATCAACCAGAGCCTG GCCTTCATCCGGAGGAGCGACGAGCTGCTCCACAAC GTGGAGGACAAGATCGAGGAGATCCTGAGCAAGATC TACCACATCGAGAACGAGATCGCCCGGATCAAGAAG CTGATCGGCGAGGCCAACACCGGCAAGAGCACCAC CAACATCATGATCACCGCCATCATCATCGTGATCATC GTGGTGTTGCTGAGCCTGATTGCCATCGGCCTGCTGC TGTACTGCAAGGCCAAGAACACACCCGTGACCCTGA GCAAAGACCAGCTGAGCGGCATCAACAATATCGCCT TCTCCAAG （SEQ ID NO: 94） |
| RSV B-003m-P215S-1×GCN4t-G46S_RQ1 | MELLIHRSSAIFLTLAINALYL TSSQNITEEFYQSTCSAVSRGY LSALRTGWYTSVITIELSNIKE TKCNGTDTKVKLIKQELDKY KNAVTDLQLLMQNTPCGGSG GGSGSAIASGIAVCKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQCNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSCEFCASISQVNEKIN QSLAFIRRSDELLHNVEDKIEE ILSKIYHIENEIARIKKLIGEAN TGKSTTNIMITAIIIVIIVVLLSL IAIGLLLYCKAKNTPVTLSKD QLSGINNIAFSK （SEQ ID NO: 82） | ATGGAGCTGCTGATCCACAGAAGCAGCGCCATCTTT CTGACACTGGCCATCAATGCCCTGTACCTGACCAGC AGCCAGAACATCACCGAGGAGTTTTATCAGAGCACC TGCAGCGCCGTGAGCAGGGGCTATCTGAGCGCCCTG AGAACCGGCTGGTACACCTCCGTGATTACCATCGAG CTGAGCAACATCAAGGAAACCAAGTGCAACGGCAC CGATACCAAGGTGAAGCTCATCAAGCAGGAGCTGGA CAAGTACAAGAACGCCGTGACCGACCTGCAACTGCT GATGCAGAATACACCTTGTGGAGGCAGCGGAGGAG GCTCTGGCTCTGCCATCGCCTCTGGCATTGCCGTGTG TAAAGTGCTGCATCTGGAGGGCGAGGTGAATAAGAT CAAGAACGCTCTGCAGCTGACCAACAAGGCCGTGG TGAGCCTGTCTAACGGCGTGAGCGTGCTGACCTTCA AGGTGCTGGACCTGAAGAATTACATCAACAACCAGC TGCTGCCCATCGTGAACCAGCAGAGCTGCCGGATCA GCAACATCGAGACAGTGATCGAGTTTCAGCAGAAGA ACAGCCGGCTGCTGGAGATCACCAGGGAGTTTAGCG TGAATGCCGGCGTGACCACCCCCCTGAGCACCTACA TGCTGACCAATAGCGAGCTGCTGTCCCTGATCAACG ACATGCCCATCACCAACGACCAGAAAAAGCTGATGA GCAGCAACGTGCAGATCGTGCGGCAGCAAAGCTAC AGCATCATGTGCATCATCAAGGAGGAGGTGCTGGCC TACGTGGTGCAGCTGCCCATTTACGGCGTGATCGAC ACCCCCTGCTGGAAGCTGCATACCTCTCCTCTGTGCA CCACCAATATCAAGGAAGGCAGCAATATCTGCCTGA CCAGAACAGATCGGGGATGGTACTGCGACAATGCCG GCAGCGTGAGCTTCTTTCCTCAGGCTGATACCTGCA AGGTGCAGTGTAACCGGGTGTTCTGCGACACCATGA ACAGCCTGACCCTGCCCAGCGAGGTGTCCCTGTGCA ACACCGACATCTTCAACAGCAAGTACGACTGCAAGA TCATGACCAGCAAGACCGACATCAGCTCTAGCGTGA TCACCAGCCTGGGCGCCATCGTGAGCTGTTACGGCA AGACCAAATGCACCGCCAGCAACAAGAACCGGGGA ATCATCAAGACCTTCAGCAACGGCTGCGACTACGTG AGCAACAAGGGCGTGGACACCGTGTCTGTGGGCAAT ACCCTGTACTACGTGAATAAGCTGGAGGGCAAGAAC CTGTACGTGAAGGGCGAGCCCATCATCAACTACTAC GACCCCCTGGTGTTCCCCAGCTGTGAGTTTTGCGCC AGCATCAGCCAGGTGAACGAGAAGATCAACCAGAG CCTGGCCTTCATCAGGAGGAGCGATGAGCTGCTGCA CAACGTGGAGGACAAGATCGAGGAGATCCTGAGCA AGATCTACCACATCGAGAACGAGATCGCCCGGATCA AGAAGCTGATCGGCGAGGCCAACACCGGCAAGAGC ACCACAAACATCATGATCACCGCCATCATCATCGTGA TCATCGTGGTGCTGCTGAGCCTGATCGCCATCGGCCT GCTGCTGTACTGCAAGGCCAAGAACACACCCGTGAC CCTGAGCAAAGATCAGCTGAGCGGCATCAATAACAT CGCCTTCTCCAAG （SEQ ID NO: 95） |
| RSV B-003m-P215S-1×GCN4t-G46S_RQ2 | (SEQ ID NO: 82) | ATGGAGCTGCTGATCCACAGAAGCAGCGCCATCTTT CTGACACTGGCCATCAATGCCCTGTACCTGACCAGC AGCCAGAACATCACCGAGGAGTTTTATCAGAGCACC TGCAGCGCCGTGAGCAGGGGCTATCTGAGCGCCCTG AGGACCGGCTGGTACACATCCGTGATCACAATCGAG CTGAGCAACATCAAGGAAACCAAGTGCAATGGCACC GACACCAAGGTGAAGCTCATCAAGCAGGAGCTGGA CAAGTACAAGAACGCCGTGACCGACCTGCAACTGCT GATGCAGAATACACCTTGTGGAGGCAGCGGAGGAG GCTCTGGATCTGCCATCGCCAGCGGCATTGCCGTGTG TAAAGTGCTGCACCTGGAAGGCGAGGTGAATAAGAT CAAGAACGCTCTGCAGCTGACCAACAAGGCCGTGG TGAGCCTGTCTAACGGCGTGAGCGTGCTGACCTTCA AGGTGCTGGACCTGAAGAATTACATCAACAACCAGC TGCTGCCCATCGTGAACCAGCAGAGCTGCCGGATCA GCAACATCGAGACAGTGATCGAGTTTCAGCAGAAGA ACAGCCGGCTGCTGGAGATCACAAGGGAGTTTAGCG TGAACGCCGGCGTGACCACCCCCCTGAGCACCTACA TGCTGACCAATAGCGAGCTGCTGTCCCTGATCAACG ACATGCCTATCACCAACGACCAGAAAAAGCTGATGA GCAGCAACGTGCAGATCGTGCGGCAGCAAAGCTAC AGCATCATGTGCATCATCAAGGAGGAGGTGCTGGCC TACGTGGTGCAGCTGCCCATTTACGGCGTGATCGAC ACCCCCTGCTGGAAGCTGCATACCAGCCCTCTGTGC ACCACCAATATCAAGGAAGGCAGCAATATCTGCCTG ACAAGAACAGATCGGGGATGGTACTGCGACAATGCC GGCAGCGTGAGCTTCTTTCCTCAGGCTGATACCTGC AAGGTGCAGTGTAACCGGGTGTTCTGCGACACCATG AACAGCCTGACCCTGCCCAGCGAGGTGTCCCTGTGC AACACCGACATCTTCAACAGCAAGTACGACTGCAAG ATCATGACCAGCAAGACCGACATCAGCTCTAGCGTG ATCACCAGCCTGGGCGCCATCGTGAGCTGTTACGGC AAGACCAAATGCACCGCCAGCAACAAGAACCGGGG AATCATCAAGACCTTCAGCAACGGCTGCGACTACGT GAGCAACAAGGGCGTGGACACCGTCTCTGTGGGCA ATACCCTGTACTACGTGAATAAGCTGGAGGGCAAGA ACCTGTACGTGAAGGGCGAGCCCATCATCAACTACT ACGACCCCCTGGTGTTCCCCAGCTGTGAGTTTTGCG CAAGCATCAGCCAGGTGAACGAGAAGATCAACCAG AGCCTGGCCTTCATCCGGAGGAGCGATGAGCTGCTG CACAACGTGGAGGACAAGATCGAGGAGATCCTGAG CAAGATCTACCACATCGAGAACGAGATCGCCCGGAT CAAGAAGCTGATCGGCGAGGCCAACACCGGCAAGA GCACCACAAACATCATGATCACCGCCATCATCATCGT GATCATCGTGGTGCTGCTGAGCCTGATCGCCATCGGC CTGCTGCTGTACTGCAAGGCCAAGAACACACCCGTG ACCCTGAGCAAAGATCAGCTGTCTGGCATCAATAAC ATCGCCTTCTCCAAG （SEQ ID NO: 96） |
| RSV B-003m-P215S-1×GCN4t-G46S_RQ3 | (SEQ ID NO: 82) | ATGGAGCTGCTGATCCACAGAAGCTCTGCCATCTTTC TGACACTGGCCATCAACGCCCTGTACCTGACCAGCA GCCAGAACATCACCGAAGAGTTTTATCAGAGCACCT GCAGCGCCGTGAGCCGGGGCTACCTGAGCGCCCTGC GCACTGGGTGGTACACTTCTGTGATCACAATCGAGC TGTCCAACATCAAGGAAACCAAGTGCAATGGCACCG ACACCAAGGTGAAGCTCATCAAGCAGGAGCTGGAC AAGTACAAGAACGCCGTGACCGACCTGCAACTGCTG ATGCAGAATACACCATGTGGAGGCAGCGGAGGAGGC TCTGGATCTGCCATCGCCTCCGGGATTGCCGTGTGTA AAGTGCTGCATCTGGAAGGCGAGGTGAACAAGATCA AGAACGCTCTGCAGCTGACCAACAAGGCCGTGGTG AGCCTGTCTAACGGGGTGAGCGTGCTGACATTCAAG GTGCTGGACCTGAAGAATTACATCAACAACCAGCTG CTGCCCATCGTGAACCAGCAGAGCTGCAGGATCAGC AACATCGAAACCGTGATCGAGTTTCAGCAGAAGAAC AGCCGGCTCCTGGAGATCACACGGGAGTTTAGCGTG AACGCCGGGGTGACCACCCCCCTGAGCACCTACATG CTGACCAATTCCGAGCTGCTGTCCCTGATCAACGAC ATGCCTATCACCAACGACCAGAAAAAGCTGATGAGC AGCAACGTGCAGATCGTGCGGCAGCAATCCTACAGC ATCATGTGCATCATCAAGGAGGAGGTGCTGGCCTAC GTGGTGCAGCTGCCCATTTACGGGGTGATCGACACC CCCTGCTGGAAGCTGCATACCTCCCCACTGTGCACC ACCAATATCAAGGAAGGCTCCAATATCTGCCTGACA AGAACAGATCGGGGATGGTACTGCGACAATGCCGGC AGCGTGAGCTTCTTTCCTCAGGCCGATACCTGCAAG GTGCAGTGTAACCGGGTGTTCTGCGACACCATGAAT AGCCTGACCCTGCCCAGCGAGGTGTCCCTGTGCAAC ACCGACATCTTCAACTCCAAGTACGACTGCAAGATC ATGACCAGCAAAACCGATATCAGCTCCAGCGTGATC ACCAGCCTGGGCGCCATTGTGAGCTGCTACGGGAAG ACCAAATGCACCGCCAGCAACAAGAACCGGGGCAT CATTAAGACCTTCAGCAACGGCTGCGACTACGTGAG CAACAAGGGCGTGGACACCGTGTCTGTGGGCAATAC CCTGTACTACGTGAATAAGCTGGAGGGCAAGAACCT GTACGTGAAGGGCGAGCCCATCATCAACTACTACGA CCCCCTGGTGTTCCCCAGCTGTGAGTTTTGCGCCAG CATCAGCCAGGTGAACGAGAAGATCAACCAATCCCT GGCCTTCATCCGGAGGTCCGACGAGCTGCTTCACAA CGTGGAGGACAAGATCGAGGAGATCCTGAGCAAGAT CTACCACATCGAGAACGAGATCGCCCGGATCAAGAA GCTGATCGGCGAGGCCAACACCGGCAAGAGCACCA CGAACATCATGATCACCGCCATCATCATCGTGATCAT CGTGGTGTTGCTGAGCCTGATTGCCATCGGCCTGCTG CTGTACTGCAAGGCCAAGAACACACCCGTGACCCTG AGCAAAGACCAGCTGAGCGGCATCAATAACATCGCC TTCTCCAAG （SEQ ID NO: 97） |
| RSV B-003m-P215S-1×GCN4t-D92E_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTELQLLMQNTPCGGSG GGSGSAIASGIAVCKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQCNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSCEFCASISQVNEKIN QSLAFIRRSDELLHNVEDKIEE ILSKIYHIENEIARIKKLIGEAN TGKSTTNIMITAIIIVIIVVLLSL IAIGLLLYCKAKNTPVTLSKD QLSGINNIAFSK （SEQ ID NO: 83） | ATGGAGCTGCTGATCCACAGAAGCAGCGCCATTTTC CTGACACTGGCCATCAATGCCCTGTACCTGACCTCTA GCCAGAACATCACCGAGGAGTTTTACCAGAGCACCT GTAGCGCCGTGTCTAGGGGCTATCTGGGCGCTCTGC GGACCGGCTGGTACACCTCCGTGATCACCATCGAGC TGAGCAATATCAAGGAAACCAAGTGCAACGGCACCG ACACCAAGGTGAAGCTGATTAAGCAGGAGCTGGAC AAGTACAAGAACGCCGTGACCGAGCTGCAGTTGCTG ATGCAGAATACACCTTGTGGCGGATCTGGAGGAGGC TCTGGCTCTGCTATCGCCAGCGGAATCGCCGTGTGTA AAGTGCTGCACCTGGAGGGCGAGGTGAATAAGATTA AGAACGCCCTGCAGCTGACAAACAAGGCCGTGGTG AGCCTGAGCAATGGCGTGAGCGTGCTGACCTTCAAG GTGCTGGACCTGAAGAATTACATCAACAACCAGCTG CTGCCCATCGTGAACCAGCAGAGCTGCCGGATCAGC AACATCGAAACCGTGATCGAGTTTCAGCAGAAGAAC AGCCGGCTGCTGGAGATCACCAGGGAGTTTAGCGTG AACGCCGGCGTGACCACCCCTCTGAGCACCTACATG CTGACCAACAGCGAGCTGCTGTCCCTGATCAACGAT ATGCCCATCACCAACGACCAGAAAAAGCTGATGAGC AGCAACGTGCAGATCGTGCGGCAACAGAGCTACAG CATCATGTGCATCATCAAGGAGGAGGTGCTGGCCTA CGTGGTGCAGCTGCCCATTTACGGCGTGATCGACAC CCCCTGCTGGAAGCTGCACACAAGCCCTCTGTGTAC CACCAACATCAAGGAAGGCAGCAATATCTGTCTGAC ACGGACAGATCGGGGATGGTACTGCGATAATGCCGG CAGCGTGAGCTTCTTTCCTCAGGCCGATACCTGCAA GGTGCAGTGCAATAGGGTGTTCTGCGACACCATGAA CAGCCTGACCCTGCCCAGCGAGGTGAGCCTCTGCAA CACCGACATCTTCAACAGCAAGTACGACTGCAAGAT CATGACCAGCAAGACCGACATCAGCAGCAGCGTGAT CACATCTCTGGGCGCCATCGTGAGCTGCTACGGCAA GACCAAGTGTACCGCCAGCAACAAGAACCGGGGCA TCATTAAGACCTTCAGCAACGGCTGCGACTACGTGA GCAACAAGGGCGTGGACACAGTGAGCGTGGGAAAC ACCCTGTACTACGTGAATAAGCTGGAGGGCAAGAAC CTGTACGTGAAGGGCGAGCCCATCATCAACTACTAC GACCCCCTGGTGTTCCCCAGCTGCGAATTTTGCGCC AGCATCAGCCAGGTGAACGAGAAGATTAACCAGAG CCTGGCCTTCATCAGACGGAGCGACGAGCTGCTCCA CAACGTGGAGGACAAGATCGAGGAGATCCTGAGCA AGATCTACCACATCGAGAACGAGATCGCCCGGATCA AGAAGCTGATCGGCGAGGCCAACACCGGCAAGAGC ACCACAAACATCATGATCACCGCCATCATCATCGTGA TCATCGTGGTGCTGCTGAGCCTGATCGCCATCGGCCT GCTGCTGTACTGCAAGGCCAAGAACACCCCTGTGAC CCTGTCTAAAGACCAGCTGAGCGGCATCAACAATAT CGCCTTCTCCAAG （SEQ ID NO: 98） |
| RSV B-003m-P215S-1×GCN4t-D92E_RQ2 | (SEQ ID NO: 83) | ATGGAGCTGCTGATCCACAGAAGCTCTGCCATTTTCC TGACACTGGCCATCAATGCCCTGTACCTGACCTCTAG CCAGAACATCACCGAGGAGTTTTACCAGAGCACCTG TAGCGCCGTGTCTCGGGGCTATCTGGGCGCTCTGCG GACCGGCTGGTATACCTCCGTGATCACCATCGAGCTG TCCAATATCAAGGAAACCAAGTGCAACGGCACCGAC ACCAAGGTGAAGCTGATTAAGCAGGAGCTGGACAA GTACAAGAACGCCGTGACCGAGCTGCAGTTGCTGAT GCAGAATACACCCTGTGGCGGATCAGGAGGAGGCTC TGGCTCTGCTATCGCTAGCGGCATCGCCGTGTGTAAG GTGCTGCATCTGGAGGGCGAGGTGAATAAGATTAAG AACGCCCTGCAGCTGACAAACAAGGCCGTGGTGAG CCTGAGCAATGGCGTGAGCGTGCTGACCTTCAAGGT GCTGGACCTGAAGAATTACATCAACAACCAGCTGCT GCCCATCGTGAACCAGCAGAGCTGCCGGATCAGCAA CATCGAAACCGTGATCGAGTTTCAGCAGAAGAACAG CCGGCTGCTGGAGATCACCAGGGAGTTTAGCGTGAA CGCCGGCGTGACAACCCCCCTGAGCACCTACATGCT GACCAACAGCGAGCTGCTGTCCCTGATCAACGATAT GCCCATCACCAACGACCAGAAGAAACTGATGAGCA GCAACGTGCAGATCGTGCGGCAACAGAGCTACAGC ATCATGTGCATCATCAAGGAGGAGGTGCTGGCCTAC GTGGTGCAGCTGCCCATTTACGGCGTGATCGACACC CCCTGCTGGAAGCTGCACACAAGCCCTCTGTGTACC ACCAACATCAAGGAAGGCAGCAATATCTGTCTGACA CGGACAGATCGGGGATGGTACTGCGATAATGCCGGC AGCGTGAGCTTCTTTCCTCAGGCCGATACCTGCAAG GTGCAGTGCAATAGGGTGTTCTGCGACACCATGAAC AGCCTGACCCTGCCCAGCGAGGTGAGCCTCTGCAAC ACCGACATCTTCAACAGCAAGTACGACTGCAAGATC ATGACCAGCAAGACCGACATCAGCAGCAGCGTGATC ACATCTCTGGGCGCCATCGTGAGCTGCTACGGCAAG ACCAAGTGTACCGCCAGCAACAAGAACCGGGGCAT CATTAAGACCTTCAGCAACGGCTGCGACTACGTGAG CAACAAGGGCGTGGACACAGTGAGCGTGGGAAACA CCCTGTACTACGTGAATAAGCTGGAGGGCAAGAACC TGTACGTGAAGGGCGAGCCCATCATCAACTACTACG ACCCCCTGGTGTTCCCCAGCTGCGAATTTTGCGCCA GCATCAGCCAGGTGAACGAGAAGATTAACCAGAGCC TGGCCTTCATCAGGCGGAGCGACGAGCTGCTCCACA ACGTGGAGGACAAGATCGAGGAGATCCTGAGCAAG ATCTACCACATCGAGAACGAGATCGCCCGGATCAAG AAGCTGATCGGCGAGGCCAACACCGGCAAGAGTAC CACAAACATCATGATCACCGCCATCATCATCGTGATC ATCGTGGTGCTGCTGAGCCTGATCGCCATCGGCCTGC TGCTGTACTGCAAGGCCAAGAACACCCCTGTGACCC TGAGCAAAGACCAGCTGAGCGGCATCAACAATATCG CCTTCTCCAAG （SEQ ID NO: 99） |
| RSV B-003m- | (SEQ ID NO: 83) | ATGGAGCTGCTGATCCACAGAAGCTCTGCCATCTTCC |
| P215S-1×GCN4t-D92E_RQ3 | | TGACACTGGCCATCAATGCCCTGTACCTGACCAGTA GCCAGAACATCACCGAGGAGTTTACCAGAGCACCT GCAGCGCCGTGTCTCGCGGCTATCTGGGCGCTCTGC GCACCGGCTGGTACACCTCTGTGATCACCATCGAGC TGTCTAATATCAAGGAAACCAAGTGCAACGGCACCG ACACCAAGGTGAAGCTGATTAAGCAGGAGCTGGAC AAGTACAAGAATGCCGTGACCGAGCTGCAGTTGCTG ATGCAGAATACTCCCTGTGGCGGAAGCGGAGGAGGC TCTGGAAGCGCAATTGCTAGCGGCATCGCCGTGTGT AAGGTGCTGCATCTGGAGGGCGAGGTGAATAAGATT AAGAACGCCCTGCAGCTGACAAACAAGGCCGTGGT GAGCCTGTCCAACGGCGTGAGCGTGCTGACCTTCAA GGTGCTGGACCTGAAGAATTACATCAACAACCAGCT GCTGCCCATCGTGAACCAGCAGAGCTGCCGGATCTC CAACATCGAAACCGTGATCGAATTTCAGCAGAAGAA CAGCCGGCTGCTGGAGATCACACGGGAGTTTAGCGT GAACGCCGGCGTGACAACCCCCCTGTCCACCTACAT GCTGACCAACTCCGAGCTGCTGTCCCTGATCAACGA TATGCCCATCACCAACGACCAGAAGAAACTGATGAG CAGCAACGTGCAGATCGTGCGGCAGCAAAGCTACA GCATCATGTGCATCATCAAGGAGGAGGTGCTGGCCT ACGTGGTGCAGCTGCCCATTTACGGCGTGATCGACA CCCCCTGCTGGAAGCTGCACACATCTCCTCTGTGCA CCACCAACATCAAAGAAGGCAGCAATATCTGTCTGA CTCGCACAGATCGGGGCTGGTACTGCGACAATGCCG GCAGTGTGTCATTTTTCCCTCAGGCCGATACCTGCAA GGTGCAGTGCAACCGGGTGTTTTGCGACACCATGAA CAGCCTGACCCTGCCCAGCGAGGTGAGCCTCTGCAA CACCGACATCTTCAACTCCAAGTACGACTGCAAGAT CATGACCAGCAAGACCGACATCAGCAGCAGCGTGAT CACAAGCCTGGGCGCTATCGTGTCCTGCTACGGCAA GACCAAGTGTACCGCCAGCAATAAGAACCGGGGCAT CATTAAGACCTTCAGCAACGGCTGCGACTACGTGAG CAACAAGGGCGTGGATACCGTGAGCGTCGGCAACA CCCTGTATTACGTGAACAAGCTGGAGGGCAAGAACC TGTACGTGAAGGGCGAGCCCATCATCAACTACTACG ACCCCCTGGTGTTCCCCAGCTGCGAGTTTGTGCCA GCATCAGCCAGGTGAATGAGAAGATCAACCAATCCC TGGCCTTCATCAGGCGGAGCGACGAGCTGCTCCACA ACGTGGAGGACAAGATCGAGGAGATCCTGAGCAAG ATCTACCACATCGAGAACGAGATCGCCCGGATCAAG AAGCTGATCGGCGAGGCCAACACCGGCAAGAGTAC CACGAACATCATGATCACCGCCATCATCATCGTGATC ATCGTGGTGCTGCTGAGCCTGATCGCCATCGGCCTGC TGCTGTACTGCAAGGCCAAGAATACCCCAGTGACCC TGAGCAAAGACCAGCTGAGCGGCATCAACAATATCG CCTTCTCCAAG （SEQ ID NO: 100） |
| RSV B-003m-P215S-1×GCN4t-F190S_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPCGGSG GGSGSAIASGIAVCKVLHLEG EVNKIKNALQLTNKAVVSLSN GVSVLTSKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQCNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSCEFCASISQVNEKIN QSLAFIRRSDELLHNVEDKIEE ILSKIYHIENEIARIKKLIGEAN TGKSTTNIMITAIIIVIIVVLLSL IAIGLLLYCKAKNTPVTLSKD QLSGINNIAFSK （SEQ ID NO: 84） | ATGGAGCTGCTGATCCACAGAAGCTCCGCCATCTTTC TGACACTGGCCATCAATGCCCTGTACCTGACCTCTAG CCAGAACATCACCGAGGAGTTTTATCAGAGCACCTG CAGCGCTGTGAGCAGGGGCTACCTGGGCGCCCTGAG GACCGGCTGGTACACCTCCGTGATCACCATCGAGCT GAGCAATATTAAGGAAACCAAGTGCAATGGCACCGA CACCAAGGTCAAGCTGATCAAGCAGGAGCTGGACA AGTACAAGAACGCCGTGACTGATCTGCAGCTCCTGA TGCAGAATACACCTTGTGGCGGCTCTGGAGGAGGCT CTGGCAGCGCCATTGCCTCTGGCATCGCTGTGTGTAA GGTGCTGCACCTGGAGGGCGAGGTGAATAAAATCAA GAATGCCCTGCAGCTGACCAACAAAGCCGTGGTGAG CCTGTCTAACGGCGTGAGCGTGCTGACAAGCAAGGT GCTGGACCTGAAAAACTACATCAACAACCAGCTGCT GCCCATCGTGAACCAGCAGAGCTGCCGGATCTCCAA CATCGAGACAGTGATCGAGTTTCAGCAGAAGAACAG CCGCCTGCTGGAGATCACCAGAGAGTTTAGCGTGAA CGCCGGCGTGACCACCCCCCTGAGCACCTACATGCT GACCAATAGCGAGCTGCTGTCCCTGATCAACGACAT GCCCATCACCAACGACCAGAAGAAACTGATGAGCA GCAACGTGCAGATCGTGCGGCAGCAGAGTTACAGCA TCATGTGCATCATCAAGGAGGAGGTGCTGGCCTACG TGGTGCAGCTGCCCATTTACGGCGTGATCGACACCC CCTGCTGGAAGCTGCACACAAGCCCTCTGTGTACCA CCAATATCAAGGAAGGCAGCAACATCTGTCTGACAA GGACCGATAGAGGCTGGTACTGCGATAACGCCGGCA GCGTGAGCTTCTTTCCTCAGGCCGACACATGCAAGG TGCAGTGTAATCGGGTGTTCTGCGACACCATGAACA GCCTGACCCTGCCCAGCGAGGTGTCCCTGTGCAACA CCGACATCTTCAACAGCAAGTACGACTGCAAGATCA TGACCAGCAAGACCGACATCAGCAGCAGCGTGATTA CCTCTCTGGGCGCCATCGTGAGCTGCTACGGCAAGA CCAAGTGTACCGCCAGCAACAAGAACCGGGGCATTA TCAAGACCTTCAGCAACGGCTGCGACTACGTGAGCA ACAAGGGCGTGGACACAGTGAGCGTGGGCAATACC CTGTACTACGTGAATAAGCTGGAGGGCAAGAACCTG TACGTGAAGGGCGAGCCCATCATCAACTACTACGAC CCCCTGGTGTTCCCCAGCTGTGAGTTTTGCGCTAGCA TCAGCCAGGTGAACGAGAAGATCAACCAGAGCCTG GCCTTCATTCGGCGGAGCGATGAGCTGCTGCACAAC GTGGAGGACAAGATCGAGGAGATCCTGAGCAAGATC TACCACATCGAGAACGAGATCGCCCGGATCAAGAAG CTGATCGGCGAGGCCAATACCGGCAAGAGCACCACC AACATCATGATCACCGCCATCATCATCGTGATCATCGT GGTGCTGCTGAGCCTGATCGCCATCGGCCTGCTGCT GTACTGCAAGGCCAAGAACACCCCTGTGACCCTGTC TAAAGACCAGCTGAGCGGCATCAATAACATCGCCTT CTCCAAG （SEQ ID NO: 101） |
| RSV B-003m-P215S-1×GCN4t-F190S_RQ2 | (SEQ ID NO: 84) | ATGGAGCTGCTGATCCACAGAAGCTCTGCCATCTTTC TGACACTGGCCATCAATGCCCTGTACCTGACCTCTAG CCAGAACATCACCGAGGAGTTTTACCAGAGCACCTG CAGCGCTGTGAGCAGGGGCTACCTGGGCGCCCTGAG GACCGGCTGGTACACCAGCGTGATCACCATCGAGCT GAGCAATATTAAGGAAACCAAGTGCAATGGCACAGA CACCAAGGTCAAGCTGATCAAGCAGGAGCTGGACA AGTACAAGAACGCCGTGACCGATCTGCAGCTCCTGA TGCAGAATACACCTTGTGGAGGAAGCGGAGGAGGC AGCGGCAGCGCCATTGCCAGCGGCATCGCTGTGTGT AAGGTGCTGCACCTGGAAGGCGAGGTGAATAAAATC AAGAATGCCCTGCAGCTGACCAACAAAGCCGTGGTG AGCCTGTCTAACGGCGTGAGCGTGCTGACAAGCAAG GTGCTGGACCTGAAAAACTACATCAACAACCAGCTG CTGCCCATCGTGAACCAGCAGAGCTGCCGGATCTCC AACATCGAGACAGTGATCGAGTTTCAGCAGAAGAAC AGCAGGCTGCTGGAGATCACCAGGGAGTTTAGCGTG AACGCCGGCGTGACCACCCCCCTGAGCACCTACATG CTGACCAATAGCGAGCTGCTGTCCCTGATCAACGAC ATGCCCATCACCAACGACCAGAAGAAACTGATGAGC AGCAACGTGCAGATCGTGCGGCAGCAAAGCTACAG CATCATGTGCATCATCAAGGAGGAGGTGCTGGCCTA CGTGGTGCAGCTGCCCATTTACGGCGTGATCGACAC CCCCTGCTGGAAGCTGCACACAAGCCCTCTGTGTAC CACCAATATCAAGGAAGGCAGCAACATCTGTCTGAC AAGGACCGATAGAGGCTGGTACTGCGATAACGCCGG CAGCGTGAGCTTCTTTCCTCAGGCCGACACATGCAA GGTGCAGTGTAATCGGGTGTTCTGCGACACCATGAA CAGCCTGACCCTGCCCAGCGAGGTGTCCCTGTGCAA CACCGACATCTTCAACAGCAAGTACGACTGCAAGAT CATGACCAGCAAGACCGACATCAGCAGCAGCGTGAT TACCTCTCTGGGAGCCATCGTGAGCTGCTACGGCAA GACCAAGTGTACCGCCAGCAACAAGAACCGGGGCA TTATCAAGACCTTCAGCAACGGCTGCGACTACGTGA GCAACAAGGGCGTGGACACCGTGAGCGTCGGCAAT ACACTGTACTACGTGAACAAGCTGGAGGGCAAGAA CCTGTACGTGAAGGGCGAGCCAATCATCAACTACTA CGACCCCCTGGTGTTCCCCAGCTGCGAATTTTGCGCT AGCATCAGCCAGGTGAACGAGAAGATCAACCAGAG CCTGGCCTTCATTCGGCGGAGCGATGAGCTGCTGCA CAACGTGGAGGACAAGATCGAGGAGATCCTGAGCA AGATCTACCACATCGAGAACGAGATCGCCCGGATCA AGAAGCTGATCGGCGAGGCCAATACCGGCAAGAGC ACCACCAACATCATGATCACCGCCATCATCATCGTGA TCATCGTGGTGCTGCTGAGCCTGATCGCCATCGGCCT GCTGCTGTACTGCAAGGCCAAGAACACACCTGTGAC CCTGAGCAAAGACCAGCTGAGCGGAATCAATAACAT CGCCTTCTCCAAG （SEQ ID NO: 102） |
| RSV B-003m-P215S-1×GCN4t-F190S_RQ3 | (SEQ ID NO: 84) | ATGGAGCTGCTGATCCATAGAAGCTCTGCCATCTTTC TGACACTGGCCATCAATGCCCTGTACCTGACCAGCA GCCAGAACATCACCGAGGAGTTTTACCAGAGCACCT GCAGCGCTGTGTCCCGGGGCTACCTGGGCGCCCTGA GGACCGGCTGGTACACAAGCGTGATCACCATCGAGC TGAGCAATATTAAGGAAACCAAGTGCAATGGCACAG ACACCAAGGTCAAGCTGATCAAGCAGGAGCTGGAC AAGTACAAGAACGCCGTGACCGATCTGCAGCTCCTG ATGCAGAATACACCTTGTGGAGGAAGCGGAGGAGG CTCCGGCAGCGCCATTGCCAGCGGGATCGCAGTGTG TAAGGTGCTGCATCTGGAGGGCGAGGTGAATAAAAT CAAGAATGCCCTGCAGCTGACCAACAAAGCCGTGGT GAGCCTGTCTAACGGCGTGAGCGTGCTGACTAGCAA GGTGCTGGACCTGAAAAACTACATCAACAACCAGCT GCTGCCCATCGTCAACCAGCAGAGCTGCCGGATCTC CAACATCGAGACAGTGATCGAGTTTCAGCAGAAGAA CAGCAGGCTGCTGGAGATCACCAGGGAGTTTAGCGT GAACGCCGGGGTGACCACCCCCCTGTCCACCTACAT GCTGACCAATAGCGAGCTGCTGTCGCTGATCAATGA CATGCCCATCACCAACGACCAGAAGAAACTGATGAG CAGCAACGTGCAGATCGTGCGGCAGCAAAGCTACTC CATCATGTGCATCATCAAGGAGGAGGTGCTGGCCTA CGTGGTGCAGCTGCCCATTTACGGCGTGATCGACAC CCCCTGTTGGAAGCTGCACACAAGCCCTCTGTGCAC CACCAACATCAAGGAAGGCAGCAACATCTGTCTGAC ACGCACCGATAGAGGATGGTACTGCGATAATGCCGG GTCCGTGAGCTTTTTCCTCAGGCCGACACCTGCAA GGTGCAGTGCAATCGGGTGTTTTGCGACACCATGAA CAGCCTGACCCTGCCCAGCGAGGTGTCCCTGTGCAA CACCGACATCTTCAACAGCAAGTACGACTGCAAGAT CATGACCTCCAAGACCGACATCAGCAGCAGCGTGAT TACCAGCCTGGGAGCCATCGTGAGCTGCTACGGGAA GACCAAGTGTACCGCCAGCAACAAGAACCGGGGGA TCATTAAGACCTTCAGCAACGGCTGCGACTACGTGA GCAACAAGGGCGTGGATACCGTGTCCGTCGGCAATA CACTGTACTACGTGAACAAGCTGGAGGGCAAGAAC CTGTACGTGAAGGGCGAGCCCATCATCAACTACTAC GACCCCCTGGTGTTCCCCAGCTGCGAATTTTGCGCC AGCATTAGCCAGGTGAACGAGAAGATCAACCAATCC CTGGCCTTCATTCGGCGGAGCGACGAGCTGCTCCAC AACGTGGAGGACAAGATCGAGGAGATCCTGTCCAA GATCTACCACATCGAGAACGAGATCGCCCGGATCAA GAAGCTGATCGGCGAGGCCAACACCGGCAAGAGCA CCACGAACATCATGATCACCGCCATCATCATCGTGAT CATCGTGGTGTTGCTGAGCCTGATTGCCATCGGCCTG CTGCTGTACTGCAAGGCCAAGAACACACCCGTGACC CTGAGCAAAGACCAGCTGAGCGGCATCAACAATATC GCCTTCTCCAAG （SEQ ID NO: 103） |
| RSV B-003m-P215S-1×GCN4t-C155S-C290S_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPCGGSG GGSGSAIASGIAVSKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIMS IIKEEVLAYVVQLPIYGVIDTP CWKLHTSPLCTTNIKEGSNIC LTRTDRGWYCDNAGSVSFFP QADTCKVQCNRVFCDTMNSL TLPSEVSLCNTDIFNSKYDCKI MTSKTDISSSVITSLGAIVSCY GKTKCTASNKNRGIIKTFSNG CDYVSNKGVDTVSVGNTLYY VNKLEGKNLYVKGEPIINYYD PLVFPSCEFCASISQVNEKINQ SLAFIRRSDELLHNVEDKIEEI LSKIYHIENEIARIKKLIGEAN TGKSTTNIMITAIIIVIIVVLLSL IAIGLLLYCKAKNTPVTLSKD QLSGINNIAFSK （SEQ ID NO: 85） | ATGGAGCTGCTGATCCACAGAAGCAGCGCCATCTTT CTGACCCTGGCTATTAATGCCCTGTACCTGACCTCTA GCCAGAATATCACCGAGGAGTTTTACCAGAGCACCT GTAGCGCCGTGAGCAGGGGCTATCTGGGCGCCCTGC GGACCGGATGGTACACCAGCGTGATCACCATCGAGC TGAGCAACATCAAGGAAACAAAGTGCAACGGCACC GACACCAAGGTGAAGCTCATCAAGCAGGAGCTGGA CAAGTACAAGAACGCCGTGACAGATCTGCAGCTTCT GATGCAGAATACACCATGTGGCGGATCAGGAGGCGG CTCTGGCTCTGCTATCGCCAGCGGCATTGCCGTGTCC AAAGTGCTGCACCTGGAGGGCGAAGTGAATAAGATC AAGAATGCCCTGCAGCTGACCAACAAGGCCGTGGTG AGCCTGTCTAACGGCGTGAGCGTGCTGACATTCAAG GTGCTGGACCTGAAGAATTACATCAACAACCAGCTG CTGCCCATCGTGAACCAGCAGAGCTGCCGGATCAGC AACATCGAAACCGTGATCGAGTTTCAGCAGAAGAAC AGCCGGCTGCTGGAGATCACACGGGAGTTTAGCGTG AACGCCGGCGTGACCACACCCCTGAGCACCTACATG CTGACCAATAGCGAGCTGCTGTCCCTGATCAATGAC ATGCCCATCACCAACGACCAGAAGAAGCTCATGAGC AGCAACGTGCAGATCGTGCGGCAACAGAGCTACAG CATCATGAGCATCATCAAGGAGGAGGTGCTGGCCTA CGTGGTGCAGCTGCCCATTTACGGCGTGATCGACAC CCCCTGCTGGAAGCTGCATACCAGCCCTCTGTGTAC AACCAACATCAAAGAGGGCAGCAATATCTGTCTGAC CAGGACCGATAGGGGCTGGTACTGTGACAATGCCGG CAGCGTGAGCTTTTTCCCTCAGGCCGATACCTGTAAG GTGCAGTGCAATCGGGTGTTCTGCGACACCATGAAC AGCCTGACCCTGCCCAGCGAGGTGAGCCTCTGCAAC ACCGACATCTTCAACAGCAAGTACGACTGCAAGATC ATGACCAGCAAGACCGATATCAGCAGCAGCGTGATT ACCAGCCTGGGCGCCATTGTGAGCTGCTACGGCAAG ACCAAGTGTACCGCCAGCAACAAGAACCGGGGCATT ATCAAGACCTTCAGCAACGGCTGCGACTACGTGAGC AACAAGGGCGTGGACACCGTGTCAGTGGGCAACAC CCTGTACTACGTGAATAAGCTGGAGGGCAAGAACCT GTACGTGAAGGGCGAGCCCATCATCAACTACTACGA CCCTCTGGTGTTCCCCAGCTGCGAGTTTGCGCCAG CATCAGCCAGGTGAATGAGAAGATCAACCAGAGCCT GGCCTTCATCAGACGGAGCGACGAGCTGCTCCACAA CGTGGAGGACAAGATCGAGGAGATCCTGAGCAAGAT CTACCACATCGAGAACGAGATCGCCCGGATCAAGAA GCTGATCGGCGAGGCCAACACCGGCAAGAGCACCA CAAACATCATGATCACCGCCATCATCATCGTGATCAT CGTGGTGCTGCTGAGCCTGATCGCCATCGGCCTGCT GCTGTACTGCAAGGCCAAGAACACCCCTGTGACACT GAGCAAGGATCAGCTGTCTGGCATCAACAATATCGC CTTCTCCAAG （SEQ ID NO: 104） |
| RSV B-003m- | (SEQ ID NO: 85) | ATGGAGCTGCTGATCCACAGAAGCAGCGCCATTTTT |
| P215S-1×GCN4t-C155S-C290S_RQ2 | | CTGACCCTGGCTATTAATGCCCTGTACCTGACCTCTA GCCAGAACATCACCGAGGAGTTTACCAGAGCACCT GTAGCGCCGTGAGCAGGGGCTATCTGGGCGCCCTGC GGACCGGATGGTACACCAGCGTGATCACCATCGAGC TGAGCAACATCAAGGAAACAAAGTGCAACGGCACC GACACCAAGGTGAAGCTCATCAAGCAGGAGCTGGA CAAGTACAAGAATGCCGTGACAGATCTGCAGCTTCT GATGCAGAATACACCATGTGGCGGAAGCGGAGGCGG CTCTGGCTCTGCTATCGCCAGCGGCATCGCCGTGTCC AAAGTGCTGCACCTGGAAGGCGAGGTGAATAAGATT AAGAATGCCCTGCAGCTGACCAACAAGGCCGTGGTG AGCCTGTCTAACGGCGTGAGCGTGCTGACATTCAAG GTGCTGGACCTGAAGAATTACATCAACAACCAGCTG CTGCCCATCGTGAACCAGCAGAGCTGCCGGATCAGC AACATCGAAACCGTGATCGAGTTTCAGCAGAAGAAC AGCCGGCTGCTGGAGATCACACGGGAGTTTAGCGTG AACGCCGGCGTGACCACACCCCTGAGCACCTACATG CTGACCAATAGCGAGCTGCTGTCCCTGATCAATGAC ATGCCCATCACCAACGACCAGAAGAAGCTCATGAGC AGCAACGTGCAGATCGTGCGGCAACAGAGCTACAG CATCATGTCCATCATCAAGGAGGAGGTGCTGGCCTAC GTGGTGCAGCTGCCCATTTACGGCGTGATCGACACC CCCTGCTGGAAGCTGCATACCAGCCCTCTGTGTACA ACCAACATCAAAGAGGGCAGCAATATCTGTCTGACC AGGACCGATAGGGGCTGGTACTGTGACAATGCCGGC AGCGTGAGCTTTTTCCCTCAGGCCGATACCTGTAAG GTGCAGTGCAATCGGGTGTTCTGCGACACCATGAAC AGCCTGACCCTGCCCAGCGAGGTGAGCCTCTGCAAC ACCGACATCTTCAACAGCAAGTACGACTGCAAGATC ATGACCAGCAAGACCGACATCAGCAGCAGCGTGATT ACCAGCCTGGGCGCCATTGTGAGCTGCTATGGCAAG ACCAAGTGTACCGCCAGCAACAAGAACCGGGGCATT ATCAAGACCTTCAGCAACGGCTGCGACTACGTGAGC AACAAGGGCGTGGACACCGTGTCCGTGGGCAATACC CTGTACTACGTGAACAAGCTGGAGGGCAAGAACCTG TACGTGAAGGGCGAGCCCATCATTAACTACTACGAC CCTCTGGTGTTCCCCAGCTGCGAGTTTTGCGCCAGC ATCAGCCAGGTGAATGAGAAGATCAACCAGAGCCTG GCCTTCATCAGACGGAGCGACGAGCTGCTCCACAAC GTGGAGGACAAGATCGAGGAGATCCTGAGCAAGATC TACCACATCGAGAACGAGATCGCCCGGATCAAGAAG CTGATCGGCGAGGCCAACACCGGCAAGAGCACCAC AAACATCATGATCACCGCCATCATCATCGTGATCATC GTGGTGCTGCTGAGCCTGATCGCCATCGGCCTGCTG CTGTACTGCAAGGCCAAGAACACCCCTGTGACACTG AGCAAAGACCAGCTGAGCGGCATCAACAATATCGCC TTCTCCAAG （SEQ ID NO: 105） |
| RSV B-003m-P215S-1×GCN4t-C155S-C290S_RQ3 | (SEQ ID NO: 85) | ATGGAGCTGCTGATTCACAGGAGCAGCGCCATTTTTC TGACCCTGGCTATTAATGCCCTGTACCTGACCTCTTC CCAGAACATCACCGAGGAGTTTTACCAGAGCACCTG TAGCGCCGTGAGCAGGGGCTATCTGGGCGCCCTGCG CACCGGTTGGTACACCAGCGTGATCACCATCGAGCT GAGCAACATCAAGGAAACAAAGTGCAACGGCACCG ACACCAAGGTGAAGCTCATCAAGCAGGAGCTGGAC AAGTACAAGAATGCCGTGACTGATCTGCAGCTTCTG ATGCAGAATACACCATGTGGCGGAAGCGGAGGCGGC AGTGGCTCTGCTATCGCCAGCGGCATCGCCGTGTCC AAAGTGCTGCACCTGGAAGGCGAGGTGAATAAGATT AAGAATGCCCTGCAGCTGACCAACAAGGCCGTGGTG TCCCTGTCTAACGGCGTGAGCGTGCTGACATTCAAG GTGCTCGACCTGAAGAATTACATCAACAACCAGCTG CTGCCCATCGTGAACCAGCAGAGCTGCCGGATCAGC AACATCGAAACCGTGATCGAGTTTCAGCAGAAGAAC AGCCGGCTCCTGGAGATCACACGGAGTTTAGCGTG AACGCCGGGGTGACCACTCCCCTGAGCACCTACATG CTGACCAATAGCGAGCTGCTGTCCCTGATCAATGAC ATGCCCATCACCAACGACCAGAAGAAGCTCATGAGC AGCAACGTGCAGATCGTGCGCCAACAGAGCTACAGC ATCATGTCCATCATCAAGGAGGAGGTGCTGGCCTAC GTGGTGCAGCTGCCCATTTACGGCGTGATCGACACC CCTTGCTGGAAGCTGCACACCTCCCCTCTGTGTACTA CCAACATCAAAGAGGGAAGCAATATCTGCTTGACAC GCACCGATAGGGGCTGGTACTGTGACAACGCCGGCA GCGTGTCTTTTTTCCCACAGGCCGATACATGCAAGGT GCAGTGCAACCGGGTGTTCTGTGACACCATGAACAG CCTGACCCTGCCCAGCGAGGTGAGCCTCTGCAACAC CGACATCTTCAACTCCAAGTACGACTGCAAGATCAT GACCAGCAAGACCGATATCAGCAGCAGCGTGATTAC CAGCCTGGGCGCCATCGTGAGCTGCTATGGCAAGAC CAAGTGTACCGCCAGCAACAAGAACCGGGGCATCAT CAAAACCTTCAGCAACGGCTGCGACTACGTGAGCAA CAAGGGGGTGGACACCGTGTCCGTGGGCAATACCCT GTACTACGTGAACAAGCTGGAGGGCAAGAATCTGTA CGTGAAGGGCGAGCCCATCATTAACTACTACGACCC TCTGGTGTTCCCCAGCTGCGAGTTTTGCGCCAGCATC AGCCAGGTGAATGAGAAGATTAACCAGAGCCTGGCC TTCATCCGGCGGAGCGACGAGCTGCTCCACAACGTG GAGGACAAGATCGAGGAGATCCTGAGCAAGATCTAC CACATCGAGAACGAGATCGCCCGGATCAAGAAGCTG ATCGGCGAGGCCAACACCGGAAAGAGCACCACGAA CATCATGATCACCGCCATCATCATCGTGATCATCGTGG TGTTGCTGAGCCTGATCGCCATCGGCCTGCTGCTGTA CTGCAAGGCCAAGAACACCCCTGTGACCCTGAGCA AAGACCAGCTGAGCGGCATCAACAATATCGCCTTCT CCAAG （SEQ ID NO: 106） |
| RSV B-003m-P215S-1×GCN4t-C486D-C489D_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPCGGSG GGSGSAIASGIAVCKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQCNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSDEFDASISQVNEKIN QSLAFIRRSDELLHNVEDKIEE ILSKIYHIENEIARIKKLIGEAN TGKSTTNIMITAIIIVIIVVLLSL IAIGLLLYCKAKNTPVTLSKD QLSGINNIAFSK （SEQ ID NO: 86） | ATGGAGCTGCTGATCCACAGGAGCTCTGCCATCTTTC TGACACTGGCCATCAATGCCCTGTACCTGACCAGCTC TCAGAATATCACCGAGGAGTTTTACCAGAGCACCTG CAGCGCCGTGAGCAGGGGATATCTGGGCGCCCTGAG AACCGGCTGGTACACCAGCGTGATCACCATCGAGCT GAGCAACATCAAGGAAACCAAGTGCAACGGCACCG ACACCAAGGTGAAGCTGATTAAGCAGGAGCTGGAC AAGTACAAGAATGCCGTGACAGACCTGCAACTGCTG ATGCAGAATACCCCTTGCGGCGGATCTGGAGGAGGC TCTGGCAGCGCCATTGCTAGCGGAATCGCCGTGTGT AAAGTGCTGCACCTGGAAGGCGAGGTGAATAAGATC AAGAACGCCCTGCAGCTGACCAACAAGGCCGTGGT GTCCCTGTCTAATGGCGTGAGCGTGCTGACCTTCAA GGTGCTGGACCTGAAGAATTACATCAACAACCAGCT GCTGCCCATCGTGAACCAGCAGAGCTGCCGGATCAG CAACATCGAAACCGTGATCGAGTTTCAGCAGAAGAA CAGCAGGCTGCTGGAGATCACCCGGGAGTTTAGCGT GAACGCCGGCGTGACCACCCCCCTGAGCACCTACAT GCTGACCAATAGCGAGCTGCTGTCCCTGATCAATGA CATGCCCATCACCAACGACCAGAAGAAACTGATGAG CAGCAACGTGCAGATCGTGCGGCAGCAAAGCTACA GCATCATGTGCATCATCAAGGAGGAGGTGCTGGCCT ACGTGGTGCAGCTGCCCATTTACGGCGTGATCGACA CCCCCTGCTGGAAGCTGCACACCTCTCCTCTGTGCA CAACCAATATCAAGGAAGGCAGCAATATCTGCCTGA CACGGACAGATAGAGGCTGGTACTGCGACAATGCCG GCAGCGTGAGCTTCTTTCCTCAGGCCGATACCTGCA AGGTGCAGTGCAACAGAGTGTTCTGCGACACCATGA ACAGCCTGACCCTGCCCAGCGAGGTGAGCCTGTGCA ACACCGACATCTTCAACAGCAAGTACGACTGCAAGA TCATGACCAGCAAGACCGACATTAGCAGCAGCGTCA TCACCAGCCTGGGCGCTATCGTGAGCTGTTACGGCA AGACCAAGTGTACCGCCAGCAACAAGAACCGGGGG ATCATCAAGACCTTCAGCAACGGCTGCGACTACGTG AGCAACAAGGGCGTGGACACCGTGAGCGTCGGCAA TACACTGTACTACGTGAATAAGCTGGAGGGCAAGAA CCTGTACGTGAAGGGCGAGCCCATCATCAACTACTA CGACCCCCTGGTGTTCCCTAGCGACGAGTTTGACGC CTCCATTAGCCAGGTGAACGAGAAGATCAACCAGAG CCTGGCCTTCATCCGGAGGAGCGATGAGCTGCTCCA CAACGTGGAGGACAAGATCGAGGAGATCCTGAGCA AGATCTACCACATCGAGAACGAGATCGCCCGGATCA AGAAGCTGATCGGCGAGGCCAATACCGGCAAGAGC ACCACCAACATCATGATCACCGCCATCATCATCGTGA TCATCGTGGTGCTGCTGAGCCTGATCGCCATCGGCCT GCTGCTGTACTGCAAGGCCAAGAATACACCCGTGAC CCTGAGCAAAGACCAGCTGAGCGGCATCAACAATAT CGCCTTCTCCAAG （SEQ ID NO: 107） |
| RSV B-003m-P215S-1×GCN4t-C486D-C489D_RQ2 | (SEQ ID NO: 86) | ATGGAGCTGCTGATCCACAGGAGCTCTGCCATCTTTC TGACACTGGCCATCAATGCCCTGTACCTGACCAGCTC TCAGAACATTACCGAGGAGTTTTACCAGAGCACATG CAGCGCCGTGAGCCGGGGATATCTGGGCGCCCTGAG AACCGGCTGGTACACCAGCGTGATCACCATCGAGCT GAGCAACATCAAGGAAACCAAGTGCAACGGCACCG ACACCAAGGTGAAGCTGATTAAGCAGGAGCTGGAC AAGTACAAGAATGCCGTGACAGACCTGCAACTGCTG ATGCAGAATACCCCTTGCGGCGGATCTGGAGGAGGC TCTGGCAGCGCCATTGCTAGCGGAATCGCCGTGTGT AAAGTGCTGCACCTGGAAGGCGAGGTGAATAAGATC AAGAACGCCCTGCAGCTGACCAACAAGGCCGTGGT GTCCCTGTCAAATGGCGTGAGCGTGCTGACCTTCAA GGTGCTGGACCTGAAGAATTACATCAACAACCAGCT GCTGCCCATCGTGAACCAGCAGAGCTGCCGCATCAG CAACATCGAAACCGTGATCGAGTTTCAGCAGAAGAA CAGCAGGCTGCTGGAGATCACACGGGAGTTTAGCGT GAACGCCGGCGTGACCACCCCCCTGAGCACCTACAT GCTGACCAATAGCGAGCTGCTGTCCCTGATCAACGA CATGCCTATCACCAACGACCAGAAGAAACTGATGAG CAGCAACGTGCAGATCGTGCGGCAGCAAAGCTACA GCATCATGTGCATCATCAAGGAGGAGGTGCTGGCCT ACGTGGTGCAGCTGCCCATTTACGGCGTGATCGACA CCCCCTGCTGGAAGCTGCACACCTCTCCTCTGTGCA CAACCAATATCAAGGAAGGCAGCAATATCTGCCTGA CACGGACAGATAGAGGCTGGTACTGCGACAATGCCG GCAGCGTGAGCTTCTTTCCACAGGCCGATACCTGCA AGGTGCAGTGCAACAGAGTGTTCTGCGACACCATGA ACAGCCTGACCCTGCCCAGCGAGGTGAGCCTGTGCA ACACCGACATCTTCAACAGCAAGTACGACTGCAAGA TCATGACCAGCAAGACCGACATTAGCAGCAGCGTCA TCACCAGCCTGGGCGCTATCGTGAGCTGCTACGGCA AGACCAAGTGTACCGCCAGCAATAAGAACCGGGGG ATCATCAAGACCTTCAGCAACGGCTGCGACTACGTG AGCAACAAGGGCGTGGACACCGTGAGCGTCGGCAA TACACTGTACTACGTGAATAAGCTGGAGGGCAAGAA CCTGTACGTGAAGGGCGAGCCCATCATCAACTACTA CGACCCCCTGGTGTTCCCTAGCGACGAGTTTGACGC CAGCATCAGCCAGGTGAACGAGAAGATCAATCAGAG CCTGGCCTTCATCCGGAGGAGCGACGAGCTGCTCCA CAATGTGGAGGACAAGATCGAGGAGATCCTGAGCAA GATCTACCACATCGAGAACGAGATCGCCCGGATCAA GAAGCTGATCGGCGAGGCCAATACCGGCAAGAGCA CCACCAACATCATGATCACCGCCATCATCATCGTGAT CATCGTGGTGCTGCTGAGCCTGATCGCCATCGGCCTG CTGCTGTACTGCAAGGCCAAGAATACACCCGTGACC CTGAGCAAAGACCAGCTGAGCGGCATCAACAATATC GCCTTCTCCAAG （SEQ ID NO: 108） |
| RSV B-003m-P215S-1×GCN4t-C486D-C489D_RQ3 | (SEQ ID NO: 86) | ATGGAGCTGCTGATCCACAGAAGCTCTGCCATCTTTC TGACACTGGCCATCAATGCCCTGTACCTGACCTCCTC TCAGAACATTACCGAGGAGTTTTACCAGAGCACATG CAGCGCCGTGAGCCGGGGGTACCTGGGCGCCCTGA GAACCGGCTGGTACACCAGCGTGATCACCATTGAGC TGAGCAACATCAAGGAAACCAAGTGCAACGGCACC GACACCAAGGTGAAGCTGATTAAGCAGGAGCTGGA CAAGTACAAGAATGCCGTGACAGACCTGCAACTGCT GATGCAGAATACCCCTTGTGGCGGATCTGGAGGAGG CAGCGGGAGCGCCATCGCTAGCGGAATCGCCGTGTG TAAAGTGCTGCATCTGGAAGGCGAGGTCAACAAAAT CAAGAACGCCCTGCAGCTGACCAACAAGGCCGTGG TGTCCCTGTCCAATGGCGTGTCCGTGCTGACCTTCAA GGTGCTGGACCTGAAGAATTACATCAACAACCAGCT GCTGCCCATCGTGAATCAGCAGAGCTGCCGGATCTC CAACATCGAAACCGTGATCGAGTTTCAGCAGAAGAA CAGCCGGCTGCTGGAGATCACACGCGAGTTTTCCGT GAACGCCGGGGTGACCACCCCCCTGAGCACCTACAT GCTGACCAATAGCGAGCTGCTGTCCCTGATCAACGA CATGCCTATCACCAACGACCAGAAGAAACTGATGAG CAGCAACGTGCAGATCGTGCGCCAGCAAAGCTACAG CATCATGTGCATCATCAAGGAGGAGGTGCTGGCCTA CGTGGTGCAGCTGCCCATTTACGGCGTGATCGACAC CCCCTGCTGGAAGCTGCACACCTCTCCACTGTGCAC AACCAATATCAAGGAAGGGAGCAATATCTGCCTGAC ACGCACAGATAGAGGCTGGTACTGCGACAATGCCGG CTCCGTGAGCTTCTTCCCACAGGCCGATACATGCAA GGTGCAGTGCAACAGAGTGTTCTGCGACACCATGAA CAGCCTGACCCTGCCCAGCGAGGTGAGCCTGTGCAA CACCGACATCTTCAACTCCAAGTACGACTGCAAGAT CATGACCAGCAAGACCGACATTTCCAGCAGCGTCAT CACCAGCCTGGGCGCAATCGTGAGCTGCTACGGGAA GACCAAGTGTACCGCCAGCAATAAGAACCGGGGGAT CATCAAGACCTTCAGCAACGGCTGCGACTACGTGTC CAACAAGGGCGTGGACACCGTGAGCGTCGGCAATA CCCTGTACTATGTGAATAAGCTGGAGGGCAAGAACC TGTACGTGAAGGGCGAGCCCATCATCAACTACTACG ACCCCCTGGTGTTCCCTAGCGACGAGTTTGACGCCA GCATCAGCCAGGTGAACGAGAAAATCAACCAATCCC TGGCCTTCATCCGGAGGAGCGACGAGCTGCTCCACA ACGTGGAGGACAAGATCGAGGAGATCCTGAGCAAG ATCTACCACATCGAGAACGAGATCGCCCGGATCAAG AAGCTGATCGGCGAGGCCAACACCGGCAAGAGCAC CACCAACATCATGATCACCGCCATCATCATCGTGATT ATCGTGGTGCTGCTGAGCCTGATCGCCATCGGCCTGC TGCTGTACTGCAAGGCCAAGAATACTCCCGTGACCC TGAGCAAAGACCAGCTGAGCGGCATCAACAATATCG CCTTCTCCAAG （SEQ ID NO: 109） |
| RSV B-003m-P215S-1×GCN4t-ori_linker_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPCVNNR ARREAPQYMNYTINTTKNLN VSISKKRKRRFLGFLLGVGSA IASGIAVCKVLHLEGEVNKIK NALQLTNKAVVSLSNGVSVLT FKVLDLKNYINNQLLPIVNQQ SCRISNIETVIEFQQKNSRLLEI TREFSVNAGVTTPLSTYMLTN SELLSLINDMPITNDQKKLMS SNVQIVRQQSYSIMCIIKEEVL AYVVQLPIYGVIDTPCWKLHT SPLCTTNIKEGSNICLTRTDRG WYCDNAGSVSFFPQADTCKV QCNRVFCDTMNSLTLPSEVSL CNTDIFNSKYDCKIMTSKTDI SSSVITSLGAIVSCYGKTKCTA SNKNRGIIKTFSNGCDYVSNK GVDTVSVGNTLYYVNKLEGK NLYVKGEPIINYYDPLVFPSCE FCASISQVNEKINQSLAFIRRS DELLHNVEDKIEEILSKIYHIE NEIARIKKLIGEANTGKSTTNI MITAIIIVIIVVLLSLIAIGLLLY CKAKNTPVTLSKDQLSGINNI AFSK （SEQ ID NO: 87） | ATGGAGCTGCTGATCCACAGGAGCAGCGCCATCTTT CTGACCCTGGCCATCAACGCCCTGTACCTGACCAGC AGCCAGAACATTACAGAGGAGTTTTACCAGAGCACA TGCAGCGCCGTGAGCAGGGGCTATCTGGGCGCTCTG AGGACCGGATGGTATACAAGCGTGATCACCATCGAG CTGAGCAACATCAAGGAAACCAAGTGCAACGGCAC CGACACCAAGGTCAAGCTGATCAAGCAGGAGCTGG ACAAGTACAAGAACGCCGTGACCGACCTGCAGCTCC TGATGCAGAATACACCATGCGTGAACAACCGGGCCC GGCGGGAGGCCCCCCAGTACATGAATTACACCATCA ACACCACCAAGAACCTGAACGTGAGCATCTCCAAGA AGAGAAAGAGAAGGTTCCTGGGCTTCCTGCTGGGC GTGGGATCTGCCATTGCTTCTGGAATCGCTGTGTGTA AAGTGCTGCACCTGGAGGGCGAGGTGAACAAGATC AAGAATGCCCTGCAGCTGACCAACAAGGCCGTGGTG AGCCTGAGCAACGGCGTGAGCGTGCTGACCTTCAAG GTGCTGGACCTGAAAAACTACATCAACAACCAGCTG CTGCCCATCGTGAACCAGCAGAGCTGCCGGATCAGC AACATCGAAACCGTGATCGAGTTTCAGCAGAAGAAC AGCCGGCTGCTGGAGATCACCAGGGAGTTTTCCGTG AATGCCGGCGTGACCACCCCCCTGAGCACCTACATG CTGACCAATAGCGAGCTGCTGTCCCTGATCAACGAC ATGCCCATCACCAACGACCAGAAAAAGCTGATGAGC AGCAACGTGCAGATCGTGCGGCAGCAAAGCTACAG CATCATGTGCATCATCAAGGAGGAGGTGCTGGCCTA CGTGGTGCAGCTGCCCATTTACGGCGTGATCGACAC CCCCTGTTGGAAGCTGCACACAAGCCCTCTGTGCAC CACCAACATCAAAGAGGGCAGCAATATCTGCCTGAC AAGAACAGACCGGGGATGGTACTGCGACAATGCCG GCAGCGTGTCTTTCTTTCCTCAGGCCGACACCTGTAA GGTGCAGTGCAACAGAGTGTTCTGCGACACCATGAA CAGCCTGACCCTGCCCAGCGAGGTGAGCCTCTGCAA CACCGACATCTTCAACAGCAAGTACGACTGCAAGAT CATGACCAGCAAGACCGACATCAGCAGCAGCGTGAT TACCAGCCTGGGCGCCATCGTGAGCTGCTACGGCAA GACCAAATGCACCGCCTCTAACAAGAACCGGGGCAT CATTAAGACCTTCAGCAACGGCTGCGACTACGTGAG CAACAAGGGCGTGGACACCGTGAGCGTCGGAAATA CCCTGTACTACGTGAACAAGCTGGAGGGCAAGAATC TGTACGTGAAGGGCGAGCCCATCATCAACTACTACG ACCCCCTGGTGTTCCCCAGCTGTGAGTTTTGCGCCA GCATCAGCCAGGTGAACGAGAAGATCAACCAGAGC CTGGCCTTCATCAGACGGAGCGACGAGCTGCTCCAC AACGTGGAGGACAAGATCGAGGAGATCCTGAGCAA GATCTACCACATCGAGAACGAGATCGCCAGGATCAA GAAGCTGATCGGCGAGGCCAACACCGGCAAGAGCA CCACAAACATCATGATCACCGCCATCATCATCGTGAT CATCGTGGTGCTGCTGAGCCTGATCGCCATCGGCCTG CTGCTGTACTGCAAGGCCAAGAACACCCCCGTGACC CTGAGCAAAGATCAGCTGAGCGGCATCAACAATATC GCCTTCTCCAAG （SEQ ID NO: 110） |
| RSV B-003m-P215S-1×GCN4t-ori_linker_RQ2 | (SEQ ID NO: 87) | ATGGAGCTGCTGATCCACAGAAGCAGCGCCATCTTT CTGACCCTGGCCATCAATGCCCTGTACCTGACCAGC AGCCAGAACATTACAGAGGAGTTTTACCAGAGCACA TGCAGCGCCGTGAGCAGGGGCTATCTGGGCGCTCTG AGGACCGGCTGGTATACAAGCGTGATCACCATCGAG CTGAGCAACATCAAGGAAACCAAGTGCAACGGCAC CGACACCAAGGTCAAGCTGATCAAGCAGGAGCTGG ACAAGTACAAGAACGCCGTGACCGACCTGCAGCTCC TGATGCAGAATACACCTTGCGTGAACAACCGGGCCC GGCGGGAGGCCCCCCAGTACATGAATTACACCATCA ACACCACCAAGAACCTGAACGTGAGCATCAGCAAG AAGAGAAAGAGAAGGTTCCTGGGCTTCCTGCTTGGC GTGGGAAGCGCCATTGCTAGCGGAATCGCTGTGTGT AAAGTGCTGCACCTGGAGGGAGAGGTGAACAAGAT CAAGAACGCTCTGCAGCTGACCAACAAGGCCGTGG TGAGCCTGAGCAACGGCGTGAGCGTGCTGACCTTCA AGGTGCTGGACCTGAAAAACTACATCAACAACCAGC TGCTGCCCATCGTGAACCAGCAGAGCTGCCGGATCA GCAACATCGAAACCGTGATCGAGTTTCAGCAGAAGA ACAGCCGGCTGCTGGAGATCACCAGGGAGTTTTCTG TGAACGCCGGCGTGACCACCCCCCTGAGCACCTACA TGCTGACCAATAGCGAGCTGCTGTCCCTGATCAACG ACATGCCCATCACCAACGACCAGAAAAAGCTGATGA GCAGCAACGTGCAGATCGTGCGGCAGCAAAGCTAC AGCATCATGTGCATCATCAAGGAGGAGGTGCTGGCC TACGTGGTGCAGCTGCCCATTTACGGCGTGATCGAC ACCCCCTGTTGGAAGCTGCACACAAGCCCTCTGTGC ACCACCAACATCAAAGAGGGCAGCAATATCTGCCTG ACTAGAACAGACCGGGGATGGTACTGCGACAATGCC GGCAGCGTGTCTTTCTTTCCTCAGGCCGACACCTGTA AGGTGCAGTGCAACAGAGTGTTCTGCGACACCATGA ACAGCCTGACCCTGCCCAGCGAGGTGAGCCTCTGCA ACACCGACATCTTCAACAGCAAGTACGACTGCAAGA TCATGACCAGCAAGACCGACATCAGCAGCAGCGTGA TTACCAGCCTGGGCGCCATCGTGAGCTGCTACGGCA AGACCAAATGCACCGCCTCTAACAAGAACCGGGGC ATCATTAAGACCTTCAGCAACGGCTGCGACTACGTG AGCAACAAGGGCGTGGACACCGTGAGCGTCGGAAA TACCCTGTACTACGTGAACAAGCTGGAGGGCAAGAA TCTGTACGTGAAGGGCGAGCCCATCATCAACTACTAC GACCCCCTGGTGTTCCCTAGCTGCGAGTTTTGCGCC AGCATCAGCCAGGTGAACGAGAAGATCAACCAGAG CCTGGCCTTCATCAGACGGAGCGACGAGCTGCTCCA CAACGTGGAGGACAAGATCGAGGAGATCCTGAGCA AGATCTACCACATCGAGAACGAGATCGCCAGGATCA AGAAGCTGATCGGCGAGGCCAACACCGGCAAGAGC ACCACGAACATCATGATCACCGCCATCATCATCGTGA TCATCGTGGTGTTGCTGAGCCTGATCGCCATCGGCCT GCTGCTGTACTGCAAGGCCAAGAACACCCCCGTGAC CCTGAGCAAAGATCAGCTGTCTGGCATCAACAATATC GCCTTCTCCAAG （SEQ ID NO: 111） |
| RSV B-003m-P215S-1×GCN4t-ori_linker_RQ3 | (SEQ ID NO: 87) | ATGGAGCTGCTGATCCACAGAAGCTCTGCCATCTTTC TGACCCTGGCCATCAATGCCCTGTACCTGACCAGCA GCCAGAACATTACAGAGGAGTTTTACCAGAGCACAT GCAGCGCCGTGAGCCGGGGCTATCTGGGCGCTCTGC GCACCGGCTGGTATACAAGCGTGATCACCATCGAGC TGTCTAACATCAAGGAAACCAAGTGCAACGGCACCG ACACCAAGGTCAAGCTGATCAAGCAGGAGCTGGAC AAGTACAAGAATGCCGTGACCGACCTGCAGCTCCTG ATGCAGAATACTCCTTGCGTGAACAACCGGGCCCGG CGGGAGGCCCCCCAGTACATGAATTACACCATCAAC ACCACCAAGAACCTGAACGTGAGCATCTCTAAGAAG AGGAAGAGACGGTTTCTGGGCTTCCTGTTGGGCGTG GGAAGCGCCATTGCAAGCGGAATCGCTGTGTGTAAA GTGCTGCACCTGGAGGGTGAGGTGAACAAGATCAA GAACGCTCTGCAGCTGACCAACAAGGCCGTGGTGA GCCTGAGCAACGGCGTGAGCGTGCTGACCTTCAAGG TGCTCGACCTGAAAAACTACATCAACAACCAGCTGC TGCCCATCGTGAACCAGCAGAGCTGCCGGATCAGCA ACATCGAAACCGTGATCGAGTTCAGCAGAAGAACA GCCGGCTCCTGGAGATCACCAGGGAGTTTTCTGTGA ACGCCGGGGTGACCACCCCCCTGTCCACCTACATGC TGACCAATAGCGAGCTGCTGTCCCTGATCAACGACA TGCCCATCACCAACGACCAGAAAAAGCTGATGAGCA GCAACGTGCAGATCGTGCGGCAGCAAAGCTACAGC ATCATGTGCATCATCAAGGAGGAGGTGCTGGCCTAC GTGGTGCAGCTGCCCATTTACGGCGTGATCGACACC CCCTGTTGGAAGCTGCACACCAGCCCTCTGTGCACA ACCAACATCAAAGAGGGCTCCAACATTTGTCTGACT AGGACAGACCGGGGATGGTATTGCGACAACGCCGGC AGCGTGTCTTTCTTTCCTCAGGCCGACACCTGTAAG GTGCAGTGCAACAGAGTGTTCTGCGACACCATGAAC AGCCTGACACTGCCCAGCGAGGTGAGCCTCTGCAAC ACCGACATCTTCAACTCCAAGTACGACTGCAAGATC ATGACCAGCAAGACCGACATCAGCAGCAGCGTGATT ACCAGCCTGGGCGCCATCGTGTCCTGCTACGGGAAG ACCAAATGCACCGCCAGTAACAAGAACCGGGGCATC ATTAAGACCTTCAGCAACGGCTGCGACTACGTGAGC AACAAGGGCGTGGACACCGTGAGCGTCGGCAATAC CCTGTACTATGTGAACAAGCTGGAGGGCAAGAATCT GTACGTGAAGGGCGAGCCCATCATCAACTACTACGA CCCCCTGGTGTTCCCTAGCTGCGAGTTTTGCGCCAGC ATCTCCCAGGTCAACGAGAAGATCAACCAGAGCCTG GCCTTCATCAGACGGAGCGACGAGCTGCTCCACAAC GTGGAGGACAAGATCGAGGAGATCCTGAGCAAGATC TACCACATCGAGAACGAGATCGCCCGGATCAAGAAG CTGATCGGCGAGGCCAACACCGGCAAGAGCACCAC GAACATCATGATCACCGCCATCATCATCGTGATCATC GTGGTGTTGCTGAGCCTGATCGCCATCGGCCTGCTGC TGTACTGCAAGGCCAAGAACACACCCGTGACCCTGA GCAAAGACCAGCTGTCTGGCATCAACAATATCGCCT TCTCCAAG （SEQ ID NO: 112） |
| RSV B-003m-P215S_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPCGGSG GGSGSAIASGIAVCKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQCNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSCEFCASISQVNEKIN QSLAFIRRSDELLHNVNTGKS TTNIMITAIIIVIIVVLLSLIAIGL LLYCKAKNTPVTLSKDQLSGI NNIAFSK （SEQ ID NO: 88） | ATGGAGCTGCTGATCCACAGAAGCTCTGCCATTTTCC TGACCCTGGCCATCAATGCTCTGTACCTGACCAGCA GCCAGAACATCACCGAGGAGTTTTACCAGAGCACCT GTAGCGCCGTGAGCAGAGGCTATCTGGGCGCCCTGA GGACCGGCTGGTACACCAGCGTGATCACCATCGAGC TGAGCAACATTAAGGAGACAAAGTGCAATGGCACCG ACACCAAGGTGAAGCTGATCAAGCAGGAGCTGGAC AAGTACAAGAACGCCGTGACCGATCTGCAGCTCCTG ATGCAGAATACACCATGTGGGGGATCTGGAGGCGGC TCTGGCTCTGCTATCGCCTCTGGAATTGCCGTGTGCA AAGTGCTGCACCTGGAGGGCGAGGTGAATAAGATCA AGAATGCCCTGCAGCTGACCAATAAGGCCGTGGTGA GCCTGAGCAATGGCGTGAGCGTGCTGACCTTCAAGG TGCTCGACCTGAAGAATTACATCAACAACCAGCTGC TGCCCATCGTGAACCAGCAGAGCTGCCGGATCAGCA ACATCGAAACCGTGATCGAGTTTCAGCAGAAGAACA GCCGGCTGCTGGAGATCACCAGGGAGTTTAGCGTGA ACGCCGGCGTGACCACCCCACTGAGCACCTACATGC TGACCAACAGCGAGCTGCTGTCCCTGATCAATGACA TGCCAATCACCAACGACCAGAAGAAGCTGATGAGCA GCAACGTGCAGATCGTGCGGCAACAGAGCTACAGC ATCATGTGCATCATCAAGGAGGAGGTGCTGGCCTAC GTGGTGCAGCTGCCTATCTACGGCGTGATCGACACC CCCTGCTGGAAGCTGCATACCAGCCCCCTGTGTACA ACAAACATCAAGGAAGGCAGCAATATCTGCCTGACC AGGACAGACAGAGGCTGGTACTGTGACAATGCCGG CAGCGTGAGCTTCTTCCCTCAGGCTGATACCTGTAAG GTGCAGTGCAACCGGGTGTTCTGCGATACCATGAAC AGCCTGACCCTCCCCAGCGAGGTGAGCCTCTGCAAC ACCGACATCTTCAACAGCAAGTACGACTGCAAGATC ATGACCAGCAAGACCGACATCAGCAGCAGCGTGATT ACCAGCCTGGGCGCTATCGTGAGCTGCTACGGCAAG ACCAAGTGCACCGCCAGCAACAAGAACAGGGGCAT CATTAAGACCTTCAGCAACGGCTGCGACTACGTGAG CAACAAGGGCGTGGACACAGTGAGCGTGGGCAACA CCCTGTATTACGTGAACAAGCTGGAGGGCAAGAATC TGTACGTGAAGGGCGAGCCCATCATCAACTACTACG ACCCCCTGGTGTTCCCTAGCTGCGAGTTTTGCGCCA GCATCAGCCAGGTGAACGAGAAGATTAACCAGAGCC TGGCCTTTATCCGGCGGAGCGATGAGCTGCTGCACA ACGTGAACACCGGCAAGAGCACCACCAACATCATGA TCACCGCCATCATCATCGTGATCATCGTGGTGCTGCT GAGCCTGATTGCCATCGGCCTGCTGCTGTACTGTAAG GCCAAGAACACCCCTGTGACCCTGAGCAAGGATCAG CTGAGCGGCATCAACAATATCGCCTTCTCCAAG （SEQ ID NO: 113） |
| RSV B-003m-P215S_RQ2 | (SEQ ID NO: 88) | ATGGAGCTGCTGATCCACAGAAGCTCTGCCATCTTCC TGACCCTGGCCATCAATGCTCTGTACCTGACCAGCA GCCAGAACATCACCGAGGAGTTTTACCAGAGCACCT GTAGCGCCGTGAGCCGGGGCTATCTGGGCGCCCTGA GGACCGGATGGTATACCAGCGTGATCACCATCGAGC TGTCCAACATTAAGGAGACAAAGTGCAATGGCACCG ACACCAAGGTGAAGCTGATCAAGCAGGAGCTGGAC AAGTACAAGAACGCCGTGACCGATCTGCAGCTCCTG ATGCAGAATACACCTTGTGGCGGATCTGGAGGCGGC TCTGGCTCTGCTATCGCCTCTGGAATTGCCGTGTGCA AGGTGCTGCACCTGGAGGGAGAGGTGAATAAGATCA AGAATGCCCTGCAGCTGACCAATAAGGCCGTGGTGA GCCTGAGCAACGGCGTGAGCGTGCTGACCTTCAAGG TGCTCGACCTGAAGAATTACATCAACAACCAGCTGC TGCCAATCGTGAACCAGCAGAGCTGCCGGATCAGCA ACATCGAAACCGTGATCGAGTTTCAGCAGAAGAACA GCCGGCTGCTGGAGATCACCAGGGAGTTTAGCGTGA ACGCCGGCGTGACCACCCCTCTGAGCACCTACATGC TGACCAACAGCGAGCTGCTGTCCCTGATCAATGACA TGCCAATCACCAACGACCAGAAGAAGCTGATGAGCA GCAACGTGCAGATCGTGCGGCAACAGAGCTACAGC ATCATGTGCATCATCAAGGAGGAGGTGCTGGCCTAC GTGGTGCAGCTGCCTATCTACGGCGTGATCGACACC CCCTGCTGGAAGCTGCATACCAGCCCCCTGTGTACA ACAAACATCAAAGAGGGCAGCAATATCTGCCTGACC AGGACAGACAGAGGCTGGTACTGTGACAATGCCGG CAGCGTGAGCTTCTTCCCTCAGGCTGATACCTGTAAA GTGCAGTGCAACCGGGTGTTCTGCGACACCATGAAC AGCCTGACCCTCCCCAGCGAGGTGTCCCTCTGCAAC ACCGACATCTTCAACAGCAAGTACGACTGCAAGATC ATGACCAGCAAGACCGACATCAGCAGCAGCGTGATT ACCAGCCTGGGCGCTATCGTGAGCTGCTACGGCAAG ACCAAGTGCACCGCCAGCAACAAGAACAGGGGCAT CATTAAGACCTTCAGCAACGGCTGCGACTACGTGAG CAACAAGGGCGTGGACACAGTGAGCGTGGGCAACA CCCTGTATTACGTGAACAAGCTGGAGGGCAAGAATC TGTACGTGAAGGGCGAGCCCATCATCAACTACTACG ACCCCCTGGTGTTCCCTAGCTGCGAGTTTTGCGCCA GCATCAGCCAGGTGAACGAGAAGATTAACCAGAGCC TGGCCTTTATCCGGCGGAGCGATGAGCTGCTGCACA ACGTGAACACCGGCAAGAGCACCACCAACATCATGA TCACCGCCATCATCATCGTGATCATCGTGGTGCTGCT GAGCCTGATTGCCATCGGCCTGCTGCTGTACTGTAAG GCCAAGAACACCCCTGTGACCCTGTCTAAGGACCAG CTGAGCGGCATCAACAATATCGCCTTCTCCAAG （SEQ ID NO: 114） |
| RSV B-003m-P215S_RQ3 | (SEQ ID NO: 88) | ATGGAGCTGCTGATCCATAGAAGCTCTGCCATTTTCC TGACCCTGGCCATCAATGCCCTGTACCTGACCAGCA GCCAGAACATCACCGAGGAGTTTTACCAGAGCACCT GCTCCGCCGTGTCCCGGGCTACCTTGGCGCCCTGC GCACCGGATGGTACACCAGCGTGATCACCATTGAGC TGAGCAACATTAAGGAAACAAAATGCAATGGCACCG ACACCAAGGTGAAGCTGATCAAGCAGGAGCTGGAC AAGTACAAGAACGCCGTGACCGATCTGCAGCTCCTG ATGCAGAATACACCTTGTGGCGGATCTGGCGGCGGC TCTGGCTCTGCTATCGCCAGCGGAATTGCCGTGTGCA AGGTGCTGCACCTGGAGGGAGAGGTGAACAAGATC AAGAATGCTCTGCAGCTGACAAACAAGGCCGTGGTG AGCCTGAGCAATGGCGTGAGCGTGCTGACCTTCAAG GTGCTCGATCTGAAGAATTACATCAACAACCAGCTG CTGCCAATCGTGAACCAGCAGAGCTGCCGGATCAGC AACATCGAAACCGTGATCGAGTTTCAGCAGAAGAAC AGCCGGCTGCTGGAGATCACCAGGGAGTTTAGCGTG AACGCCGGCGTGACCACCCCTCTGAGCACCTACATG CTGACCAACAGCGAGCTGCTGTCCCTGATCAATGAC ATGCCCATCACCAACGATCAGAAGAAGCTGATGAGC AGCAACGTGCAGATCGTGCGGCAGCAATCCTACAGC ATCATGTGCATCATCAAGGAGGAGGTGCTGGCCTAC GTGGTGCAGCTGCCTATCTACGGCGTGATCGACACC CCCTGCTGGAAGCTGCACACAAGCCCCCTGTGTACT ACAAACATCAAAGAGGGCTCCAACATTTGTCTGACC AGGACAGACAGAGGCTGGTACTGTGACAACGCCGG CAGCGTGTCTTTTTTCCCAGGCTGACACCTGCAA AGTGCAGTGCAACCGGGTGTTCTGCGACACCATGAA TAGCCTGACCCTCCCCAGCGAGGTGAGCCTCTGCAA CACCGACATCTTCAACAGCAAGTACGACTGCAAGAT CATGACCAGCAAGACCGACATCAGCAGCAGCGTGAT TACCAGCCTGGGCGCAATCGTGAGCTGCTACGGCAA GACCAAGTGCACCGCCTCCAACAAGAACAGGGGCA TCATTAAGACCTTCAGCAACGGCTGCGACTACGTGA GCAACAAGGGCGTGGACACAGTGAGCGTGGGCAAC ACCCTGTACTATGTGAACAAGCTGGAGGGCAAGAAT CTGTACGTGAAGGGCGAGCCCATCATCAACTACTAC GACCCCCTGGTGTTCCCCAGCTGCGAGTTTTGCGCC AGCATTAGCCAGGTGAACGAGAAGATCAACCAATCC CTGGCCTTTATCCGGCGGAGCGATGAGCTGCTCCAC AACGTGAACACCGGCAAGAGCACCACCAACATCATG ATCACCGCCATCATCATCGTGATCATCGTGGTGCTGC TGAGCCTGATTGCCATCGGCCTGCTGCTGTACTGCAA GGCCAAGAACACCCCAGTGACCCTGTCAAAGGACC AGCTGAGCGGCATCAACAATATCGCCTTCTCCAAG （SEQ ID NO: 115） |
| RSV B-003m-P215S-1×GCN4t-C102A-C362S_RQ1 | MELLIHRSSAIFLTLAINALYL TSSQNITEEFYQSTCSAVSRGY LGALRTGWYTSVITIELSNIKE TKCNGTDTKVKLIKQELDKY KNAVTDLQLLMQNTPAGGSG GGSGSAIASGIAVCKVLHLEG EVNKIKNALQLTNKAVVSLSN GVSVLTFKVLDLKNYINNQLL PIVNQQSCRISNIETVIEFQQK NSRLLEITREFSVNAGVTTPLS TYMLTNSELLSLINDMPITND QKKLMSSNVQIVRQQSYSIM CIIKEEVLAYVVQLPIYGVIDT PCWKLHTSPLCTTNIKEGSNI CLTRTDRGWYCDNAGSVSFF PQADTCKVQSNRVFCDTMNS LTLPSEVSLCNTDIFNSKYDC KIMTSKTDISSSVITSLGAIVSC YGKTKCTASNKNRGIIKTFSN GCDYVSNKGVDTVSVGNTLY YVNKLEGKNLYVKGEPIINYY DPLVFPSCEFCASISQVNEKIN QSLAFIRRSDELLHNVEDKIEE ILSKIYHIENEIARIKKLIGEAN TGKSTTNIMITAIIIVIIVVLLSL IAIGLLLYCKAKNTPVTLSKD QLSGINNIAFSK （SEQ ID NO: 89） | ATGGAGCTGCTGATCCACAGAAGCAGCGCCATCTTT CTGACCCTGGCCATCAACGCTCTGTACCTGACCTCTA GCCAGAATATCACCGAGGAGTTTTACCAGAGCACAT GCAGCGCCGTGAGCAGAGGCTACCTGGGAGCCCTG AGGACCGGCTGGTACACCAGCGTGATCACCATCGAG CTGAGCAATATCAAGGAAACCAAGTGCAACGGCACC GACACCAAGGTCAAGCTGATCAAGCAGGAGCTGGA CAAGTACAAGAATGCCGTGACCGATCTGCAGCTCCT GATGCAGAATACACCTGCTGGAGGATCTGGCGGAGG AAGCGGCTCTGCTATTGCCAGCGGCATCGCCGTGTG TAAAGTGCTGCACCTGGAGGGAGAAGTGAATAAGAT CAAGAACGCCCTGCAGCTGACCAACAAGGCCGTGG TGAGCCTGAGCAACGGCGTGTCTGTGCTGACCTTCA AGGTGCTGGACCTGAAGAATTACATCAACAACCAGC TGCTGCCCATCGTGAACCAGCAGAGCTGCCGGATCA GCAACATCGAAACCGTGATCGAGTTTCAGCAGAAGA ACAGCCGGCTGCTGGAGATCACCCGGGAGTTTTCTG TGAACGCCGGCGTGACCACCCCCCTGAGCACCTACA TGCTGACCAATAGCGAGCTGCTGTCCCTGATCAATGA CATGCCCATCACCAACGACCAGAAGAAACTGATGAG CAGCAACGTGCAGATCGTGCGGCAGCAAAGCTACA GCATCATGTGCATCATCAAGGAGGAGGTGCTGGCCT ACGTGGTGCAGCTGCCTATCTACGGCGTGATCGACA CCCCCTGCTGGAAGCTGCACACAAGCCCTCTGTGCA CAACCAACATCAAAGAGGGAAGCAACATCTGTCTGA CCAGGACCGATAGAGGCTGGTACTGTGACAATGCCG GCAGCGTGAGCTTCTTTCCTCAGGCCGATACCTGCA AAGTGCAGAGCAACAGGGTGTTCTGCGACACCATGA ACAGCCTGACCCTGCCCAGCGAGGTCAGCCTGTGCA ACACCGACATCTTCAACAGCAAGTACGACTGCAAGA TCATGACCAGCAAGACCGACATCAGCAGCAGCGTGA TTACAAGCCTGGGCGCCATCGTGAGCTGCTACGGCA AGACCAAATGTACCGCCAGCAACAAGAACCGGGGC ATCATTAAGACCTTCAGCAACGGCTGCGACTACGTG AGCAACAAGGGCGTGGACACCGTGAGCGTGGGCAA TACCCTGTACTACGTGAACAAGCTGGAGGGCAAGAA TCTGTACGTGAAGGGCGAGCCCATCATCAACTACTAC GACCCCCTGGTGTTCCCCAGCTGTGAATTTTGCGCCA GCATCAGCCAGGTGAACGAGAAGATCAACCAGAGC CTGGCCTTCATCAGGCGGAGCGACGAGCTGCTCCAC AACGTGGAGGACAAGATCGAGGAGATCCTGTCCAA GATCTACCACATCGAGAACGAGATCGCCAGGATCAA GAAGCTGATCGGCGAGGCCAACACCGGCAAGAGCA CCACCAATATCATGATCACCGCCATCATCATCGTGATC ATCGTGGTGCTGCTGAGCCTGATCGCCATCGGCCTGC TGCTGTACTGCAAGGCCAAGAACACCCCCGTGACAC TGAGCAAGGATCAGCTGAGCGGCATCAATAATATCG CCTTCTCCAAG （SEQ ID NO: 116） |
| RSV B-003m-P215S-1×GCN4t-C102A-C362S_RQ2 | (SEQ ID NO: 89) | ATGGAGCTGCTGATCCACAGAAGCAGCGCCATCTTT CTGACCCTGGCCATCAACGCTCTGTATCTGACCTCTA GCCAGAATATCACCGAGGAGTTTTACCAGAGCACAT GCAGCGCCGTGAGCCGGGGCTACCTGGGAGCCCTG AGGACCGGGTGGTACACCAGCGTGATCACAATCGAG CTGAGCAATATCAAGGAAACCAAGTGCAACGGCACC GACACCAAGGTCAAGCTGATCAAGCAGGAGCTGGAT AAGTACAAGAATGCCGTGACCGATCTGCAGCTCCTG ATGCAGAATACACCAGCCGGAGGATCTGGCGGAGGA AGCGGCTCTGCTATTGCCAGCGGCATCGCCGTGTGTA AGGTGCTGCATCTGGAGGGAGAGGTGAATAAGATCA AGAACGCCCTGCAGCTGACCAACAAGGCCGTGGTG AGCCTGAGCAACGGCGTGTCTGTGCTGACCTTCAAG GTGCTCGACCTGAAGAATTACATCAATAACCAGCTG CTGCCCATCGTGAACCAGCAGAGCTGCCGGATCAGC AACATCGAAACCGTGATCGAGTTTCAGCAGAAGAAC AGCCGGCTGCTGGAGATCACCCGGGAGTTTTCTGTG AACGCCGGCGTGACCACCCCCCTGAGCACCTACATG CTGACCAATAGCGAGCTGCTGTCCCTGATCAATGAC ATGCCCATCACCAACGACCAGAAGAAACTGATGAGC TCCAACGTGCAGATCGTGCGGCAGCAAAGCTACAGC ATCATGTGCATCATCAAGGAGGAGGTGCTGGCCTAC GTGGTGCAGCTGCCAATCTACGGCGTGATCGACACC CCCTGCTGGAAGCTGCACACAAGCCCTCTGTGCACA ACCAACATCAAAGAGGGAAGCAACATCTGCCTGACA AGGACCGATAGAGGCTGGTACTGTGACAATGCCGGC AGCGTGAGCTTCTTTCCTCAGGCCGATACCTGCAAA GTGCAGAGCAACAGGGTGTTCTGCGACACCATGAAC AGCCTGACCCTGCCCAGCGAGGTCAGCCTGTGCAAC ACCGACATCTTCAACAGCAAGTACGACTGCAAGATC ATGACCAGCAAGACCGACATCAGCAGCAGCGTGATT ACAAGCCTGGGCGCCATCGTGAGCTGCTACGGCAAG ACCAAATGTACCGCCAGCAACAAGAACCGGGGCATC ATTAAGACCTTCAGCAACGGCTGCGACTACGTGAGC AACAAGGGCGTGGACACCGTGAGCGTGGGCAATAC CCTGTACTACGTGAACAAGCTGGAGGGCAAGAATCT GTACGTGAAGGGCGAGCCCATCATCAACTACTACGA CCCCCTGGTGTTTCCCAGCTGTGAGTTTTGCGCCAGC ATCAGCCAGGTGAACGAGAAGATCAACCAGAGCCT GGCCTTCATCAGGCGGAGCGACGAGCTGCTCCACAA CGTGGAGGACAAGATCGAGGAGATCCTGTCCAAGAT CTACCACATCGAGAACGAGATCGCCAGGATCAAGAA GCTGATCGGCGAGGCCAACACCGGCAAGAGCACCA CCAATATCATGATCACCGCCATCATCATCGTGATCATC GTGGTGCTGCTGAGCCTGATCGCCATCGGCCTGCTG CTGTACTGCAAGGCCAAGAACACACCCGTGACCCTG AGCAAGGATCAGCTGAGCGGCATCAATAATATCGCCT TCTCCAAG （SEQ ID NO: 117） |
| RSV B-003m-P215S-1×GCN4t-C102A-C362S_RQ3 | (SEQ ID NO: 89) | ATGGAGCTGCTGATCCACAGAAGCTCTGCCATTTTTC TGACCCTGGCCATCAACGCACTGTATCTGACCTCCAG CCAGAACATCACCGAGGAGTTTTACCAGAGCACATG CAGCGCCGTGAGCCGGGGCTACCTGGGAGCCCTGCG CACCGGATGGTACACCAGCGTGATCACAATCGAGCT GAGCAATATCAAGGAAACCAAGTGCAATGGCACCGA CACCAAGGTCAAGCTGATCAAGCAGGAGCTGGATAA GTACAAGAACGCCGTGACAGATCTGCAGCTCCTGAT GCAGAATACTCCTGCCGGAGGATCAGGCGGAGGCAG CGGCAGCGCTATTGCCTCCGGAATCGCCGTGTGCAA GGTGCTGCATCTTGAGGGAGAAGTGAACAAGATTAA GAACGCCCTGCAGCTGACCAACAAGGCTGTGGTGA GCCTGAGCAACGGCGTGTCAGTGCTGACCTTCAAGG TGCTCGACCTGAAAAACTACATCAATAACCAGCTGC TGCCCATCGTGAACCAGCAGAGCTGCCGGATCAGCA ACATCGAAACCGTGATCGAGTTTCAGCAGAAGAACA GCCGGCTCCTGGAGATCACCCGGGAGTTTTCTGTGA ACGCCGGCGTGACCACCCCCCTGAGCACCTACATGC TGACCAATAGCGAGCTGCTGTCCCTGATCAACGACA TGCCCATCACCAACGACCAGAAGAAACTGATGAGCT CTAACGTGCAGATCGTGCGGCAGCAAAGCTACTCCA TCATGTGCATCATCAAGGAGGAGGTGCTGGCCTACG TGGTGCAGCTGCCAATCTACGGCGTGATCGACACCC CCTGCTGGAAGCTGCACACAAGCCCTCTGTGTACCA CCAACATCAAAGAGGGAAGCAACATCTGCCTGACCC GAACAGATAGAGGCTGGTACTGCGACAATGCCGGCA GCGTGAGCTTTTTTCCTCAGGCCGACACCTGCAAAG TGCAGTCAAACCGCGTGTTCTGCGACACCATGAACA GCCTGACCCTGCCTAGCGAGGTCAGCCTGTGCAACA CCGACATCTTCAACTCCAAGTACGACTGCAAGATCAT GACCAGCAAGACCGACATCAGCAGCAGCGTGATTAC ATCCCTGGGCGCCATCGTGAGCTGCTACGGCAAGAC CAAATGCACCGCCAGCAATAAGAACCGGGGCATCAT TAAGACCTTCAGCAACGGCTGCGACTACGTGAGCAA CAAGGGGGTGGACACCGTGAGCGTGGGCAACACCC TGTACTACGTGAATAAGCTGGAGGGCAAGAATCTGT ACGTGAAGGGCGAGCCCATCATCAACTACTACGACC CCCTGGTGTTCCCCAGCTGTGAGTTTTGCGCCAGCAT CAGCCAGGTGAATGAGAAAATCAACCAATCCCTGGC CTTCATCCGGCGGAGCGACGAGCTGCTCCACAACGT GGAGGACAAGATCGAGGAGATCCTGAGCAAGATCTA CCACATCGAGAACGAGATCGCCCGGATCAAGAAGCT GATCGGCGAGGCCAACACCGGCAAGAGCACCACCA ATATCATGATCACCGCCATCATCATCGTGATCATCGTG GTGCTGCTGAGCCTGATTGCCATCGGCCTGCTGCTGT ACTGCAAGGCCAAGAACACACCCGTGACCCTGAGC AAAGACCAGCTGAGCGGCATCAATAATATCGCCTTCT CCAAG （SEQ ID NO: 118） |
| Post-F | MELLILKANAITTILTAVTFCF ASGQNITEEFYQSTCSAVSKG YLSALRTGWYTSVITIELSNIK ENKCNGTDAKVKLIKQELDK YKNAVTELQLLMQSTPATNN RARRELPRFMNYTLNNAKKT NVTLSKKRKRRAIASGVAVSK VLHLEGEVNKIKSALLSTNKA VVSLSNGVSVLTSKVLDLKN YIDKQLLPIVNKQSCSISNIET VIEFQQKNNRLLEITREFSVN AGVTTPVSTYMLTNSELLSLI NDMPITNDQKKLMSNNVQIV RQQSYSIMSIIKEEVLAYVVQ LPLYGVIDTPCWKLHTSPLCT TNTKEGSNICLTRTDRGWYC DNAGSVSFFPQAETCKVQSN RVFCDTMNSLTLPSEVNLCNV DIFNPKYDCKIMTSKTDVSSS VITSLGAIVSCYGKTKCTASN KNRGIIKTFSNGCDYVSNKGV DTVSVGNTLYYVNKQEGKSL YVKGEPIINFYDPLVFPSDEFD ASISQVNEKINQSLAFIRKSDE LLHNVNAGKSTTNIMITTIIIVI IVILLSLIAVGLLLYCKARSTP VTLSKDQLSGINNIAFSN （SEQ ID NO: 201） | atggaactgcttatcctgaaagctaatgctattacaaccattctgactgccgttacattctg ctttgccagtggccagaatatcacggaggaattctaccagagcacctgcagcgcagtt tccaaaggatatctttcagcattgcggactggctggtatacctcagttattacaattgagc tgagcaacattaaggaaaataagtgcaacggaacagacgccaaagtcaagctgatca agcaggagctggataagtacaaaaatgctgtcacagagcttcagctcctcatgcagag cactcccgccaccaataaccgagctcgacgagagctgcctaggttcatgaactacact cttaataatgctaaaaaaactaacgtgacactttcaaagaagcgaaagcggagagcaa tcgcaagcggagtggccgtgagtaaggtgttgcatttggaaggcgaggtgaataaaa ttaagagcgcactgctcagcaccaacaaggccgttgtgagcctgagcaatggagtca gtgtgcttacctctaaggtcctggacctcaagaactacatcgataagcagctgctccca attgtgaataagcaatcctgttctatctcaaacatcgagaccgtaatcgagttccaacaa aaaaacaacaggctgctggaaatcaccagggagttctccgttaacgcaggggtgacc acccccgtctctacctacatgctgacaaacagcgaattgctgtcattgattaacgatatg ccgattaccaatgatcagaaaaaactcatgtccaacaatgtgcaaattgtgcggcagc agtcctactccatcatgagtattatcaaagaggaagtgctcgcttacgtggttcagcttc ccctgtatggcgttatcgatacaccctgctggaaattgcacacatcacccctgtgcacc accaatacgaaggagggaagcaacatctgtctgactaggaccgatcgcggctggtac tgcgacaatgcaggtagtgtgtcatttttcctcaggcagaaacatgcaaagtccagtcc aatcgcgtgttctgcgataccatgaacagtttgacgctgccctccgaggtgaatctctg caatgtagatatctttaatcctaagtatgattgcaagatcatgacttccaaaaccgacgtg tcatcttctgttattactagccttggcgcgattgtcagctgttacggtaagaccaagtgca ccgcctccaataagaatcgcggaatcattaagactttttcaaatggatgcgactacgtg agtaataaaggagttgacacggtgtcagtgggcaatacgttgtattacgtcaacaagca ggaggggaagagcctctacgtgaagggagaaccgataattaacttctacgacccctct ggtgttcccctcagatgaatttgacgcatccatttcccaggtaaatgagaagatcaacc agagtctggcgtttattcgaaaatctgacgaactgcttcacaatgtgaatgctggcaaaa gtactacgaacatcatgatcactaccattattatcgtaattatcgtgattttgctgagcctg atcgctgtgggcctgttgctctattgtaaggccagatccaccccagtgactctttcaaag gaccagctgagtggcatcaacaacattgccttcagtaat（SEQ ID NO: 202） |

While specific embodiments of the present invention have been described above, those skilled in the art will appreciate that such embodiments are provided by way of illustration only. Various modifications and changes may be made without departing from the spirit and scope of the present invention.

Accordingly, the scope of the present invention is defined by the appended claims and equivalents thereof.

## Claims

1. **An RSV antigen,** wherein the RSV antigen comprises mutations A102C and S362C relative to the amino acid sequence set forth in SEQ ID NO: 1 or SEQ ID NO: 80.

2. The RSV antigen of claim 1, wherein the RSV antigen further comprises one or more mutations selected from S46G, E92D, S155C, S190F, S215P, S290C, D486C, D489C, A149C, and Y458C;
preferably, the RSV antigen comprises D486C, D489C, A149C, and Y458C;
and/or wherein the RSV antigen further comprises a linker;
preferably, the linker comprises an amino acid sequence selected from SEQ ID NOs: 2-5 or GGS; and/or wherein the linker replaces amino acid residues 103-144 of SEQ ID NO: 1.

3. The RSV antigen of claim 1 or 2, wherein the RSV antigen further comprises an inserted fragment positioned between amino acid residues 516 and 517 of SEQ ID NO: 1;
preferably, the inserted fragment comprises the amino acid sequence set forth in SEQ ID NO: 6 or SEQ ID NO: 7.

4. The RSV antigen of claim 1, wherein the RSV antigen satisfies either of the following:
(I) the RSV antigen differs from SEQ ID NO: 1 in that it comprises mutations S155C, S290C, S190F, S215P, S46G, E92D, D486C, D489C, A102C, and S362C, and amino acid residues 103-144 of SEQ ID NO: 1 are replaced with a linker comprising an amino acid sequence set forth in SEQ ID NO: 2, SEQ ID NO: 3, SEQ ID NO: 4, SEQ ID NO: 5, or GGS, and further comprises one or more of:
(a) a reversion mutation P215S; (b) an inserted fragment comprising an amino acid sequence set forth in SEQ ID NO: 6, SEQ ID NO: 8, or SEQ ID NO: 140; and/or (c) a replacement fragment comprising an amino acid sequence set forth in SEQ ID NO: 7;
or
(II) the RSV antigen differs from SEQ ID NO: 80 in that it comprises mutations S155C, S290C, S190F, S46G, E92D, D486C, D489C, A102C, and S362C, and amino acid residues 103-144 of SEQ ID NO: 80 are replaced with a linker comprising the amino acid sequence set forth in SEQ ID NO: 2, and further comprises an inserted fragment comprising the amino acid sequence set forth in SEQ ID NO: 6.

5. The RSV antigen of claim 4, wherein the RSV antigen further satisfies one or more of the following:
(1) in (I) or (II), the inserted fragment is positioned between residues 516 and 517 of SEQ ID NO: 1 or SEQ ID NO: 80;
(2) in (I), the replacement fragment replaces residues 525-574 of SEQ ID NO: 1;
(3) in (I), the RSV antigen further comprises mutation L513I relative to SEQ ID NO: 1;
(4) in (I), the inserted fragment is positioned between residues 513 and 514, between residues 519 and 520, or between residues 522 and 523 of SEQ ID NO: 1.

6. The RSV antigen of claim 1, 4, or 5, wherein the RSV antigen further comprises a post-translational modification, preferably glycosylation;
and/or wherein, in (I), the RSV antigen further comprises reversion mutation C486D and/or C489D;
and/or wherein, in (II), the RSV antigen further comprises one or more reversion mutations selected from:
(a) G46S; (b) D92E; (c) F190S; (d) C155S and C290S; (e) C486D and C489D; or (f) C102A and C362S;
preferably, the glycosylation is N-linked glycosylation; and/or the glycosylation occurs on the inserted fragment.

7. The RSV antigen of any one of claims 1-6, wherein the RSV antigen comprises an amino acid sequence set forth in any one of SEQ ID NO: 11, SEQ ID NO: 12, SEQ ID NO: 16, SEQ ID NO: 20, SEQ ID NO: 24, SEQ ID NO: 28, SEQ ID NO: 33, SEQ ID NOs: 34-40, SEQ ID NOs: 81-89, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, or SEQ ID NOs: 166-176.

8. An isolated nucleic acid comprising a nucleotide sequence encoding the RSV antigen according to any one of claims 1-7.

9. The isolated nucleic acid of claim 8, wherein the isolated nucleic acid is mRNA, preferably a codon-optimized mRNA;
more preferably, the mRNA comprises one or more of a promoter region, a 5' cap structure, a 5' untranslated region (5' UTR), a protein tag, and a 3' untranslated region (3' UTR) and poly(A) tail;
even more preferably, the promoter is a T7 promoter; and/or the protein tag is HA-HiBiT;
and/or wherein the nucleotide sequence encoding the RSV antigen comprises a nucleotide sequence set forth in any one of SEQ ID NO: 42, 43, 47, 51, 55, 59, 64-79, 92-118, 121, 123, 125, 127, 129, 131, 133, 135, 137, 139, 142, 144, 146, 148, 150, 152, 154, 156, 158, or 182-200.

10. The isolated nucleic acid of claim 8, wherein the nucleic acid is DNA;
preferably, the DNA further comprises sequences encoding one or more of a 5' cap structure, a 5' UTR, a 3' UTR, a 3' poly(A) sequence, and a protein tag.

11. A recombinant expression vector comprising a starting plasmid and the isolated nucleic acid according to any one of claims 8-10.

12. A transformed cell comprising the isolated nucleic acid according to any one of claims 8-10 or the recombinant expression vector according to claim 11.

13. A method for preparing the isolated nucleic acid according to any one of claims 8-10, wherein the method comprises subjecting the recombinant expression vector according to claim 11 to in vitro transcription.

14. A method for producing an RSV antigen, comprising culturing the transformed cell according to claim 12 under conditions suitable for expression of the RSV antigen.

15. A pharmaceutical composition comprising:
(1) the nucleic acid according to claim 8 or 9; and
(2) a delivery vehicle;
preferably, the delivery vehicle comprises a lipid nanoparticle (LNP);
more preferably, the LNP comprises:
(A) an ionizable or cationic lipid selected from SM-102, cationic lipid RL151, LQ104-54, LQ104-56, LQ104-E16b-2, or Compound 6; (B) cholesterol; (C) DSPC; and (D) a PEGylated lipid. 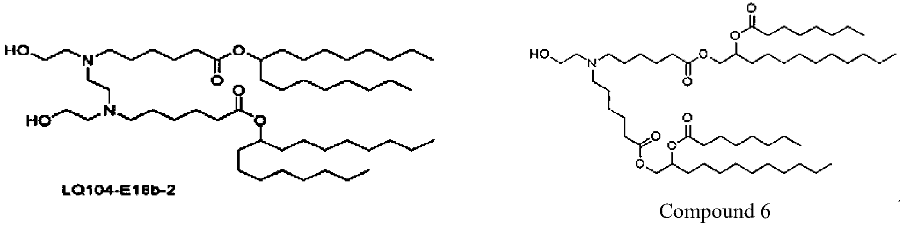 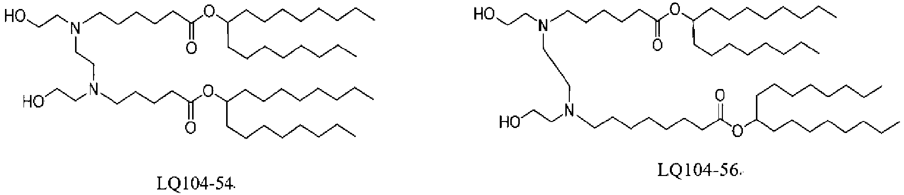 ;
preferably, the molar ratio of cationic lipid RL151, cholesterol, DSPC, and PEGylated lipid, for example DMG-PEG2000, is 50:38.5:10:1.5;
or wherein the molar ratio of LQ104-E16b-2, cholesterol, DSPC, and DMG-PEG2000 is 50:38.5:10:1.5;
or wherein the molar ratio of Compound 6, cholesterol, DSPC, and DMG-PEG2000 is 50:38.5:10:1.5;
or wherein the molar ratio of LQ104-54, cholesterol, DSPC, and DMG-PEG2000 is 50:38.5:10:1.5;
or wherein the molar ratio of LQ104-56, cholesterol, DSPC, and DMG-PEG2000 is 50:38.5:10:1.5;
more preferably, the pharmaceutical composition is a vaccine formulation;
and/or wherein the pharmaceutical composition optionally further comprises a pharmaceutically acceptable carrier and/or excipient;
preferably, the pharmaceutical composition further comprises an adjuvant.

16. A kit or pharmaceutical package comprising one or more of:
the RSV antigen according to any one of claims 1-7;
the isolated nucleic acid according to any one of claims 8-10;
the recombinant expression vector according to claim 11;
the transformed cell according to claim 12; and
the pharmaceutical composition according to claim 15.

17. Use of one or more of:
the RSV antigen according to any one of claims 1-7;
the isolated nucleic acid according to any one of claims 8-10;
the recombinant expression vector according to claim 11;
the transformed cell according to claim 12; and
the pharmaceutical composition according to claim 15,
in the manufacture of a medicament for alleviating, preventing, and/or treating a disease caused by RSV.
